# EUROPEAN PATENT APPLICATION

(11) **EP 3 822 265 A1**
(43) Date of publication of application: **19.05.2021**
(21) Application number: 19383013.0
(22) Date of filing: 15.11.2019
(51) Int. Cl.: C07D 403/12, C07D 403/14, C07D 401/14, C07D 405/14, C07D 413/12, C07D 413/14, C07D 417/14, A61K 31/4192, A61K 31/4439, A61P 9/00

(54) **SUBSTITUTED HYDANTOINAMIDES AS ADAMTS7 ANTAGONISTS**

(71) Applicant: Bayer AG, 51373 Leverkusen (DE); Broad Institute, Cambridge, Massachusetts 02142 (US)
(72) Inventor: Meibom, Daniel, 51373 Leverkusen (DE); Cancho Grande, Yolanda, 51373 Leverkusen (DE); Wasnaire, Pierre, 51373 Leverkusen (DE); Johannes, Sarah, 51373 Leverkusen (DE); Lindner, Niels, 51373 Leverkusen (DE); Beyer, Kristin, 51373 Leverkusen (DE); Freudenberger, Till, 51373 Leverkusen (DE); Brockschnieder, Damian, 51373 Leverkusen (DE); Mathar, Ilka, 51373 Leverkusen (DE); Klar, Jürgen, 51373 Leverkusen (DE); Zubov, Dmitry, 51373 Leverkusen (DE); Menshykau, Dzianis, 51373 Leverkusen (DE); Krainz, Tanja, Cambridge, MA Massachusetts 2142 (US); Stefan, Eric, Cambridge, MA 2142 (US); MacDonald, Bryan, Cambridge, MA Massachusetts 2142 (US); Xing, Yi, Cambridge, MA Massachusetts 2142 (US); Elowe, Nadine, Cambridge, MA Massachusetts 2142 (US); Sanchez, Guzman, 30320 Murcia (ES)
(74) Representative: Clarke Modet & Co.

(57) **Abstract**

The application relates to substituted hydantoinamides of formula (I) as ADAMTS7 antagonists, to processes for their preparation, their use alone or in combination for the treatment or prophylaxis of diseases, in particular of cardiovascular diseases, including atherosclerosis, coronary artery disease (CAD), peripheral vascular disease (PAD), arterial occlusive disease or restenosis after angioplasty. 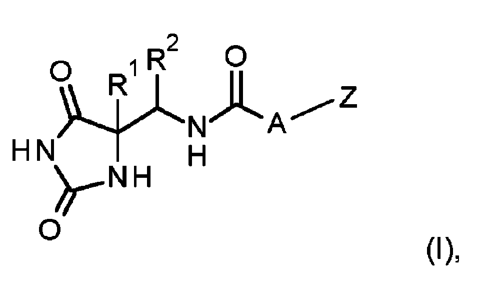 R ¹ is hydrogen, alkyl, cycloalkyl, 5- to 6-membered heterocycloalkyl, 5- to 6-membered heteroaryl or phenyl; R ² is hydrogen or alkyl; A is 5-membered heteroaryl; Z is 6- to 10-membered aryl or 5- to 10-membered heteroaryl; all groups being optionally substituted.

SEQUENCE LISTING

<110>  Bayer AG
       BROAD Institute

<120>  SUBSTITUTED HYDANTOINAMIDES AND THEIR USE / ASSAY FOR
       CHARACTERIZATION OF ADAMTS-7 AND/OR ADAMTS-12 MODULATORS

<130>  BHC181036, BHC191005, BHC191056

<160>  26

<170>  PatentIn version 3.5

<210>  1
<211>  562
<212>  PRT
<213>  Artificial Sequence

<220>
<223>  rPro-hCD (Rat 1-217/Human 237-537)-TEV-2Strep-6His

<220>
<221>  ADAMTS7 rat prodomain (wild-type)
<222>  (1)..(217)

<220>
<221>  ADAMTS7 human catalytic domain
<222>  (218)..(518)

<220>
<221>  TEV cleavage site, 2xStrep tag, and a His tag
<222>  (519)..(562)

<400>  1

Met His Arg Gly Leu Asn Leu Leu Leu Ile Leu Cys Ala Leu Ala Pro
1               5                   10                  15

His Val Leu Gly Pro Ala Ser Gly Leu Pro Thr Glu Gly Arg Ala Gly
            20                  25                  30

Leu Asp Ile Val His Pro Val Arg Val Asp Ala Gly Gly Ser Phe Leu
        35                  40                  45

Ser Tyr Glu Leu Trp Pro Arg Val Leu Arg Lys Arg Asp Val Ser Ala
    50                  55                  60

Ala Gln Ala Ser Ser Ala Phe Tyr Gln Leu Gln Tyr Gln Gly Arg Glu
65                  70                  75                  80

Leu Leu Phe Asn Leu Thr Thr Asn Pro Tyr Leu Leu Ala Pro Gly Phe
                85                  90                  95

Val Ser Glu Ile Arg Arg Arg Ser Asn Leu Ser Asn Val His Ile Gln
            100                 105                 110

Thr Ser Val Pro Thr Cys His Leu Leu Gly Asp Val Gln Asp Pro Glu
        115                 120                 125

Leu Glu Gly Gly Phe Ala Ala Ile Ser Ala Cys Asp Gly Leu Arg Gly
    130                 135                 140

Val Phe Gln Leu Ser Asn Glu Asp Tyr Phe Ile Glu Pro Leu Asp Glu
145                 150                 155                 160

Val Pro Ala Gln Pro Gly His Ala Gln Pro His Met Val Tyr Lys His
                165                 170                 175

Lys Arg Ser Gly Gln Gln Asp Asp Ser Arg Thr Ser Gly Thr Cys Gly
            180                 185                 190

Val Gln Gly Ser Pro Glu Leu Lys His Gln Arg Glu His Trp Glu Gln
        195                 200                 205

Arg Gln Gln Lys Arg Arg Gln Gln Arg Ser Val Ser Lys Glu Lys Trp
    210                 215                 220

Val Glu Thr Leu Val Val Ala Asp Ala Lys Met Val Glu Tyr His Gly
225                 230                 235                 240

Gln Pro Gln Val Glu Ser Tyr Val Leu Thr Ile Met Asn Met Val Ala
                245                 250                 255

Gly Leu Phe His Asp Pro Ser Ile Gly Asn Pro Ile His Ile Thr Ile
            260                 265                 270

Val Arg Leu Val Leu Leu Glu Asp Glu Glu Glu Asp Leu Lys Ile Thr
        275                 280                 285

His His Ala Asp Asn Thr Leu Lys Ser Phe Cys Lys Trp Gln Lys Ser
    290                 295                 300

Ile Asn Met Lys Gly Asp Ala His Pro Leu His His Asp Thr Ala Ile
305                 310                 315                 320

Leu Leu Thr Arg Lys Asp Leu Cys Ala Ala Met Asn Arg Pro Cys Glu
                325                 330                 335

Thr Leu Gly Leu Ser His Val Ala Gly Met Cys Gln Pro His Arg Ser
            340                 345                 350

Cys Ser Ile Asn Glu Asp Thr Gly Leu Pro Leu Ala Phe Thr Val Ala

355                 360                 365

His Glu Leu Gly His Ser Phe Gly Ile Gln His Asp Gly Ser Gly Asn
    370                 375                 380

Asp Cys Glu Pro Val Gly Lys Arg Pro Phe Ile Met Ser Pro Gln Leu
385                 390                 395                 400

Leu Tyr Asp Ala Ala Pro Leu Thr Trp Ser Arg Cys Ser Arg Gln Tyr
                405                 410                 415

Ile Thr Arg Phe Leu Asp Arg Gly Trp Gly Leu Cys Leu Asp Asp Pro
            420                 425                 430

Pro Ala Lys Asp Ile Ile Asp Phe Pro Ser Val Pro Pro Gly Val Leu
        435                 440                 445

Tyr Asp Val Ser His Gln Cys Arg Leu Gln Tyr Gly Ala Tyr Ser Ala
    450                 455                 460

Phe Cys Glu Asp Met Asp Asn Val Cys His Thr Leu Trp Cys Ser Val
465                 470                 475                 480

Gly Thr Thr Cys His Ser Lys Leu Asp Ala Ala Val Asp Gly Thr Arg
                485                 490                 495

Cys Gly Glu Asn Lys Trp Cys Leu Ser Gly Glu Cys Val Pro Val Gly
            500                 505                 510

Phe Arg Pro Glu Ala Val Gly Ser Glu Asn Leu Tyr Phe Gln Ser Gly
        515                 520                 525

Trp Ser His Pro Gln Phe Glu Lys Gly Gly Gly Ser Gly Gly Gly Ser
    530                 535                 540

Gly Gly Gly Ser Trp Ser His Pro Gln Phe Glu Lys His His His His
545                 550                 555                 560

His His

<210>  2
<211>  562
<212>  PRT
<213>  Artificial Sequence

<220>
<223>  rPro-hCD-FM2 (Rat 1-217/Human 237-537 FM2
       (Q216K))-TEV-2Strep-6His

<220>
<221>  ADAMTS7 rat prodomain (furin mutant Q216K)
<222>  (1)..(217)

<220>
<221>  ADAMTS7 human catalytic domain
<222>  (218)..(518)

<220>
<221>  TEV cleavage site, 2xStrep tag, and a His tag
<222>  (519)..(562)

<400>  2

Met His Arg Gly Leu Asn Leu Leu Leu Ile Leu Cys Ala Leu Ala Pro
1               5                   10                  15

His Val Leu Gly Pro Ala Ser Gly Leu Pro Thr Glu Gly Arg Ala Gly
            20                  25                  30

Leu Asp Ile Val His Pro Val Arg Val Asp Ala Gly Gly Ser Phe Leu
        35                  40                  45

Ser Tyr Glu Leu Trp Pro Arg Val Leu Arg Lys Arg Asp Val Ser Ala
    50                  55                  60

Ala Gln Ala Ser Ser Ala Phe Tyr Gln Leu Gln Tyr Gln Gly Arg Glu
65                  70                  75                  80

Leu Leu Phe Asn Leu Thr Thr Asn Pro Tyr Leu Leu Ala Pro Gly Phe
                85                  90                  95

Val Ser Glu Ile Arg Arg Arg Ser Asn Leu Ser Asn Val His Ile Gln
            100                 105                 110

Thr Ser Val Pro Thr Cys His Leu Leu Gly Asp Val Gln Asp Pro Glu
        115                 120                 125

Leu Glu Gly Gly Phe Ala Ala Ile Ser Ala Cys Asp Gly Leu Arg Gly
    130                 135                 140

Val Phe Gln Leu Ser Asn Glu Asp Tyr Phe Ile Glu Pro Leu Asp Glu
145                 150                 155                 160

Val Pro Ala Gln Pro Gly His Ala Gln Pro His Met Val Tyr Lys His
                165                 170                 175

Lys Arg Ser Gly Gln Gln Asp Asp Ser Arg Thr Ser Gly Thr Cys Gly
            180                 185                 190

Val Gln Gly Ser Pro Glu Leu Lys His Gln Arg Glu His Trp Glu Gln
        195                 200                 205

Arg Gln Gln Lys Arg Arg Gln Lys Arg Ser Val Ser Lys Glu Lys Trp
    210                 215                 220

Val Glu Thr Leu Val Val Ala Asp Ala Lys Met Val Glu Tyr His Gly
225                 230                 235                 240

Gln Pro Gln Val Glu Ser Tyr Val Leu Thr Ile Met Asn Met Val Ala
                245                 250                 255

Gly Leu Phe His Asp Pro Ser Ile Gly Asn Pro Ile His Ile Thr Ile
            260                 265                 270

Val Arg Leu Val Leu Leu Glu Asp Glu Glu Glu Asp Leu Lys Ile Thr
        275                 280                 285

His His Ala Asp Asn Thr Leu Lys Ser Phe Cys Lys Trp Gln Lys Ser
    290                 295                 300

Ile Asn Met Lys Gly Asp Ala His Pro Leu His His Asp Thr Ala Ile
305                 310                 315                 320

Leu Leu Thr Arg Lys Asp Leu Cys Ala Ala Met Asn Arg Pro Cys Glu
                325                 330                 335

Thr Leu Gly Leu Ser His Val Ala Gly Met Cys Gln Pro His Arg Ser
            340                 345                 350

Cys Ser Ile Asn Glu Asp Thr Gly Leu Pro Leu Ala Phe Thr Val Ala
        355                 360                 365

His Glu Leu Gly His Ser Phe Gly Ile Gln His Asp Gly Ser Gly Asn
    370                 375                 380

Asp Cys Glu Pro Val Gly Lys Arg Pro Phe Ile Met Ser Pro Gln Leu
385                 390                 395                 400

Leu Tyr Asp Ala Ala Pro Leu Thr Trp Ser Arg Cys Ser Arg Gln Tyr
                405                 410                 415

Ile Thr Arg Phe Leu Asp Arg Gly Trp Gly Leu Cys Leu Asp Asp Pro
            420                 425                 430

Pro Ala Lys Asp Ile Ile Asp Phe Pro Ser Val Pro Pro Gly Val Leu
        435                 440                 445

Tyr Asp Val Ser His Gln Cys Arg Leu Gln Tyr Gly Ala Tyr Ser Ala
    450                 455                 460

Phe Cys Glu Asp Met Asp Asn Val Cys His Thr Leu Trp Cys Ser Val
465                 470                 475                 480

Gly Thr Thr Cys His Ser Lys Leu Asp Ala Ala Val Asp Gly Thr Arg
                485                 490                 495

Cys Gly Glu Asn Lys Trp Cys Leu Ser Gly Glu Cys Val Pro Val Gly
            500                 505                 510

Phe Arg Pro Glu Ala Val Gly Ser Glu Asn Leu Tyr Phe Gln Ser Gly
        515                 520                 525

Trp Ser His Pro Gln Phe Glu Lys Gly Gly Gly Ser Gly Gly Gly Ser
    530                 535                 540

Gly Gly Gly Ser Trp Ser His Pro Gln Phe Glu Lys His His His His
545                 550                 555                 560

His His

<210>  3
<211>  573
<212>  PRT
<213>  Artificial Sequence

<220>
<223>  rADAMTS7 (Rat 1-575)-Flag

<400>  3

Met Pro Cys Ala Gln Gly Asn Trp Met Ala Lys Leu Ser Met Val Ala
1               5                   10                  15

Gln Leu Leu Asn Phe Gly Ala Phe Cys His Gly Arg Gln Ala Gln Pro
            20                  25                  30

Trp Pro Val Arg Phe Pro Asp Pro Lys Gln Glu His Phe Ile Lys Ser
        35                  40                  45

Leu Pro Glu Tyr His Ile Val Ser Pro Val Gln Val Asp Ala Ser Gly
    50                  55                  60

His Phe Leu Ser Tyr Gly Leu His His Pro Val Thr Gly Ser Arg Lys
65                  70                  75                  80

Lys Arg Ala Ala Gly Gly Ser Gly Asp Gln Val Tyr Tyr Arg Ile Ser
                85                  90                  95

His Glu Glu Lys Asn Leu Phe Phe Asn Leu Thr Val Asn Trp Glu Phe
            100                 105                 110

Leu Ser Asn Gly Tyr Val Val Glu Arg Arg Tyr Gly Asn Leu Ser His
        115                 120                 125

Val Lys Met Ala Ala Ser Ser Gly Gln Pro Cys His Leu Arg Gly Thr
    130                 135                 140

Val Leu Gln Gln Gly Pro Thr Ile Arg Met Gly Thr Ala Ala Leu Ser
145                 150                 155                 160

Ala Cys Gln Gly Leu Thr Gly Phe Phe His Leu Pro His Gly Asp Phe
                165                 170                 175

Phe Ile Glu Pro Val Lys Lys His Pro Leu Thr Glu Glu Gly Tyr Gln
            180                 185                 190

Pro His Val Ile Tyr Arg Arg Gln Ser Tyr Arg Val Pro Glu Thr Lys
        195                 200                 205

Glu Pro Thr Cys Gly Leu Lys Asp Ser Leu Asp Asn Ser Val Lys Gln
    210                 215                 220

Glu Leu Gln Arg Glu Lys Trp Glu Arg Lys Asn Trp Pro Ser Arg Ser
225                 230                 235                 240

Leu Ser Arg Arg Ser Ile Ser Lys Glu Arg Trp Val Glu Thr Leu Val
                245                 250                 255

Val Ala Asp Thr Lys Met Ile Glu Tyr His Gly Ser Glu Asn Val Glu
            260                 265                 270

Ser Tyr Ile Leu Thr Ile Met Asn Met Val Thr Gly Leu Phe His Asn
        275                 280                 285

Pro Ser Ile Gly Asn Ala Ile His Ile Val Val Val Arg Leu Ile Leu
    290                 295                 300

Leu Glu Glu Glu Glu Gln Gly Leu Lys Ile Val His His Ala Glu Lys
305                 310                 315                 320

Thr Leu Ser Ser Phe Cys Lys Trp Gln Lys Ser Ile Asn Pro Lys Ser
                325                 330                 335

Asp Leu Asn Pro Val His His Asp Val Ala Val Leu Leu Thr Arg Lys
            340                 345                 350

Asp Ile Cys Ala Gly Phe Asn Arg Pro Cys Glu Thr Leu Gly Leu Ser
        355                 360                 365

His Leu Ser Gly Met Cys Gln Pro His Arg Ser Cys Asn Ile Asn Glu
    370                 375                 380

Asp Ser Gly Leu Pro Leu Ala Phe Thr Ile Ala His Glu Leu Gly His
385                 390                 395                 400

Ser Phe Gly Ile Gln His Asp Gly Lys Glu Asn Asp Cys Glu Pro Val
                405                 410                 415

Gly Arg His Pro Tyr Ile Met Ser Arg Gln Leu Gln Tyr Asp Pro Thr
            420                 425                 430

Pro Leu Thr Trp Ser Lys Cys Ser Glu Glu Tyr Ile Thr Arg Phe Leu
        435                 440                 445

Asp Arg Gly Trp Gly Phe Cys Leu Asp Asp Ile Pro Lys Lys Lys Gly
    450                 455                 460

Leu Lys Ser Lys Val Ile Ala Pro Gly Val Ile Tyr Asp Val His His
465                 470                 475                 480

Gln Cys Gln Leu Gln Tyr Gly Pro Asn Ala Thr Phe Cys Gln Glu Val
                485                 490                 495

Glu Asn Val Cys Gln Thr Leu Trp Cys Ser Val Lys Gly Phe Cys Arg
            500                 505                 510

Ser Lys Leu Asp Ala Ala Ala Asp Gly Thr Gln Cys Gly Glu Lys Lys
        515                 520                 525

Trp Cys Met Ala Gly Lys Cys Ile Thr Val Gly Lys Lys Pro Glu Ser
    530                 535                 540

Ile Pro Gly Gly Gly Gly Ser Asp Tyr Lys Asp His Asp Gly Asp Tyr
545                 550                 555                 560

Lys Asp His Asp Ile Asp Tyr Lys Asp Asp Asp Asp Lys
                565                 570

<210>  4

<211>  16
<212>  PRT
<213>  Artificial Sequence

<220>
<223>  Test substrate for ADAMTS7/12: (HiLyteFluor-488
       )-DELSSMVLELRGLRT-K(QXL520)-NH2

<400>  4

Asp Glu Leu Ser Ser Met Val Leu Glu Leu Arg Gly Leu Arg Thr Lys
1               5                   10                  15


<210>  5
<211>  16
<212>  PRT
<213>  Artificial Sequence

<220>
<223>  Test Substrate for ADAMTS7/12:
       (HiLyteFluor-488)-SSMVLELRGLRTIVT-K(QXL520)-NH2

<400>  5

Ser Ser Met Val Leu Glu Leu Arg Gly Leu Arg Thr Ile Val Thr Lys
1               5                   10                  15


<210>  6
<211>  16
<212>  PRT
<213>  Artificial Sequence

<220>
<223>  Test Substrate for ADAMTS7/12:
       (HiLyteFluor-488)-KVTEENKELANELRR-K(QXL520)-NH2

<400>  6

Lys Val Thr Glu Glu Asn Lys Glu Leu Ala Asn Glu Leu Arg Arg Lys
1               5                   10                  15


<210>  7
<211>  16
<212>  PRT
<213>  Artificial Sequence

<220>
<223>  Test Substrate for ADAMTS7/12:
       (HiLyteFluor-488)-EENKELANELRRPPL-K(QXL520)-NH2

<400>  7

Glu Glu Asn Lys Glu Leu Ala Asn Glu Leu Arg Arg Pro Pro Leu Lys
1               5                   10                  15


<210>  8
<211>  13
<212>  PRT
<213>  Artificial Sequence

<220>
<223>  Test Substrate for ADAMTS7/12:
      (HiLyteFluor-488)-SSMVLELRGLRT-K(QXL520)-NH2

<400>  8

Ser Ser Met Val Leu Glu Leu Arg Gly Leu Arg Thr Lys
1               5                   10


<210>  9
<211>  12
<212>  PRT
<213>  Artificial Sequence

<220>
<223>  Test Substrate for ADAMTS7/12: (Donor) GMQQSVRTGLPS (K-Quencher)

<400>  9

Gly Met Gln Gln Ser Val Arg Thr Gly Leu Pro Ser
1               5                   10


<210>  10
<211>  14
<212>  PRT
<213>  Artificial Sequence

<220>
<223>  Test Substrate for ADAMTS7/12: (Donor) SPGFRCEACPPGYS
      (K-Quencher)

<400>  10

Ser Pro Gly Phe Arg Cys Glu Ala Cys Pro Pro Gly Tyr Ser
1               5                   10


<210>  11
<211>  16
<212>  PRT
<213>  Artificial Sequence

<220>
<223>  Substrate for ADAMTS7 and for ADAMTS12:
      (HiLyteFluor-488)-DELSSMVLELRGLRT-K(QXL520)-E-NH2

<400>  11

Asp Glu Leu Ser Ser Met Val Leu Glu Leu Arg Gly Leu Arg Thr Lys
1               5                   10                  15


<210>  12
<211>  16
<212>  PRT
<213>  Artificial Sequence

<220>
<223>  Test Substrate for ADAMTS7/12:
      (HiLyteFluor-488)-DELSSMVLELRGLRT-K(QXL520)-K-NH2

<400>  12

Asp Glu Leu Ser Ser Met Val Leu Glu Leu Arg Gly Leu Arg Thr Lys
1               5                   10                  15


<210>  13
<211>  16
<212>  PRT
<213>  Artificial Sequence

<220>
<223>  Test Substrate for ADAMTS7/12:
       (HiLyteFluor-488)-DELSSMVLELRGLRT-K(QXL520)-OH

<400>  13

Asp Glu Leu Ser Ser Met Val Leu Glu Leu Arg Gly Leu Arg Thr Lys
1               5                   10                  15


<210>  14
<211>  10
<212>  PRT
<213>  Artificial Sequence

<220>
<223>  Substrate for ADAMTS4 and for ADAMTS5:
       Dabcyl-EEVKAKVQPY-Glu(Edans)-NH2

<400>  14

Glu Glu Val Lys Ala Lys Val Gln Pro Tyr
1               5                   10


<210>  15
<211>  573
<212>  PRT
<213>  Artificial Sequence

<220>
<223>  rPro-hCD for ADAMTS12

<400>  15

Met Pro Cys Ala Gln Gly Asn Trp Met Ala Lys Leu Ser Met Val Ala
1               5                   10                  15


Gln Leu Leu Asn Phe Gly Ala Phe Cys His Gly Arg Gln Ala Gln Pro
            20                  25                  30


Trp Pro Val Arg Phe Pro Asp Pro Lys Gln Glu His Phe Ile Lys Ser
        35                  40                  45


Leu Pro Glu Tyr His Ile Val Ser Pro Val Gln Val Asp Ala Ser Gly
    50                  55                  60

His Phe Leu Ser Tyr Gly Leu His His Pro Val Thr Gly Ser Arg Lys
65                  70                  75                  80

Lys Arg Ala Ala Gly Gly Ser Gly Asp Gln Val Tyr Tyr Arg Ile Ser
                85                  90                  95

His Glu Glu Lys Asn Leu Phe Phe Asn Leu Thr Val Asn Trp Glu Phe
            100                 105                 110

Leu Ser Asn Gly Tyr Val Val Glu Arg Arg Tyr Gly Asn Leu Ser His
        115                 120                 125

Val Lys Met Ala Ala Ser Ser Gly Gln Pro Cys His Leu Arg Gly Thr
    130                 135                 140

Val Leu Gln Gln Gly Pro Thr Ile Arg Met Gly Thr Ala Ala Leu Ser
145                 150                 155                 160

Ala Cys Gln Gly Leu Thr Gly Phe Phe His Leu Pro His Gly Asp Phe
                165                 170                 175

Phe Ile Glu Pro Val Lys Lys His Pro Leu Thr Glu Glu Gly Tyr Gln
            180                 185                 190

Pro His Val Ile Tyr Arg Arg Gln Ser Tyr Arg Val Pro Glu Thr Lys
        195                 200                 205

Glu Pro Thr Cys Gly Leu Lys Asp Ser Leu Asp Asn Ser Val Lys Gln
    210                 215                 220

Glu Leu Gln Arg Glu Lys Trp Glu Arg Lys Asn Trp Pro Ser Arg Ser
225                 230                 235                 240

Leu Ser Arg Arg Ser Ile Ser Lys Glu Arg Trp Val Glu Thr Leu Val
                245                 250                 255

Val Ala Asp Thr Lys Met Ile Glu Tyr His Gly Ser Glu Asn Val Glu
            260                 265                 270

Ser Tyr Ile Leu Thr Ile Met Asn Met Val Thr Gly Leu Phe His Asn
        275                 280                 285

Pro Ser Ile Gly Asn Ala Ile His Ile Val Val Val Arg Leu Ile Leu
    290                 295                 300

Leu Glu Glu Glu Glu Gln Gly Leu Lys Ile Val His His Ala Glu Lys
305                 310                 315                 320

Thr Leu Ser Ser Phe Cys Lys Trp Gln Lys Ser Ile Asn Pro Lys Ser
                325                 330                 335

Asp Leu Asn Pro Val His His Asp Val Ala Val Leu Leu Thr Arg Lys
            340                 345                 350

Asp Ile Cys Ala Gly Phe Asn Arg Pro Cys Glu Thr Leu Gly Leu Ser
        355                 360                 365

His Leu Ser Gly Met Cys Gln Pro His Arg Ser Cys Asn Ile Asn Glu
    370                 375                 380

Asp Ser Gly Leu Pro Leu Ala Phe Thr Ile Ala His Glu Leu Gly His
385                 390                 395                 400

Ser Phe Gly Ile Gln His Asp Gly Lys Glu Asn Asp Cys Glu Pro Val
                405                 410                 415

Gly Arg His Pro Tyr Ile Met Ser Arg Gln Leu Gln Tyr Asp Pro Thr
            420                 425                 430

Pro Leu Thr Trp Ser Lys Cys Ser Glu Glu Tyr Ile Thr Arg Phe Leu
        435                 440                 445

Asp Arg Gly Trp Gly Phe Cys Leu Asp Asp Ile Pro Lys Lys Lys Gly
    450                 455                 460

Leu Lys Ser Lys Val Ile Ala Pro Gly Val Ile Tyr Asp Val His His
465                 470                 475                 480

Gln Cys Gln Leu Gln Tyr Gly Pro Asn Ala Thr Phe Cys Gln Glu Val
                485                 490                 495

Glu Asn Val Cys Gln Thr Leu Trp Cys Ser Val Lys Gly Phe Cys Arg
            500                 505                 510

Ser Lys Leu Asp Ala Ala Ala Asp Gly Thr Gln Cys Gly Glu Lys Lys
        515                 520                 525

Trp Cys Met Ala Gly Lys Cys Ile Thr Val Gly Lys Lys Pro Glu Ser
    530                 535                 540

Ile Pro Gly Gly Gly Gly Ser Asp Tyr Lys Asp His Asp Gly Asp Tyr
545                 550                 555                 560

Lys Asp His Asp Ile Asp Tyr Lys Asp Asp Asp Asp Lys
                565                 570

<210>  16
<211>  14
<212>  PRT
<213>  Artificial Sequence

<220>
<223>  ADAMTS Catalytic Motif Consensus Sequence


<220>
<221>  misc_feature
<222>  (3)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (12)..(12)
<223>  Xaa can be any naturally occurring amino acid

<400>  16

His Glu Xaa Xaa His Xaa Asx Gly Asn Ser Asx Xaa His Asp
1               5                   10


<210>  17
<211>  7
<212>  PRT
<213>  Artificial Sequence

<220>
<223>  Substrate for MMP12: Mca-Pro-Leu-Gly-Leu-Glu-Glu-Ala-Dap(Dnp)-NH2

<400>  17

Pro Leu Gly Leu Glu Glu Ala
1               5


<210>  18
<211>  8
<212>  PRT
<213>  Artificial Sequence

<220>
<223>  Substrate for MMP15: MCA-Lys-Pro-Leu-Gly-Leu-DPA-Ala-Arg-NH2


<220>
<221>  X
<222>  (6)..(6)
<223>  DPA

<400>  18

Lys Pro Leu Gly Leu Xaa Ala Arg

1               5


<210>  19
<211>  7
<212>  PRT
<213>  Artificial Sequence

<220>
<223>  Substrate for MMP2: MCA-Pro-Leu-Gly-Leu-DPA-Ala-Arg-NH2


<220>
<221>  X
<222>  (5)..(5)
<223>  DPA

<400>  19

Pro Leu Gly Leu Xaa Ala Arg
1               5


<210>  20
<211>  12
<212>  PRT
<213>  Artificial Sequence

<220>
<223>  Substrate for ADAM17:
       Mca-Pro-Leu-Ala-Gln-Ala-Val-Dap(Dnp)-Arg-Ser-Ser-Ser-Arg-NH2


<220>
<221>  X
<222>  (7)..(7)
<223>  Dap(Dnp)

<400>  20

Pro Leu Ala Gln Ala Val Xaa Arg Ser Ser Ser Arg
1               5                   10


<210>  21
<211>  1686
<212>  PRT
<213>  Artificial Sequence

<220>
<223>  Human ADAMTS7 from Q9UKP4

<400>  21

Met Pro Gly Gly Pro Ser Pro Arg Ser Pro Ala Pro Leu Leu Arg Pro
1               5                   10                  15


Leu Leu Leu Leu Leu Cys Ala Leu Ala Pro Gly Ala Pro Gly Pro Ala
            20                  25                  30


Pro Gly Arg Ala Thr Glu Gly Arg Ala Ala Leu Asp Ile Val His Pro

        35                  40                  45

Val Arg Val Asp Ala Gly Gly Ser Phe Leu Ser Tyr Glu Leu Trp Pro
    50                  55                  60

Arg Ala Leu Arg Lys Arg Asp Val Ser Val Arg Arg Asp Ala Pro Ala
65                  70                  75                  80

Phe Tyr Glu Leu Gln Tyr Arg Gly Arg Glu Leu Arg Phe Asn Leu Thr
                85                  90                  95

Ala Asn Gln His Leu Leu Ala Pro Gly Phe Val Ser Glu Thr Arg Arg
            100                 105                 110

Arg Gly Gly Leu Gly Arg Ala His Ile Arg Ala His Thr Pro Ala Cys
        115                 120                 125

His Leu Leu Gly Glu Val Gln Asp Pro Glu Leu Glu Gly Gly Leu Ala
    130                 135                 140

Ala Ile Ser Ala Cys Asp Gly Leu Lys Gly Val Phe Gln Leu Ser Asn
145                 150                 155                 160

Glu Asp Tyr Phe Ile Glu Pro Leu Asp Ser Ala Pro Ala Arg Pro Gly
                165                 170                 175

His Ala Gln Pro His Val Val Tyr Lys Arg Gln Ala Pro Glu Arg Leu
            180                 185                 190

Ala Gln Arg Gly Asp Ser Ser Ala Pro Ser Thr Cys Gly Val Gln Val
        195                 200                 205

Tyr Pro Glu Leu Glu Ser Arg Arg Glu Arg Trp Glu Gln Arg Gln Gln
    210                 215                 220

Trp Arg Arg Pro Arg Leu Arg Arg Leu His Gln Arg Ser Val Ser Lys
225                 230                 235                 240

Glu Lys Trp Val Glu Thr Leu Val Val Ala Asp Ala Lys Met Val Glu
                245                 250                 255

Tyr His Gly Gln Pro Gln Val Glu Ser Tyr Val Leu Thr Ile Met Asn
            260                 265                 270

Met Val Ala Gly Leu Phe His Asp Pro Ser Ile Gly Asn Pro Ile His
        275                 280                 285

Ile Thr Ile Val Arg Leu Val Leu Leu Glu Asp Glu Glu Glu Asp Leu
    290                 295                 300

Lys Ile Thr His His Ala Asp Asn Thr Leu Lys Ser Phe Cys Lys Trp
305                 310                 315                 320

Gln Lys Ser Ile Asn Met Lys Gly Asp Ala His Pro Leu His His Asp
                325                 330                 335

Thr Ala Ile Leu Leu Thr Arg Lys Asp Leu Cys Ala Ala Met Asn Arg
            340                 345                 350

Pro Cys Glu Thr Leu Gly Leu Ser His Val Ala Gly Met Cys Gln Pro
        355                 360                 365

His Arg Ser Cys Ser Ile Asn Glu Asp Thr Gly Leu Pro Leu Ala Phe
    370                 375                 380

Thr Val Ala His Glu Leu Gly His Ser Phe Gly Ile Gln His Asp Gly
385                 390                 395                 400

Ser Gly Asn Asp Cys Glu Pro Val Gly Lys Arg Pro Phe Ile Met Ser
                405                 410                 415

Pro Gln Leu Leu Tyr Asp Ala Ala Pro Leu Thr Trp Ser Arg Cys Ser
            420                 425                 430

Arg Gln Tyr Ile Thr Arg Phe Leu Asp Arg Gly Trp Gly Leu Cys Leu
        435                 440                 445

Asp Asp Pro Pro Ala Lys Asp Ile Ile Asp Phe Pro Ser Val Pro Pro
    450                 455                 460

Gly Val Leu Tyr Asp Val Ser His Gln Cys Arg Leu Gln Tyr Gly Ala
465                 470                 475                 480

Tyr Ser Ala Phe Cys Glu Asp Met Asp Asn Val Cys His Thr Leu Trp
                485                 490                 495

Cys Ser Val Gly Thr Thr Cys His Ser Lys Leu Asp Ala Ala Val Asp
            500                 505                 510

Gly Thr Arg Cys Gly Glu Asn Lys Trp Cys Leu Ser Gly Glu Cys Val
        515                 520                 525

Pro Val Gly Phe Arg Pro Glu Ala Val Asp Gly Gly Trp Ser Gly Trp
    530                 535                 540

Ser Ala Trp Ser Ile Cys Ser Arg Ser Cys Gly Met Gly Val Gln Ser
545                 550                 555                 560

Ala Glu Arg Gln Cys Thr Gln Pro Thr Pro Lys Tyr Lys Gly Arg Tyr
                565                 570                 575

Cys Val Gly Glu Arg Lys Arg Phe Arg Leu Cys Asn Leu Gln Ala Cys
            580                 585                 590

Pro Ala Gly Arg Pro Ser Phe Arg His Val Gln Cys Ser His Phe Asp
        595                 600                 605

Ala Met Leu Tyr Lys Gly Gln Leu His Thr Trp Val Pro Val Val Asn
    610                 615                 620

Asp Val Asn Pro Cys Glu Leu His Cys Arg Pro Ala Asn Glu Tyr Phe
625                 630                 635                 640

Ala Glu Lys Leu Arg Asp Ala Val Val Asp Gly Thr Pro Cys Tyr Gln
                645                 650                 655

Val Arg Ala Ser Arg Asp Leu Cys Ile Asn Gly Ile Cys Lys Asn Val
            660                 665                 670

Gly Cys Asp Phe Glu Ile Asp Ser Gly Ala Met Glu Asp Arg Cys Gly
        675                 680                 685

Val Cys His Gly Asn Gly Ser Thr Cys His Thr Val Ser Gly Thr Phe
    690                 695                 700

Glu Glu Ala Glu Gly Leu Gly Tyr Val Asp Val Gly Leu Ile Pro Ala
705                 710                 715                 720

Gly Ala Arg Glu Ile Arg Ile Gln Glu Val Ala Glu Ala Ala Asn Phe
                725                 730                 735

Leu Ala Leu Arg Ser Glu Asp Pro Glu Lys Tyr Phe Leu Asn Gly Gly
            740                 745                 750

Trp Thr Ile Gln Trp Asn Gly Asp Tyr Gln Val Ala Gly Thr Thr Phe
        755                 760                 765

Thr Tyr Ala Arg Arg Gly Asn Trp Glu Asn Leu Thr Ser Pro Gly Pro
    770                 775                 780

Thr Lys Glu Pro Val Trp Ile Gln Leu Leu Phe Gln Glu Ser Asn Pro
785                 790                 795                 800

Gly Val His Tyr Glu Tyr Thr Ile His Arg Glu Ala Gly Gly His Asp
                805                 810                 815

Glu Val Pro Pro Pro Val Phe Ser Trp His Tyr Gly Pro Trp Thr Lys
            820                 825                 830

Cys Thr Val Thr Cys Gly Arg Gly Val Gln Arg Gln Asn Val Tyr Cys
        835                 840                 845

Leu Glu Arg Gln Ala Gly Pro Val Asp Glu Glu His Cys Asp Pro Leu
    850                 855                 860

Gly Arg Pro Asp Asp Gln Gln Arg Lys Cys Ser Glu Gln Pro Cys Pro
865                 870                 875                 880

Ala Arg Trp Trp Ala Gly Glu Trp Gln Leu Cys Ser Ser Ser Cys Gly
                885                 890                 895

Pro Gly Gly Leu Ser Arg Arg Ala Val Leu Cys Ile Arg Ser Val Gly
            900                 905                 910

Leu Asp Glu Gln Ser Ala Leu Glu Pro Pro Ala Cys Glu His Leu Pro
        915                 920                 925

Arg Pro Pro Thr Glu Thr Pro Cys Asn Arg His Val Pro Cys Pro Ala
    930                 935                 940

Thr Trp Ala Val Gly Asn Trp Ser Gln Cys Ser Val Thr Cys Gly Glu
945                 950                 955                 960

Gly Thr Gln Arg Arg Asn Val Leu Cys Thr Asn Asp Thr Gly Val Pro
                965                 970                 975

Cys Asp Glu Ala Gln Gln Pro Ala Ser Glu Val Thr Cys Ser Leu Pro
            980                 985                 990

Leu Cys Arg Trp Pro Leu Gly Thr  Leu Gly Pro Glu Gly  Ser Gly Ser
        995                 1000                 1005

Gly Ser  Ser Ser His Glu Leu  Phe Asn Glu Ala Asp  Phe Ile Pro
    1010                 1015                 1020

His His  Leu Ala Pro Arg Pro  Ser Pro Ala Ser Ser  Pro Lys Pro
    1025                 1030                 1035

Gly Thr  Met Gly Asn Ala Ile  Glu Glu Glu Ala Pro  Glu Leu Asp

1040                1045                1050

Leu Pro Gly Pro Val Phe Val Asp Asp Phe Tyr Tyr Asp Tyr Asn
    1055                1060                1065

Phe Ile Asn Phe His Glu Asp Leu Ser Tyr Gly Pro Ser Glu Glu
    1070                1075                1080

Pro Asp Leu Asp Leu Ala Gly Thr Gly Asp Arg Thr Pro Pro Pro
    1085                1090                1095

His Ser His Pro Ala Ala Pro Ser Thr Gly Ser Pro Val Pro Ala
    1100                1105                1110

Thr Glu Pro Pro Ala Ala Lys Glu Glu Gly Val Leu Gly Pro Trp
    1115                1120                1125

Ser Pro Ser Pro Trp Pro Ser Gln Ala Gly Arg Ser Pro Pro Pro
    1130                1135                1140

Pro Ser Glu Gln Thr Pro Gly Asn Pro Leu Ile Asn Phe Leu Pro
    1145                1150                1155

Glu Glu Asp Thr Pro Ile Gly Ala Pro Asp Leu Gly Leu Pro Ser
    1160                1165                1170

Leu Ser Trp Pro Arg Val Ser Thr Asp Gly Leu Gln Thr Pro Ala
    1175                1180                1185

Thr Pro Glu Ser Gln Asn Asp Phe Pro Val Gly Lys Asp Ser Gln
    1190                1195                1200

Ser Gln Leu Pro Pro Pro Trp Arg Asp Arg Thr Asn Glu Val Phe
    1205                1210                1215

Lys Asp Asp Glu Glu Pro Lys Gly Arg Gly Ala Pro His Leu Pro
    1220                1225                1230

Pro Arg Pro Ser Ser Thr Leu Pro Pro Leu Ser Pro Val Gly Ser
    1235                1240                1245

Thr His Ser Ser Pro Ser Pro Asp Val Ala Glu Leu Trp Thr Gly
    1250                1255                1260

Gly Thr Val Ala Trp Glu Pro Ala Leu Glu Gly Gly Leu Gly Pro
    1265                1270                1275

Val Asp Ser Glu Leu Trp Pro Thr Val Gly Val Ala Ser Leu Leu
    1280                1285                1290

Pro Pro Pro Ile Ala Pro Leu Pro Glu Met Lys Val Arg Asp Ser
    1295                1300                1305

Ser Leu Glu Pro Gly Thr Pro Ser Phe Pro Thr Pro Gly Pro Gly
    1310                1315                1320

Ser Trp Asp Leu Gln Thr Val Ala Val Trp Gly Thr Phe Leu Pro
    1325                1330                1335

Thr Thr Leu Thr Gly Leu Gly His Met Pro Glu Pro Ala Leu Asn
    1340                1345                1350

Pro Gly Pro Lys Gly Gln Pro Glu Ser Leu Ser Pro Glu Val Pro
    1355                1360                1365

Leu Ser Ser Arg Leu Leu Ser Thr Pro Ala Trp Asp Ser Pro Ala
    1370                1375                1380

Asn Ser His Arg Val Pro Glu Thr Gln Pro Leu Ala Pro Ser Leu
    1385                1390                1395

Ala Glu Ala Gly Pro Pro Ala Asp Pro Leu Val Val Arg Asn Ala
    1400                1405                1410

Gly Trp Gln Ala Gly Asn Trp Ser Glu Cys Ser Thr Thr Cys Gly
    1415                1420                1425

Leu Gly Ala Val Trp Arg Pro Val Arg Cys Ser Ser Gly Arg Asp
    1430                1435                1440

Glu Asp Cys Ala Pro Ala Gly Arg Pro Gln Pro Ala Arg Arg Cys
    1445                1450                1455

His Leu Arg Pro Cys Ala Thr Trp His Ser Gly Asn Trp Ser Lys
    1460                1465                1470

Cys Ser Arg Ser Cys Gly Gly Gly Ser Ser Val Arg Asp Val Gln
    1475                1480                1485

Cys Val Asp Thr Arg Asp Leu Arg Pro Leu Arg Pro Phe His Cys
    1490                1495                1500

Gln Pro Gly Pro Ala Lys Pro Pro Ala His Arg Pro Cys Gly Ala
    1505                1510                1515

Gln Pro Cys Leu Ser Trp Tyr Thr Ser Ser Trp Arg Glu Cys Ser
    1520                1525                1530

Glu Ala Cys Gly Gly Gly Glu Gln Gln Arg Leu Val Thr Cys Pro
    1535                1540                1545

Glu Pro Gly Leu Cys Glu Glu Ala Leu Arg Pro Asn Thr Thr Arg
    1550                1555                1560

Pro Cys Asn Thr His Pro Cys Thr Gln Trp Val Val Gly Pro Trp
    1565                1570                1575

Gly Gln Cys Ser Gly Pro Cys Gly Gly Gly Val Gln Arg Arg Leu
    1580                1585                1590

Val Lys Cys Val Asn Thr Gln Thr Gly Leu Pro Glu Glu Asp Ser
    1595                1600                1605

Asp Gln Cys Gly His Glu Ala Trp Pro Glu Ser Ser Arg Pro Cys
    1610                1615                1620

Gly Thr Glu Asp Cys Glu Pro Val Glu Pro Pro Arg Cys Glu Arg
    1625                1630                1635

Asp Arg Leu Ser Phe Gly Phe Cys Glu Thr Leu Arg Leu Leu Gly
    1640                1645                1650

Arg Cys Gln Leu Pro Thr Ile Arg Thr Gln Cys Cys Arg Ser Cys
    1655                1660                1665

Ser Pro Pro Ser His Gly Ala Pro Ser Arg Gly His Gln Arg Val
    1670                1675                1680

Ala Arg Arg Met Pro Gly Gly Pro Ser Pro Arg Ser Pro Ala Pro Leu
1685 1 5 10

Leu Arg Pro Leu Leu Leu Leu Leu Cys Ala Leu Ala Pro Gly Ala Pro
15 20 25

Gly Pro Ala Pro Gly Arg Ala Thr Glu Gly Arg Ala Ala Leu Asp Ile
30 35 40 45

Val His Pro Val Arg Val Asp Ala Gly Gly Ser Phe Leu Ser Tyr Glu
50 55 60

Leu Trp Pro Arg Ala Leu Arg Lys Arg Asp Val Ser Val Arg Arg Asp
65 70 75

Ala Pro Ala Phe Tyr Glu Leu Gln Tyr Arg Gly Arg Glu Leu Arg Phe
80 85 90

Asn Leu Thr Ala Asn Gln His Leu Leu Ala Pro Gly Phe Val Ser Glu
95 100 105

Thr Arg Arg Arg Gly Gly Leu Gly Arg Ala His Ile Arg Ala His Thr
110 115 120 125

Pro Ala Cys His Leu Leu Gly Glu Val Gln Asp Pro Glu Leu Glu Gly
130 135 140

Gly Leu Ala Ala Ile Ser Ala Cys Asp Gly Leu Lys Gly Val Phe Gln
145 150 155

Leu Ser Asn Glu Asp Tyr Phe Ile Glu Pro Leu Asp Ser Ala Pro Ala
160 165 170

Arg Pro Gly His Ala Gln Pro His Val Val Tyr Lys Arg Gln Ala Pro
175 180 185

Glu Arg Leu Ala Gln Arg Gly Asp Ser Ser Ala Pro Ser Thr Cys Gly
190 195 200 205

Val Gln Val Tyr Pro Glu Leu Glu Ser Arg Arg Glu Arg Trp Glu Gln
210 215 220

Arg Gln Gln Trp Arg Arg Pro Arg Leu Arg Arg Leu His Gln Arg Ser
225 230 235

Val Ser Lys Glu Lys Trp Val Glu Thr Leu Val Val Ala Asp Ala Lys
240 245 250

Met Val Glu Tyr His Gly Gln Pro Gln Val Glu Ser Tyr Val Leu Thr
255 260 265

Ile Met Asn Met Val Ala Gly Leu Phe His Asp Pro Ser Ile Gly Asn
270 275 280 285

Pro Ile His Ile Thr Ile Val Arg Leu Val Leu Leu Glu Asp Glu Glu
290 295 300

Glu Asp Leu Lys Ile Thr His His Ala Asp Asn Thr Leu Lys Ser Phe
305 310 315

Cys Lys Trp Gln Lys Ser Ile Asn Met Lys Gly Asp Ala His Pro Leu

320 325 330

His His Asp Thr Ala Ile Leu Leu Thr Arg Lys Asp Leu Cys Ala Ala
335 340 345

Met Asn Arg Pro Cys Glu Thr Leu Gly Leu Ser His Val Ala Gly Met
350 355 360 365

Cys Gln Pro His Arg Ser Cys Ser Ile Asn Glu Asp Thr Gly Leu Pro
370 375 380

Leu Ala Phe Thr Val Ala His Glu Leu Gly His Ser Phe Gly Ile Gln
385 390 395

His Asp Gly Ser Gly Asn Asp Cys Glu Pro Val Gly Lys Arg Pro Phe
400 405 410

Ile Met Ser Pro Gln Leu Leu Tyr Asp Ala Ala Pro Leu Thr Trp Ser
415 420 425

Arg Cys Ser Arg Gln Tyr Ile Thr Arg Phe Leu Asp Arg Gly Trp Gly
430 435 440 445

Leu Cys Leu Asp Asp Pro Pro Ala Lys Asp Ile Ile Asp Phe Pro Ser
450 455 460

Val Pro Pro Gly Val Leu Tyr Asp Val Ser His Gln Cys Arg Leu Gln
465 470 475

Tyr Gly Ala Tyr Ser Ala Phe Cys Glu Asp Met Asp Asn Val Cys His
480 485 490

Thr Leu Trp Cys Ser Val Gly Thr Thr Cys His Ser Lys Leu Asp Ala
495 500 505

Ala Val Asp Gly Thr Arg Cys Gly Glu Asn Lys Trp Cys Leu Ser Gly
510 515 520 525

Glu Cys Val Pro Val Gly Phe Arg Pro Glu Ala Val Asp Gly Gly Trp
530 535 540

Ser Gly Trp Ser Ala Trp Ser Ile Cys Ser Arg Ser Cys Gly Met Gly
545 550 555

Val Gln Ser Ala Glu Arg Gln Cys Thr Gln Pro Thr Pro Lys Tyr Lys
560 565 570

Gly Arg Tyr Cys Val Gly Glu Arg Lys Arg Phe Arg Leu Cys Asn Leu
    575                 580                 585

Gln Ala Cys Pro Ala Gly Arg Pro Ser Phe Arg His Val Gln Cys Ser
590                 595                 600                 605

His Phe Asp Ala Met Leu Tyr Lys Gly Gln Leu His Thr Trp Val Pro
                610                 615                 620

Val Val Asn Asp Val Asn Pro Cys Glu Leu His Cys Arg Pro Ala Asn
            625                 630                 635

Glu Tyr Phe Ala Glu Lys Leu Arg Asp Ala Val Val Asp Gly Thr Pro
        640                 645                 650

Cys Tyr Gln Val Arg Ala Ser Arg Asp Leu Cys Ile Asn Gly Ile Cys
    655                 660                 665

Lys Asn Val Gly Cys Asp Phe Glu Ile Asp Ser Gly Ala Met Glu Asp
670                 675                 680                 685

Arg Cys Gly Val Cys His Gly Asn Gly Ser Thr Cys His Thr Val Ser
                690                 695                 700

Gly Thr Phe Glu Glu Ala Glu Gly Leu Gly Tyr Val Asp Val Gly Leu
            705                 710                 715

Ile Pro Ala Gly Ala Arg Glu Ile Arg Ile Gln Glu Val Ala Glu Ala
        720                 725                 730

Ala Asn Phe Leu Ala Leu Arg Ser Glu Asp Pro Glu Lys Tyr Phe Leu
    735                 740                 745

Asn Gly Gly Trp Thr Ile Gln Trp Asn Gly Asp Tyr Gln Val Ala Gly
750                 755                 760                 765

Thr Thr Phe Thr Tyr Ala Arg Arg Gly Asn Trp Glu Asn Leu Thr Ser
                770                 775                 780

Pro Gly Pro Thr Lys Glu Pro Val Trp Ile Gln Leu Leu Phe Gln Glu
            785                 790                 795

Ser Asn Pro Gly Val His Tyr Glu Tyr Thr Ile His Arg Glu Ala Gly
        800                 805                 810

Gly His Asp Glu Val Pro Pro Pro Val Phe Ser Trp His Tyr Gly Pro
    815                 820                 825

Trp Thr Lys Cys Thr Val Thr Cys Gly Arg Gly Val Gln Arg Gln Asn
830                 835                 840                 845

Val Tyr Cys Leu Glu Arg Gln Ala Gly Pro Val Asp Glu Glu His Cys
                850                 855                 860

Asp Pro Leu Gly Arg Pro Asp Asp Gln Gln Arg Lys Cys Ser Glu Gln
            865                 870                 875

Pro Cys Pro Ala Arg Trp Trp Ala Gly Glu Trp Gln Leu Cys Ser Ser
        880                 885                 890

Ser Cys Gly Pro Gly Gly Leu Ser Arg Arg Ala Val Leu Cys Ile Arg
    895                 900                 905

Ser Val Gly Leu Asp Glu Gln Ser Ala Leu Glu Pro Pro Ala Cys Glu
910                 915                 920                 925

His Leu Pro Arg Pro Pro Thr Glu Thr Pro Cys Asn Arg His Val Pro
                930                 935                 940

Cys Pro Ala Thr Trp Ala Val Gly Asn Trp Ser Gln Cys Ser Val Thr
            945                 950                 955

Cys Gly Glu Gly Thr Gln Arg Arg Asn Val Leu Cys Thr Asn Asp Thr
        960                 965                 970

Gly Val Pro Cys Asp Glu Ala Gln Gln Pro Ala Ser Glu Val Thr Cys
    975                 980                 985

Ser Leu Pro Leu Cys Arg Trp Pro Leu Gly Thr  Leu Gly Pro Glu Gly
990                 995                 1000                1005

Ser Gly Ser Gly Ser  Ser Ser His Glu Leu  Phe Asn Glu Ala Asp
                1010                 1015                 1020

Phe Ile Pro His His  Leu Ala Pro Arg Pro  Ser Pro Ala Ser Ser
                1025                 1030                 1035

Pro Lys Pro Gly Thr  Met Gly Asn Ala Ile  Glu Glu Glu Ala Pro
                1040                 1045                 1050

Glu Leu Asp Leu Pro  Gly Pro Val Phe Val  Asp Asp Phe Tyr Tyr
                1055                 1060                 1065

Asp Tyr Asn Phe Ile  Asn Phe His Glu Asp  Leu Ser Tyr Gly Pro
                1070                 1075                 1080

Ser Glu Glu Pro Asp Leu Asp Leu Ala Gly Thr Gly Asp Arg Thr
1085 1090 1095

Pro Pro Pro His Ser His Pro Ala Ala Pro Ser Thr Gly Ser Pro
1100 1105 1110

Val Pro Ala Thr Glu Pro Pro Ala Ala Lys Glu Glu Gly Val Leu
1115 1120 1125

Gly Pro Trp Ser Pro Ser Pro Trp Pro Ser Gln Ala Gly Arg Ser
1130 1135 1140

Pro Pro Pro Pro Ser Glu Gln Thr Pro Gly Asn Pro Leu Ile Asn
1145 1150 1155

Phe Leu Pro Glu Glu Asp Thr Pro Ile Gly Ala Pro Asp Leu Gly
1160 1165 1170

Leu Pro Ser Leu Ser Trp Pro Arg Val Ser Thr Asp Gly Leu Gln
1175 1180 1185

Thr Pro Ala Thr Pro Glu Ser Gln Asn Asp Phe Pro Val Gly Lys
1190 1195 1200

Asp Ser Gln Ser Gln Leu Pro Pro Pro Trp Arg Asp Arg Thr Asn
1205 1210 1215

Glu Val Phe Lys Asp Asp Glu Glu Pro Lys Gly Arg Gly Ala Pro
1220 1225 1230

His Leu Pro Pro Arg Pro Ser Ser Thr Leu Pro Pro Leu Ser Pro
1235 1240 1245

Val Gly Ser Thr His Ser Ser Pro Ser Pro Asp Val Ala Glu Leu
1250 1255 1260

Trp Thr Gly Gly Thr Val Ala Trp Glu Pro Ala Leu Glu Gly Gly
1265 1270 1275

Leu Gly Pro Val Asp Ser Glu Leu Trp Pro Thr Val Gly Val Ala
1280 1285 1290

Ser Leu Leu Pro Pro Pro Ile Ala Pro Leu Pro Glu Met Lys Val
1295 1300 1305

Arg Asp Ser Ser Leu Glu Pro Gly Thr Pro Ser Phe Pro Thr Pro

1310                    1315                    1320

Gly Pro Gly Ser Trp  Asp Leu Gln Thr Val  Ala Val Trp Gly Thr
                1325                    1330                    1335

Phe Leu Pro Thr Thr  Leu Thr Gly Leu Gly  His Met Pro Glu Pro
                1340                    1345                    1350

Ala Leu Asn Pro Gly  Pro Lys Gly Gln Pro  Glu Ser Leu Ser Pro
                1355                    1360                    1365

Glu Val Pro Leu Ser  Ser Arg Leu Leu Ser  Thr Pro Ala Trp Asp
                1370                    1375                    1380

Ser Pro Ala Asn Ser  His Arg Val Pro Glu  Thr Gln Pro Leu Ala
                1385                    1390                    1395

Pro Ser Leu Ala Glu  Ala Gly Pro Pro Ala  Asp Pro Leu Val Val
                1400                    1405                    1410

Arg Asn Ala Gly Trp  Gln Ala Gly Asn Trp  Ser Glu Cys Ser Thr
                1415                    1420                    1425

Thr Cys Gly Leu Gly  Ala Val Trp Arg Pro  Val Arg Cys Ser Ser
                1430                    1435                    1440

Gly Arg Asp Glu Asp  Cys Ala Pro Ala Gly  Arg Pro Gln Pro Ala
                1445                    1450                    1455

Arg Arg Cys His Leu  Arg Pro Cys Ala Thr  Trp His Ser Gly Asn
                1460                    1465                    1470

Trp Ser Lys Cys Ser  Arg Ser Cys Gly Gly  Gly Ser Ser Val Arg
                1475                    1480                    1485

Asp Val Gln Cys Val  Asp Thr Arg Asp Leu  Arg Pro Leu Arg Pro
                1490                    1495                    1500

Phe His Cys Gln Pro  Gly Pro Ala Lys Pro  Pro Ala His Arg Pro
                1505                    1510                    1515

Cys Gly Ala Gln Pro  Cys Leu Ser Trp Tyr  Thr Ser Ser Trp Arg
                1520                    1525                    1530

Glu Cys Ser Glu Ala  Cys Gly Gly Gly Glu  Gln Gln Arg Leu Val
                1535                    1540                    1545

Thr Cys Pro Glu Pro Gly Leu Cys Glu Glu Ala Leu Arg Pro Asn
1550 1555 1560

Thr Thr Arg Pro Cys Asn Thr His Pro Cys Thr Gln Trp Val Val
1565 1570 1575

Gly Pro Trp Gly Gln Cys Ser Gly Pro Cys Gly Gly Gly Val Gln
1580 1585 1590

Arg Arg Leu Val Lys Cys Val Asn Thr Gln Thr Gly Leu Pro Glu
1595 1600 1605

Glu Asp Ser Asp Gln Cys Gly His Glu Ala Trp Pro Glu Ser Ser
1610 1615 1620

Arg Pro Cys Gly Thr Glu Asp Cys Glu Pro Val Glu Pro Pro Arg
1625 1630 1635

Cys Glu Arg Asp Arg Leu Ser Phe Gly Phe Cys Glu Thr Leu Arg
1640 1645 1650

Leu Leu Gly Arg Cys Gln Leu Pro Thr Ile Arg Thr Gln Cys Cys
1655 1660 1665

Arg Ser Cys Ser Pro Pro Ser His Gly Ala Pro Ser Arg Gly His
1670 1675 1680

Gln Arg Val Ala Arg Arg
1685

<210> 22
<211> 1595
<212> PRT
<213> Artificial Sequence

<220>
<223> Rat ADAMTS7 from Q1EHB3

<400> 22

Met His Arg Gly Leu Asn Leu Leu Leu Ile Leu Cys Ala Leu Ala Pro
1               5                   10                  15

His Val Leu Gly Pro Ala Ser Gly Leu Pro Thr Glu Gly Arg Ala Gly
            20                  25                  30

Leu Asp Ile Val His Pro Val Arg Val Asp Ala Gly Gly Ser Phe Leu
        35                  40                  45

Ser Tyr Glu Leu Trp Pro Arg Val Leu Arg Lys Arg Asp Val Ser Ala

50                  55                  60

Ala Gln Ala Ser Ser Ala Phe Tyr Gln Leu Gln Tyr Gln Gly Arg Glu
65                  70                  75                  80

Leu Leu Phe Asn Leu Thr Thr Asn Pro Tyr Leu Leu Ala Pro Gly Phe
                85                  90                  95

Val Ser Glu Ile Arg Arg Arg Ser Asn Leu Ser Asn Val His Ile Gln
            100                 105                 110

Thr Ser Val Pro Thr Cys His Leu Leu Gly Asp Val Gln Asp Pro Glu
        115                 120                 125

Leu Glu Gly Gly Phe Ala Ala Ile Ser Ala Cys Asp Gly Leu Arg Gly
    130                 135                 140

Val Phe Gln Leu Ser Asn Glu Asp Tyr Phe Ile Glu Pro Leu Asp Glu
145                 150                 155                 160

Val Pro Ala Gln Pro Gly His Ala Gln Pro His Met Val Tyr Lys His
                165                 170                 175

Lys Arg Ser Gly Gln Gln Asp Asp Ser Arg Thr Ser Gly Thr Cys Gly
            180                 185                 190

Val Gln Gly Ser Pro Glu Leu Lys His Gln Arg Glu His Trp Glu Gln
        195                 200                 205

Arg Gln Gln Lys Arg Arg Gln Gln Arg Ser Ile Ser Lys Glu Lys Trp
    210                 215                 220

Val Glu Thr Leu Val Val Ala Asp Ser Lys Met Val Glu Tyr His Gly
225                 230                 235                 240

Gln Pro Gln Val Glu Ser Tyr Val Leu Thr Ile Met Asn Met Val Ala
                245                 250                 255

Gly Leu Tyr His Asp Pro Ser Ile Gly Asn Pro Ile His Ile Thr Val
            260                 265                 270

Val Arg Leu Ile Ile Leu Glu Asp Glu Glu Lys Asp Leu Lys Ile Thr
        275                 280                 285

His His Ala Asp Asp Thr Leu Lys Asn Phe Cys Arg Trp Gln Lys Asn
    290                 295                 300

Val Asn Met Lys Gly Asp Asp His Pro Gln His His Asp Thr Ala Ile
305                 310                 315                 320

Leu Leu Thr Arg Lys Asp Leu Cys Ala Thr Met Asn His Pro Cys Glu
                325                 330                 335

Thr Leu Gly Leu Ser His Val Ala Gly Leu Cys His Pro Gln Leu Ser
            340                 345                 350

Cys Ser Val Ser Glu Asp Thr Gly Leu Pro Leu Ala Phe Thr Val Ala
        355                 360                 365

His Glu Leu Gly His Ser Phe Gly Ile Gln His Asp Gly Thr Gly Asn
    370                 375                 380

Asp Cys Glu Ser Ile Gly Lys Arg Pro Phe Ile Met Ser Pro Gln Leu
385                 390                 395                 400

Leu Tyr Asp Arg Gly Ile Pro Leu Thr Trp Ser Arg Cys Ser Arg Glu
                405                 410                 415

Tyr Ile Thr Arg Phe Leu Asp Arg Gly Trp Gly Leu Cys Leu Asp Asp
            420                 425                 430

Arg Pro Ser Lys Gly Val Ile Asn Phe Pro Ser Val Leu Pro Gly Val
        435                 440                 445

Leu Tyr Asp Val Asn His Gln Cys Arg Leu Gln Tyr Gly Pro Ser Ser
    450                 455                 460

Ala Tyr Cys Glu Asp Val Asp Asn Val Cys Tyr Thr Leu Trp Cys Ser
465                 470                 475                 480

Val Gly Thr Thr Cys His Ser Lys Met Asp Ala Ala Val Asp Gly Thr
                485                 490                 495

Ser Cys Gly Lys Asn Lys Trp Cys Leu Asn Gly Glu Cys Val Pro Glu
            500                 505                 510

Gly Phe Gln Pro Glu Thr Val Asp Gly Gly Trp Ser Gly Trp Ser Ala
        515                 520                 525

Trp Ser Val Cys Ser Arg Ser Cys Gly Val Gly Val Arg Ser Ser Glu
    530                 535                 540

Arg Gln Cys Thr Gln Pro Val Pro Lys Asn Lys Gly Lys Tyr Cys Val
545                 550                 555                 560

Gly Glu Arg Lys Arg Tyr Arg Leu Cys Asn Leu Gln Ala Cys Pro Pro
                565                 570                 575

Asp Arg Pro Ser Phe Arg His Thr Gln Cys Ser Gln Phe Asp Ser Met
            580                 585                 590

Leu Tyr Lys Gly Lys Leu His Lys Trp Val Pro Val Leu Asn Asp Glu
        595                 600                 605

Asn Pro Cys Glu Leu His Cys Arg Pro Phe Asn Tyr Ser Asn Arg Glu
    610                 615                 620

Lys Leu Arg Asp Ala Val Met Asp Gly Thr Pro Cys Tyr Gln Gly Arg
625                 630                 635                 640

Ile Ser Arg Asp Ile Cys Ile Asp Gly Ile Cys Lys Lys Val Gly Cys
                645                 650                 655

Asp Phe Glu Leu Asp Ser Gly Ala Glu Glu Asp Arg Cys Gly Val Cys
            660                 665                 670

Arg Gly Asp Gly Ser Thr Cys His Thr Val Ser Arg Thr Phe Lys Glu
        675                 680                 685

Ala Glu Gly Met Gly Tyr Val Asp Val Gly Leu Ile Pro Ala Gly Ala
    690                 695                 700

Arg Glu Ile Leu Ile Glu Glu Val Ala Glu Ala Ala Asn Phe Leu Ala
705                 710                 715                 720

Leu Arg Ser Glu Asp Pro Asp Lys Tyr Phe Leu Asn Gly Gly Trp Thr
                725                 730                 735

Ile Gln Trp Asn Gly Asp Tyr Gln Val Ala Gly Thr Thr Phe Thr Tyr
            740                 745                 750

Thr Arg Lys Gly Asn Trp Glu Thr Leu Thr Ser Pro Gly Pro Thr Thr
        755                 760                 765

Glu Pro Val Trp Ile Gln Leu Leu Phe Gln Glu Arg Asn Pro Gly Val
    770                 775                 780

His Tyr Lys Tyr Thr Ile Gln Arg Ala Ser His Ser Glu Ala Gln Pro
785                 790                 795                 800

Pro Glu Phe Ser Trp His Tyr Gly Pro Trp Ser Lys Cys Pro Val Thr
                805                 810                 815

Cys Gly Thr Gly Val Gln Arg Gln Ser Leu Tyr Cys Met Glu Lys Gln
            820                 825                 830

Ala Gly Ile Val Asp Glu Gly His Cys Asp His Leu Ser Arg Pro Arg
        835                 840                 845

Asp Arg Lys Arg Lys Cys Asn Glu Glu Pro Cys Pro Ala Arg Trp Trp
    850                 855                 860

Val Gly Asp Trp Gln Pro Cys Ser Arg Ser Cys Gly Pro Gly Gly Phe
865                 870                 875                 880

Phe Arg Arg Ala Val Phe Cys Thr Arg Ser Val Gly Leu Asp Glu Gln
                885                 890                 895

Arg Ala Leu Glu Pro Ser Ala Cys Gly His Leu Pro Arg Pro Leu Ala
            900                 905                 910

Glu Ile Pro Cys Tyr His Tyr Val Ala Cys Pro Ser Ser Trp Gly Val
        915                 920                 925

Gly Asn Trp Ser Gln Cys Ser Val Thr Cys Gly Ala Gly Ile Arg Gln
    930                 935                 940

Arg Ser Val Leu Cys Ile Asn Asn Thr Gly Val Pro Cys Asp Gly Ala
945                 950                 955                 960

Glu Arg Pro Ile Thr Glu Thr Phe Cys Phe Leu Gln Pro Cys Gln Tyr
                965                 970                 975

Ser Thr Tyr Ile Val Asp Thr Gly Ala Ser Gly Ser Gly Ser Ser Ser
            980                 985                 990

Pro Glu Leu Phe Asn Glu Val Asp  Phe Asp Pro His Gln  Pro Val Pro
        995                 1000                1005

Arg Pro  Ser Pro Ala Ser Ser  Pro Lys Pro Val Ser  Ile Ser Asn
    1010                1015                1020

Ala Ile  Asp Glu Glu Asp Pro  Glu Leu Asp Pro Pro  Gly Pro Val
    1025                1030                1035

Phe Val  Asp Asp Phe Tyr Tyr  Asp Tyr Asn Phe Ile  Asn Phe His
    1040                1045                1050

Glu Asp  Leu Ser Tyr Gly Ser  Phe Glu Glu Ser His  Ser Asp Leu

1055                 1060                 1065

Val Asp Ile Gly Gly Gln Thr Val Pro Pro His Ile Arg Pro Thr
    1070                 1075                 1080

Glu Pro Pro Ser Asp Ser Pro Val Pro Thr Ala Gly Ala Pro Gly
    1085                 1090                 1095

Ala Glu Glu Glu Gly Ile Gln Gly Ser Trp Ser Pro Ser Pro Leu
    1100                 1105                 1110

Leu Ser Glu Ala Ser His Ser Pro Pro Val Leu Leu Glu Asn Thr
    1115                 1120                 1125

Pro Val Asn Pro Leu Ala Asn Phe Leu Thr Glu Glu Glu Ser Pro
    1130                 1135                 1140

Ile Gly Ala Pro Glu Leu Gly Leu Pro Ser Val Ser Trp Pro Pro
    1145                 1150                 1155

Ala Ser Val Asp Gly Met Val Thr Ser Val Ala Pro Gly Asn Pro
    1160                 1165                 1170

Asp Glu Leu Leu Val Arg Glu Asp Thr Gln Ser Gln Pro Ser Thr
    1175                 1180                 1185

Pro Trp Ser Asp Arg Asn Lys Leu Ser Lys Asp Gly Asn Pro Leu
    1190                 1195                 1200

Gly Pro Thr Ser Pro Ala Leu Pro Lys Ser Pro Phe Pro Thr Gln
    1205                 1210                 1215

Pro Ser Ser Pro Ser Asn Ser Thr Thr Gln Ala Ser Leu Ser Pro
    1220                 1225                 1230

Asp Ala Val Glu Val Ser Thr Gly Trp Asn Val Ala Leu Asp Pro
    1235                 1240                 1245

Val Leu Glu Ala Asp Leu Lys Pro Val His Ala Pro Thr Asp Pro
    1250                 1255                 1260

Gly Leu Leu Asp Gln Ile Gln Thr Pro His Thr Glu Gly Thr Gln
    1265                 1270                 1275

Ser Pro Gly Leu Leu Pro Arg Pro Ala Gln Glu Thr Gln Thr Asn
    1280                 1285                 1290

Ser Ser Lys Asp Pro Ala Val Gln Pro Leu Gln Pro Ser Leu Val
    1295                1300                1305

Glu Asp Gly Ala Pro Thr Asp Leu Leu Pro Ala Lys Asn Ala Ser
    1310                1315                1320

Trp Gln Val Gly Asn Trp Ser Gln Cys Ser Thr Thr Cys Gly Leu
    1325                1330                1335

Gly Ala Ile Trp Arg Leu Val Arg Cys Ser Ser Gly Asn Asp Glu
    1340                1345                1350

Asp Cys Thr Leu Ser Ser Arg Pro Gln Pro Ala Arg His Cys His
    1355                1360                1365

Leu Arg Pro Cys Ala Ala Trp Arg Ala Gly Asn Trp Ser Lys Cys
    1370                1375                1380

Ser Arg Asn Cys Gly Gly Gly Ser Ala Thr Arg Asp Val Gln Cys
    1385                1390                1395

Val Asp Thr Arg Asp Leu Arg Pro Leu Arg Pro Phe His Cys Gln
    1400                1405                1410

Pro Gly Pro Thr Lys Pro Pro Thr Arg Gln Leu Cys Gly Thr Gln
    1415                1420                1425

Pro Cys Leu Pro Trp Tyr Thr Ser Ser Trp Arg Glu Cys Ser Glu
    1430                1435                1440

Ala Cys Gly Gly Gly Glu Gln Gln Arg Leu Val Thr Cys Pro Glu
    1445                1450                1455

Pro Gly Leu Cys Glu Glu Ser Leu Arg Pro Asn Asn Thr Arg Pro
    1460                1465                1470

Cys Asn Thr His Pro Cys Thr Gln Trp Val Val Gly Pro Trp Gly
    1475                1480                1485

Gln Cys Ser Ala Pro Cys Gly Gly Gly Val Gln Arg Arg Leu Val
    1490                1495                1500

Lys Cys Val Asn Thr Gln Thr Gly Leu Ala Glu Glu Asp Ser Asp
    1505                1510                1515

Leu Cys Ser His Glu Ala Trp Pro Glu Ser Ser Arg Pro Cys Ala
    1520                1525                1530

Thr Glu Asp Cys Glu Leu Val Glu Pro Ser Arg Cys Glu Arg Asp
    1535                1540                1545

Arg Leu Pro Phe Asn Phe Cys Glu Thr Leu Arg Leu Leu Gly Arg
    1550                1555                1560

Cys Gln Leu Pro Thr Ile Arg Ala Gln Cys Cys Arg Ser Cys Pro
    1565                1570                1575

Pro Leu Ser Arg Gly Val Pro Ser Arg Gly His Gln Arg Val Ala
    1580                1585                1590

Arg Arg
    1595

<210> 23
<211> 1657
<212> PRT
<213> Artificial Sequence

<220>
<223> Mouse ADAMTS7 from Q68SA9

<400> 23

Met His Arg Gly Pro Ser Leu Leu Leu Ile Leu Cys Ala Leu Ala Ser
1               5                   10                  15

Arg Val Leu Gly Pro Ala Ser Gly Leu Val Thr Glu Gly Arg Ala Gly
            20                  25                  30

Leu Asp Ile Val His Pro Val Arg Val Asp Ala Gly Gly Ser Phe Leu
        35                  40                  45

Ser Tyr Glu Leu Trp Pro Arg Val Leu Arg Lys Arg Asp Val Ser Thr
    50                  55                  60

Thr Gln Ala Ser Ser Ala Phe Tyr Gln Leu Gln Tyr Gln Gly Arg Glu
65                  70                  75                  80

Leu Leu Phe Asn Leu Thr Thr Asn Pro Tyr Leu Met Ala Pro Gly Phe
                85                  90                  95

Val Ser Glu Ile Arg Arg His Ser Thr Leu Gly His Ala His Ile Gln
            100                 105                 110

Thr Ser Val Pro Thr Cys His Leu Leu Gly Asp Val Gln Asp Pro Glu
        115                 120                 125

Leu Glu Gly Gly Phe Ala Ala Ile Ser Ala Cys Asp Gly Leu Arg Gly
    130                 135                 140

Val Phe Gln Leu Ser Asn Glu Asp Tyr Phe Ile Glu Pro Leu Asp Gly
145                 150                 155                 160

Val Ser Ala Gln Pro Gly His Ala Gln Pro His Val Val Tyr Lys His
                165                 170                 175

Gln Gly Ser Arg Lys Gln Ala Gln Gln Gly Asp Ser Arg Pro Ser Gly
            180                 185                 190

Thr Cys Gly Met Gln Val Pro Pro Asp Leu Glu Gln Gln Arg Glu His
        195                 200                 205

Trp Glu Gln Gln Gln Gln Lys Arg Arg Gln Gln Arg Ser Val Ser Lys
    210                 215                 220

Glu Lys Trp Val Glu Thr Leu Val Val Ala Asp Ser Lys Met Val Glu
225                 230                 235                 240

Tyr His Gly Gln Pro Gln Val Glu Ser Tyr Val Leu Thr Ile Met Asn
                245                 250                 255

Met Val Ala Gly Leu Phe His Asp Pro Ser Ile Gly Asn Pro Ile His
            260                 265                 270

Ile Ser Ile Val Arg Leu Ile Ile Leu Glu Asp Glu Glu Lys Asp Leu
        275                 280                 285

Lys Ile Thr His His Ala Glu Glu Thr Leu Lys Asn Phe Cys Arg Trp
    290                 295                 300

Gln Lys Asn Ile Asn Ile Lys Gly Asp Asp His Pro Gln His His Asp
305                 310                 315                 320

Thr Ala Ile Leu Leu Thr Arg Lys Asp Leu Cys Ala Ser Met Asn Gln
                325                 330                 335

Pro Cys Glu Thr Leu Gly Leu Ser His Val Ser Gly Leu Cys His Pro
            340                 345                 350

Gln Leu Ser Cys Ser Val Ser Glu Asp Thr Gly Met Pro Leu Ala Phe
        355                 360                 365

Thr Val Ala His Glu Leu Gly His Ser Phe Gly Ile Gln His Asp Gly
    370                 375                 380

Thr Gly Asn Asp Cys Glu Ser Ile Gly Lys Arg Pro Phe Ile Met Ser
385                 390                 395                 400

Pro Gln Leu Leu Tyr Asp Arg Gly Ile Pro Leu Thr Trp Ser Arg Cys
                405                 410                 415

Ser Arg Glu Tyr Ile Thr Arg Phe Leu Asp Arg Gly Trp Gly Leu Cys
            420                 425                 430

Leu Asp Asp Arg Pro Ser Lys Asp Val Ile Ala Leu Pro Ser Val Leu
        435                 440                 445

Pro Gly Val Leu Tyr Asp Val Asn His Gln Cys Arg Leu Gln Tyr Gly
    450                 455                 460

Ser His Ser Ala Tyr Cys Glu Asp Met Asp Asp Val Cys His Thr Leu
465                 470                 475                 480

Trp Cys Ser Val Gly Thr Thr Cys His Ser Lys Leu Asp Ala Ala Val
                485                 490                 495

Asp Gly Thr Ser Cys Gly Lys Asn Lys Trp Cys Leu Lys Gly Glu Cys
            500                 505                 510

Val Pro Glu Gly Phe Gln Pro Glu Ala Val Asp Gly Gly Trp Ser Gly
        515                 520                 525

Trp Ser Ala Trp Ser Asp Cys Ser Arg Ser Cys Gly Val Gly Val Arg
    530                 535                 540

Ser Ser Glu Arg Gln Cys Thr Gln Pro Val Pro Lys Asn Arg Gly Lys
545                 550                 555                 560

Tyr Cys Val Gly Glu Arg Lys Arg Ser Gln Leu Cys Asn Leu Pro Ala
                565                 570                 575

Cys Pro Pro Asp Arg Pro Ser Phe Arg His Thr Gln Cys Ser Gln Phe
            580                 585                 590

Asp Gly Met Leu Tyr Lys Gly Lys Leu His Lys Trp Val Pro Val Pro
        595                 600                 605

Asn Asp Asp Asn Pro Cys Glu Leu His Cys Arg Pro Ser Asn Ser Ser
    610                 615                 620

Asn Thr Glu Lys Leu Arg Asp Ala Val Val Asp Gly Thr Pro Cys Tyr
625                 630                 635                 640

Gln Ser Arg Ile Ser Arg Asp Ile Cys Leu Asn Gly Ile Cys Lys Asn
                645                 650                 655

Val Gly Cys Asp Phe Val Ile Asp Ser Gly Ala Glu Glu Asp Arg Cys
            660                 665                 670

Gly Val Cys Arg Gly Asp Gly Ser Thr Cys Gln Thr Val Ser Arg Thr
        675                 680                 685

Phe Lys Glu Thr Glu Gly Gln Gly Tyr Val Asp Ile Gly Leu Ile Pro
    690                 695                 700

Ala Gly Ala Arg Glu Ile Leu Ile Glu Glu Val Ala Glu Ala Ala Asn
705                 710                 715                 720

Phe Leu Ala Leu Arg Ser Glu Asp Pro Asp Lys Tyr Phe Leu Asn Gly
                725                 730                 735

Gly Trp Thr Ile Gln Trp Asn Gly Asp Tyr Arg Val Ala Gly Thr Thr
            740                 745                 750

Phe Thr Tyr Ala Arg Lys Gly Asn Trp Glu Asn Leu Thr Ser Pro Gly
        755                 760                 765

Pro Thr Ser Glu Pro Val Trp Ile Gln Leu Leu Phe Gln Glu Lys Asn
    770                 775                 780

Pro Gly Val His Tyr Gln Tyr Thr Ile Gln Arg Asp Ser His Asp Gln
785                 790                 795                 800

Val Arg Pro Pro Glu Phe Ser Trp His Tyr Gly Pro Trp Ser Lys Cys
                805                 810                 815

Thr Val Thr Cys Gly Thr Gly Val Gln Arg Gln Ser Leu Tyr Cys Met
            820                 825                 830

Glu Arg Gln Ala Gly Val Val Ala Glu Glu Tyr Cys Asn Thr Leu Asn
        835                 840                 845

Arg Pro Asp Glu Arg Gln Arg Lys Cys Ser Glu Glu Pro Cys Pro Pro
    850                 855                 860

Arg Trp Trp Ala Gly Glu Trp Gln Pro Cys Ser Arg Ser Cys Gly Pro
865                 870                 875                 880

Glu Gly Leu Ser Arg Arg Ala Val Phe Cys Ile Arg Ser Met Gly Leu

885                 890                 895

Asp Glu Gln Arg Ala Leu Glu Leu Ser Ala Cys Glu His Leu Pro Arg
            900                 905                 910

Pro Leu Ala Glu Thr Pro Cys Asn Arg His Val Ile Cys Pro Ser Thr
        915                 920                 925

Trp Gly Val Gly Asn Trp Ser Gln Cys Ser Val Thr Cys Gly Ala Gly
    930                 935                 940

Ile Arg Gln Arg Ser Val Leu Cys Ile Asn Asn Thr Asp Val Pro Cys
945                 950                 955                 960

Asp Glu Ala Glu Arg Pro Ile Thr Glu Thr Phe Cys Phe Leu Gln Pro
                965                 970                 975

Cys Gln Tyr Pro Met Tyr Ile Val Asp Thr Gly Ala Ser Gly Ser Gly
            980                 985                 990

Ser Ser Ser Pro Glu Leu Phe Asn  Glu Val Asp Phe Ile  Pro Asn Gln
        995                 1000                 1005

Leu Ala  Pro Arg Pro Ser Pro  Ala Ser Ser Pro Lys  Pro Val Ser
    1010                 1015                 1020

Ile Ser  Asn Ala Ile Asp Glu  Glu Glu Leu Asp Pro  Pro Gly Pro
    1025                 1030                 1035

Val Phe  Val Asp Asp Phe Tyr  Tyr Asp Tyr Asn Phe  Ile Asn Phe
    1040                 1045                 1050

His Glu  Asp Leu Ser Tyr Gly  Ser Phe Glu Glu Pro  His Pro Asp
    1055                 1060                 1065

Leu Val  Asp Asn Gly Gly Trp  Thr Ala Pro Pro His  Ile Arg Pro
    1070                 1075                 1080

Thr Glu  Ser Pro Ser Asp Thr  Pro Val Pro Thr Ala  Gly Ala Leu
    1085                 1090                 1095

Gly Ala  Glu Ala Glu Asp Ile  Gln Gly Ser Trp Ser  Pro Ser Pro
    1100                 1105                 1110

Leu Leu  Ser Glu Ala Ser Tyr  Ser Pro Pro Gly Leu  Glu Gln Thr
    1115                 1120                 1125

Ser Ile Asn Pro Leu Ala Asn Phe Leu Thr Glu Glu Asp Thr Pro
    1130                1135                1140

Met Gly Ala Pro Glu Leu Gly Phe Pro Ser Leu Pro Trp Pro Pro
    1145                1150                1155

Ala Ser Val Asp Asp Met Met Thr Pro Val Gly Pro Gly Asn Pro
    1160                1165                1170

Asp Glu Leu Leu Val Lys Glu Asp Glu Gln Ser Pro Pro Ser Thr
    1175                1180                1185

Pro Trp Ser Asp Arg Asn Lys Leu Ser Thr Asp Gly Asn Pro Leu
    1190                1195                1200

Gly His Thr Ser Pro Ala Leu Pro Gln Ser Pro Ile Pro Thr Gln
    1205                1210                1215

Pro Ser Pro Pro Ser Ile Ser Pro Thr Gln Ala Ser Pro Ser Pro
    1220                1225                1230

Asp Val Val Glu Val Ser Thr Gly Trp Asn Ala Ala Trp Asp Pro
    1235                1240                1245

Val Leu Glu Ala Asp Leu Lys Pro Gly His Gly Glu Leu Pro Ser
    1250                1255                1260

Thr Val Glu Val Ala Ser Pro Pro Leu Leu Pro Met Ala Thr Val
    1265                1270                1275

Pro Gly Ile Trp Gly Arg Asp Ser Pro Leu Glu Pro Gly Thr Pro
    1280                1285                1290

Thr Phe Ser Ser Pro Glu Leu Ser Ser Gln His Leu Lys Thr Leu
    1295                1300                1305

Thr Met Pro Gly Thr Leu Leu Leu Thr Val Pro Thr Asp Leu Arg
    1310                1315                1320

Ser Pro Gly Pro Ser Gly Gln Pro Gln Thr Pro Asn Leu Glu Gly
    1325                1330                1335

Thr Gln Ser Pro Gly Leu Leu Pro Thr Pro Ala Arg Glu Thr Gln
    1340                1345                1350

Thr Asn Ser Ser Lys Asp Pro Glu Val Gln Pro Leu Gln Pro Ser
    1355                1360                1365

Leu Glu Glu Asp Gly Asp Pro Ala Asp Pro Leu Pro Ala Arg Asn
    1370                1375                1380

Ala Ser Trp Gln Val Gly Asn Trp Ser Gln Cys Ser Thr Thr Cys
    1385                1390                1395

Gly Leu Gly Ala Ile Trp Arg Leu Val Ser Cys Ser Ser Gly Asn
    1400                1405                1410

Asp Glu Asp Cys Thr Leu Ala Ser Arg Pro Gln Pro Ala Arg His
    1415                1420                1425

Cys His Leu Arg Pro Cys Ala Ala Trp Arg Thr Gly Asn Trp Ser
    1430                1435                1440

Lys Cys Ser Arg Asn Cys Gly Gly Gly Ser Ser Thr Arg Asp Val
    1445                1450                1455

Gln Cys Val Asp Thr Arg Asp Leu Arg Pro Leu Arg Pro Phe His
    1460                1465                1470

Cys Gln Pro Gly Pro Thr Lys Pro Pro Asn Arg Gln Leu Cys Gly
    1475                1480                1485

Thr Gln Pro Cys Leu Pro Trp Tyr Thr Ser Ser Trp Arg Glu Cys
    1490                1495                1500

Ser Glu Ala Cys Gly Gly Gly Glu Gln Gln Arg Leu Val Thr Cys
    1505                1510                1515

Pro Glu Pro Gly Leu Cys Glu Glu Ser Leu Arg Pro Asn Asn Ser
    1520                1525                1530

Arg Pro Cys Asn Thr His Pro Cys Thr Gln Trp Val Val Gly Pro
    1535                1540                1545

Trp Gly Gln Cys Ser Ala Pro Cys Gly Gly Gly Val Gln Arg Arg
    1550                1555                1560

Leu Val Arg Cys Val Asn Thr Gln Thr Gly Leu Ala Glu Glu Asp
    1565                1570                1575

Ser Asp Leu Cys Ser His Glu Ala Trp Pro Glu Ser Ser Arg Pro
    1580                1585                1590

Cys Ala Thr Glu Asp Cys Glu Leu Val Glu Pro Pro Arg Cys Glu
    1595                1600                1605

Arg Asp Arg Leu Ser Phe Asn Phe Cys Glu Thr Leu Arg Leu Leu
    1610                1615                1620

Gly Arg Cys Gln Leu Pro Thr Ile Arg Ala Gln Cys Cys Arg Ser
    1625                1630                1635

Cys Pro Pro Leu Ser Arg Gly Val Pro Ser Arg Gly His Gln Arg
    1640                1645                1650

Val Ala Arg Arg
    1655

<210> 24
<211> 1594
<212> PRT
<213> Artificial Sequence

<220>
<223> Human ADAMTS12 from P58397

<400> 24

Met Pro Cys Ala Gln Arg Ser Trp Leu Ala Asn Leu Ser Val Val Ala
1               5                   10                  15

Gln Leu Leu Asn Phe Gly Ala Leu Cys Tyr Gly Arg Gln Pro Gln Pro
            20                  25                  30

Gly Pro Val Arg Phe Pro Asp Arg Arg Gln Glu His Phe Ile Lys Gly
        35                  40                  45

Leu Pro Glu Tyr His Val Val Gly Pro Val Arg Val Asp Ala Ser Gly
    50                  55                  60

His Phe Leu Ser Tyr Gly Leu His Tyr Pro Ile Thr Ser Ser Arg Arg
65                  70                  75                  80

Lys Arg Asp Leu Asp Gly Ser Glu Asp Trp Val Tyr Tyr Arg Ile Ser
                85                  90                  95

His Glu Glu Lys Asp Leu Phe Phe Asn Leu Thr Val Asn Gln Gly Phe
            100                 105                 110

Leu Ser Asn Ser Tyr Ile Met Glu Lys Arg Tyr Gly Asn Leu Ser His
        115                 120                 125

Val Lys Met Met Ala Ser Ser Ala Pro Leu Cys His Leu Ser Gly Thr
    130                 135                 140

Val Leu Gln Gln Gly Thr Arg Val Gly Thr Ala Ala Leu Ser Ala Cys
145                 150                 155                 160

His Gly Leu Thr Gly Phe Phe Gln Leu Pro His Gly Asp Phe Phe Ile
                165                 170                 175

Glu Pro Val Lys Lys His Pro Leu Val Glu Gly Gly Tyr His Pro His
            180                 185                 190

Ile Val Tyr Arg Arg Gln Lys Val Pro Glu Thr Lys Glu Pro Thr Cys
        195                 200                 205

Gly Leu Lys Asp Ser Val Asn Ile Ser Gln Lys Gln Glu Leu Trp Arg
    210                 215                 220

Glu Lys Trp Glu Arg His Asn Leu Pro Ser Arg Ser Leu Ser Arg Arg
225                 230                 235                 240

Ser Ile Ser Lys Glu Arg Trp Val Glu Thr Leu Val Val Ala Asp Thr
                245                 250                 255

Lys Met Ile Glu Tyr His Gly Ser Glu Asn Val Glu Ser Tyr Ile Leu
            260                 265                 270

Thr Ile Met Asn Met Val Thr Gly Leu Phe His Asn Pro Ser Ile Gly
        275                 280                 285

Asn Ala Ile His Ile Val Val Val Arg Leu Ile Leu Leu Glu Glu Glu
    290                 295                 300

Glu Gln Gly Leu Lys Ile Val His His Ala Glu Lys Thr Leu Ser Ser
305                 310                 315                 320

Phe Cys Lys Trp Gln Lys Ser Ile Asn Pro Lys Ser Asp Leu Asn Pro
                325                 330                 335

Val His His Asp Val Ala Val Leu Leu Thr Arg Lys Asp Ile Cys Ala
            340                 345                 350

Gly Phe Asn Arg Pro Cys Glu Thr Leu Gly Leu Ser His Leu Ser Gly
        355                 360                 365

Met Cys Gln Pro His Arg Ser Cys Asn Ile Asn Glu Asp Ser Gly Leu
    370                 375                 380

Pro Leu Ala Phe Thr Ile Ala His Glu Leu Gly His Ser Phe Gly Ile
385                 390                 395                 400

Gln His Asp Gly Lys Glu Asn Asp Cys Glu Pro Val Gly Arg His Pro
                405                 410                 415

Tyr Ile Met Ser Arg Gln Leu Gln Tyr Asp Pro Thr Pro Leu Thr Trp
            420                 425                 430

Ser Lys Cys Ser Glu Glu Tyr Ile Thr Arg Phe Leu Asp Arg Gly Trp
        435                 440                 445

Gly Phe Cys Leu Asp Asp Ile Pro Lys Lys Lys Gly Leu Lys Ser Lys
    450                 455                 460

Val Ile Ala Pro Gly Val Ile Tyr Asp Val His His Gln Cys Gln Leu
465                 470                 475                 480

Gln Tyr Gly Pro Asn Ala Thr Phe Cys Gln Glu Val Glu Asn Val Cys
                485                 490                 495

Gln Thr Leu Trp Cys Ser Val Lys Gly Phe Cys Arg Ser Lys Leu Asp
            500                 505                 510

Ala Ala Ala Asp Gly Thr Gln Cys Gly Glu Lys Lys Trp Cys Met Ala
        515                 520                 525

Gly Lys Cys Ile Thr Val Gly Lys Lys Pro Glu Ser Ile Pro Gly Gly
    530                 535                 540

Trp Gly Arg Trp Ser Pro Trp Ser His Cys Ser Arg Thr Cys Gly Ala
545                 550                 555                 560

Gly Val Gln Ser Ala Glu Arg Leu Cys Asn Asn Pro Glu Pro Lys Phe
                565                 570                 575

Gly Gly Lys Tyr Cys Thr Gly Glu Arg Lys Arg Tyr Arg Leu Cys Asn
            580                 585                 590

Val His Pro Cys Arg Ser Glu Ala Pro Thr Phe Arg Gln Met Gln Cys
        595                 600                 605

Ser Glu Phe Asp Thr Val Pro Tyr Lys Asn Glu Leu Tyr His Trp Phe
    610                 615                 620

Pro Ile Phe Asn Pro Ala His Pro Cys Glu Leu Tyr Cys Arg Pro Ile
625                 630                 635                 640

Asp Gly Gln Phe Ser Glu Lys Met Leu Asp Ala Val Ile Asp Gly Thr

                645                 650                 655

Pro Cys Phe Glu Gly Gly Asn Ser Arg Asn Val Cys Ile Asn Gly Ile
            660                 665                 670

Cys Lys Met Val Gly Cys Asp Tyr Glu Ile Asp Ser Asn Ala Thr Glu
        675                 680                 685

Asp Arg Cys Gly Val Cys Leu Gly Asp Gly Ser Ser Cys Gln Thr Val
    690                 695                 700

Arg Lys Met Phe Lys Gln Lys Glu Gly Ser Gly Tyr Val Asp Ile Gly
705                 710                 715                 720

Leu Ile Pro Lys Gly Ala Arg Asp Ile Arg Val Met Glu Ile Glu Gly
                725                 730                 735

Ala Gly Asn Phe Leu Ala Ile Arg Ser Glu Asp Pro Glu Lys Tyr Tyr
            740                 745                 750

Leu Asn Gly Gly Phe Ile Ile Gln Trp Asn Gly Asn Tyr Lys Leu Ala
        755                 760                 765

Gly Thr Val Phe Gln Tyr Asp Arg Lys Gly Asp Leu Glu Lys Leu Met
    770                 775                 780

Ala Thr Gly Pro Thr Asn Glu Ser Val Trp Ile Gln Leu Leu Phe Gln
785                 790                 795                 800

Val Thr Asn Pro Gly Ile Lys Tyr Glu Tyr Thr Ile Gln Lys Asp Gly
                805                 810                 815

Leu Asp Asn Asp Val Glu Gln Gln Met Tyr Phe Trp Gln Tyr Gly His
            820                 825                 830

Trp Thr Glu Cys Ser Val Thr Cys Gly Thr Gly Ile Arg Arg Gln Thr
        835                 840                 845

Ala His Cys Ile Lys Lys Gly Arg Gly Met Val Lys Ala Thr Phe Cys
    850                 855                 860

Asp Pro Glu Thr Gln Pro Asn Gly Arg Gln Lys Lys Cys His Glu Lys
865                 870                 875                 880

Ala Cys Pro Pro Arg Trp Trp Ala Gly Glu Trp Glu Ala Cys Ser Ala
                885                 890                 895

Thr Cys Gly Pro His Gly Glu Lys Lys Arg Thr Val Leu Cys Ile Gln
            900                 905                 910

Thr Met Val Ser Asp Glu Gln Ala Leu Pro Pro Thr Asp Cys Gln His
        915                 920                 925

Leu Leu Lys Pro Lys Thr Leu Leu Ser Cys Asn Arg Asp Ile Leu Cys
    930                 935                 940

Pro Ser Asp Trp Thr Val Gly Asn Trp Ser Glu Cys Ser Val Ser Cys
945                 950                 955                 960

Gly Gly Gly Val Arg Ile Arg Ser Val Thr Cys Ala Lys Asn His Asp
                965                 970                 975

Glu Pro Cys Asp Val Thr Arg Lys Pro Asn Ser Arg Ala Leu Cys Gly
            980                 985                 990

Leu Gln Gln Cys Pro Ser Ser Arg Arg Val Leu Lys Pro Asn Lys Gly
        995                 1000                1005

Thr Ile Ser Asn Gly Lys Asn Pro Pro Thr Leu Lys Pro Val Pro
    1010                1015                1020

Pro Pro Thr Ser Arg Pro Arg Met Leu Thr Thr Pro Thr Gly Pro
    1025                1030                1035

Glu Ser Met Ser Thr Ser Thr Pro Ala Ile Ser Ser Pro Ser Pro
    1040                1045                1050

Thr Thr Ala Ser Lys Glu Gly Asp Leu Gly Gly Lys Gln Trp Gln
    1055                1060                1065

Asp Ser Ser Thr Gln Pro Glu Leu Ser Ser Arg Tyr Leu Ile Ser
    1070                1075                1080

Thr Gly Ser Thr Ser Gln Pro Ile Leu Thr Ser Gln Ser Leu Ser
    1085                1090                1095

Ile Gln Pro Ser Glu Glu Asn Val Ser Ser Ser Asp Thr Gly Pro
    1100                1105                1110

Thr Ser Glu Gly Gly Leu Val Ala Thr Thr Thr Ser Gly Ser Gly
    1115                1120                1125

Leu Ser Ser Ser Arg Asn Pro Ile Thr Trp Pro Val Thr Pro Phe
    1130                1135                1140

Tyr Asn Thr Leu Thr Lys Gly Pro Glu Met Glu Ile His Ser Gly
    1145                1150                1155

Ser Gly Glu Glu Arg Glu Gln Pro Glu Asp Lys Asp Glu Ser Asn
    1160                1165                1170

Pro Val Ile Trp Thr Lys Ile Arg Val Pro Gly Asn Asp Ala Pro
    1175                1180                1185

Val Glu Ser Thr Glu Met Pro Leu Ala Pro Pro Leu Thr Pro Asp
    1190                1195                1200

Leu Ser Arg Glu Ser Trp Trp Pro Pro Phe Ser Thr Val Met Glu
    1205                1210                1215

Gly Leu Leu Pro Ser Gln Arg Pro Thr Thr Ser Glu Thr Gly Thr
    1220                1225                1230

Pro Arg Val Glu Gly Met Val Thr Glu Lys Pro Ala Asn Thr Leu
    1235                1240                1245

Leu Pro Leu Gly Gly Asp His Gln Pro Glu Pro Ser Gly Lys Thr
    1250                1255                1260

Ala Asn Arg Asn His Leu Lys Leu Pro Asn Asn Met Asn Gln Thr
    1265                1270                1275

Lys Ser Ser Glu Pro Val Leu Thr Glu Glu Asp Ala Thr Ser Leu
    1280                1285                1290

Ile Thr Glu Gly Phe Leu Leu Asn Ala Ser Asn Tyr Lys Gln Leu
    1295                1300                1305

Thr Asn Gly His Gly Ser Ala His Trp Ile Val Gly Asn Trp Ser
    1310                1315                1320

Glu Cys Ser Thr Thr Cys Gly Leu Gly Ala Tyr Trp Arg Arg Val
    1325                1330                1335

Glu Cys Ser Thr Gln Met Asp Ser Asp Cys Ala Ala Ile Gln Arg
    1340                1345                1350

Pro Asp Pro Ala Lys Arg Cys His Leu Arg Pro Cys Ala Gly Trp
    1355                1360                1365

Lys Val Gly Asn Trp Ser Lys Cys Ser Arg Asn Cys Ser Gly Gly
    1370                1375                1380

Phe Lys Ile Arg Glu Ile Gln Cys Val Asp Ser Arg Asp His Arg
    1385                1390                1395

Asn Leu Arg Pro Phe His Cys Gln Phe Leu Ala Gly Ile Pro Pro
    1400                1405                1410

Pro Leu Ser Met Ser Cys Asn Pro Glu Pro Cys Glu Ala Trp Gln
    1415                1420                1425

Val Glu Pro Trp Ser Gln Cys Ser Arg Ser Cys Gly Gly Gly Val
    1430                1435                1440

Gln Glu Arg Gly Val Phe Cys Pro Gly Gly Leu Cys Asp Trp Thr
    1445                1450                1455

Lys Arg Pro Thr Ser Thr Met Ser Cys Asn Glu His Leu Cys Cys
    1460                1465                1470

His Trp Ala Thr Gly Asn Trp Asp Leu Cys Ser Thr Ser Cys Gly
    1475                1480                1485

Gly Gly Phe Gln Lys Arg Thr Val Gln Cys Val Pro Ser Glu Gly
    1490                1495                1500

Asn Lys Thr Glu Asp Gln Asp Gln Cys Leu Cys Asp His Lys Pro
    1505                1510                1515

Arg Pro Pro Glu Phe Lys Lys Cys Asn Gln Gln Ala Cys Lys Lys
    1520                1525                1530

Ser Ala Asp Leu Leu Cys Thr Lys Asp Lys Leu Ser Ala Ser Phe
    1535                1540                1545

Cys Gln Thr Leu Lys Ala Met Lys Lys Cys Ser Val Pro Thr Val
    1550                1555                1560

Arg Ala Glu Cys Cys Phe Ser Cys Pro Gln Thr His Ile Thr His
    1565                1570                1575

Thr Gln Arg Gln Arg Arg Gln Arg Leu Leu Gln Lys Ser Lys Glu
    1580                1585                1590

Leu

<210>  25

<211>  1599
<212>  PRT
<213>  Artificial Sequence

<220>
<223>  Rat ADAMTS12 from D3ZTJ3

<400>  25

Met Pro Cys Ala Gln Gly Asn Trp Met Ala Lys Leu Ser Met Val Ala
1               5                   10                  15

Gln Leu Leu Asn Phe Gly Ala Phe Cys His Gly Arg Gln Ala Gln Pro
            20                  25                  30

Trp Pro Val Arg Phe Pro Asp Pro Lys Gln Glu His Phe Ile Lys Ser
        35                  40                  45

Leu Pro Glu Tyr His Ile Val Ser Pro Val Gln Val Asp Ala Ser Gly
    50                  55                  60

His Phe Leu Ser Tyr Gly Leu His His Pro Val Thr Gly Ser Arg Lys
65                  70                  75                  80

Lys Arg Ala Ala Gly Gly Ser Gly Asp Gln Val Tyr Tyr Arg Ile Ser
                85                  90                  95

His Glu Glu Lys Asn Leu Phe Phe Asn Leu Thr Val Asn Trp Glu Phe
            100                 105                 110

Leu Ser Asn Gly Tyr Val Val Glu Arg Arg Tyr Gly Asn Leu Ser His
        115                 120                 125

Val Lys Met Ala Ala Ser Ser Gly Gln Pro Cys His Leu Arg Gly Thr
    130                 135                 140

Val Leu Gln Gln Gly Pro Thr Ile Arg Met Gly Thr Ala Ala Leu Ser
145                 150                 155                 160

Ala Cys Gln Gly Leu Thr Gly Phe Phe His Leu Pro His Gly Asp Phe
                165                 170                 175

Phe Ile Glu Pro Val Lys Lys His Pro Leu Thr Glu Glu Gly Tyr Gln
            180                 185                 190

Pro His Val Ile Tyr Arg Arg Gln Ser Tyr Arg Val Pro Glu Thr Lys
        195                 200                 205

Glu Pro Thr Cys Gly Leu Lys Asp Ser Leu Asp Asn Ser Val Lys Gln
    210                 215                 220

Glu Leu Gln Arg Glu Lys Trp Glu Arg Lys Asn Trp Pro Ser Arg Ser
225                 230                 235                 240

Leu Ser Arg Arg Ser Ile Ser Lys Glu Arg Trp Val Glu Thr Leu Val
                245                 250                 255

Val Ala Asp Thr Lys Met Val Glu Tyr His Gly Ser Glu Asn Val Glu
            260                 265                 270

Ser Tyr Ile Leu Thr Ile Met Asn Met Val Thr Gly Leu Phe His Asn
        275                 280                 285

Pro Ser Ile Gly Asn Ala Val His Ile Val Val Val Arg Leu Ile Leu
    290                 295                 300

Leu Glu Glu Glu Glu Gln Gly Leu Lys Ile Val His His Ala Glu Lys
305                 310                 315                 320

Thr Leu Ser Ser Phe Cys Lys Trp Gln Lys Ser Ile Asn Pro Lys Ser
                325                 330                 335

Asp Leu Asn Pro Val His His Asp Val Ala Val Leu Ile Thr Arg Lys
            340                 345                 350

Asp Ile Cys Ala Gly Val Asn Arg Pro Cys Glu Thr Leu Gly Leu Ser
        355                 360                 365

Gln Leu Ser Gly Met Cys Gln Pro His Arg Ser Cys Asn Ile Asn Glu
    370                 375                 380

Asp Ser Gly Leu Pro Leu Ala Phe Thr Ile Ala His Glu Leu Gly His
385                 390                 395                 400

Ser Phe Gly Ile Gln His Asp Gly Lys Glu Asn Asp Cys Glu Pro Val
                405                 410                 415

Gly Arg His Pro Tyr Ile Met Ser Gln Gln Ile Gln Tyr Asp Pro Thr
            420                 425                 430

Pro Leu Thr Trp Ser Lys Cys Ser Lys Glu Tyr Ile Thr Arg Phe Leu
        435                 440                 445

Asp Arg Gly Arg Gly Phe Cys Leu Asp Asp Val Pro Arg Lys Lys Gly
    450                 455                 460

Leu Lys Ser Asn Val Ile Ala Pro Gly Val Ile Tyr Asp Val His His

465                 470                 475                 480

Gln Cys Gln Leu Gln Tyr Gly Pro Asn Ala Thr Phe Cys Gln Glu Val
                485                 490                 495

Glu Asn Val Cys Gln Thr Leu Trp Cys Ser Val Lys Gly Phe Cys Arg
            500                 505                 510

Ser Lys Leu Asp Ala Ala Ala Asp Gly Thr Arg Cys Gly Glu Lys Lys
        515                 520                 525

Trp Cys Met Ala Gly Lys Cys Ile Thr Val Gly Lys Lys Pro Glu Ser
    530                 535                 540

Ile Pro Gly Gly Trp Gly Arg Trp Ser Pro Trp Ser His Cys Ser Arg
545                 550                 555                 560

Thr Cys Gly Ala Gly Ala Gln Ser Ala Glu Arg Leu Cys Asn Asn Pro
                565                 570                 575

Glu Pro Lys Phe Gly Gly Lys Tyr Cys Thr Gly Glu Arg Lys Arg Tyr
            580                 585                 590

Arg Leu Cys Asn Val His Pro Cys Arg Ser Asp Thr Pro Thr Phe Arg
        595                 600                 605

Gln Met Gln Cys Ser Glu Phe Asp Thr Val Pro Tyr Lys Asn Gln Phe
    610                 615                 620

Tyr Arg Trp Phe Pro Val Phe Asn Pro Ala His Pro Cys Glu Leu Tyr
625                 630                 635                 640

Cys Arg Pro Ile Asp Glu Gln Phe Ser Glu Arg Met Leu Glu Ala Val
                645                 650                 655

Ile Asp Gly Thr Pro Cys Phe Glu Gly Gly Asn Ser Arg Asn Val Cys
            660                 665                 670

Ile Asn Gly Ile Cys Lys Arg Val Gly Cys Asp Tyr Glu Ile Asp Ser
        675                 680                 685

Asn Ala Thr Glu Asp Arg Cys Gly Val Cys Leu Gly Asp Gly Ser Ala
    690                 695                 700

Cys Gln Thr Val Lys Lys Val Phe Arg Gln Lys Glu Gly Ser Gly Tyr
705                 710                 715                 720

Ile Asp Ile Gly Leu Ile Pro Lys Gly Ala Arg Asp Ile Arg Val Met
                725                 730                 735

Glu Ile Lys Ala Ala Gly Asn Phe Leu Ala Ile Arg Ser Glu Asp Pro
            740                 745                 750

Glu Lys Tyr Tyr Leu Asn Gly Gly Phe Ile Ile Gln Trp Asn Gly Asn
        755                 760                 765

Tyr Lys Leu Ala Gly Thr Val Phe Gln Tyr Asp Arg Lys Gly Asp Leu
    770                 775                 780

Glu Arg Leu Met Ala Pro Gly Pro Thr Asn Glu Ser Val Trp Leu Gln
785                 790                 795                 800

Leu Leu Phe Gln Val Thr Asn Pro Gly Ile Lys Tyr Glu Tyr Thr Val
                805                 810                 815

Arg Lys Asp Gly Leu Asp Asn Asp Val Glu Lys Leu Leu Tyr Phe Trp
            820                 825                 830

Gln Phe Gly Arg Trp Thr Glu Cys Ser Val Thr Cys Gly Thr Gly Ile
        835                 840                 845

Arg Arg Gln Thr Ala His Cys Val Lys Lys Gly His Gly Ile Val Lys
    850                 855                 860

Thr Thr Phe Cys Asn Pro Glu Thr Gln Pro Ser Val Arg Gln Lys Lys
865                 870                 875                 880

Cys Tyr Glu Lys Asp Cys Pro Pro Arg Trp Trp Ala Gly Glu Trp Glu
                885                 890                 895

Ala Cys Ser Met Thr Cys Gly Pro Tyr Gly Glu Lys Lys Arg Thr Val
            900                 905                 910

Leu Cys Ile Gln Thr Met Gly Ser Asp Glu Gln Ala Leu Pro Ala Thr
        915                 920                 925

Asp Cys Gln His Leu Leu Lys Pro Lys Thr Leu Val Ser Cys Asn Arg
    930                 935                 940

Asp Ile Leu Cys Pro Ser Asp Trp Thr Val Gly Asn Trp Ser Glu Cys
945                 950                 955                 960

Ser Val Ser Cys Gly Gly Gly Val Arg Ile Arg Ser Val Thr Cys Ala
                965                 970                 975

Lys Asn Leu Asn Glu Pro Cys Asp Lys Thr Arg Lys Pro Asn Ser Arg
            980                 985                 990

Ala Leu Cys Gly Leu Gln Gln Cys Pro Phe Ser Arg Arg Val Leu Lys
        995                 1000                1005

Pro Asn Lys Asp Thr Val Pro Ser Gly Lys Asn Pro Thr Thr Ser
    1010                1015                1020

Glu His Asp His Phe Lys Pro Ile Pro Ala Ser Thr Ser Arg Pro
    1025                1030                1035

Thr Pro Leu Ser Thr Pro Thr Val Pro Glu Ser Val Ser Thr Ser
    1040                1045                1050

Thr Pro Thr Ile Asn Ser Leu Gly Ser Thr Ile Thr Ser Gln Glu
    1055                1060                1065

Glu Pro Asp Gly Ile Gly Trp Gln Asn Asn Ser Thr Gln Ala Glu
    1070                1075                1080

Glu Asp Ser His Ile Pro Thr Ser Val Gly Ser Thr Ser Gln Thr
    1085                1090                1095

Pro Leu Thr Ser Trp Ser Trp Ser Met Gln Pro Asp Asp Glu Asn
    1100                1105                1110

Val Ser Ser Ser Ala Ile Gly Pro Thr Ser Glu Ser Asp Phe Trp
    1115                1120                1125

Ala Thr Thr Ser Asp Ser Gly Leu Ser Ser Ser Asn Ala Met Thr
    1130                1135                1140

Trp Gln Val Thr Pro Phe Tyr Ser Thr Ala Thr Thr Glu Pro Glu
    1145                1150                1155

Val Glu Ile His Ser Gly Ser Gly Glu Asp Ser Asp Gln Pro Leu
    1160                1165                1170

Asn Lys Glu Glu Asn Asn Ser Val Leu Trp Asn Lys Ile Arg Val
    1175                1180                1185

Pro Glu Arg Asp Ala Pro Met Glu Met Asp Ala Glu Ile Pro Leu
    1190                1195                1200

Gly Pro Pro Pro Thr Ser Tyr Val Thr Glu Glu Ser Ser Trp Pro
    1205                1210                1215

Pro Phe Ser Thr Met Met Lys Ser Ser Leu Pro Ala Trp Ser Phe
    1220                1225                1230

Lys Asn Glu Thr Pro Arg Asp Glu Gly Met Ile Thr Glu Lys Ser
    1235                1240                1245

Gly Asn Ile Pro Leu Pro Leu Gly Gly Asp His Gln Thr Thr Ser
    1250                1255                1260

Pro Glu Lys Leu Gly Asn Asn Asp Gln Leu Ala Ser Ala Asn Ser
    1265                1270                1275

Thr Asn Pro Thr Gln Gly Ser Gly Pro Val Leu Thr Glu Glu Asp
    1280                1285                1290

Ala Ser Thr Leu Ile Glu Glu Gly Phe Leu Leu Asn Ala Ser Asn
    1295                1300                1305

Tyr Lys His Leu Met Lys Asp His Ser Pro Ala His Trp Ile Val
    1310                1315                1320

Gly Asn Trp Ser Lys Cys Ser Thr Thr Cys Gly Leu Gly Ala Tyr
    1325                1330                1335

Trp Arg Ser Val Glu Cys Ser Thr Gly Met Asn Ala Asp Cys Ala
    1340                1345                1350

Ala Ile Gln Arg Pro Asp Pro Ala Lys Lys Cys His Leu Arg Pro
    1355                1360                1365

Cys Ala Gly Trp Arg Val Gly Asn Trp Ser Lys Cys Ser Arg Asn
    1370                1375                1380

Cys Ser Gly Gly Phe Lys Ile Arg Glu Val Gln Cys Met Asp Gly
    1385                1390                1395

Val Asp His His Arg Ser Leu Arg Pro Phe His Cys Gln Phe Leu
    1400                1405                1410

Ala Gly Val Pro Pro Pro Leu Ser Met Ser Cys Asn Leu Glu Pro
    1415                1420                1425

Cys Glu Glu Trp Lys Val Glu Pro Trp Ser Gln Cys Ser Arg Ser
    1430                1435                1440

Cys Gly Gly Gly Val Gln Glu Arg Gly Val Phe Cys Pro Gly Gly

1445                    1450                    1455

Leu Cys  Asp Trp Thr Lys Arg  Pro Ala Ser Thr Val  Pro Cys Asn
    1460                    1465                    1470

Arg His  Leu Cys Cys His Trp  Ala Thr Gly Asn Trp  Glu Leu Cys
    1475                    1480                    1485

Thr Thr  Ser Cys Gly Gly Gly  Ser Gln Lys Arg Thr  Val His Cys
    1490                    1495                    1500

Ile Pro  Ser Glu Asn Ser Thr  Thr Glu Asp Gln Asp  Gln Cys Phe
    1505                    1510                    1515

Cys Asp  His Gln Ala Arg Pro  Pro Glu Phe Gln Asn  Cys Asn Gln
    1520                    1525                    1530

Gln Ala  Cys Arg Lys Ser Ala  Asp Leu Thr Cys Thr  Lys Asp Arg
    1535                    1540                    1545

Leu Ser  Thr Ser Phe Cys Gln  Thr Leu Lys Ser Met  Lys Lys Cys
    1550                    1555                    1560

Ser Val  Pro Ser Val Arg Val  Gln Cys Cys Leu Ser  Cys Pro Gln
    1565                    1570                    1575

Thr Gln  Ser Ile His Thr Gln  Arg Gln Arg Lys Gln  Gln Met Leu
    1580                    1585                    1590

Gln Asn  His Asp Thr Leu
    1595

<210>  26
<211>  1600
<212>  PRT
<213>  Artificial Sequence

<220>
<223>  Mouse ADAMTS12 from Q811B3

<400>  26

Met Pro Cys Ala Arg Gly Ser Trp Leu Ala Lys Leu Ser Ile Val Ala
1               5                   10                  15

Gln Leu Ile Asn Phe Gly Ala Phe Cys His Gly Arg Gln Thr Gln Pro
            20                  25                  30

Trp Pro Val Arg Phe Pro Asp Pro Arg Gln Glu His Phe Ile Lys Ser
        35                  40                  45

Leu Pro Glu Tyr His Ile Val Ser Pro Val Gln Val Asp Ala Gly Gly
    50                  55                  60

His Val Leu Ser Tyr Gly Leu His His Pro Val Thr Ser Ser Arg Lys
65                  70                  75                  80

Lys Arg Ala Ala Gly Gly Ser Gly Asp Gln Leu Tyr Tyr Arg Ile Ser
                85                  90                  95

His Glu Glu Lys Asp Leu Phe Phe Asn Leu Thr Val Asn Trp Glu Phe
            100                 105                 110

Leu Ser Asn Gly Tyr Val Val Glu Lys Arg Tyr Gly Asn Leu Ser His
        115                 120                 125

Val Lys Met Val Ala Ser Ser Gly Gln Pro Cys His Leu Arg Gly Thr
    130                 135                 140

Val Leu Gln Gln Gly Thr Thr Val Gly Ile Gly Thr Ala Ala Leu Ser
145                 150                 155                 160

Ala Cys Gln Gly Leu Thr Gly Phe Phe His Leu Pro His Gly Asp Phe
                165                 170                 175

Phe Ile Glu Pro Val Lys Lys His Pro Leu Thr Glu Glu Gly Ser Tyr
            180                 185                 190

Pro His Val Val Tyr Arg Arg Gln Ser Ile Arg Ala Pro Glu Thr Lys
        195                 200                 205

Glu Pro Ile Cys Gly Leu Lys Asp Ser Leu Asp Asn Ser Val Lys Gln
    210                 215                 220

Glu Leu Gln Arg Glu Lys Trp Glu Arg Lys Thr Leu Arg Ser Arg Ser
225                 230                 235                 240

Leu Ser Arg Arg Ser Ile Ser Lys Glu Arg Trp Val Glu Thr Leu Val
                245                 250                 255

Val Ala Asp Thr Lys Thr Val Glu Tyr His Gly Ser Glu Asn Val Glu
            260                 265                 270

Ser Tyr Ile Leu Thr Ile Met Asn Met Val Thr Gly Leu Phe His Ser
        275                 280                 285

Pro Ser Ile Gly Asn Leu Val His Ile Val Val Val Arg Leu Ile Leu

290                 295                 300

Leu Glu Glu Glu Glu Gln Gly Leu Lys Ile Val His His Ala Glu Lys
305                 310                 315                 320

Thr Leu Ser Ser Phe Cys Lys Trp Gln Lys Ser Ile Asn Pro Lys Ser
                325                 330                 335

Asp Leu Asn Pro Val His His Asp Val Ala Val Leu Ile Thr Arg Lys
            340                 345                 350

Asp Ile Cys Ala Gly Val Asn Arg Pro Cys Glu Thr Leu Gly Leu Ser
        355                 360                 365

Gln Leu Ser Gly Met Cys Gln Pro His Arg Ser Cys Asn Ile Asn Glu
    370                 375                 380

Asp Ser Gly Leu Pro Leu Ala Phe Thr Ile Ala His Glu Leu Gly His
385                 390                 395                 400

Ser Phe Gly Ile Gln His Asp Gly Lys Glu Asn Asp Cys Glu Pro Val
                405                 410                 415

Gly Arg His Pro Tyr Ile Met Ser Gln Gln Ile Gln Tyr Asp Pro Thr
            420                 425                 430

Pro Leu Thr Trp Ser Lys Cys Ser Lys Glu Tyr Ile Thr Arg Phe Leu
        435                 440                 445

Asp Arg Gly Arg Gly Phe Cys Leu Asp Asp Ile Pro Ser Lys Lys Gly
    450                 455                 460

Leu Lys Ser Asn Val Ile Ala Pro Gly Val Ile Tyr Asp Val His His
465                 470                 475                 480

Gln Cys Gln Leu Gln Tyr Gly Pro Asn Ala Thr Phe Cys Gln Glu Val
                485                 490                 495

Glu Asn Val Cys Gln Thr Leu Trp Cys Ser Val Lys Gly Phe Cys Arg
            500                 505                 510

Ser Lys Leu Asp Ala Ala Ala Asp Gly Thr Arg Cys Gly Glu Lys Lys
        515                 520                 525

Trp Cys Met Ala Gly Lys Cys Ile Thr Val Gly Lys Lys Pro Glu Ser
    530                 535                 540

Ile Pro Gly Gly Trp Gly Arg Trp Ser Pro Trp Ser His Cys Ser Arg
545                 550                 555                 560

Thr Cys Gly Ala Gly Ala Gln Ser Ala Glu Arg Leu Cys Asn Asn Pro
                565                 570                 575

Glu Pro Lys Phe Gly Gly Lys Tyr Cys Thr Gly Glu Arg Lys Arg Tyr
            580                 585                 590

Arg Leu Cys Asn Val His Pro Cys Arg Ser Asp Thr Pro Thr Phe Arg
        595                 600                 605

Gln Met Gln Cys Ser Glu Phe Asp Thr Val Pro Tyr Lys Asn Gln Phe
    610                 615                 620

Tyr Arg Trp Phe Pro Val Phe Asn Ala Ala His Pro Cys Glu Leu Tyr
625                 630                 635                 640

Cys Arg Pro Ile Asp Glu Gln Phe Ser Glu Arg Met Leu Glu Ala Val
                645                 650                 655

Ile Asp Gly Thr Pro Cys Phe Glu Gly Gly Asn Ser Arg Asn Val Cys
            660                 665                 670

Ile Asn Gly Ile Cys Lys Arg Val Gly Cys Asp Tyr Glu Ile Asp Ser
        675                 680                 685

Asn Ala Thr Glu Asp Arg Cys Gly Val Cys Leu Gly Asp Gly Ser Ala
    690                 695                 700

Cys Gln Thr Val Lys Lys Leu Phe Arg Gln Lys Glu Gly Ser Gly Tyr
705                 710                 715                 720

Val Asp Ile Gly Leu Ile Pro Lys Gly Ala Arg Asp Ile Arg Val Met
                725                 730                 735

Glu Ile Lys Ala Ala Gly Asn Phe Leu Ala Ile Arg Ser Glu Asp Pro
            740                 745                 750

Glu Lys Tyr Tyr Leu Asn Gly Gly Phe Ile Ile Gln Trp Asn Gly Asn
        755                 760                 765

Tyr Lys Leu Ala Gly Thr Val Phe Gln Tyr Asp Arg Lys Gly Asp Leu
    770                 775                 780

Glu Lys Leu Ile Ala Pro Gly Pro Thr Asn Glu Ser Val Trp Leu Gln
785                 790                 795                 800

Leu Leu Phe Gln Val Thr Asn Pro Gly Ile Lys Tyr Glu Tyr Thr Val
                805                 810                 815

Arg Lys Asp Gly Leu Asp Asn Asp Val Glu Lys Leu Leu Tyr Phe Trp
            820                 825                 830

Gln Phe Gly Arg Trp Thr Glu Cys Ser Val Thr Cys Gly Thr Gly Ile
        835                 840                 845

Arg Arg Gln Ala Ala His Cys Val Lys Lys Gly His Gly Ile Val Lys
    850                 855                 860

Thr Thr Phe Cys Asn Pro Glu Thr Gln Pro Ser Val Arg Gln Lys Lys
865                 870                 875                 880

Cys His Glu Lys Asp Cys Pro Pro Arg Trp Trp Ala Gly Glu Trp Glu
                885                 890                 895

Ala Cys Ser Thr Thr Cys Gly Pro Tyr Gly Glu Lys Lys Arg Thr Val
            900                 905                 910

Leu Cys Ile Gln Thr Met Gly Ser Asp Glu Gln Ala Leu Pro Ala Thr
        915                 920                 925

Asp Cys Gln His Leu Leu Lys Pro Lys Ala Leu Val Ser Cys Asn Arg
    930                 935                 940

Asp Ile Leu Cys Pro Ser Asp Trp Thr Val Gly Asn Trp Ser Glu Cys
945                 950                 955                 960

Ser Val Ser Cys Gly Gly Gly Val Arg Ile Arg Ser Val Thr Cys Ala
                965                 970                 975

Lys Asn Leu Asn Glu Pro Cys Asp Lys Thr Arg Lys Pro Asn Ser Arg
            980                 985                 990

Ala Leu Cys Gly Leu Gln Gln Cys  Pro Phe Ser Arg Arg  Val Leu Lys
        995                 1000                1005

Pro Asn  Lys Asp Ile Ala Pro  Ser Gly Lys Asn Gln  Ser Thr Ala
    1010                1015                1020

Glu His  Asp Pro Phe Lys Pro  Ile Pro Ala Pro Thr  Ser Arg Pro
    1025                1030                1035

Thr Pro  Leu Ser Thr Pro Thr  Val Pro Glu Ser Met  Ser Thr Ser
    1040                1045                1050

Thr Pro Thr Ile Asn Ser Leu Gly Ser Thr Ile Ala Ser Gln Glu
    1055                1060                1065

Asp Ala Asn Gly Met Gly Trp Gln Asn Asn Ser Thr Gln Ala Glu
    1070                1075                1080

Glu Gly Ser His Phe Pro Thr Ser Ser Gly Ser Thr Ser Gln Val
    1085                1090                1095

Pro Val Thr Ser Trp Ser Leu Ser Ile Gln Pro Asp Asp Glu Asn
    1100                1105                1110

Val Ser Ser Ser Ala Ile Gly Pro Thr Ser Glu Gly Asp Phe Trp
    1115                1120                1125

Ala Thr Thr Thr Ser Asp Ser Gly Leu Ser Ser Ser Asp Ala Met
    1130                1135                1140

Thr Trp Gln Val Thr Pro Phe Tyr Ser Thr Met Thr Thr Asp Pro
    1145                1150                1155

Glu Val Glu Ile His Ser Gly Ser Gly Glu Asp Ser Asp Gln Pro
    1160                1165                1170

Leu Asn Lys Asp Lys Ser Asn Ser Val Ile Trp Asn Lys Ile Gly
    1175                1180                1185

Val Pro Glu His Asp Ala Pro Met Glu Thr Asp Ala Glu Leu Pro
    1190                1195                1200

Leu Gly Pro Pro Pro Thr Ser Tyr Met Gly Glu Glu Pro Ser Trp
    1205                1210                1215

Pro Pro Phe Ser Thr Lys Met Glu Gly Ser Leu Pro Ala Trp Ser
    1220                1225                1230

Phe Lys Asn Glu Thr Pro Arg Asp Asp Gly Met Ile Ala Glu Lys
    1235                1240                1245

Ser Arg Lys Ile Pro Leu Pro Leu Ala Gly Asp His His Pro Ala
    1250                1255                1260

Thr Ser Glu Lys Leu Glu Asn His Asp Lys Leu Ala Leu Pro Asn
    1265                1270                1275

Thr Thr Asn Pro Thr Gln Gly Phe Gly Pro Val Leu Thr Glu Glu

1280                1285                1290

Asp Ala Ser Asn Leu Ile Ala Glu Gly Phe Leu Leu Asn Ala Ser
    1295                1300                1305

Asp Tyr Lys His Leu Met Lys Asp His Ser Pro Ala Tyr Trp Ile
    1310                1315                1320

Val Gly Asn Trp Ser Lys Cys Ser Thr Thr Cys Gly Leu Gly Ala
    1325                1330                1335

Tyr Trp Arg Ser Val Glu Cys Ser Ser Gly Val Asp Ala Asp Cys
    1340                1345                1350

Thr Thr Ile Gln Arg Pro Asp Pro Ala Lys Lys Cys His Leu Arg
    1355                1360                1365

Pro Cys Ala Gly Trp Arg Val Gly Asn Trp Ser Lys Cys Ser Arg
    1370                1375                1380

Asn Cys Ser Gly Gly Phe Lys Ile Arg Glu Val Gln Cys Met Asp
    1385                1390                1395

Ser Leu Asp His His Arg Ser Leu Arg Pro Phe His Cys Gln Phe
    1400                1405                1410

Leu Ala Gly Ala Pro Pro Pro Leu Ser Met Ser Cys Asn Leu Glu
    1415                1420                1425

Pro Cys Gly Glu Trp Gln Val Glu Pro Trp Ser Gln Cys Ser Arg
    1430                1435                1440

Ser Cys Gly Gly Gly Val Gln Glu Arg Gly Val Ser Cys Pro Gly
    1445                1450                1455

Gly Leu Cys Asp Trp Thr Lys Arg Pro Ala Thr Thr Val Pro Cys
    1460                1465                1470

Asn Arg His Leu Cys Cys His Trp Ala Thr Gly Asn Trp Glu Leu
    1475                1480                1485

Cys Asn Thr Ser Cys Gly Gly Gly Ser Gln Lys Arg Thr Ile His
    1490                1495                1500

Cys Ile Pro Ser Glu Asn Ser Thr Thr Glu Asp Gln Asp Gln Cys
    1505                1510                1515

Leu Cys Asp His Gln Val Lys Pro Pro Glu Phe Gln Thr Cys Asn
    1520                1525                1530

Gln Gln Ala Cys Arg Lys Ser Ala Asp Leu Thr Cys Leu Lys Asp
    1535                1540                1545

Arg Leu Ser Ile Ser Phe Cys Gln Thr Leu Lys Ser Met Arg Lys
    1550                1555                1560

Cys Ser Val Pro Ser Val Arg Ala Gln Cys Cys Leu Ser Cys Pro
    1565                1570                1575

Gln Ala Pro Ser Ile His Thr Gln Arg Gln Arg Lys Gln Gln Leu
    1580                1585                1590

Leu Gln Asn His Asp Met Leu
    1595                1600

## Description

### FIELD OF THE INVENTION

The present application relates to substituted hydantoinamides, to processes for their preparation, their use alone or in combination for the treatment and/or prophylaxis of diseases as well as their use in the manufacturing-process of drugs which are used to treat and/or prophylactically treat diseases, in particular for the treatment and/or prophylaxis of heart diseases, vascular diseases, and/or cardiovascular diseases, including atherosclerosis, coronary artery disease (CAD), peripheral vascular disease (PAD)/arterial occlusive disease and/or restenosis after angioplasty (including the use of drug-coated or non drug-coated balloons and/or stent-implantation) and/or for the treatment and/or prophylaxis of lung diseases, inflammatory diseases, fibrotic diseases, metabolic diseases, cardiometabolic diseases and/or diseases/disease states affecting the kidneys and/or the central nervous and/or neurological system as well as gastrointestinal and/or urologic and/or ophthalmologic diseases/disease states.

### BACKGROUND

The pathogenesis of cardiovascular diseases and/or lung diseases as well as metabolic, inflammatory and/or vascular disease states including atherosclerosis and post-angioplasty restenosis is - at least in part - characterized by vascular remodeling (Wang et al., Sheng Li Xue Bao. 2010 Aug 25;62(4):285-94), a process which - amongst others - involves (metallo-)proteinases (Galis & Kathri, Circ Res., 2002 Feb 22;90(3):251-62). Accordingly, it may be beneficial to therapeutically interfere with these proteases in the context of the aforementioned diseases/diseases states. However, a lot of clinical trials having used pan-metalloproteinase inhibitors failed, in part due to severe side effects. Peterson et al. ascribe these negative outcomes to an inadequate assessment of the therapeutic index of the drugs tested, leading to the assumption that it is even more so important to explore the role of individual proteases (Wang et al., Sheng Li Xue Bao. 2010 Aug 25;62(4):285-94; Peterson, Cardiovasc. Res. 2006 Feb 15;69(3):677-87. Epub 2006 Jan 17).

ADAMTS (a disintegrin and metalloproteinase with thrombospondin motifs) constitute a family of metalloproteases which consists of 19 secreted enzymes that have been described to be proteolytically active. ADAMTS play an important role in different processes such as assembly and degradation of extracellular matrix, angiogenesis, homeostasis, organogenesis, cancer, genetic disorders and arthritis (Zhang et al., Mediators Inflamm. 2015;2015:801546. doi: 10.1155/2015/801546. Epub 2015 Nov 30). The 19 ADAMTS enzymes (human) have been subclassified into 8 clusters.

The first 2 clusters comprise the aggrecanse- und proteoglycanase-groups (ADAMTS-1, -4, -5, -8, -15, and ADAMTS-9 & -20). The third cluster, consisting of pro-collagen N-propeptidases, is constituted by ADAMTS-2, -3 und -14 which are crucially involved in the maturation of collagen-fibrils. The only member of the fourth cluster is ADAMTS-13, a protease that cleaves von-Willebrand factor. ADAMTS-7 und ADAMTS-12 are members of the fifth cluster and they have been described to cleave cartilage oligomeric matrix protein (COMP). These two enzymes are unique inasmuch as chondroitinsulfate chains are part of these enzymes and thus confer a proteoglycan status to them (Kelwick et al., Genome Biol., 2015 May 30;16:113. doi: 10.1186/s13059-015-0676-3). In human samples, the highest expression of ADAMTS7 was found in heart, kidney, sceletal muscle, liver and pancreas (Somerville, J. Biol. Chem., 2004 Aug 20;279(34):35159-75. Epub 2004 Jun 10). Likewise, ADAMTS-7 was found to be expressed in bone, cartilage, synovium, tendons and ligaments. Additionally, it was detected in meniscus as well as fat (Liu, Nat Clin Pract Rheumatol. 2009 Jan;5(1):38-45. doi: 10.1038/ncprheum0961).

The three clusters not yet refered to, contain two ADAMTS enzymes each (ADAMTS-6 and -10, ADAMTS-16 and -18, ADAMTS-17 and -19) and are characterized by the organization of the domains of their respective ADAMTS-members.

ADAMTS have been linked to divergent dieseases: For example, mutations in ADAMTS13 have been associated with thrombotic, thrombozyopenic purpura (Levy et al., Nature. 2001 Oct 4;413(6855):488-94). ADAMTS-4 and -5 have been described to be responsible for the degradation of aggrecan in the cartilage of patients suffering from osteoarthritis (Malfait et al., J Biol Chem. 2002 Jun 21;277(25):22201-8. Epub 2002 Apr 15).

The consensus sequence HEXXHXBG(/N/S)BXHD (SEQ ID NO: 16) is shared as catalytic motif by ADAMTS with the three histidine residues coordinating a Zn2+-ion (Kelwick et al., Genome Biol., 2015 May 30;16:113. doi: 10.1186/s13059-015-0676-3). Different from other metalloproteinases, ADAMTS family members show a narrow substrate specificity which may make ADAMTS enzymes a potentially "safe" molecular target (Wu et al., Eur J Med Res, 2015 Mar 21;20:27. doi: 10.1186/s40001-015-0118-4; Wang et al., Sheng Li Xue Bao. 2010 Aug 25;62(4):285-94).

In the context of the initially mentioned diseases/disease states ADAMTS-7 is of special interest since genome-wide association studies showed a correlation between ADAMTS-7 genetic variants and coronary artery disease (CAD). As opposed to genome-wide association studies showing variants in ADAMTS-7 to be associated with the prevalence of CAD and myocardial infarction (MI) (Chan et al., J Am Heart Assoc., 2017 Oct 31;6(11). pii: e006928. doi: 10.1161/JAHA.117.006928), Reilly et al. indentified variant in the ADAMTS-7 gene as being associated with CAD, however, not with MI (Reilly et al., Lancet. 2011 Jan 29;377(9763):383-92. doi: 10.1016/S0140-6736(10)61996-4. Epub 2011 Jan 14). Further supporting a CAD-association, also a meta-analysis of 14 genome-wide association studies identified ADAMTS-7 as locus being linked to CAD (Schunkert et al., Nat. Genet. 2011 Mar 6;43(4):333-8. doi: 10.1038/ng.784).
One of the leading SNPs in the ADAMTS7 locus, rs3825807, shows an adenine (A)-to-guanine (G) exchange in the ADAMTS-7 gene with the G-allel being associated with a reduced risk for CAD. The above mentioned SNP (Single Nucleotide Polymorphism) leads to an amino acid exchange in the pro-domain of ADAMTS-7 and thereby has an impact on the maturation of ADAMTS-7. Furthermore, it has been described that the above mentioned SNP finds itself in a linkage disequilibrium with further SNPs within the ADAMTS-7 locus, such as rs1994016 and rs7178051 (Chan et al., J Am Heart Assoc., 2017 Oct 31;6(11). pii: e006928. doi: 10.1161/JAHA.117.006928). Supporting the aforementioned notions, rs3825807 has also been described to be associated with the susceptibility towards CAD in a chinese population and this association persisted even after correction for clinical co-variates (You et al., Mol Genet. Genomics. 2016 Feb;291(1):121-8. doi: 10.1007/s00438-015-1092-9. Epub 2015 Jul 19).

Investigation of a potential relationship between plasma ADAMTS-7 levels and heart and/or lung function as well as analysis of ADAMTS-7 levels in patients suffering from an acute MI on the one hand showed that plasma ADAMTS-7 levels were higher in patients with a left ventricular ejection fraction (LVEF) below or equaling 35 %, on the other hand revealed that plasma ADAMTS-7 levels positively correlated with, e.g., brain natriuretic peptide (BNP) and left ventricular end-systolic diameter (LVESD) while showing a negative correlation with the 6-min-walking distance (Wu et al., Eur J Med Res, 2015 Mar 21;20:27. doi: 10.1186/s40001-015-0118-4). Furthermore, high ADAMTS-7 levels correlated with a high lipid-content and accumulation of cluster of differentiation (CD) 68 and at the same time with a low smooth muscle cell- as well as a decreased collagen-content being characteristic for vulnerable plaques. Accordingly, ADAMTS-7-levels above median were associated with an increased risk for post-surgery cardiovascular events (Bengtsson et al., Sci Rep., 2017 Jun 16;7(1):3753. doi: 10.1038/s41598-017-03573-4).

Opposing the above mentioned studies, one study failed to show an association between initima- or media-thickness with the G-allel of the ADAMTS-7-SNP. However, a significant association was reported between the rs3825807 G-allel and a reduced thickness of the fibrous cap as well as a decreased portion of intimal α-actin accumulation. Notably, the G-allel of the ADAMTS-7-SNP was linked to a CAD-protective effect (16 % - 19 % reduced risk to be taken ill with each allel after correction for age and gender). Further supporting former reports, no significant association was found between the G-allel and MI.

In a hispanic subgroup, an analysis of the MESA (Multi-Ethnic Study of Artherosclerose)--cohort revealed an association of an ADAMTS-7-SNP different from the one described above and coronary artery calcification (Chan et al., J Am Heart Assoc., 2017 Oct 31;6(11). pii: e006928. doi: 10.1161/JAHA.117.006928).

Taken together, the well documented association with CAD allows the conclusion that ADAMTS-7 increases the risk for CAD via effects on atherosclerosis, potentially without influencing mechanisms which are supposed to favor plaque rupture or thrombosis which themselves are drivers for MI. This assumption is underlined by the observation that the protective G-allel of the ADAMTS-7 variant had no influence on overall mortality or MI (Chan et al., J Am Heart Assoc., 2017 Oct 31;6(11). pii: e006928. doi: 10.1161/JAHA.117.006928).

Experimentally, ADAMTS-7 has been described to be involved in the migration of smooth muslce cells (SMC) and the development of neointimal hyperplasia. Immunohistochemistry showed colocalisation of ADAMTS-7 with vascular smooth muscle cells (VSMC) within the neointima (Wang et al., Circ Res., 2009 Mar 13;104(5):688-98. doi: 10.1161/CIRCRESAHA.108.188425. Epub 2009 Jan 22) and an increased expression of ADAMTS-7 in early- and late-stage human atherosclerotic plaques was described (Wang et al., Sheng Li Xue Bao. 2010 Aug 25;62(4):285-94).

Formation of a neointima is characterized by a media-to-intima migration of VSMC and their subsequent proliferation. Interestingly, migration as well as proliferation of VSMC were accelerated by ADAMTS-7 in vitro (Wang et al., Sheng Li Xue Bao. 2010 Aug 25;62(4):285-94). Along with this observation, overexpression of ADAMTS-7 led to an increased neointima formation in vivo, wereas a knockdown reduced/delayed injury-induced intimal hyperplasia (Wang et al, Circ Res., 2009 Mar 13;104(5):688-98. doi: 10.1161/CIRCRESAHA.108.188425. Epub 2009 Jan 22).

Intersting to note is that pro-inflammatory cytokines such as TNF-α, IL-1β and PDGF-BB were able to induce ADAMTS-7 expression whereas TGF-β reduced its expression. Likewise, reactive oxygen species and H₂O₂ increased, while oxLDL and homocysteine did not change ADAMTS-7 expression in VSMC. All of the aforementioned factors are involved in vascular injury and may contribute to induction of ADAMTS-7 in injured arteries in vivo (Wang et al, Circ Res., 2009 Mar 13;104(5):688-98. doi: 10.1161/CIRCRESAHA.108.188425. Epub 2009 Jan 22).

Mechanistically, ADAMTS-7 may be involved in the pathogenesis of the above mentioned diseases/disease states such as, e.g., vascular disorders by cleaving COMP and thrombospondin-1 (TSP-1), by influencing migration and proliferation of VSMC and by regulating inflammatory cytokines (Zhang et al., Mediators Inflamm. 2015;2015:801546. doi: 10.1155/2015/801546. Epub 2015 Nov 30). In this context, it has been described that ADAMTS-7 directly binds to and cleaves COMP (Liu et al., FASEB J. 2006 May;20(7):988-90. Epub 2006 Apr 3) with the catalytic domain of ADAMTS-7 being responsible for COMP-cleavage (Liu, Nat Clin Pract Rheumatol., 2009 Jan;5(1):38-45. doi: 10.1038/ncprheum0961). The disintegrin-like domain may be involved in the regulation of ADAMTS-7-activity by providing substrate-surfaces (Stanton et al., Biochim Biophys Acta., 2011 Dec;1812(12):1616-29. doi: 10.1016/j.bbadis.2011.08.009. Epub 2011 Sep 2). While ADAMTS-7 accelerates VMSC-migration via COMP-degradation, COMP has no effect on VSMC-proliferation. Interestingly, ADAMTS-7-mediated COMP-degradation in response to injury accelerated VSMC-migration and neointima hyperplasia. Taking into account that during restenosis (after vessel-wall injury) VSMC de-differentiate towards a synthetic phenotype, this may - in the context of the findings described above - lead to the hypothesis that COMP preserves a contractile phenotype in VSMC. Thus, ADAMTS-7 may constitute a new molecular target in conditions of atherosclerosis and/or restenosis after angioplasty (Wang et al., Sheng Li Xue Bao. 2010 Aug 25;62(4):285-94).

Furthermore it has been described that ADAMTS-7 is able to contribute to endothelial repair via direct binding to TSP-1, independent of COMP (Zhang et al., Mediators Inflamm. 2015;2015:801546. doi: 10.1155/2015/801546. Epub 2015 Nov 30). α2-macroglobulin has also been described to associate with ADAMTS-7 and to be its substrate (Luan et al., Osteoarthritis Cartilage. 2008 Nov;16(11):1413-20. doi: 10.1016/j.joca.2008.03.017. Epub 2008 May 15).

In addition, it has been described that ADAMTS7 is involved in VSMC-calcification (Du et al., Arterioscler Thromb Vasc Biol., 2012 Nov;32(11):2580-8. doi: 10.1161/ATVBAHA.112.300206. Epub 2012 Sep 20) and that it contributes to oval cell activation as well as being an important regulator in the context of biliary fibrosis.

Furthermore, ADAMTS-7 seems to be involved in interactions of host and pathogen (Zhang et al., Mediators Inflamm. 2015;2015:801546. doi: 10.1155/2015/801546. Epub 2015 Nov 30.)

ADAMTS-7 and ADAMTS-12 were found to be significantly increased in cartilage and synovium of patients suffering from arthritis (Liu, Nat Clin Pract Rheumatol. 2009 Jan;5(1):38-45. doi: 10.1038/ncprheum0961) and ADAMTS-7 could be detected in the urine of patients with different cancer-entities such as prostate and bladder cancer (Roy et al., Clin Cancer Res. 2008 Oct 15;14(20):6610-7. doi: 10.1158/1078-0432.CCR-08-1136).

Additionally, ADAMTS-7 expression was increased in patients with aortic aneurysms while expression of COMP was markedly reduced in this group of patients as compared to controls. Accordingly, increased expression of ADAMTS-7 and decreased levels of COMP seem to be associated with aortic aneurysms (Qin et al., Mol Med Rep., 2017 Oct;16(4):5459-5463. doi: 10.3892/mmr.2017.7293. Epub 2017 Aug 21).

New therapeutic concepts are necessary to improve the lifes/outcomes of patients who suffer from lung diseases, heart diseases, inflammatory diseases, fibrotic diseases, metabolic diseases, cardiometabolic diseases, vascular diseases and/or cardiovascular diseases, including atherosclerosis, coronary artery disease, peripheral vascular disease/arterial occlusive disease and/or restenosis after angioplasty (including the use of drug-coated or non drug-coated balloons and/or stent-implantation).

Likewise, identification of low molecular weight compounds for a preventive treatment or a treatment to slow progression of these diseases are conceivable and pursued.

### BRIEF DESCRIPTION OF THE FIGURES

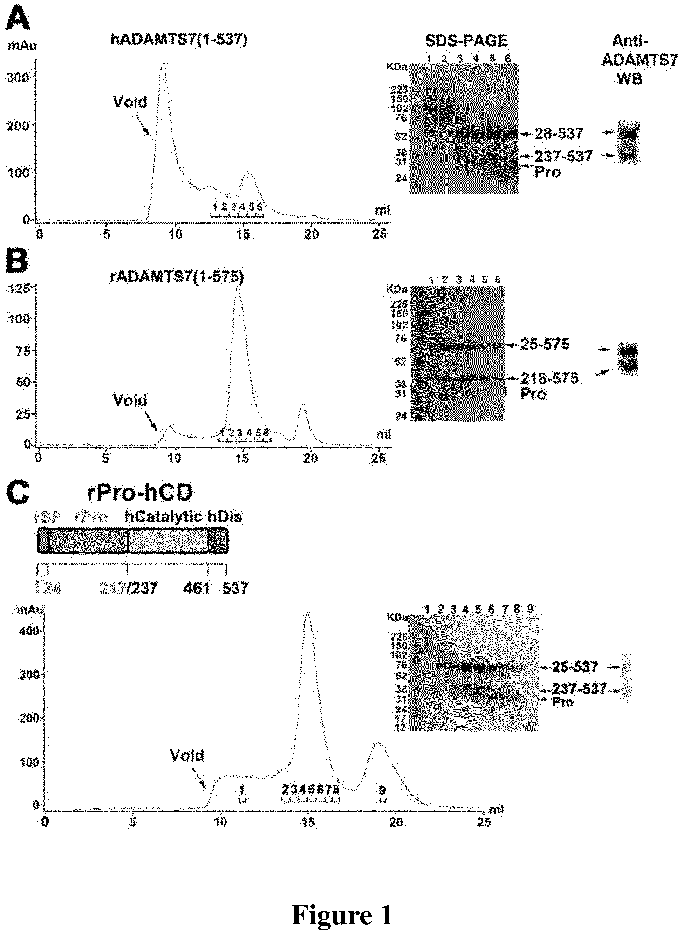

**Figure 1** (consisting of panels A, B, and C) shows comparisons of recombinantly expressed wild type (WT) human ADAMTS-7, WT rat ADAMTS-7, and hybrid ADAMTS-7. Lane numbers of the SDS PAGE correspond with annotated SEC fractions. Figure 1 panel A shows size exclusion (SEC) profile, SDS-page, and western blot (WB) analysis of human ADAMTS-7 (hADAMTS-7) (1-537) from mammalian cell culture. Figure 1 panel B shows SEC profile, SDS-page, and WB analysis of rat ADAMTS-7 (rADAMTS-7) (1-575) from mammalian cell culture. Figure 1 panel C shows the domain design of human and rat hybrid ADAMTS-7 (rPro-hCD) with amino acid position indicated below, as well as SEC profile, SDS-page, and WB analysis of purified hybrid protein from mammalian cell culture. Abbreviations: rSP: rat signal peptide; rPro: rat prodomain; hCatalytic: human catalytic domain; hDis: human disintegrin domain.

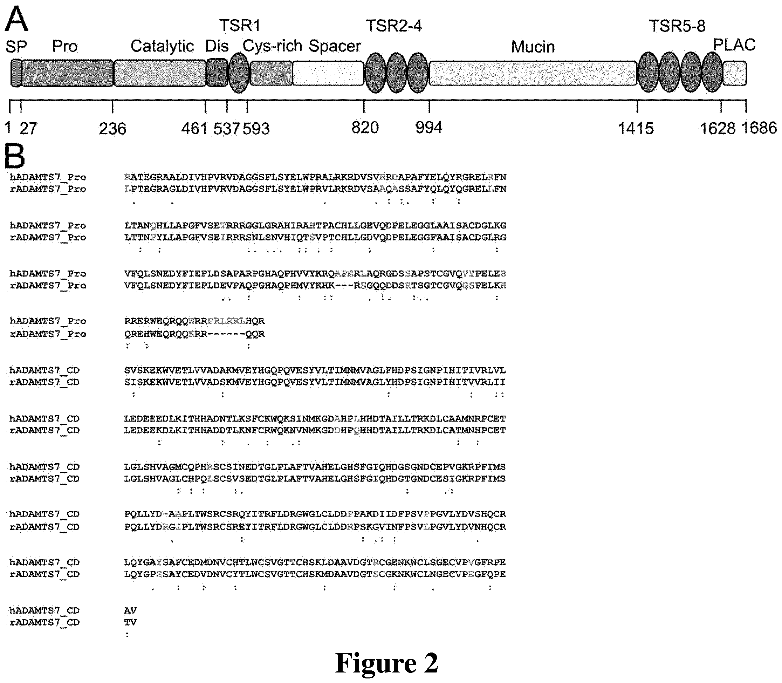

**Figure 2** (consisting of panels A and B) shows the primary structure and sequence comparison of ADAMTS-7. Figure 2 panel A shows the primary structure of hADAMTS-7 with residue numbers labeled at the domain boundaries (SP: signal peptide; Pro: Prodomain; Dis: Disintegrin domain; TSR: thrombospondin repeat; PLAC: protease and lacunin domain. Figure 2 panel B shows a sequence alignment of human and rat ADAMTS-7 in the Pro and CD (catalytic + disintegrin) domains. Different residues were colored in grey, and identical residues were black. Strongly similar residues were black marked with double dots and weakly similar residues were black marked with single dots. Sequence alignment was performed using CLUSTAWL.

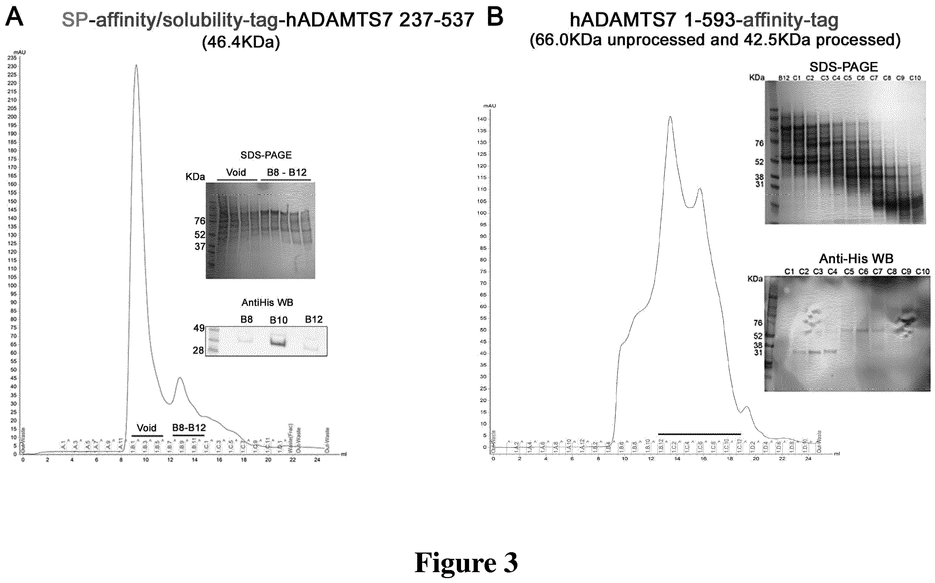

**Figure 3** (consisting of panels A and B) shows two WT constructs for mammalian expression of ADAMTS-7 proteins. Figure 3 panel A shows that the SEC profile of hADAMTS-7 CD domain (residues 237-537) reveals production of soluble proteins (fractions B8-B12) are detectible only in the western blot. Figure 3 panel B shows that the SEC profile of hADAMTS-7 Pro-CD-TSR1 (residues 1-593) yielded little soluble proteins in the expected elution volumes underlined.

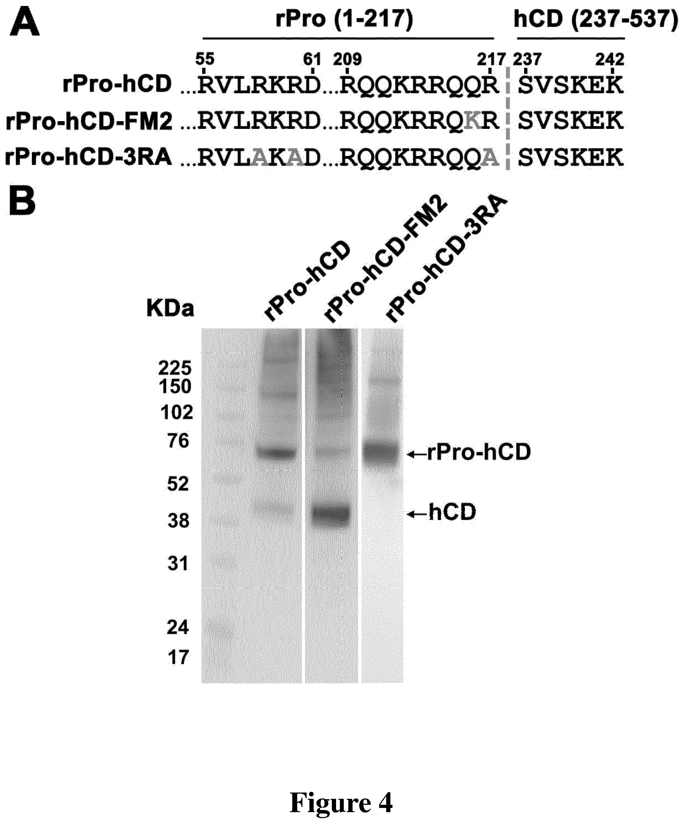

**Figure 4** (consisting of panels A and B) shows that furin cleavage site mutants of ADAMTS-7 improved the yield of processed or unprocessed ADAMTS-7. Figure 4 panel A shows the mutations (grey) introduced into the sequence of the hybrid molecule rPro-hCD to change the furin cleavage efficiency during mammalian cell expression. Q216K mutation in a furin cleavage site and a triple mutant R58A/R61A/R217A were named as rPro-hCD-FM2 (SEQ ID No. 2) and rPro-hCD-3RA respectively. Figure 4 panel B shows that rPro-hCD-FM2 (SEQ ID No. 2) improved the processing to generate more hCD domains during mammalian production and rPro-hCD-3RA abolished the processing to generate unprocessed protein only. Anti-His WB that targets the 6xHis tag at the C-terminus of the protein was used to analyze the raw media of the mammalian cell culture two days post-transfection.

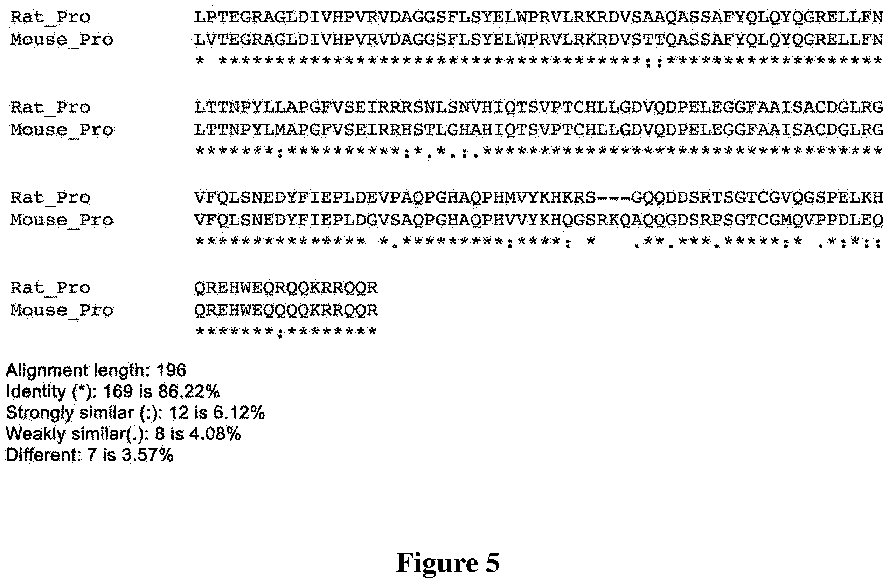

**Figure 5** shows an alignment between mouse ADAMTS-7 prodomain and rat ADAMTS-7 prodomain.

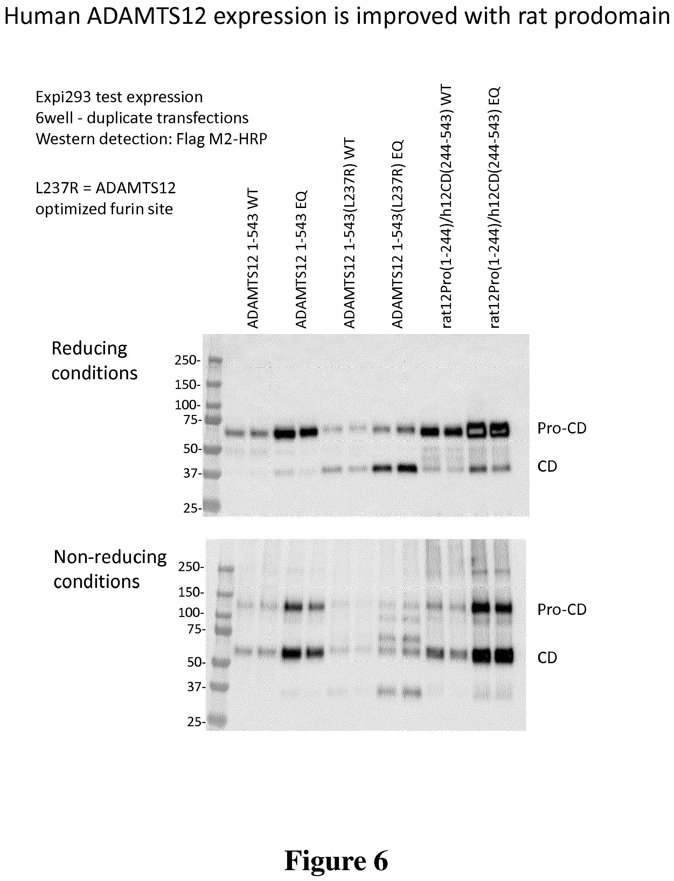

**Figure 6** shows that ADAMTS-12 expression is improved when rat prodomain is used. The first gel was run under non-reducing conditions, whereas the second gel was run under reducing conditions. E393Q substitution (EQ) resulted in increased protein yield. Mutation numbering E393Q follows the species based positions for the human ADAMTS12 region of the construct, i.e. rat ADAMTS12 SP-Pro (1-244) followed by human ADAMTS12 CD (241-544).

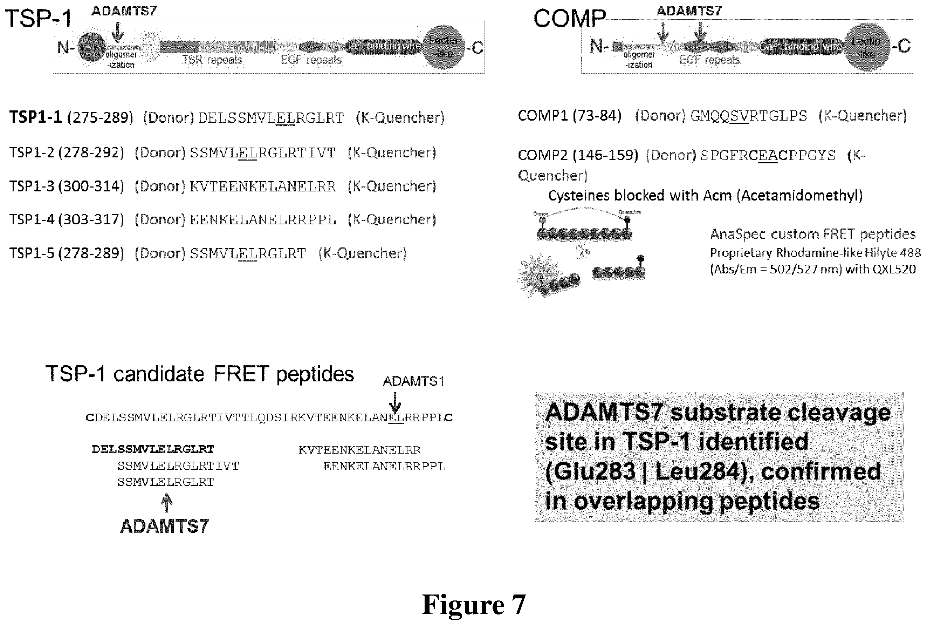

**Figure 7** shows FRET peptide substrate evaluation for TSP-1 and COMP candidate regions.

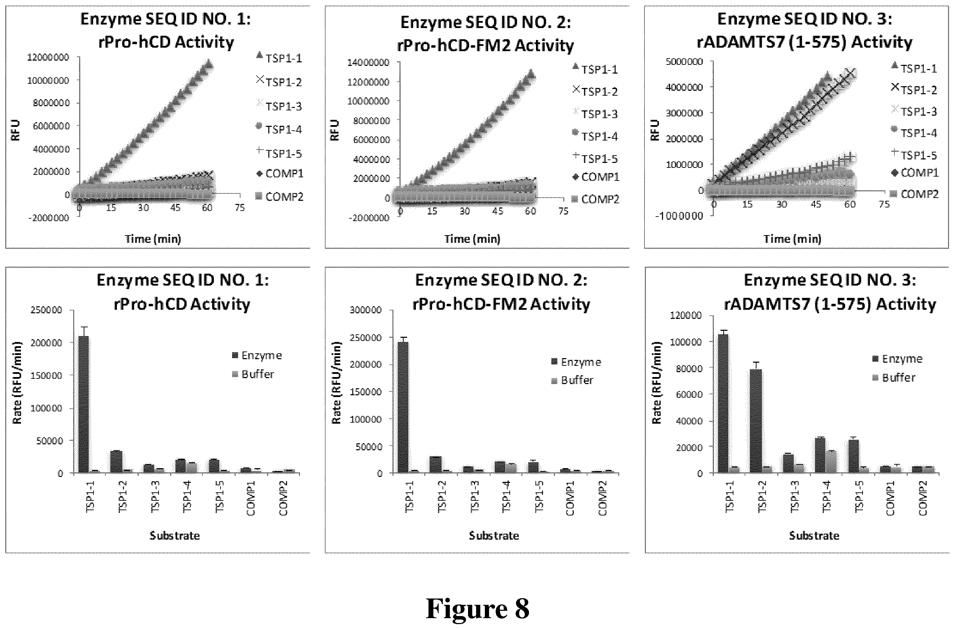

**Figure 8****:** The top panels show curves following enzymatic activity over time with different substrates. The lower panels illustrate the rates of activity for rPro-hCD (cf. SEQ ID No. 1), rPro-hCD-FM2 (cf. SEQ ID No. 2) and rADAMTS-7(1-575) (cf. SEQ ID No. 3), respectively, using different substrates. TSP1-1 substrate (SEQ ID NO:4) is most efficiently turned over by all ADAMTS-7 active constructs.

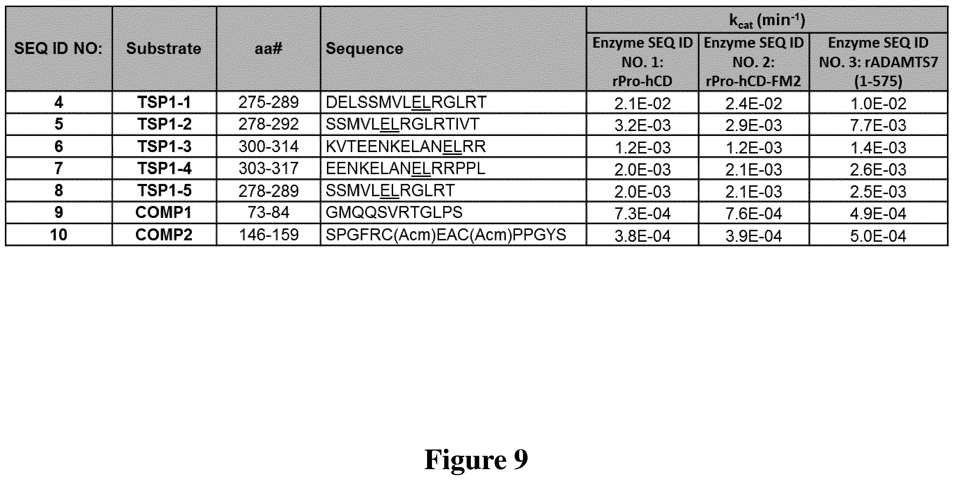

**Figure 9****:** Specific activities for the proteolysis of substrate SEQ ID NO: 4 - 10 show that TSP1-1 substrate is most efficiently turned over by the active ADAMTS-7 constructs. The TSP substrate sequences indicate the cleavage site (underlined) between glutamate and leucine as determined by mass spectrometry. COMP substrate cleavage products were below the detection limit of the mass spectrometer.

### BRIEF DESCRIPTION OF THE SEQUENCE IDs

The sequence listing provided with the application via electronic filing is included herein in its entirety. Where the sequence information or listing apart from the amino acid sequence comprises modifications, fluorophores and/or quencher (i.e. within the feature data) these shall not be read restrictively but only in an exemplary way. The same holds true for non essential modifications such as tags such as FLAG tags or HIS tags.

| SEQ ID NO. | VARIANT | TYPE |
|---|---|---|
| 1 | rPro-hCD (Rat 1-217/Human 237-537)-TEV-2Strep-6His | PRT |
| | MHRGLNLLLILCALAPHVLGPASGLPTEGRAGLDIVHPVRVDAGGSFLSYELWPRVLRKRDVSAAQASS AFYQLQYQGRELLFNLTTNPYLLAPGFVSEIRRRSNLSNVHIQTSVPTCHLLGDVQDPELEGGFAAISAC DGLRGVFQLSNEDYFIEPLDEVPAQPGHAQPHMVYKHKRSGQQDDSRTSGTCGVQGSPELKHQREHW EQRQQKRRQQRSVSKEKWVETLVVADAKMVEYHGQPQVESYVLTIMNMVAGLFHDPSIGNPIHITIVRL VLLEDEEEDLKITHHADNTLKSFCKWQKSINMKGDAHPLHHDTAILLTRKDLCAAMNRPCETLGLSHVAG MCQPHRSCSINEDTGLPLAFTVAHELGHSFGIQHDGSGNDCEPVGKRPFIMSPQLLYDAAPLTWSRCSR QYITRFLDRGWGLCLDDPPAKDIIDFPSVPPGVLYDVSHQCRLQYGAYSAFCEDMDNVCHTLWCSVGTT CHSKLDAAVDGTRCGENKWCLSGECVPVGFRPEAVGSENLYFQSGWSHPQFEKGGGSGGGSGGGS WSHPQFEKHHHHHH | |
| 2 | rPro-hCD-FM2 (Rat 1-217/Human 237-537 FM2 (Q216K))-TEV-2Strep-6His | PRT |
| | MHRGLNLLLILCALAPHVLGPASGLPTEGRAGLDIVHPVRVDAGGSFLSYELWPRVLRKRDVSAAQASS AFYQLQYQGRELLFNLTTNPYLLAPGFVSEIRRRSNLSNVHIQTSVPTCHLLGDVQDPELEGGFAAISAC DGLRGVFQLSNEDYFIEPLDEVPAQPGHAQPHMVYKHKRSGQQDDSRTSGTCGVQGSPELKHQREHW EQRQQKRRQKRSVSKEKWVETLVVADAKMVEYHGQPQVESYVLTIMNMVAGLFHDPSIGNPIHITIVRLV LLEDEEEDLKITHHADNTLKSFCKWQKSINMKGDAHPLHHDTAILLTRKDLCAAMNRPCETLGLSHVAGM CQPHRSCSINEDTGLPLAFTVAHELGHSFGIQHDGSGNDCEPVGKRPFIMSPQLLYDAAPLTWSRCSRQ YITRFLDRGWGLCLDDPPAKDIIDFPSVPPGVLYDVSHQCRLQYGAYSAFCEDMDNVCHTLWCSVGTTC HSKLDAAVDGTRCGENKWCLSGECVPVGFRPEAVGSENLYFQSGWSHPQFEKGGGSGGGSGGGSW SHPQFEKHHHHHH | |
| 3 | rADAMTS-7 (Rat 1-575)-Flag | PRT |
| | MHRGLNLLLILCALAPHVLGPASGLPTEGRAGLDIVHPVRVDAGGSFLSYELWPRVLRKRDVSAAQASS AFYQLQYQGRELLFNLTTNPYLLAPGFVSEIRRRSNLSNVHIQTSVPTCHLLGDVQDPELEGGFAAISAC DGLRGVFQLSNEDYFIEPLDEVPAQPGHAQPHMVYKHKRSGQQDDSRTSGTCGVQGSPELKHQREHW EQRQQKRRQQRSISKEKWVETLVVADSKMVEYHGQPQVESYVLTIMNMVAGLYHDPSIGNPIHITVVRLI ILEDEEKDLKITHHADDTLKNFCRWQKNVNMKGDDHPQHHDTAILLTRKDLCATMNHPCETLGLSHVAG LCHPQLSCSVSEDTGLPLAFTVAHELGHSFGIQHDGTGNDCESIGKRPFIMSPQLLYDRGIPLTWSRCSR EYITRFLDRGWGLCLDDRPSKGVINFPSVLPGVLYDVNHQCRLQYGPSSAYCEDVDNVCYTLWCSVGT TCHSKMDAAVDGTSCGKNKWCLNGECVPEGFQPETVDGGWSGWSAWSVCSRSCGVGVRSSERQCT QPVPKNKGKYCVGERKRYRLCNLQACPENLYFQGDYKDDDDK | |
| 4 | Peptide Substrate for ADAMTS-7/12 | PRT |
| | (HiLyteFluor-488)-DELSSMVLELRGLRT-K(QXL520)-NH2 | |
| 5 | Peptide Substrate for ADAMTS-7/12 | PRT |
| | (HiLyteFluor-488)-SSMVLELRGLRTIVT-K(QXL520)-NH2 | |
| 6 | Peptide Substrate for ADAMTS-7/12: | PRT |
| | (HiLyteFluor-488)-KVTEENKELANELRR-K(QXL520)-NH2 | |
| 7 | Peptide Substrate for ADAMTS-7/12: | PRT |
| | (HiLyteFluor-488)-EENKELANELRRPPL-K(QXL520)-NH2 | |
| 8 | Peptide Substrate for ADAMTS-7/12: | PRT |
| | (HiLyteFluor-488)-SSMVLELRGLRT-K(QXL520)-NH2 | |
| 9 | Peptide Substrate for ADAMTS-7/12: | PRT |
| | (Donor) GMQQSVRTGLPS (K-Quencher) | |
| 10 | Peptide Substrate for ADAMTS-7/12: | PRT |
| | (Donor) SPGFRCEACPPGYS (K-Quencher) | |
| 11 | Peptide Substrate for ADAMTS-7 and ADAMTS-12: | PRT |
| | (HiLyteFluor-488)-DELSSMVLELRGLRT-K(QXL520)-E-NH2 | |
| 12 | Peptide Substrate for ADAMTS-7/12: | PRT |
| | (HiLyteFluor-488)-DELSSMVLELRGLRT-K(QXL520)-K-NH2 | |
| 13 | Peptide Substrate for ADAMTS-7/12: | PRT |
| | (HiLyteFluor-488)-DELSSMVLELRGLRT-K(QXL520)-OH | |
| 14 | Peptide Substrate for ADAMTS4 and for ADAMTS5: | PRT |
| | Dabcyl-EEVKAKVQPY-Glu(Edans)-NH2 | |
| 15 | rPro-hCD for ADAMTS-12 | PRT |
| | MPCAQGNWMAKLSMVAQLLNFGAFCHGRQAQPWPVRFPDPKQEHFIKSLPEYHIVSPVQVDASGHFL SYGLHHPVTGSRKKRAAGGSGDQVYYRISHEEKNLFFNLTVNWEFLSNGYVVERRYGNLSHVKMAASS GQPCHLRGTVLQQGPTIRMGTAALSACQGLTGFFHLPHGDFFIEPVKKHPLTEEGYQPHVIYRRQSYRV PETKEPTCGLKDSLDNSVKQELQREKWERKNWPSRSLSRRSISKERWVETLVVADTKMIEYHGSENVE SYILTIMNMVTGLFHNPSIGNAIHIVVVRLILLEEEEQGLKIVHHAEKTLSSFCKWQKSINPKSDLNPVHHDV AVLLTRKDICAGFNRPCETLGLSHLSGMCQPHRSCNINEDSGLPLAFTIAHELGHSFGIQHDGKENDCEP VGRHPYIMSRQLQYDPTPLTWSKCSEEYITRFLDRGWGFCLDDIPKKKGLKSKVIAPGVIYDVHHQCQLQ YGPNATFCQEVENVCQTLWCSVKGFCRSKLDAAADGTQCGEKKWCMAGKCITVGKKPESIPGGGGSD YKDHDGDYKDHDIDYKDDDDK | |
| 16 | ADAMTS Catalytic Motif Consensus Sequence: HEXXHXBG(/N/S)BXHD | PRT |
| 17 | Peptide Substrate for MMP12 Mca-Pro-Leu-Gly-Leu-Glu-Glu-Ala-Dap(Dnp)-NH2 | PRT |
| 18 | Peptide Substrate for MMP15 MCA-L ys-Pro-Leu-Gly-Leu-DPA-Ala-Arg-NH2 | PRT |
| 19 | Peptide Substrate for MMP2 MCA-Pro-Leu-Gly-Leu-DPA-Ala-Arg-NH2 | PRT |
| 20 | Peptide Substrate for ADAM17 Mca-Pro-Leu-Ala-GIn-Ala-Val-Dap(Dnp)-Arg-Ser-Ser-Ser-Arg-NH2 | PRT |
| 21 | Human ADAMTS-7 from Q9UKP4 | PRT |
| | MPGGPSPRSPAPLLRPLLLLLCALAPGAPGPAPGRATEGRAALDIVHPVRVDAGGSFLSY ELWPRALRKRDVSVRRDAPAFYELQYRGRELRFNLTANQHLLAPGFVSETRRRGGLGRAH IRAHTPACHLLGEVQDPELEGGLAAISACDGLKGVFQLSNEDYFIEPLDSAPARPGHAQP HVVYKRQAPERLAQRGDSSAPSTCGVQVYPELESRRERWEQRQQWRRPRLRRLHQRSVSK EKWVETLVVADAKMVEYHGQPQVESYVLTIMNMVAGLFHDPSIGNPIHITIVRLVLLEDE EEDLKITHHADNTLKSFCKWQKSINMKGDAHPLHHDTAILLTRKDLCAAMNRPCETLGLS HVAGMCQPHRSCSINEDTGLPLAFTVAHELGHSFGIQHDGSGNDCEPVGKRPFIMSPQLL YDAAPLTWSRCSRQYITRFLDRGWGLCLDDPPAKDIIDFPSVPPGVLYDVSHQCRLQYGA YSAFCEDMDNVCHTLWCSVGTTCHSKLDAAVDGTRCGENKWCLSGECVPVGFRPEAVDGG WSGWSAWSICSRSCGMGVQSAERQCTQPTPKYKGRYCVGERKRFRLCNLQACPAGRPSFR HVQCSHFDAMLYKGQLHTWVPVVNDVNPCELHCRPANEYFAEKLRDAVVDGTPCYQVRAS RDLCINGICKNVGCDFEIDSGAMEDRCGVCHGNGSTCHTVSGTFEEAEGLGYVDVGLIPA GAREIRIQEVAEAANFLALRSEDPEKYFLNGGWTIQWNGDYQVAGTTFTYARRGNWENLT SPGPTKEPVWIQLLFQESNPGVHYEYTIHREAGGHDEVPPPVFSWHYGPWTKCTVTCGRG VQRQNVYCLERQAGPVDEEHCDPLGRPDDQQRKCSEQPCPARWWAGEWQLCSSSCGPGGL SRRAVLCIRSVGLDEQSALEPPACEHLPRPPTETPCNRHVPCPATWAVGNWSQCSVTCGE GTQRRNVLCTNDTGVPCDEAQQPASEVTCSLPLCRWPLGTLGPEGSGSGSSSHELFNEAD FIPHHLAPRPSPASSPKPGTMGNAIEEEAPELDLPGPVFVDDFYYDYNFINFHEDLSYGP SEEPDLDLAGTGDRTPPPHSHPAAPSTGSPVPATEPPAAKEEGVLGPWSPSPWPSQAGRS PPPPSEQTPGNPLINFLPEEDTPIGAPDLGLPSLSWPRVSTDGLQTPATPESQNDFPVGK DSQSQLPPPWRDRTNEVFKDDEEPKGRGAPHLPPRPSSTLPPLSPVGSTHSSPSPDVAEL WTGGTVAWEPALEGGLGPVDSELWPTVGVASLLPPPIAPLPEMKVRDSSLEPGTPSFPTP GPGSWDLQTVAVWGTFLPTTLTGLGHMPEPALNPGPKGQPESLSPEVPLSSRLLSTPAWD SPANSHRVPETQPLAPSLAEAGPPADPLVVRNAGWQAGNWSECSTTCGLGAVWRPVRCSS GRDEDCAPAGRPQPARRCHLRPCATWHSGNWSKCSRSCGGGSSVRDVQCVDTRDLRPLRP FHCQPGPAKPPAHRPCGAQPCLSWYTSSWRECSEACGGGEQQRLVTCPEPGLCEEALRPN | |
| | TTRPCNTHPCTQWVVGPWGQCSGPCGGGVQRRLVKCVNTQTGLPEEDSDQCGHEAWPESS RPCGTEDCEPVEPPRCERDRLSFGFCETLRLLGRCQLPTIRTQCCRSCSPPSHGAPSRGH QRVARR | |
| 22 | Rat ADAMTS-7 from Q1EHB3 | PRT |
| | MHRGLNLLLILCALAPHVLGPASGLPTEGRAGLDIVHPVRVDAGGSFLSYELWPRVLRKR DVSAAQASSAFYQLQYQGRELLFNLTTNPYLLAPGFVSEIRRRSNLSNVHIQTSVPTCHL LGDVQDPELEGGFAAISACDGLRGVFQLSNEDYFIEPLDEVPAQPGHAQPHMVYKHKRSG QQDDSRTSGTCGVQGSPELKHQREHWEQRQQKRRQQRSISKEKWVETLVVADSKMVEYHG QPQVESYVLTIMNMVAGLYHDPSIGNPIHITVVRLIILEDEEKDLKITHHADDTLKNFCR WQKNVNMKGDDHPQHHDTAILLTRKDLCATMNHPCETLGLSHVAGLCHPQLSCSVSEDTG LPLAFTVAHELGHSFGIQHDGTGNDCESIGKRPFIMSPQLLYDRGIPLTWSRCSREYITR FLDRGWGLCLDDRPSKGVINFPSVLPGVLYDVNHQCRLQYGPSSAYCEDVDNVCYTLWCS VGTTCHSKMDAAVDGTSCGKNKWCLNGECVPEGFQPETVDGGWSGWSAWSVCSRSCGVGV RSSERQCTQPVPKNKGKYCVGERKRYRLCNLQACPPDRPSFRHTQCSQFDSMLYKGKLHK WVPVLNDENPCELHCRPFNYSNREKLRDAVMDGTPCYQGRISRDICIDGICKKVGCDFEL DSGAEEDRCGVCRGDGSTCHTVSRTFKEAEGMGYVDVGLIPAGAREILIEEVAEAANFLA LRSEDPDKYFLNGGWTIQWNGDYQVAGTTFTYTRKGNWETLTSPGPTTEPVWIQLLFQER NPGVHYKYTIQRASHSEAQPPEFSWHYGPWSKCPVTCGTGVQRQSLYCMEKQAGIVDEGH CDHLSRPRDRKRKCNEEPCPARWWVGDWQPCSRSCGPGGFFRRAVFCTRSVGLDEQRALE PSACGHLPRPLAEIPCYHYVACPSSWGVGNWSQCSVTCGAGIRQRSVLCINNTGVPCDGA ERPITETFCFLQPCQYSTYIVDTGASGSGSSSPELFNEVDFDPHQPVPRPSPASSPKPVS ISNAIDEEDPELDPPGPVFVDDFYYDYNFINFHEDLSYGSFEESHSDLVDIGGQTVPPHI RPTEPPSDSPVPTAGAPGAEEEGIQGSWSPSPLLSEASHSPPVLLENTPVNPLANFLTEE ESPIGAPELGLPSVSWPPASVDGMVTSVAPGNPDELLVREDTQSQPSTPWSDRNKLSKDG NPLGPTSPALPKSPFPTQPSSPSNSTTQASLSPDAVEVSTGWNVALDPVLEADLKPVHAP TDPGLLDQIQTPHTEGTQSPGLLPRPAQETQTNSSKDPAVQPLQPSLVEDGAPTDLLPAK NASWQVGNWSQCSTTCGLGAIWRLVRCSSGNDEDCTLSSRPQPARHCHLRPCAAWRAGNW SKCSRNCGGGSATRDVQCVDTRDLRPLRPFHCQPGPTKPPTRQLCGTQPCLPWYTSSWRE CSEACGGGEQQRLVTCPEPGLCEESLRPNNTRPCNTHPCTQWVVGPWGQCSAPCGGGVQR RLVKCVNTQTGLAEEDSDLCSHEAWPESSRPCATEDCELVEPSRCERDRLPFNFCETLRL LGRCQLPTIRAQCCRSCPPLSRGVPSRGHQRVARR | |
| 23 | Mouse ADAMTS-7 from Q68SA9 | PRT |
| | MHRGPSLLLILCALASRVLGPASGLVTEGRAGLDIVHPVRVDAGGSFLSYELWPRVLRKR DVSTTQASSAFYQLQYQGRELLFNLTTNPYLMAPGFVSEIRRHSTLGHAHIQTSVPTCHL LGDVQDPELEGGFAAISACDGLRGVFQLSNEDYFIEPLDGVSAQPGHAQPHVVYKHQGSR KQAQQGDSRPSGTCGMQVPPDLEQQREHWEQQQKRRQQRSVSKEKWVETLVVADSKMVE YHGQPQVESYVLTIMNMVAGLFHDPSIGNPIHISIVRLIILEDEEKDLKITHHAEETLKN FCRWQKNINIKGDDHPQHHDTAILLTRKDLCASMNQPCETLGLSHVSGLCHPQLSCSVSE DTGMPLAFTVAHELGHSFGIQHDGTGNDCESIGKRPFIMSPQLLYDRGIPLTWSRCSREY ITRFLDRGWGLCLDDRPSKDVIALPSVLPGVLYDVNHQCRLQYGSHSAYCEDMDDVCHTL WCSVGTTCHSKLDAAVDGTSCGKNKWCLKGECVPEGFQPEAVDGGWSGWSAWSDCSRSCG VGVRSSERQCTQPVPKNRGKYCVGERKRSQLCNLPACPPDRPSFRHTQCSQFDGMLYKGK LHKWVPVPNDDNPCELHCRPSNSSNTEKLRDAVVDGTPCYQSRISRDICLNGICKNVGCD FVIDSGAEEDRCGVCRGDGSTCQTVSRTFKETEGQGYVDIGLIPAGAREILIEEVAEAAN FLALRSEDPDKYFLNGGWTIQWNGDYRVAGTTFTYARKGNWENLTSPGPTSEPVWIQLLF QEKNPGVHYQYTIQRDSHDQVRPPEFSWHYGPWSKCTVTCGTGVQRQSLYCMERQAGVVA EEYCNTLNRPDERQRKCSEEPCPPRWWAGEWQPCSRSCGPEGLSRRAVFCIRSMGLDEQR ALELSACEHLPRPLAETPCNRHVICPSTWGVGNWSQCSVTCGAGIRQRSVLCINNTDVPC DEAERPITETFCFLQPCQYPMYIVDTGASGSGSSSPELFNEVDFIPNQLAPRPSPASSPK PVSISNAIDEEELDPPGPVFVDDFYYDYNFINFHEDLSYGSFEEPHPDLVDNGGWTAPPH IRPTESPSDTPVPTAGALGAEAEDIQGSWSPSPLLSEASYSPPGLEQTSINPLANFLTEE DTPMGAPELGFPSLPWPPASVDDMMTPVGPGNPDELLVKEDEQSPPSTPWSDRNKLSTDG NPLGHTSPALPQSPIPTQPSPPSISPTQASPSPDVVEVSTGWNAAWDPVLEADLKPGHGE LPSTVEVASPPLLPMATVPGIWGRDSPLEPGTPTFSSPELSSQHLKTLTMPGTLLLTVPT DLRSPGPSGQPQTPNLEGTQSPGLLPTPARETQTNSSKDPEVQPLQPSLEEDGDPADPLP ARNASWQVGNWSQCSTTCGLGAIWRLVSCSSGNDEDCTLASRPQPARHCHLRPCAAWRTG NWSKCSRNCGGGSSTRDVQCVDTRDLRPLRPFHCQPGPTKPPNRQLCGTQPCLPWYTSSW RECSEACGGGEQQRLVTCPEPGLCEESLRPNNSRPCNTHPCTQWVVGPWGQCSAPCGGGV QRRLVRCVNTQTGLAEEDSDLCSHEAWPESSRPCATEDCELVEPPRCERDRLSFNFCETL RLLGRCQLPTIRAQCCRSCPPLSRGVPSRGHQRVARR | |
| 24 | Human ADAMTS-12 from P58397 | PRT |
| | MPCAQRSWLANLSVVAQLLNFGALCYGRQPQPGPVRFPDRRQEHFIKGLPEYHVVGPVRV DASGHFLSYGLHYPITSSRRKRDLDGSEDWVYYRISHEEKDLFFNLTVNQGFLSNSYIME KRYGNLSHVKMMASSAPLCHLSGTVLQQGTRVGTAALSACHGLTGFFQLPHGDFFIEPVK KHPLVEGGYHPHIVYRRQKVPETKEPTCGLKDSVNISQKQELWREKWERHNLPSRSLSRR | |
| | SISKERWVETLVVADTKMIEYHGSENVESYILTIMNMVTGLFHNPSIGNAIHIVVVRLIL LEEEEQGLKIVHHAEKTLSSFCKWQKSINPKSDLNPVHHDVAVLLTRKDICAGFNRPCET LGLSHLSGMCQPHRSCNINEDSGLPLAFTIAHELGHSFGIQHDGKENDCEPVGRHPYIMS RQLQYDPTPLTWSKCSEEYITRFLDRGWGFCLDDIPKKKGLKSKVIAPGVIYDVHHQCQL QYGPNATFCQEVENVCQTLWCSVKGFCRSKLDAAADGTQCGEKKWCMAGKCITVGKKPES IPGGWGRWSPWSHCSRTCGAGVQSAERLCNNPEPKFGGKYCTGERKRYRLCNVHPCRSEA PTFRQMQCSEFDTVPYKNELYHWFPIFNPAHPCELYCRPIDGQFSEKMLDAVIDGTPCFE GGNSRNVCINGICKMVGCDYEIDSNATEDRCGVCLGDGSSCQTVRKMFKQKEGSGYVDIG LIPKGARDIRVMEIEGAGNFLAIRSEDPEKYYLNGGFIIQWNGNYKLAGTVFQYDRKGDL EKLMATGPTNESVWIQLLFQVTNPGIKYEYTIQKDGLDNDVEQQMYFWQYGHWTECSVTC GTGIRRQTAHCIKKGRGMVKATFCDPETQPNGRQKKCHEKACPPRWWAGEWEACSATCGP HGEKKRTVLCIQTMVSDEQALPPTDCQHLLKPKTLLSCNRDILCPSDWTVGNWSECSVSC GGGVRIRSVTCAKNHDEPCDVTRKPNSRALCGLQQCPSSRRVLKPNKGTISNGKNPPTLK PVPPPTSRPRMLTTPTGPESMSTSTPAISSPSPTTASKEGDLGGKQWQDSSTQPELSSRY LISTGSTSQPILTSQSLSIQPSEENVSSSDTGPTSEGGLVATTTSGSGLSSSRNPITWPV TPFYNTLTKGPEMEIHSGSGEEREQPEDKDESNPVIWTKIRVPGNDAPVESTEMPLAPPL TPDLSRESWWPPFSTVMEGLLPSQRPTTSETGTPRVEGMVTEKPANTLLPLGGDHQPEPS GKTANRNHLKLPNNMNQTKSSEPVLTEEDATSLITEGFLLNASNYKQLTNGHGSAHWIVG NWSECSTTCGLGAYWRRVECSTQMDSDCAAIQRPDPAKRCHLRPCAGWKVGNWSKCSRNC SGGFKIREIQCVDSRDHRNLRPFHCQFLAGIPPPLSMSCNPEPCEAWQVEPWSQCSRSCG GGVQERGVFCPGGLCDWTKRPTSTMSCNEHLCCHWATGNWDLCSTSCGGGFQKRTVQCVP SEGNKTEDQDQCLCDHKPRPPEFKKCNQQACKKSADLLCTKDKLSASFCQTLKAMKKCSV PTVRAECCFSCPQTHITHTQRQRRQRLLQKSKEL | |
| 25 | Rat ADAMTS-12 from D3ZTJ3 | PRT |
| | MPCAQGNWMAKLSMVAQLLNFGAFCHGRQAQPWPVRFPDPKQEHFIKSLPEYHIVSPVQV DASGHFLSYGLHHPVTGSRKKRAAGGSGDQVYYRISHEEKNLFFNLTVNWEFLSNGYVVE RRYGNLSHVKMAASSGQPCHLRGTVLQQGPTIRMGTAALSACQGLTGFFHLPHGDFFIEP VKKHPLTEEGYQPHVIYRRQSYRVPETKEPTCGLKDSLDNSVKQELQREKWERKNWPSRS LSRRSISKERWVETLVVADTKMVEYHGSENVESYILTIMNMVTGLFHNPSIGNAVHIVVV RLILLEEEEQGLKIVHHAEKTLSSFCKWQKSINPKSDLNPVHHDVAVLITRKDICAGVNR PCETLGLSQLSGMCQPHRSCNINEDSGLPLAFTIAHELGHSFGIQHDGKENDCEPVGRHP YIMSQQIQYDPTPLTWSKCSKEYITRFLDRGRGFCLDDVPRKKGLKSNVIAPGVIYDVHH QCQLQYGPNATFCQEVENVCQTLWCSVKGFCRSKLDAAADGTRCGEKKWCMAGKCITVGK KPESIPGGWGRWSPWSHCSRTCGAGAQSAERLCNNPEPKFGGKYCTGERKRYRLCNVHPC RSDTPTFRQMQCSEFDTVPYKNQFYRWFPVFNPAHPCELYCRPIDEQFSERMLEAVIDGT PCFEGGNSRNVCINGICKRVGCDYEIDSNATEDRCGVCLGDGSACQTVKKVFRQKEGSGY IDIGLIPKGARDIRVMEIKAAGNFLAIRSEDPEKYYLNGGFIIQWNGNYKLAGTVFQYDR KGDLERLMAPGPTNESVWLQLLFQVTNPGIKYEYTVRKDGLDNDVEKLLYFWQFGRWTEC SVTCGTGIRRQTAHCVKKGHGIVKTTFCNPETQPSVRQKKCYEKDCPPRWWAGEWEACSM TCGPYGEKKRTVLCIQTMGSDEQALPATDCQHLLKPKTLVSCNRDILCPSDWTVGNWSEC SVSCGGGVRIRSVTCAKNLNEPCDKTRKPNSRALCGLQQCPFSRRVLKPNKDTVPSGKNP TTSEHDHFKPIPASTSRPTPLSTPTVPESVSTSTPTINSLGSTITSQEEPDGIGWQNNST QAEEDSHIPTSVGSTSQTPLTSWSWSMQPDDENVSSSAIGPTSESDFWATTSDSGLSSSN AMTWQVTPFYSTATTEPEVEIHSGSGEDSDQPLNKEENNSVLWNKIRVPERDAPMEMDAE IPLGPPPTSYVTEESSWPPFSTMMKSSLPAWSFKNETPRDEGMITEKSGNIPLPLGGDHQ TTSPEKLGNNDQLASANSTNPTQGSGPVLTEEDASTLIEEGFLLNASNYKHLMKDHSPAH WIVGNWSKCSTTCGLGAYWRSVECSTGMNADCAAIQRPDPAKKCHLRPCAGWRVGNWSKC SRNCSGGFKIREVQCMDGVDHHRSLRPFHCQFLAGVPPPLSMSCNLEPCEEWKVEPWSQC SRSCGGGVQERGVFCPGGLCDWTKRPASTVPCNRHLCCHWATGNWELCTTSCGGGSQKRT VHCIPSENSTTEDQDQCFCDHQARPPEFQNCNQQACRKSADLTCTKDRLSTSFCQTLKSM KKCSVPSVRVQCCLSCPQTQSIHTQRQRKQQMLQNHDTL | |
| 26 | Mouse ADAMTS-12 from Q811 B3 | PRT |
| | MPCARGSWLAKLSIVAQLINFGAFCHGRQTQPWPVRFPDPRQEHFIKSLPEYHIVSPVQV DAGGHVLSYGLHHPVTSSRKKRAAGGSGDQLYYRISHEEKDLFFNLTVNWEFLSNGYVVE KRYGNLSHVKMVASSGQPCHLRGTVLQQGTTVGIGTAALSACQGLTGFFHLPHGDFFIEP VKKHPLTEEGSYPHVVYRRQSIRAPETKEPICGLKDSLDNSVKQELQREKWERKTLRSRS LSRRSISKERWVETLVVADTKTVEYHGSENVESYILTIMNMVTGLFHSPSIGNLVHIVVV RLILLEEEEQGLKIVHHAEKTLSSFCKWQKSINPKSDLNPVHHDVAVLITRKDICAGVNR PCETLGLSQLSGMCQPHRSCNINEDSGLPLAFTIAHELGHSFGIQHDGKENDCEPVGRHP YIMSQQIQYDPTPLTWSKCSKEYITRFLDRGRGFCLDDIPSKKGLKSNVIAPGVIYDVHH QCQLQYGPNATFCQEVENVCQTLWCSVKGFCRSKLDAAADGTRCGEKKWCMAGKCITVGK KPESIPGGWGRWSPWSHCSRTCGAGAQSAERLCNNPEPKFGGKYCTGERKRYRLCNVHPC RSDTPTFRQMQCSEFDTVPYKNQFYRWFPVFNAAHPCELYCRPIDEQFSERMLEAVIDGT PCFEGGNSRNVCINGICKRVGCDYEIDSNATEDRCGVCLGDGSACQTVKKLFRQKEGSGY VDIGLIPKGARDIRVMEIKAAGNFLAIRSEDPEKYYLNGGFIIQWNGNYKLAGTVFQYDR KGDLEKLIAPGPTNESVWLQLLFQVTNPGIKYEYTVRKDGLDNDVEKLLYFWQFGRWTEC | |
| | SVTCGTGIRRQAAHCVKKGHGIVKTTFCNPETQPSVRQKKCHEKDCPPRWWAGEWEACST TCGPYGEKKRTVLCIQTMGSDEQALPATDCQHLLKPKALVSCNRDILCPSDWTVGNWSEC SVSCGGGVRIRSVTCAKNLNEPCDKTRKPNSRALCGLQQCPFSRRVLKPNKDIAPSGKNQ STAEHDPFKPIPAPTSRPTPLSTPTVPESMSTSTPTINSLGSTIASQEDANGMGWQNNST QAEEGSHFPTSSGSTSQVPVTSWSLSIQPDDENVSSSAIGPTSEGDFWATTTSDSGLSSS DAMTWQVTPFYSTMTTDPEVEIHSGSGEDSDQPLNKDKSNSVIWNKIGVPEHDAPMETDA ELPLGPPPTSYMGEEPSWPPFSTKMEGSLPAWSFKNETPRDDGMIAEKSRKIPLPLAGDH HPATSEKLENHDKLALPNTTNPTQGFGPVLTEEDASNLIAEGFLLNASDYKHLMKDHSPA YWIVGNWSKCSTTCGLGAYWRSVECSSGVDADCTTIQRPDPAKKCHLRPCAGWRVGNWSK CSRNCSGGFKIREVQCMDSLDHHRSLRPFHCQFLAGAPPPLSMSCNLEPCGEWQVEPWSQ CSRSCGGGVQERGVSCPGGLCDWTKRPATTVPCNRHLCCHWATGNWELCNTSCGGGSQKR TIHCIPSENSTTEDQDQCLCDHQVKPPEFQTCNQQACRKSADLTCLKDRLSISFCQTLKS MRKCSVPSVRAQCCLSCPQAPSIHTQRQRKQQLLQNHDML | |

According to a first aspect the present invention provides novel low-molecular-weight compounds which act as potent antagonists of the ADAMTS7 receptor and are thus suitable for treatment and/or prevention of lung diseases, heart diseases, inflammatory diseases, fibrotic diseases, metabolic diseases, cardiometabolic diseases, vascular diseases and/or cardiovascular diseases.

Further embodiments are directed to the identification of ADAMTS7 antagonists that are suitable for treatment and/or prevention of atherosclerosis, athersclerosis-related diseases such as coronary artery disease or peripheral vascular disease/arterial occlusive disease as well as post-surgery complications of these diseases such as restenosis after angioplasty.

Further embodiments are directed to the identification of selective ADAMTS7 antagonists in respect to ADAMTS4 antagonistic effect.

WO 2014/06651 discloses substituted hydantoinamides as ADAMTS4 and 5 inhibitors for use in the treatment of arthritis in particular osteoarthritis.

WO 2004/024721 and WO2004024715 describe hydantoin derivatives and their use as TACE (ADAMT17) inhibitors.

WO2004/108086 and WO2002/096426 disclose Hydantoin derivatives as TACE inhibitors.

WO 2017/211666 and WO2017/211667 disclose Hydantoin derivatives as ADAMTS4 and 5 inhibitors for the treatment of inflammatory diseases preferably osteoarthritis.

WO01/44200 generically discloses a broad range of compounds as selective neurokinin antagonists.

WO2018/069532 discloses inhibitors of alpha-amino-beta carboxymuconic acid semialdehyde decarboxylase. 9 distinct compunds with the following CAS numbers 2224459-87-2, 2224418-23-7, 2224397-97-9, 2224363-23-7, 2224363-22-6, 2224237-51-6, 2224237-28-7, 2224196-43-2 and2224145-02-0 have been published without further information with regard to any pharmaceutical use.

It has now been found that compounds of the present invention have surprising and advantageous properties.

In particular, it has been found that compounds of the present invention are ADAMTS7 antagonists, many of them potent and selective antagonists for ADAMTS7. Moreover, many of the compounds of the invention are selective with respect to ADAMTS4 - preferably 10 times, or even 50 times more selective against ADAMTS7 relative to ADAMTS4.

The present invention provides compounds of general formula (I): 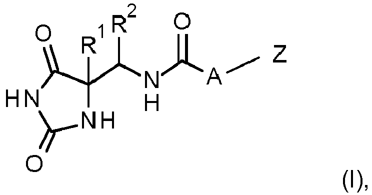 in which
- R¹: represents a group selected from hydrogen, (C₁-C₆)-alkyl, (C₃-C₆)-cycloalkyl, 5- to 6-membered heterocycloalkyl, 5- to 6-membered heteroaryl and phenyl,
wherein said (C₁-C₆)-alkyl, (C₃-C₆)-cycloalkyl, 5- to 6-membered heterocycloalkyl, and 5- to 6-membered heteroaryl, phenyl are optionally substituted with one or two groups independently selected from cyano, halogen, amino, hydroxy, oxo, (C₁-C₃)-alkyl, (C₁-C₄)-alkoxy, (C₁-C₄)-alkylcarbonyl, mono-(C₁-C₄)-alkylamino, di-(C₁-C₄)-alkylamino, (C₁-C₄)-alkylsulfonyl, (C₃-C₆)-cycloalkyl, ,
wherein each said (C₁-C₃)-alkyl, (C₃-C₆)-cycloalkyl, and (C₁-C₄)-alkoxy is optionally substituted with up to 5 fluorine atoms,
- R²: represents a group selected from hydrogen or (C₁-C₄)-alkyl
wherein said (C₁-C₄)-alkyl is optionally substituted with up to five fluorine atoms,
- A: represents a group selected from 5-membered heteroaryl
wherein said 5-membered heteroaryl is optionally substituted with one, two or three groups independently selected from halogen, cyano, hydroxy, methylsulfonyl, (C₃-C₆)-cycloalkyl, (C₁-C₄)-alkyl, and (C₁-C₄)-alkoxy,
wherein each said (C₃-C₆)-cycloalkyl, (C₁-C₄)-alkyl and (C₁-C₄)-alkoxy is optionally substituted with up to three fluorine atoms,
- Z: represents a group selected from 6- to 10-membered aryl and 5- to 10-membered heteroaryl,
wherein said 6- to 10-membered aryl or 5- to 10-membered heteroaryl is optionally substituted with one, two or three groups independently selected from halogen, cyano, hydroxy, (C₁-C₄)-alkylsulfonyl, (C₃-C₆)-cycloalkyl, (C₁-C₄)-alkyl, and (C₁-C₄)-alkoxy,
wherein each said (C₁-C₄)-alkyl and (C₁-C₄)-alkoxy is optionally substituted with (C₃-C₆)-cycloalkyl and optionally substituted with up to five fluorine atoms,
and pharmaceutically acceptable salts thereof, solvates thereof and the solvates of the salts thereof.

In certain embodiments, the present invention provides compounds of general formula (I) with the proviso that the following compounds (Xa) to (Xi) are excluded:
4-(4-chlorophenyl)-N-[[4-(1-methylimidazol-2-yl)-2,5-dioxo-imidazolidin-4-yl]methyl]-1H-pyrrole-2-carboxamide 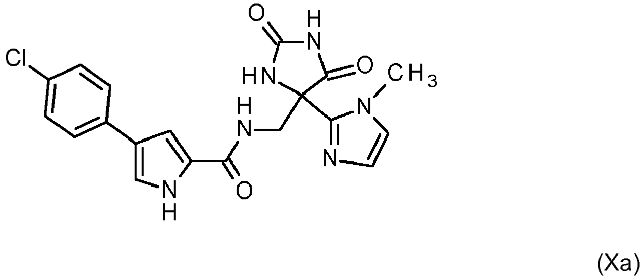
1-(4-fluorophenyl)-N-[[4-(1-methylimidazol-2-yl)-2,5-dioxo-imidazolidin-4-yl]methyl]pyrazole-4-carboxamide 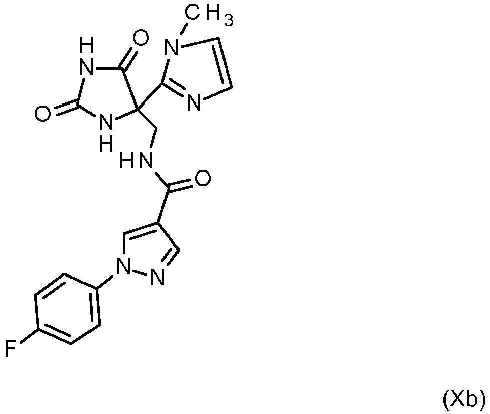
1-methyl-N-[[4-(1-methylimidazol-2-yl)-2,5-dioxo-imidazolidin-4-yl]methyl]-5-phenyl-pyrrole-2-carboxamide 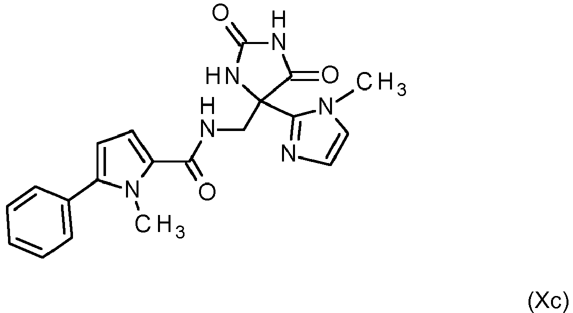
4-(5-chloro-2-thienyl)-N-[[4-(1-methylimidazol-2-yl)-2,5-dioxo-imidazolidin-4-yl]methyl]-1 H-pyrrole-3-carboxamide 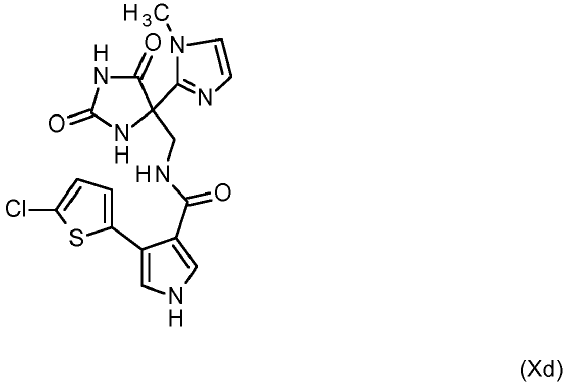
4-methyl-N-[[4-(1-methylimidazol-2-yl)-2,5-dioxo-imidazolidin-4-yl]methyl]-2-(2-thienyl)thiazole-5-carboxamide 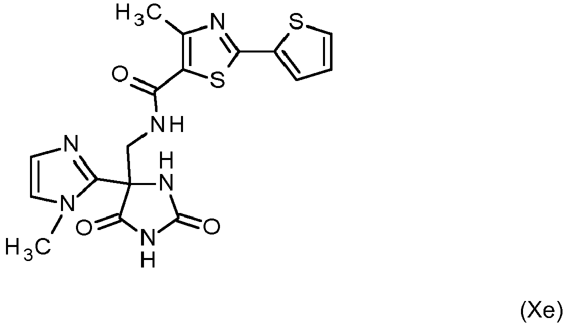
N-[[4-(1-methylimidazol-2-yl)-2,5-dioxo-imidazolidin-4-yl]methyl]-2-(4-pyridyl)thiazole-5-carboxamide 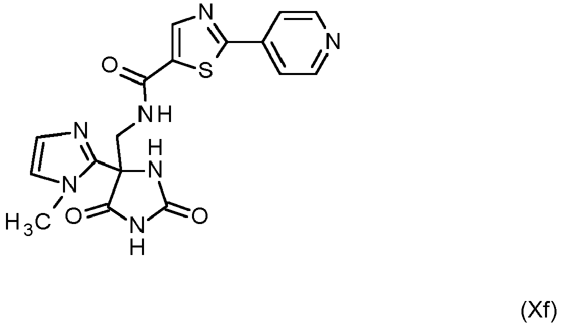
5-methyl-N-[[4-(1-methylimidazol-2-yl)-2,5-dioxo-imidazolidin-4-yl]methyl]-1-(4-pyridyl)pyrazole-4-carboxamide 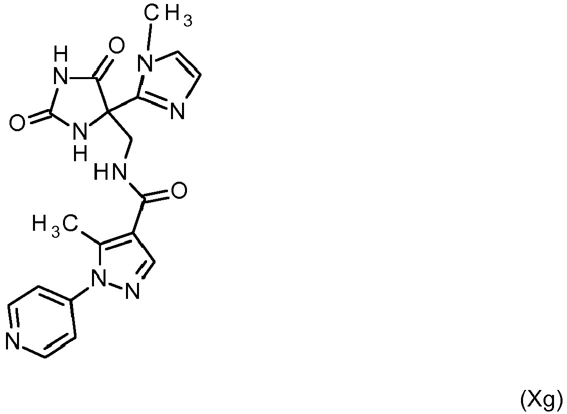
N-[[4-(1-methylimidazol-2-yl)-2,5-dioxo-imidazolidin-4-yl]methyl]-5-(1 H-pyrazol-3-yl)thiophene-2-carboxamide 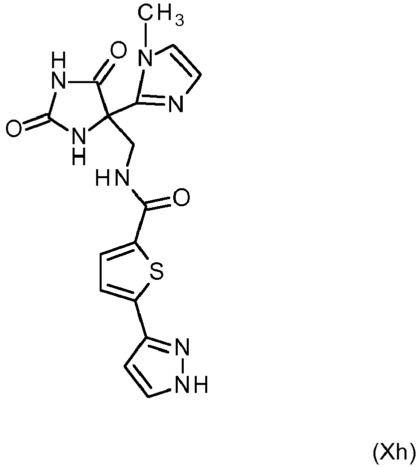
N-[[4-(1-methylimidazol-2-yl)-2,5-dioxo-imidazolidin-4-yl]methyl]-5-phenyl-1H-pyrazole-3-carboxamide

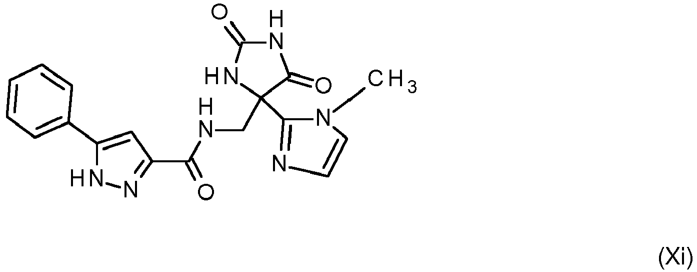

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of non-hydrogen substituents can be equal to or different from zero. Unless otherwise indicated, optionally substituted groups may be substituted with as many optional substituents as can be accommodated by replacing a hydrogen atom with a non-hydrogen substituent on any available carbon atom or heteroatom.

When groups in the compounds according to the invention are substituted, said groups may be mono-substituted or poly-substituted with substituent(s), unless otherwise specified. Within the scope of the present invention, the meanings of all groups which occur repeatedly are independent from one another. Groups in the compounds according to the invention may, for example, be substituted with one, two or three identical or different substituents.

As used herein, an "oxo" substituent represents an oxygen atom, which is bound to a carbon atom or to a sulfur atom via a double bond.

As used herein "allyl" is a substituent with the structural formula H₂C=CH-CH₂*, where * is the connection to the rest of the molecule. It consists of a methylene bridge (-CH₂-) attached to a vinyl group (-CH=CH₂).

The term "ring substituent" means a non-hydrogen substituent attached to an aromatic or nonaromatic ring which replaces an available hydrogen atom on the ring.

The term "comprising" when used in the specification includes "consisting of".

If within the present text any item is referred to as "as mentioned herein", it means that it may be mentioned anywhere in the present text.

The terms as mentioned in the present text have the following meanings:
The term "halogen" or "halogen atom" means a fluorine, chlorine, bromine or iodine atom, preferably a fluorine, chlorine or bromine atom, even more preferably fluorine or chlorine most preferred fluorine.

The term "C₁-C₃ -alkyl", "C₁-C₄-alkyl" and "C₁-C₆-alkyl" means a linear or branched, saturated, monovalent hydrocarbon group having 1, 2 or 3, 1, 2, 3, or 4 carbon atoms, and 1, 2, 3, 4, 5 or 6 carbon atoms, *e.g.* a methyl, ethyl, propyl, isopropyl, butyl, sec-butyl, isobutyl, *tert*-butyl, pentyl, isopentyl, 2-methylbutyl, 1-methylbutyl, 1-ethylpropyl, 1,2-dimethylpropyl, neo-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer thereof. Preferably, said group has 1, 2, 3 or 4 carbon atoms ("C₁-C₄-alkyl"), e.g., a methyl, ethyl, propyl, isopropyl, butyl, sec-butyl isobutyl, or *tert*-butyl group, more preferably 1, 2 or 3 carbon atoms ("C₁-C₃-alkyl"), *e.g.,* a methyl, ethyl, n-propyl or isopropyl group.

The terms "C₁-C₄-alkoxy" means a linear or branched, saturated, monovalent group of formula (C₁-C₄-alkyl)-O-, in which the term "C₁-C₄-alkyl" is as defined *supra, e.g.,* a methoxy, ethoxy, n-propoxy, isopropoxy, butoxy or an isomer thereof, preferably a methoxy-group or ethoxy-group.

The term "C₃-C₆-cycloalkyl" means a saturated, monovalent, mono- or bicyclic hydrocarbon ring which contains 3, 4, 5 or 6 carbon atoms, respectively. Said C₃-C₆-cycloalkyl group may be for example, a monocyclic hydrocarbon ring, e.g., a cyclopropyl, cyclobutyl, cyclopentyl or cyclohexyl group, or a bicyclic hydrocarbon ring. The term "3- to 6-membered cycloalkyl" is equivalent to a "C₃-C₆-cycloalkyl". Thus a "4-membered cycloalkyl group" has the same meaning as a "C₄-cycloalkyl group", and a "C₃-cycloalkyl" is a cyclopropyl-group.

The term "5- to 6-membered heterocycloalkyl means a monocyclic, saturated heterocycloalkyl with 5 or 6 ring atoms in total, respectively, which contains one or two identical or different ring heteroatoms from the series N, S or O. The heterocycloalkyl group may be attached to the rest of the molecule via any one of the carbon or nitrogen atoms. A heterocycloalkyl group which contains at least one ring nitrogen atom may be referred to as aza-heterocycloalkyl, and a heterocycloalkyl group which contains at least one ring oxygen atom may be referred to as oxa-heterocycloalkyl. Preferably, an aza-heterocycloalkyl group contains only nitrogen and carbon atoms in the ring, and an oxa-heterocycloalkyl group contains only ring oxygen and carbon atoms in the ring.

Said heterocycloalkyl without being limited thereto, can be a 5-membered ring, such as tetrahydrofuranyl, 1,3-dioxolanyl, thiolanyl, pyrrolidinyl, imidazolidinyl, pyrazolidinyl, 1,1-dioxidothiolanyl, 1,2-oxazolidinyl, 1,3-oxazolidinyl or 1,3-thiazolidinyl, for example; or a 6-membered ring, such as tetrahydropyranyl, tetrahydrothiopyranyl, piperidinyl, morpholinyl, dithianyl, thiomorpholinyl, piperazinyl, 1,3-dioxanyl, 1,4-dioxanyl or 1,2-oxazinanyl, for example.

The term 4- to 5-membered aza-heterocycloalkyl means a monocyclic saturated or unsaturated heterocycloalkyl group with 4 or 5 ring atoms in total, respectively, which contains at least one ring nitrogen atom, and which is attached to the rest of the molecule via a ring carbon atom or optionally via a ring nitrogen atom (if allowed by valency). The following heterocycloalkyls may be mentioned by way of example: Pyrrolidin, Pyrrolin, Azetidin, Oxazolidin

The term "6- to 10-membered aryl" means a mono- or optionally bicyclic aromatic ring with 6 to 10 (preferably 6) carbon ring atoms in total, such as phenyl or naphthyl.

The term "5- to 10-membered heteroaryl", "5- to 6-membered heteroaryl", "5-membered heteroaryl" and "6-membered heteroaryl" means a mono- or optionally bicyclic aromatic ring with 5 to 10, 5 to 6, 5, or 6 ring atoms in total, respectively, which contains at least one ring heteroatom and optionally one, two or three further ring heteroatoms from the series: N, O and/or S, and which is attached to the rest of the molecule via a ring carbon atom or optionally via a ring nitrogen atom (if allowed by valency).

The following representative heteroaryls may be mentioned by way of example: furyl, pyrrolyl, thienyl, pyrazolyl, imidazolyl, thiazolyl, oxazolyl, isoxazolyl, isothiazolyl, triazolyl, oxadiazolyl, thiadiazolyl, pyridyl, pyrimidinyl, pyridazinyl, pyrazinyl, triazinyl, benzofuranyl, benzothienyl, benzimidazolyl, benzoxazolyl, benzothiazolyl, benzotriazolyl, indolyl, indazolyl, quinolinyl, isoquinolinyl, naphthyridinyl, quinazolinyl, quinoxalinyl, phthalazinyl, and pyrazolo[3,4-b]pyridinyl.

Said heteroaryl group can be a 5-membered heteroaryl group, such as, for example, thienyl, furanyl, pyrrolyl, oxazolyl, thiazolyl, imidazolyl, pyrazolyl, isoxazolyl, isothiazolyl, oxadiazolyl, triazolyl, thiadiazolyl or tetrazolyl; or a 6-membered heteroaryl group, such as, for example, pyridinyl, pyridazinyl, pyrimidinyl, pyrazinyl or triazinyl; or a tricyclic heteroaryl group, such as, for example, carbazolyl, acridinyl or phenazinyl.

"5-membered aza-heteroaryl" in the context of the invention means an aromatic heterocyclic group (heteroaromatic) having 5 ring atoms in total, which contains at least one ring nitrogen atom and optionally one or two further ring heteroatoms selected from N, O and S, and which is bound via a ring carbon atom or optionally via a ring nitrogen atom (if allowed by valency), in particular a 5-membered aza-heteroaryl containing one ring nitrogen atom and one or two further ring heteroatoms selected from N and O, such as: pyrrolyl, pyrazolyl, imidazolyl, oxazolyl, thiazolyl, isoxazolyl, isothiazolyl, triazolyl, oxadiazolyl, and thiadiazolyl, for example, preferred 5-membered aza-heteroaryls being pyrazolyl, imidazolyl, oxazolyl, thiazolyl, triazolyl, isoxazolyl, and oxadiazolyl. Particularly preferred 5-membered aza-heteroarylss are triazolyl, oxazolyl, isoxazolyl, pyrazolyl, thiazolyl, 1,2,4-oxadiazolyl and 1,3,4-oxadiazolyl.

mono-(C₁-C₄)-alkylamino in the context of the invention means an amino group with one straight-chain or branched alkyl substituent which contains 1, 2, 3 or 4 carbon atoms, such as: methylamino, ethylamino, n-propylamino, isopropylamino, n-butylamino, and *tert*-butylamino, for example.

di-(C₁-C₄)-alkylamino in the context of the invention means an amino group with two identical or different straight-chain or branched alkyl substituents which each contain 1, 2, 3 or 4 carbon atoms, such as: *N,N*-dimethylamino, *N,N*-diethylamino, *N*-ethyl-*N*-methylamino, *N*-methyl-*N-n-*propylamino, *N*-isopropyl-*N*-methylamino, *N*-isopropyl-*N-n*-propylamino, *N*,*N*-diisopropylamino, *N-n*-butyl-*N*-methylamino, and *N*-*tert*-butyl-N-methylamino, for example.

(C₁-C₄)-Alkylcarbonyl in the context of the invention means a straight-chain or branched alkyl group having 1, 2, 3 or 4 carbon atoms which is bound to the rest of the molecule via a carbonyl group [-C(=O)-], such as: acetyl, propionyl, *n*-butyryl, isobutyryl, n-pentanoyl, and pivaloyl, for example.

(C₁-C₄)-alkylsulfonyl in the context of the invention means a group which is bound to the rest of the molecule via a sulfonyl group [-S(=O)₂-] and which has one straight-chain or branched alkyl substituent having 1, 2, 3 or 4 carbon atoms, such as: methylsulfonyl, ethylsulfonyl, n-propylsulfonyl, isopropylsulfonyl, n-butylsulfonyl, and *tert*-butylsulfonyl, for example.

An oxo substituent in the context of the invention means an oxygen atom which is bound to a carbon atom via a double bond.

In general, and unless otherwise mentioned, the heteroaryl or heteroarylene groups include all possible isomeric forms thereof, *e.g.:* tautomers and positional isomers with respect to the point of linkage to the rest of the molecule.Thus, for some illustrative non-restricting examples, the term pyridinyl includes pyridin-2-yl, pyridin-3-yl and pyridin-4-yl; or the term thienyl includes thien-2-yl and thien-3-yl.

The term "C₁-C₄", as used in the present text, e.g., in the context of the definition of "C₁-C₄-alkyl", "C₁-C₄-alkoxy", " or "C₁-C₄-alkylsulfanyl", means an alkyl group having 1 to 4 carbon atoms, *i.e.,* 1, 2, 3, or 4 carbon atoms.

The term "C₁-C₆", as used in the present text, e.g., in the context of the definition of "C₁-C₆-alkyl", means an alkyl group having 1 to 6 carbon atoms, *i.e.,* 1, 2, 3, 4, 5 or 6 carbon atoms.

Further, as used herein, the term "C₃-C₆", as used in the present text, e.g., in the context of the definition of "C₃-C₆-cycloalkyl", means a cycloalkyl group having 3 to 6 carbon atoms, *i.e.,* 3, 4, 5 or 6 carbon atoms.

When a range of values is given, said range encompasses each value and sub-range within said range.

For example:
"C₁-C₄" encompasses C₁, C2, C3, C4, C1-C4, C1-C3, C1-C2, C2-C4, C2-C3, and C₃-C₄;
"C₁-C₃" encompasses C₁, C2, C3, C1-C3, C1-C2, and C2-C3;
"C2-C4" encompasses C2, C3, C4, C2-C4, C2-C3, and C3-C4;
"C3-C6" encompasses C3, C4, C5, C6, C3-C6, C3-C5, C3-C4, C4-C6, C4-C5, and C₅-C₆;

As used herein, the term "leaving group" means an atom or a group of atoms that is displaced in a chemical reaction as stable species taking with it the bonding electrons. Preferred such leaving groups include halide (e.g., fluoride, chloride, bromide or iodide), and sulfonate (e.g., (methylsulfonyl)oxy (mesyl(ate), Ms), [(trifluoromethyl)sulfonyl]oxy (triflyl/(ate), Tf), [(nonafluorobutyl)sulfonyl]oxy (nonaflate, Nf), (phenylsulfonyl)oxy, [(4-methylphenyl)sulfonyl]oxy, [(4-bromophenyl)sulfonyl]oxy, [(4-nitrophenyl)sulfonyl]oxy, [(2-nitrophenyl)sulfonyl]oxy, [(4-isopropylphenyl)sulfonyl]oxy, [(2,4,6-triisopropylphenyl)sulfonyl]oxy, [(2,4,6-trimethylphenyl)sulfonyl]oxy, [(4-*tert*-butylphenyl)sulfonyl]oxy and [(4-methoxyphenyl)sulfonyl]oxy).

It is possible for the compounds of general formula (I) to exist as isotopic variants. The invention therefore includes one or more isotopic variant(s) of the compounds of general formula (I), particularly deuterium-containing compounds of general formula (I).

The term "Isotopic variant" of a compound or a reagent is defined as a compound exhibiting an unnatural proportion of one or more of the isotopes of the atoms that constitute such a compound.

The term "Isotopic variant of the compound of general formula (I)" is defined as a compound of general formula (I) exhibiting an unnatural proportion of one or more of the isotopes of the atoms that constitute such a compound.

The expression "unnatural proportion" means a proportion of such isotope which is higher than its natural abundance. The natural abundances of isotopes to be applied in this context are described in "Isotopic Compositions of the Elements 1997", Pure Appl. Chem., 70(1), 217-235, 1998.

Examples of such isotopes include stable and radioactive isotopes of hydrogen, carbon, nitrogen, oxygen, phosphorus, sulfur, fluorine, chlorine, bromine and iodine, such as ²H (deuterium), ³H (tritium), ¹¹C, ¹³C, ¹⁴C, ¹⁵N, ¹⁷O ¹⁸O ³²P ³³P ³³S, ³⁴S, ³⁵S, ³⁶S, ¹⁸F, ³⁶Cl, ⁸²Br, ¹²³I, ¹²⁴I, ¹²⁵I, ¹²⁹I and ¹³¹I, respectively.

With respect to the treatment and/or prophylaxis of the disorders specified herein, isotopic variant(s) of the compounds of general formula (I) preferably contain elevated levels of deuterium ("deuterium-containing compounds of general formula (I)"). Isotopic variants of the compounds of general formula (I) in which one or more radioactive isotopes, such as ³H or ¹⁴C, are incorporated are useful, e.g., in drug and/or substrate tissue distribution studies. These isotopes are particularly preferred for the ease of their incorporation and detectability. Positron-emitting isotopes such as ¹⁸F or ¹¹C may be incorporated into a compound of general formula (I). These isotopic variants of the compounds of general formula (I) are useful for in vivo imaging applications. Deuterium-containing and ¹³C-containing compounds of general formula (I) can be used in mass spectrometry analyses in the context of preclinical or clinical studies.

Isotopic variants of the compounds of general formula (I) can generally be prepared by methods known to a person skilled in the art, such as those described in the schemes and/or examples herein, e.g., by substituting a reagent for an isotopic variant of said reagent, preferably for a deuterium-containing reagent. Depending on the desired sites of deuteration, in some cases deuterium from D₂O can be incorporated either directly into the compounds or into reagents that are useful for synthesizing such compounds (Esaki et al., Tetrahedron, 2006, 62, 10954; Esaki et al., Chem. Eur. J., 2007, 13, 4052). Deuterium gas is also a useful reagent for incorporating deuterium into molecules. Catalytic deuteration of olefinic bonds (H. J. Leis et al., Curr. Org. Chem., 1998, 2, 131; J. R. Morandi et al., J. Org. Chem., 1969, 34 (6), 1889) and acetylenic bonds (N. H. Khan, J. Am. Chem. Soc., 1952, 74 (12), 3018; S. Chandrasekhar et al., Tetrahedron Letters, 2011, 52, 3865) is a rapid route for incorporation of deuterium. Metal catalysts (e.g., Pd, Pt, and Rh) in the presence of deuterium gas can be used to directly exchange deuterium for hydrogen in functional groups containing hydrocarbons (J. G. Atkinson et al., US Patent 3966781). A variety of deuterated reagents and synthetic building blocks are commercially available from companies such as for example C/D/N Isotopes, Quebec, Canada; Cambridge Isotope Laboratories Inc., Andover, MA, USA; and CombiPhos Catalysts, Inc., Princeton, NJ, USA. Further information on the state of the art with respect to deuterium-hydrogen exchange is given for example in Hanzlik et al., J. Org. Chem. 55, 3992-3997, 1990; R. P. Hanzlik et al., Biochem. Biophys. Res. Commun. 160, 844, 1989; P. J. Reider et al., J. Org. Chem. 52, 3326-3334, 1987; M. Jarman et al., Carcinogenesis 16(4), 683-688, 1995; J. Atzrodt et al., Angew. Chem., Int. Ed. 2007, 46, 7744; K. Matoishi et al., Chem. Commun. 2000, 1519-1520; K. Kassahun et al., WO2012/112363.

The term "deuterium-containing compound of general formula (I)" is defined as a compound of general formula (I), in which one or more hydrogen atom(s) is/are replaced by one or more deuterium atom(s) and in which the abundance of deuterium at each deuterated position of the compound of general formula (I) is higher than the natural abundance of deuterium, which is about 0.015%. Particularly, in a deuterium-containing compound of general formula (I) the abundance of deuterium at each deuterated position of the compound of general formula (I) is higher than 10%, 20%, 30%, 40%, 50%, 60%, 70% or 80%, preferably higher than 90%, 95%, 96% or 97%, even more preferably higher than 98% or 99% at said position(s). It is understood that the abundance of deuterium at each deuterated position is independent of the abundance of deuterium at other deuterated position(s).

The selective incorporation of one or more deuterium atom(s) into a compound of general formula (I) may alter the physicochemical properties (such as for example acidity [C. L. Perrin, et al., J. Am. Chem. Soc., 2007, 129, 4490; A. Streitwieser et al., J. Am. Chem. Soc., 1963, 85, 2759;], basicity [C. L. Perrin et al., J. Am. Chem. Soc., 2005, 127, 9641; C. L. Perrin, et al., J. Am. Chem. Soc., 2003, 125, 15008; C. L. Perrin in Advances in Physical Organic Chemistry, 44, 144], lipophilicity [B. Testa et al., Int. J. Pharm., 1984, 19(3), 271]) and/or the metabolic profile of the molecule and may result in changes in the ratio of parent compound to metabolites or in the amounts of metabolites formed. Such changes may result in certain therapeutic advantages and hence may be preferred in some circumstances. Reduced rates of metabolism and metabolic switching, where the ratio of metabolites is changed, have been reported (A. E. Mutlib et al., Toxicol. Appl. Pharmacol., 2000, 169, 102; D. J. Kushner et al., Can. J. Physiol. Pharmacol., 1999, 77, 79). These changes in the exposure to parent drug and metabolites can have important consequences with respect to the pharmacodynamics, tolerability and efficacy of a deuterium-containing compound of general formula (I). In some cases deuterium substitution reduces or eliminates the formation of an undesired or toxic metabolite and/or enhances the formation of a desired metabolite (e.g., Nevirapine: A. M. Sharma et al., Chem. Res. Toxicol., 2013, 26, 410; Efavirenz: A. E. Mutlib et al., Toxicol. Appl. Pharmacol., 2000, 169, 102). In other cases the major effect of deuteration is to reduce the rate of systemic clearance. As a result, the biological half-life of the compound is increased. The potential clinical benefits would include the ability to maintain similar systemic exposure with decreased peak levels and increased trough levels, i.e., reduced peak-trough variation. This could result in lower side effects and/or enhanced efficacy, depending on the particular compound's pharmacokinetic/ pharmacodynamic relationship. ML-337 (C. J. Wenthur et al., J. Med. Chem., 2013, 56, 5208) and Odanacatib (K. Kassahun et al., WO2012/112363) are examples of this deuterium effect. Still other cases have been reported in which reduced rates of metabolism result in an increase in exposure of the drug without changing the rate of systemic clearance (e.g., Rofecoxib: F. Schneider et al., Arzneim. Forsch. / Drug. Res., 2006, 56, 295; Telaprevir: F. Maltais et al., J. Med. Chem., 2009, 52, 7993). Deuterated drugs showing this effect may have reduced dosing requirements (e.g., lower number of doses or lower dosage to achieve the desired effect) and/or may produce lower metabolite loads.

A compound of general formula (I) may have multiple potential sites of attack for metabolism. To optimize the above-described effects on physicochemical properties and metabolic profile, deuterium-containing compounds of general formula (I) having a certain pattern of one or more deuterium-hydrogen exchange(s) can be selected. Particularly, the deuterium atom(s) of deuterium-containing compound(s) of general formula (I) is/are attached to a carbon atom and/or is/are located at those positions of the compound of general formula (I) which are sites of attack for metabolizing enzymes such as, e.g., cytochrome P₄₅₀.

In certain embodiments, the present invention concerns a deuterium-containing compound of general formula (I) having 1, 2, 3 or 4 deuterium atoms (i.e., 1, 2, 3, or 4 sites where the isotopic balance of hydrogen is enriched for deuterium), preferably with 1, 2 or 3 deuterium atoms.

Where the plural form of the word compounds, salts, polymorphs, hydrates, solvates and the like, is used herein, this is taken to include also a single compound, salt, polymorph, isomer, hydrate, solvate or the like.

By "stable compound' or "stable structure" is meant a compound that is sufficiently robust to survive isolation to a useful degree of purity from a reaction mixture, and is capable of being subjected to further chemical transformation or, preferably, formulation into an efficacious therapeutic agent.

The compounds of the present invention optionally contain one or more asymmetric centres, depending upon the location and nature of the various substituents desired. It is possible that one or more asymmetric carbon atoms are present in the (R) or (S) configuration, which can result in racemic mixtures in the case of a single asymmetric centre, and in diastereomeric mixtures in the case of multiple asymmetric centres. In certain instances, it is possible that asymmetry also be present due to restricted rotation about a given bond, for example, the central bond adjoining two substituted aromatic rings of the specified compounds (i.e., atropisomers).

Preferred compounds are those which produce the more desirable biological activity. Separated, pure or partially purified isomers and stereoisomers or racemic or diastereomeric mixtures of the compounds of the present invention are also included within the scope of the present invention. The purification and the separation of such materials can be accomplished by standard techniques known in the art.

Separated optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e.g.*, HPLC columns using a chiral phase), with or without conventional derivatisation, optimally chosen to maximise the separation of the enantiomers. Suitable HPLC columns using a chiral phase are commercially available, such as those manufactured by Daicel, *e.g.,* Chiracel OD and Chiracel OJ, for example, among many others, which are all routinely selectable. Enzymatic separations, with or without derivatisation, are also useful. The optically active compounds of the present invention can likewise be obtained by chiral syntheses utilizing optically active starting materials or optically active catalysts.

In order to distinguish different types of isomers from each other, reference is made to IUPAC Rules Section E (Pure Appl Chem 45, 11-30, 1976).

The present invention includes all possible stereoisomers of the compounds of the present invention as single stereoisomers, or as any mixture of said stereoisomers, *e.g.,* (R)- or (S)-isomers, in any ratio. Isolation of a single stereoisomer, *e.g.,* a single enantiomer or a single diastereomer, of a compound of the present invention is achieved by any suitable state of the art method, such as chromatography, especially chiral chromatography, for example.

Further, it is possible for the compounds of the present invention to exist as tautomers. For example, a compound of the present invention which contains an imidazopyridine moiety as a heteroaryl group for example can exist as a 1H tautomer, or a 3H tautomer, or even a mixture in any amount of the two tautomers, namely:

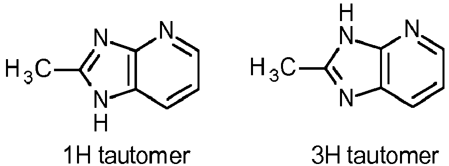

Moreover, in the course of the synthesis of a 1 H-pyrazole group, the 1 H-pyrazol-3-yl tautomer as well as the 1 H-pyrazol-5-yl tautomer are formed.

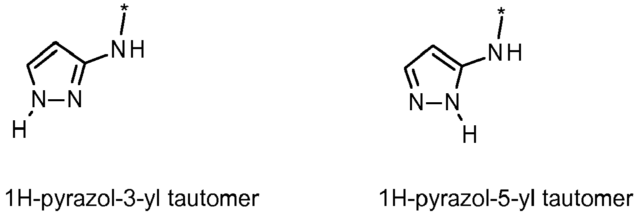

The present invention includes all possible tautomers of the compounds of the present invention as single tautomers, or as any mixture of said tautomers, in any ratio.

The present invention also covers useful forms of the compounds of the present invention, such as metabolites, hydrates, solvates, prodrugs, salts, in particular pharmaceutically acceptable salts, and/or co-precipitates.

The compounds of the present invention can exist as a hydrate, or as a solvate, wherein the compounds of the present invention contain polar solvents, preferably water, methanol or ethanol, for example, as a structural element of the crystal lattice of the compounds. It is possible for the amount of polar solvents, in particular water, to exist in a stoichiometric or non-stoichiometric ratio. In the case of stoichiometric solvates, *e.g.,* a hydrate, hemi-, (semi-), mono-, sesqui-, di-, tri-, tetra-, penta- *etc.* solvates or hydrates, respectively, are possible. The present invention includes all such hydrates or solvates.

Further, it is possible for the compounds of the present invention to exist in free form, e.g., as a free base, or as a free acid, or as a zwitterion, or to exist in the form of a salt, preferably as a free acid. Said salt may be any salt, either an organic or inorganic addition salt, particularly any pharmaceutically acceptable organic or inorganic addition salt, which is customarily used in pharmacy, or which is used, for example, for isolating or purifying the compounds of the present invention.

The term "pharmaceutically acceptable salt" refers to an inorganic or organic acid addition salt of a compound of the present invention. For example, see S. M. Berge, et al. "Pharmaceutical Salts," J. Pharm. Sci. 1977, 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, or "mineral acid", such as hydrochloric, hydrobromic, hydroiodic, sulfuric, sulfamic, bisulfuric, phosphoric, or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, 3-phenylpropionic, pivalic, 2-hydroxyethanesulfonic, itaconic, trifluoromethanesulfonic, dodecylsulfuric, ethanesulfonic, benzenesulfonic, para-toluenesulfonic, methanesulfonic, 2-naphthalenesulfonic, naphthalinedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium, magnesium or strontium salt, or an aluminium or a zinc salt, or an ammonium salt derived from ammonia or from an organic primary, secondary or tertiary amine having 1 to 20 carbon atoms, such as ethylamine, diethylamine, triethylamine, ethyldiisopropylamine, monoethanolamine, diethanolamine, triethanolamine, dicyclohexylamine, dimethylaminoethanol, diethylaminoethanol, tris(hydroxymethyl)aminomethane, procaine, dibenzylamine, *N*-methylmorpholine, arginine, lysine, 1,2-ethylenediamine, *N*-methylpiperidine, *N*-methyl-glucamine, *N,N*-dimethyl-glucamine, *N*-ethyl-glucamine, 1,6-hexanediamine, glucosamine, sarcosine, serinol, 2-amino-1,3-propanediol, 3-amino-1,2-propanediol, 4-amino-1,2,3-butanetriol, or a salt with a quarternary ammonium ion having 1 to 20 carbon atoms, such as tetramethylammonium, tetraethylammonium, tetra(*n*-propyl)ammonium, tetra(*n*-butyl)ammonium, *N*-benzyl-*N,N,N-*trimethylammonium, choline or benzalkonium.

In accordance with preferred embodiments of the first aspect, the present invention covers a pharmaceutically acceptable salt of compounds of general formula (I), , *supra,* which is an alkali metal salt, in particular a sodium or potassium salt, or an ammonium salt derived from an organic tertiary amine, in particular choline.

Those skilled in the art will further recognise that it is possible for acid addition salts of the claimed compounds to be prepared by reaction of the compounds with the appropriate inorganic or organic acid via any of a number of known methods. Alternatively, alkali and alkaline earth metal salts of acidic compounds of the present invention are prepared by reacting the compounds of the present invention with the appropriate base via a variety of known methods.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

Unless specified otherwise, suffixes to chemical names or structural formulae relating to salts, such as "hydrochloride", "trifluoroacetate", "sodium salt", or "x HCl", "x CF₃COOH", "x Na⁺", for example, mean a salt form, the stoichiometry of which salt form not being specified.

This applies analogously to cases in which synthesis intermediates or example compounds or salts thereof have been obtained, by the preparation and/or purification processes described, as solvates, such as hydrates, with (if defined) unknown stoichiometric composition.

As used herein, the term *"in vivo* hydrolysable ester" means an *in vivo* hydrolysable ester of a compound of the present invention containing a carboxy or hydroxy group, for example, a pharmaceutically acceptable ester which is hydrolysed in the human or animal body to produce the parent acid or alcohol. Suitable pharmaceutically acceptable esters for carboxy include for example alkyl, cycloalkyl and optionally substituted phenylalkyl, in particular benzyl esters, C₁-C₆ alkoxymethyl esters, *e.g.,* methoxymethyl, C₁-C₆ alkanoyloxymethyl esters, *e.g.,* pivaloyloxymethyl, phthalidyl esters, C₃-C₈ cycloalkoxy-carbonyloxy-C₁-C₆ alkyl esters, *e.g.* 1-cyclohexylcarbonyloxyethyl; 1,3-dioxolen-2-onylmethyl esters, *e.g.,* 5-methyl-1,3-dioxolen-2-onylmethyl; and C₁-C₆-alkoxycarbonyloxyethyl esters, *e.g.,* 1-methoxycarbonyloxyethyl, it being possible for said esters to be formed at any carboxy group in the compounds of the present invention.

An *in vivo* hydrolysable ester of a compound of the present invention containing a hydroxy group includes inorganic esters such as phosphate esters and [alpha]-acyloxyalkyl ethers and related compounds which as a result of the *in vivo* hydrolysis of the ester breakdown to give the parent hydroxy group. Examples of [alpha]-acyloxyalkyl ethers include acetoxymethoxy and 2,2-dimethylpropionyloxymethoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. The present invention covers all such esters.

Furthermore, the present invention includes all possible crystalline forms, or polymorphs, of the compounds of the present invention, either as single polymorph, or as a mixture of more than one polymorph, in any ratio.

Moreover, the present invention also includes prodrugs of the compounds according to the invention. The term "prodrugs" here designates compounds which themselves can be biologically active or inactive, but are converted (for example metabolically or hydrolytically) into compounds according to the invention during their residence time in the body.Typical examples of prodrugs would be e.g.esters.

Preference is given to compounds of formula (I) in which
- R¹: represents a group selected from hydrogen, (C₁-C₆)-alkyl, (C₃-C₆)-cycloalkyl, 5- to 6-membered heterocycloalykl, 5- to 6-membered heteroaryl and phenyl,
wherein said (C₁-C₆)-alkyl, (C₃-C₆)-cycloalkyl, 5- to 6-membered heterocycloalkyl, 5- to 6-membered heteroaryl and phenyl are optionally substituted with one or two groups independently selected from cyano, halogen, amino, hydroxy, oxo, (C₁-C₃)-alkyl, (C₁-C₄)-alkoxy, (C₁-C₄)-alkylcarbonyl, mono-(C₁-C₄)-alkylamino, di-(C₁-C₄)-alkylamino, (C₁-C₄)-alkylsulfonyl, (C₃-C₆)-cycloalkyl ,
wherein each said (C₁-C₃)-alkyl, (C₃-C₆)-cycloalkyl and (C₁-C₄)-alkoxy is optionally substituted with up to 5 fluorine atoms,
- R²: represents a group selected from hydrogen or (C₁-C₄)-alkyl,
wherein said (C₁-C₄)-alkyl is optionally substituted with up to five halogen atoms,
- A: represents a group selected from 5-membered aza-heteroaryl,
wherein said 5-membered aza-heteroaryl is optionally substituted with one, two or three groups independently selected from halogen, cyano, hydroxy, methylsulfonyl, (C₃-C₆)-cycloalkyl, (C₁-C₄)-alkyl and (C₁-C₄)-alkoxy,
wherein each said (C₁-C₄)-alkyl and (C₁-C₄)-alkoxy is optionally substituted with up to three fluorine atoms,
- Z: represents a group selected from Phenyl and 5- to 6-membered heteroaryl,
optionally substituted with one, two or three groups independently selected from halogen, cyano, hydroxy, (C₁-C₄)-alkylsulfonyl, (C₃-C₆)-cycloalkyl, (C₁-C₄)-alkyl and (C₁-C₄)-alkoxy,
wherein each said (C₁-C₄)-alkyl and (C₁-C₄)-alkoxy is optionally substituted with (C₃-C₆)-cycloalkyl and optionally substituted with up to five fluorine atoms,
and pharmaceutically acceptable salts thereof, solvates thereof and the solvates of the salts thereof.

Preference is further given to compounds of formula (I) in which
- R¹: represents a group selected from hydrogen, (C₁-C₆)-alkyl, (C₃-C₆)-cycloalkyl, 5- to 6-membered heterocycloalkyl, 5- to 6-membered heteroaryl and phenyl
wherein said (C₁-C₆)-alkyl (C₃-C₆)-cycloalkyl, 5- to 6-membered heterocycloalkyl, 5- to 6-membered heteroaryl and phenyl are optionally substituted with one or two groups independently selected from cyano, halogen, amino, hydroxy, oxo, (C₁-C₃)-alkyl, (C₁-C₄)-alkoxy, (C₁-C₄)-alkylcarbonyl, mono-(C₁-C₄)-alkylamino, di-(C₁-C₄)-alkylamino, (C₁-C₄)-alkylsulfonyl, (C₃-C₆)-cycloalkyl, ,
wherein each said (C₁-C₃)-alkyl, (C₃-C₆)-cycloalkyl and (C₁-C₄)-alkoxy is optionally substituted with up to 5 fluorine atoms,
- R²: represents a group selected from hydrogen or methyl
- A: represents a group selected from triazolyl, oxazolyl, isoxazolyl, pyrazolyl, thiazolyl, 1,2,4-oxadiazolyl and 1,3,4-oxadiazolyl
optionally substituted with one, two or three groups independently selected from halogen, cyano, hydroxy, methylsulfonyl, (C₃-C₆)-cycloalkyl, (C₁-C₄)-alkyl, and (C₁-C₄)-alkoxy,
wherein each said (C₁-C₄)-alkyl, (C₁-C₄)-alkoxy is optionally substituted with up to three fluorine atoms,
- Z: represents a group selected from Phenyl and 5 to 6 membered heteroaryl,
optionally substituted, with one, two or three groups independently selected from halogen, cyano, hydroxy, methylsulfonyl, (C₃-C₆)-cycloalkyl, (C₁-C₄)-alkyl, (C₁-C₄)-alkoxy,
wherein each said (C₁-C₄)-alkyl and (C₁-C₄)-alkoxy is optionally substituted with, (C₃-C₆)-cycloalkyl and optionally substituted with up to five fluorine atoms,
and pharmaceutically acceptable salts thereof, solvates thereof and the solvates of the salts thereof.

Preference is further given to compounds of the formula (I) in which
- R¹: represents a group selected from hydrogen, (C₁-C₆)-alkyl, (C₃-C₆)-cycloalkyl, 5- to 6-membered heterocycloalkyl, 5- to 6-membered heteroaryl and phenyl
wherein said (C₁-C₆)-alkyl (C₃-C₆)-cycloalkyl, 5- to 6-membered heterocycloalkyl, 5- to 6-membered heteroaryl and phenyl are optionally substituted, with one or two groups independently selected from cyano, halogen, amino, hydroxy, oxo, C₁-C₃-alkyl, (C₁-C₄)-alkoxy, (C₁-C₄)-alkylcarbonyl, mono-(C₁-C₄)-alkylamino, di-(C₁-C₄)-alkylamino, (C₁-C₄)-alkylsulfonyl, (C₃-C₆)-cycloalkyl,
wherein each said C₁-C₃-alkyl, (C₃-C₆)-cycloalkyl, (C₁-C₄)-alkoxy is optionally substituted with up to 5 fluorine atoms
- R²: represents a group selected from hydrogen or methyl
- A: represents a group selected from triazolyl, oxazolyl, isoxazolyl, pyrazolyl, thiazolyl, 1,2,4-oxadiazolyl and 1,3,4-oxadiazolyl
optionally substituted with one, two or three groups independently selected from halogen, cyano, hydroxy, methylsulfonyl, (C₃-C₆)-cycloalkyl, (C₁-C₄)-alkyl and (C₁-C₄)-alkoxy
wherein each said (C₁-C₄)-alkyl, (C₁-C₄)-alkoxy is optionally substituted with up to three fluorine atoms,
with the provisio that for A representing thiazolyl or pyrazolyl R¹ will not represent methylimidazol
- Z: represents a group selected from Phenyl and 5- to 6- membered heteroaryl,
optionally substituted with one, two or three groups independently selected from halogen, cyano, hydroxy, methylsulfonyl, (C₃-C₆)-cycloalkyl, (C₁-C₄)-alkyl, and (C₁-C₄)-alkoxy,
wherein each said (C₁-C₄)-alkyl and (C₁-C₄)-alkoxy is optionally substituted with (C₃-C₆)-cycloalkyl and optionally substituted with up to five fluorine atoms,
and pharmaceutically acceptable salts thereof, solvates thereof and the solvates of the salts thereof.

Preference is further given to compounds of the formula (I) in which
- R¹: represents a group selected from hydrogen, (C₁-C₆)-alkyl, (C₃-C₆)-cycloalkyl, 5- to 6-membered heterocycloalkyl, 5- to 6-membered heteroaryl and phenyl
wherein said (C₁-C₆)-alkyl (C₃-C₆)-cycloalkyl, 5- to 6-membered heterocycloalkyl, 5- to 6-membered heteroaryl and phenyl are optionally substituted, with one or two groups independently selected from cyano, halogen, amino, hydroxy, oxo, C₁-C₃-alkyl, (C₁-C₄)-alkoxy, (C₁-C₄)-alkylcarbonylmono-(C₁-C₄)-alkylamino, di-(C₁-C₄)-alkylamino, (C₁-C₄)-alkylsulfonyl, (C₃-C₆)-cycloalkyl, ,
wherein each said (C₁-C₃)-alkyl, (C₃-C₆)-cycloalkyl, and (C₁-C₄)-alkoxy is optionally substituted with up to 5 fluorine atoms,
- R²: represents a group selected from hydrogen or methyl,
- A: represents a triazolyl
optionally substituted with one, two or three groups independently selected from halogen, cyano, hydroxy, methylsulfonyl, (C₃-C₆)-cycloalkyl, (C₁-C₄)-alkyl, and (C₁-C₄)-alkoxy,
wherein each said (C₁-C₄)-alkyl and (C₁-C₄)-alkoxy is optionally substituted with up to three fluorine atoms,
- Z: represents a group selected from Phenyl and 5 to 6 membered heteroaryl,
optionally substituted, with one, two or three groups independently selected from halogen, cyano, hydroxy, methylsulfonyl, (C₃-C₆)-cycloalkyl, (C₁-C₄)-alkyl and (C₁-C₄)-alkoxy,
wherein each said (C₁-C₄)-alkyl and (C₁-C₄)-alkoxy is optionally substituted with (C₃-C₆)-cycloalkyl and optionally substituted with up to five fluorine atoms,
and pharmaceutically acceptable salts thereof, solvates thereof and the solvates of the salts thereof.

Preference is given to compounds of the formula (I) selected from the group consisting of
N-{[(4R)-4-cyclopropyl-2,5-dioxoimidazolidin-4-yl]methyl}-2-(2,4-difluorophenyl)-2H-1,2,3-triazole-4-carboxamide 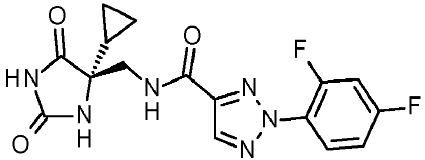
N-{[(4R)-4-cyclopropyl-2,5-dioxoimidazolidin-4-yl]methyl}-2-(4-fluorophenyl)-2H-1,2,3-triazole-4-carboxamide 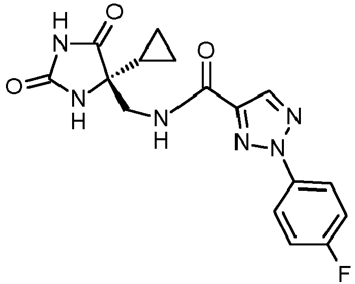
2-(3-chloro-4-fluorophenyl)-N-{[(4R)-4-cyclopropyl-2,5-dioxoimidazolidin-4-yl]methyl}-2H-1,2,3-triazole-4-carboxamide 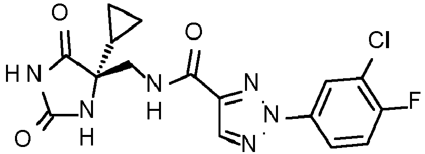
*ent*-N-[(2,5-dioxo-4-(propan-2-yl)imidazolidin-4-yl)methyl]-2-(4-fluorophenyl)-2H-1,2,3-triazole-4-carboxamide 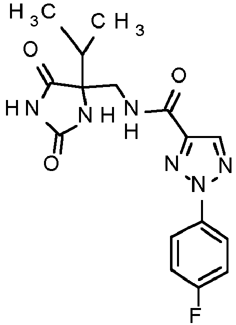
*ent*-N-[(4-cyclobutyl-2,5-dioxoimidazolidin-4-yl)methyl]-2-(4-fluorophenyl)-2H-1,2,3-triazole-4-carboxamide 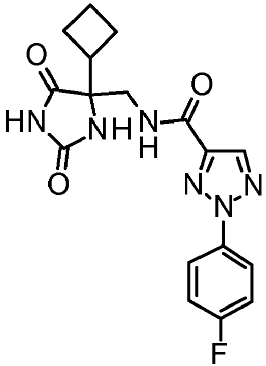
*ent*-N-[(4-cyclobutyl-2,5-dioxoimidazolidin-4-yl)methyl]-2-(3,4,5-trifluorophenyl)-2H-1,2,3-triazole-4-carboxamide 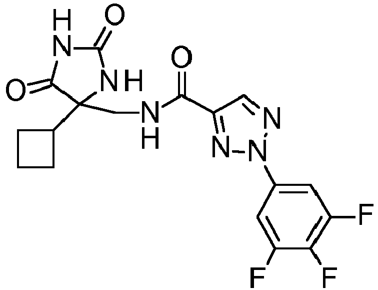
*ent*-N-[(4-cyclobutyl-2,5-dioxoimidazolidin-4-yl)methyl]-2-(3-fluorophenyl)-2H-1,2,3-triazole-4-carboxamide 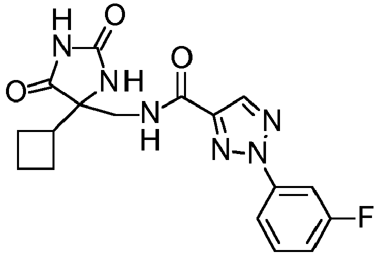 and
*ent*-N-[(4-cyclobutyl-2,5-dioxoimidazolidin-4-yl)methyl]-2-(3,4-difluorophenyl)-2H-1,2,3-triazole-4-carboxamide 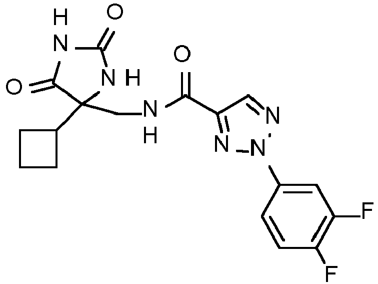 and pharmaceutically acceptable salts thereof, solvates thereof and the solvates of the salts thereof.

Preference is also given to compounds of formula (I) in which R¹ represents cyclopropyl, cyclobutyl, isopropyl or methylimidazole.

Particular preference is also given to compounds of formula (I) in which R¹ represents cyclopropyl.

Preference is also given to compounds of formula (I) in which R² represents hydrogen or methyl.

Particular preference is also given to compounds of formula (I) in which R² represents hydrogen.

Preference is also given to compounds of formula (I) in which A represents a triazole, an isoxazole, an oxazole, a pyrazole or an oxadiazole.

Particular preference is also given to compounds of formula (I) in which A represents a triazole.

Preference is also given to compounds of formula (I) in which Z represents phenyl optionally substituted with one, two or three groups independently selected from halogen, cyano, hydroxy, (C₁-C₄)-alkoxy, trifluoromethoxy and difluoromethoxy.

Particular preference is given to compounds of formula (I) in which Z represents phenyl substituted with 1 to 3 halogens, preferably independently selected from chlorine or fluorine.

Particular preference is also given to compounds of formula (I) in which Z represents p-fluorophenyl.

Another object of the present invention is a process for the preparation of the compounds of formula (I) characterized by reacting a compound of formula (II) 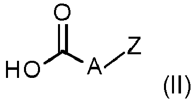 in which A and Z have the meaning given above, in an inert solvent with a condensing agent such as 1-(3-dimethylaminopropyl)-3-ethylcarbodiimide hydrochloride in the presence of a base such as N,N-diisopropylethylamine and an auxiliary reagent such as HOBt with a compound of formula (III) which may be used as salt (e.g., hydrochloride) 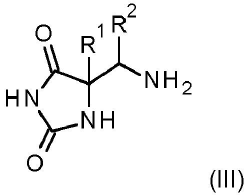 in which R¹ and R² have the meaning given above.

Inert solvents for process step (II) + (III) → (I) include, for example, halogenated hydrocarbons, such as dichloromethane, trichlorethylene, chloroform or chlorobenzene, ethers, such as diethyl ether, dioxane, tetrahydrofuran, 1,2-dimethoxyethane or diglyme, hydrocarbons such as benzene, toluene, xylene, hexane, cyclohexane or petroleum fractions or other solvents such as acetonitrile, N,N-dimethylformamide, N,N-dimethylacetamide, dimethyl sulfoxide, N,N-dimethylpropyleneurea (DMPU), N-methyl-2-pyrrolidone (NMP) or pyridine. It is also possible to use mixtures of the said solvents. Preference is given to using dichloromethane or N,N-dimethylformamide. Dichloromethane is particularly preferably used.

Suitable condensing agents for amide formation in process step (II) + (III) → (I) include, for example, carbodiimides such as N,N'-diethyl, N,N'-dipropyl, N,N'-diisopropyl, N,N'-dicyclohexylcarbodiimide (DCC) or N-(3-dimethylamino-propyl)-N'-ethylcarbodiimide hydrochloride (EDC), phosgene derivatives such as N,N'-carbonyldiimidazole (CDI), 1,2-oxazolium compounds such as 2-ethyl-5-phenyl-1,2-oxazolium-3-sulfate or 2-tert-butyl-5-methyl-isoxazolinium perchlorate, acylamino compounds such as 2-ethoxy-1-ethoxy-carbonyl-1,2-dihydrohydroquinoline, or isobutyl chloroformate, propanephosphonic acid anhydride (T3P), 1-chloro-N,N,2-trimethylprop-1-en-1-amine, cyanophosphonic acid diethyl ester, (2-oxo-3-oxazolidinyl)-phosphoryl chloride, benzotriazazol-1-yloxy-tris (dimethylamino) phosphonium-hexafluorophosphate, benzotriazol-1-yl-oxytripyrrolidinophosphonium hexafluorophosphate (PyBOP), 2-(1H-Benzotriazole-1-yl)-1,1,3,3-tetramethylaminium tetrafluoroborate (TBTU), 2-(1H-benzotriazol-1-yl)-1,1,3,3-tetramethyluronium hexafluorophosphate (HBTU), 0-(1,2-Dihydro-2-oxo-1-pyridyl)-N,N,N'-N'-tetramethyluronium tetrafluoroborate (TPTU), 1-[Bis(dimethylamino)methylene]-1H-1,2,3-triazolo[4,5-b]pyridinium 3-oxid hexafluorophosphate (HATU) or O-(6-Chloro-1-hydrocibenzotriazol-1-yl)-1,1,3,3-tetramethyluronium tetrafluoroborate (TCTU), if appropriate in combination with other excipients such as 1 hydroxybenzotriazole (HOBt), 1-hydroxy-7-azabenzotriazole (HOAt) or N-hydroxysuccinimide (HOSu). Preferably EDC and HATU is used. Particular preference is given to using EDC.

Suitable bases for amide formation in process step (II) + (III) → (I) include, for example, alkali metal carbonates, e.g., sodium or potassium carbonate or hyrogen carbonate, or organic bases such as trialkylamines, e.g., triethylamine (TEA), trimethylamine, N methylmorpholine, N-methylpiperidine or N,N-diisopropylethylamine (DIPEA) or 4-(dimethylamino) pyridine (DMAP). Preferably, DIPEA, trimethylamine or TEA is used. Particular preference is given to using DIPEA.

The condensation (II) + (III) → (I) is generally carried out in a temperature range from -20°C to +100°C, preferably at 0°C to +60°C. The reaction can be carried out at normal, elevated or reduced pressure (for example from 0.5 to 5 bar). In general, one works at room temperature and normal pressure.

Alternatively, the carboxylic acid of the formula (II) can also first be converted into the corresponding carboxylic acid chloride and then reacted directly or in a separate reaction with a compound of the formula (III) to give the compounds according to the invention. The formation of carboxylic acid chlorides from carboxylic acids is carried out by the methods known to those skilled in the art, for example, by treating (II) or the corresponding carboxylate with thionyl chloride, sulfuryl chloride or oxalyl chloride in the presence of a suitable base, for example in the presence of pyridine, and optionally with the addition of dimethylformamide, optionally in a suitable inert solvent.

The compounds used are commercially available, known from the literature or can be prepared analogously to processes known from the literature.

The general procedure is exemplified by the scheme below (Scheme 1) 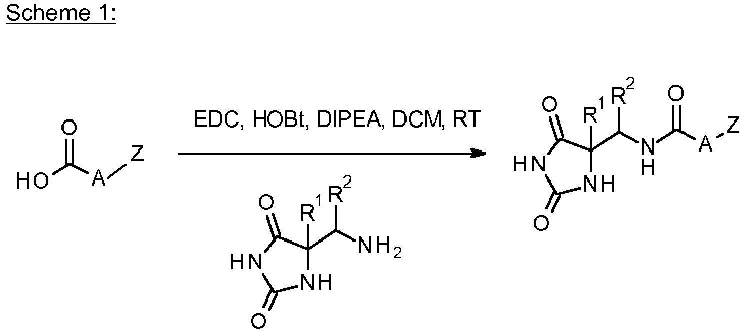 wherein A, Z, R¹ and R² are as defined above.

Amines of the formula (III) can be prepared by the process exemplified in Scheme 2 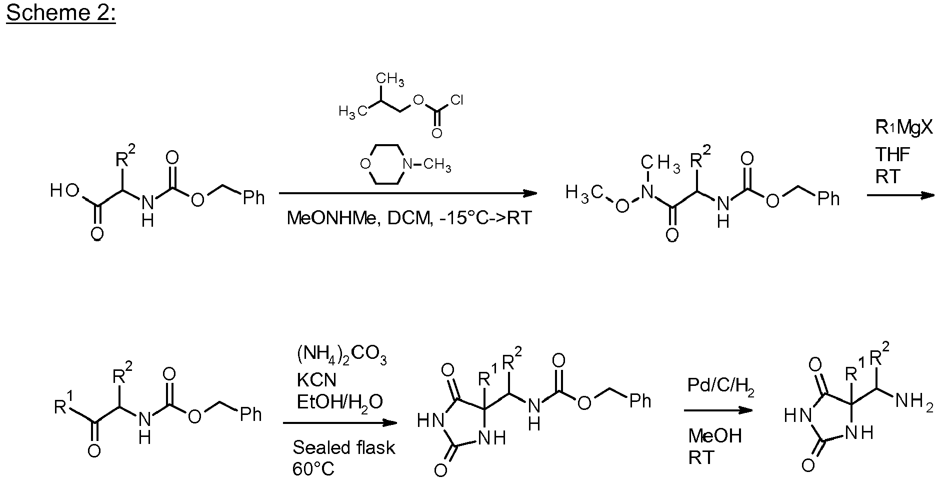
wherein R¹ is 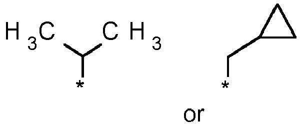
R² is as defined above
X is Cl or Br
and * represents the point of attachment.

An alternative process variant is shown in Scheme 3 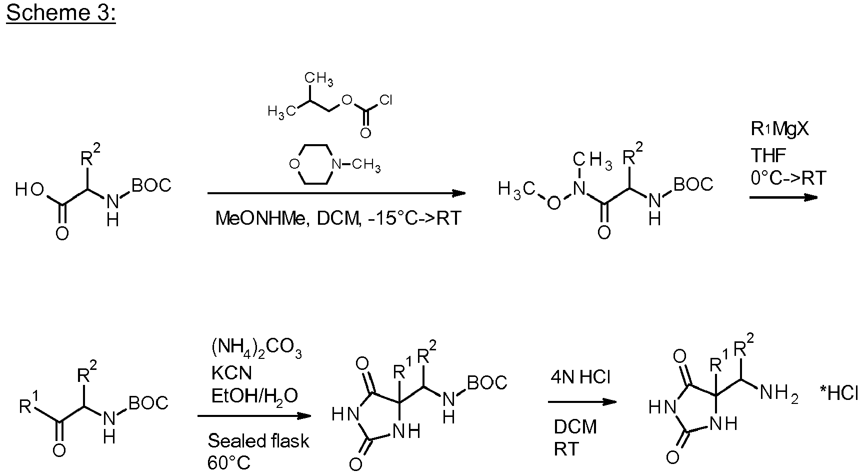
wherein R¹ is 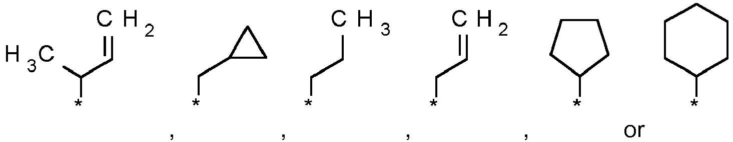
R² is as defined above
X is Cl or Br
and * represents the point of attachment.

Another alternative process variant is shown in Scheme 4 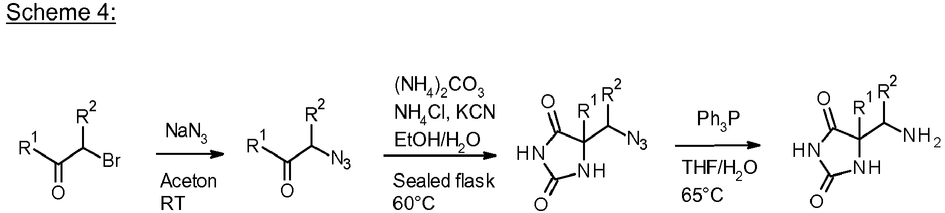
wherein R¹ is 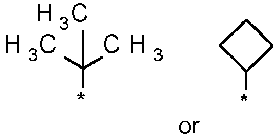
R² is as defined above
and * represents the point of attachment.

Another alternative process variant is shown in Scheme 5 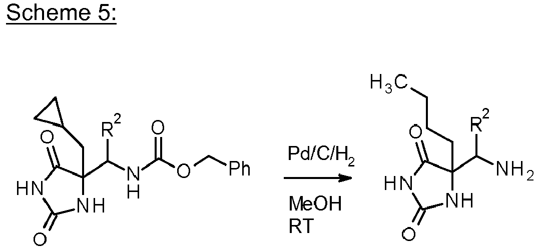 wherein R² is as defined above.

Another alternative process variant is shown in Scheme 8 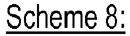 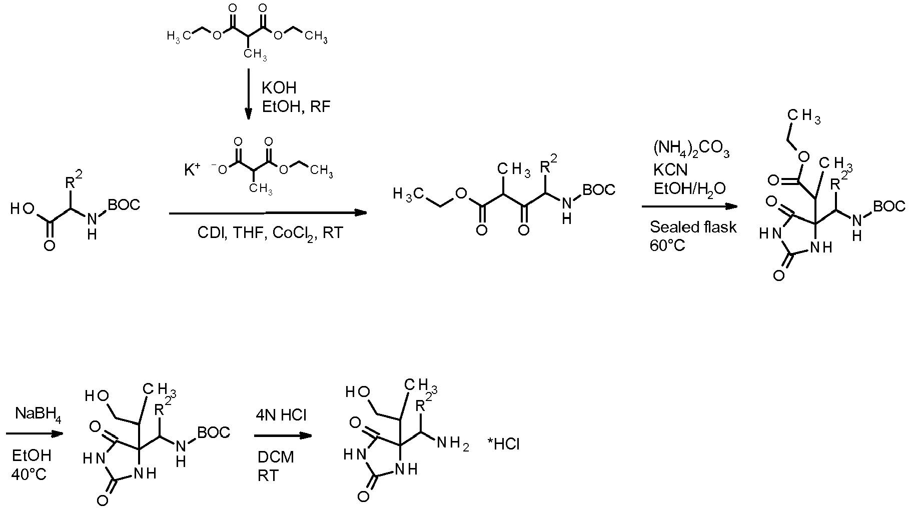 wherein R² is as defined above.

Another alternative process variant is shown in Scheme 9 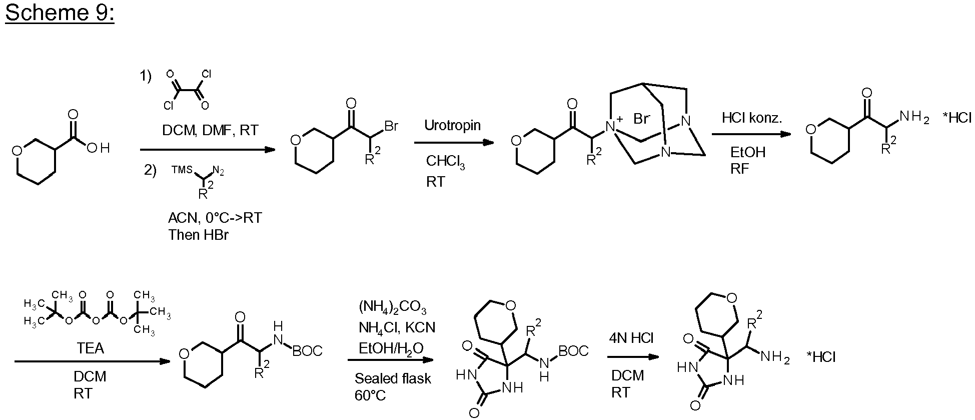 wherein R² is as defined above.

Another alternative process variant is shown in Scheme 10 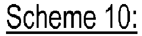 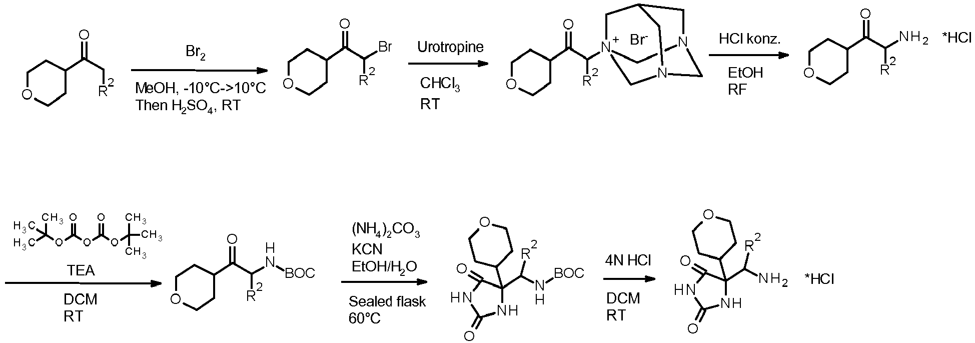 wherein R² is as defined above.

Another alternative process variant is shown in Scheme 11 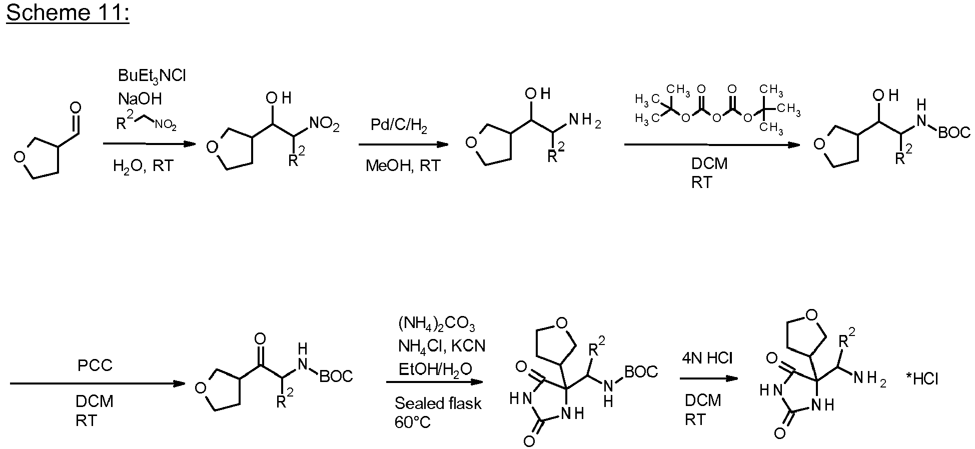 wherein R² is as defined above.

Another alternative process variant is shown in Scheme 12 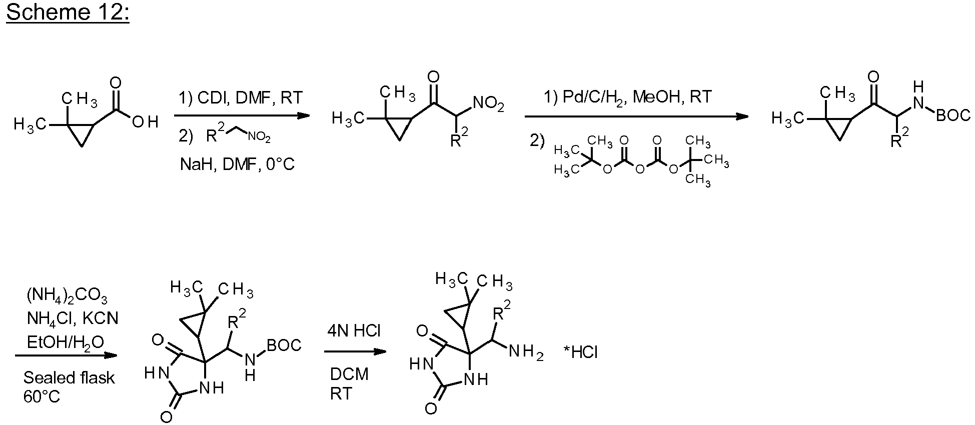 wherein R² is as defined above.

Another alternative process variant is shown in Scheme 13 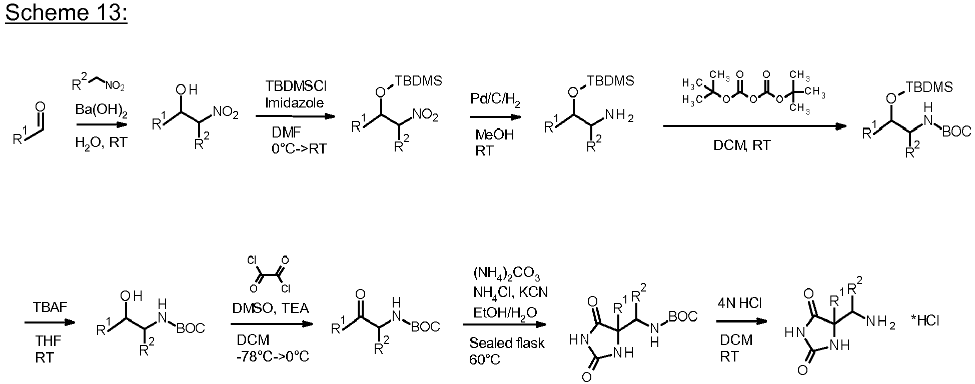
wherein R¹ is 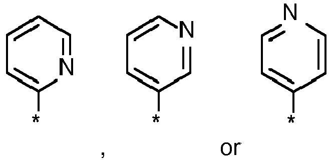
R² is as defined above
and * represents the point of attachment.

Another alternative process variant is shown in Scheme 14 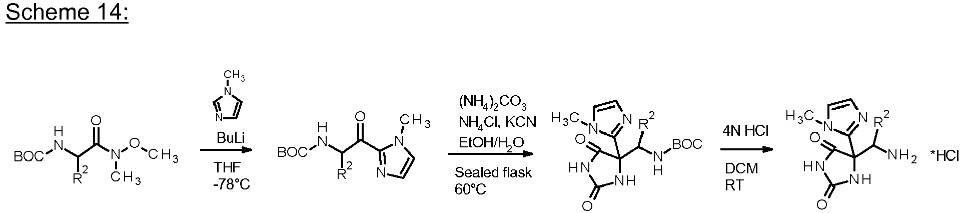 wherein R² is as defined above.

Another alternative process variant is shown in Scheme 15 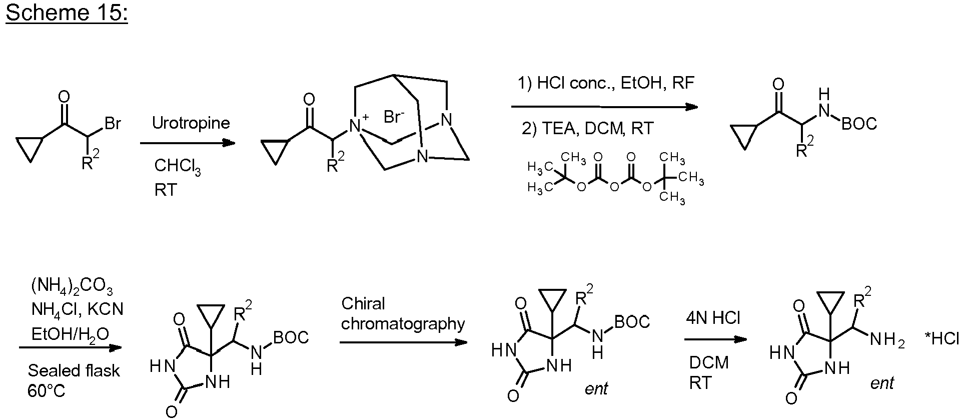 wherein R² is as defined above.

Another alternative process variant is shown in Scheme 16.

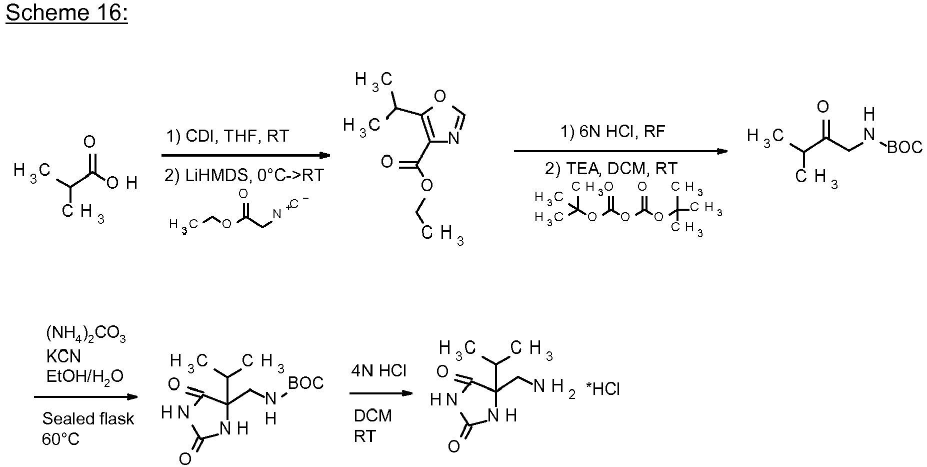

Another alternative process variant is shown in Scheme 17 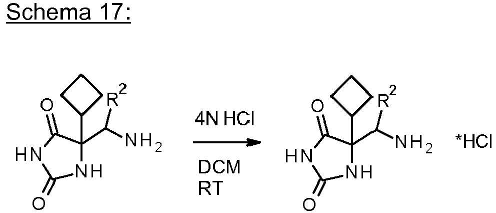 wherein R² is as defined above.

Another alternative process variant is shown in Scheme 18 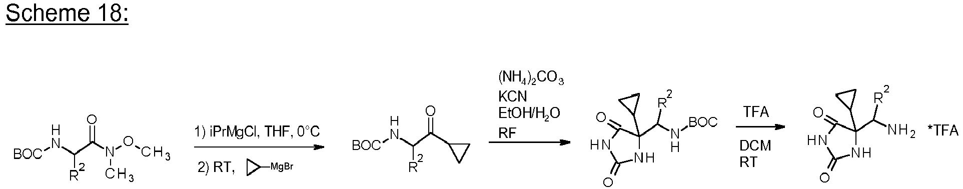 wherein R² is as defined above.

Acids of formula (II) can be prepared by the method exemplified in Scheme 19 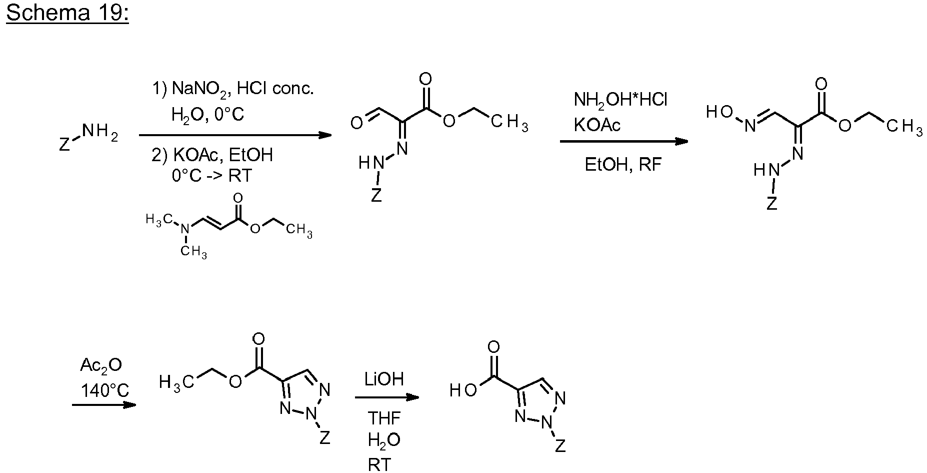
wherein Z is 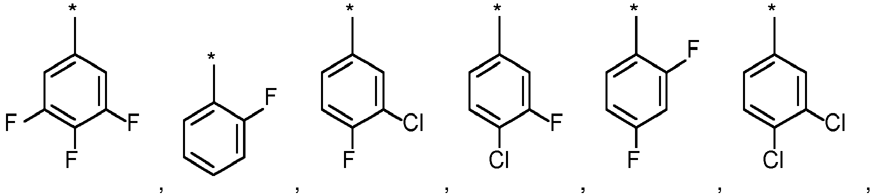 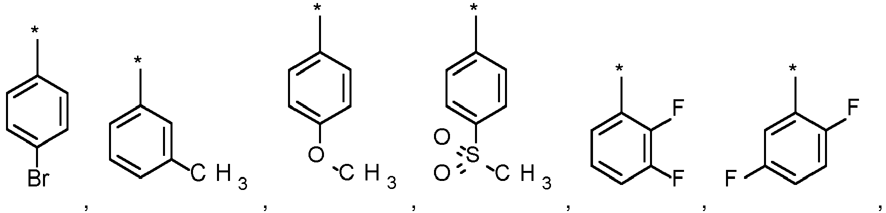 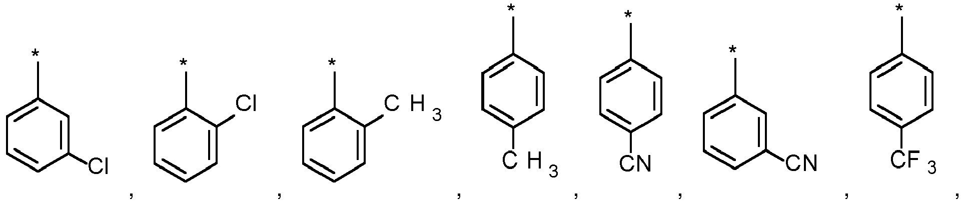 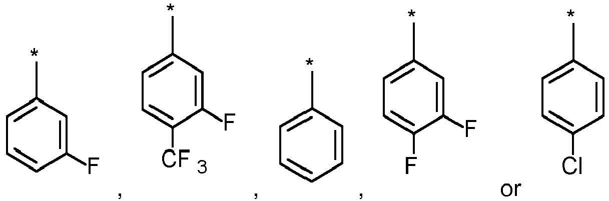
and * represents the point of attachment.

An alternative process variant is shown in Scheme 20.

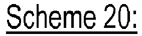

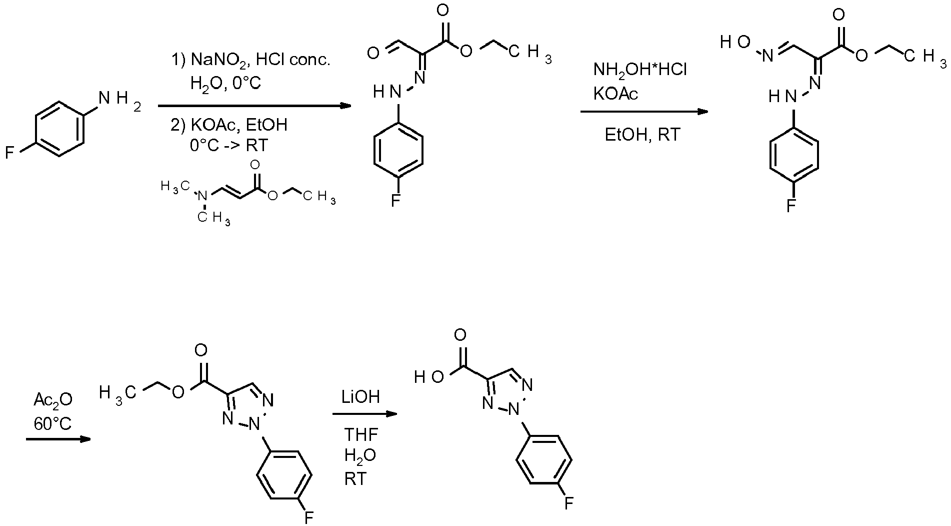

Another alternative process variant is shown in Scheme 21 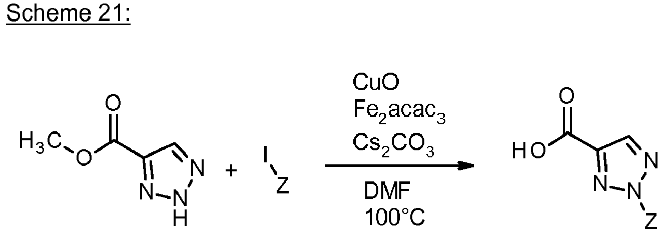
wherein Z is 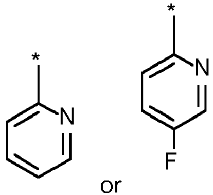
and * represents the point of attachment.

Another alternative process variant is shown in Scheme 22.

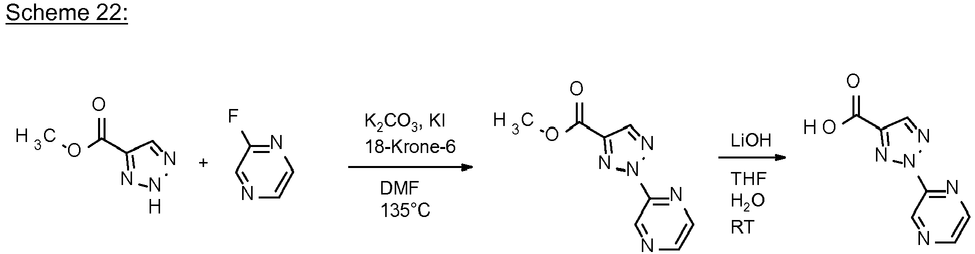

Another alternative process variant is shown in Scheme 23.

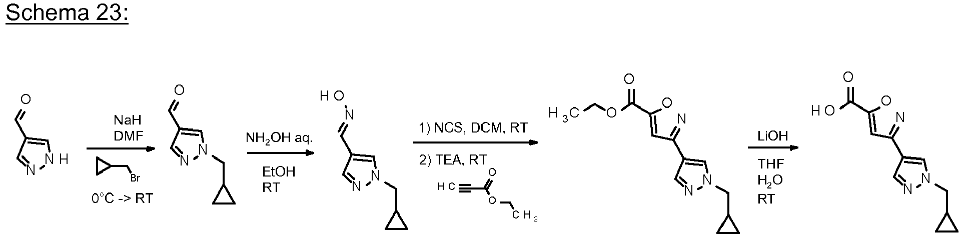

Another alternative process variant is shown in Scheme 24.

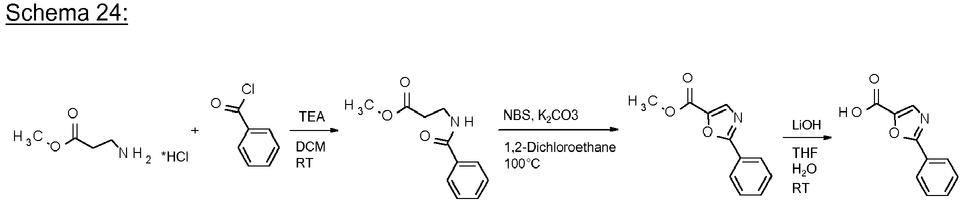

Another alternative process variant is shown in Scheme 25.

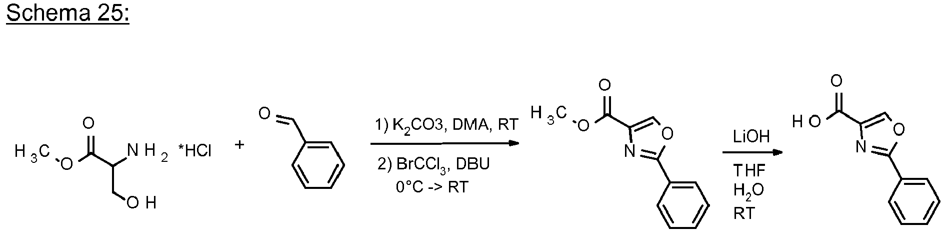

Another alternative process variant is shown in Scheme 26.

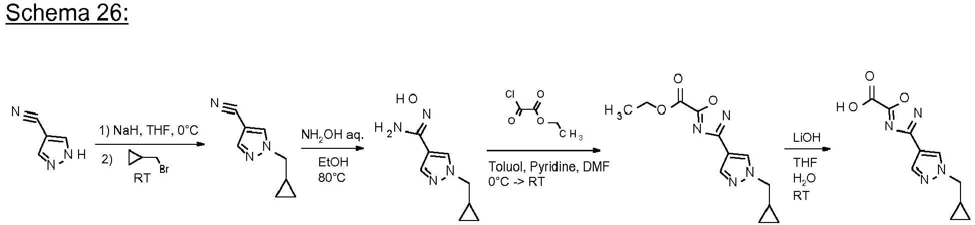

Another alternative process variant is shown in Scheme 27 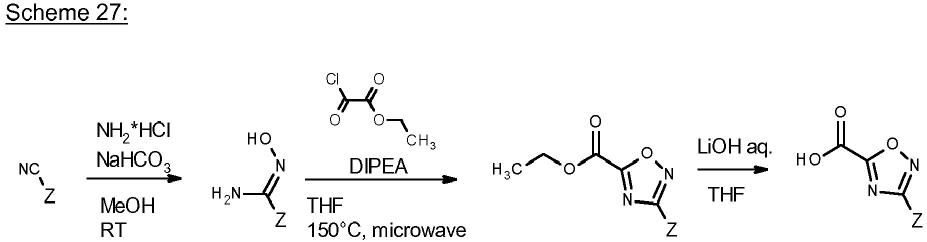
,wherein Z is 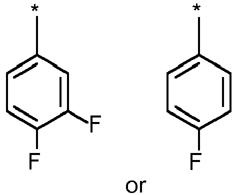
and * represents the point of attachment.

Detailed instructions are also to be found in the experimental part in the section for preparing the starting compounds and intermediates.

Compounds of the current invention possess valuable pharmacological properties and can be used for the treatment and/or prophylaxis of diseases/disease state affecting human beings and animals.

Compounds concerning the present invention are potent, chemically stable antagonists of the ADAMTS7 metalloprotease and are suited for the treatment and/or prevention of diseases in human beings and animals such as, heart diseases, vascular diseases, and/or cardiovascular diseases, including atherosclerosis, coronary artery disease (CAD), peripheral vascular disease (PAD)/arterial occlusive disease and/or restenosis after angioplasty (including the use of drug-coated or non drug-coated balloons and/or stent-implantation) and/or for the treatment and/or prophylaxis of lung diseases, inflammatory diseases, fibrotic diseases, metabolic diseases, cardiometabolic diseases and/or diseases/disease states affecting the kidneys and/or the central nervous and/or neurological system as well as gastrointestinal and/or urologic and/or ophthalmologic diseases/disease states.

In the context of the present invention heart diseases, vascular diseases and/or cardiovascular diseases or disease of the cardiovascular system include, e.g., acute and chronic heart failure, arterial hypertension, coronary heart disease, stable and instable angina pectoris, myocardial ischemia, myocardial infarction, coronary microvascular dysfunction, microvascular obstruction, no-reflow-phenomenon, shock, atherosclerosis, coronary artery disease, peripheral artery disease, peripheral arterial disease, intermittent claudication, severe intermittent claudication, limb ischemia, critical limb ischemia, hypertrophy of the heart, cardiomyopathies of any etiology (such as, e.g., dilatative cardiomyopathy, restrictive cardiomyopathy, hypertrophic cardiomyopathy, ischemic cardiomyopathy), fibrosis of the heart, atrial and ventricular arrhythmias, transitory and/or ischemic attacks, apoplexy, ischemic and/or hemorrhagic stroke, preeclampsia, inflammatory cardiovascular diseases, metabolic diseases, diabetes, type-I-diabetes, type-II-diabetes, diabetes mellitus, peripheral and autonomic neuropathies, diabetic neuropathies, diabetic microangiopathies, diabetic retinopathy, diabetic ulcera at the extremities, gangrene, CREST-syndrome, hypercholesterolemia, hypertriglyceridemia, lipometabolic disorder, metabolic syndrome, increased levels of fibrinogen and low-density lipoproteins (i.e. LDL), increased concentrations of plasminogen-activator inhibitor 1 (PAI-1), as well as peripheral vascular and cardiac vascular diseases, peripheral circulatory disorders, primary and secondary Raynaud syndrome, disturbances of the microcirculation, arterial pulmonary hypertension, spasms of coronary and peripheral arteries, thromboses, thromboembolic diseases, edema-formation, such as pulmonary edema, brain-edema, renal edema, myocardial edema, myocardial edema associated with heart failure, restenosis after i.e. thrombolytic therapies, percutaneous-transluminal angioplasties (PTA), transluminal coronary angioplasties (PTCA), heart transplantations, bypass-surgeries as well as micro- and macrovascular injuries (e.g., vasculitis), reperfusion-damage, arterial and venous thromboses, microalbuminuria, cardiac insufficiency, endothelial dysfunction.

In the light of the present invention, heart failure includes more specific or related kinds of diseases such as acute decompensated heart failure, right heart failure, left heart failure, global insufficiency, ischemic cardiomyopathy, dilatative cardiomyopathy, congenital heart defect(s), valve diseases, heart failure related to valve diseases, mitral valve stenosis, mitral valve insufficiency, aortic valve stenosis, aortic valve insufficiency, tricuspid valve stenosis, tricuspid valve insufficiency, pulmonary valve stenosis, pulmonary valve insufficiency, combined valvular defects, inflammation of the heart muscle (myocarditis), chronic myocarditis, acute myocarditis, viral myocarditis, bacterial myocarditis, diabetic heart failure, alcohol-toxic cardiomyopathy, cardiac storage diseases, heart failure with preserved ejection fraction (HFpEF), diastolic heart failure, heart failure with reduced ejection fraction (HFrEF), systolic heart failure.

In the context of the present invention, the terms atrial arrhythmias and ventricular arrhythmias also include more specific and related disease-entitites, such as: Atrial fibrillation, paroxysmal atrial fibrillation, intermittent atrial fibrillation, persistent atrial fibrillation, permanent atrial fibrillation, atrial flutter, sinus arrhythmia, sinus tachycardia, passive heterotopy, active heterotopy, replacement systoles, extrasystoles, disturbances in the conduction of impulses, sick-sinus syndrome, hypersensitive carotis-sinus, tachycardias, AV-node re-entry tachycardias, atrioventricular re-entry tachycardia, WPW-syndrome (Wolff-Parkinson-White syndrome), Mahaim-tachycardia, hidden accessory pathways/tracts, permanent junctional re-entry tachycardia, focal atrial tachycardia, junctional ectopic tachycardia, atrial re-entry tachycardia, ventricular tachycardia, ventricular flutter, ventricular fibrillation, sudden cardiac death.

In the context of the present invention, the term coronary heart disease also includes more specific or related diseases entities, such as: Ischemic heart disease, stable angina pectoris, acute coronary syndrome, instable angina pectoris, NSTEMI (non-ST-segement-elevation myocardial infarction), STEMI (ST-segement-elevation myocardial infarction), ischemic damage of the heart, arrhythmias, and myocardial infarction.

In the context of the present invention, diseases of the central nervous and neurological system or central nervous and neurological diseases/diseases states refer to, e.g., the following diseases/diseases states: Transitory and ischemic attacks, stroke/apoplexy, ischemic and hemorrhagic stroke, depression, anxiety disorder, post-traumatic stress-disorder, poly-neuropathy, diabetic poly-neuropathy, stress-induced hypertension.

Compounds concerning the present invention are furthermore suited for the prophylaxis and/or treatment of poly-cystic kindney-disease (PCKD) and the syndrome of inadequate ADH-secretion (SIADH). Furthermore, the compounds described in the present invention are suited for the treatment and/or prophylaxis of kidney diseases, especially of acute and chronic renal insufficiency as well as of acute and chronic renal failure.

In the context of the present invention, the term acute renal insufficiency/renal failure includes acute presentations of kidney diseases, kidney failure and/or renal insufficiency with or without the dependency on dialysis as well as underlying or related kidney diseases such as renal hypoperfusion, hypotension during dialysis, lack of volume (i.e. dehydration, blood-loss), shock, acute glomerulonephritis, hemolytic-uremic syndrome (HUS), vascular catastrophe (arterial or venous thrombosis or embolism), cholesterol-embolism, acute Bence-Jones-kidney associated with plasmacytoma, acute supravesical or subvesical outlow obstructions, immunologic kidney diseases such as kidney transplant rejection, immuncomplex-induced kidney diseases, tubular dilatation, hyperphosphatemia and/or akute kidney diseases which may be characterized by the need for dialysis. Also included are conditions of partial nephrectomy, dehydration caused by force diuresis, uncontrolled increase in blood pressure accompanied by malignant hypertension, urinary tract obstructions and infections and amyloidosis as well as systemic disorders with glomerular participation such as rheumatologic-immunologic systemic disorders, such as Lupus erythematodes, renal artery thrombosis, renal vein thrombosis, analgesics-induced nephropathy and renal-tubular acidosis as well as radio-opaque substance- as well as drug-induced acute interstitial kidney diseases.

In the context of the present invention the term chronic renal insufficiency/chronic renal failure includes chronic manifestations/presentations of kidney diseases, renal failure and/or renal insufficiency with and without the dependency on dialysis as well as underlying or related kidney diseases such as renal hypoperfusion, hypotension during dialysis, obstructive uropathy, glomerulopathies, glomerular and tubular proteinuria, renal edema, hematuria, primary, secondary as well as chronic glomerulonephritis, membraneous and membraneous-proliferative glomerulonephritis, Alport-syndrome, glomerulosclerosis, tubulointerstitial diseases, nephropathic diseases such as primary and hereditary kidney disease(s), renal inflammation, immunologic kidney diseases such as transplant rejection, immuncomplex-induced kidney diseases, diabetic and non-diabetic nephropathy, pyelonephritis, renal cysts, nephrosclerosis, hypertensive nephrosclerosis and nephrotic syndrome, which are diagnostically characterized by i.e. abnormally reduced creatinine- and/or water-excretion, abnormally increased blood-concentrations of urea, nitrogen, potassium and/or creatinine, altered activity of kidney enzymes, such as, e.g., glutamylsynthase, altered urinary osmolarity or volume, increased microalbuminuria, macroalbuminuria, lesions associated with glomeruli and arterioles, tubular dilatation, hyperphosphatemia and/or the need for dialysis; likewise included are renal cell carcinomas, conditions after partial kidney-resection, dehydration attributed to force diuresis, uncontrolled increase in blood pressure with malignant hypertension, urinary tract obstruction and urinary tract infection and amyloidosis as well as systemic diseases with glomerular participation such as rheumatologic-immunologic systemic diseases, such as lupus erythematodes, as well as renal artery stenosis, renal artery thrombosis, renal vein thrombosis, analgesics-induced nephropathy and renal-tubular acidosis. Furthermore, included are radio-opaque substance- or drug-induced chronic interstitial kidney diseases, metabolic syndrome and dyslipidemia. The current invention also includes the use of the drugs of the current invention for the treatment and/or prophylaxis of after-effects of renal insufficiency such as lung edema, heart failure, uremia, anemia, disturbances in electrolytes (e.g., hyperkalemia, hyponatremia) and disturbances in bone- and carbohydrate-metabolism.

Additionally, compounds of the current invention are suited for the treatment and/or prophylaxis of lung diseases (partially also seen as vascular diseases), such as, e.g., pulmonary arterial hypertension (PAH) and other forms of pulmonary hypertension (PH), chronic-obstructive pulmonary disease (COPD), acute respiratory distress syndrome (ARDS), acute lung injury (ALI), lung fibrosis, lung emphysema (e.g., lung emphysema induced by cigarette smoke), cystic fibrosis (CF) as well as for the treatment and/or prophylaxis of alpha-1-antitrypsin deficiency (AATD), acute coronary syndrome (ACS), inflammation of the heart muscle (myocarditis) and other autoimmune diseases of the heart (pericarditis, endocarditis, valvolitis, aortitis, cardiomyopathies), cardiogenic shock, aneurysms, sepsis (SIRS), multiple organ failure (MODS, MOF), inflammatory kidney diseases, chronic bowel diseases (IBD, Crohn's Disease, UC), pancreatitis, peritonitis, rheumatoid diseases, inflammatory skin diseases as well as inflammatory eye diseases.

Furthermore, compounds of the current invention can be used for the treatment and/or prophylaxis of asthmatic diseases of different severity with intermittent or persistent courses (refractive asthma, bronchial asthma, allergic asthma, intrinsic asthma, extrinsic asthma, asthma induced by drugs or dust), of different kinds of bronchitis (chronic bronchitis, infectious bronchitis, eosinophilic bronchitis), of bronchiolitis obliterans, bronchiectasia, pneumonia, idiopathic interstitial pneumonia, farmer's lung and related diseases, coughing and common cold diseases (chronic inflammatory cough, iatrogenic cough), inflammations of the nasal mucosa (including drug-induced rhinitis, vasomotor rhinitis and season-dependent allergic rhinitis, e.g., allergic coryza) as well as of polyps.

The compounds described in the current invention also represent active compounds for the treatment of diseases of the central nervous system, characterized by disturbances of the NO/cGMP-system. They are especially suited for improvement of perception, concentration-performance, learning-behaviour or memory-performance after cognitive disturbances as they occur with conditions/illnesses/syndromes such as "mild cognitive impairment", age-associated learning- and memory-disturbances, age-associated memory-loss, vascular dementia, craniocerebral injury, stroke, dementia occurring after stroke ("post stroke dementia"), post-traumatic craniocerebral injury, general concentration-disturbances, concentration-disturbances affecting children with learning- and memory-problems, Alzheimer's disease, dementia with Lewy-bodies, dementia with degeneration of the frontal lobe including Pick's syndrome, Parkinson's Disease, dementia with corticobasal degeneration, amyotrophic lateral sclerosis (ALS), Huntington's Disease, demyelination, multiple sclerosis, thalamic degeneration, Creutzfeld-Jacob-dementia, HIV-dementia, schizophrenia with dementia or Korsakoff-psychosis. They are also suited for the treatment and or prevention of diseases/disease states of the central nervous system such as conditions of anxiety, tension/pressure and depressions, bipolar disorder, sexual dysfunction due to disturbances in the central nervous system as well as sleep abnormalities and for regulation of pathological disturbances of food-, luxury food- and 'dependence causing substance'-intake.

Furthermore, the compounds of the current invention are also suited for the treatment and/or prophylaxis of urologic diseases/disease states such as, e.g., urinary incontinence, stress-induced incontinence, urge incontinence, reflex incontinence and overflow incontinence, detrusor hyperactivity, neurogenic detrusor hyperactivity, idiopathic detrusor hyperacitivity, benign prostate hyperplasia (BPH-syndrome), lower urinary tract symptoms.

The compounds of the current invention are further suited for the treatment and/or prevention of conditions of pain, such as, e.g., menstrual disorders, dysmenorrhea, endometriosis, preterm delivery, tocolysis.

The compounds of the current invention are likewise suited for the treatment and/or prevention of erythematosis, onychomycosis, rheumatic diseases as well as for facilitation of wound healing.

The compounds of the current invention are also suited for the treatment and/or prevention of gastrointestinal diseases such as, e.g., diseases/disease states affecting the oesophagus, vomiting, achalasia, gastrooesophageal reflux disease, diseases of the stomach, such as, e.g., gastritis, diseases of the bowel, such as, e.g., diarrhea, constipation, malassimilation syndromes, syndromes of bile acid-loss, Crohn's Disease, Colitis ulcerosa, microscopic colitis, irritable bowel syndrome.

Furthermore, compounds of the current invention are suited for the treatment and/or prophylactic treatment of fibrotic diseases of inner organs such as lung, heart, kidney, bone marrow, and escpecially liver as well as dermatological fibrosis and fibrotic eye diseases. In the context of the current invention the term fibrotic diseases includes liver fibrosis, liver cirrhosis, lung fibrosis, endomyocardial fibrosis, cardiomyopathy, nephropathy, glomerulonephritis, interstitial kidney fibrosis, fibrotic damage as a consequence of diabetes, bone marrow fibrosis and similar fibrotic diseases, scleroderma, morphaea, keloids, hypertrophic scarring (also after surgical intervention), naevus, diabetic retinopathy and proliferative vitroretinopathy.

In addition, the compounds of the current invention can be used to treat and/or prophylactically treat dyslipidemias (hypercholesterolemia, hypertriglyceridemia, increased concentrations of post-prandial plasma triglycerides, hypo-alphalipoproteinemia, combined hyperlipidemias), metabolic diseases (type I and type II diabetes, metabolic syndrome, overweight, adipositas), nepropathy and neuropathy, cancer (skin cancer, brain tumors, breast cancer, tumors of the bone marrow, leukemias, liposarcoma, carcinoma of the gastrointestinal tract, liver, pancreas, lung, kidney, ureter, prostate and gential tract as well as carcinoma of the lymphoproliferative system such as, e.g., Hodgkin's and Non-Hodgkin's lymphoma), of gastrointestinal and abdominal diseases (glossitis, gingivitis, periodontitis, esophagitis, eosinophilic gastroenteritis, mastocytosis, Crohn's disease, colitis, proctitis, anal pruritis, diarrhea, celiac disease, hepatitis, chronic hepatitis, liver fibrosis, liver zirrhosis, pancreatitis and cholecystitis), skin diseases (allergic skin diseases, psoriasis, acne, eczema, neurodermatitis, multiple kinds of dermatitis, as well as keratitis, bullosis, vasculitis, cellulitis, panniculitis, lupus erythematodes, erythema, lymphoma, skin cancer, Sweet-syndrome, Weber-Christian-syndrome, scarring, wart formation, chilblains), of diseases of the sceletal bones and the joints as well as of sceletal muscle (multiple kinds of arthritis, multiple kinds of arthropathies, scleroderma as well as of further diseases with inflammatory or immunologic components, such as, e.g., paraneoplastic syndrome, rejection reactions after organ transplantations and for wound healing and angiogenesis, especially with chronic wounds.

The compounds of the current invention are suited for the treatment and/or prophylactic treatment of ophthalmologic diseases such as, e.g., glaucoma, normotensive glaucoma, increased/high ocular pressure and their combination, of age-related macula degeneration (AMD), dry (non-exudative) AMD, wet (exudative, neovascular) AMD, choroidal neovascularization (CNV), retinal detachment, diabetic retinopathy, atrophic changes of the retinal pigmented epithelium (RPE), hypertrophic changes of the retinal pigmented epithelium, diabetic macula edema, diabetic retinopathy, retinal vein occlusion, choroidal retinal vein occlusion, macula edema, diabetic macula edema, macula edema as a consequence of retinal vein occlusion, angiogenesis at the front-side of the eye such as corneal angiogenesis i.e. after keratitis, cornea transplantation or keratoplasty, corneal angiogenesis due to hypoxia (extensive wearing of contact lenses), Pterygium conjunctivae, sub-retinal edema and intra-retinal edema.

Furthermore, compounds of the current invention are suited for the treatment and/or prophylactic treatment of increased and high inner ocular pressure as a result of traumatic hyphema, periorbital edema, post-operative viscoelastic retention, intra-ocular inflammation, corticosteroid-use, pupil-block or idiopathic causes such as increased inner ocular pressure after trabeculectomy and due to pre-operative additives.

Furthermore, compounds of the current invention are suited for the treatment and/or prophylaxis of hepatitis, neoplasms, osteoporosis, glaucoma and gastroparesis.

Likewise, compounds of the current invention are suited for the regulation of cerebral blood circulation and represent useful agents for the treatment and or prophylaxis of migraine. They are also suited for the treatment and prophylaxis of cerebral infarcts such as stroke, cerebral ischemias and traumatic brain injury. Likewise, compunds of the current invention can be used for the treatment and/or prophylactic treatment of pain, neuralgias and tinnitus.

The aforementioned, well characterized human diseases may occur in other mammalians with a comparable etiology as well can be treated with the compounds of the current invention.

In the context of the current invention the term "treatment" or "treat" or "treated" includes inhibition, retardation, stopping, relief, reduction, attenuation, restriction, minimizing, suppression, repression or healing of a disease, a suffering, an illness, an injury or a health disorder, or the development, course and progression of such states/conditions and/or symptoms of these states/conditions.

The terms "prevention" and "prophylaxis" are used synonymously in the context of the present invention und refer to avoiding or reducing the risk to get, receive, experience, suffer from a disease, a suffering, an illness, an injury or a health disorder, the development, course and progression of such states/conditions and/or symptoms of these states/conditions.

Treatment or prevention of a disease, a suffering, an illness, an injury or a health disorder can be pursued in part or completely.

Other embodiments of the current invention therefore are directed to the use of the compounds of the current invention for the treatment and/or prevention of diseases, especially of the aforementioned diseases/disease states.

Other embodiments of the current invention therefore are directed to the use of the compounds of the current invention for the production/manufacturing of a compound/of compounds for the treatment and/or prevention of diseases, especially of the aforementioned diseases/disease states.

Other embodiments of the current invention are directed to a drug containing at least one of the compounds of the present invention for the treatment and/or prevention of diseases, especially of the aforementioned diseases/disease states.

Other embodiments of the current invention therefore are directed to the use of the compounds of the current invention in a process for the treatment and/or prevention of diseases, especially of the aforementioned diseases/disease states.

Other embodiments of the current invention are directed to a process for the treatment and/or prevention of diseases, especially of the aforementioned diseases, by use of an effective dose/amount of at least one of the compounds of the current invention.

Other embodiments of the current invention are directed to drugs and pharmaceutical formulations which contain at least one compound of the current invention, typically together with one or more inert, non-toxic, pharmaceutically suited additives, as well as their use for the aforementioned purposes.

Further embodiments of the current invention include the use of compound of formula (I) in a method for the treatment and/or prevention of heart diseases, vascular diseases, cardiovascular diseases, lung diseases, inflammatory diseases, fibrotic diseases, metabolic diseases and/or cardiometabolic diseases.

Further embodiments of the current invention include the use of compound of formula (I) in a method for the treatment and/or prevention of Atherosclerosis, athersclerosis-related diseases such as coronary artery disease or peripheral vascular disease/arterialocclusive disease as well as post-surgery complications of these diseases such asrestenosis after angioplasty.

Further embodiments include the use of a compound according to formula (I) for the manufacture of a pharmaceutical composition for the treatment and/or preventin atherosclerosis, athersclerosis-related diseases such as coronary artery disease o peripheral vascular disease/arterial occlusive disease as well as post-surgerycomplications of these diseases such as restenosis after angioplasty.

Further embodiments include the use of a compound according to the current invention for the manufacture of a pharmaceutical composition for the treatment and/or prevention atherosclerosis ,athersclerosis-related diseases such as coronary artery disease or peripheral vascular disease/arterial occlusive disease as well as post-surgery complications of these diseases such as restenosis after angioplasty.

Further embodiments include a pharmaceutical composition comprising a compound according to the current invention and one or more pharmaceutically acceptable excipients.

Other embodiments include a pharmaceutical composition comprising one or more first active ingredients, in particular compounds according to the current invention and one or more further active ingredients, in particular one or more additional therapeutic agents selected from the group consisting of angiotensin-converting enzyme inhibitors, angiotensin-receptor blockers, mineralocorticoid-receptor antagonists, endothelin antagonists, renin inhibitors, calcium blockers, beta-receptor blockers, vasopeptidase inhibitors, Sodium-Glucose-Transport-Antagonists, Metformin, Pioglitazones and Dipeptidyl-peptidase-IV inhibitors.

Further embodiments of the current invention include the pharmaceutical composition as defined above for the treatment and/or prevention of atherosclerosis ,athersclerosis-related diseases such as coronary artery disease or peripheral vascular disease/arterial occlusive disease as well as post-surgery complications of these diseases such as restenosis after angioplasty.

Further embodiments of the invention include a method for the treatment and/or prevention atherosclerosis ,athersclerosis-related diseases such as coronary artery disease or peripheral vascular disease/arterial occlusive disease as well as post-surgery complications of these diseases such as restenosis after angioplastyin a human or other mammal, comprising administering to a human or other mammal in need thereof a therapeutically effective amount of one or more compounds according to the current invention or of a pharmaceutical composition as definedabove.

The compounds according to the invention can be used alone or in combination with one or more other pharmacologically active compounds if necessary as long as these combinations do not lead to unacceptable side effects. The present invention further relates to drugs containing at least one of the compounds according to the invention and one or more further active compounds/active components, in particular for the treatment and/or prophylaxis of the aforementioned diseases. As suitable active compounds/active components for combination, the following ones are mentioned exemplaryily and preferably:
- Positive inotropic compounds, such as, e.g., cardiac glycosides (digoxin), beta-adrenergic and dopaminergic agonists, such as isoprenaline, adrenaline, noradrenaline, dopamine or dobutamine and serelaxine;
- Vasopressin-receptor antagonists, for example and preferably Conivaptan, Tolvaptan, Lixivaptan, Mozavaptan, Satavaptan, SR-121463, RWJ 676070 or BAY 86-8050, as well as the compounds described in WO 2010/105770, WO 2011/104322 and WO 2016/071212;
- Natriuretic peptides, for example and preferably atrial natriuretic peptide (ANP), natriuretic peptide type B (BNP, Nesiritide), natriuretic peptide type C (CNP) or urodilatin;
- Activators of cardiac myosin, for example and preferably e.g., Omecamtiv mecarbil (CK-1827452);
- Calcium-sensitizers, for example and preferably Levosimendan;
- Compounds affecting mitochondrial function and/or production of reactive oxygen species (ROS), for example Bendavia/Elamipritide;
- Compounds influencing cardiac energy-metabolism, for example and preferably etomoxir, dichloroacetate, ranolazine, trimetazidine, full or partial adenosine A1 receptor agonists such as GS-9667 (formerly known as CVT-3619), capadenosone and neladenosone;
- Compounds with an effect on heart rate, e.g., ivabradine
- Organic nitrates and NO-donors, such as sodium nitroprusside, nitroglycerin, isosorbide mononitrate, isosorbide dinitrate, molsidomine or SIN-1, and inhalational NO;
- Compounds that inhibit the degradation of cyclic guanosine monophosphate (cGMP) and/or cyclic adenosine monophosphate (cAMP), such as inhibitors of phosphodiesterases (PDE) 1, 2, 3, 4 and/or 5, in particular PDE 4 inhibitors such as roflumilast or revamilast and PDE 5 inhibitors such as sildenafil, vardenafil, tadalafil, udenafil, dasantafil, avanafil, mirodenafil, lodenafil or PF-00489791;
- Compounds increasing cGMP synthesis, such as, e.g., sGC modulators in particular riociguat, nelociguat, vericiguat, cinaciguat and the compounds described in WO 00/06568, WO 00/06569, WO 02/42301, WO 03/095451, WO 2011/147809, WO 2012/004258, WO 2012/028647, WO 2012/059549, WO 2014/068099 and WO 2014/131760 as well as the compounds described in WO 01/19355, WO 01/19780, WO 2012/139888 and WO 2014/012934;
- Compounds which inhibit the soluble epoxid-hydrolase (sEH), such as, e.g., N,N'-dicyclohexylurea, 12-(3-Adamantan-1-yl-ureido)-dodecanoic acid or 1-Adamantan-1-yl-3-{5-[2-(2-ethoxyethoxy)ethoxy]pentyl}-urea;
- Compounds modulating neurotransmitters, such as, e.g., tricyclic antidepressants such as, e.g., amitryptiline and imaprimine, monoaminooxidase (MAO) inhibitors such as moclobemide, serotonin-noradrenaline-reuptake inibitors such as venlaflaxine, selective serotonin-reuptake inhibitors such as sertraline or noradrenergic and specific serotonergic antidepressants such as mirtazapine.
- Compounds with anxiolytic, sedative and hypnotic properties, so-called tranquilizers such as, e.g., short- as well as mid-long acting benzodiazepines.
- Prostacyclin analogs and IP receptor agonists, for example and preferably iloprost, bera-prost, treprostinil, epoprostenol, NS-304, selexipag, or ralinepag;
- Compounds which inhibit the signal transduction cascade, in particular from the group of the tyrosine kinase inhibitors and/or from the group of serine/threoninekinase-inhibitors, for example and preferably dasatinib, nilotinib, bosutinib, regorafenib, sorafenib, sunitinib, cediranib, axitinib, telatinib, imatinib, brivanib, pazopanib, vatalanib, gefitinib, erlotinib, lapatinib, canertinib, lestaurtinib, pelitinib, semaxanib, masitinib or tandutinib, Rho kinase inhibitors, such as fasudil, Y-27632, SLx-2119, BF-66851, BF-66852, BF-66853, KI-23095 or BA-1049;
- Anti-obstructive agents as used, for example, for the therapy of chronic-obstructive pulmonary disease (COPD) or bronchial asthma, for example and preferably inhalatively or systemically administered beta-receptor mimetics (e.g., bedoradrine) or inhalatively administered anti-muscarinergic substances;
- Compounds with a bronchodilatory effect, for example and preferably from the group of β-adrenergic receptor-agonists, such as particularly albuterol, isoproterenol, metaproterenol, terbutaline, formoterol or salmeterol or from the group of anti-cholinergics, such as particularly ipratropiumbromide;
- Anti-inflammatory and/or immunosuppressive agents as used, for example for the therapy of chronic-obstructive pulmonary disease (COPD), of bronchial asthma or pulmonary fibrosis, for example and preferably from the group of corticosteroids, such as particularly prednisone, prednisolone, methylprednisolone, triamcinolone, dexamethasone, beclomethasone, betamethasone, flunisolide, budesonide or fluticasone as well as from the group of non-steroidal anti-inflammatory drugs (NSAIDs), such as particularly acetylsalicylic acid (Aspirin), ibuprofen and naproxen, 5-aminosalicylic acid derivatives, leukotriene/leukotriene receptor antagonists,
   TNF-α inhibitors and chemokine receptor antagonists, such as, e.g., CCR-1, -2 and/or -5 inhibitors. Furthermore, drugs such as pirfenidone, acetylcysteine, azathioprine or BIBF-1120;
- Chemotherapeutics as used, for example, for the therapy of neoplasias of the lung or other organs;
- Active compounds used for the systemic and/or inhalative treatment of pulmonary disorders, for example for cystic fibrosis (alpha-1-antitrypsin, aztreonam, ivacaftor, lumacaftor, ataluren, amikacin, levofloxacin), chronic obstructive pulmonary diseases (COPD) (LAS40464, PT003, SUN-101), acute respiratory distress syndrome (ARDS) and acute lung injury (ALI) (interferon-beta-1a, traumakines), obstructive sleep apnea (VI-0521), bronchiectasis (mannitol, ciprofloxacin), Bronchiolitis obliterans (cyclosporine, aztreonam) and sepsis (pagibaximab, Voluven, ART-123);
- Active compounds used for treating muscular dystrophy, for example idebenone;
- Antithrombotic agents, for example and preferably from the group of platelet aggregation inhibiting drugs (platelet aggregation inhibitors, thrombocyte aggregation inhibitors), anticoagulants or compounds with anticoagulant properties or profibrinolytic substances;
- Active compounds for lowering blood pressure, for example and preferably from the group of calcium antagonists, angiotensin All antagonists, ACE inhibitors, NEP inhibitors, vasopeptidase inibitors, and combinations thereof, such as, e.g., sacubitril/valsartan (Entresto®), furthermore nicorandil, endothelin antagonists/endothelin receptor antagonists, such as bosentan, darusentan, ambrisentan, macicentan or sitaxentan, thromboxane A2 (TXA2) antagonists/thromboxane A2 (TBX2) receptor antagonists, renin inhibitors, alpha-receptor blockers, beta-receptor blockers, mineralocorticoid receptor antagonists, Rho-kinase inhibitors, diuretics as well as further vasoactive compounds/active components such as, e.g., adenosine and adenosine receptor agonists.
- Compounds which inhibit degradation and remodelling of the extracellular matrix, for example and preferably inhibitors of matrix metalloproteases (MMPs), in particular chymase-inhibitors, stromelysin-inhibitors, collagenase-inhibitors, gelatinase-inhibitors and aggrecanase-inhibitors (in these terms especially MMP-1, MMP-3, MMP-8, MMP-9, MMP-10, MMP-11 and MMP13) as well as inhibitors of the metallo-elastase MMP-12 as well as neutrophil elastase (HNE) inhibitors, for example and preferably sivelestat or DX-890 (Reltran);
- Compounds which inhibit the binding of serotonin to its receptor, for example and preferably antagonists of the 5-HT₁-, 5-HT₂ₐ-, 5-HT_{2b}-, 5-HT_{2c}-, 5-HT₃- and 5-HT₄-receptors;
- Anti-arrhythmic compounds/active components, for example and preferably sodium channel inhibitors, beta-receptor blockers, potassium channel blockers, calcium antagonists, I_{f}-channel blockers, digitalis, parasympatholytics (vagolytics), sympathomimetics and other anti-arrhythmic drugs such as, e.g., adenosine, adenosine-receptor agonists as well as vernakalant.
- Active compounds that alter fat metabolism, for example and preferably from the group of thyroid receptor agonists, cholesterol synthesis inhibitors, for example and preferably HMG-CoA-reductase or squalene synthesis inhibitors, ACAT inhibitors, CETP inhibitors, MTP inhibitors, PPAR-alpha-, PPAR-gamma- and/or PPAR-delta-agonists, cholesterol absorption inhibitors, lipase inhibitors, polymeric bile acid adsorbers, bile acid reabsorption inhibitors and lipoprotein(a) antagonists.
- Active ingredients which inhibit neoangiogenesis, for example and preferably inhibitors of the VEGF and/or PDGF signalling pathways, inhibitors of the integrin signalling pathways, inhibitors of the angiopoietin-Tie signalling pathways, inhibitors of the PI3K-Akt-mTor signalling pathways, inhibitors of the Ras-Raf-Mek-Erk signalling pathway, inhibitors of the MAPK signalling pathways, inhibitors of the FGF signalling pathways, inhibitors of the sphingosine-1-phosphate signalling pathways, inhibitors of endothelial cell proliferation or apoptosis-inducing active ingredients;
- Active ingredients which reduce vascular wall permeability (edema formation), for example and preferably corticosteroids, inhibitors of the ALK1-Smad1/5 signalling pathway, inhibitors of the VEGF and/or PDGF signalling pathways, cyclooxygenase inhibitors, inhibitors of the kallikrein-kinin system or inhibitors of the sphingosine-1-phosphate signalling pathways;
- Active ingredients which reduce damage to the retina under oxidative stress, for example and perferably addressing the complement system, especially antagonists of the complement C5a receptor, or agonists of the 5-HT1A receptor;
- Antioxidants and free-radical scavengers;
- Active hypotensive ingredients, for example and preferably from the group of the calcium antagonists, angiotensin All antagonists, ACE inhibitors, beta-receptor blockers, alpha-receptor blockers, diuretics, phosphodiesterase inhibitors, sGC stimulators, cGMP elevators, aldosterone antagonists, mineralocorticoid receptor antagonists, ECE inhibitors and vasopeptidase inhibitors;
- Antidiabetics and accordingly compounds/active components changing glucose metabolism, for example and preferably from the group of the insulins and/or insulin derivatives, biguanides, sulfonylureas, acarbose, DPP4-inhibitors, meglitinide derivatives, glucosidase inhibitors, GLP 1 receptor agonists/GLP1 analogues, SGLT-2 inhibitors, glucagon antagonists, PPAR-gamma agonists/insulin sensitizers, such as, e.g., thiazolidinediones, CCK1 receptor agonists, leptin receptor agonists, potassium channel antagonists and the inhibitors of hepatic enzymes that are involved in the stimulation of gluconeogenesis and/or glycogenolysis;
- Antiinfectives, for example and in particular from the group of the antibacterial, antifungal and/or antiviral substances;
- Substances for treatment of glaucoma, for example and in particular from the group of the adrenergics, beta-receptor blockers, carbonic anhydrase inhibitors, parasympathomimetics and prostaglandins;
- Pain-reducing compounds such as opiates.

Antithrombotic agents are for example and preferably to be understood as compounds from the group of platelet aggregation inhibiting drugs (platelet aggregation inhibitors, thrombocyte aggregation inhibitors), anticoagulants or compounds with anticoagulant properties or profibrinolytic substances.

In preferred embodiments of the invention, the compounds according to the invention are administered in combination with compounds from the group of platelet aggregation inhibiting drugs (platelet aggregation inhibitors, thrombocyte aggregation inhibitors), for example and preferably aspirin, clopidogrel, prasugrel, ticlopidine, ticagrelor, cangrelor, elinogrel, tirofiban, PAR1-antagonists such as, e.g., vorapaxar, PAR4-antagonists, EP3-antagonists, such as, e.g., DG041 or inhibitors of adenosine-transport, such as dipyridamole;

In preferred embodiments of the invention, the compounds according to the invention are administered in combination with a thrombin inhibitor, for example and preferably ximelagatran, melagatran, dabigatran, bivalirudin or Clexane.

In preferred embodiments of the invention, the compounds according to the invention are administered in combination with a GPIIb/IIIa antagonist, for example and preferably tirofiban or abciximab.

In preferred embodiments of the invention, the compounds according to the invention are administered in combination with a factor Xa inhibitor, for example and preferably rivaroxaban, apixaban, edoxaban (DU-176b), darexaban, betrixaban, otamixaban, letaxaban, fidexaban, razaxaban, fondaparinux, idraparinux, as well as thrombin-inhibitors, for example and preferably dabigatran, dual thrombin/factor Xa-inhibitors, such as for example and preferably tanogitran or with factor XI- or factor Xla-inhibitors.

In preferred embodiments of the invention, the compounds according to the invention are administered in combination with heparin or a low molecular weight (LMW) heparin derivatives, such as, e.g., tinzaparin, certoparin, parnaparin, nadroparin, ardeparin, enoxaparin, reviparin, dalteparin, danaparoid, semuloparin (AVE 5026), adomiparin (M118) and EP-42675/ORG42675.

In preferred embodiments of the invention, the compounds according to the invention are administered in combination with a vitamin K antagonist, for example and preferably coumarines, such as marcumar/phenprocoumon.

In preferred embodiments of the invention, the compounds according to the invention are administered in combination with pro-fibrinolytic substances, for example and preferably streptokinase, urokinase or plasminogen-activator.

The agents for lowering blood pressure are preferably to be understood as compounds from the group of calcium antagonists, angiotensin All antagonists, ACE inhibitors, endothelin antagonists/endothelin receptor antagonists, thromboxane A2 (TBX2)-antagonists/thromboxane A2 (TBX2) receptor antagonists, renin inhibitors, alpha-receptor blockers, beta-receptor blockers, mineralocorticoid-receptor antagonists, Rho-kinase inhibitors as well as diuretics.

In preferred embodiments of the invention, the compounds according to the invention are administered in combination with a calcium antagonist, for example and preferably nifedipine, amlodipine, verapamil or diltiazem.

In preferred embodiments of the invention, the compounds according to the invention are administered in combination with an alpha-1-receptor blocker, for example and preferably prazosin.

In preferred embodiments of the invention, the compounds according to the invention are administered in combination with a beta-receptor blocker, for example and preferably propranolol, atenolol, timolol, pindolol, alprenolol, oxprenolol, penbutolol, bupranolol, metipranolol, nadolol, mepindolol, carazolol, sotalol, metoprolol, betaxolol, celiprolol, bisoprolol, carteolol, esmolol, labetalol, carvedilol, adaprolol, landiolol, nebivolol, epanolol or bucindolol.

In preferred embodiments of the invention, the compounds according to the invention are administered in combination with an angiotensin All antagonist, for example and preferably losartan, candesartan, valsartan, telmisartan or embursatan, irbesartan, olmesartan, eprosartan or azilsartan or a dual angiotensin AII-antagonist/NEP-inhibitor, for example and preferably Entresto (LCZ696, Valsartan/Sacubitril).

In preferred embodiments of the invention, the compounds according to the invention are administered in combination with an ACE-inhibitor, for example and preferably enalapril, captopril, lisinopril, ramipril, delapril, fosinopril, quinopril, perindopril or trandopril.

In preferred embodiments of the invention, the compounds according to the invention are administered in combination with an endothelin antagonist/endothelin receptor antagonist, for example and preferably bosentan, darusentan, ambrisentan, avosentan, macicentan, atrasentan or sitaxsentan.

In preferred embodiments of the invention, the compounds according to the invention are administered in combination with a renin inhibitor, for example and preferably aliskiren, SPP-600 or SPP-800.

In preferred embodiments of the invention, the compounds according to the invention are administered in combination with a thromboxane A2 (TBX2)-antagonist, for example and preferably seratrodast or KP-496.

In preferred embodiments of the invention, the compounds according to the invention are administered in combination with a mineralocorticoid-receptor antagonist, for example and preferably spironolactone, eplerenone or finerenone.

In preferred embodiments of the invention, the compounds according to the invention are administered in combination with a diuretic, for example and preferably furosemide, torasemide bumetanide and piretanide, with potassium-saving diuretics, such as, e.g., amiloride or triamterene as well as with thiazide diuretics, such as, e.g., hydrochlorthiazide, chlorthalidone, xipamide and indapamide. Likewise, the combination with further diuretics is applicable, for example and preferably with bendroflumethiazide, chlorthiazide, hydroflumethiazide, methyclothiazide, polythiazide, trichlormethiazide, metolazone, quinethazone, acetazolamide, dichlorphenamide, methazolamide, glycerol, isosorbide or mannitol.

In preferred embodiments of the invention, the compounds according to the invention are administered in combination with a Rho-kinase inhibitor, for example and preferably fasudil, Y 27632, SLx-2119, BF-66851, BF-66852, BF-66853, KI-23095, SB-772077, GSK-269962A or BA-1049.

In preferred embodiments of the invention, the compounds according to the invention are administered in combination with natriuretic peptides, such as, for example "atrial natriuretic peptide" (ANP, Anaritide), "B-type natriuretic peptide", "brain natriuretic peptide" (BNP, Nesiritide), "C-type natriuretic peptide" (CNP) or Urodilatin;

In preferred embodiments of the invention, the compounds according to the invention are administered in combination with inhibitors of the endopeptidase (NEP-inhibitors), for example Sacubitril, Omapatrilat orAVE-7688, or as dual combinations ('ARNIs') with Angiotensin receptor antagonists (for example Valsartan), such as, for example Entresto/LCZ696.

In preferred embodiments of the invention, the compounds according to the invention are administered in combination with type II antidiabetic drugs, such as inhibitors of the sodium-glucose co-transporter 2 (SGLT2 inhibitors), for example Empagliflozin, Canagliflozin, Dapagliflozin, Ipragliflozin, Tofogliflozin and inhibitors of the dipeptidyl peptidase 4 (DPP-4 inhibitors), for example sitagliptin, saxagliptin, linagliptin, alogliptin.

Substances altering fat metabolism are preferably to be understood as compounds from the group of CETP inhibitors, thyroid receptor agonists, cholesterol synthesis inhibitors such as HMG-CoA-reductase or squalene synthesis inhibitors, the ACAT inhibitors, MTP inhibitors, PPAR-alpha, PPAR-gamma and/or PPAR-delta agonists, cholesterol-absorption inhibitors, polymeric bile acid adsorbers, bile acid reabsorption inhibitors, lipase inhibitors as well as the lipoprotein(a) antagonists.

In preferred embodiments of the invention, the compounds according to the invention are administered in combination with a CETP inhibitor, for example and preferably torcetrapib (CP-529414), anacetrapib, JJT-705 or CETP-vaccine (Avant).

In preferred embodiments of the invention, the compounds according to the invention are administered in combination with a thyroid receptor agonist, for example and preferably D-thyroxin, 3,5,3'-triiodothyronin (T3), CGS 23425 or axitirome (CGS 26214).

In preferred embodiments of the invention, the compounds according to the invention are administered in combination with a HMG-CoA-reductase inhibitor from the class of statins, for example and preferably lovastatin, simvastatin, pravastatin, fluvastatin, atorvastatin, rosuvastatin or pitavastatin.

In preferred embodiments of the invention, the compounds according to the invention are administered in combination with a squalene synthesis inhibitor, for example and preferably BMS-188494 or TAK-475.

In preferred embodiments of the invention, the compounds according to the invention are administered in combination with an ACAT inhibitor, for example and preferably avasimibe, melinamide, pactimibe, eflucimibe or SMP-797.

In preferred embodiments of the invention, the compounds according to the invention are administered in combination with an MTP inhibitor, for example and preferably implitapide, BMS-201038, R-103757 or JTT-130.

In preferred embodiments of the invention, the compounds according to the invention are administered in combination with a PPAR-gamma agonist, for example and preferably pioglitazone or rosiglitazone.

In preferred embodiments of the invention, the compounds according to the invention are administered in combination with a PPAR-delta agonist, for example and preferably GW 501516 or BAY 68-5042.

In preferred embodiments of the invention, the compounds according to the invention are administered in combination with a cholesterol-absorption inhibitor, for example and preferably ezetimibe, tiqueside or pamaqueside.

In preferred embodiments of the invention, the compounds according to the invention are administered in combination with a lipase inhibitor, for example and preferably orlistat.

In preferred embodiments of the invention, the compounds according to the invention are administered in combination with a polymeric bile acid adsorber, for example and preferably cholestyramine, colestipol, colesolvam, CholestaGel or colestimide.

In preferred embodiments of the invention, the compounds according to the invention are administered in combination with a bile acid reabsorption inhibitor, for example and preferably ASBT (= IBAT) inhibitors, such as, e.g., AZD-7806, S-8921, AK-105, BARI-1741, SC-435 or SC-635.

In preferred embodiments of the invention, the compounds according to the invention are administered in combination with a lipoprotein(a) antagonist, for example and preferably gemcabene calcium (Cl-1027) or nicotinic acid.

Substances inhibiting signal transduction are preferably to be understood as compounds from the group of the tyrosine-kinase inhibitors and/or serine/threonine-kinase-inhibitors.

In preferred embodiments of the invention, the compounds according to the invention are administered in combination with a kinase-inhibitor, for example and preferably canertinib, erlotinib, gefitinib, dasatinib, imatinib, lapatinib, lestaurtinib, lonafarnib, nintedanib, nilotinib, bosutinib, axitinib, telatinib, brivanib, pazo¬panib, pegaptinib, peli¬tinib, semaxa¬nib, regora¬fenib, sora-fenib, sunitinib, tandutinib, tipifarnib, vatalanib, cediranib, masitinib, fasudil, lonidamine, leflunomide, BMS-3354825 or Y-27632.

Substances modulating glucose metabolism are preferably to be understood as compounds from the group of insulins, sulfonylureas, acarbose, DPP4-inhibitors, GLP-1 analogues or SGLT-2 inhibitors.

Substances modulating neurotransmitters are preferably to be understood as compounds from the group of tricyclic antidepressants, monoaminooxidase (MAO)-inhibitors, serotonin-noradrenaline-reuptake inhibitors (SNRI) and noradrenergic and specific serotonergic antidepressants (NaSSa).

In preferred embodiments of the invention, the compounds according to the invention are administered in combination with a tricyclic antidepressant, for example and preferably amitryptilin or imipramin.

In preferred embodiments of the invention, the compounds according to the invention are administered in combination with a monoaminooxidase (MAO)-inhibitor, for example and preferably moclobemide.

In preferred embodiments of the invention, the compounds according to the invention are administered in combination with a selective serotonine-noradrenaline reuptake inhibitor (SNRI), for example and preferably venlafaxine.

In preferred embodiments of the invention, the compounds according to the invention are administered in combination with a selective serotonine reuptake inhibitor (SSRI), such as, e.g., sertraline.

In preferred embodiments of the invention, the compounds according to the invention are administered in combination with a noradrenergic and specific serotonergic antidepressants (NaSSa), for example and preferably mirtazapine.

Substances with pain-reducing, anxiolytic or sedatative properties are preferably to be understood as compounds from the group of opiates and benzodiazepines.

In preferred embodiments of the invention, the compounds according to the invention are administered in combination with an opiate, for example and preferably morphine or sulfentanyl or fentanyl.

In preferred embodiments of the invention, the compounds according to the invention are administered in combination with a benzodiazepine, for example and preferably midazolam or diazepam.

Substances modulating cGMP-synthesis, such as, e.g., sGC-modulators, are preferably to be understood as compounds that stimulate or activate the soluble guanylate cyclase.

In preferred embodiments of the invention, the compounds according to the invention are administered in combination with sGC modulators, for example and preferably in riociguat, nelociguat, vericiguat, cinciguat and the compounds described in WO 00/06568, WO 00/06569, WO 02/42301, WO 03/095451, WO 2011/147809, WO 2012/004258, WO 2012/028647, WO 2012/059549, WO 2014/068099 and WO 2014/131760 as well as the compounds described in WO 01/19355, WO 01/19780, WO 2012/139888 and WO 2014/012934;

In preferred embodiments of the invention, the compounds according to the invention are administered in combination with full or partial adenosine A1 receptor agonists, such as, e.g., GS-9667 (formerly known as CVT-3619), capadenosone and neladenosone or compounds affecting mitochondrial function/ROS-production such as, e.g., Bendavia/elamipritide.

In preferred embodiments of the invention, the compounds according to the invention are administered in combination with a TGF-beta antagonist, for example and preferably pirfenidone or fresolimumab.

In preferred embodiments of the invention, the compounds according to the invention are administered in combination with a TNF-alpha antagonist, for example and preferably adalimumab.

In preferred embodiments of the invention, the compounds according to the invention are administered in combination with HIF-PH-inhibitors, for example and preferably molidustat or roxadustat.

In preferred embodiments of the invention, the compounds according to the invention are administered in combination with a serotonin-receptor antagonist, for example and preferably PRX-08066.

It is possible for the compounds according to the invention to have systemic and/or local activity. For this purpose, they can be administered in a suitable manner, such as, for example, via the oral, parenteral, pulmonary, nasal, sublingual, lingual, buccal, rectal, vaginal, dermal, transdermal, conjunctival, otic route or as an implant or stent.

For these administration routes, it is possible for the compounds according to the invention to be administered in suitable administration forms.

For oral administration, it is possible to formulate the compounds according to the invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.

Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal). Administration forms which are suitable for parenteral administration are, inter alia, preparations for injection and infusion in the form of solutions, suspensions, emulsions, lyophylisates or sterile powders.

Examples which are suitable for other administration routes are pharmaceutical forms for inhalation [inter alia powder inhalers, nebulizers], nasal drops, nasal solutions, nasal sprays; tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, aqueous suspensions (lotions, mixturae agitandae), lipophilic suspensions, emulsions, ointments, creams, transdermal therapeutic systems (such as, for example, patches), milk, pastes, foams, dusting powders, implants or stents.

The compounds according to the invention can be incorporated into the stated administration forms. This can be effected in a manner known per se by mixing with pharmaceutically suitable excipients. Pharmaceutically suitable excipients include, inter alia,
fillers and carriers (for example cellulose, microcrystalline cellulose (such as, for example, Avicel®), lactose, mannitol, starch, calcium phosphate (such as, for example, Di-Cafos®)),
ointment bases (for example petroleum jelly, paraffins, triglycerides, waxes, wool wax, wool wax alcohols, lanolin, hydrophilic ointment, polyethylene glycols),
bases for suppositories (for example polyethylene glycols, cacao butter, hard fat),
solvents (for example water, ethanol, isopropanol, glycerol, propylene glycol, medium chain-length triglycerides fatty oils, liquid polyethylene glycols, paraffins),
surfactants, emulsifiers, dispersants or wetters (for example sodium dodecyl sulfate), lecithin, phospholipids, fatty alcohols (such as, for example, Lanette®), sorbitan fatty acid esters (such as, for example, Span®), polyoxyethylene sorbitan fatty acid esters (such as, for example, Tween®), polyoxyethylene fatty acid glycerides (such as, for example, Cremophor®), polyoxethylene fatty acid esters, polyoxyethylene fatty alcohol ethers, glycerol fatty acid esters, poloxamers (such as, for example, Pluronic®),
buffers, acids and bases (for example phosphates, carbonates, citric acid, acetic acid, hydrochloric acid, sodium hydroxide solution, ammonium carbonate, trometamol, triethanolamine),
isotonicity agents (for example glucose, sodium chloride),
adsorbents (for example highly-disperse silicas),
viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol®); alginates, gelatine),
disintegrants (for example modified starch, carboxymethylcellulose-sodium, sodium starch glycolate (such as, for example, Explotab®), cross- linked polyvinylpyrrolidone, croscarmellose-sodium (such as, for example, AcDiSol®)),
flow regulators, lubricants, glidants and mould release agents (for example magnesium stearate, stearic acid, talc, highly-disperse silicas (such as, for example, Aerosil®)),
coating materials (for example sugar, shellac) and film formers for films or diffusion membranes which dissolve rapidly or in a modified manner (for example polyvinylpyrrolidones (such as, for example, Kollidon®), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit®)),
capsule materials (for example gelatine, hydroxypropylmethylcellulose),
synthetic polymers (for example polylactides, polyglycolides, polyacrylates, polymethacrylates (such as, for example, Eudragit®), polyvinylpyrrolidones (such as, for example, Kollidon®), polyvinyl alcohols, polyvinyl acetates, polyethylene oxides, polyethylene glycols and their copolymers and blockcopolymers),
plasticizers (for example polyethylene glycols, propylene glycol, glycerol, triacetine, triacetyl citrate, dibutyl phthalate),
penetration enhancers,
stabilisers (for example antioxidants such as, for example, ascorbic acid, ascorbyl palmitate, sodium ascorbate, butylhydroxyanisole, butylhydroxytoluene, propyl gallate),
preservatives (for example parabens, sorbic acid, thiomersal, benzalkonium chloride, chlorhexidine acetate, sodium benzoate),
colourants (for example inorganic pigments such as, for example, iron oxides, titanium dioxide),
flavourings, sweeteners, flavour- and/or odour-masking agents.

### BIOLOGICAL DEFINITIONS

Unless otherwise defined, all scientific and technical terms used in the description, figures and claims have their ordinary meaning as commonly understood by one of ordinary skill in the art. All publications, patent applications, patents, and other references mentioned herein are incorporated by reference in their entirety. In case of conflict, the present specification, including definitions, will prevail. If two or more documents incorporated by reference include conflicting and/or inconsistent disclosure with respect to each other, then the document having the later effective date shall control. The materials, methods, and examples are illustrative only and not intended to be limiting. Unless stated otherwise, the following terms used in this document, including the description and claims, have the definitions given below.

The terms **"comprising", "including", "containing", "having"** etc. shall be read expansively or open-ended and without limitation.

Singular forms such as **"a", "an"** or **"the"** include plural references unless the context clearly indicates otherwise.

Unless otherwise indicated, the term **"at least"** preceding a series of elements is to be understood to refer to every element in the series. The terms **"at least one"** and **"at least one of"** include for example, one, two, three, four, or five or more elements.

**Mutation numbering** follows the species based positions for each construct, i.e. rat ADAMTS7 SP-Pro (1-217) followed by human ADAMTS7 CD (237-537).

The term **"ADAMTS-7"** (also ADAMTS7, ADAM-TS 7, ADAM-TS7) refers to the protein A disintegrin and metalloproteinase with thrombospondin motifs 7. The ADAMTS-7 protein is encoded by the gene ADAMTS-7. The ADAMTS-7 protein comprises human, murine, rat and further mammalian and non-mamalian homologues. Sequence(s) for human ADAMTS-7 are accessible via UniProt Identifier Q9UKP4 (ATS7_HUMAN), for instance human isoform Q9UKP4-1. Sequence(s) for murine ADAMTS-7 are accessible via UniProt Identifier Q68SA9 (ATS7_MOUSE). Different isoforms, variants and SNPs may exist for the different species and are all comprised by the term ADAMTS-7. Also comprised are ADAMTS-7 molecules before and after maturation, i.e., independent of cleavage of one or more pro-domains. In addition, synthetic variants of the ADAMTS-7 protein may be generated and are comprised by the term ADAMTS-7. The protein ADAMTS-7 may furthermore be subject to various modifications, e.g, synthetic or naturally occurring modifications. Recombinant functional human ADAMTS-7 (e.g. according to SEQ ID No. 01 and 02) can be manufactured as described in the examples.

The term **"ADAMTS-12"** (also ADAMTS12, ADAM-TS 12, ADAM-TS12) refers to the protein A disintegrin and metalloproteinase with thrombospondin motifs 12. Such proteins preferably include a ADAMTS-12 catalytic domain. The ADAMTS-12 protein is encoded by the gene ADAMTS-12. The ADAMTS-12 protein comprises human, murine, rat and further mammalian and non-mamalian homologues. Sequence(s) for human ADAMTS-12 including the catalytic domains are accessible via UniProt Identifier P58397 (ATS12_HUMAN), for instance human isoform P58397-1. Sequence(s) for murine ADAMTS-12 are accessible via UniProt Identifier Q811B3 (ATS12_MOUSE). Different isoforms and variants may exist for the different species and are all comprised by the term ADAMTS-12. Also comprised are ADAMTS-12 molecules before and after maturation, i.e., independent of cleavage of one or more pro-domains. In addition, synthetic variants of the ADAMTS-12 protein may be generated and are comprised by the term ADAMTS-12. The protein ADAMTS-12 may furthermore be subject to various modifications, e.g., synthetic or naturally occurring modifications. Recombinant functional human ADAMTS-12 (e.g., according to SEQ ID NO: 15) can be manufactured as described in the examples.

The terms **"ADAMTS-4"** and **"ADAMTS-5"** refer to the protein A disintegrin and metalloproteinase with thrombospondin motifs 4 and 5, respecitively. The ADAMTS-4 and -5 proteins are encoded by the genes ADAMTS4 and ADAMTS-5, respectively. These proteins comprises human, murine, rat and further mammalian and non-mamalian homologues. Sequence(s) for human ADAMTS-4/-5 are accessible via UniProt Identifier O75173 (ATS4_HUMAN) / Q9UNA0 (ATS5_HUMAN), respectively. Different isoforms and variants may exist. Recombinant active human ADAMTS-4 and ADAMTS-5 can be manufactured as known in the art.

The terms **"MMP2", "MMP12",** and **"MMP15"** refer to the 72 kDa type IV collagenase, Macrophage metalloelastase 2 and 12 and Matrix metalloproteinase-15, respectively. The MMP2, MMP12, and MMP15 proteins are encoded by the genes MMP2, MMP12, and MMP15, respectively. The proteins comprises human, murine, rat and further mammalian and non-mamalian homologues. Sequence(s) for human ADAMTS-4/-5 are accessible via UniProt Identifier P08253 (MMP2_HUMAN), P39900 (MMP12_HUMAN) and P51511 (MMP15_HUMAN), respectively. Different isoforms and variants may exist. Recombinant active human ADAMTS-4 and ADAMTS-5 can be manufactured as known in the art.

The term **"ADAM17"** refers to Disintegrin and metalloproteinase domain-containing protein 17, encoded by the gene ADAM17. The protein comprises human, murine, rat and further mammalian and non-mamalian homologues. Sequence(s) for human ADAM17 are accessible via UniProt Identifier P78536 (ADA17_HUMAN). Different isoforms and variants may exist. Recombinant active human ADAM17 can be manufactured as known in the art.

The term **"prodomain"** includes parts of ADAMTS-7 or ADAMTS-12 that are relatively N-terminal to the respective protein's functional chain (e.g., parts having metalloprotease function and disintergrin motifs). For example, a prodomain of ADAMTS-7 as provided in SEQ ID NO: 1 can include its signal peptide (residues 1-20) and its propeptide (residues 21-217), both of which are N-terminal to its peptidase domain, although not necessarily immediately N-terminal to it. In some embodiments, prodomain of ADAMTS-7 or ADAMTS-12 includes 75%, 80%, 85%, 90%, 95%, or 100% of the N-terminal part of the respective protein with its signal peptide plus its propeptide. The term "prodomain" also encompasses the parts of the encoded polypeptide that are processed (e.g., cleaved off) before generation of the functional enzymatic chain in the natural environment of the enzyme.

A **"furin cleavage site"** or **furin consensus site** is R-x-K/R-R↓ D/S, cf. Shiryaev 2013 PLoS One. The ADAMTS7 prodomain contains multiple Furin protease cleavage sites, the last of which is thought to fully process the zymogen into the active form. Mutational analysis was described by Sommerville 2004 JBC for rat ADAMTS7 with R60A and R217A (referred to as mouse R220A in publication). R60A changes rat ADAMTS7 from LRKR↓D to LRKA↓D and R217A changes rat ADAMTS7 RQQR↓S to RQQA↓S.

The term **"catalytic domain"** includes parts of ADAMTS-7 or ADAMTS-12 that have ADAMTS-7 or ADAMTS-12 functionality, respectively, and that are C-terminal to the respective protein's prodomain. In some embodiments, the term "catalytic domain" refers to the peptidase plus disintegrin part of the respective protein (e.g., as characterized by UniProt), potentially also including any residues C-terminal to the respective protein's prodomain and N-terminal to the respective protein's peptidase domain. In some embodiments, the catalytic domain includes 75%, 80%, 85%, 90%, 95%, or 100% of the part of the respective enzyme having its disintegrin domain, its peptidase domain, and any residues it might have between its prodomain and its peptidase domain.

The term **"functional protein"** refers to a protein which has biological activity. For example, functional ADAMTS-7 refers to ADAMTS-7 which is able to catalyze the proteolytic cleavage of its (natural) substrate(s), e.g. TSP1 and/or COMP.

The term **"metalloproteinase"** refers to a protease enzyme whose catalytic mechanism involves a metal. Therefore a functional metalloproteinase is a functional protein, wherein the protein is a protease and wherein the protease is a metalloproteinase according to the foregoing definitions.

The expression "a **cleavage site for a protease"** refers to any peptide or protein sequence which is recognized and cleaved by the functional protease. A cleavage site for ADAMTS-7 thus refers to any peptide or protein sequence which is recognized and cleaved by functional ADAMTS-7. For example, being natural substrates of ADAMTS-7, the sequences of proteins COMP and TSP1 both comprise cleavage sites for ADAMTS-7. In particular the subsequence DELSSMVLELRGLRT (derived from TSP1, residues 275 - 289) constitutes or comprises a cleavage site for ADAMTS-7 and ADAMTS-12.

A **"substrate"** is a molecule upon which an enzyme acts. For example, the substrate of a proteinase can be a peptide or protein or derivative thereof, which is cleaved by the proteinase. Metalloproteinase paralogs ADAMTS-7/-12 on the one hand and their common peptide substrate on the other hand are interrelated in the sense of a plug and socket relationship.

The term **"COMP",** TSP-5 or TSP5 refers to the protein Cartilage oligomeric matrix protein. The COMP protein is encoded by the gene COMP. The COMP protein comprises human, murine, rat and further mammalian and homologues. Sequence(s) for human COMP are accessible via UniProt Identifier P49747 (COMP_HUMAN), for instance human isoform P49747-1. Sequence(s) for murine COMP are accessible via UniProt Identifier Q9R0G6 (COMP_MOUSE). Different isoforms and variants may exist for the different species and are all comprised by the term COMP. Also comprised are COMP molecules before and after maturation, i.e., independent of cleavage of one or more pro-domains. In addition, synthetic variants of the COMP protein may be generated and are comprised by the term COMP. The protein COMP may furthermore be subject to various modifications, e.g, synthetic or naturally occurring modifications. Recombinant human COMP or derivatives thereof can be manufactured as described in the examples.

The term **"TSP1"** (also THBS1 or TSP) refers to the protein Thrombospondin-1. The TSP1 protein is encoded by the gene THBS1. The TSP1 protein comprises human, murine, rat and further mammalian and non-mammalian homologues. Sequence(s) for human TSP1 are accessible via UniProt Identifier P07996 (TSP1_HUMAN), for instance human isoform P07996-1. Sequence(s) for murine TSP1 are accessible via UniProt Identifier P35441 (TSP1_MOUSE). Different isoforms and variants may exist for the different species and are all comprised by the term TSP1. Also comprised are TSP1 molecules before and after maturation, i.e., independent of cleavage of one or more pro-domains. In addition, synthetic variants of the TSP1 protein may be generated and are comprised by the term TSP1. The protein TSP1 may furthermore be subject to various modifications, e.g, synthetic or naturally occurring modifications. Recombinant human TSP1 or derivatives thereof can be manufactured as described in the examples.

The term **"fluorophore"** according to the current invention refers to a molecule or chemical group which has the ability to absorb energy from light, transfer this energy internally, and emit this energy as light of a characteristic wavelength. Without being bound by theory, since some energy is lost during this process, the energy of the emitted fluorescence light is lower than the energy of the absorbed light, and therefore emission occurs at a longer wavelength than absorption. A variety of fluorophores and quenchers has been described in the art and can be used according to the current invention, see for example Bajar et al, Sensors 2016, A Guide to Fluorescent Protein FRET Pairs.

The term **"quencher"** according to the current invention refers to a molecule or chemical group which has the ability to decrease the fluorescence intensity of a given fluorophore. Without being bound by theory a variety of processes can result in quenching, such as excited state reactions, energy transfer, complex-formation and collisional quenching. Dark quenchers are dyes with no native fluorescence. Quencher fluorescence can increase background noise due to overlap between the quencher and reporter fluorescence spectra. A variety of fluorophores and quenchers has been described in the art and can be used according to the current invention, see for example Bajar et al, Sensors 2016, A Guide to Fluorescent Protein FRET Pairs. Suitable quenchers according to the current invention include DDQ-I A (430 nm), Dabcyl (475 nm), Eclipse B (530 nm), Iowa Black FQ C (532 nm), BHQ-1D (534 nm), QSY-7 E (571 nm), BHQ-2 D (580 nm), DDQ-II A (630 nm), Iowa Black RQ C (645 nm), QSY-21 E (660 nm) or BHQ-3 D (670 nm). Preferred examples of quenchers according to the current invention include Dabsyl (dimethylaminoazobenzenesulfonic acid), which absorbs in the green spectrum and is often used with fluorescein, black hole quenchers which are capable of quenching across the entire visible spectrum, Qxl quenchers which span the full visible spectrum, Iowa black FQ (absorbs in the green-yellow part of the spectrum), Iowa black RQ (blocks in the orange-red part of the spectrum) and IRDye QC-1 (quenches dyes from the visible to the near-infrared range (500-900 nm)).

Suitable internally quenched fluorescent (IQF) / FRET pairs include ABz-Tyr(NO2), ABz-EDDNP, Trp-Dansyl, and 7-methoxy-coumarin-4-yl acetic acid-2,4-dinitrophenyl-lysine (MCA-Lys(DNP)) (Poreba, Marcin et al., Highly sensitive and adaptable fluorescence-quenched pair discloses the substrate specificity profiles in diverse protease families. Scientific reports 7, 43135. (2017), doi:10.1038/srep43135). Further suitable examples are listed in: Poreba M. & Drag M. Current strategies for probing substrate specificity of proteases. Curr Med Chem 17, 3968-3995 (2010).

The term **"construct"** refers to nucleic acids (e.g., double stranded DNA, which can be in the form of a plasmid).

The term **"align"** in the context of two sequences (e.g., amino acid sequences), includes arranging the two sequences with respect to each other (e.g., the first sequence along a first row, and the second sequence along a second row, potentially with gap(s) in one or both sequences) to obtain a measure of their relationship to each other (e.g., % identity, % similarity, alignment score). A particular alignment of two sequences can be optimal (i.e., there are no alignments of the two sequences that result in a higher alignment score, if alignment score is the metric of concern for optimality) or non-optimal. In addition, a particular alignment can be global or local with respect to either sequence. For example, if the alignment is global with respect to both sequences (i.e., a global-global alignment), then all residues of both of the sequences are factored in to the calculation of the measure of their relationship, including any internal or external gaps in either sequence. In contrast, in a global-local alignment, while all residues and gaps in the first sequence are considered, no external gaps in the second sequence are considered, thereby allowing fitting the first sequence into a part of the second sequence.

The term **"Needleman-Wunsch score"** implies that either a global-global or a global-local (or local-global) alignment has been used to generate the alignment score. When partiality modifiers are used for both the first sequence (e.g., "portion") and the second sequence (e.g., "segment"), this implies that the alignment is global-global. A "Needleman-Wunsch score" includes scores calculated by the originally published method (S.B. Needleman & C.D. Wunsch, A general method applicable to the search for similarities in the amino acid sequence of two proteins, J. Mol. Biol. 48(3):443-53 (1970)) as well as by subsequent refinements of the method. Although Needleman-Wunsch algorithm is able to, and is designed to, find an optimal alignment, and thereby a maximum alignment score, the term "Needleman-Wunsch score" as used here is not restricted to the optimal/maximum score; it can be the alignment score of any alignment of the two sequences as long as at least one of the sequences (e.g., as defined, for example as a residue range) is considered globally.

Alignment methods (e.g., Needleman-Wunsch) that generate an alignment score (e.g., Needleman-Wunsch score) can make use of a substitution matrix. A particular substitution matrix that can be used is BLOSUM62, which is reproduced below in Table B1.

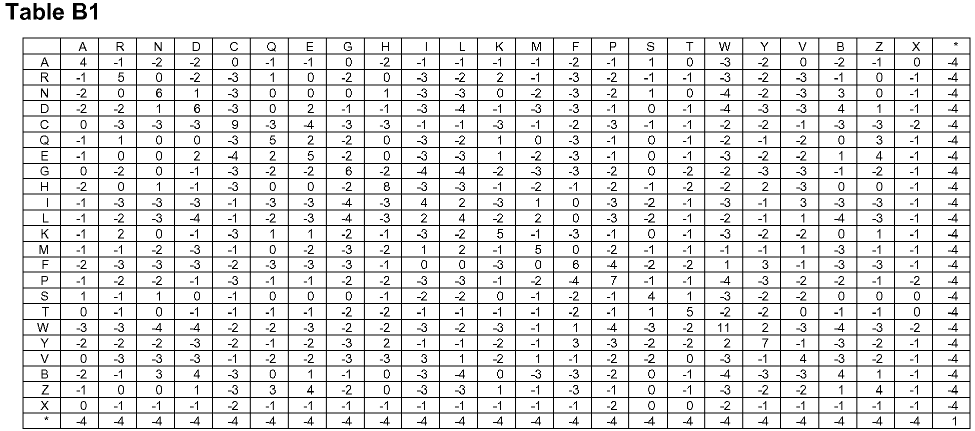

In this table, the first 20 columns (and the first 20 rows) labelled with single-letter amino acid codes represent the standard amino acids. The remaining columns/rows represent additional residue types (e.g., "B" for Asx (Asn/Asp), "Z" for Glx (Gln/Glu), "X" for any amino acid, and "*" for a translation stop encoded by a termination codon). If the sequences of interest have only a subset of these residues, then a corresponding sub-matrix of BLOSUM62 can also be alternatively sufficient for aligning them (e.g., the scores from the upper-left 20x20 part of the scores if only standard amino acids that are singly-identified are of concern).

As a demonstration of using BLOSUM62 in calculating an alignment score, if residues 1-8 of SEQ ID NO: 14 ("EEVKAKVQ") were aligned with themselves, the alignment score would be the sum of scores for each of those amino acids pairing with itself (e.g., when residue "A" in the first sequence pairs with itself, an "A," in the second sequence, it contributes a score of 4, as seen in the cell at row 2, column 2 of the BLOSUM62 matrix). Therefore, the alignment score in this hypothetical example would be 37 (5+5+4+5+4+5+4+5). If the "A" in the second sequence, in this hypothetical, is changed into a "W," the alignment score would be 30 (5+5+4+5-3+5+4+5). If the "E" at residue one, or the "K" at residue four, or the "Q" at residue eight is deleted in the second sequence instead, in this hypothetical, the alignment score in each case would be 20 (the original total score of 37 is lessened by 5 due to the deleted residue, and there is an additional total gap penalty of 12 in this case, as explained further next). The total gap penalty is calculated by summing a gap extension penalty as multiplied by the number of residues in the gap with a gap-opening penalty. As an example, a gap-opening penalty of 11 and a gap extension penalty of 1 can be used. In that case, a single gap as in the last hypothetical example would result in a total gap penalty of 12 (11 + 1^{∗}1), regardless of whether the gap is internal or external, which can be the case for global-global alignments. If the gap were three amino acids long in the middle of a sequence, the total gap penalty would be 14 (11 + 1^{∗}3), which would be subtracted from of the scores of the aligning residues (identical as well as non-identical) to arrive at the alignment score.

Using a substitution matrix such as BLOSUM62 is superior to cruder methods such as percent identity calculations, at least because different aligned identical residues can give different contributions to the overall score depending on how rare or common themselves or their mutations are (e.g., a W:W alignment contributes 11, as seen in Table B1, while an A:A alignment contributes only 4). In addition, mutations into different residues can also be treated differently with a substitution matrix (e.g., a D:E change has a positive score, 2, as seen in Table B1, while a D:L change has a negative score, -4, whereas each of these changes would be clumped as the same non-identical change in a percent-identity approach). Overall, using a substitution matrix like BLOSUM62 provides a dramatically more sensitive measure for inferring sequence relatedness than percent identity methods, since the matrix allows calibrating the score of changing each of the amino acids (e.g., 20) into each of the amino acids (e.g., 20) individually, while with percent identity the changes are limited to merely identical and non-identical ones. As a result, a polypeptide sequence defined with respect to its alignment to a reference sequence in terms of a Needleman-Wunsch score obtained by using BLOSUM62 matrix is significantly more likely to have structural features, physical properties, and functional features in common with the polypeptide of the reference sequence.

For percent sequence identity values, the same alignment method (e.g., Needleman-Wunsch algorithm for global-global alignment, using BLOSUM62 matrix, with gap opening penalty of 11 and a gap extension penalty of 1) can be used to obtain the alignment, after which the pairs of aligned identical residues can be counted and then divided by the total length of the alignment (including gaps, internal as well as external) to arrive at the percent identity value. For percent similarity values, the same approach as for percent identity values can be used, except that what is counted, instead of pairs of identical residues, would be the aligned residue pairs with BLOSUM62 values that are not negative (i.e., ≥0).

Numerous programs as well as websites exist for calculation of alignment scores. For example, executables for local use can be downloaded from the UVa FASTA Server (available from World Wide Web at fasta.bioch.virginia.edu/fasta_www2/fasta_list2.shtml). Among the programs available at the UVa FASTA server, ggsearch can perform global-global alignments (e.g., using Needleman-Wunsch algorithm, and BLOSUM62 matrix with gap opening penalty of 11 and a gap extension penalty of 1) and glsearch can perform global-local alignments. Similar functionality is also available through an online submission form at the same server (e.g., World Wide Web at fasta.bioch.virginia.edu/fasta_www2/fasta_www.cgi?rm=select&pgm=gnw, after selecting "Align two sequences" to align two sequences rather than one sequence against a database). Additional online sources for similar functionality include the National Center for Biotechnology Information (available through the World Wide Web at https://blast.ncbi.nlm.nih .gov/Blast.cgi) and the European Bioinformatics Institute of the European Molecular Biology Laboratory (available through the World Wide Web at https://www.ebi.ac.uk/Tools/sss/fasta/).

### BIOLOGICAL EMBODIMENTS

According to a **first biological aspect** of the current invention, there is provided a recombinant nucleic acid sequence for the improved expression and purification of functional ADAMTS-7.

According to some first embodiments according to the first biological aspect, there is provided a recombinant nucleic acid for expression of an ADAMTS-7 polypeptide that comprises a rodent prodomain of ADAMTS-7 as a first portion and a functional human ADAMTS-7 as a second portion.

Commercial expression and purification of recombinant functional ADAMTS-7 has so far been a challenge (cf. figure 3), rendering the development of an assay for the identification and characterization of modulators of ADAMTS-7 extremely difficult. For example, as shown in figure 3, expression of hADAMTS-7 (residues 237-537) or hADAMTS-7 Pro-CD-TSR1 (residues 1-593) yielded little soluble proteins. The nucleic acids according to the first biological aspect solved the problem of protein solubility as well as expression yield, both of which were lower for fully human ADAMTS-7 constructs compared to hybrid constructs (cf. example B2).

The constructs according to the first biological aspect are suited for the production of sufficient amounts of ADAMTS-7 with reproducible activity and purity. The resulting recombinant functional ADAMTS-7 can therefore be used in an assay for the identification and characterization of modulators of ADAMTS-7.

In order to obtain a suitable construct for the expression of functional ADAMTS-7, more than 50 *E. coli* constructs, 20 constructs from *Baculovirus* and 30 *HEK293* constructs were designed and evaluated to achieve the efficient expression of functional ADAMTS-7. The recombinant nucleic acid according to the first biological aspect surprisingly solved these problems and enabled the efficient expression and purification of functional ADAMTS-7 protein: In particular it was surprisingly found that a rodent prodomain is more effective in driving folding of the catalytic domain of human ADAMTS-7, thereby improving the yield of the soluble ADAMTS-7 proteins -10 fold (see example B2).

In some embodiments according to the first biological aspect, the recombinant nucleic acid sequence encodes for a recombinant polypeptide that comprises a first portion and a second portion, wherein the first portion has a sequence identity of > 80 % with the sequence of residues 1-217 of SEQ ID NO: 1 or with the sequence of residues 1-217 of SEQ ID NO: 2, and the second portion has a sequence identity of > 80 % with the sequence of residues 218-518 of SEQ ID NO: 1.

In some embodiments according to the first biological aspect, the recombinant nucleic acid sequence encodes for a recombinant polypeptide that comprises a first portion and a second portion, wherein the first portion has a sequence identity of > 90 % with the sequence of residues 1-217 of SEQ ID NO: 1 or with the sequence of residues 1-217 of SEQ ID NO: 2, and the second portion has a sequence identity of > 90 % with the sequence of residues 218-518 of SEQ ID NO: 1.

In some embodiments according to the first biological aspect, the recombinant nucleic acid sequence encodes for a recombinant polypeptide that comprises a first portion and a second portion, wherein the first portion has a sequence identity of > 95 % with the sequence of residues 1-217 of SEQ ID NO: 1 or with the sequence of residues 1-217 of SEQ ID NO: 2, and the second portion has a sequence identity of > 95 % with the sequence of residues 218-518 of SEQ ID NO: 1.

In some embodiments according to the first biological aspect, the recombinant nucleic acid sequence encodes for a recombinant polypeptide that comprises a first portion and a second portion, wherein the first portion has a sequence identity of > 98 % with the sequence of residues 1-217 of SEQ ID NO: 1 or with the sequence of residues 1-217 of SEQ ID NO: 2, and the second portion has a sequence identity of > 98 % with the sequence of residues 218-518 of SEQ ID NO: 1.

In some embodiments according to the first biological aspect, the recombinant nucleic acid sequence encodes for a recombinant polypeptide that comprises a first portion and a second portion, wherein the first portion comprises residues 1-217 of SEQ ID NO: 1 or residues 1-217 of SEQ ID NO: 2, and/or the second portion comprises residues 218-518 of SEQ ID NO: 1 (cf. example B1). In some embodiments the second portion is C-terminal to the first portion.

In some embodiments according to the first biological aspect, the recombinant nucleic acid sequence encodes for a polypeptide that has a first portion and a second portion. The first portion of the polypeptide, in some embodiments, has an amino acid sequence that when aligned with an amino acid sequence of a ADAMTS-7 prodomain or a fragment thereof from a first species generates a Needleman-Wunsch score greater than 700, if BLOSUM62 matrix, a gap opening penalty of 11, and a gap extension penalty of 1 are used. In some embodiments, this generated Needleman-Wunsch score is greater than 750, 800, 850, 900, 950, 1000, 1050, or 1100. In some embodiments, the first portion and the ADAMTS-7 prodomain share a sequence identity that is at least 70%, 75%, 80%, 85%, 90%, 95%, 97% or 98%. The second portion of the polypeptide, in some embodiments, has an amino acid sequence that when aligned with an amino acid sequence of a ADAMTS-7 catalytic domain or a fragment thereof from a second species generates a Needleman-Wunsch score greater than 1000 if BLOSUM62 matrix, a gap opening penalty of 11, and a gap extension penalty of 1 are used. In some embodiments, this generated Needleman-Wunsch score is greater than 1050, 1100, 1150, 1200, 1250, 1300, 1350, 1400, 1450, 1500, 1550, 1600, or 1650. In some embodiments, the second portion and the ADAMTS-7 catalytic domain share a sequence identity that is at least 70%, 75%, 80%, 85%, 90%, 95%, 97% or 98%.

In some embodiments according to the first biological aspect, the recombinant nucleic acid sequence encodes for a recombinant polypeptide that comprises a first portion having an amino acid sequence that aligns with an amino acid sequence of an ADAMTS-7 prodomain or a fragment thereof from a first species with a Needleman-Wunsch score greater than 700, when BLOSUM62 matrix, a gap opening penalty of 11, and a gap extension penalty of 1 are used; and a second portion having an amino acid sequence that aligns with an amino acid sequence of a ADAMTS-7 catalytic domain or a fragment thereof from a second species with a Needleman-Wunsch score greater than 1000 when BLOSUM62 matrix, a gap opening penalty of 11, and a gap extension penalty of 1 are used.

In some embodiments, the first species is a non-human species, such as a rodent species, such as rat. In some different or the same embodiments the second species is human, e.g. the catalytic domain is derived from human ADAMTS-7. In some embodiments the first species is a rodent species and the second species is human.

In some embodiments according to the first biological aspect the first portion of the polypeptide comprises a mutation within the furin cleavage site. Figure 4 shows that furin cleavage site mutants of ADAMTS-7 improved the yield of processed or unprocessed ADAMTS-7. For example, in some of these embodiments the motif RQQR is mutated to RQKR (Q216K). In some embodiments, the motif RQQR within the first portion of the polypeptide is altered, preferably into RQKR. Thus, in some embodiments the first portion comprises a mutation, e.g. at position 216, such as the mutation Q216K. This mutation was found to improve cleavage by Furin between the prodomain and the catalytic domain of ADAMTS-7, thereby leading to improved yields of processed ADAMTS-7 compared to the wild type (WT). (cf. example B1, cf. figure 4). Q216K mutation changes rat ADAMTS7 motif RQQR↓S to RQKR↓S, i.e. to a more optimized consensus to increase Furin processing of the zymogen into the active catalytic form.

In some embodiments the first portion comprises triple mutant R58A/R61A/R217A (rPro-hCD-3RA). Figure 4 panel B shows that these mutations abolished the processing to generate unprocessed protein only. For example, R58A/R60A/R217A prevents all furin cleavage resulting in a zymogen form.

According to some embodiments of the first biological aspect the recombinant nucleic acid sequence encodes at least for a rat pro-domain of ADAMTS-7 (SEQ ID No. 1, residues 1-217) and a catalytic domain of human ADAMTS-7, wherein optionally within the rat pro-domain of ADAMTS-7 the motif RQQR is mutated to RQKR (SEQ ID No. 2, cf. residues 1-217). In some of these embodiments said rat pro-domain of ADAMTS-7 comprises or consists of a sequence according to SEQ ID No. 1, residues 1-217 or SEQ ID No. 2, residues 1-217 and/or said catalytic domain of human ADAMTS-7 comprises or consists of a sequence according to SEQ ID No. 1, residues 218-518 or SEQ ID No. 2, residues 218-518.

In some embodiments the polypeptide or a fragment thereof encoded by the recombinant nucleic acid is suited to cleave a peptide comprising standard residues 1-15 of the amino acid sequence of SEQ ID NO: 4. For example, the polypeptide is suited to cleave a peptide comprising standard residues 1-15 of the amino acid sequence of SEQ ID NO: 4 and/or SEQ ID No. 11 with a a kcat/KM of at least 20% of a corresponding kcat/KM of human ADAMTS-7.

The produced polypeptide (e.g., having both the first portion and the second portion, or having only the second portion) can have a catalytic activity close to that of human ADAMTS-7 enzyme, e.g., in terms of kcat / KM, which can be even higher than the kcat / KM of the human enzyme, or can be within 50%, 45%, 40%, 35%, 30%, 25%, 20%, 15%, 10%, or 5% of the kcat / KM of the human enzyme, for example when a peptide having the sequence of of SEQ ID No. 11 is used as the substrate.

In some embodiments, for a given substrate, the produced polypeptide (e.g., having both the first portion and the second portion, or having only the second portion) has a catalytic activity in the same order of magnitude as rat ADAMTS-7, e.g., in terms of kcat, cf. figures 8, 9. In some embodiments, for a substrate of SEQ ID No. 4, the produced polypeptide has a kcat (min⁻¹) in the order of magnitude of 10⁻². In some embodiments, for a substrate of SEQ ID No. 5, the produced polypeptide has a kcat (min⁻¹) in the order of magnitude of 10⁻³.

Sequential cleavage or processing of ADAMTS-7 at furin cleavage sites by cellular enzyme furin leading to a complete removal of the Pro domain from the rest of the protein is likely a necessary step to a fully active or mature ADAMTS-7.

In some embodiments the recombinant nucleic acid sequence furthermore encodes for additional residues, such as a purification tag, such as a FLAG tag, a His tag, a Strep tag or any combination or repetition thereof. In some embodiments of the first biological aspect, the encoded polypeptide can have additional residues (e.g., purification tags such as His tag, Strep tag, 2xStrep tag, FLAG tag, 3xFLAG tag, and cleavage sequences such as TEV cleavage site). The presence of these additional resiudes is compatible with each of the described embodiments of the biological aspect. Purification of a polypeptide obtained from a nucleic acid according to the current invention can occur as described in example B3.

The recombinant nucleic acid according to the first biological aspect can be used for the expression of functional ADAMTS7 as described in example B3. For example, the construct can be inserted into a plasmid which is compatible with a certain expression system. In certain preferred embodiments, the construct can be cloned into a plasmid, preferably into a mammalian expression vector, such as pcDNA6mycHis. Expression can be performed in a compatible expression system, such as in a mammalian expression system, such as in HEK cells or Expi293 cells as known in the art. The Gibco Expi293 Expression System is a commercially available high-yield transient expression system based on suspension-adapted Human Embryonic Kidney (HEK) cells.

According to a **second biological aspect** of the current invention, there is provided a recombinant nucleic acid for the expression of functional ADAMTS-12. It was surprisingly found that human ADAMTS-12 expression is improved when a non human prodomain is used (cf. example B9, cf. figure 6). In particular it was found that human ADAMTS-12 with a rodent prodomain demonstrated a better expression profile compared to human ADAMTS-12 with a human prodomain. The recombinant nucleic acids according to the second biological aspect are therefore suited for the improved production of sufficient amounts of ADAMTS-12 with reproducible activity and purity. The resulting recombinant functional ADAMTS-12 can be used in an assay for the characterization of modulators of ADAMTS-7, i.e. where the selectivity for ADAMTS-7 is assessed.

According to some first embodiments according to the second biological aspect, there is provided a recombinant nucleic acid for expression of an ADAMTS-12 polypeptide that comprises a rodent prodomain of ADAMTS-12 as a first portion and a functional human ADAMTS-12 as a second portion.

In some embodiments according to the second biological aspect, the recombinant nucleic acid sequence encodes for a recombinant polypeptide that comprises a first portion and a second portion, wherein the first portion has a sequence identity of > 80 % with the sequence of residues 1-244 of SEQ ID NO: 15, and the second portion has a sequence identity of > 80 % with the sequence of residues 245-547 of SEQ ID NO: 15 (cf. 241 - 544 of ADAMTS12).

For example, ADAMTS12 rat/human hybrid can be made by fusing respective rat ADAMTS12 SP-Pro (1-244) followed by human ADAMTS12 CD (241-544).

In some embodiments according to the second biological aspect, the recombinant nucleic acid sequence encodes for a recombinant polypeptide that comprises a first portion and a second portion, wherein the first portion has a sequence identity of > 90 % with the sequence of residues 1-244 of SEQ ID NO: 15, and the second portion has a sequence identity of > 90 % with the sequence of residues 245-547 of SEQ ID NO: 15.

In some embodiments according to the second biological aspect, the recombinant nucleic acid sequence encodes for a recombinant polypeptide that comprises a first portion and a second portion, wherein the first portion has a sequence identity of > 95 % with the sequence of residues 1-244 of SEQ ID NO: 15, and the second portion has a sequence identity of > 95 % with the sequence of residues 245-547 of SEQ ID NO: 15.

In some embodiments according to the second biological aspect, the recombinant nucleic acid sequence encodes for a recombinant polypeptide that comprises a first portion and a second portion, wherein the first portion has a sequence identity of > 98 % with the sequence of residues 1-244 of SEQ ID NO: 15, and the second portion has a sequence identity of > 98 % with the sequence of residues 245-547 of SEQ ID NO: 15.

In some embodiments, according to the second biological aspect, the recombinant nucleic acid sequence encodes for a recombinant polypeptide that comprises a first portion and a second portion, wherein the first portion comprises residues 1-244 of SEQ ID NO: 15, and/or the second portion comprises residues 245-547 of SEQ ID NO: 15. In some different or the same embodiments the second portion is immediately C-terminal to the first portion.

In some embodiments according to the second biological aspect, the recombinant nucleic acid encodes a polypeptide that has a first portion and a second portion. The first portion of the polypeptide, in some embodiments, has an amino acid sequence that when aligned with an amino acid sequence of an ADAMTS-12 prodomain (or a fragment thereof) from a first species generates a Needleman-Wunsch score greater than 800 if BLOSUM62 matrix, a gap opening penalty of 11, and a gap extension penalty of 1 are used. In some embodiments, this generated Needleman-Wunsch score is greater than 850, 900, 950, 1000, 1050, 1100, 1150, 1200, 1250, or 1300. In some embodiments, the first portion and the ADAMTS-12 prodomain share a sequence identity that is at least 70%, 75%, 80%, 85%, 90%, 95%, 97% or 98%. The second portion of the polypeptide, in some embodiments, has an amino acid sequence that when aligned with an amino acid sequence of a human ADAMTS-12 catalytic domain (or a fragment thereof) generates a Needleman-Wunsch score greater than 1000 if BLOSUM62 matrix, a gap opening penalty of 11, and a gap extension penalty of 1 are used. In some embodiments, this generated Needleman-Wunsch score is greater than 1050, 1100, 1150, 1200, 1250, 1300, 1350, 1400, 1450, 1500, 1550, 1600, or 1650. In some embodiments, the second portion and the ADAMTS-12 catalytic domain share a sequence identity that is at least 70%, 75%, 80%, 85%, 90%, 95%, 97% or 98%.

In some embodiments according to the second biological aspect, the recombinant nucleic acid for expression of an ADAMTS-12 polypeptide encodes for a recombinant polypeptide that comprises a first portion having an amino acid sequence that aligns with an amino acid sequence of a ADAMTS-12 prodomain or a fragment thereof from a non-human species with a Needleman-Wunsch score greater than 800 when BLOSUM62 matrix, a gap opening penalty of 11, and a gap extension penalty of 1 are used; and a second portion having an amino acid sequence that aligns with an amino acid sequence of a ADAMTS-12 catalytic domain or a fragment thereof from a second species with a Needleman-Wunsch score greater than 1000 when BLOSUM62 matrix, a gap opening penalty of 11, and a gap extension penalty of 1 are used.

In some embodiments, the first species is a non-human species, such as a rodent species, such as rat. In some different or the same embodiments the second species is human, e.g. the catalytic domain is derived from human ADAMTS-12. In some embodiments the first species is a rodent species and the second species is human.

In some embodiments the second portion comprises a mutation at position E393, such as E393Q (EQ). This mutation results in increased protein yield similar to ADAMTS-7 catalytic mutations. Mutation numbering E393Q follows the species based positions for the human ADAMTS12 region of the construct, i.e. rat ADAMTS12 SP-Pro (1-244) followed by human ADAMTS12 CD (241-544).

In some embodiments the polypeptide or a fragment thereof encoded by the recombinant nucleic acid is suited to cleave a peptide comprising standard residues 1-15 of the amino acid sequence of SEQ ID NO: 4. For example, in some embodiments the polypeptide is suited to cleave a peptide comprising standard residues 1-15 of the amino acid sequence of SEQ ID NO: 4 and/or SEQ ID No. 11 with a a kcat/KM of at least 20% of a corresponding kcat/KM of human ADAMTS-12.

The produced polypeptide (e.g., having both the first portion and the second portion, or having only the second portion) can have a catalytic activity close to that of human ADAMTS-12 enzyme (e.g., in terms of kcat / KM, which can be even higher than the kcat / KM of the human enzyme, or can be within 50%, 45%, 40%, 35%, 30%, 25%, 20%, 15%, 10%, or 5% of the kcat / KM of the human enzyme, for example when a peptide having the sequence of of SEQ ID No. 4 or 11 is used as the substrate.

In some embodiments the recombinant nucleic acid sequence furthermore encodes for additional residues such as a purification tag, such as a FLAG tag, a His tag, a Strep tag or any combination or repetition thereof. In some embodiments of the second biological aspect, the encoded polypeptide can have additional residues (e.g., purification tags such as His tag, Strep tag, 2xStrep tag, FLAG tag, 3xFLAG tag, and cleavage sequences such as TEV cleavage site). The presence of these additional resiudes is compatible with each of the described embodiments of the biological aspect. Purification of the functional ADAMTS-12 obtained from a recombinant nucleic acid according to the current invention can occur as described in example B4.

According to some embodiments of the second biological aspect the recombinant nucleic acid sequence encodes at least for a rat pro-domain of ADAMTS-12, such as amino acids 1-244 of rat sequence UniProt D3ZTJ3 and/or encodes for a catalytic domain of human ADAMTS-12, such as amino acids 241-543 of human sequence UniProt P58397, cf. example B1. In certain preferred embodiments, said recombinant nucleic acid encodes for a sequence according to SEQ ID No. 15.

The recombinant nucleic acid according to the second biological aspect can be used for the expression of functional ADAMTS-12 as described in example B4. For example, the recombinant nucleic acid can be inserted into a plasmid which is compatible with a certain expression system. In certain preferred embodiments, the construct can be inserted into a plasmid, preferably into a mammalian expression vector, such as pcDNA3.4. Expression can be performed in a compatible expression system, such as in a mammalian expression system, such as in HEK cells or Expi293 cells as known in the art. The Gibco Expi293 Expression System is a commercially available high-yield transient expression system based on suspension-adapted Human Embryonic Kidney (HEK) cells.

According to a **third biological aspect** of the current invention, there is provided a recombinant polypeptide, wherein the recombinant polypeptide is the recombinant polypeptide encoded by a recombinant nucleic acid according to the first or second biological aspect, or a fragment thereof. In some embodiments the fragment is the processed polypeptide which results after Furin cleavage. In some embodiments, the Furin cleavage occurs at the site known in the art or described herein.

In some embodiments the recombinant polypeptide according to the third biological aspect or a fragment thereof is suited to cleave a peptide substrate comprising standard residues 1-15 of SEQ ID NO: 4. The recombinant polypeptide according to the third biological aspect or a fragment thereof can be used in an assay for the identification and characterization of modulators of ADAMTS-7 and/or ADAMTS-12, as shown within the examples.

In some embodiments, the recombinant polypeptide according to the third biological aspect or a fragment thereof is a functional ADAMTS-7 protein. In some embodiments the polypeptide comprises a rodent prodomain of ADAMTS-7 as a first portion and a functional human ADAMTS-7 as a second portion.

In some embodiments the polypeptide comprises a first portion and a second portion, wherein the first portion has a sequence identity of > 80 % with the sequence of residues 1-217 of SEQ ID NO: 1 or with the sequence of residues 1-217 of SEQ ID NO: 2, and the second portion has a sequence identity of > 80 % with the sequence of residues 218-518 of SEQ ID NO: 1.

In some embodiments according to the third biological aspect, the recombinant polypeptide comprises a first portion having an amino acid sequence that aligns with an amino acid sequence of an ADAMTS-7 prodomain or a fragment thereof from a first species with a Needleman-Wunsch score greater than 700, when BLOSUM62 matrix, a gap opening penalty of 11, and a gap extension penalty of 1 are used; and a second portion having an amino acid sequence that aligns with an amino acid sequence of a ADAMTS-7 catalytic domain or a fragment thereof from a second species with a Needleman-Wunsch score greater than 1000 when BLOSUM62 matrix, a gap opening penalty of 11, and a gap extension penalty of 1 are used. In some embodiments the first species is a rodent species such as rat and the second species is human.

In some embodiments the first portion of the polypeptide comprises a mutation within the furin cleavage site. In some embodiments, the motif RQQR within the first portion of the polypeptide is altered, preferably into RQKR. Thus, in some embodiments the first portion comprises a mutation, e.g. at position 216, such as the mutation Q216K. This mutation was found to improve cleavage by Furin between the prodomain and the catalytic domain of ADAMTS-7, thereby leading to improved yields of processed ADAMTS-7 compared to the wild type (WT). (cf. example B1, cf. figure 4).

In some embodiments the polypeptide comprises a rodent prodomain of ADAMTS-12 as a first portion and a functional human ADAMTS-12 as a second portion.

In some embodiments the polypeptide comprises a first portion and a second portion, wherein the first portion has a sequence identity of > 80 % with the sequence of residues 1-244 of SEQ ID NO: 15, and the second portion has a sequence identity of > 80 % with the sequence of residues 245-547 of SEQ ID NO: 15.

In some embodiments according to the current biological aspect, the polypeptide comprises a first portion having an amino acid sequence that aligns with an amino acid sequence of a ADAMTS-12 prodomain or a fragment thereof from a first species with a Needleman-Wunsch score greater than 800 when BLOSUM62 matrix, a gap opening penalty of 11, and a gap extension penalty of 1 are used; and a second portion having an amino acid sequence that aligns with an amino acid sequence of a ADAMTS-12 catalytic domain or a fragment thereof from a second species with a Needleman-Wunsch score greater than 1000 when BLOSUM62 matrix, a gap opening penalty of 11, and a gap extension penalty of 1 are used. In some embodiments, the first species is a non-human species, such as a rodent species, such as rat. In some different or the same embodiments the second species is human, e.g. the catalytic domain is derived from human ADAMTS-12. In some embodiments the first species is a rodent species and the second species is human.

In some embodiments the second portion comprises a mutation at position E393, such as E393Q (EQ). This mutation results in increased protein yield.

In some embodiments the polypeptide (e.g., having both the first portion and the second portion, or having only the second portion) has a catalytic activity close to that of human ADAMTS-7 or ADAMTS-12 enzyme (e.g., in terms of kcat / KM, which can be even higher than the kcat / KM of the human enzyme, or can be within 50%, 45%, 40%, 35%, 30%, 25%, 20%, 15%, 10%, or 5% of the kcat / KM of the human enzyme, for example when a peptide having the sequence of of SEQ ID No. 4 or 11 is used as the substrate.

In some embodiments the polypeptide or a fragment thereof is suited to cleave a peptide comprising standard residues 1-15 of the amino acid sequence of SEQ ID NO: 4. In some embodiments, the polypeptide is suited to cleave a peptide comprising standard residues 1-15 of the amino acid sequence of SEQ ID NO: 4 and/or SEQ ID No. 11 with a a kcat/KM of at least 20 % of a corresponding kcat/KM of human ADAMTS-7 or human ADAMTS-12.

In some embodiments the polypeptide comprises a purification tag, such as a FLAG tag, a His tag, a Strep tag or any combination or repetition thereof. In some embodiments of the first biological aspect, the encoded polypeptide can have additional residues (e.g., purification tags such as His tag, Strep tag, 2xStrep tag, FLAG tag, 3xFLAG tag, and cleavage sequences such as TEV cleavage site). The presence of these additional resiudes is compatible with each of the described embodiments of the biological aspect.

Surprisingly, the recombinant polypeptide according to the current biological aspect was found to fold into a functional ADAMTS, e.g. ADAMTS-7 or ADAMTS-12. Expression and purification of recombinant functional ADAMTS protein according to the third biological aspect can occur as described in example B3.

According to a **fourth biological aspect** of the current invention there is provided a recombinant peptide substrate for ADAMTS-7 and/or ADAMTS-12. The peptide substrate according to the current invention can be used as an artificial substrate for ADAMTS-7 and/or ADAMTS-12. The peptide substrate can furthermore be used in order to determine the proteolytic activity of ADAMTS-7 and/or ADAMTS-12, identify modulators of ADAMTS-7 and/or ADAMTS-12, and/or to determine the degree of modulation induced by an agonist or antagonist.

In some embodiments the peptide substrate comprises a subsequence of a natural ADAMTS-7 and/or ADAMTS-12 substrate, such as a subsequence of TSP1 or COMP. In some embodiments, the peptide substrate according to the fourth biological aspect comprises at least a sequence according to any of SEQ ID No. 4, 5, 8, 11, 12 or 13, or a fragment thereof comprising the amino acids EL. It was surprisingly found that theses sequences can be used as cleavage site for ADAMTS-7 and ADAMTS-12: For example, the sequence DELSSMVLELRGLRT, derived from TSP1, residues 275 - 289 has been surprisingly identified as a suitable substrate for ADAMTS-7 and ADAMTS-12. Without being bound by theory, cleavage occurs between Glu283 and Leu284 (EL).

In some embodiments the peptide substrate comprises (a) residues 1-15 of the amino acid sequence of SEQ ID NO: 4, or (b) residues 1-15 of the amino acid sequence of SEQ ID NO: 5, or (c) residues 1-13 of the amino acid sequence of SEQ ID NO: 8, or (d) a fragment of any of the sequences according to a), b) or c), the fragment comprising the amino acid sequence EL.

In some embodiments, the peptide substrate according to the fourth biological aspect comprises at least the amino acids EL. In some embodiments, the peptide substrate according to the fourth biological aspect comprise at least the SEQ ID No. 4, or a fragment thereof comprising the amino acids EL. In some embodiments, the peptide substrate according to the fourth biological aspect comprises at least the SEQ ID No. 5, or a fragment thereof comprising the amino acids EL. In some embodiments, the peptide substrate according to the fourth biological aspect comprises at least the SEQ ID No. 8, or a fragment thereof comprising the amino acids EL.

In some embodiments, the peptide substrate according to the fourth biological aspect comprises at least the SEQ ID No. 11, 12 or 13 or a fragment thereof comprising the amino acids EL. It was surprisingly found that the solubility of the described peptide substrate according to the fourth biological aspect could be improved by including an additional hydrophilic moiety without affecting the activity profile (cf. SEQ ID No. 11, 12, 13).

In some embodiments the peptide substrate according to the fourth biological aspect comprises a first moiety conjugated to a residue that is N-terminal to sequence fragment EL as comprised within SEQ ID No. 4, 5, or 8 or the fragment thereof, and a second moiety conjugated to a residue that is C-terminal to said sequence fragment EL. In some embodiments the first moiety comprises a fluorophore and the second moiety comprises a quencher, or the first moiety comprises a quencher and the second moiety comprises a fluorophore.

Without being bound by theory, the fluorophore of the peptide substrate can be excited at a suitable wavelength, e.g. by exposing it to light. The suitability of a wavelength depends on the specific fluorophore and can be determined as known in the art. Without being bound by theory, the excited fluorophore transfers the energy to the closely located quencher, which has the ability to decrease the fluorescence intensity of the fluorophore, such that no fluorescence or only background fluorescence is detected at the emission wavelength of the fluorophore. If the peptide substrate according to the current invention is however exposed to or contacted with a functional ADAMTS-7 or ADAMTS-12, the enzyme cleaves the peptide, thereby separating fluorophore and quencher. In the absence of the quencher, the excited fluorophore emits light in returning to the ground state and an increase in fluorescence can be detected.

Using a combination of functional ADAMTS-7 and the peptide substrate comprising a fluorophore and a quencher according to the fourth biological aspect as a substrate thus allows for the robust and reproducible identification and characterization of ADAMTS-7 modulators and/or ADAMTS-12 modulators.

The skilled person understands that according to the various biological aspects and embodiments of the current invention, multiple sites can be used to attach the fluorophore and the quencher to the peptide as long as a) the distance between fluorophore and quencher allows for the transfer of energy between fluorophore and quencher and b) the ADAMTS-7 cleavage site is arranged in such a way that fluorophore and quencher are separated upon ADAMTS-7 cleavage of the peptide. The latter effect can be obtained by interposing the ADAMTS-7 cleavage site between the fluorophore and the quencher.

A variety of suitable pairs of fluorophores and quenchers have been described in the literature. The skilled person is well aware which pairs of fluorophore and quencher can be combined. To obtain a peptide according to the biological aspect at hand, the distance between fluorophore and quencher has to be adjusted to allow for the necessary transfer of energy as described herein. The specific distance depends on the specific selection of fluorophore and quencher and can be adjusted as known in the art. For example in some embodiments according to the biological aspect at hand, EDANS as a fluorophore can be paired with DABCYL or DABSYL as a quencher. When EDANS and DABCYL are in a close proximity (10-100 Å), the energy emitted from EDANS will be quenched by Dabcyl, resulting in low or no fluorescence. However, if the compounds are separated EDANS will fluoresce. The optimal absorbance and emission wavelengths of EDANS are λabs=336 nm and λem=490 nm respectively, and for Dabcyl, the maximum absorbance wavelength is λabs=472 nm, which, to a large extent, overlap with the emission spectra of EDANS.

Another pair of fluorophore and quencher where the compounds can be used alone or in combination is 7-methoxy-coumarin-4-yl acetic acid (MCA) as the fluorophore with Lys(DNP) as a quencher. In some preferred embodiments according to the biological aspect at hand the fluorophore is MCA and the quencher is Lys(DNP). A further pair which can be used according to the current invention comprises 7-amino-4-carbamoylmethylcoumarin (ACC) as the fluorophore and 2,4-dinitrophenyl-lysine (Lys(DNP)) as the quencher. In some preferred embodiments according to the biological aspect at hand the fluorophore is ACC and the quencher is Lys(DNP). Another pair of fluorophore and quencher that can be used alone or in combination is HiLyteFluor, e.g. HiLyteFluor-488 as the fluorophore with QXL, e.g. QXL520 as a quencher. HiLyte Fluor fluorophores are commercially available for various wavelengths and can be prepared as known in the art (Jungbauer, L M et al. "Preparation of fluorescently-labeled amyloid-beta peptide assemblies: the effect of fluorophore conjugation on structure and function" Journal of molecular recognition : JMR vol. 22,5 (2009): 403-13.). QXL quenchers are commercially available for various wavelengths and can be prepared as known in the art. QXL 570 dyes are optimized quenchers for rhodamines (such as TAMRA, sulforhodamine B, ROX) and Cy3 fluorophores. Their absorption spectra overlap with the fluorescence spectra of TAMRA, sulforhodamine B, ROX and Cy3.

According to some embodiments according to the fourth biological aspect said fluorophore is HiLyteFluor, such as HiLyteFluor-488 and the quencher is a matching QXL quencher, such as QXL520.

According to some embodiments according to the fourth biological aspect said peptide substrate comprises or consists of a subsequence of a natural ADAMTS-7 and/or ADAMTS-12 substrate, such as a subsequence of TSP1 or COMP, such as (a) residues 1-15 of the amino acid sequence of SEQ ID NO: 4, or (b) residues 1-15 of the amino acid sequence of SEQ ID NO: 5, or (c) residues 1-13 of the amino acid sequence of SEQ ID NO: 8, or (d) a fragment of any of the sequences according to a), b) or c), the fragment comprising the amino acid sequence EL, said fluorophore is HiLyteFluor, such as HiLyteFluor -488 and the quencher is a matching QXL quencher such as QXL520.

According to some embodiments according to the fourth biological aspect said peptide substrate comprises or consists of a subsequence of a natural ADAMTS-7 and/or ADAMTS-12 substrate, such as a subsequence of TSP1 or COMP, such as (a) residues 1-15 of the amino acid sequence of SEQ ID NO: 4, or (b) residues 1-15 of the amino acid sequence of SEQ ID NO: 5, or (c) residues 1-13 of the amino acid sequence of SEQ ID NO: 8, or (d) a fragment of any of the sequences according to a), b) or c), the fragment comprising the amino acid sequence EL, said fluorophore is MCA and said quencher is Lys(DNP).

According to some embodiments according to the fourth biological aspect said peptide substrate comprises or consists of a subsequence of a natural ADAMTS-7 and/or ADAMTS-12 substrate, such as a subsequence of TSP1 or COMP, such as (a) residues 1-15 of the amino acid sequence of SEQ ID NO: 4, or (b) residues 1-15 of the amino acid sequence of SEQ ID NO: 5, or (c) residues 1-13 of the amino acid sequence of SEQ ID NO: 8, or (d) a fragment of any of the sequences according to a), b) or c), the fragment comprising the amino acid sequence EL, said fluorophore is ACC and said quencher is Lys(DNP).

In some embodiments, the fluorophore, such as HiLyte fluor 488 is attached to the N-terminus of the peptide and the quencher, such as QXL520, is attached to the C-terminus or vice versa. In certain preferred embodiments, according to the biological aspect at hand, an additional negative residue, such as carboxyl position glutamic acid is attached C-terminal of the quencher, e.g. after the QXL520 quencher. The addition of the residue improved the solubility behavior of the peptide substrate and thereby lead to an improved reproducibility of the assay.

According to some embodiments according to the biological aspect at hand said peptide substrate for ADAMTS-7 and/or ADAMTS-12 comprises or consists of a subsequence of a natural ADAMTS-7 substrate, such as a subsequence of TSP1 or COMP, such as (a) residues 1-15 of the amino acid sequence of SEQ ID NO: 4, or (b) residues 1-15 of the amino acid sequence of SEQ ID NO: 5, or (c) residues 1-13 of the amino acid sequence of SEQ ID NO: 8, or (d) a fragment of any of the sequences according to a), b) or c), the fragment comprising the amino acid sequence EL, and the peptide substrate is further characterized in comprising an additional negatively charged residue such as a carboxyl position glutamic acid, e.g. C-terminal of the quencher.

According to some embodiments according to the biological aspect at hand said peptide substrate for ADAMTS-7 and/or ADAMTS-12 comprises or consists of a subsequence of a natural ADAMTS-7 substrate, such as a subsequence of TSP1 or COMP, such as (a) residues 1-15 of the amino acid sequence of SEQ ID NO: 4, or (b) residues 1-15 of the amino acid sequence of SEQ ID NO: 5, or (c) residues 1-13 of the amino acid sequence of SEQ ID NO: 8, or (d) a fragment of any of the sequences according to a), b) or c), the fragment comprising the amino acid sequence EL, said fluorophore is HiLyteFluor, such as HiLyteFluor -488, said quencher is a matching QXL quencher such as QXL520 and the peptide substrate is further characterized in comprising an additional negatively charged residue such as a carboxyl position glutamic acid, e.g. C-terminal of the quencher.

According to a **fifth biological aspect** of the current invention there is provided a method for the identification or characterization of an ADAMTS-7 and/or ADAMTS-12 modulator comprising the steps of
a) contacting a recombinant polypeptide or a fragment thereof according to the third biological aspect, and
b) contacting said recombinant polypeptide or fragment thereof with a peptide substrate according to the fourth biological aspect, wherein the peptide substrate comprises a fluorophore and a quencher, and
c) detecting fluorescence as a measure for the activity of said recombinant polypeptide or a fragment thereof.

In some embodiments, the recombinant polypeptide is suited to cleave a peptide comprising standard residues 1-15 of the amino acid sequence of SEQ ID NO: 4.

In some embodiments, the recombinant polypeptide comprises a first portion and a second portion, the first portion having a sequence identity of at least 80 % with the sequence of residues 1-217 of SEQ ID NO: 1 and/or having a sequence identity of at least 80 % with the sequence of residues 1-217 of SEQ ID NO: 2, and the second portion having a sequence identity of > 80 % with the sequence of residues 218-518 of SEQ ID NO: 1 and the peptide substrate comprises (a) residues 1-15 of the amino acid sequence of SEQ ID NO: 4, or (b) residues 1-15 of the amino acid sequence of SEQ ID NO: 5, or (c) residues 1-13 of the amino acid sequence of SEQ ID NO: 8, or (d) a fragment of any of the sequences according to a), b) or c), the fragment comprising the amino acid sequence EL.

In some embodiments, the recombinant polypeptide comprises a first portion and a second portion, the first portion having a sequence identity of > 80 % with the sequence of residues 1-244 of SEQ ID NO: 15, and the second portion having a sequence identity of > 80 % with the sequence of residues 245-547 of SEQ ID NO: 15 and the peptide substrate comprises (a) residues 1-15 of the amino acid sequence of SEQ ID NO: 4, or (b) residues 1-15 of the amino acid sequence of SEQ ID NO: 5, or (c) residues 1-13 of the amino acid sequence of SEQ ID NO: 8, or (d) a fragment of any of the sequences according to a), b) or c), the fragment comprising the amino acid sequence EL.

In some embodiments, the recombinant polypeptide comprises a sequence according to SEQ ID No. 01, SEQ ID No. 02 or SEQ ID No. 15 and/or the peptide substrate comprises (a) residues 1-15 of the amino acid sequence of SEQ ID NO: 4, or (b) residues 1-15 of the amino acid sequence of SEQ ID NO: 5, or (c) residues 1-13 of the amino acid sequence of SEQ ID NO: 8, or (d) a fragment of any of the sequences according to a), b) or c), the fragment comprising the amino acid sequence EL, and optionally comprises a further carboxy group.

Contacting of different compounds can be performed by preparing a solution comprising the respective compounds, e.g. in an appropriate concentration, e.g. as described in the examples. The test compound can be any molecule, such as any small molecule provided for throughout this application.

There is no specific order with regard to the steps of the method, except for the obvious restrictions resulting from the mode of action. In some embodiments the method according to the biological aspect at hand is further characterized in that step c) is performed for a solution comprising said recombinant polypeptide, said test compound, and said peptide substrate.

In some embodiments, the method according to the biological aspect at hand can be used to determine the half maximal inhibitory concentration (IC50) or the half maximal effective concentration (EC50) of an ADAMTS-7 and/or ADAMTS-12 modulator. According to the FDA, IC50 represents the concentration of a drug that is required for 50% inhibition *in vitro.* The determined IC50 as described herein is a measure of the potency of the test compound in inhibiting ADAMTS-7 and/or ADAMTS-12 induced cleavage of a natural or artificial substrate. Half maximal effective concentration (EC50) refers to the concentration of a test compound which induces a response halfway between the baseline and maximum after a specified exposure time and can be used as a measure of agonistic potency of a compound. IC50 and EC50 can be determined as known in the art. In brief, the IC50 of a drug can be determined by constructing a dose-response curve and analyzing the resulting fluorescence for different concentrations of the test compound.

In some embodiments according to the fifth biological aspect a test compound can be or is selected as a modulator (e.g. agonist or inhibitor) if after contacting the recombinant polypeptide with the at least one test compound according to step a) and after contacting said recombinant polypeptide with a peptide substrate according to step b) no significant increase of fluorescence is detected or if an increase of fluorescence is detected which is significantly lower than the increase of fluorescence which is detectable for a control in the absence of the test compound.

In some embodiments according to the fifth biological aspect a test compound is selected as an ADAMTS-7 and/or ADAMTS-12 agonist if after contacting the recombinant polypeptide with the at least one test compound according to step a) and after contacting said recombinant polypeptide with a peptide substrate according to step b) an increase of fluorescence is detected which is significantly higher than the increase of fluorescence which is detectable for a control in the absence of the test compound. An exemplary way how the method can be performed is described in example B5.

According to some embodiments according to the current biological aspect there is provided a method for evaluating the selectivity of an ADAMTS-7 and/or ADAMTS-12 modulator comprising the method according to the fifth biological aspect further characterized in comprising the steps of
a) contacting a functional recombinant metalloproteinase different from ADAMTS-7 and ADAMTS-12 with the at least one test compound, and
b) contacting said functional recombinant metalloproteinase with a peptide substrate comprising a fluorophore and a quencher, wherein said peptide substrate comprises a cleavage site for said functional recombinant metalloproteinase different from ADAMTS-7 and/or ADAMTS-12; and
c) detecting the fluorescence as a measure for the activity of functional recombinant metalloproteinase different from ADAMTS-7 and ADAMTS-12.

In certain preferred embodiments, the functional recombinant metalloproteinase is different from ADAMTS-7 and ADAMTS-12 is ADAMTS4, ADAMTS5, MMP12, MMP15, MMP2 or ADAM17 or any other enzyme listed in table 2. In some preferred embodiments, the peptide comprising a fluorophore and a quencher is the peptide as specified in table 2 and / or comprises a cleavage site of said functional recombinant metalloproteinase different from ADAMTS-7 and/or ADAMTS-12 as disclosed in table 2.

For characterization of the inhibition, based on the fluorescence IC50 values can be calculated from percentage of inhibition of enzyme activity as a function of test compound concentration.

According to a **sixth biological aspect** there is provided a modulator of ADAMTS-7 and/or ADAMTS-12 identified by a method according to the fifth biological aspect for use in the treatment of heart disease, vascular disease, and/or cardiovascular disease, including atherosclerosis, coronary artery disease (CAD), myocardial infarction (MI), peripheral vascular disease (PAD) / arterial occlusive disease and/or restenosis after angioplasty (including the use of drug-coated or non drug-coated balloons and/or stent-implantation) and/or for the treatment and/or prophylaxis of lung disease, inflammatory disease, fibrotic disease, metabolic disease, cardiometabolic disease and/or diseases/disease states affecting the kidneys and/or the central nervous and/or neurological system as well as gastrointestinal and/or urologic and/or ophthalmologic disease/disease states.

For these diseases or disease states, alterations or aberrant expression with regard to ADAMTS-7 and/ or ADAMTS-12 have been described earlier. In some embodiments the modulator of ADAMTS-7 and/or ADAMTS-12 is a modulator for use in the treatment of coronary artery disease (CAD), peripheral vascular disease (PAD) and myocardial infarction (MI). In some embodiments, the modulator according to the sixth biological aspect is a small molecule. In some embodiments, the modulator according to the seventh biological aspect is an antagonist of human ADAMTS-7. In some different or the same embodiments, the modulator according to the sixth biological aspect is an antagonist of human ADAMTS-12. In some different or the same embodiments, the modulator according to the sixth biological aspect is an antagonist of human ADAMTS4. In some embodiments the modulator according to the sixth biological aspect is a small molecule, e.g. as provided in the examples. While the identification of selective ADAMTS-7 and ADAMTS-12 modulators has been extremly challenging in the past, the provided assay now offers all means to obtain these moulators according to the current biological aspect.

According to a **seventh biological aspect** there is provided a method of producing a recombinant polypeptide according to any ot the previous biological aspects, the method comprising cultivating a recombinant host cell comprising a recombinant nucleic acid according to any biological aspect described herein and recovering the recombinant polypeptide of a fragment thereof.

According to an **eigth biological aspect** there is provided a kit of parts comprising a recombinant nucleic acid according to the first and/or second biological aspect and/or a polypeptide according to the third biological aspect and a peptide substrate according to the fourth biological aspect. In some embodiments the kit of parts can be used to perform a method according to the fifth biological aspect in a convenient and reproducible way. In some embodiments the provided kit of parts can be used to evaluate whether a test molecule is a modulator of ADAMTS-7. In some different or the same embodiments the provided kit of parts can be used to evaluate wether a test molecule is a modulator of ADAMTS-12. In some embodiments the provided kit of parts can be used to evaluate wether a test molecule is a modulator of ADAMTS-7 and a modulator of ADAMTS-12.

### EXPERIMENTAL SECTION

The following table 1 lists the abbreviations used in this paragraph and in the Examples section as far as they are not explained within the text body. Other abbreviations have their meanings customary *per se* to the skilled person.

**Table 1: Abbreviations**

| | |
|---|---|
| The following table lists the abbreviations used herein. | |
| **Abbreviation** | **Meaning** |
| Ac | acetate |
| acac | acetylacetonate |
| ACN | acetonitrile |
| aq. | aqueous |
| bp | broad peak |
| br | broad (¹H-NMR signal) |
| Bu | butyl |
| CDI | carbonyldiimidazole |
| CI | chemical ionisation |
| conc. | concentrated |
| d | doublet (¹H-NMR signal) |
| DBU | 1,8-Diazabicyclo[5.4.0]undec-7-ene |
| DCM | dichloromethane |
| dd | double-doublet (¹H-NMR signal) |
| ddd | doublet of doublets of doublets |
| diamix | mixture of four stereoisomers containing diastereoisomers and enantiomers |
| DIPEA | diisopropylethylamine |
| DMA | dimethylacetamide |
| DMF | N,N-Dimethylformamide |
| DMSO | dimethylsulfoxide |
| EDC | 1-ethyl-3-(3-dimethylaminopropyl)carbodiimide hydrochloride |
| ent | enantiomerically pure compound |
| eq. | equivalents |
| ESI | electrospray (ES) ionisation |
| Et | ethyl |
| EtOH | ethanol |
| h | hour(s) |
| HATU | (1-[bis(dimethylamino)methylene)-1H-1,2,3-triazolo[4,5-b]pyridinium-3-oxide hexafluorophosphate |
| HOBt | 1-hydroxybenzotriazole hydrate |
| HPLC | high performance liquid chromatography |
| iPr | isopropyl |
| LC-MS | liquid chromatography mass spectrometry |
| LiHMDS | Lithium bis(trimethylsilyl)amide |
| m | multiplet (¹H-NMR signal) |
| MeOH | methanol |
| min | minute(s) |
| MS | mass spectrometry |
| N | normal (concentration) |
| NBS | N-bromosuccinimide |
| NCS | N-chlorosuccinimide |
| NMR | nuclear magnetic resonance spectroscopy: chemical shifts (δ) are given in ppm. The chemical shifts were corrected by setting the DMSO signal to 2.50 ppm unless otherwise stated. |
| PCC | Pyridinium chlorochromate |
| q | quartet |
| rac | racemic mixture |
| RF | reflux |
| RP | Reversed-Phase chromatography |
| Rt | retention time (as measured either with HPLC or UPLC) in minutes |
| RT | Room Temperature |
| s | singlet (¹H-NMR signal) |
| sat. | saturated |
| SQD | Single-Quadrupole-Detector |
| t | triplet (¹H-NMR signal) |
| TBAF | tetrabutylammoniumfluoride |
| TBDMSCl | tert.-butyldimethylsilylchlorid |
| td | triple-doublet (¹H-NMR signal) |
| TEA | triethylamine |
| TFA | trifluoroacetic acid |
| THF | tetrahydrofuran |
| TLC | Thin Layer Chromatography |
| UPLC | ultra performance liquid chromatography |

### EXPERIMENTAL SECTION - GENERAL PART

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartidges KP-Sil® or KP-NH® in combination with a Biotage autopurifier system (SP4® or Isolera Four®) and eluents such as gradients of hexane/ethyl acetate or DCM/methanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

Chemical names were generated using the ACD/Name software from ACD/Labs. In some cases generally accepted names of commercially available reagents were used in place of ACD/Name generated names.

The various aspects of the invention described in this application are illustrated by the following examples which are not meant to limit the invention in any way.

The example testing experiments described herein serve to illustrate the present invention and the invention is not limited to the examples given.

### NMR analytical method:

NMR peak forms are stated as they appear in the spectra, possible higher order effects have not been considered.

The ¹H NMR data of selected synthesis intermediates and working examples are stated in the form of ¹H NMR peak lists. For each signal peak, first the δ[ppm] = value in ppm and then the signal intensity in round brackets are listed. The δ[ppm] = value/signal intensity number pairs for different signal peaks are listed with separation from one another by commas. The peak list for an example therefore takes the following form: δ[ppm] = ₁ (intensity₁), δ[ppm] = ₂ (intensity₂), ... , δ[ppm] = ᵢ (intensityᵢ), ... , δ[ppm] = ₙ (intensityₙ).

The intensity of sharp signals correlates with the height of the signals in a printed example of an NMR spectrum in cm and shows the true ratios of the signal intensities in comparison with other signals. In the case of broad signals, several peaks or the middle of the signal and the relative intensity thereof may be shown in comparison to the most intense signal in the spectrum. The lists of the ¹H NMR peaks are similar to the conventional ¹H NMR printouts and thus usually contain all peaks listed in a conventional NMR interpretation. In addition, like conventional ¹H NMR printouts, they may show solvent signals, signals of stereoisomers of the target compounds which are likewise provided by the invention, and/or peaks of impurities. The peaks of stereoisomers of the target compounds and/or peaks of impurities usually have a lower intensity on average than the peaks of the target compounds (for example with a purity of > 90%). Such stereoisomers and/or impurities may be typical of the particular preparation process. Their peaks can thus help in identifying reproduction of our preparation process with reference to "by-product fingerprints". An expert calculating the peaks of the target compounds by known methods (MestreC, ACD simulation, or using empirically evaluated expected values) can, if required, isolate the peaks of the target compounds, optionally using additional intensity filters. This isolation would be similar to the peak picking in question in conventional ¹H NMR interpretation. A detailed description of the presentation of NMR data in the form of peak lists can be found in the publication "Citation of NMR Peaklist Data within Patent Applications" (cf. Research Disclosure Database Number 605005, 2014, 1 August 2014 or http://www.researchdisclosure.com/searching-disclosures). In the peak picking routine described in Research Disclosure Database Number 605005, the parameter "MinimumHeight" can be set between 1% and 4%. Depending on the type of chemical structure and/or depending on the concentration of the compound to be analysed, it may be advisable to set the parameters "MinimumHeight" to values of < 1%.

### LC-MS Procedures

### General remarks:

Method 1-6 were performed using an Agilent G1956A LC/MSD quadrupole coupled to an Agilent 1100 series liquid chromatography (LC) system consisting of a binary pump with degasser, autosampler, thermostated column compartment and diode array detector. The mass spectrometer (MS) was operated with an atmospheric pressure electro-spray ionization (API-ES) source in positive ion mode **(Mass method 1**). The capillary voltage was set to 3000 V, the fragmentor voltage to 70 V and the quadrupole temperature was maintained at 100°C. The drying gas flow and temperature values were 12.0 L/min and 350 °C, respectively. Nitrogen was used as the nebuliser gas, at a pressure of 35 psi. Data acquisition was performed with Agilent Chemstation software.

### Method 1:

Analyses were carried out on a YMC pack ODS-AQ C18 column (50 mm long x 4.6 mm I.D..; 3 µm particle size) at 35 °C, with a flow rate of 2.6 mL/min. A gradient elution was performed from 95% (Water + 0.1% Formic acid)/5% Acetonitrile to 5% (Water + 0.1% Formic acid)/95% Acetonitrile in 4.8 min; the resulting composition was held for 1.0 min; from 5% (Water + 0.1% formic acid)/95% Acetonitrile to 95% (Water + 0.1% formic acid)/5% Acetonitrile in 0.2 min. The standard injection volume was 2 µL. Acquisition ranges were set to 190-400 nm for the UV-PDA detector and 100-1400 m/z for the MS detector.

### Method 2:

Analyses were carried out on a Phenomenex Kinetex 00B-4475-AN C18 column (50 mm long x 2.1 mm I.D.; 1.7 µm particles) at 60 °C, with a flow rate of 1.5 mL/min. A gradient elution was performed from 90% (Water + 0.1 % Formic acid) / 10% Acetonitrile to 10% (Water + 0.1 % Formic acid) / 90% Acetonitrile in 1.50 minutes; the resulting composition was held for 0.40 min; then the final mobile phase composition; from 10% (Water + 0.1% Formic acid) / 90% Acetonitrile to 90% (Water + 0.1% Formic acid) / 10% Acetonitrile in 0.10 minutes. The injection volume was 2 µL with Agilent autosampler injector or 5 µL with Gerstel MPS injector. MS acquisition range and DAD detector were set to 100-800 m/z and 190-400 nm respectively.

### Method 3:

Analyses were carried out on a Thermo Scientific Accucore C18 (50 mm long x 2.1 mm I.D., 2.6 µm) at 35 °C, with a flow rate of 1.50 mL/min. A gradient elution was performed from 95% (Water + 0.1% Formic acid) / 5% Acetonitrile to 5% (Water + 0.1% Formic acid) / 95% Acetonitrile in 1.30 minutes; the resulting composition was held for 0.5 min; then the final mobile phase composition; from 5% (Water + 0.1% Formic acid) / 95% Acetonitrile to 90% (Water + 0.1% Formic acid) / 10% Acetonitrile in 0.10 minutes. The injection volume was 1 µL. MS acquisition range and UV detector were set to 100-1000 m/z and 190-400 nm respectively.

### Method 4:

Analyses were carried out on a Thermo Scientific Accucore C18 column PN17126-054630 (50 mm long x 4.6 mm I.D.; 2.6 µm particles) at 30 °C, with a flow rate of 3 mL/min. A gradient elution was performed from 90% (Water + 0.1% Formic acid) / 10% Acetonitrile to 5% (Water + 0.1% Formic acid) / 95% Acetonitrile in 1.50 minutes; the resulting composition was held for 0.90 min; then the final mobile phase composition; from 10% (Water + 0.1% Formic acid) / 90% Acetonitrile to 90% (Water + 0.1% Formic acid) / 10% Acetonitrile in 0.10 minutes. The injection volume was 2 µL. MS acquisition range and DAD detector were set to 100-1000 m/z and 200-400 nm respectively.

### Method 5:

Analyses were carried out on a Thermo Scientific Accucore AQ C18 (50 mm long x 2.1 mm I.D., 2.6 µm) at 35 °C, with a flow rate of 1.50 mL/min. A gradient elution was performed from 95% (Water + 0.1% Formic acid) / 5% Acetonitrile to 5% (Water + 0.1% Formic acid) / 95% Acetonitrile in 1.50 minutes; the resulting composition was held for 0.3 min; then the final mobile phase composition; from 5% (Water + 0.1% Formic acid) / 95% Acetonitrile to 95% (Water + 0.1% Formic acid) / 5% Acetonitrile in 0.10 minutes. The injection volume was 1 µL. MS acquisition range and UV detector were set to 100-1000 m/z and 190-400 nm respectively.

### Method 6:

Analyses were carried out on a YMC pack ODS-AQ C18 column (50 mm long x 4.6 mm I.D.; 3 µm particle size) at 35 °C, with a flow rate of 2.6 mL/min. A gradient elution was performed using ISET 2V1.0 Emulated Agilent Pump G1312A V1.0 from 94.51% (Water + 0.1% Formic acid)/5.49% Acetonitrile to 5% (Water + 0.1% Formic acid)/95% Acetonitrile in 4.8 min; the resulting composition was held for 1.0 min; from 5% (Water + 0.1% formic acid)/95% Acetonitrile to 95% (Water + 0.1% formic acid)/5% Acetonitrile in 0.2 min. The standard injection volume was 2 µL. Acquisition ranges were set to 190-400 nm for the UV-PDA detector and 100-1000 m/z for the TOF-MS detector.

### Method 7:

Instrument MS: Thermo Scientific FT-MS; Instrument type UHPLC+: Thermo Scientific UltiMate 3000; Column: Waters, HSST3, 2.1 x 75 mm, C18 1.8 µm; eluent A: 1 L water + 0.01% formic acid; eluent B: 1 L acetonitrile + 0.01% formic acid; gradient: 0.0 min 10% B → 2.5 min 95% B → 3.5 min 95% B; oven: 50°C; flow rate: 0.90 ml/min; UV detection: 210 nm/ optimum integration path 210-300 nm.

### Method 8:

Instrument: Waters ACQUITY SQD UPLC System; Column: Waters Acquity UPLC HSS T3 1.8 µ 50 x 1 mm; eluent A: 1 L water + 0.25 ml 99% formic acid, eluent B: 1 L acetonitrile + 0.25 ml 99% formic acid; gradient: 0.0 min 90% A → 1.2 min 5% A → 2.0 min 5% A; oven: 50°C; flow rate: 0.40 ml/min; UV detection: 210 nm.

### Method 9:

Instrument: Waters Single Quad MS System; Instrument Waters UPLC Acquity; Column: Waters BEH C18 1.7 µ 50 x 2.1 mm; eluent A: 1 L water + 1.0 mL (25% ammonia)/L, eluent B: 1 L acetonitrile; gradient: 0.0 min 92% A → 0.1 min 92% A → 1.8 min 5% A → 3.5 min 5% A; oven: 50°C; flow rate: 0.45 mL/min; UV-detection: 210 nm.

### Method 10:

Instrument: Agilent MS Quad 6150; HPLC: Agilent 1290; column: Waters Acquity UPLC HSS T3 1.8 µm 50 x 2.1 mm; eluent A: 1 L water + 0.25 ml 99% formic acid, eluent B: 1 L acetonitrile + 0.25 ml 99% formic acid; gradient: 0.0 min 90% A → 0.3 min 90% A → 1.7 min 5% A → 3.0 min 5% A; ofen: 50°C; flow rate: 1.20 mL/min; UV-detection: 205 - 305 nm.

### Method 11:

Instrument: Agilent MS Quad 6150; HPLC: Agilent 1290; column: Waters Acquity UPLC HSS T3 1.8 µm 50 x 2.1 mm; eluent A: 1 L water + 0.25 ml 99% formic acid, eluent B: 1 L acetonitrile + 0.25 ml 99% formic acid; gradient: 0.0 min 90% A → 0.3 min 90% A → 1.7 min 5% A → 3.0 min 5% A; oven: 50°C; flow rate: 1,20 ml/min; UV-detection: 205 - 305 nm.

### Methode 12:

Instrument: Waters ACQUITY SQD UPLC System; column: Waters Acquity UPLC HSS T3 1.8 µm 50 x 1 mm; eluent A: 1 l water + 0.25 ml 99% formic acid, Eluent B: 1 l Acetonitril + 0.25 ml 99% formic acid; gradient: 0.0 min 95% A → 6.0 min 5% A → 7.5 min 5% A; oven: 50°C; flow rate: 0.35 ml/min; UV-detection: 210 nm.

### Methode 13:

Instrument MS: Waters SQD; Instrument type HPLC: Waters Alliance 2795; Column: Waters, Cortecs, 3.0 x 30 mm, C18 2.7 µm; eluent A: 1 L water + 0.01% formic acid; eluent B: 1 L acetonitrile + 0.01% formic acid; gradient: 0.0 min 5% B → 1.75 min 95% B → 2.35 min 95% B; oven 45°C: flow rate: 1.75 mL/min; UV detection: 210 nm.

### Methode 14:

Instrument MS: Waters SQD; Instrument HPLC: Waters UPLC; column: Zorbax SB-Aq (Agilent), 50 mm x 2.1 mm, 1.8 µm; eluent A: water + 0.025% formic acid, eluent B: acetonitrile (ULC) + 0.025% formic acid; gradient: 0.0 min 98%A - 0.9 min 25%A - 1.0 min 5%A - 1.4 min 5%A - 1.41 min 98%A- 1.5 min 98%A; oven: 40°C; flow: 0.600 ml/min; UV-detection: DAD; 210 nm.

### GC-MS Procedures

### Method A:

Instrument: Thermo Scientific DSQII, Thermo Scientific Trace GC Ultra; column: Restek RTX-35MS, 15 m x 200 µm x 0.33 µm; constant flow with helium: 1.20 ml/min; oven: 60°C; Inlet: 220°C; gradient: 60°C, 30°C/min → 300°C (3.33 min hold).

### Enantiomeric separation, preparative scale:

Method 1D: Phase: Daicel Chiralpak IF, 5 µm 250 mm x 20 mm, eluent: n-heptane/ethanol 50:50; flow: 20 ml/min, temperature: 40°C; UV-Detection: 220 nm.
Method 2D: Phase: Daicel Chiralpak IE, 5 µm 250 mm x 20 mm, eluent: n-heptane/2-propanol 60:40; flow: 15 ml/min, temperature: 50°C; UV-Detection: 220 nm.
Method 3D: Phase: Daicel Chiralpak IG, 5 µm 250 mm x 20 mm, eluent: ethanol 100%; flow: 15 ml/min, temperature: 50°C; UV-Detection: 220 nm.
Method 4D: Phase: Daicel Chiralpak AD-H, 5 µm 250 mm x 20 mm, eluent: n-heptane:ethanol 40:60; flow: 25 ml/min, temperature: 35°C; UV-Detection: 220 nm.
Method 5D: Phase: Daicel Chiralpak AD-H, 5 µm 250 mm x 20 mm, eluent: n-heptane:ethanol 50:50; flow: 25 ml/min, temperature: 30°C; UV-Detection: 220 nm.
Method 6D: Phase: Daicel Chiralpak IF, 5 µm 250 mm x 20 mm, eluent: n-heptane:ethanol 50.50; flow: 15 ml/min, temperature: 40°C; UV-Detection: 210 nm.
Method 7D: Phase: Daicel Chiralpak IF, 5 µm 250 mm x 20 mm, Eluent: n-heptane:ethanol 50:50; flow: 15 ml/min, temperature: 40°C; UV-Detection: 220 nm.
Method 8D: Phase: Daicel Chiralpak AD-H, 5 µm 250 mm x 20 mm, Eluent: i-hexand:ethanol 50:50; flow: 20 ml/min, temperature: 40°C; UV-Detection: 210 nm.
Method 9D: Phase: Daicel Chiralpak IF, 5 µm 250 mm x 20 mm, eluent: n-heptane:ethanol 40:60; flow: 15 ml/min, temperature: 40°C; UV-Detection: 210 nm.
Method 10D: Phase: Daicel Chiralpak IG, 5 µm 250 mm x 20 mm, eluent: n-heptane:ethanol 30:70; flow: 15 ml/min, temperature: 30°C; UV-Detection: 220 nm.
Method 11D: Phase: Daicel Chiralpak IG, 5 µm 250 mm x 20 mm, eluent: ethanol 100%; flow: 15 ml/min, temperature: 50°C; UV-Detection: 220 nm.
Method 12D: Phase: Daicel Chiralpak IG, 5 µm 250 mm x 20 mm, eluent: n-heptane:ethanol 10:90; flow: 15 ml/min, temperature: 50°C; UV-Detection: 220 nm.
Method 13D: Phase: Daicel Chiralpak IG, 5 µm 250 mm x 20 mm, Eluent: ethanol 100%; flow: 15 ml/min, temperature: 50°C; UV-Detection: 220 nm.
Method 14D: Phase: Daicel Chiralpak IF, 5 µm 250 mm x 20 mm, Eluent: ethanol 100%; flow: 15 ml/min, temperature: 50°C; UV-Detection: 220 nm.
Method 15D: Phase: Daicel Chiralpak IG, 5 µm 250 mm x 20 mm, eluent: n-heptane:ethanol 60:40; flow: 15 ml/min, temperature: 50°C; UV-Detection: 235 nm.
Method 16D: Phase: Daicel Chiralpak IG, 5 µm 250 mm x 20 mm, eluent: n-heptane:ethanol 10:90; flow: 15 ml/min, temperature: 30°C; UV-Detection: 235 nm.
Method 17D: Phase: Daicel Chiralpak IF, 5 µm 250 mm x 20 mm, eluent: ethanol 100%; flow: 15 ml/min, temperature: 70°C; UV-Detection: 220 nm.

### Enantiomeric separation, analytical scale:

Method 1E: Phase: Chiratek IF-3; eluent: i-hexane/ethanol 50:50; flow: 1 ml/min; UV-Detection: 220 nm.
Method 2E: Phase: Daicel Chiralpak IF, 5 µm 250 mm x 4.6 mm; eluent: hexane/2-propanol 30:70; flow: 1.0 ml/min, temperature: 60°C; UV-Detection: 270 nm.
Method 3E: Phase: Daicel Chiralpak IG, 5 µm 250 mm x 4.6 mm; eluent: ethanol 100%; flow: 1.0 ml/min, temperature: 50°C; UV-Detection: 220 nm.
Method 4E: Phase: Daicel AD, 5 µm 250 mm x 4.60 mm; eluent: ethanol 100%; flow: 1.0 ml/min; UV-Detection: 220 nm.
Method 5E: Phase: Daicel Chiralpak AD-H, 5 µm 250 mm x 4.6 mm; eluent: ethanol 100%; flow: 1.0 ml/min, UV-Detection: 220 nm.
Method 6E: Phase: Daicel Chiralpak IF, 5 µm 50 mm x 4.6 mm; eluent: n-heptane:ethanol 50:50; flow: 1.0 ml/min, Temperature: 25°C; UV-Detection: 220 nm.
Method 7E: Phase: Daicel Chiralpak IF, 5 µm 50 mm x 4.6 mm; eluent: n-heptane:ethanol 50:50; flow: 1.0 ml/min, UV-Detection: 220 nm.
Method 8E: Phase: Chiracel-H AD, 5 µm 250 mm x 4.6 mm Eluent: n-heptane:ehanol 50:50; flow: 1.0 ml/min, UV-Detection: 220 nm.
Method 9E: Phase: Daicel Chiralpak IF-3, 5 µm 50 mm x 4.6 mm; eluent: n-heptane:ehanol 50:50; flow: 1.0 ml/min, UV-Detection: 220 nm.
Method 10E: Phase: Daicel IG-3, 5 µm 50 mm x 4.6 mm; flow: 1.0 ml/min, UV-Detection: 220 nm.
Method 11E: Phase: Daicel Chiralpak IG-3, 5 µm 250 mm x 4.6 mm; eluent: ethanol 100%, flow: 1.0 ml/min, temperature: 50 °C; UV-Detection: 220 nm.
Method 12E: Phase: Daicel Chiralpak IG-3, 5 µm 250 mm x 4.6 mm; eluent: ethanol 100%, flow: 1 ml/min, temperature: 50 °C; UV-Detection: 220 nm.
Method 13E: Phase: Daicel Chiralpak IG-3, 5 µm 250 mm x 4.6 mm; eluent: ethanol 100%, flow: 1.0 ml/min, temperature:50 °C; UV-Detection: 220 nm.
Method 14E: Phase: Daicel Chiralpak IF, 5 µm 50mm x 4.60 mm; eluent: ethanol 100%, flow: 1.0 ml/min, UV-Detection: 220 nm.
Method 15E: Phase: Daicel Chiralpak IF, 5 µm 250mm x 4.60 mm; eluent: ethanol 100% , flow: 1.0 ml/min, temperature: 50 °C;UV-Detection: 220 nm.
Method 16E: Phase: Daicel IG-3 3 µm 50mm x 4.60 mm; eluent: ethanol 100% , flow 1.0 ml/min; UV-Detection: 220 nm.
Method 17E: Phase: Daicel Chiralpak IG 5 µm 50mm x 4.60 mm; eluent: ethanol 100% , flow 1.0 ml/min; temperature:70 °C, UV-Detection: 220 nm.

### Preparative HPLC:

### Method 1f

Column: Chromatorex C18 10 µm; 125 x 20 mm; Eluent A: water + 0.05% trifluoroacetic acid, Eluent B: acetonitrile + 0.05% trifluoroacetic acid; Gradient: 10% B → 100% B; Flow: 20 ml/min UV-Detection 210 nm.

### Method 2f

Column: Chromatorex C18 10 µm; 125 x 30 mm; Eluent A: water + 0.05% trifluoroacetic acid, Eluent B: acetonitrile + 0.05% trifluoroacetic acid; Gradient: 10% B → 100% B; Flow: 50 till 100 ml/min UV-Detection 210 nm.

### Method 3f

Instrument: Waters Prep LC/MS System, Column: Phenomenex Kinetex C18 5µm 100x30 mm; Eluent A: water, Eluent B: acetonitrile, Eluent C: 2% formic acid in Water, Eluent D: acetonitrile/water (80Vol%/20Vol%); Flow rate: 80 ml/min, room temperature, UV-detection 200-400 nm; Gradient: Eluent A 0 → 2 min 63 ml, Eluent B 0 → 2min 7 ml, Eluent A 2 → 10 min from 63 ml till 39 ml and Eluent B from 7 ml till 31 ml, 10 → 12 min 0 ml Eluent A and 70 ml Eluent B. Eluent C and Eluent D constant flow of 5 ml/min during the complete HPLC run.

### Method 4f

Instrument: Waters Prep LC/MS System, Column: Phenomenex Kinetex C18 5µm 100x30 mm; Eluent A: water, Eluent B: acetonitrile, Eluent C: 2% formic acid in Water, Eluent D: acetonitrile/water (80Vol.%/20Vol%); Flow rate: 80 ml/min, room temperature, UV-detection 200-400 nm; Gradient: Eluent A 0 → 2 min 55 ml, Eluent B 0 → 2min 15 ml, Eluent A 2 → 10 min from 55 ml till 31 ml and Eluent B from 15 ml till 39 ml, 10 → 12 min 0 ml Eluent A and 70 ml Eluent B. Eluent C and Eluent D constant flow of 5 ml/min during the complete HPLC run.

### Methode 5f

Instrument: Waters Prep LC/MS System, Column: Phenomenex Kinetex C18 5µm 100x30 mm; Eluent A: water, Eluent B: acetonitrile, Eluent C: 2% formic acid in Water, Eluent D: acetonitrile/water (80Vol.%/20Vol%); Flow rate: 80 ml/min, room temperature, UV-detection 200-400 nm; Gradient: Eluent A 0 → 2 min 70 ml, Eluent B 0 → 2min 0 ml, Eluent A 2 → 10 min from 70 ml till 55 ml and Eluent B from 0 ml till 15 ml, 10 → 12 min 0 ml Eluent A and 70 ml Eluent B. Eluent C and Eluent D constant flow of 5 ml/min during the complete HPLC run.

### EXPERIMENTAL SECTION - STARTING MATERIALS AND INTERMEDIATES

### Intermediate 1

### Benzyl {2-[methoxy(methyl)amino]-2-oxoethyl}carbamate

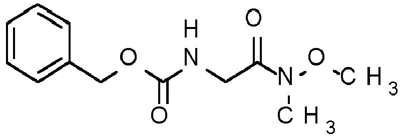

14.00 mL (110.00 mmol) of isobutyl chloroformate was added to a solution of 15.00 g (71.70 mmol) of N-[(benzyloxy)carbonyl]glycine and 16.00 mL (140.00 mmol) of 4-methylmorpholine in 170 mL of dichloromethane at -15 °C and the mixture was stirred for 1 hour. Then 7.69 g (78.90 mmol) of N,O-dimethylhydroxiamine hydrochloride in 30 mL of dichloromethane was added to the solution at -15 °C and the reaction was further stirred at room temperature for 2 hours. The mixture was quenched with water and extracted with dichloromethane. The combined organic layers were dried over magnesium sulfate, filtered and concentrated. The corresponding residue was purified by flash chromatography on silica gel eluting with dichloromethane/methanol mixtures to afford 11.80 g (65%) of the product as a white powder.
¹H-NMR (300 MHz, DMSO-*d*₆): δ [ppm] = 3.10 (s, 3H), 3.69 (s, 3H), 3.92 (d, 2H), 5.04 (s, 2H), 7.16-7.43 (m, 6H).
LC-MS (Method 3): Rₜ = 0.571 min. MS (Mass method 1): m/z = 253 (M + H)⁺

### Intermediate 2

### Benzyl (3-methyl-2-oxobutyl)carbamate

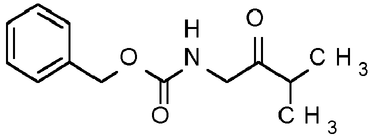

A solution of 24.00 mL (69.00 mmol) of isopropyl magnesium bromide (2.9M in 2-methyltetrahydrofuran) was slowly added drop wise at 0 °C to a solution of 5.00 g (19.80 mmol) of benzyl {2-[methoxy(methyl)amino]-2-oxoethyl}carbamate in 60 mL of anhydrous tetrahydrofuran under inert atmosphere and the mixture was stirred at room temperature for 10 hours. The reaction was quenched with saturated ammonium chloride(aq.) and the resulting slurry was extracted with ethyl acetate. The combined organic extracts were dried over magnesium sulfate, filtered, concentrated and chromatographed in heptane/ethyl acetate mixtures to afford 3.00 g (64%) of the expected compound as a colourless oil.
¹H-NMR (300 MHz, DMSO-*d*₆): δ [ppm] = 0.97 (d, 6H), 2.60-2.75 (m, 1H), 3.93 (d, 2H), 5.01 (s, 2H), 7.20-7.42 (m, 5H), 7.43 (t, 1H).
LC-MS (Method 3): Rₜ = 0.769 min. MS (Mass method 1): m/z = 258 (M + Na)⁺

### Intermediate 3

### rac-benzyl [(4-isopropyl-2,5-dioxoimidazolidin-4-yl)methyl]carbamate

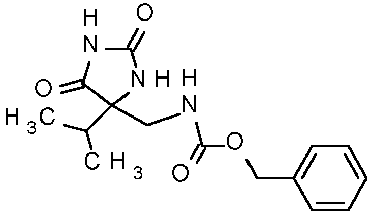

A solution of 1.40 g (5.95 mmol) of benzyl (3-methyl-2-oxobutyl)carbamate in 10 mL of ethanol was added to a solution of 0.77 g (11.90 mmol) of potassium cyanide and 2.86 g (29.80 mmol) of ammonium carbonate in 10 mL of water into a pressure flask, the container was sealed and the mixture was stirred at 60 °C for 24 hours. After that time, the solvent was partially removed and the resulting precipitate was filtered off to give 1.43 g (78%) of the product as a withe solid. The compound was used as such in the next step.
¹H-NMR (300 MHz, DMSO-*d*₆): δ [ppm] = 0.80 (d, 3H), 0.89 (d, 3H), 1.85-2.00 (m, 1H), 5.02 (d, 2H), 7.20-7.40 (m, 6H), 7.71 (s, 1H), 10.56 (bp, 1H).
LC-MS (Method 3): Rₜ = 0.555 min. MS (Mass method 1): m/z = 306 (M + H)⁺

### Intermediate 4

### rac-5-(aminomethyl)-5-(propan-2-yl)imidazolidine-2,4-dione

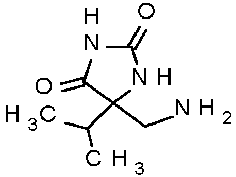

2.85 g (9.33 mmol) of rac-benzyl [(4-isopropyl-2,5-dioxoimidazolidin-4-yl)methyl]carbamate were dissolved in 30 mL of methanol and 0.10 g (0.93 mmol) mmol) of palladium on carbon (Degussa type, 10% loading, wet basis) was added and the mixture was stirred at room temperature under hydrogen atmosphere for 14 hours. The black suspension was filtered through a pad of celite and the filtrates were concentrated under vacuum to give 1.40 g (88%) of the product as a withe solid. The compound was used as such in the next step.
¹H-NMR (300 MHz, DMSO-*d*₆): δ [ppm] = 0.78 (d, 3H), 0.83 (d, 3H), 1.75-1.98 (m, 1H), 7.63 (s, 1H), 10.42 (bp, 1H).
LC-MS (Method 1): Rₜ = 0.506 min. MS (Mass method 1): m/z = 172 (M + H)⁺

### Intermediate 5

### tert-Butyl {2-[methoxy(methyl)amino]-2-oxoethyl}carbamate

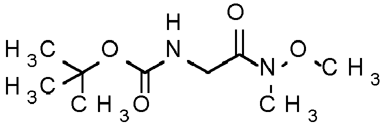

7.30 mL (56.00 mmol) of isobutyl chloroformate was added to a solution of 6.53 g (37.30 mmol) of N-BOC-glycine and 8.20 mL (75.00 mmol) of 4-methylmorpholine in 100 mL of dichloromethane at -15 °C and the mixture was stirred for 1 hour. Then 4.00 g (41.00 mmol) of N,O-dimethylhydroxiamine hydrochloride in 20 mL of dichloromethane was added to the solution at -15 °C and the reaction was further stirred at room temperature for 2 hours. The mixture was quenched with water and extracted with dichloromethane. The combined organic layers were dried over magnesium sulfate, filtered and concentrated. The corresponding residue was purified by flash chromatography on silica gel eluting with dichloromethane/methanol mixtures to afford 4.62 g (57%) of the product as a white powder.
¹H-NMR (300 MHz, DMSO-*d*₆): δ [ppm] = 137 (s, 9H), 3.08 (s, 3H), 3.67 (s, 3H), 3.82 (d, 2H), 6.82 (bp, 1H).
LC-MS (Method 3): Rₜ = 0.499 min. MS (Mass method 1): m/z = 163 (M - tBu + H)⁺

### Intermediate 6

### rac-tert-butyl (3-methyl-2-oxopent-4-en-1-yl)carbamate

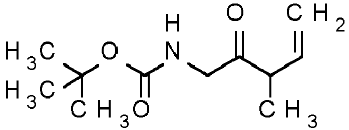

120 mL (58.00 mmol) of 1-methyl-2-propenylmagnesium chloride (0.5M in tetrahydrofuran) were slowly added drop wise at 0 °C to a solution of 3.62 g (16.60 mmol) of tert-butyl {2-[methoxy(methyl)amino]-2-oxoethyl}carbamate in 40 mL of tetrahydrofuran under nitrogen atmosphere and the mixture was stirred at room temperature for 10 hours. The reaction was quenched with saturated ammonium chloride solution in water and the resulting slurry was extracted with acetate. The combined organic extracts were dried over magnesium sulfate, filtered, concentrated and chromatographed in heptane/ethyl acetate mixtures to afford 3.11 g (88%) of the product as a colourless oil.
¹H-NMR (300 MHz, DMSO-*d*₆): δ [ppm] = 1.07 (d, 2H), 1.37 (s, 9H), 1.56 (d, 1H), 3.18 (d, 1H), 3.77 (d, 1H), 3.84 (d, 1H), 5.09-5.20 (m, 1H), 5.45-5.65 (m, 1H), 5.73-5.90 (m, 1H), 6.91-7.05 (m, 1H).
LC-MS (Method 3): Rₜ = 0.745 min. MS (Mass method 1): m/z = 158 (M - tBu + H)⁺

### Intermediate 7

### rac-tert-butyl {[4-(but-3-en-2-yl)-2,5-dioxoimidazolidin-4-yl]methyl}carbamate

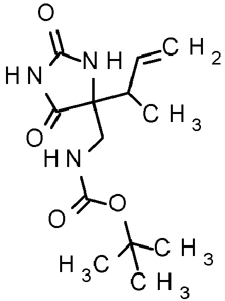

A solution of 3.10 g (14.50 mmol) of rac-tert-butyl (3-methyl-2-oxopent-4-en-1-yl)carbamate in 20 mL of ethanol was added to a solution of 1.89 g (29.10 mmol) of potassium cyanide and 14.00 g (145.00 mmol) of ammonium carbonate in 20 mL of water into a pressure flask, the container was sealed and the mixture was stirred at 60 °C for 24 hours. After that time, the solvent was partially removed and the resulting precipitate was filtered off to give 2.30 g (56%) of the product as a withe solid. The compound was used as such in the next step.
¹H-NMR (300 MHz, DMSO-*d*₆): δ [ppm] = 0.85-1.25 (m, 1H), 1.36 (s, 9H), 1.50-1.60 (m, 3H), 2.15-2.47 (m, 1H), 3.19 (d, 2H), 4.97-5.27 (m, 1H), 5.50-5.70 (m, 1H), 6.57-6.90 (m, 1H), 7.60 (s, 1H), 10.55 (bp, 1H).
LC-MS (Method 3): Rₜ = 0.576 min. MS (Mass method 1): m/z = 228 (M - tBu + H)⁺

### Intermediate 8

### rac-5-(aminomethyl)-5-[but-3-en-2-yl]imidazolidine-2,4-dione hydrochloride

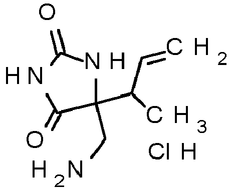

2.63 g (9.28 mmol) of rac-tert-butyl {[4-(but-3-en-2-yl)-2,5-dioxoimidazolidin-4-yl]methyl}carbamate was dissolved in 30 mL of dichloromethane and 12.00 mL (46.00 mmol) of 4M hydrochloric acid in dioxane were added and the resulting suspension was stirred at room temperature for 12 hours. After that time, the solvent was removed by bubbling a stream of nitrogen through the suspension to give 2.00 g (98%) of the product a solid. The compound was used as such in the next step.
¹H-NMR (300 MHz, DMSO-*d*₆): δ [ppm] = 0.85-1.10 (m, 1H), 1.55-1.68 (m, 3H), 2.35-2.48 (m, 1H), 2.84-3.01 (m, 1H), 3.03-3.20 (m, 1H), 5.06-5.30 (m, 1H), 5.60-5.77 (m, 1H), 8.00 (s, 1H), 8.11 (bp, 3H), 10.92-11.09 (m, 1H).
LC-MS (Method 1): Rₜ = 0.511 min. MS (Mass method 1): m/z = 184 (M + H)⁺

### Intermediate 9

### 1-Azido-3,3-dimethylbutan-2-one

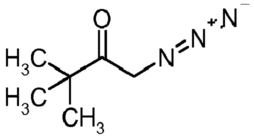

0.78 g (11.98 mmol) of sodium azide were suspended in 30 mL of acetone and 1.65 g (9.21 mmol) of 1-bromopinacolone were added. The resulting mixture was stirred at room temperature for 4 hours. The precipitate was filtered off and discarded and the filtrates were concentrated *in vacuo* to give 1.15 g (88%) of the product as a pale yellow oil. The compound was used as such in the next step.
¹H-NMR (300 MHz, DMSO-*d*₆): δ [ppm] = 1.10 (s, 9H), 4.38 (s, 2H).
LC-MS (Method 3): Rₜ = 0.138 min. MS (Mass method 1): m/z = no ionisation

### Intermediate 10

### rac-5-(azidomethyl)-5-tert-butylimidazolidine-2,4-dione

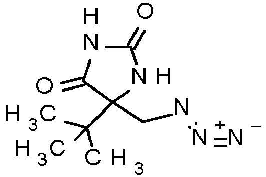

To a stirring solution of 25.52 g (0.26 mol) of ammonium carbonate and 3.79 g (0.07 mol) of ammonium chloride in 30 mL of water previously placed in a sealed tube was added a solution of 2.50 g (0.02 mol) of 1-azido-3,3-dimethylbutan-2-one in 30 mL of ethanol. After 15 min of stirring at room temperature, 5.19 g (0.08 mol) of potassium cyanide were added and the flask was sealed and stirred at 60 °C for 16 hours. After that time, the organic solvent was removed and the resulting precipitate was filtered off and washed with water to give 2.40 g (64%) of the product as a light brown solid. The compound was used as such in the next step.
¹H-NMR (300 MHz, DMSO-*d*₆): δ [ppm] = 0.93 (s, 9H), 3.65 (dd, 2H), 8.13 (s, 1H), 10.72 (s, 1H).
LC-MS (Method 3): Rₜ = 0.398 min. MS (Mass method 1): m/z = 212 (M + H)⁺

### Intermediate 11

### rac-5-(aminomethyl)-5-tert-butylimidazolidine-2,4-dione

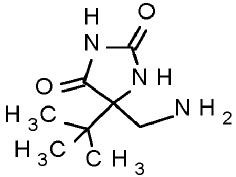

2.40 g (11.40 mmol) of rac-5-(azidomethyl)-5-tert-butylimidazolidine-2,4-dione and 2.98 g (11.40 mmol) of triphenylphosphine were dissolved in 40 mL of tetrahydrofuran and 8 mL of water and the mixture was stirred at 65 °C for 4 h. The solvent was removed and the pure compound was precipitated with dichloromethane/methanol 9:1 to give 1.30 g (61%) of the product as a white powder. The compound was used in the next step without further purification.
¹H-NMR (300 MHz, DMSO-*d*₆): δ [ppm] = 0.91 (s, 9H), 1.22 (bp, 2H), 2.73 (d, 1H), 2.93 (d, 1H), 7.59 (s, 1H), 10.39 (bp, 1H).
LC-MS (Method 3): Rₜ = 0.239 min. MS (Mass method 1): m/z = 186 (M + H)⁺

### Intermediate 12

### tert-Butyl (3-cyclopropyl-2-oxopropyl)carbamate

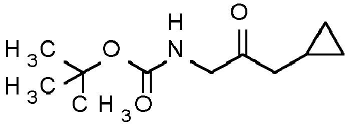

In a flame-dried three-necked round-bottom flask equipped with a constant-pressure addition funnel under nitrogen atmosphere were placed 1.66 g (68.10 mmol) of magnesium turnings and 70 mL of anhydrous tetrahydrofuran was added *via* syringe. 5.70 mL (59.00 mmol) of cyclopropylmethyl bromide in 10 mL of anhydrous tetrahydrofuran was placed in the funnel and added drop wise to the mixture. Once the addition finished, the reaction was stirred at 65 °C for 1 hour. After that time, the mixture was allowed to stand and the supernatant solution (c.a. 0.842M) was added drop wise to a solution of 5.00 g (22.90 mmol) of tert-butyl {2-[methoxy(methyl)amino]-2-oxoethyl}carbamate in 20 mL of anhydrous tetrahydrofuran at 0 °C under nitrogen atmosphere. The resulting mixture was warmed to room temperature and stirred under the same conditions for 12 hours. The reaction was then concentrated, partitioned between ethyl acetate and aqueous ammonium chloride(sat.) and extracted with ethyl acetate. The combined organic extracts were dried over magnesium sulfate, filtered, concentrated and purified by flash column chromatography on silica gel eluting with heptane/ethyl acetate to give 3.75 g (77%) of the product as a yellowish oil.
¹H-NMR (300 MHz, DMSO-*d*₆): δ [ppm] = 0.80-0.90 (m, 1H), 1.38 (s, 9H), 2.15-2.28 (m, 2H), 2.43-2.50 (m, 1H), 3.73 (d, 2H), 4.88-5.08 (m, 2H), 5.70-5.88 (m, 1H), 7.04 (t, 1H).
LC-MS (Method 3): Rₜ = 0.725 min. MS (Mass method 1): m/z = 159 (M - tBu + H)⁺

### Intermediate 13

### rac-tert-butyl {[4-(cyclopropylmethyl)-2,5-dioxoimidazolidin-4-yl]methyl}carbamate

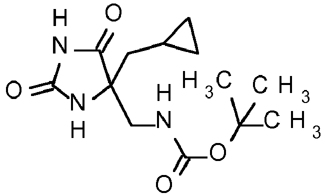

A solution of 3.75 g (17.60 mmol) of tert-butyl (3-cyclopropyl-2-oxopropyl)carbamate in 20 mL of ethanol was added to a solution of 2.29 g (35.20 mmol) of potassium cyanide and 16.90 g (176.00 mmol) of ammonium carbonate in 30 mL of water into a pressure flask, the container was sealed and the mixture was stirred at 60 °C for 12 hours. The organic solvent was removed *in vacuo* and the resulting precipitate was collected by filtration and washed with water. 2.86 g (57%) of the product were obtained as an off-white powder. The compound was used as such in the next step.
¹H-NMR (300 MHz, DMSO-*d*₆): δ [ppm] = 1.37 (s, 9H), 1.50-1.73 (m, 2H), 1.79-1.90 (m, 1H), 1.95-2.10 (m, 1H), 3.17 (d, 2H), 4.80-5.05 (m, 2H), 5.65-5.85 (m, 1H), 6.83 (t, 1H), 7.59 (s, 1H).
LC-MS (Method 3): Rₜ = 0.542 min. MS (Mass method 1): m/z = 228 (M -tBu + H)⁺

### Intermediate 14

### rac-5-(aminomethyl)-5-(cyclopropylmethyl)imidazolidine-2,4-dione hydrochloride

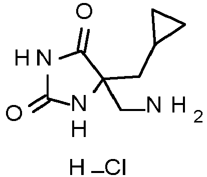

2.75 g (9.71 mmol) of *rac*-tert-butyl {[4-(cyclopropylmethyl)-2,5-dioxoimidazolidin-4-yl]methyl}carbamate were dissolved in 55 mL of dichloromethane and 12.00 mL (49.00 mmol) of 4N hydrochloric acid in dioxane was added. The resulting yellowish solution was allowed to stir at room temperature for 12 hours. The solvent was removed under reduced pressure and the resulting precipitate was filtered off, washed with dichloromethane, ethyl acetate and diethyl ether to give 1.92 g (89%) of the product as a white powder. The compound was used as such in the next step
¹H-NMR (300 MHz, DMSO-*d*₆): δ [ppm] = 1.70-1.93 (m, 3H), 2.00-2.10 (m, 1H), 2.95 (d, 1H), 3.11 (d, 1H), 4.93-5.10 (m, 2H), 5.69-5.84 (m, 1H), 8.00 (s, 1H), 8.22 (bp, 1H), 11.02 (s, 1H).
LC-MS (Method 1): Rₜ = 0.500 min. MS (Mass method 1): m/z = 184 (M + H)⁺

### Intermediate 15

### Benzyl (3-cyclopropyl-2-oxopropyl)carbamate

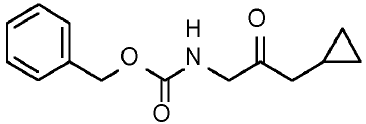

In a flame-dried three-necked round-bottom flask equipped with a constant-pressure addition funnel under nitrogen atmosphere were placed 1.66 g (68.10 mmol) of magnesium turnings and 70 mL of anhydrous tetrahydrofuran was added *via* syringe. 5.70 mL (59.00 mmol) of cyclopropylmethyl bromide in 10 mL of anhydrous tetrahydrofuran was placed in the funnel and added drop wise to the mixture. Once the addition finished, the reaction was stirred at 65 °C for 1 hour. After that time, the mixture was allowed to stand and the supernatant solution (c.a. 0.842M) was added drop wise to a solution of 9.10 g (20.00 mmol) of benzyl {2-[methoxy(methyl)amino]-2-oxoethyl}carbamate in 35 mL of anhydrous tetrahydrofuran at 0 °C under nitrogen atmosphere. The resulting mixture was warmed to room temperature and stirred under the same conditions for 12 hours. The reaction was then concentrated, partitioned between ethyl acetate and aqueous ammonium chloride(sat.) and extracted with ethyl acetate. The combined organic extracts were dried over magnesium sulfate, filtered, concentrated and purified by flash column chromatography on silica gel eluting with heptane/ethyl acetate to give 4.62 g (94%) of the product as a yellowish oil.
¹H-NMR (300 MHz, DMSO-*d*₆): δ [ppm] = 2.15-2.30 (m, 2H), 3.86 (d, 2H), 4.93-5.00 (m, 2H), 5.04 (s, 2H), 5.70-5.90 (m, 1H), 7.25-7.40 (m, 5H), 7.50 (t, 1H).
LC-MS (Method 3): Rₜ = 0.769 min. MS (Mass method 1): m/z = 248 (M + H)⁺

### Intermediate 16

### rac-Benzyl {[4-(cyclopropylmethyl)-2,5-dioxoimidazolidin-4-yl]methyl}carbamate

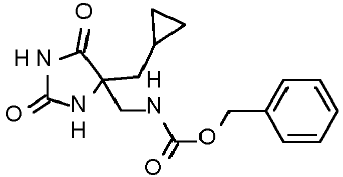

A solution of 4.60 g (18.60 mmol) of benzyl (3-cyclopropyl-2-oxopropyl)carbamate in 20 mL of ethanol was added to a solution of 2.42 g (37.20 mmol) of potassium cyanide and 17.87 g (186.01 mmol) of ammonium carbonate in 20 mL of water into a pressure flask, the container was sealed and the mixture was stirred at 60 °C for 12 hours. After that time, the organic solvent was removed *in vacuo* and the resulting precipitate was filtered off, washed with cold water and dried under reduced pressure to give 4.02 g (68%) of the product as white needles.
¹H-NMR (300 MHz, DMSO-*d*₆): δ [ppm] = 1.58-1.72 (m, 2H), 1.79-1.89 (m, 1H), 1.90-2.07 (m, 1H), 3.25 (t, 2H), 4.90-5.10 (m, 5H), 5.65-5.88 (m, 1H), 7.25-7.45 (m, 5H), 7.72 (s, 1H).
LC-MS (Method 3): Rₜ = 0.606 min. MS (Mass method 1): m/z = 318 (M + H)⁺

### Intermediate 17

### rac-5-(aminomethyl)-5-butylimidazolidine-2,4-dione

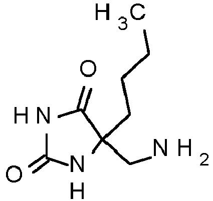

4.80 g (15.10 mmol) of rac-Benzyl {[4-(cyclopropylmethyl)-2,5-dioxoimidazolidin-4-yl]methyl}carbamate was dissolved in 40 mL of methanol and 0.16 g (1.51 mmol) of palladium on carbon (Degussa type, 10% loading, wet basis) was added allowing the suspension to stir at room temperature under hydrogen atmosphere for 12 hours. After that time, the black slurry was filtered through a pad of celite and the filtrates were concentrated under vacuum to afford 1.42 g (50%) of the product as a white powder. The compound was used as such in the next step.
¹H-NMR (300 MHz, DMSO-*d*₆): δ [ppm] = 0.80-0.88 (m, 4H), 1.00-1.10 (m, 1H), 1.17-1.29 (m, 4H), 1.40-1.51 (m, 2H), 2.60 (d, 1H), 2.71 (d, 1H), 7.63 (s, 1H).
LC-MS (Method 1): Rₜ = 0.760 min. MS (Mass method 1): m/z = 186 (M + H)⁺

### Intermediate 18

### tert-Butyl (2-oxopentyl)carbamate

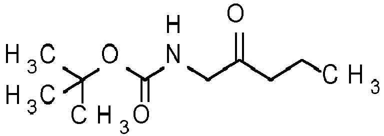

6.90 mL (14.00 mmol) of propylmagnesium chloride (2M in diethyl ether) was slowly added drop wise at 0 °C to a solution of 1.50 g (6.87 mmol) of tert-butyl {2-[methoxy(methyl)amino]-2-oxoethyl}carbamate in 10 mL of anhydrous tetrahydrofuran under nitrogen atmosphere and the mixture was stirred at room temperature for 10 hours. The reaction was quenched with saturated ammonium chloride(aq.) and the resulting slurry was extracted with ethyl acetate. The combined organic extracts were dried over magnesium sulfate, filtered, concentrated and chromatographed in heptane/ethyl acetate mixtures (potassium permanganate stain required) to afford 0.55 g (40%) of the expected compound as a colourless oil.
¹H-NMR (300 MHz, DMSO-*d*₆): δ [ppm] = 0.84 (t, 3H), 1.38 (s, 9H), 1.47 (q, 2H), 2.35 (t, 2H), 3.71 (d, 2H), 7.01 (t, 1H).
LC-MS (Method 3): Rₜ = 0.682 min. MS (Mass method 1): m/z = 224 (M + Na)⁺

### Intermediate 19

### rac-tert-Butyl {[2,5-dioxo-4-propylimidazolidin-4-yl]methyl}carbamate

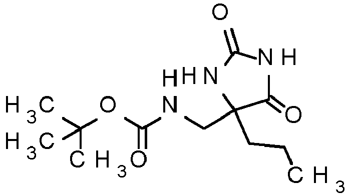

To a stirring solution of 3.94 g (41.00 mmol) of ammonium carbonate and 0.73 g (13.70 mmol) of ammonium chlroride in 10 mL of water placed into a sealed flask, was added 0.55 g (2.73 mmol) of tert-butyl (2-oxopentyl)carbamate in 10 mL of ethanol. After 15 min, 0.80 g (12.30 mmol) of potassium cyanide was added and the mixture was heated at 60 °C for 16 hours. The organic solvent was removed and the resulting aqueous phase was allowed to stand at 0 °C for 12 hours. A white precipitate appeared after that time, which was collected by filtration giving 0.60 g (81%) of the product as a white crystalline solid. The compound was used as such in the next step.
¹H-NMR (300 MHz, DMSO-*d*₆): δ [ppm] = 0.84 (t, 3H), 0.99-1.10 (m, 1H), 1.23-1.33 (m, 1H), 1.36 (s, 9H), 1.40-1.55 (m, 2H), 3.12 (d, 2H), 6.78 (bp, 1H), 7.55 (s, 1H), 10.55 (bp, 1H).
LC-MS (Method 3): Rₜ = 0.513 min. MS (Mass method 1): m/z = 272 (M + H)⁺

### Intermediate 20

### rac-5-(aminomethyl)-5-propylimidazolidine-2,4-dione hydrochloride

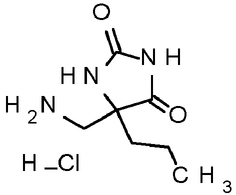

2.80 mL (11.00 mmol) of 4M hydrochloric acid in dioxane were added to a solution of 0.60 g (2.21 mmol) of *rac*-tert-Butyl {[2,5-dioxo-4-propylimidazolidin-4-yl]methyl}carbamate in 20 mL of dichloromethane and the mixture was stirred at room temperature for 72 hours. The resulting precipitate was filtered off and washed with dichloromethane, ethyl acetate and diethyl ether to afford 0.45 g (98%) of the product as a white powder. The compound was used in the next step without further purification.
¹H-NMR (300 MHz, DMSO-*d*₆): δ [ppm] = 0.89 (t, 3H), 1.00-1.16 (m, 1H), 1.20-1.37 (m, 1H), 1.50-1.68 (m, 2H), 2.90 (d, 1H), 3.08 (d, 1H), 7.94 (s, 1H), 8.09 (bp, 3H), 10.99 (bp, 1H).
LC-MS (Method 3): Rₜ = 0.122 min. MS (Mass method 1): m/z = 172 (M + H)⁺

### Intermediate 21

### tert-Butyl (2-oxopent-4-en-1-yl)carbamate

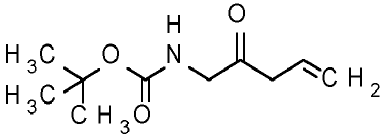

To a solution of 1.50 g (6.87 mmol) of tert-butyl {2-[methoxy(methyl)amino]-2-oxoethyl}carbamate in 20 mL of anhydrous tetrahydrofuran under inert atmosphere at 0 °C was added drop wise 17.00 mL (17.00 mmol) of allylmagnesium bromide solution (1M in tetrahydrofuran) and the mixture was stirred at room temperature for 12 hours. The solvent was partially removed then the mixture was diluted with ethyl acetate and quenched with saturated ammonium chloride aqueous solution. The organic extract was isolated, dried over magnesium sulfate, filtered and concentrated to afford a residue, which was purified by flash column chromatography on silica gel eluting with heptane/ethyl acetate mixtures to give 1.20 g (87%) of the product as a yellowish oil.
LC-MS (Method 3): Rₜ = 0.623 min. MS (Mass method 1): m/z = 222 (M + Na)⁺

### Intermediate 22

### rac-tert-Butyl {[2,5-dioxo-4-(prop-2-en-1-yl)imidazolidin-4-yl]methyl}carbamate

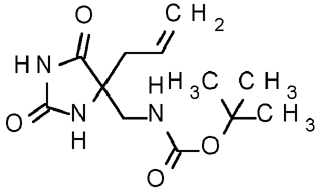

5.79 g (60.20 mmol) of ammonium carbonate was placed into a sealed flask and 10 mL of water were added. To this mixture, was added a solution of 1.20 g (6.02 mmol) of tert-butyl (2-oxopent-4-en-1-yl)carbamate in 10 mL of ethanol. After 15 min of stirring, 0.78 g (12.00 mmol) of potassium cyanide were added, the flask was sealed and the reaction was heated up to 60 °C for 16 hours. The organic solvent was removed and the resulting aqueous phase was allowed to stand at 0 °C for 12 hours. A white precipitate appeared after that time, which was collected by filtration giving 0.44 g (27%) of the product as a white crystalline solid. The compound was used as such in the next step.
¹H-NMR (300 MHz, DMSO-*d*₆): δ [ppm] = 1.36 (s, 9H), 2.28 (d, 2H), 3.17 (d, 2H), 5.05-5.17 (m, 2H), 5.50-5.70 (m, 1H), 6.83 (t, 1H), 7.62 (s, 1H), 10.57 (s, 1H).
LC-MS (Method 3): Rₜ = 0.479 min. MS (Mass method 1): m/z = 214 (M - tBu + H)⁺

### Intermediate 23

### rac-5-(aminomethyl)-5-(prop-2-en-1-yl)imidazolidine-2,4-dione hydrochloride

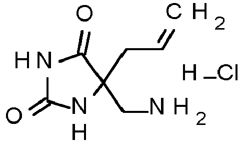

0.44 g (1.64 mmol) of *rac*-tert-Butyl {[2,5-dioxo-4-(prop-2-en-1-yl)imidazolidin-4-yl]methyl}carbamate were dissolved in 15 mL of dichloromethane and 2.10 mL (8.20 mmol) of 4M hydrochloric acid in dioxane was added, allowing the resulting yellowish solution to stir at room temperature for 12 hours. The solvent was then partially removed by bubbling nitrogen through the solution and the resulting precipitate was filtered off and washed with dichloromethane, ethyl acetate and diethyl ether giving 0.18 g (54%) of the product as an off-white powder.
¹H-NMR (300 MHz, DMSO-*d*₆): δ [ppm] = 2.41 (d, 2H), 2.93 (d, 1H), 3.12 (d, 1H), 5.18 (d, 2H), 5.50-5.70 (m, 1H), 8.00 (s, 1H), 8.21 (bp, 3H), 10.98 (bp, 1H).

### Intermediate 24

### tert-Butyl (2-cyclopentyl-2-oxoethyl)carbamate

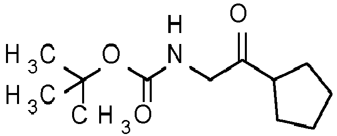

To a solution of 2.20 g (10.10 mmol) of tert-butyl {2-[methoxy(methyl)amino]-2-oxoethyl}carbamate in 10 mL of anhydrous tetrahydrofuran under inert atmosphere at 0 °C was added dropwise 13.00 mL (25.00 mmol) of cyclopentylmagnesium chloride solution (2M in tetrahydrofuran) and the reaction was stirred at room temperature for 12 hours. The solvent was partially removed and the mixture was diluted with ethyl acetate and quenched with saturated ammonium chloride(aq.). The organic layer was isolated, dried over magnesium sulfate, filtered and concentrated to afford a residue, which was purified by flash column chromatography on silica gel eluting with heptane/ethyl acetate to give 0.58 g (25%) of the product as a yellowish oil.
¹H-NMR (300 MHz, DMSO-*d*₆): δ [ppm] = 1.37 (s, 9H), 1.49-1.67 (m, 6H), 1.68-1.84 (m, 2H), 2.85-3.00 (m, 1H), 3.80 (d, 2H), 6.98 (t, 1H).
LC-MS (Method 3): Rₜ = 0.795 min. MS (Mass method 1): m/z = 172 (M - tBu + H)⁺

### Intermediate 25

### rac-tert-Butyl {[4-cyclopentyl-2,5-dioxoimidazolidin-4-yl]methyl}carbamate

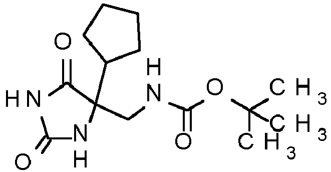

A solution of 0.58 g (2.56 mmol) of tert-butyl (2-cyclopentyl-2-oxoethyl)carbamate in 5 mL of ethanol was added to another solution of 0.33 g (5.12 mmol) of potassium cyanide and 2.46 g (25.60 mmol) of ammonium carbonate in 5 mL of water into a pressure flask, the container was sealed and the mixture was stirred at 60 °C for 12 hours. The ethanol was removed under reduced pressure and the resulting precipitate was collected by filtration, washed with water and dried *in vacuo* to give 0.46 g (60%) of the product as white solid.
¹H-NMR (300 MHz, DMSO-*d*₆): δ [ppm] = 1.09-1.31 (m, 2H), 1.36 (s, 9H), 1.40-1.60 (m, 5H), 1.61-1.75 (m, 1H), 2.01-2.16 (m, 1H), 3.20-3.25 (m, 2H), 6.74 (t, 1H), 7.61 (s, 1H), 10.53 (bp, 1H).
LC-MS (Method 3): Rₜ = 0.601 min. MS (Mass method 1): m/z = 320 (M + Na)⁺

### Intermediate 26

### rac-5-(aminomethyl)-5-cyclopentylimidazolidine-2,4-dione hydrochloride

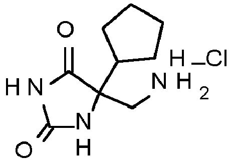

1.90 mL (7.70 mmol) of 4N hydrochloric acid in dioxane were added to a solution of 0.46 g (1.55 mmol) of *rac*-tert-Butyl {[4-cyclopentyl-2,5-dioxoimidazolidin-4-yl]methyl}carbamate in 10 mL of dichloromethane and the mixture was allowed to stir at room temperature for 16 hours. The resulting precipitate was collected by filtration and washed with dichloromethane, ethyl acetate and diethyl ether to give 0.32 g (88%) of the product as a yellow sticky solid. The compound was used as such in the next step.
¹H-NMR (300 MHz, DMSO-*d*₆): δ [ppm] = 1.13-1.38 (m, 2H), 1.40-1.63 (m, 5H), 1.65-1.77 (m, 1H), 2.10-2.25 (m, 1H), 2.95 (d, 1H), 3.12 (d, 1H), 8.09 (s, 1H), 8.20 (bp, 3H), 10.99 (bp, 1H).
LC-MS (Method 1): Rₜ = 0.801 min. MS (Mass method 1): m/z = 198 (M + H)⁺

### Intermediate 27

### 2-Bromo-1-cyclobutylethan-1-one

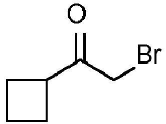

1.31 mL (25.47 mmol) of bromine were added drop wise to a solution of 2.50 g (25.47 mmol) of cyclobutyl methyl ketone in 25 mL of methanol at 0 °C and the solution was then stirred for 1 hour under the same conditions. The solvent was removed under vacuum and the mixture was partitioned between ethyl acetate and water. The organic layer was isolated, dried over magnesium sulfate, filtered and concentrated to afford 3.80 g (84%) of the product as a dark oil. The compound was used as such in the next step.
¹H-NMR (300 MHz, DMSO-*d*₆): δ [ppm] 1.62-1.81 (m, 1H), 1.84-2.02 (m, 1H), 2.05-2.20 (m, 4H), 3.42-3.58 (m, 1H), 4.51 (s, 2H).
LC-MS (Method 1): Rₜ = 2.665 min. MS (Mass method 1): m/z = no ionisation.

### Intermediate 28

### 2-Azido-1-cyclobutylethan-1-one

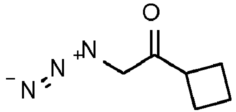

0.75 g (4.24 mmol) of 2-bromo-1-cyclobutylethan-1-one were added to a suspension of 0.36 g (5.51 mmol) of sodium azide in 20 mL of acetone and the mixture was stirred at room temperature for 16 hours. The resulting precipitate was filtered and the filtrate concentrated to give 0.43 g (73%) of the product as a pale orange oil. The compound was used as such in the next step.
¹H-NMR (300 MHz, DMSO-*d*₆): δ [ppm] = 01.68-1.83 (m, 1H), 1.84-2.01 (m, 1H), 2.02-2.22 (m, 4H), 3.22-3.32 (m, 1H), 4.17 (, 1H).
LC-MS (Method 3): Rₜ = 0.570 min. MS (Mass method 1): m/z = no ionisation.

### Intermediate 29

### rac-5-(azidomethyl)-5-cyclobutylimidazolidine-2,4-dione

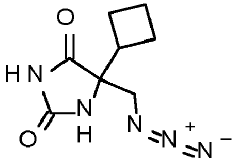

A solution of 0.43 g (3.10 mmol) of 2-azido-1-cyclobutylethan-1-one in 5 mL of ethanol was added to a solution of 0.41 g (6.21 mmol) of potassium cyanide and 2.98 g (31.00 mmol) of ammonium carbonate in 5 mL water into a pressure flask. The container was sealed and the mixture was stirred at 60 °C for 12 hours. The ethanolic fraction was removed under reduced pressure and the mixture was extracted with ethyl acetate. The combined organic extracts were dried over magnesium sulfate, filtered and concentrated and the resulting precipitate was filtered off, washed with dichloromethane and dried *in vacuo* to give 0.23 g (35%) of the product as an orange solid.
¹H-NMR (300 MHz, DMSO-*d*₆): δ [ppm] = 1.58-1.95 (m, 6H), 2.52-2.65 (m, 1H), 3.39 (s, 2H), 8.26 (s, 1H), 10.76 (bp, 1H).

### Intermediate 30

### rac-5-(aminomethyl)-5-cyclobutylimidazolidine-2,4-dione

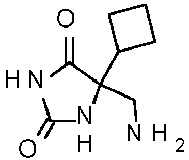

230 mg (1.10 mmol) of rac-5-(azidomethyl)-5-cyclobutylimidazolidine-2,4-dione were dissolved in 15 mL of methanol in a round-bottom flask with a stirrer then the system was purged with nitrogen. 23 mg (0.22 mmol) of palladium on carbon (Degussa type, 10% loading, wet basis) was added the mixture was stirred at room temperature under hydrogen atmosphere for 12 hours. The black suspension was filtered through a pad of celite and the filtrates were concentrated to afford a pale-orange solid, which was washed with dichloromethane/methanol (9:1) give 180 mg (90%) of the product as white crystals.
¹H-NMR (300 MHz, DMSO-*d*₆): δ [ppm] = 1.59-1.91 (m, 6H), 2.53-2.59 (m, 1H), 2.64 (d, 2H), 7.82 (s, 1H), 10.51 (bp, 1H).
LC-MS (Method 3): Rₜ = 0.119 min. MS (Mass method 1): m/z = 184 (M + H)⁺

### Intermediate 31

### tert-Butyl (2-cyclohexyl-2-oxoethyl)carbamate

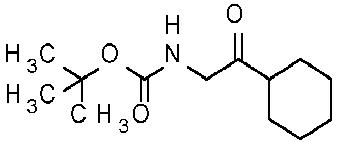

38.00 mL (38.00 mmol) of cyclohexylmagnesium chloride (1M is tetrahydrofuran) were added drop wise to a solution of 3.30 g (15.10 mmol) of tert-butyl {2-[methoxy(methyl)amino]-2-oxoethyl}carbamate in 40 mL of anhydrous tetrahydrofuran at 0°C the reaction was allowed to stir at room temperature for 12 hours. The solvent was partially removed, the mixture was diluted with ethyl acetate and the reaction was quenched with saturated ammonium chloride(aq.). The organic extract was isolated, dried over magnesium sulfate, filtered and concentrated to afford a residue, which was purified by flash column chromatography on silica gel eluting with heptane/ethyl acetate to give 1.70 g (47%) of the product as a yellowish oil.
¹H-NMR (300 MHz, DMSO-*d*₆): δ [ppm] = 1.11-1.27 (m, 5H), 1.37 (s, 9H), 1.56-1.78 (m, 5H), 2.37-2.48 (m, 1H), 3.80 (d, 2H), 6.93 (t, 1H).
LC-MS (Method 3): Rₜ = 0.868 min. MS (Mass method 1): m/z = 142 (M - tBu + H)⁺

### Intermediate 32

### rac-tert-Butyl {[4-cyclohexyl-2,5-dioxoimidazolidin-4-yl]methyl}carbamate

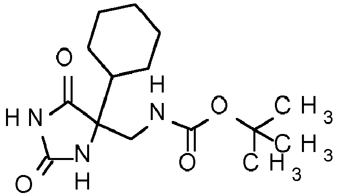

A solution of 1.70 g (7.04 mmol) of tert-butyl (2-cyclohexyl-2-oxoethyl)carbamate in 10 mL of ethanol was added to a solution of 0.92 g (14.10 mmol) of potassium cyanide and 6.77 g (70.40 mmol) of ammonium carbonate in 10 mL water into a pressure flask. The container was sealed and the mixture was stirred at 60 °C for 12 hours. The ethanolic fraction was removed under reduced pressure and the resulting precipitate was collected by filtration, washed with water and dried to give 1.68 g (77%) of the product as a white solid.
¹H-NMR (300 MHz, DMSO-*d*₆): δ [ppm] = 0.88-1.25 (m, 5H), 1.36 (s, 9H), 1.39-1.49 (m, 1H), 1.51-1.78 (m, 5H), 3.13-3.31 (m, 2H), 6.65 (t, 1H), 7.57 (s, 1H), 10.52 (s, 1H).
LC-MS (Method 3): Rₜ = 0.659 min. MS (Mass method 1): m/z = 256 (M - tBu + H)⁺

### Intermediate 33

### rac-5-(aminomethyl)-5-cyclohexylimidazolidine-2,4-dione hydrochloride

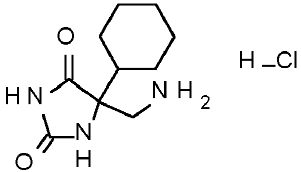

6.70 mL (27.00 mmol) of 4N hydrochloric acid in dioxane were added to a solution of 1.68 g (5.40 mmol) of rac-tert-Butyl {[4-cyclohexyl-2,5-dioxoimidazolidin-4-yl]methyl}carbamate in 30 mL of dichloromethane and the mixture was allowed to stir at room temperature for 16 hours. The resulting precipitate was collected by filtration and washed with dichloromethane, ethyl acetate and diethyl ether to give 1.33 g (99%) of the product as a yellow sticky solid. The compound was used as such in the next step.
¹H-NMR (300 MHz, DMSO-*d*₆): δ [ppm] = 0.90-1.29 (m, 5H), 1.38-1.52 (m, 1H), 1.54-1.81 (m, 5H), 3.06 (1, 2H), 8.01 (s, 1H), 8.14 (bp, 3H), 10.96 (bp, 1H).
LC-MS (Method 1): Rₜ = 1.152 min. MS (Mass method 1): m/z = 212 (M + H)⁺

### Intermediate 34

### rac-Potassium 3-ethoxy-2-methyl-3-oxopropanoate

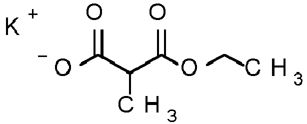

A solution of 1.84 g (27.90 mmol) of potassium hydroxide in 50 mL of absolute ethanol was added to a stirring solution of 5.00 mL (29.00 mmol) of diethyl methylmalonate in 20 mL of absolute ethanol and the mixture was refluxed for 2 hour. After completion of the reaction (as judged by TLC), the mixture was cooled down to room temperature and the solvent was concentrated to dryness to give an oil which crystallised upon standing. The resulting crystals were washed with diisoporpyl ether to afford 4.90 g (91%) of the product as a white crystalline solid.
¹H-NMR (300 MHz, DMSO-*d*₆): δ [ppm] = 1.07-1.16 (m, 6H), 2.99 (q, 1H), 3.97 (q, 2H).

### Intermediate 35

### rac-Ethyl 4-[(tert-butoxycarbonyl)amino]-2-methyl-3-oxobutanoate

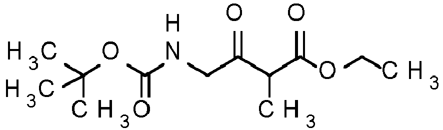

1.58 g (9.77 mmol) of 1,1'-carbonyldiimidazole was added to a solution of 1.43 g (8.14 mmol) of N-(tert-butoxycarbonyl)glycine in 20 mL of anhydrous tetrahydrofuran under nitrogen atmosphere and the mixture was stirred at room temperature for 2 hours. Then, a solid mixture of 1.04 g (7.98 mmol) of cobalt(II) dichloride and 2.25 g (12.20 mmol) of potassium rac-3-ethoxy-2-methyl-3-oxopropanoate was incorporated and the reaction was further stirred under the same conditions for 24 hours. The resulting deep blue slurry was quenched with 1N hydrochloric acid provoking a colour change from deep blue to pale red. Ethyl acetate was then added and the mixture was washed with 1N hydrochloric acid, aqueous sodium hydrogen carbonate(sat.) and water. The organic layer was dried over magnesium sulfate, filtered, concentrated and chromatographed in heptane/ethyl acetate mixtures (potassium permanganate stain required) to give 1.00 g (47%) of the product as a colourless oil.
¹H-NMR (300 MHz, DMSO-*d*₆): δ [ppm] = 1.13-1.25 (m, 6H), 1.37 (s, 9H), 3.76 (q, 1H), 3.90 (d, 2H), 4.09 (q, 2H), 7.11 (t, 1H).
LC-MS (Method 3): Rₜ = 0.713 min. MS (Mass method 1): m/z = 204 (M - tBu + H)⁺

### Intermediate 36

### rac-Ethyl 2-[4-{[(tert-butoxycarbonyl)amino]methyl}-2,5-dioxoimidazolidin-4-yl]propanoate

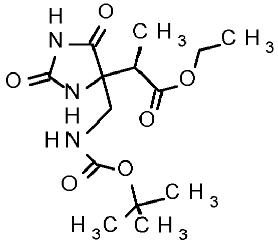

A solution of 1.00 g (3.86 mmol) of ethyl rac-4-[(tert-butoxycarbonyl)amino]-2-methyl-3-oxobutanoate in 15 mL of ethanol was added to a solution of 1.13 g (17.40 mmol) of potassium cyanide and 5.56 g (57.80 mmol) of ammonium carbonate in 15 mL water into a pressure flask. The container was sealed and the mixture was stirred at 60 °C for 12 hours. The ethanolic fraction was removed under reduced pressure and the resulting precipitate was collected by filtration, washed with water and dried to give 0.78 g (62%) of the product as a white solid.
¹H-NMR (300 MHz, DMSO-*d*₆): δ [ppm] = 0.76-0.89 (m, 1.5H), 1.03-1.23 (m, 5.5H), 1.36 (s, 9H), 2.70 (q, 0.5H), 2.85 (q, 0.5H), 3.22 (d, 2H), 3.91-4.11 (m, 2H), 6.51-6.82 (m, 1H), 7.62 (s, 1H), 10.58 (bp, 1H).
LC-MS (Method 3): Rₜ = 0.520, 0.566 min (split peak). MS (Mass method 1): m/z = 274 (M - tBu + H)⁺

### Intermediate 37

### rac-tert-Butyl ({4-[1-hydroxypropan-2-yl]-2,5-dioxoimidazolidin-4-yl}methyl)carbamate

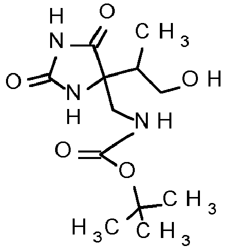

293 mg (7.76 mmol) of sodium borohydride were added to a solution of 730 mg (2.22 mmol) of *rac*-Ethyl 2-[4-{[(tert-butoxycarbonyl)amino]methyl}-2,5-dioxoimidazolidin-4-yl]propanoate in 29 mL of ethanol at 40 °C for 16 hours. After that time, more sodium borohydride was added (293 mg) and the reaction was continued for 16 additional hours. The mixture was concentrated *in vacuo,* diluted with ethyl acetate and partitioned between ethyl acetate and water. The aqueous layer was concentrated to dryness to afford a sticky pale-yellow foam. This foam was dissolved in ethanol provoking the precipitation of the most part of the salts presents. The resulting precipitate was filtered off and thoroughly washed with ethanol. The filtrates were concentrated to afford 680 mg (100%) of the product as a pale-yellow solid.
¹H-NMR (300 MHz, DMSO-*d*₆): δ [ppm] = 0.74 (d, 3H), 1.36 (s, 9H), 1.60 (bp, 2H), 1.69-1.81 (m, 1H), 2.98 (d, 1H), 3.12-3.28 (m, 2H), 4.62 (s, 1H), 5.66 (s, 1H), 5.96 (s, 1H).
LC-MS (Method 3): Rₜ = 0.346 min. MS (Mass method 1): m/z = 232 (M - tBu + H)⁺

### Intermediate 38

### rac-5-(aminomethyl)-5-[1-hydroxypropan-2-yl]imidazolidine-2,4-dione hydrochloride

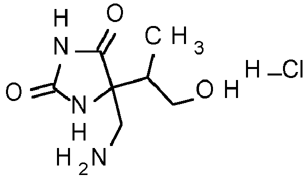

3.00 mL (12.00 mmol) of 4N hydrochloric acid in dioxane were added to a solution of 0.68 g (2.37 mmol) of *rac*-tert-Butyl ({4-[1-hydroxypropan-2-yl]-2,5-dioxoimidazolidin-4-yl}methyl)carbamate in 15 mL of dichloromethane and the mixture was allowed to stir at room temperature for 16 hours. The resulting precipitate was collected by filtration and washed with dichloromethane, ethyl acetate and diethyl ether to give 0.44 g (83%) of the product as a yellow sticky solid. The compound was used as such in the next step.
¹H-NMR (300 MHz, DMSO-*d*₆): δ [ppm] = 0.80 (d, 3H), 1.98-2.06 (m, 1H), 3.06-3.15 (m, 1H), 3.21-3.30 (m, 1H), 3.23-3.40 (m, 2H), 7.99 (s, 1H), 8.25 (bp, 3H), 10.98 (s, 1H).
LC-MS (Method 6): Rₜ = 0.351 min. MS (Mass method 1): m/z = 188 (M + H)⁺

### Intermediate 39

### rac-2-bromo-1-[oxan-3-yl]ethan-1-one

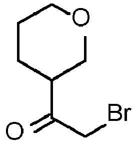

1.00 g (7.68 mmol) of rac-tetrahydro-2H-pyran-3-carboxylic acid were dissolved in 30 mL of dichloromethane. Then, 0.80 mL (9.20 mmol) of oxalyl chloride were added dropwise at room temperature followed by 2 drops of N,N-dimethylformamide (vigorous gas evolution was observed). The reaction mixture was stirred at room temperature for 3.5 hours and then it was concentrated to dryness to afford a yellow oil. The aforementioned residue was dissolved in 15 mL of acetonitrile and 11.00 mL (22.00 mmol) of trimethylsilyldiazomethane (2.0M in hexanes) were added. The reaction mixture was stirred at room temperature for 2 hours and then cooled to 0 °C. 0.40 mL (6.40 mmol) of acetic acid and 4.70 mL (28.00 mmol) of hydrobromic acid (33% in acetic acid) were sequentially added dropwise; again, vigorous gas evolution was observed. After the addition was complete, the ice bath was removed and the mixture was warmed to room temperature and stirred for 2 hours. After that time, the reaction was quenched with water and extracted twice with dichloromethane. The organic layers were combined, dried over magnesium sulfate, filtered and concentrated to afford 0.84 g (53%) of the product which was used without further purification.
¹H-NMR (300 MHz, DMSO-*d*₆): δ [ppm] = 1.40-1.67 (m, 3H), 1.90-2.03 (m, 1H), 2.71-2.94 (m, 1H), 3.24-3.44 (m, 2H), 3.74 (d, 1H), 3.94 (d, 1H), 4.49 (s, 1H), 4.65 (s, 1H).

### Intermediate 40

### rac-1-{2-[oxan-3-yl]-2-oxoethyl}-1,3,5,7-tetraazatricyclo[3.3.1.1^{3,7}]decan-1-ium bromide

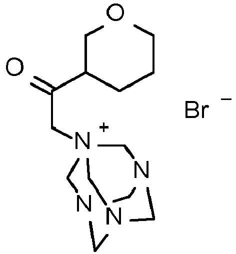

0.84 g (4.06 mmol) of *rac*-2-bromo-1-[oxan-3-yl]ethan-1-one was dissolved in 12 mL of chloroform and 0.57 g (4.06 mmol) of hexamethylenetetramine was added allowing the mixture to stir at room temperature for 12 hours. The solvent was partially removed and the resulting precipitate was filtered off, washed with cold chloroform and isolated. The remaining mother liquors were concentrated again, filtered and washed with cold chloroform several times until no more precipitate was appreciated. All the collected solids were dried *in vacuo* to give 1.40 g (99%) of the product as a white powder. The compound was used as such in the next synthetic step.
LC-MS (Method 3): Rₜ = 0.187 min. MS (Mass method 1): m/z = 267 (M)⁺

### Intermediate 41

### rac-2-amino-1-[oxan-3-yl]ethan-1-one hydrochloride

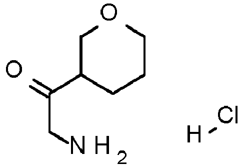

1.40 g (4.03 mmol) of *rac*-1-{2-[oxan-3-yl]-2-oxoethyl}-1,3,5,7-tetraazatricyclo[3.3.1.1^{3,7}]decan-1-ium bromide was dissolved in 9 mL of ethanol and 3 mL of hydrochloric acid(conc). and the mixture was refluxed for 30 min. The resulting precipitate was filtered off, washed with ethanol and discarded. The filtrates were evaporated and dried to yield 0.72 g (99%) of the product as a thick oil. The compound was used as such in the next step.
LC-MS (Method 3): Rₜ = 0.150 min. MS (Mass method 1): m/z = 144 (M + H)⁺

### Intermediate 42

### rac-tert-Butyl {2-[oxan-3-yl]-2-oxoethyl}carbamate

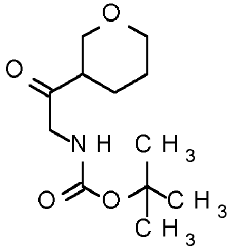

1.10 mL (8.00 mmol) of triethylamine and 1.10 mL (4.80 mmol) of di-tert-butyl dicarbonate were added to a solution of 0.72 g (4.01 mmol) of *rac*-2-amino-1-[oxan-3-yl]ethan-1-one hydrochloride in 15 mL of dichloromethane and the mixture was stirred at room temperature for 12 hours. Water was added to the reaction and the phases were separated. The organic layer was dried over magnesium sulfate, filtered and evaporated. The corresponding residue was purified by flash chromatography on silica gel eluting with heptane and ethyl acetate. 0.38 g (40%) of the product were isolated as a pale yellow oil.
¹H-NMR (300 MHz, DMSO-*d*₆): δ [ppm] = 1.38 (s, 9H), 1.48-1.65 (m, 3H), 1.83-1.94 (m, 1H), 2.65-2.79 (m, 1H), 3.29 (d, 1H), 3.37 (d, 1H), 3.70-3.91 (m, 4H), 6.99 (t, 1H).
LC-MS (Method 3): Rₜ = 0.590 min. MS (Mass method 1): m/z = 266 (M + Na)⁺

### Intermediate 43

### rac-tert-Butyl ({4-[oxan-3-yl]-2,5-dioxoimidazolidin-4-yl}methyl)carbamate

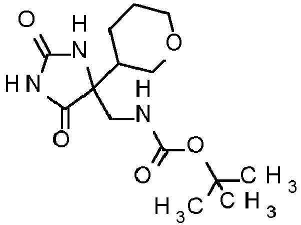

To a stirring solution of 2.25 g (23.40 mmol) of ammonium carbonate and 0.33 g (6.25 mmol) of ammonium chloride in 8 mL of water was added 0.38 g (1.56 mmol) of *rac*-tert-Butyl {2-[oxan-3-yl]-2-oxoethyl}carbamate in 8 mL of ethanol. After 15 min, 0.46 g (7.03 mmol) of potassium cyanide were added and the mixture was heated up to 60 °C for 16 hours into a sealed pressure flask. The yellow solution was concentrated until only a small fraction of water remained (a white precipitate appeared). Then, more water was added and the suspension was allowed to stand at 0 °C for 1 hour. After that time, the resulting solid was filtered off and washed with water and diethyl ether to give 0.18 g (36%) of the product as a white powder. The compound was used as such in the next step.
¹H-NMR (300 MHz, DMSO-*d*₆): δ [ppm] = 1.13-1.31 (m, 1H), 1.37 (s, 9H), 1.43-1.64 (m, 2H), 1.76-1.91 (m, 2H), 3.06-3.27 (m, 4H), 3.61-3.95 (m, 2H), 6.77 (t, 1H), 7.65 (s, 1H), 10.64 (bp, 1H).
LC-MS (Method 3): Rₜ = 0.450 min. MS (Mass method 1): m/z = 258 (M - tBu + H)⁺

### Intermediate 44

### rac-5-(aminomethyl)-5-[oxan-3-yl]imidazolidine-2,4-dione hydrochloride

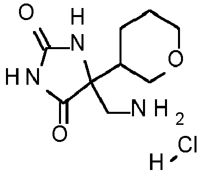

0.72 mL (2.90 mmol) of 4N hydrochloric acid in dioxane were added to a solution of 0.18 g (0.57 mmol) of *rac*-tert-Butyl ({4-[oxan-3-yl]-2,5-dioxoimidazolidin-4-yl}methyl)carbamate in 5 mL of dichloromethane and the mixture was allowed to stir at room temperature for 16 hours. The resulting precipitate was collected by filtration and washed with dichloromethane, ethyl acetate and diethyl ether to give 0.14 g (97%) of the product as a white solid. The compound was used as such in the next step.
¹H-NMR (300 MHz, DMSO-*d*₆): δ [ppm] = 1.16-1.35 (m, 1H), 1.36-1.67 (m, 3H), 1.76-2.02 (m, 2H), 3.08-3.25 (m, 3H), 3.66-3.90 (m, 2H), 8.02-8.18 (m, 4H), 11.09 (s, 1H).
LC-MS (Method 3): Rₜ = 0.121 min. MS (Mass method 1): m/z = 214 (M + H)⁺

### Intermediate 45

### 2-bromo-1-(oxan-4-yl)ethan-1-one

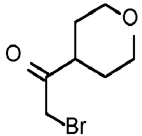

1.20 mL (23.00 mmol) of bromine were added drop wise to a stirred solution of 3.00 g (23.40 mmol) of 1-(oxan-4-yl)ethan-1-one in 15 mL of methanol at -10 °C and the mixture was stirred at 0 °C for 45 min and at 10 °C for 45 min. After this time, an aqueous 11M solution of sulfuric acid (20 ml) was added allowing the mixture to stir at room temperature for 16 hours. Water was added and the reaction was extracted with diethyl ether. The combined organic layers were washed with saturated sodium bicarbonate(aq.) and water. The organic layer was dried over magnesium sulfate, filtered and concentrated to give 3.28 g (68%) of the product as a tan oil. The compound was used as such in the next synthetic step.
¹H-NMR (300 MHz, DMSO-*d*₆): δ [ppm] = 1.49 (qd, 2H), 1.75 (d, 2H), 2.87 (tt, 1H), 3.33 (t, 2H), 3.85 (d, 2H), 4.49 (s, 2H).
LC-MS (Method 3): Rₜ = 0.403 min. MS (Mass method 1): m/z = 206 (M + H)⁺

### Intermediate 46

### 1-[2-(oxan-4-yl)-2-oxoethyl]-1,3,5,7-tetraazatricyclo[3.3.1.1^{3,7}]decan-1-ium bromide

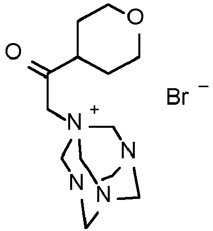

2.09 g (10.10 mmol) of 2-bromo-1-(oxan-4-yl)ethan-1-one was dissolved in 30 mL of chloroform and 1.41 g (10.10 mmol) of hexamethylenetetramine was added allowing the mixture to stir at room temperature for 12 hours. The solvent was partially removed and the resulting precipitate was filtered off, washed with cold chloroform and isolated. The remaining mother liquors were concentrated again, filtered and washed with cold chloroform several times until no more precipitate was appreciated. All the collected solids were dried *in vacuo* to give 2.16 g (62%) of the product as a white powder. The compound was used as such in the next synthetic step.
¹H-NMR (300 MHz, DMSO-*d*₆): δ [ppm] = 1.49 (qd, 2H), 1.77 (2H), 2.74 (tt, 1H), 3.88 (d, 2H), 4.19 (s, 2H), 4.59 (dd, 6H), 5.29 (s, 6H).
LC-MS (Method 3): Rₜ = 0.125 min. MS (Mass method 1): m/z = 267 (M)⁺

### Intermediate 47

### 2-amino-1-(oxan-4-yl)ethan-1-one hydrochloride

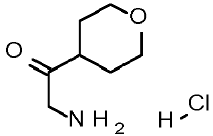

2.16 g (6.22 mmol) of 1-[2-(oxan-4-yl)-2-oxoethyl]-1,3,5,7-tetraazatricyclo[3.3.1.1^{3,7}]decan-1-ium bromide were dissolved in 30 mL of ethanol and 10 mL of hydrochloric acid(conc). and the mixture was refluxed for 30 min. The resulting precipitate was filtered off, washed with ethanol and discarded. The filtrates were evaporated and dried to yield 1.12 g (99%) of the product as a thick oil. The compound was used as such in the next step.
LC-MS (Method 3): Rₜ = 0.116 min. MS (Mass method 1): m/z = 144 (M + H)⁺

### Intermediate 48

### tert-Butyl [2-(oxan-4-yl)-2-oxoethyl]carbamate

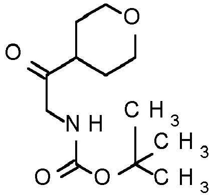

2.03 mL (16.00 mmol) of triethylamine and 1.80 mL (7.70 mmol) of di-tert-butyl dicarbonate were added to a solution of 1.16 g (6.46 mmol) of 2-amino-1-(oxan-4-yl)ethan-1-one hydrochloride in 20 mL of dichloromethane and the mixture was stirred at room temperature for 12 hours. Water was added to the reaction and the phases were separated. The organic layer was dried over magnesium sulfate, filtered and evaporated. The corresponding residue was purified by flash chromatography on silica gel eluting with heptane and ethyl acetate. 1.05 g (67%) of the product were isolated as a colourless oil.
¹H-NMR (300 MHz, DMSO-*d*₆): δ [ppm] = 1.37 (s, 9H), 1.44 (qd, 2H), 1.66 (d, 2H), 2.68 (tt, 1H), 3.32 (t, 2H), 3.78-3.88 (m, 4H), 6.94 (t, 1H).
LC-MS (Method 3): Rₜ = 0.548 min. MS (Mass method 1): m/z = 144 (M - Boc + H)⁺

### Intermediate 49

### rac-tert-Butyl {[4-(oxan-4-yl)-2,5-dioxoimidazolidin-4-yl]methyl}carbamate

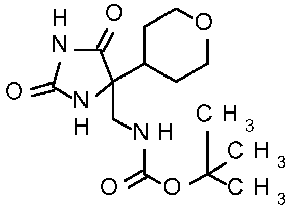

To a stirring solution of 6.22 g (64.70 mmol) of ammonium carbonate and 1.15 g (21.60 mmol) of ammonium chloride in 10 mL of water was added 1.05 g (4.32 mmol) of tert-butyl [2-(oxan-4-yl)-2-oxoethyl]carbamate in 10 mL of ethanol. After 15 min, 1.26 g (19.40 mmol) of potassium cyanide were added and the mixture was heated up to 60 °C for 16 hours into a sealed pressure flask. The yellow solution was concentrated until only a small fraction of water remained (a white precipitate appeared). Then, more water was added and the suspension was allowed to stand at 0 °C for 1 hour. After that time, the resulting solid was filtered off and washed with water and diethyl ether to give 0.92 g (68%) of the product as an off-white powder. The compound was used as such in the next step.
¹H-NMR (300 MHz, DMSO-*d*₆): δ [ppm] = 1.19-1.50 (m, 12H), 1.55 (d, 1H), 1.84 (t, 1H), 3.15-3.28 (m, 3H), 3.84 (t, 2H), 6.73 (t, 1H), 7.66 (s, 1H), 10.58 (s, 1H).
LC-MS (Method 3): Rₜ = 0.428 min. MS (Mass method 1): m/z = 258 (M - tBu + H)⁺

### Intermediate 50

### rac-5-(aminomethyl)-5-(oxan-4-yl)imidazolidine-2,4-dione hydrochloride

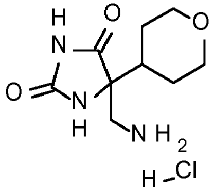

3.70 mL (15.00 mmol) of 4N hydrochloric acid in dioxane were added to a solution of 0.92 g (2.94 mmol) of *rac*-tert-Butyl {[4-(oxan-4-yl)-2,5-dioxoimidazolidin-4-yl]methyl}carbamate in 20 mL of dichloromethane and the mixture was allowed to stir at room temperature for 16 hours. The resulting precipitate was collected by filtration and washed with dichloromethane, ethyl acetate and diethyl ether to give 0.70 g (95%) of the product as a white solid. The compound was used as such in the next step.
¹H-NMR (300 MHz, DMSO-*d*₆): δ [ppm] = 1.19-1.59 (m, 4H), 1.97 (t, 1H), 3.07 (q, 2H), 3.23 (t, 2H), 3.87 (td, 2H), 8.11 (s, 1H), 8.22 (bp, 3H), 11.03 (bp, 1H).
LC-MS (Method 1): Rₜ = 0.331 min. MS (Mass method 1): m/z = 214 (M + H)⁺

### Intermediate 51

### rac-2-nitro-1-[oxolan-3-yl]ethan-1-ol

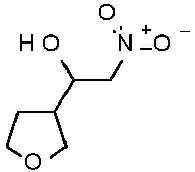

0.57 g (2.50 mmol) of benzyltriethylammonium chloride were added to a solution of 1.40 mL (25.00 mmol) of nitromethane and 4.50 mL of tetrahydrofuran-3-carboxaldehyde (50% in water) in75 mL of 0.025M sodium hydroxide(aq.) and the mixture was stirred at room temperature for 15 hours. After that time, the reaction was saturated with solid sodium chloride and extracted with diethyl ether. The combined organic phases were dried over magnesium sulfate, filtered and concentrated. The corresponding crude nitro alcohol was purified by flash chromatography on silica gel eluting with heptane and ethyl acetate to give 1.73 g (43%) of the product as a light yellow oil.
¹H-NMR (300 MHz, DMSO-*d*₆): δ [ppm] = 1.48-164 (m, 0.5H), 1.70-1.95 (m, 1.5H), 2.17-2.35 (m, 1H), 3.44 (t, 0.5H), 3.53-3.65 (m, 1.5H), 3.66-3.79 (m, 2H), 3.95-4.11 (m, 1H), 4.29-4.42 (m, 1H), 4.70 (ddd, 1H), 5.58 (t, 1H).
LC-MS (Method 3): Rₜ = 0.149 min. MS (Mass method 1): m/z = 162 (M + H)⁺

### Intermediate 52

### rac-2-amino-1-[oxolan-3-yl]ethan-1-ol

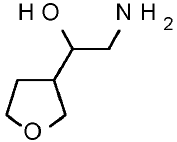

0.09 g (0.83 mmol) of palladium on carbon (10% loading, Degussa type, wet basis) were added to a solution of 1.33 g (8.25 mmol) of *rac*-2-nitro-1-[oxolan-3-yl]ethan-1-ol in 25 mL of methanol and the resulting suspension was stirred at room temperature under hydrogen atmosphere for 16 hours. The reaction was filtered through a pad of celite and the filtrates were evaporated giving 1.07 g (99%) of the crude product as an oil. The compound was used as such in the next step.
LC-MS (Method 3): Rₜ = 0.108 min. MS (Mass method 1): m/z = 132 (M + H)⁺

### Intermediate 53

### rac-tert-Butyl {2-hydroxy-2-[oxolan-3-yl]ethyl}carbamate

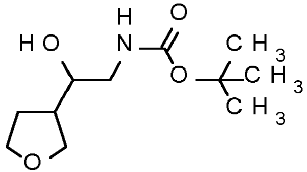

1.90 mL (8.50 mmol) of di-tert-butyl dicarbonate were added to a solution of 1.06 g (8.08 mmol) of rac-2-amino-1-[oxolan-3-yl]ethan-1-ol in 25 mL of dichloromethane and the mixture was stirred at room temperature for 2 hours. Volatiles were removed under reduced pressure and the resulting residue was purified by flash chromatography on silica gel eluting with heptane and ethyl acetate to give 1.36 g (73%) of the product as a thick oil.
¹H-NMR (300 MHz, DMSO-*d*₆): δ [ppm] = 1.49 (s, 9H), 1.45-1.92 (m, 2H), 2.04-2.22 (m, 1H), 2.76-3.12 (m, 2H), 3.47-3.77 (m, 4H), 4.76 (d, 1H), 6.62-6.74 (m, 1H).
LC-MS (Method 3): Rₜ = 0.458 min. MS (Mass method 1): m/z = 254 (M + Na)⁺

### Intermediate 54

### rac-tert-Butyl {2-oxo-2-[oxolan-3-yl]ethyl}carbamate

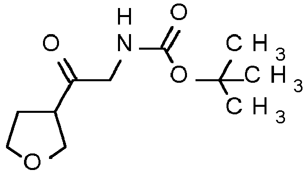

2.16 g (10.00 mmol) of pyridinium chlorochromate were added in portions to a stirred solution of 1.16 g (5.02 mmol) of rac-tert-Butyl {2-hydroxy-2-[oxolan-3-yl]ethyl}carbamate in 15 mL of dichloromethane and the resulting suspension was stirred at room temperature for 15 hours. The reaction was filtered through a pad of celite and the filtrates were washed with water and brine. The organic layer was dried over magnesium sulfate, filtered, concentrated and purified by flash chromatography on silica gel eluting with heptane and ethyl acetate to afford 0.71 g (62%) of the product as a colourless oil.
¹H-NMR (300 MHz, DMSO-*d*₆): δ [ppm] = 1.38 (s, 9H), 1.87-2.08 (m, 2H), 3.23-3.38 (m, 3H), 3.57-3.86 (m, 6H), 7.08 (t, 1H).
LC-MS (Method 3): Rₜ = 0.508 min. MS (Mass method 1): m/z = 252 (M + Na)⁺

### Intermediate 55

### rac-tert-Butyl ({2,5-dioxo-4-[oxolan-3-yl]imidazolidin-4-yl}methyl)carbamate

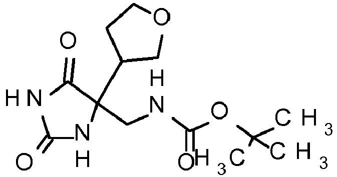

To a stirring solution of 4.46 g (46.50 mmol) of ammonium carbonate and 0.66 g (12.40 mmol) of ammonium chloride in 5 mL of water was added 0.71 g (3.10 mmol) of rac-tert-Butyl {2-oxo-2-[oxolan-3-yl]ethyl}carbamate in 15 mL of ethanol. After 15 min, 0.91 g (13.90 mmol) of potassium cyanide were added and the mixture was heated up to 60 °C for 16 hours into a sealed pressure flask. The yellow solution was concentrated until only a small fraction of water remained (a white precipitate appeared). Then, more water was added and the suspension was allowed to stand at 0 °C for 1 hour. After that time, the resulting solid was filtered off and washed with water and diethyl ether to give 0.64 g (69%) of the product as an off-white powder. The compound was used as such in the next step.
¹H-NMR (300 MHz, DMSO-*d*₆): δ [ppm] = 1.37 (s, 9H), 1.46-2.03 (m, 2H), 3.06-3.29 (m, 3H), 3.38-3.84 (m, 4H), 6.78-6.95 (m, 1H), 7.77 (d, 1H), 10.67 (bp, 1H).
LC-MS (Method 3): Rₜ = 0.347 min. MS (Mass method 1): m/z = 322 (M + Na)⁺

### Intermediate 56

### rac-5-(aminomethyl)-5-[oxolan-3-yl]imidazolidine-2,4-dione hydrochloride

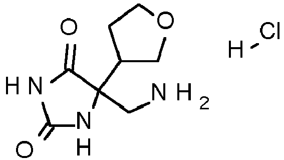

2.60 mL (11.00 mmol) of 4N hydrochloric acid in dioxane were added to a solution of 0.63 g (2.10 mmol) of *rac*-tert-Butyl ({2,5-dioxo-4-[oxolan-3-yl]imidazolidin-4-yl}methyl)carbamate in 10 mL of dichloromethane and the mixture was allowed to stir at room temperature for 16 hours. The resulting precipitate was collected by filtration and washed with dichloromethane, ethyl acetate and diethyl ether to give 0.44 g (89%) of the product as a white solid. The compound was used as such in the next step.
¹H-NMR (300 MHz, DMSO-*d*₆): δ [ppm] = 1.51-2.05 (m, 2H), 2.54-2.69 (m, 1H), 2.90-3.06 (m, 1H), 3.09-3.22 (m, 1H), 3.43-3.84 (m, 4H), 8.15-3.38 (m, 4H), 11.11 (s, 1H).
LC-MS (Method 1): Rₜ = 0.313 min. MS (Mass method 1): m/z = 200 (M + H)⁺

### Intermediate 57

### rac-1-[2,2-dimethylcyclopropyl]-2-nitroethan-1-one

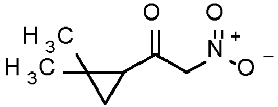

5.00 g (43.80 mmol) of 2,2-dimethylcyclopropanecarboxylic acid and 8.52 g (52.60 mmol) of 1,1'-carbonyldiimidazole where dissolved in 70 mL of N,N-dimethylformamide under nitrogen atmosphere at room temperature and the mixture was stirred for 2 hours. In a separate flask, 3.60 mL (66.00 mmol) of nitromethane were slowly added to a suspension of 2.10 g (52.60 mmol) of sodium hydride in 70 mL of N,N-dimethylformamide under nitrogen atmosphere at room temperature and the mixture was stirred for 2 hours. After that time, this suspension was cooled to 0 °C and the solution containing the activated carboxylic acid was added slowly over 5 min keeping internal temperature below 10 °C. Once the addition finished, the mixture was stirred at 0 °C for 2 hours and at room temperature for 12 additional hours. The reaction mixture was cooled at 0 °C and quenched with 2N hydrochloric acid(aq.) and water. The resulting mixture was extracted with dichloromethane and the combined organic extracts were dried over magnesium sulfate, filtered and concentrated to afford a yellow residue, which was purified by flash column chromatography on silica gel eluting with heptane/ethyl acetate mixtures to give 4.93 g (72%) of the product as a yellowish oil.
¹H-NMR (300 MHz, DMSO-*d*₆): δ [ppm] = 1.06 (s, 4H), 1.21 (s, 4H), 2.02 (t, 1H), 5.92 (d, 2H).
LC-MS (Method 3): Rₜ = 0.631 min. MS (Mass method 1): m/z = 158 (M + H)⁺

### Intermediate 58

### rac-tert-Butyl {2-[2,2-dimethylcyclopropyl]-2-oxoethyl}carbamate

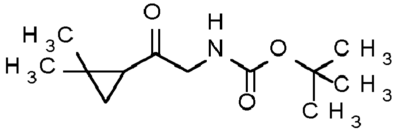

0.03 g (0.29 mmol) of palladium on carbon (10% loading, Degussa type, wet basis) were added to a solution of 4.57 g (29.10 mmol) of *rac*-1-[2,2-dimethylcyclopropyl]-2-nitroethan-1-one and 7.00 mL (31.00 mmol) of di-tert-butyl dicarbonate in 10 mL of methanol and the mixture was stirred at room temperature under hydrogen atmosphere for 16 hours. The solution was then filtered through a pad of celite, and the filtrates were concentrated to dryness to give a residue which was purified by flash chromatography on silica gel eluting with heptane/ethyl acetate mixtures to afford 3.80 g (57%) of the product as a colourless oil.
¹H-NMR (300 MHz, DMSO-*d*₆): δ [ppm] = 0.82-0.90 (m, 1H), 0.93-1.01 (m, 4H), 1.02-1.09 (m, 1H), 1.15 (s, 3H), 1.38 (s, 9H), 1.97 (t, 1H), 3.76 (ddd, 2H), 7.09 (t, 1H).
LC-MS (Method 3): Rₜ = 0.813 min. MS (Mass method 1): m/z = 250 (M + Na)⁺

### Intermediate 59

### rac-tert-Butyl ({4-[2,2-dimethylcyclopropyl]-2,5-dioxoimidazolidin-4-yl}methyl)carbamate

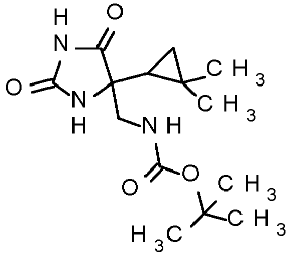

To a stirring solution of 6.34 g (66.00 mmol) of ammonium carbonate and 0.94 g (17.60 mmol) of ammonium chloride in 10 mL of water was added 1.00 g (4.40 mmol) of *rac*-tert-Butyl {2-[2,2-dimethylcyclopropyl]-2-oxoethyl}carbamate in 10 mL of ethanol. After 15 min, 1.29 g (19.80 mmol) of potassium cyanide were added and the mixture was heated up to 60 °C for 16 hours into a sealed pressure flask. The yellow solution was concentrated until only a small fraction of water remained (a white precipitate appeared). Then, more water was added and the suspension was allowed to stand at 0 °C for 1 hour. After that time, the resulting solid was filtered off and washed with water and diethyl ether to give 0.80 g (61%) of the product as an off-white powder. The compound was used as such in the next step.
¹H-NMR (300 MHz, DMSO-*d*₆): δ [ppm] = 0.32-0.48 (m, 2H), 0.79-0.85 (m, 1H), 0.87 (s, 3H), 1.00 (s, 3H), 1.06 (s, 2H), 1.36 (s, 9H), 3.26-3.31 (m, 2H), 6.73 (t, 1H), 7.29 (s, 1H), 10.64 (bp, 1H).
LC-MS (Method 3): Rₜ = 0.646 min. MS (Mass method 1): m/z = 242 (M - tBu + H)⁺

### Intermediate 60

### rac-5-(aminomethyl)-5-[2,2-dimethylcyclopropyl]imidazolidine-2,4-dione hydrochloride

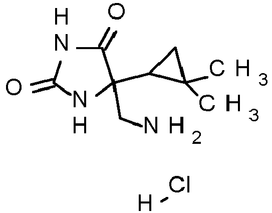

1.60 mL (6.20 mmol) of 4N hydrochloric acid in dioxane were added to a solution of 0.37 g (2.10 mmol) of *rac*-tert-Butyl ({4-[2,2-dimethylcyclopropyl]-2,5-dioxoimidazolidin-4-yl}methyl)carbamate in 10 mL of dichloromethane and the mixture was allowed to stir at room temperature for 16 hours. The resulting precipitate was collected by filtration and washed with dichloromethane, ethyl acetate and diethyl ether to give 0.33 g (98%) of the product as a white solid. The compound was used as such in the next step.
¹H-NMR (300 MHz, DMSO-*d*₆): δ [ppm] = 0.44-0.59 (m, 2H), 1.02 (s, 3H), 1.08 (s, 3H), 2.94-3.09 (m, 1H), 3.13-3.26 (m, 1H), 7.98 (s, 1H), 8.27 (bp, 3H), 11.01 (s, 1H).
LC-MS (Method 3): Rₜ = 0.144 min. MS (Mass method 1): m/z = 198 (M + H)⁺

### Intermediate 61

### rac-2-Nitro-1-(pyridin-3-yl)ethan-1-ol

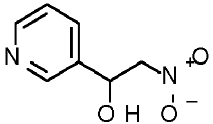

0.40 g (2.33 mmol) of barium hydroxide were added to a solution of 5.00 g (46.70 mmol) of nicotinaldehyde and 25.00 mL (470.00 mmol) of nitromethane in 135 mL of water and the mixture was stirred at room temperature for 15 min. The reaction was then extracted three times with ethyl acetate. The combined organic layers were dried over magnesium sulfate, filtered, and concentrated *in vacuo* to give 7.40 g (94%) of the crude product as a brown oil. The compound was used as such in the next step.
¹H-NMR (300 MHz, DMSO-*d*₆): δ [ppm] = 4.68 (dd, 1H), 4.92 (dd, 1H), 5.29-5.40 (m, 1H), 6.24 (d, 1H), 7.40 (dd, 1H), 7.85 (d, 1H), 8.51 (d, 1H), 8.64 (s, 1H).
LC-MS (Method 3): Rₜ = 0.115 min. MS (Mass method 1): m/z = 169 (M + H)⁺

### Intermediate 62

### rac-3-[1-{[tert-butyl(dimethyl)silyl]oxy}-2-nitroethyl]pyridine

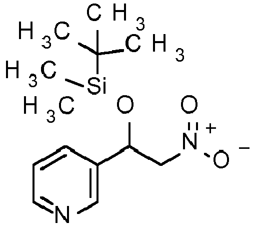

6.27 g (41.60 mmol) of tert-butyldimethylsilyl chloride were added to a solution of 2.00 (11.90 mmol) of *rac*-2-nitro-1-(pyridin-3-yl)ethan-1-ol and 4.05 g (59.50 mmol) of imidazole in 20 mL of N,N-dimethylformamide at 0 °C and the mixture was allowed to stir at room temperature for 14 hours. The solution was diluted with ethyl acetate and washed with water and brine. The organic layer was dried over magnesium sulfate, filtered and evaporated to dryness to yield a residue, which was purified by flash chromatography on silica gel eluting with heptane/ethyl acetate mixtures to afford 0.80 g (24%) of the product as a colourless oil.
LC-MS (Method 3): Rₜ = 0.929 min. MS (Mass method 1): m/z = 283 (M + H)⁺

### Intermediate 63

### rac-2-{[tert-butyl(dimethyl)silyl]oxy}-2-(pyridin-3-yl)ethan-1-amine

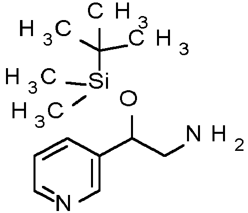

A mixture of 680 mg (2.41 mmol) of rac-3-[1-{[tert-butyl(dimethyl)silyl]oxy}-2-nitroethyl]pyridine and 3 mg (0.02 mmol) of palladium on carbon (10% loading, Degussa type, wet basis) in 10 mL of methanol was stirred under hydrogen atmosphere at room temperature for 12 hours. After that time, the catalyst was removed by filtration through a pad of celite and the cake was thoroughly washed with methanol. Evaporation of the solvent furnished a residue which was purified by flash chromatography on silica gel eluting with dichloromethane/methanol mixtures to afford 573 mg (94%) of the product as a colourless oil.
LC-MS (Method 3): Rₜ = 0.562 min. MS (Mass method 1): m/z = 253 (M + H)⁺

### Intermediate 64

### rac-tert-Butyl [2-{[tert-butyl(dimethyl)silyl]oxy}-2-(pyridin-3-yl)ethyl]carbamate

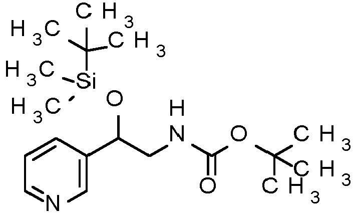

0.55 mL (2.40 mmol) of di-tert-butyl dicarbonate were added to a solution of 573 mg (2.27 mmol) of *rac*-2-{[tert-butyl(dimethyl)silyl]oxy}-2-(pyridin-3-yl)ethanamine in 20 mL of dichloromethane and the mixture was stirred at room temperature for 12 hours. The reaction was washed with water and brine and the organic layer was dried over magnesium sulfate, filtered and evaporated to dryness to afford 800 mg (99%) of the crude product as a yellow oil. The compound was used as such in the next synthetic step.
LC-MS (Method 3): Rₜ = 0.984 min. MS (Mass method 1): m/z = 353 (M + H)⁺

### Intermediate 65

### rac-tert-Butyl [2-hydroxy-2-(pyridin-3-yl)ethyl]carbamate

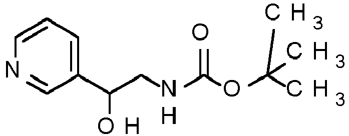

2.11 mL (2.11 mmol) of 1N tetra-n-butylammonium fluoride in tetrahydrofuran was added dropwise to a stirred solution of 495 mg (1.40 mmol) of rac-tert-Butyl [2-{[tert-butyl(dimethyl)silyl]oxy}-2-(pyridin-3-yl)ethyl]carbamate in 10 mL of anhydrous tetrahydrofuran under nitrogen atmosphere and the mixture was stirred at room temperature for 14 hours. After that time, the solution was poured onto 50 mL of water, and extracted with ethyl acetate. The combined organic extracts were washed successively with water and brine, dried over magnesium sulfate, and concentrated *in vacuo* to furnish 334 mg (99%) of the crude product as a brown powder. The compound was used as such in the next step.
LC-MS (Method 3): Rₜ = 0.249 min. MS (Mass method 1): m/z = 239 (M + H)⁺

### Intermediate 66

### tert-Butyl [2-oxo-2-(pyridin-3-yl)ethyl]carbamate

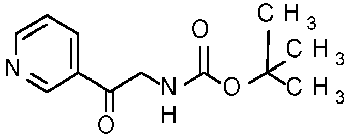

0.49 mL (6.90 mmol) of dimethyl sulfoxide were added drop wise to a solution of 0.30 mL (3.40 mmol) of oxalyl chloride in 8 mL of anhydrous dichloromethane at -78 °C and the mixture was stirred under nitrogen atmosphere under these conditions for 30 min. 409 mg (1.72 mmol) of *rac*-tert-Butyl [2-hydroxy-2-(pyridin-3-yl)ethyl]carbamate was added to the cold solution and the resulting mixture was stirred for 15 additional min. After that, 1.20 mL (8.60 mmol) of triethylamine were added and the reaction was allowed to stir at 0 °C for 30 min. Finally, the mixture was diluted with ethyl acetate and the organic layer was washed once with water and brine, dried over magnesium sulfate, filtered and concentrated *in vacuo.* Flash chromatography of the resulting residue using heptane/ethyl acetate mixtures as eluent gave 190 mg (47%) of the product as a white solid.
LC-MS (Method 3): Rₜ = 0.535 min. MS (Mass method 1): m/z = 237 (M + H)⁺

### Intermediate 67

### rac-tert-Butyl {[2,5-dioxo-4-(pyridin-3-yl)imidazolidin-4-yl]methyl}carbamate

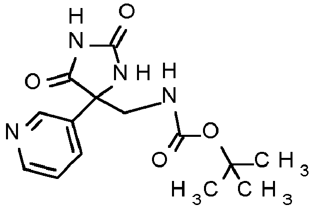

To a stirring solution of 1.10 g (11.40 mmol) of ammonium carbonate and 0.16 g (3.05 mmol) of ammonium chloride in 2 mL of water was added 0.18 g (0.76 mmol) of tert-butyl [2-oxo-2-(pyridin-3-yl)ethyl]carbamate in 2 mL of ethanol. After 15 min, 0.22 g (3.43 mmol) of potassium cyanide were added and the mixture was heated up to 60 °C for 16 hours into a sealed pressure flask. The yellow solution was concentrated until only a small fraction of water remained (a white precipitate appeared). Then, more water was added and the suspension was allowed to stand at 0 °C for 2 hour. After that time, the resulting solid was filtered off and washed with cold water and diethyl ether to give 0.14 g (60%) of the product as an off-white powder. The compound was used as such in the next step.
¹H-NMR (300 MHz, DMSO-*d*₆): δ [ppm] = 1.29 (s, 9H), 3.39-3.64 (m, 2H), 6.88 (bp, 1H), 7.38 (dd, 1H), 7.89 (d, 1H), 8.50 (d, 1H), 8.69 (s, 1H).
LC-MS (Method 3): Rₜ = 0.348 min. MS (Mass method 1): m/z = 307 (M + H)⁺

### Intermediate 68

### rac-5-(aminomethyl)-5-(pyridin-3-yl)imidazolidine-2,4-dione hydrochloride

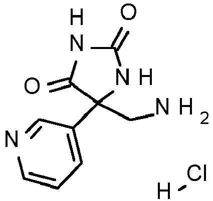

0.78 mL (3.10 mmol) of 4N hydrochloric acid in dioxane were added to a solution of 0.19 g (0.62 mmol) of *rac*-tert-Butyl ([2,5-dioxo-4-(pyridin-3-yl)imidazolidin-4-yl]methyl}carbamate in 10 mL of dichloromethane and the mixture was allowed to stir at room temperature for 16 hours. The resulting precipitate was collected by filtration and washed with dichloromethane, ethyl acetate and diethyl ether to give 0.15 g (99%) of the product as a white solid. The compound was used as such in the next step.
¹H-NMR (300 MHz, DMSO-*d*₆): δ [ppm] = 3.52-3.77 (m, 2H), 7.98 (dd, 1H), 8.53-8.73 (m, 4H), 8.88 (d, 1H), 9.00 (s, 1H), 9.34 (s, 1H), 11.41 (s, 1H).
LC-MS (Method 3): Rₜ = 0.126 min. MS (Mass method 1): m/z = 207 (M + H)⁺

### Intermediate 69

### rac-2-nitro-1-(pyridin-2-yl)ethan-1-ol

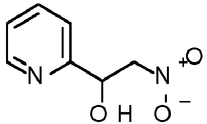

0.40 g (2.33 mmol) of barium hydroxide were added to a solution of 5.00 g (46.70 mmol) of pyridine-2-carbaldehyde and 25.00 mL (470.00 mmol) of nitromethane in 140 mL of water and the mixture was stirred at room temperature for 15 min. The reaction was then extracted three times with ethyl acetate. The combined organic layers were dried over magnesium sulfate, filtered, and concentrated *in vacuo* to give 7.70 g (98%) of the crude product as a colourles oil. The compound was used as such in the next step.
LC-MS (Method 3): Rₜ = 0.163 min. MS (Mass method 1): m/z = 169 (M + H)⁺

### Intermediate 70

### rac-2-[1-{[tert-butyl(dimethyl)silyl]oxy}-2-nitroethyl]pyridine

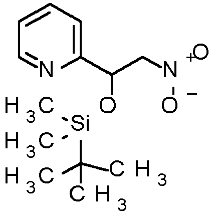

24.20 g (160.00 mmol) of tert-butyldimethylsilyl chloride were added to a solution of 7.70 (45.80 mmol) of *rac*-2-nitro-1-(pyridin-2-yl)ethan-1-ol and 15.60 g (229.00 mmol) of imidazole in 240 mL of N,N-dimethylformamide at 0 °C and the mixture was allowed to stir at room temperature for 14 hours. The solution was diluted with ethyl acetate and washed with water and brine. The organic layer was dried over magnesium sulfate, filtered and evaporated to dryness to yield a residue, which was purified by flash chromatography on silica gel eluting with heptane/ethyl acetate mixtures to afford 11.85 g (92%) of the product as a colourless oil.
¹H-NMR (300 MHz, DMSO-*d*₆): δ [ppm] = -0.10 (s, 3H), 0.01 (s, 3H), 0.82 (s, 9H), 4.75 (dd, 1H), 4.95 (dd, 1H), 5.43 (dd, 1H), 7.36 (dd, 1H), 7.54 (d, 1H), 7.88 (t, 1H), 8.56 (d, 1H).
LC-MS (Method 3): Rₜ = 1.078 min. MS (Mass method 1): m/z = 283 (M + H)⁺

### Intermediate 71

### rac-2-{[tert-butyl(dimethyl)silyl]oxy}-2-(pyridin-2-yl)ethan-1-amine

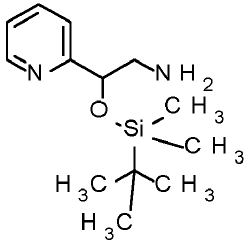

A mixture of 11.80 g (41.90 mmol) of rac-2-[1-{[tert-butyl(dimethyl)silyl]oxy}-2-nitroethyl]pyridine and 0.04 g (0.42 mmol) of palladium on carbon (10% loading, Degussa type, wet basis) in 50 mL of methanol was stirred under hydrogen atmosphere at room temperature for 12 hours. After that time, the catalyst was removed by filtration through a pad of celite and the cake was thoroughly washed with methanol. Evaporation of the solvent furnished a residue which was purified by flash chromatography on silica gel eluting with dichloromethane/methanol mixtures to afford 4.52 g (43%) of the product as a colourless oil.
¹H-NMR (300 MHz, DMSO-*d*₆): δ [ppm] = -0.08 (s, 3H), 0.06 (s, 3H), 0.87 (s, 9H), 2.74 (ddd, 2H), 4.66 (dd, 1H), 7.25 (dd, 1H), 7.41 (d, 1H), 7.78 (t, 1H), 8.48 (d, 1H).
LC-MS (Method 3): Rₜ = 0.642 min. MS (Mass method 1): m/z = 253 (M + H)⁺

### Intermediate 72

### rac-tert-Butyl [2-{[tert-butyl(dimethyl)silyl]oxy}-2-(pyridin-2-yl)ethyl]carbamate

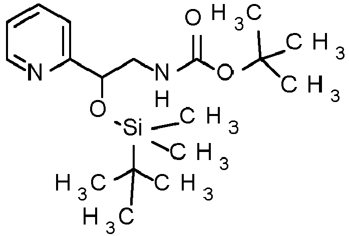

4.30 mL (19.00 mmol) of di-tert-butyl dicarbonate were added to a solution of 4.51 g (17.90 mmol) of rac-2-{[tert-butyl(dimethyl)silyl]oxy}-2-(pyridin-2-yl)ethanamine in 20 mL of dichloromethane and the mixture was stirred at room temperature for 12 hours. The reaction was washed with water and brine and the organic layer was dried over magnesium sulfate, filtered and evaporated to dryness to afford 6.31 g (99%) of the crude product as a light yellow oil. The compound was used as such in the next synthetic step.
LC-MS (Method 3): Rₜ = 1.084 min. MS (Mass method 1): m/z = 353 (M + H)⁺

### Intermediate 73

### rac-tert-Butyl [2-hydroxy-2-(pyridin-2-yl)ethyl]carbamate

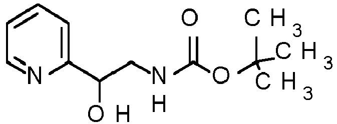

27.00 mL (27.00 mmol) of 1N tetra-n-butylammonium fluoride in tetrahydrofuran was added dropwise to a stirred solution of 6.31 g (17.90 mmol) of rac-tert-Butyl [2-{[tert-butyl(dimethyl)silyl]oxy}-2-(pyridin-2-yl)ethyl]carbamate in 100 mL of tetrahydrofuran and the mixture was stirred at room temperature for 14 hours. After that time, the solution was poured onto 50 mL of water, and extracted with ethyl acetate. The combined organic extracts were washed successively with water and brine, dried over magnesium sulfate, and concentrated *in vacuo* to furnish 4.26 g (99%) of the crude product as a colourless thick oil. The compound was used as such in the next step.
LC-MS (Method 3): Rₜ = 0.201 min. MS (Mass method 1): m/z = 239 (M + H)⁺

### Intermediate 74

### tert-Butyl [2-oxo-2-(pyridin-2-yl)ethyl]carbamate

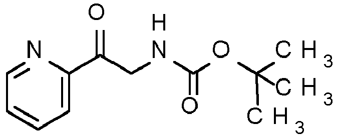

1.19 mL (16.90 mmol) of dimethyl sulfoxide were added drop wise to a solution of 0.73 mL (8.39 mmol) of oxalyl chloride in 20 mL of anhydrous dichloromethane at -78 °C and the mixture was stirred under nitrogen atmosphere under these conditions for 30 min. 1.00 g (4.20 mmol) of *rac-*tert-Butyl [2-hydroxy-2-(pyridin-2-yl)ethyl]carbamate was added to the cold solution and the resulting mixture was stirred for 15 additional min. After that, 2.92 mL (20.98 mmol) of triethylamine were added and the reaction was allowed to stir at 0 °C for 30 min. Finally, the mixture was diluted with ethyl acetate and the organic layer was washed once with water and brine, dried over magnesium sulfate, filtered and concentrated *in vacuo.* Flash chromatography of the resulting residue using heptane/ethyl acetate mixtures as eluent gave 0.41 g (41%) of the product as a light brown solid.
¹H-NMR (300 MHz, DMSO-*d*₆): δ [ppm] = 1.39 (s, 9H), 4.58 (d, 2H), 7.00 (t, 1H), 7.70 (dd, 1H), 7.93-8.08 (m, 2H), 8.73 (d, 1H).
LC-MS (Method 3): Rₜ = 0.661 min. MS (Mass method 1): m/z = 181 (M - tBu + H)⁺

### Intermediate 75

### rac-tert-Butyl {[2,5-dioxo-4-(pyridin-2-yl)imidazolidin-4-yl]methyl}carbamate

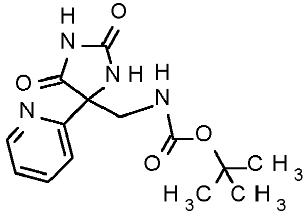

To a stirring solution of 3.29 g (34.30 mmol) of ammonium carbonate and 0.49 g (9.14 mmol) of ammonium chloride in 6 mL of water was added 0.54 g (2.29 mmol) of tert-butyl [2-oxo-2-(pyridin-2-yl)ethyl]carbamate in 6 mL of ethanol. After 15 min, 0.67 g (10.30 mmol) of potassium cyanide were added and the mixture was heated up to 60 °C for 16 hours into a sealed pressure flask. The yellow solution was concentrated until only a small fraction of water remained (a white precipitate appeared). Then, more water was added and the suspension was allowed to stand at 0 °C for 2 hour. After that time, the resulting solid was filtered off and washed with cold water and diethyl ether to give 0.70 g (98%) of the product as an off-white powder. The compound was used as such in the next step.
LC-MS (Method 3): Rₜ = 0.466 min. MS (Mass method 1): m/z = 307 (M + H)⁺

### Intermediate 76

### rac-5-(aminomethyl)-5-(pyridin-2-yl)imidazolidine-2,4-dione hydrochloride

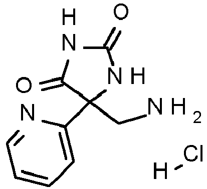

2.90 mL (11.00 mmol) of 4N hydrochloric acid in dioxane were added to a solution of 0.70 g (2.29 mmol) of *rac*-tert-Butyl {[2,5-dioxo-4-(pyridin-2-yl)imidazolidin-4-yl]methyl}carbamate in 10 mL of dichloromethane and the mixture was allowed to stir at room temperature for 16 hours. The resulting precipitate was collected by filtration and washed with dichloromethane, ethyl acetate and diethyl ether to give 0.55 g (99%) of the product as a white solid. The compound was used as such in the next step.
LC-MS (Method 3): Rₜ = 0.091 min. MS (Mass method 1): m/z = 207 (M + H)⁺

### Intermediate 77

### rac-2-nitro-1-(pyridin-4-yl)ethan-1-ol

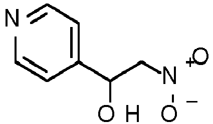

0.40 g (2.33 mmol) of barium hydroxide were added to a solution of 5.00 g (46.70 mmol) of pyridine-4-carbaldehyde and 25.00 mL (470.00 mmol) of nitromethane in 140 mL of water and the mixture was stirred at room temperature for 15 min. The reaction was then extracted three times with ethyl acetate. The combined organic layers were dried over magnesium sulfate, filtered, and concentrated *in vacuo* to give 7.00 g (89%) of the crude product as a colourles oil. The compound was used as such in the next step.
¹H-NMR (300 MHz, DMSO-*d*₆): δ [ppm] = 4.60 (dd, 1H), 4.95 (dd, 2H), 5.25-5.35 (m, 1H), 6.32 (d, 1H), 7.46 (d, 2H), 8.56 (d, 2H).
LC-MS (Method 3): Rₜ = 0.093 min. MS (Mass method 1): m/z = 169 (M + H)⁺

### Intermediate 78

### rac-4-[1-{[tert-butyl(dimethyl)silyl]oxy}-2-nitroethyl]pyridine

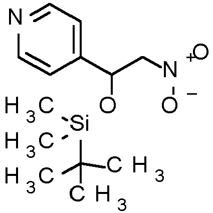

6.71 g (44.50 mmol) of tert-butyldimethylsilyl chloride were added to a solution of 3.75 (22.30 mmol) of *rac*-2-nitro-1-(pyridin-4-yl)ethan-1-ol and 6.06 g (89.10 mmol) of imidazole in 20 mL of N,N-dimethylformamide at 0 °C and the mixture was allowed to stir at room temperature for 14 hours. The solution was diluted with ethyl acetate and washed with water and brine. The organic layer was dried over magnesium sulfate, filtered and evaporated to dryness to yield a residue, which was purified by flash chromatography on silica gel eluting with heptane/ethyl acetate mixtures to afford 6.29 g (99%) of the product as a colourless oil.
¹H-NMR (300 MHz, DMSO-*d*₆): δ [ppm] = -0.13 (s, 3H), 0.01 (s, 3H), 0.81 (s, 9H), 4.66 (dd, 1H), 4.89 (dd, 1H), 5.48 (dd, 1H), 7.47 (d, 2H), 8.59 (d, 2H).
LC-MS (Method 3): Rₜ = 0.845 min. MS (Mass method 1): m/z = 283 (M + H)⁺

### Intermediate 79

### rac-2-{[tert-butyl(dimethyl)silyl]oxy}-2-(pyridin-4-yl)ethan-1-amine

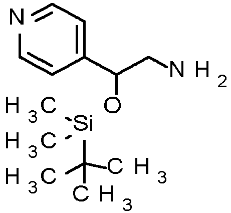

A mixture of 4.51 g (16.00 mmol) of rac-4-[1-{[tert-butyl(dimethyl)silyl]oxy}-2-nitroethyl]pyridine and 0.02 g (0.16 mmol) of palladium on carbon (10% loading, Degussa type, wet basis) in 50 mL of methanol was stirred under hydrogen atmosphere at room temperature for 12 hours. After that time, the catalyst was removed by filtration through a pad of celite and the cake was thoroughly washed with methanol. Evaporation of the solvent furnished a residue which was purified by flash chromatography on silica gel eluting with dichloromethane/methanol mixtures to afford 3.97 g (98%) of the product as a colourless oil.
LC-MS (Method 3): Rₜ = 0.357 min. MS (Mass method 1): m/z = 253 (M + H)⁺

### Intermediate 80

### rac-tert-Butyl [2-{[tert-butyl(dimethyl)silyl]oxy}-2-(pyridin-4-yl)ethyl]carbamate

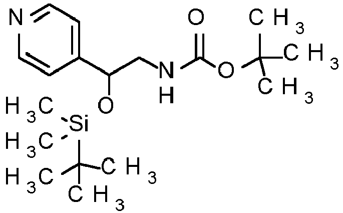

3.80 mL (17.00 mmol) of di-tert-butyl dicarbonate were added to a solution of 3.97 g (15.70 mmol) of *rac*-2-{[tert-butyl(dimethyl)silyl]oxy}-2-(pyridin-4-yl)ethanamine in 30 mL of dichloromethane and the mixture was stirred at room temperature for 12 hours. The reaction was washed with water and brine and the organic layer was dried over magnesium sulfate, filtered and evaporated to dryness to afford 5.54 g (99%) of the crude product as a light yellow oil. The compound was used as such in the next synthetic step.
LC-MS (Method 3): Rₜ = 0.829 min. MS (Mass method 1): m/z = 353 (M + H)⁺

### Intermediate 81

### rac-tert-Butyl [2-hydroxy-2-(pyridin-4-yl)ethyl]carbamate

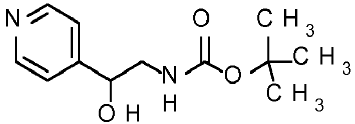

23.00 mL (23.00 mmol) of 1N tetra-n-butylammonium fluoride in tetrahydrofuran was added dropwise to a stirred solution of 5.50 g (15.60 mmol) of *rac*-tert-Butyl [2-{[tert-butyl(dimethyl)silyl]oxy}-2-(pyridin-4-yl)ethyl]carbamate in 50 mL of tetrahydrofuran and the mixture was stirred at room temperature for 14 hours. After that time, the solution was poured onto 50 mL of water, and extracted with ethyl acetate. The combined organic extracts were washed successively with water and brine, dried over magnesium sulfate, and concentrated *in vacuo* to furnish 3.72 g (99%) of the crude product as a colourless thick oil. The compound was used as such in the next step.
LC-MS (Method 3): Rₜ = 0.172 min. MS (Mass method 1): m/z = 239 (M + H)⁺

### Intermediate 82

### tert-Butyl [2-oxo-2-(pyridin-4-yl)ethyl]carbamate

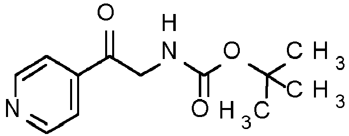

5.34 g (12.59 mmol) of Dess-Martin periodinane were added to a solution of 2.00 (8.39 mmol) of *rac*-tert-Butyl [2-hydroxy-2-(pyridin-4-yl)ethyl]carbamate in 40 mL of dichloromethane and the mixture was stirred at room temperature for 5 min. Then, a solution of 0.22 mL (12.59 mmol) of water in dichloromethane (1 uL water/1 mL dichloromethane) was added and the mixture was stirred at room temperature for 10 min. After that time, the solution was diluted with dichloromethane and washed with saturated sodium bisulfite(aq.) and saturated sodium bicarbonate(aq.). The organic layer was dried over magnesium sulfate, filtered and evaporated to dryness. The residue was purified by flash chromatography on silica gel eluting with heptane/ethyl acetate mixtures to provide 0.82 g (41%) of the product as a yellowish oil.
LC-MS (Method 3): Rₜ = 0.528 min. MS (Mass method 1): m/z = 237 (M + H)⁺

### Intermediate 83

### rac-tert-Butyl {[2,5-dioxo-4-(pyridin-4-yl)imidazolidin-4-yl]methyl}carbamate

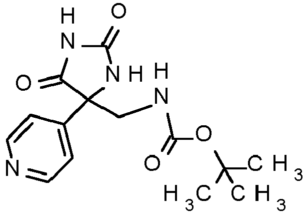

To a stirring solution of 1.55 g (16.10 mmol) of ammonium carbonate and 0.23 g (4.30 mmol) of ammonium chloride in 2 mL of water was added 0.25 g (1.08 mmol) of tert-butyl [2-oxo-2-(pyridin-4-yl)ethyl]carbamate in 2 mL of ethanol. After 15 min, 0.31 g (4.84 mmol) of potassium cyanide were added and the mixture was heated up to 60 °C for 16 hours into a sealed pressure flask. The yellow solution was concentrated until only a small fraction of water remained (a white precipitate appeared). Then, more water was added and the suspension was allowed to stand at 0 °C for 2 hour. After that time, the resulting solid was filtered off and washed with cold water and diethyl ether to give 0.33 g (98%) of the product as a brown oil. The compound was used as such in the next step.
LC-MS (Method 3): Rₜ = 0.289 min. MS (Mass method 1): m/z = 307 (M + H)⁺

### Intermediate 84

### rac-5-(aminomethyl)-5-(pyridin-4-yl)imidazolidine-2,4-dione hydrochloride

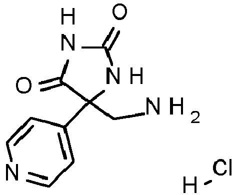

0.41 mL (1.63 mmol) of 4N hydrochloric acid in dioxane were added to a solution of 0.10 g (0.32 mmol) of *rac*-tert-Butyl {[2,5-dioxo-4-(pyridin-4-yl)imidazolidin-4-yl]methyl}carbamate in 10 mL of dichloromethane and the mixture was allowed to stir at room temperature for 16 hours. The resulting precipitate was collected by filtration and washed with dichloromethane, ethyl acetate and diethyl ether to give 0.08 g (99%) of the product as a white solid. The compound was used as such in the next step.
LC-MS (Method 3): Rₜ = 0.090 min. MS (Mass method 1): m/z = 207 (M + H)⁺

### Intermediate 85

### 1-(2-Cyclopropyl-2-oxoethyl)-3,5,7-triaza-1-azoniatricyclo[3.3.1.1^{3,7}]decane bromide

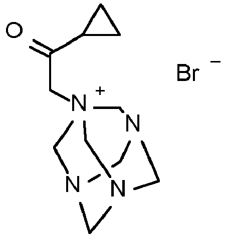

100.00 g (0.61 mol) of 2-bromo-1-cyclopropylethanone was dissolved in 1.4 L of chloroform and 86.00 g (0.61 mol) of hexamethylenetetramine was added allowing the mixture to stir at room temperature for 12 hours. The solvent was partially removed and the resulting precipitate was filtered off, washed with cold chloroform and isolated. The remaining mother liquors were concentrated again, filtered and washed with cold chloroform several times until no more precipitate was appreciated. All the collected solids were dried *in vacuo* to give 185.67 g (100%) of the product as a white powder. The compound was used as such in the next synthetic step.
¹H-NMR (300 MHz, DMSO-*d*₆): δ [ppm] = 0.98-1.10 (m, 4H), 2.02-2.17 (m, 1H), 4.31 (d, 2H), 4.51 (d, 3H), 4.65 (d, 3H), 5.32 (s, 6H).
LC-MS (Method 3): Rₜ = 0.099 min. MS (Mass method 1): m/z = 223 (M)⁺

### Intermediate 86

### tert-Butyl (2-cyclopropyl-2-oxoethyl)carbamate

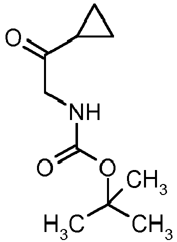

185.67 g (0.61 mol) of 1-(2-cyclopropyl-2-oxoethyl)-3,5,7-triaza-1-azoniatricyclo[3.3.1.1^{3,7}] decane bromide was dissolved in 0.60 L of ethanol and 0.20 L of hydrochloric acid (conc). and the mixture was refluxed for 30 min. The resulting precipitate was filtered off, washed with ethanol and discarded. The filtrates were evaporated and dried to yield the corresponding crude ammonium salt as a thick oil. Next, 0.10 L (0.73 mol) of triethylamine and 0.14 L (0.61 mol) of di-tert-butyl dicarbonate were added to a solution of the aforementioned crude ammonium salt in 1.3 L of dichloromethane and the mixture was stirred at room temperature for 12 hours. Water was added to the reaction and the layers were separated. The organic layer was dried over magnesium sulfate, filtered and evaporated. The corresponding residue was purified by flash chromatography on silica gel eluting with heptane and ethyl acetate. 49.00 g (40%) of the product were isolated as a dark brown oil.
¹H-NMR (300 MHz, DMSO-*d*₆): δ [ppm] = 0.78-0.85 (m, 1H), 0.86-0.94 (m, 2H), 1.38 (s, 9H), 1.99-2.11 (m, 1H), 3.88 (d, 2H), 7.03 (bp, 1H).
LC-MS (Method 3): Rₜ = 0.630 min. MS (Mass method 1): m/z = 222 (M + Na)⁺

### Intermediate 87

### rac-tert-butyl [(4-cyclopropyl-2,5-dioxoimidazolidin-4-yl)methyl]carbamate

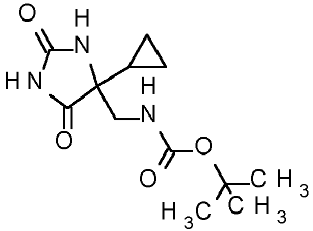

To a stirring solution of 50.63 g (526.97 mmol) of ammonium carbonate and 7.51 g (140.52 mmol) of ammonium chloride in 40 mL water were added 7.00 g (35.13 mmol) of tert-butyl (2-cyclopropyl-2-oxoethyl)carbamate in 40 mL of ethanol. After 15 min, 10.29 g (158.09 mmol) of potassium cyanide were added and the mixture was heated up to 60 °C for 16 hours in a sealed pressure flask. The yellow solution was concentrated until only a small fraction of water remained (a white precipitate appeared). Then, more water was added and the suspension was allowed to stand at 0 °C for 1 hour. After that time, the resulting solid was filtered off and washed with water and diethyl ether to give 5.04 g (53%) of the product as a beige powder. The compound was used as such in the next step.
¹H-NMR (300 MHz, DMSO-*d*₆): δ [ppm] = 0.01-0.14 (m, 1H), 0.23-0.34 (m, 1H), 0.35-0.46 (m, 2H), 0.97-1.10 (m, 1H), 1.36 (s, 9H), 3.25-3.32 (m, 2H), 6.78 (t, 1H), 7.36 (s, 1H), 10.46 (bp, 1H).
LC-MS (Method 3): Rₜ = 0.388 min. MS (Mass method 1): m/z = 214 (M - tBu + H)⁺

### Intermediate 88

### Ethyl 5-isopropyl-1,3-oxazole-4-carboxylate

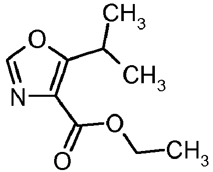

A solution of 2.47 mL (26.67 mmol) of 2-methylpropanoic acid and 5.19 g (32.00 mmol) of 1,1'-carbonyldiimidazole in 30 mL of tetrahydrofuran was stirred for 3 hours at room temperature. After that time, a solution of 3.21 mL (29.34 mmol) of ethyl isocyanoacetate in 35 mL of tetrahydrofuran and a solution of 26.67 mL (26.67 mmol) of 1M lithium bis(trimethylsilyl)amide in tetrahydrofuran were added dropwise at 0 °C . When the addition was complete the resulting mixture was stirred at room temperature for 16 hours. The reaction was concentrated *in vacuo* and extracted with ethyl acetate, the combined organic layers were filtered over magnesium sulfate and concentrated. The resulting residue was purified by flash chromatography on silica gel eluting with heptane/dichloromethane mixtures to give 2.26 g (46%) of the product as a brown oil.
¹H-NMR (300 MHz, DMSO-*d*₆): δ [ppm] = 1.23 (d, 6H), 3.62-3.75 (m, 1H), 4.26 (q, 2H), 8.34 (s, 1H).
LC-MS (Method 3): Rₜ = 0.708 min. MS (Mass method 1): m/z = 184 (M + H)⁺

### Intermediate 89

### tert-Butyl (3-methyl-2-oxobutyl)carbamate

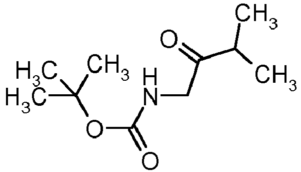

2.26 g (12.39 mmol) of ethyl 5-isopropyl-1,3-oxazole-4-carboxylate was suspended in 40 mL of 6N hydrochloric acid(aq.) and the mixture was refluxed for 12 hours. Concentration of the reaction under reduced pressure gave 1.69 g (99%) of the intermediate amino ketone hydrochloride as a brown oil. A solution of 1.69 g (12.28 mmol) of the latter compound and 3.10 mL (13.51 mmol) of di-tert-butyl dicarbonate were dissolved in 30 mL of dichloromethane and 4.28 mL (30.70 mmol) of triethylamine was added, allowing the mixture to stir at room temperature for 12 hours. The crude was diluted with ethyl acetate, washed with water, dried over magnesium sulfate, filtered and concentrated under vacuum. The resulting residue was purified by flash chromatography on silica gel eluting with heptane/ethyl acetate mixtures to provide 1.96 g (79%) of the product as an oil.
¹H-NMR (300 MHz, DMSO-*d*₆): δ [ppm] = 0.99 (d, 6H), 1.37 (s, 9H), 2.59-2.73 (m, 1H), 3.82 (d, 2H), 6.96 (t, 1H).
LC-MS (Method 3): Rₜ = 0.739 min. MS (Mass method 1): m/z = 224 (M + Na)⁺

### Intermediate 90

### rac-tert-butyl [(4-isopropyl-2,5-dioxoimidazolidin-4-yl)methyl]carbamate

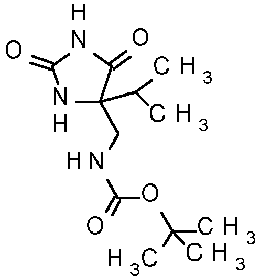

A solution of 1.93 g (9.59 mmol) of tert-butyl (3-methyl-2-oxobutyl)carbamate in 15 mL of ethanol was added to a solution of 1.25 g (19.18 mmol) of potassium cyanide and 9.21 g (95.89 mmol) of ammonium carbonate in 15 mL of water into a pressure flask, the container was sealed and the mixture was stirred at 60 °C for 24 hours. After that time, the solvent was partially removed and the resulting precipitate was filtered off to give 1.74 g (67%) of the product as a withe solid. The compound was used as such in the next step.
¹H-NMR (300 MHz, DMSO-*d*₆): δ [ppm] = 0.79 (d, 3H), 0.87 (d, 3H), 1.36 (s, 9H), 1.82-1.95 (m, 1H), 3.12-3.30 (m, 2H), 6.70 (bp, 1H), 7.38 (s, 1H), 10.53 (s, 1H).
LC-MS (Method 3): Rₜ = 0.489 min. MS (Mass method 1): m/z = 216 (M - t-Bu + H)⁺

### Intermediate 91

### rac-5-(aminomethyl)-5-isopropylimidazolidine-2,4-dione hydrochloride

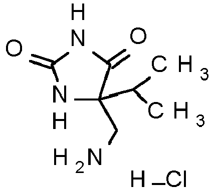

1.74 g (6.41 mmol) of rac-tert-butyl [(4-isopropyl-2,5-dioxoimidazolidin-4-yl)methyl]carbamate was dissolved in 20 mL of dichloromethane and 8.02 mL (32.06 mmol) of 4M hydrochloric acid in dioxane were added and the resulting suspension was stirred at room temperature for 12 hours. After that time, the solvent was *in vacuo* to give 1.10 g (83%) of the product a solid. The compound was used as such in the next step.
LC-MS (Method 3): Rₜ = 0.125 min. MS (Mass method 1): m/z = 172 (M + H)⁺

### Intermediate 92

### rac-5-(aminomethyl)-5-cyclobutylimidazolidine-2,4-dione hydrochloride

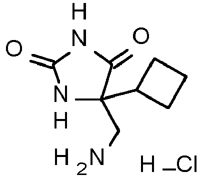

1.53 g (8.35 mmol) of rac-5-(aminomethyl)-5-cyclobutylimidazolidine-2,4-dione was dissolved in 40 mL of dichloromethane and 4.17 mL (16.70 mmol) of 4M hydrochloric acid in dioxane were added and the resulting suspension was stirred at room temperature for 12 hours. After that time, the solvent was removed *in vacuo* to give 1.76 g (96%) of the product as a solid. The compound was used as such in the next step.
¹H-NMR (300 MHz, DMSO-*d*₆): δ [ppm] = 1.68-1.82 (m, 4H), 1.87-1.97 (m, 2H), 2.62-2.83 (m, 2H), 2.96-3.06 (m, 1H), 8.20 (bp, 4H), 10.96 (s, 1H).
LC-MS (Method 6): Rₜ = 0.496 min. MS (Mass method 1): m/z = 184 (M + H)⁺

### Intermediate 93

### Ethyl 3-oxo-2-{2-[4-(trifluoromethyl)phenyl]hydrazinylidene}propanoate

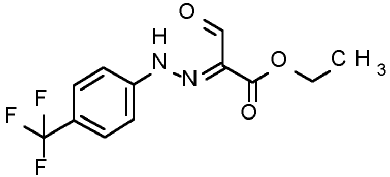

0.48 g (6.98 mmol) of sodium nitrite in 10 mL of water was added drop wise to solution of 1.13 g (6.98 mmol) of 4-aminobenzotrifluoride in 3 mL of concentrated hydrochloric acid and 5 mL of water at 0 °C and the mixture was stirred for 10 min under the same conditions. After that time, the resulting solution was added drop wise to a solution of 1.00 g (6.98 mmol) of ethyl (2E)-3-(dimethylamino)prop-2-enoate and 1.03 g (10.50 mmol) of potassium acetate in 15 mL of ethanol at 0 °C and then mixture was further stirred at room temperature for 12 hours. The ethanolic fraction of the solvent was removed and the resulting residue was diluted with ethyl acetate and washed with water. The organic layer was dried over magnesium sulfate, filtered and concentrated giving 2.00 g (99%) of the crude product as a deep red thick oil which solidified upon standing. The compound was used as such in the next step.
LC-MS (Method 3): Rₜ = 1.022 min. MS (Mass method 1): m/z = 289 (M + H)⁺

### Intermediate 94

### Ethyl 3-(hydroxyimino)-2-{2-[4-(trifluoromethyl)phenyl]hydrazinylidene}propanoate

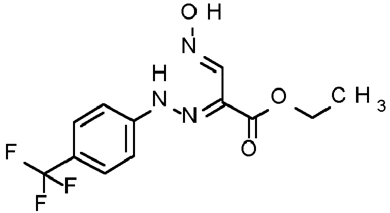

2.00 g (6.94 mmol) of ethyl 3-oxo-2-{2-[4-(trifluoromethyl)phenyl]hydrazinylidene} propanoate, 0.58 g (8.33 mmol) of hydroxylamine hydrochloride and 1.70 g (17.30 mmol) potassium acetate were mixed in 30 mL of ethanol and the mixture was heated up to 78 °C for 1 hour. The resulting red suspension was cooled down to room temperature and 20 mL of water was added. The reaction was extracted with ethyl acetate and the combined organic layers were dried over magnesium sulfate, filtered and concentrated to give 1.60 g (76%) of the crude product as a thick red oil. The compound was used as such in the next step.
LC-MS (Method 3): Rₜ = 0.934 min. MS (Mass method 1): m/z = 304 (M + H)⁺

### Intermediate 95

### Ethyl 2-[4-(trifluoromethyl)phenyl]-2H-1,2,3-triazole-4-carboxylate

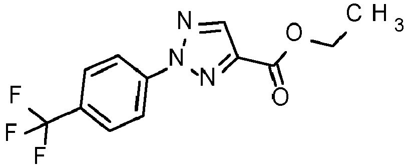

1.60 g (5.28 mmol) of ethyl 3-(hydroxyimino)-2-{2-[4-(trifluoromethyl)phenyl] hydrazinylidene}propanoate were dissolved in 20 mL of acetic anhydride and the mixture was stirred at 140 °C for 1 hour. Upon cooling, 20 mL of water were added and the reaction was extracted with dichloromethane. The combined organic layers were dried over magnesium sulfate, filtered and concentrated to give a residue which was further purified by flash chromatography on silica gel using heptane/dichloromethane mixtures to afford 0.47 g (31%) of the product as a yellow oil.
¹H-NMR (300 MHz, DMSO-*d*₆): δ [ppm] = 1.35 (t, 3H), 4.41 (q, 2H), 8.00 (d, 2H), 8.29 (d, 2H), 8.68 (s, 1H).
LC-MS (Method 3): Rₜ = 1.018 min. MS (Mass method 1): m/z = 286 (M + H)⁺

### Intermediate 96

### 2-[4-(trifluoromethyl)phenyl]-2H-1,2,3-triazole-4-carboxylic acid

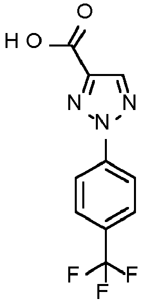

83 mg (1.98 mmol) of lithium hydroxide monohydrate was added to a solution of 470 mg (1.68 mmol) of ethyl 2-[4-(trifluoromethyl)phenyl]-2H-1,2,3-triazole-4-carboxylate in 8 mL of tetrahydrofuran and 4 mL of water and the mixture was stirred at room temperature for 2 hours. After that time, pH was set to 3-4 by addition of 2N hydrochloric acid(aq.) and the solvent was totally removed by rotary evaporation. 420 mg (99%) of the crude product was then isolated as a yellow powder. The compound was used as such in the next synthetic step.
¹H-NMR (300 MHz, DMSO-*d*₆): δ [ppm] = 7.97 (d, 2H), 8.25 (d, 2H), 8.37 (s, 1H).
LC-MS (Method 3): Rₜ = 0.753 min. MS (Mass method 1): m/z = 258 (M + H)⁺

### Intermediate 97

### Ethyl 2-[2-(4-fluorophenyl)hydrazinylidene]-3-oxopropanoate

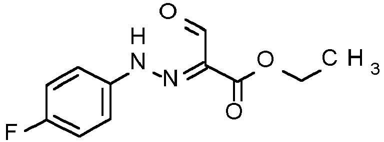

4-Fluoroaniline (3 g, 27 mmol) were treated with 30 ml of water and 6 ml of concentrated hydrochloric acid at 0°C. Sodium nitrite (1.863 g, 27 mmol) was dissolved in 15 ml of water and this solution was added dropwise to the cooled 4-fluoroaniline solution. The mixture was stirred for five minutes at 0°C before being added dropwise at 0°C to a mixture of ethyl 3-(dimethylamino)acrylate (4.252 g, 29.7 mmol) and potassium acetate (3,974 g, 40.5 mmol) in 45 ml of ethanol. The reaction mixture was stirred for 10 minutes at room temperature and then diluted with ethyl acetate. The mixture was washed twice with water and once with saturated aqueous sodium chloride solution. The organic layer was dried over sodium sulfate, filtered, Isolute® was added, and the solvent was evaporated on a rotary evaporator. The crude product was purified by column chromatography (Machine: Biotage® Isolera One; Column: Biotage® SNAP Ultra 100 g; Gradient: Cyclohexane/Ethyl acetate, 5% Ethyl acetate -> 40% Ethyl acetate; Flow: 100 ml/min). 3.89 g (50% yield, 83% purity) of the title compound were obtained.
LC-MS (Method 7): Rₜ = 1.81 min; MS (ESIpos): m/z = 239 [M+H]⁺

### Intermediate 98

### Ethyl 2-[(4-fluorophenyl)hydrazono]-3-(hydroxyimino)propanoate

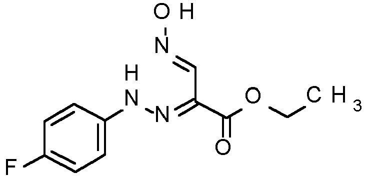

Ethyl 2-[(4-fluorophenyl)hydrazono]-3-oxopropanoate (3.885 g, 83% purity, 13.37 mmol) was dissolved in 41 ml of ethanol and treated with hydroxylamine hydrochloride (1.115 g, 16.05 mmol) and potassium acetate (3.281 g, 33.43 mmol). The mixture was stirred over night at room temperature, diluted with ethyl acetate, washed twice with water and once with saturated aqueous sodium chloride solution. The organic layer was dried over sodium sulfate and filtered. Isolute® was added and the solvent was removed on a rotary evaporator. The crude product was purified by column chromatography (Machine: Biotage® Isolera One; Column: Biotage® SNAP Ultra 100 g; Gradient: Cyclohexane/Ethyl acetate, 5% Ethyl acetate -> 40% Ethyl acetate; Flow: 100 ml/min). 3.07 g (91% yield, 100% purity) of the title compound were obtained.
LC-MS (Method 7): Rₜ = 1.72 min; MS (ESIpos): m/z = 254 [M+H]⁺

### Intermediate 99

### Ethyl 2-(4-fluorophenyl)-2H-1,2,3-triazole-4-carboxylate

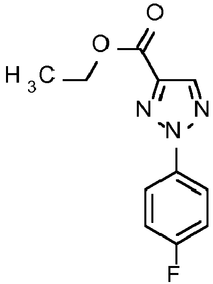

Ethyl 2-[(4-fluorophenyl)hydrazono]-3-(hydroxyimino)propanoate (3.07 g, 12.12 mmol) was dissolved in 45 ml of acetic anhydride. The solution was stirred at 60°C for 1.5 hours before being treated with water. The mixture was extracted with ethyl acetate and the organic layer was dried over sodium sulfate. Isolute® was added and the solvent was removed on a rotary evaporator. The residue was purified by column chromatography (Machine: Biotage® Isolera One; Column: Biotage® SNAP Ultra 100 g; Gradient: Cyclohexane/Ethyl acetate, 5% Ethyl acetate -> 40% Ethyl acetate; Flow: 100 ml/min). 1.84 g (65% yield, 100% purity) of the title compound were obtained.
LC-MS (Method 7): Rₜ = 1.95 min; MS (ESIpos): m/z = 236 [M+H]⁺

### Intermediate 100

### 2-(4-fluorophenyl)-2H-1,2,3-triazole-4-carboxylic acid

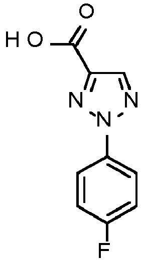

Ethyl 2-(4-fluorophenyl)-2H-1,2,3-triazole-4-carboxylate (1.38 g, 783 mmol) was dissolved in THF, treated with lithium hydroxide solution (1N in water, 7.83 ml, 7.83 mmol), and the mixture was stirred at room temperature for four hours. The THF was removed on a rotary evaporator and the residue was acidified with excess 2N aqueous hydrochloric acid. The precipitate was filtered off, washed with water and dried in vacuo. 1.38 g (85% yield, 100% purity) of the title compound were obtained.
LC-MS (Method 7): Rₜ = 1.23 min; MS (ESIpos): m/z = 206 [M-H]⁻

### Intermediate 101

### Ethyl 2-[2-(3-fluorophenyl)hydrazinylidene]-3-oxopropanoate

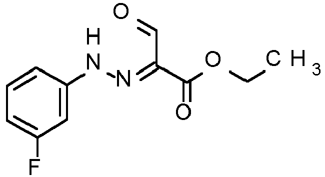

0.96 g (14.00 mmol) of sodium nitrite in 10 mL of water was added dropwise to a cold solution of 1.30 mL (14.00 mmol) of 3-fluoroaniline in 3 mL of concentrated hydrochloric acid and 10 mL of water at 0 °C and the mixture was stirred for 5 min under the same conditions. This solution was added drop wise to a cold solution of 2.00 g (14.00 mmol) of (2E)-3-(dimethylamino)prop-2-enoate and 2.06 g (21.00 mmol) of potassium acetate in 20 mL of ethanol and the reaction was stirred at room temperature for 14 hours. The mixture was then diluted with ethyl acetate and the dark red solution was washed with water and brine. The aqueous phase was dried over magnesium sulfate, filtered and concentrated *in vacuo* to afford 3.19 g (96%) of the product as a red solid, which required no further purification.
LC-MS (Method 3): Rₜ = 0.920 min. MS (Mass method 1): m/z = 239 (M + H)⁺

### Intermediate 102

### Ethyl 2-[2-(3-fluorophenyl)hydrazinylidene]-3-(hydroxyimino)propanoate

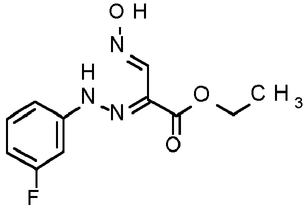

3.20 g (13.40 mmol) of ethyl 2-[2-(3-fluorophenyl)hydrazinylidene]-3-oxopropanoate, 0.93 g (13.40 mmol) of hydroxylamine hydrochloride and 3.30 g (33.60 mmol) of potassium acetate were mixed in 40 mL of ethanol and heated to 78 °C for 30 min. The reaction was cooled to room temperature and 20 mL of water were added. The mixture was extracted with ethyl acetate and the combined organic extracts were dried over magnesium sulfate, filtered and concentrated to give 3.39 g (99%) of the product as a tan thick oil. The compound was used in the next step without further purification.
LC-MS (Method 3): Rₜ = 0.901 min. MS (Mass method 1): m/z = 254 (M + H)⁺

### Intermediate 103

### Ethyl 2-(3-fluorophenyl)-2H-1,2,3-triazole-4-carboxylate

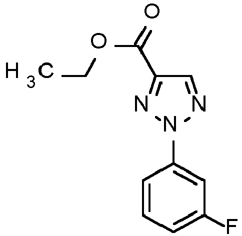

3.30 g (13.00 mmol) of ethyl 2-[2-(3-fluorophenyl)hydrazinylidene]-3-(hydroxyimino)propanoate were dissolved in 30 mL of acetic anhydride and the reaction was heated at 140 °C for 1 hour. The mixture was then cooled down to room temperature and 10 mL of water was added. The dark solution was extracted with dichloromethane and the combined organic layers were dried over magnesium sulfate, filtered and concentrated. The resulting residue was purified by flash chromatography on silica gel eluting with heptane/dichloromethane mixtures to afford 0.30 g (10%) of the product as a yellow solid.
¹H-NMR (300 MHz, DMSO-*d*₆): δ [ppm] = 1.34 (t, 3H), 4.39 (q, 2H), 7.38 (t, 1H), 7.67 (q, 1H), 7.86 (d, 1H), 7.94 (d, 1H), 8.63 (s, 1H).
LC-MS (Method 3): Rₜ = 1.004 min. MS (Mass method 1): m/z = 236 (M + H)⁺

### Intermediate 104

### 2-(3-fluorophenyl)-2H-1,2,3-triazole-4-carboxylic acid

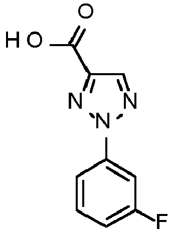

107 mg (2.55 mmol) of lithium hydroxide monohydrate were added to a solution of 300 mg (1.28 mmol) of ethyl 2-(3-fluorophenyl)-2H-1,2,3-triazole-4-carboxylate in 4 mL of tetrahydrofuran and 2 mL of water and the reaction was allowed to stir at room temperature for 2 hours. The pH was adjusted to 4-5 by addition of 2N hydrochloric acid(aq.) and the solvent was totally removed to yield 260 mg (98%) of the product as a yellow powder. The compound was used in the next step without further purification.
LC-MS (Method 3): Rₜ = 0.667 min. MS (Mass method 1): m/z = 208 (M + H)⁺

### Intermediate 105

### Ethyl 2-{[3-fluoro-4-(trifluoromethyl)phenyl]hydrazono}-3-oxopropanoate

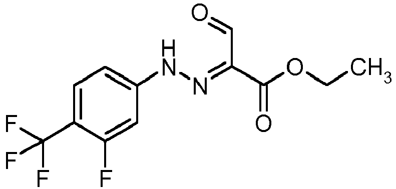

0.48 g (6.98 mmol) of sodium nitrite in 5 mL of water were added dropwise to a cold solution of 1.25 g (6.98 mmol) of 3-fluoro-4-(trifluoromethyl)aniline in 3 mL of concentrated hydrochloric acid and 5 mL of water at 0 °C and the mixture was stirred for 5 min under the same conditions. This solution was added drop wise to a cold solution of 1.00 g (6.98 mmol) of (2E)-3-(dimethylamino)prop-2-enoate and 1.03 g (10.47 mmol) of potassium acetate in 15 mL of ethanol and the reaction was stirred at room temperature for 14 hours. The mixture was then diluted with ethyl acetate and the dark red solution was washed with water and brine. The aqueous phase was dried over magnesium sulfate, filtered and concentrated *in vacuo* to afford 2.06 g (96%) of the product as a deep red thick oil, which required no further purification.
¹H-NMR (300 MHz, DMSO-*d*₆): δ [ppm] = 1.31 (t, 3H), 4.26-4.40 (q, 2H), 7.49-7.72 (m, 2H), 7.76-7.87 (m, 1H), 9.51 (s, 1H), 9.86 (s, 1H).
LC-MS (Method 3): Rₜ = 1.175 min. MS (Mass method 1): m/z = 307 (M + H)⁺

### Intermediate 106

### Ethyl 2-{2-[3-fluoro-4-(trifluoromethyl)phenyl]hydrazinylidene}-3-(hydroxyimino)propanoate

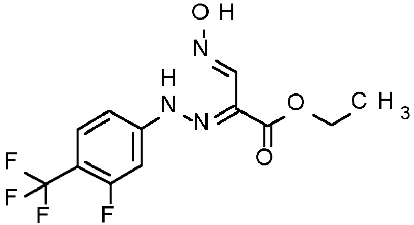

2.06 g (6.73 mmol) of ethyl 2-{2-[3-fluoro-4-(trifluoromethyl)phenyl]hydrazinylidene}-3-oxopropanoate, 0.56 g (8.07 mmol) of hydroxylamine hydrochloride and 1.65 g (16.80 mmol) of potassium acetate were mixed in 50 mL of ethanol and heated to 78 °C for 30 min. The reaction was cooled to room temperature and 20 mL of water were added. The mixture was extracted with ethyl acetate and the combined organic extracts were dried over magnesium sulfate, filtered and concentrated to give 2.10 g (97%) of the product as a red thick oil. The compound was used in the next step without further purification.
¹H-NMR (300 MHz, DMSO-*d*₆): δ [ppm] = 1.30 (t, 3H), 4.27 (q, 2H), 7.16-7.33 (m, 2H), 7.77 (t, 1H), 8.27 (s, 1H), 12.32 (s, 1H), 12.54 (s, 1H).
LC-MS (Method 3): Rₜ = 1.009 min. MS (Mass method 1): m/z = 322 (M + H)⁺

### Intermediate 107

### Ethyl 2-[3-fluoro-4-(trifluoromethyl)phenyl]-2H-1,2,3-triazole-4-carboxylate

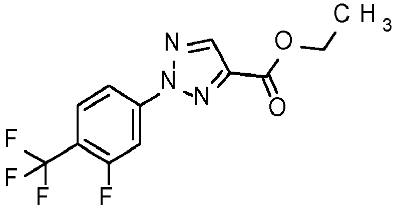

2.20 g (6.85 mmol) of ethyl 2-{2-[3-fluoro-4-(trifluoromethyl)phenyl]hydrazinylidene}-3-(hydroxyimino)propanoate were dissolved in 50 mL of acetic anhydride and the reaction was heated at 140 °C for 1 hour. The mixture was then cooled down to room temperature and 10 mL of water was added. The dark solution was extracted with dichloromethane and the combined organic layers were dried over magnesium sulfate, filtered and concentrated. The resulting residue was purified by flash chromatography on silica gel eluting with heptane/dichloromethane mixtures to afford 1.30 g (63%) of the product as a yellow solid.
¹H-NMR (300 MHz, DMSO-*d*₆): δ [ppm] = 1.35 (t, 3H), 4.41 (q, 1H), 8.00-8.23 (m, 3H), 8.73 (s, 1H).
LC-MS (Method 3): Rₜ = 1.300 min. MS (Mass method 1): m/z = 304 (M + H)⁺

### Intermediate 108

### 2-[3-fluoro-4-(trifluoromethyl)phenyl]-2H-1,2,3-triazole-4-carboxylic acid

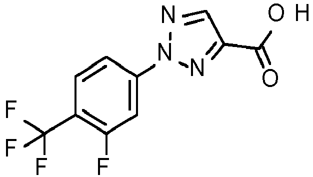

0.26 g (5.14 mmol) of lithium hydroxide monohydrate were added to a solution of 1.30 g (4.29 mmol) of ethyl 2-[3-fluoro-4-(trifluoromethyl)phenyl]-2H-1,2,3-triazole-4-carboxylate in 20 mL of tetrahydrofuran and 10 mL of water and the reaction was allowed to stir at room temperature for 2 hours. The pH was adjusted to 4-5 by addition of 2N hydrochloric acid(aq.) and the solvent was totally removed to yield 1.18 g (99%) of the product as a yellow powder. The compound was used in the next step without further purification.
¹H-NMR (300 MHz, DMSO-*d*₆): δ [ppm] = 7.94-7.86 (m, 3H), 8.09 (s, 1H).
LC-MS (Method 3): Rₜ = 0.767 min. MS (Mass method 1): m/z = 276 (M + H)⁺

### Intermediate 109

### Ethyl 3-oxo-2-(2-phenylhydrazinylidene)propanoate

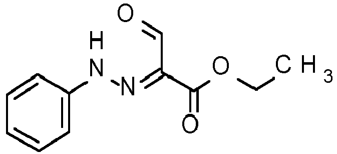

0.96 g (13.96 mmol) of sodium nitrite in 10 mL of water were added dropwise to a cold solution of 1.3 mL (13.96 mmol) of aniline in 3 mL of concentrated hydrochloric acid and 10 mL of water at 0 °C and the mixture was stirred for 5 min under the same conditions. This solution was added drop wise to a cold solution of 2.00 g (14.00 mmol) of (2E)-3-(dimethylamino)prop-2-enoate and 2.06 g (21.00 mmol) of potassium acetate in 15 mL of ethanol and the reaction was stirred at room temperature for 14 hours. The mixture was then diluted with ethyl acetate and the dark red solution was washed with water and brine. The aqueous phase was dried over magnesium sulfate, filtered and concentrated *in vacuo* to afford 2.86 g (93%) of the product as a red solid, which required no further purification.
LC-MS (Method 3): Rₜ = 0.852 min. MS (Mass method 1): m/z = 221 (M + H)⁺

### Intermediate 110

### Ethyl 3-(hydroxyimino)-2-(2-phenylhydrazinylidene)propanoate

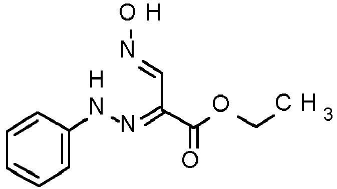

2.86 g (13.00 mmol) of ethyl 3-oxo-2-(2-phenylhydrazinylidene)propanoate, 1.08 g (15.60 mmol) of hydroxylamine hydrochloride and 3.19 g (32.50 mmol) of potassium acetate were mixed in 30 mL of ethanol and heated to 78 °C for 30 min. The reaction was cooled to room temperature and 20 mL of water were added. The mixture was extracted with ethyl acetate and the combined organic extracts were dried over magnesium sulfate, filtered and concentrated to give 1.20 g (34%) of the product as a red thick oil. The compound was used in the next step without further purification.
LC-MS (Method 3): Rₜ = 0.820 min. MS (Mass method 1): m/z = 236 (M + H)⁺

### Intermediate 111

### Ethyl 2-phenyl-2H-1,2,3-triazole-4-carboxylate

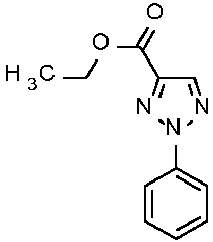

1.20 g (5.10 mmol) of ethyl 3-(hydroxyimino)-2-(2-phenylhydrazinylidene)propanoate were dissolved in 20 mL of acetic anhydride and the reaction was heated at 140 °C for 1 hour. The mixture was then cooled down to room temperature and 10 mL of water was added. The dark solution was extracted with dichloromethane and the combined organic layers were dried over magnesium sulfate, filtered and concentrated. The resulting residue was purified by flash chromatography on silica gel eluting with heptane/dichloromethane mixtures to afford 0.86 g (78%) of the product as a yellow solid.
¹H-NMR (300 MHz, DMSO-*d*₆): δ [ppm] = 1.34 (t, 3H), 4.39 (q, 2H), 7.47-7.55 (m, 1H), 7.62 (t, 2H), 8.06 (d, 2H), 8.59 (s, 1H).
LC-MS (Method 3): Rₜ = 0.918 min. MS (Mass method 1): m/z = 218 (M + H)⁺

### Intermediate 112

### 2-phenyl-2H-1,2,3-triazole-4-carboxylic acid

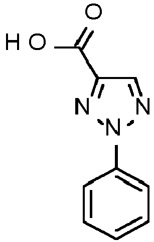

332 mg (7.92 mmol) of lithium hydroxide monohydrate were added to a solution of 860 mg (3.96 mmol) of ethyl 2-phenyl-2H-1,2,3-triazole-4-carboxylate in 10 mL of tetrahydrofuran and 5 mL of water and the reaction was allowed to stir at room temperature for 2 hours. The pH was adjusted to 4-5 by addition of 2N hydrochloric acid(aq.) and the solvent was totally removed to yield 740 mg (99%) of the product as a yellow powder. The compound was used in the next step without further purification.
¹H-NMR (300 MHz, DMSO-*d*₆): δ [ppm] = 7.39 (t, 1H), 7.55 (t, 2H), 7.97-8.05 (m, 3H).
LC-MS (Method 3): Rₜ = 0.615 min. MS (Mass method 1): m/z = 190 (M + H)⁺

### Intermediate 113

### Ethyl 2-[2-(3,4-difluorophenyl)hydrazinylidene]-3-oxopropanoate

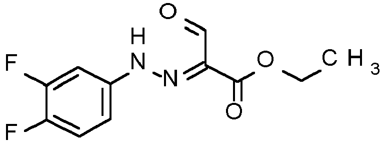

0.96 g (14.00 mmol) of sodium nitrite in 10 mL of water were added dropwise to a cold solution of 1.4 mL (14.00 mmol) of 3,4-difluoroaniline in 4 mL of concentrated hydrochloric acid and 10 mL of water at 0 °C and the mixture was stirred for 5 min under the same conditions. This solution was added drop wise to a cold solution of 2.00 g (14.00 mmol) of (2E)-3-(dimethylamino)prop-2-enoate and 2.06 g (21.00 mmol) of potassium acetate in 15 mL of ethanol and the reaction was stirred at room temperature for 14 hours. The mixture was then diluted with ethyl acetate and the dark red solution was washed with water and brine. The aqueous phase was dried over magnesium sulfate, filtered and concentrated *in vacuo* to afford 3.57 g (99%) of the product as a thick red oil, which required no further purification.
LC-MS (Method 3): Rₜ = 0.858 min. MS (Mass method 1): m/z = 257 (M + H)⁺

### Intermediate 114

### Ethyl 2-[2-(3,4-difluorophenyl)hydrazinylidene]-3-(hydroxyimino)propanoate

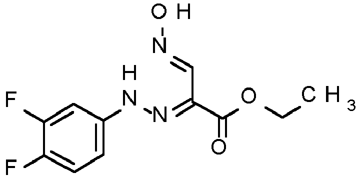

3.57 g (13.90 mmol) of ethyl 2-[2-(3,4-difluorophenyl)hydrazinylidene]-3-oxopropanoate, 1.16 g (16.70 mmol) of hydroxylamine hydrochloride and 3.42 g (34.80 mmol) of potassium acetate were mixed in 40 mL of ethanol and heated to 78 °C for 30 min. The reaction was cooled to room temperature and 20 mL of water were added. The mixture was extracted with ethyl acetate and the combined organic extracts were dried over magnesium sulfate, filtered and concentrated to give 3.77 g (99%) of the product as a red thick oil. The compound was used in the next step without further purification.
LC-MS (Method 3): Rₜ = 0.846 min. MS (Mass method 1): m/z = 272 (M + H)⁺

### Intermediate 115

### Ethyl 2-(3,4-difluorophenyl)-2H-1,2,3-triazole-4-carboxylate

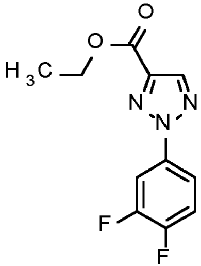

3.77 g (13.90 mmol) of ethyl 2-[2-(3,4-difluorophenyl)hydrazinylidene]-3-(hydroxyimino)propanoate were dissolved in 40 mL of acetic anhydride and the reaction was heated at 140 °C for 1 hour. The mixture was then cooled down to room temperature and 10 mL of water was added. The dark solution was extracted with dichloromethane and the combined organic layers were dried over magnesium sulfate, filtered and concentrated. The resulting residue was purified by flash chromatography on silica gel eluting with heptane/dichloromethane mixtures to afford 1.00 g (28%) of the product as a yellow solid.
¹H-NMR (300 MHz, DMSO-*d*₆): δ [ppm] = 1.34 (t, 3H), 4.39 (q, 2H), 7.71 (q, 1H), 7.93 (d, 1H), 8.12 (td, 1H), 8.63 (s, 1H).
LC-MS (Method 3): Rₜ = 0.920 min. MS (Mass method 1): m/z = 254 (M + H)⁺

### Intermediate 116

### 2-(3,4-difluorophenyl)-2H-1,2,3-triazole-4-carboxylic acid

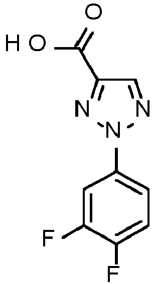

199 mg (4.74 mmol) of lithium hydroxide monohydrate were added to a solution of 1000 mg (3.95 mmol) of ethyl 2-(3,4-difluorophenyl)-2H-1,2,3-triazole-4-carboxylate in 8 mL of tetrahydrofuran and 4 mL of water and the reaction was allowed to stir at room temperature for 2 hours. The pH was adjusted to 4-5 by addition of 2N hydrochloric acid(aq.) and the solvent was totally removed to yield 880 mg (99%) of the product as a yellow powder. The compound was used in the next step without further purification.
¹H-NMR (300 MHz, DMSO-*d*₆): δ [ppm] = 7.64 (q, 1H), 7.84 (d, 1H), 7.94-8.07 (m, 2H).
LC-MS (Method 3): Rₜ = 0.636 min. MS (Mass method 1): m/z = 226 (M + H)⁺

### Intermediate 117

### Ethyl 2-[2-(4-chlorophenyl)hydrazinylidene]-3-oxopropanoate

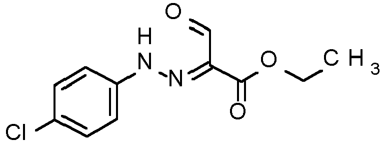

0.48 g (6.98 mmol) of sodium nitrite in 5 mL of water were added dropwise to a cold solution of 0.89 g (6.98 mmol) of 4-chloroaniline in 1.5 mL of concentrated hydrochloric acid and 5 mL of water at 0 °C and the mixture was stirred for 5 min under the same conditions. This solution was added drop wise to a cold solution of 1.00 g (6.98 mmol) of (2E)-3-(dimethylamino)prop-2-enoate and 1.03 g (10.50 mmol) of potassium acetate in 15 mL of ethanol and the reaction was stirred at room temperature for 14 hours. The mixture was then diluted with ethyl acetate and the dark red solution was washed with water and brine. The aqueous phase was dried over magnesium sulfate, filtered and concentrated *in vacuo* to afford 1.58 g (89%) of the product as a thick orange oil, which required no further purification.
LC-MS (Method 3): Rₜ = 0.914 min. MS (Mass method 1): m/z = 255 (M + H)⁺

### Intermediate 118

### Ethyl 2-[2-(4-chlorophenyl)hydrazinylidene]-3-(hydroxyimino)propanoate

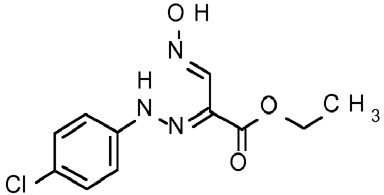

1.58 g (6.20 mmol) of ethyl 2-[2-(4-chlorophenyl)hydrazinylidene]-3-oxopropanoate, 0.52 g (7.44 mmol) of hydroxylamine hydrochloride and 1.52 g (15.50 mmol) of potassium acetate were mixed in 40 mL of ethanol and heated to 78 °C for 30 min. The reaction was cooled to room temperature and 20 mL of water were added. The mixture was extracted with ethyl acetate and the combined organic extracts were dried over magnesium sulfate, filtered and concentrated to give 1.42 g (85%) of the product as a red thick oil. The compound was used in the next step without further purification.
LC-MS (Method 3): Rₜ = 0.889 min. MS (Mass method 1): m/z = 270 (M + H)⁺

### Intermediate 119

### Ethyl 2-(4-chlorophenyl)-2H-1,2,3-triazole-4-carboxylate

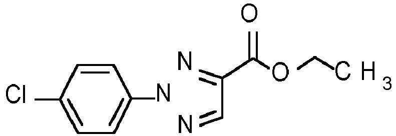

1.42 g (5.27 mmol) of ethyl 2-[2-(4-chlorophenyl)hydrazinylidene]-3-(hydroxyimino) propanoate were dissolved in 15 mL of acetic anhydride and the reaction was heated at 140 °C for 1 hour. The mixture was then cooled down to room temperature and 10 mL of water was added. The dark solution was extracted with dichloromethane and the combined organic layers were dried over magnesium sulfate, filtered and concentrated. The resulting residue was purified by flash chromatography on silica gel eluting with heptane/dichloromethane mixtures to afford 0.56 g (42%) of the product as an orange powder.
¹H-NMR (300 MHz, DMSO-*d*₆): δ [ppm] = 1.35 (t, 3H), 4.40 (q, 2H), 7.69 (d, 2H), 8.09 (d, 2H), 8.62 (s, 1H).
LC-MS (Method 3): Rₜ = 0.986 min. MS (Mass method 1): m/z = 252 (M + H)⁺

### Intermediate 120

### 2-(4-chlorophenyl)-2H-1,2,3-triazole-4-carboxylic acid

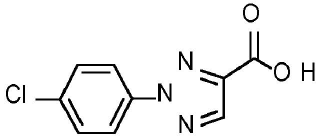

112 mg (2.67 mmol) of lithium hydroxide monohydrate were added to a solution of 561 mg (2.23 mmol) of ethyl 2-(4-chlorophenyl)-2H-1,2,3-triazole-4-carboxylate in 6 mL of tetrahydrofuran and 3 mL of water and the reaction was allowed to stir at room temperature for 2 hours. The pH was adjusted to 4-5 by addition of 2N hydrochloric acid(aq.) and the solvent was totally removed to yield 490 mg (99%) of the product as a light yellow powder. The compound was used in the next step without further purification.
¹H-NMR (300 MHz, DMSO-*d*₆): δ [ppm] = 7.68 (d, 2H), 8.07 (s, 2H), 8.53 (s, 1H).
LC-MS (Method 3): Rₜ = 0.704 min. MS (Mass method 1): m/z = 224 (M + H)⁺

### Intermediate 121

### Ethyl 2-[2-(3-chlorophenyl)hydrazinylidene]-3-oxopropanoate

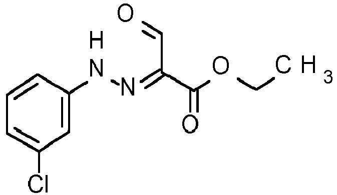

0.48 g (6.98 mmol) of sodium nitrite in 5 mL of water were added dropwise to a cold solution of 0.74 mL (7.00 mmol) of 3-chloroaniline in 1.5 mL of concentrated hydrochloric acid and 5 mL of water at 0 °C and the mixture was stirred for 5 min under the same conditions. This solution was added drop wise to a cold solution of 1.00 g (6.98 mmol) of (2E)-3-(dimethylamino)prop-2-enoate and 1.03 g (10.50 mmol) of potassium acetate in 15 mL of ethanol and the reaction was stirred at room temperature for 14 hours. The mixture was then diluted with ethyl acetate and the dark red solution was washed with water and brine. The aqueous phase was dried over magnesium sulfate, filtered and concentrated *in vacuo* to afford 1.64 g (92%) of the product as a thick dark orange oil, which required no further purification.
LC-MS (Method 3): Rₜ = 0.919 min. MS (Mass method 1): m/z = 255 (M + H)⁺

### Intermediate 122

### Ethyl 2-[2-(3-chlorophenyl)hydrazinylidene]-3-(hydroxyimino)propanoate

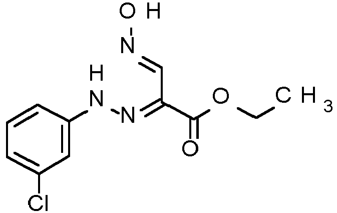

1.64 g (6.44 mmol) of ethyl 2-[2-(3-chlorophenyl)hydrazinylidene]-3-oxopropanoate, 0.54 g (7.73 mmol) of hydroxylamine hydrochloride and 1.58 g (16.10 mmol) of potassium acetate were mixed in 40 mL of ethanol and heated to 78 °C for 30 min. The reaction was cooled to room temperature and 20 mL of water were added. The mixture was extracted with ethyl acetate and the combined organic extracts were dried over magnesium sulfate, filtered and concentrated to give 1.22 g (70%) of the product as a red oil. The compound was used in the next step without further purification.
LC-MS (Method 3): Rₜ = 0.895 min. MS (Mass method 1): m/z = 270 (M + H)⁺

### Intermediate 123

### Ethyl 2-(3-chlorophenyl)-2H-1,2,3-triazole-4-carboxylate

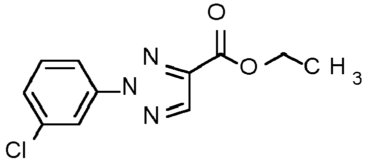

1.22 g (4.53 mmol) of ethyl 2-[2-(3-chlorophenyl)hydrazinylidene]-3-(hydroxyimino) propanoate were dissolved in 15 mL of acetic anhydride and the reaction was heated at 140 °C for 1 hour. The mixture was then cooled down to room temperature and 10 mL of water was added. The dark solution was extracted with dichloromethane and the combined organic layers were dried over magnesium sulfate, filtered and concentrated. The resulting residue was purified by flash chromatography on silica gel eluting with heptane/dichloromethane mixtures to afford 0.47 g (41%) of the product as an orange oil.
¹H-NMR (300 MHz, DMSO-*d*₆): δ [ppm] = 1.35 (t, 3H), 4.40 (q, 2H), 7.57-7.70 (m, 2H), 8.01-8.09 (m, 2H), 8.64 (s, 1H).
LC-MS (Method 3): Rₜ = 0.992 min. MS (Mass method 1): m/z = 252 (M + H)⁺

### Intermediate 124

### 2-(3-chlorophenyl)-2H-1,2,3-triazole-4-carboxylic acid

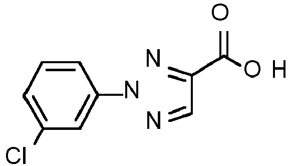

94 mg (2.25 mmol) of lithium hydroxide monohydrate were added to a solution of 472 mg (1.88 mmol) of ethyl 2-(3-chlorophenyl)-2H-1,2,3-triazole-4-carboxylate in 6 mL of tetrahydrofuran and 3 mL of water and the reaction was allowed to stir at room temperature for 2 hours. The pH was adjusted to 4-5 by addition of 2N hydrochloric acid(aq.) and the solvent was totally removed to yield 490 mg (99%) of the product as a light yellow powder. The compound was used in the next step without further purification.
¹H-NMR (300 MHz, DMSO-*d*₆): δ [ppm] = 7.55-7.71 (m, 2H), 8.01-8.09 (m, 2H), 8.56 (s, 1H).
LC-MS (Method 3): Rₜ = 0.697 min. MS (Mass method 1): m/z = 224 (M + H)⁺

### Intermediate 125

### Ethyl 2-[2-(2-chlorophenyl)hydrazinylidene]-3-oxopropanoate

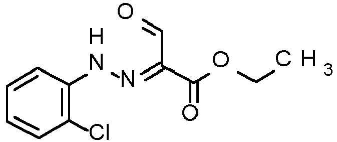

0.48 g (6.98 mmol) of sodium nitrite in 5 mL of water were added dropwise to a cold solution of 0.74 mL (7.00 mmol) of 2-chloroaniline in 1.5 mL of concentrated hydrochloric acid and 5 mL of water at 0 °C and the mixture was stirred for 5 min under the same conditions. This solution was added drop wise to a cold solution of 1.00 g (6.98 mmol) of (2E)-3-(dimethylamino)prop-2-enoate and 1.03 g (10.50 mmol) of potassium acetate in 15 mL of ethanol and the reaction was stirred at room temperature for 14 hours. The mixture was then diluted with ethyl acetate and the dark red solution was washed with water and brine. The aqueous phase was dried over magnesium sulfate, filtered and concentrated *in vacuo* to afford 1.66 g (93%) of the product as a thick dark orange oil, which required no further purification.
LC-MS (Method 3): Rₜ = 0.940 min. MS (Mass method 1): m/z = 255 (M + H)⁺

### Intermediate 126

### Ethyl 2-[2-(2-chlorophenyl)hydrazinylidene]-3-(hydroxyimino)propanoate

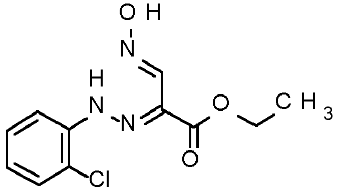

1.66 g (6.52 mmol) of ethyl 2-[2-(2-chlorophenyl)hydrazinylidene]-3-oxopropanoate, 0.54 g (7.82 mmol) of hydroxylamine hydrochloride and 1.60 g (16.30 mmol) of potassium acetate were mixed in 40 mL of ethanol and heated to 78 °C for 30 min. The reaction was cooled to room temperature and 20 mL of water were added. The mixture was extracted with ethyl acetate and the combined organic extracts were dried over magnesium sulfate, filtered and concentrated to give 1.65 g (94%) of the product as a red oil. The compound was used in the next step without further purification.
LC-MS (Method 3): Rₜ = 0.872 min. MS (Mass method 1): m/z = 270 (M + H)⁺

### Intermediate 127

### Ethyl 2-(2-chlorophenyl)-2H-1,2,3-triazole-4-carboxylate

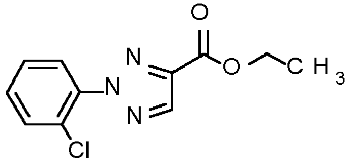

1.65 g (6.11 mmol) of ethyl 2-[2-(2-chlorophenyl)hydrazinylidene]-3-(hydroxyimino) propanoate were dissolved in 20 mL of acetic anhydride and the reaction was heated at 140 °C for 1 hour. The mixture was then cooled down to room temperature and 10 mL of water was added. The dark solution was extracted with dichloromethane and the combined organic layers were dried over magnesium sulfate, filtered and concentrated. The resulting residue was purified by flash chromatography on silica gel eluting with heptane/dichloromethane mixtures to afford 1.10 g (71%) of the product as an orange oil.
¹H-NMR (300 MHz, DMSO-*d*₆): δ [ppm] = 1.34 (t, 3H), 4.39 (q, 2H), 7.58-7.72 (m, 2H), 7.64-7.83 (m, 2H), 8.64 (s, 1H).
LC-MS (Method 3): Rₜ = 0.844 min. MS (Mass method 1): m/z = 252 (M + H)⁺

### Intermediate 128

### 2-(2-chlorophenyl)-2H-1,2,3-triazole-4-carboxylic acid

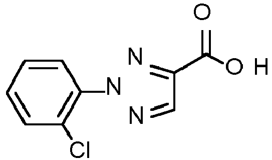

0.22 g (5.24 mmol) of lithium hydroxide monohydrate were added to a solution of 1.10 g (4.37 mmol) of ethyl 2-(2-chlorophenyl)-2H-1,2,3-triazole-4-carboxylate in 6 mL of tetrahydrofuran and 3 mL of water and the reaction was allowed to stir at room temperature for 2 hours. The pH was adjusted to 4-5 by addition of 2N hydrochloric acid(aq.) and the solvent was totally removed to yield 0.97 g (99%) of the product as a yellow powder. The compound was used in the next step without further purification.
¹H-NMR (300 MHz, DMSO-*d*₆): δ [ppm] = 7.55-7.71 (m, 2H), 7.74-7.82 (m, 2H), 8.55 (s, 1H).
LC-MS (Method 3): Rₜ = 0.557 min. MS (Mass method 1): m/z = 224 (M + H)⁺

### Intermediate 129

### Ethyl 2-[2-(2-methylphenyl)hydrazinylidene]-3-oxopropanoate

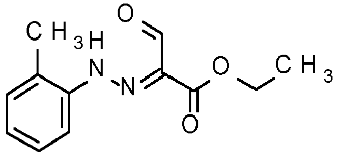

0.48 g (6.98 mmol) of sodium nitrite in 10 mL of water were added dropwise to a cold solution of 0.75 mL (7.00 mmol) of 2-methylaniline in 3 mL of concentrated hydrochloric acid and 10 mL of water at 0 °C and the mixture was stirred for 5 min under the same conditions. This solution was added drop wise to a cold solution of 1.00 g (6.98 mmol) of (2E)-3-(dimethylamino)prop-2-enoate and 1.03 g (10.50 mmol) of potassium acetate in 15 mL of ethanol and the reaction was stirred at room temperature for 14 hours. The mixture was then diluted with ethyl acetate and the dark red solution was washed with water and brine. The aqueous phase was dried over magnesium sulfate, filtered and concentrated *in vacuo* to afford 1.30 g (79%) of the product as a deep red oil, which required no further purification.
LC-MS (Method 3): Rₜ = 0.915 min. MS (Mass method 1): m/z = 234 (M + H)⁺

### Intermediate 130

### Ethyl 3-(hydroxyimino)-2-[2-(2-methylphenyl)hydrazinylidene]propanoate

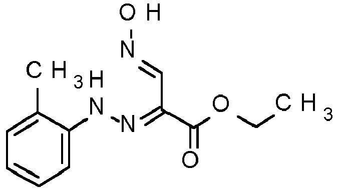

1.30 g (5.55 mmol) of ethyl 2-[2-(2-methylphenyl)hydrazinylidene]-3-oxopropanoate, 0.46 g (6.66 mmol) of hydroxylamine hydrochloride and 1.36 g (13.90 mmol) of potassium acetate were mixed in 30 mL of ethanol and heated to 78 °C for 30 min. The reaction was cooled to room temperature and 20 mL of water were added. The mixture was extracted with ethyl acetate and the combined organic extracts were dried over magnesium sulfate, filtered and concentrated to give 0.89 g (64%) of the product as a thick red oil. The compound was used in the next step without further purification.
LC-MS (Method 3): Rₜ = 0.852 min. MS (Mass method 1): m/z = 250 (M + H)⁺

### Intermediate 131

### Ethyl 2-(2-methylphenyl)-2H-1,2,3-triazole-4-carboxylate

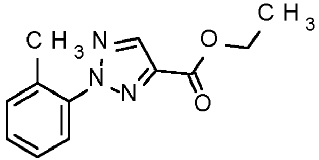

0.89 g (3.57 mmol) of ethyl 3-(hydroxyimino)-2-[2-(2-methylphenyl)hydrazinylidene propanoate were dissolved in 20 mL of acetic anhydride and the reaction was heated at 140 °C for 1 hour. The mixture was then cooled down to room temperature and 10 mL of water was added. The dark solution was extracted with dichloromethane and the combined organic layers were dried over magnesium sulfate, filtered and concentrated. The resulting residue was purified by flash chromatography on silica gel eluting with heptane/dichloromethane mixtures to afford 0.25 g (30%) of the product as a yellow oil.
¹H-NMR (300 MHz, DMSO-*d*₆): δ [ppm] = 1.33 (t, 3H), 2.26 (s, 3H), 4.38 (q, 2H), 7.39-7.53 (m, 3H), 7.59 (d, 1H), 8.58 (s, 1H).
LC-MS (Method 3): Rₜ = 0.875 min. MS (Mass method 1): m/z = 232 (M + H)⁺

### Intermediate 132

### 2-(2-methylphenyl)-2H-1,2,3-triazole-4-carboxylic acid

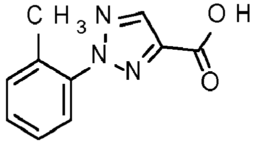

54 mg (1.30 mmol) of lithium hydroxide monohydrate were added to a solution of 250 mg (1.08 mmol) of ethyl 2-(2-methylphenyl)-2H-1,2,3-triazole-4-carboxylate in 4 mL of tetrahydrofuran and 2 mL of water and the reaction was allowed to stir at room temperature for 2 hours. The pH was adjusted to 4-5 by addition of 2N hydrochloric acid(aq.) and the solvent was totally removed to yield 220 mg (99%) of the product as a yellow powder. The compound was used in the next step without further purification.
¹H-NMR (300 MHz, DMSO-*d*₆): δ [ppm] = 2.28 (s, 3H), 7.34-7.48 (m, 3H), 7.55 (d, 1H), 8.18 (s, 1H).
LC-MS (Method 3): Rₜ = 0.589 min. MS (Mass method 1): m/z = 204 (M + H)⁺

### Intermediate 133

### Ethyl 2-[2-(4-methylphenyl)hydrazinylidene]-3-oxopropanoate

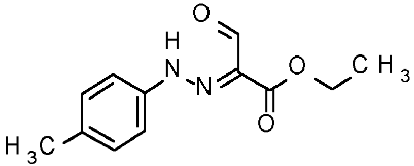

0.48 g (6.98 mmol) of sodium nitrite in 10 mL of water were added dropwise to a cold solution of 0.75 mL (7.00 mmol) of 4-methylaniline in 3 mL of concentrated hydrochloric acid and 10 mL of water at 0 °C and the mixture was stirred for 5 min under the same conditions. This solution was added drop wise to a cold solution of 1.00 g (6.98 mmol) of (2E)-3-(dimethylamino)prop-2-enoate and 1.03 g (10.50 mmol) of potassium acetate in 15 mL of ethanol and the reaction was stirred at room temperature for 14 hours. The mixture was then diluted with ethyl acetate and the dark red solution was washed with water and brine. The aqueous phase was dried over magnesium sulfate, filtered and concentrated *in vacuo* to afford 1.19 g (73%) of the product as a deep red oil, which required no further purification.
LC-MS (Method 3): Rₜ = 0.903 min. MS (Mass method 1): m/z = 234 (M + H)⁺

### Intermediate 134

### Ethyl 3-(hydroxyimino)-2-[2-(4-methylphenyl)hydrazinylidene]propanoate

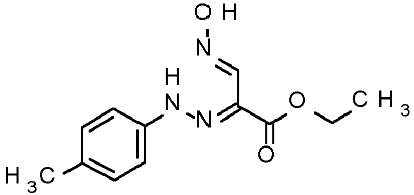

1.19 g (5.08 mmol) of ethyl 2-[2-(4-methylphenyl)hydrazinylidene]-3-oxopropanoate, 0.42 g (6.10 mmol) of hydroxylamine hydrochloride and 1.25 g (12.70 mmol) of potassium acetate were mixed in 30 mL of ethanol and heated to 78 °C for 30 min. The reaction was cooled to room temperature and 20 mL of water were added. The mixture was extracted with ethyl acetate and the combined organic extracts were dried over magnesium sulfate, filtered and concentrated to give 1.19 g (94%) of the product as a thick red oil. The compound was used in the next step without further purification.
LC-MS (Method 3): Rₜ = 0.858 min. MS (Mass method 1): m/z = 250 (M + H)⁺

### Intermediate 135

### Ethyl 2-(4-methylphenyl)-2H-1,2,3-triazole-4-carboxylate

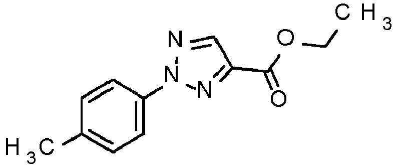

1.19 g (4.77 mmol) of ethyl 3-(hydroxyimino)-2-[2-(4-methylphenyl)hydrazinylidene propanoate were dissolved in 20 mL of acetic anhydride and the reaction was heated at 140 °C for 1 hour. The mixture was then cooled down to room temperature and 10 mL of water was added. The dark solution was extracted with dichloromethane and the combined organic layers were dried over magnesium sulfate, filtered and concentrated. The resulting residue was purified by flash chromatography on silica gel eluting with heptane/dichloromethane mixtures to afford 0.25 g (22%) of the product as a yellow solid.
¹H-NMR (300 MHz, DMSO-*d*₆): δ [ppm] = 1.34 (t, 3H), 2.38 (s, 3H), 4.38 (q, 2H), 7.41 (d, 2H), 7.95 (d, 2H), 8.56 (s, 1H).
LC-MS (Method 3): Rₜ = 0.955 min. MS (Mass method 1): m/z = 232 (M + H)⁺

### Intermediate 136

### 2-(4-methylphenyl)-2H-1,2,3-triazole-4-carboxylic acid

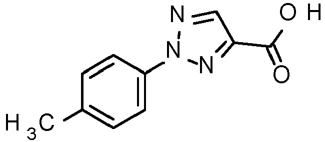

46 mg (1.09 mmol) of lithium hydroxide monohydrate were added to a solution of 210 mg (0.91 mmol) of ethyl 2-(4-methylphenyl)-2H-1,2,3-triazole-4-carboxylate in 4 mL of tetrahydrofuran and 2 mL of water and the reaction was allowed to stir at room temperature for 2 hours. The pH was adjusted to 4-5 by addition of 2N hydrochloric acid(aq.) and the solvent was totally removed to yield 180 mg (97%) of the product as a yellow powder. The compound was used in the next step without further purification.
¹H-NMR (300 MHz, DMSO-*d*₆): δ [ppm] = 2.37 (s, 3H), 7.37 (d, 2H), 7.90 (d, 2H), 8.13 (s, 1H).
LC-MS (Method 3): Rₜ = 0.670 min. MS (Mass method 1): m/z = 204 (M + H)⁺

### Intermediate 137

### Ethyl 2-[2-(4-cyanophenyl)hydrazinylidene]-3-oxopropanoate

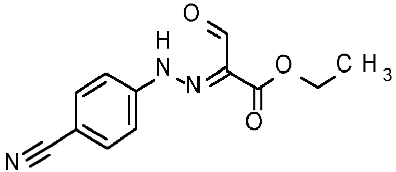

0.48 g (6.98 mmol) of sodium nitrite in 10 mL of water were added dropwise to a cold solution of 0.82 g (7.00 mmol) of 4-aminobenzonitrile in 3 mL of concentrated hydrochloric acid and 10 mL of water at 0 °C and the mixture was stirred for 5 min under the same conditions. This solution was added drop wise to a cold solution of 1.00 g (6.98 mmol) of (2E)-3-(dimethylamino)prop-2-enoate and 1.03 g (10.50 mmol) of potassium acetate in 15 mL of ethanol and the reaction was stirred at room temperature for 14 hours. The mixture was then diluted with ethyl acetate and the dark red solution was washed with water and brine. The aqueous phase was dried over magnesium sulfate, filtered and concentrated *in vacuo* to afford 1.70 g (99%) of the product as a deep red orange oil, which required no further purification.
LC-MS (Method 3): Rₜ = 0.745 min. MS (Mass method 1): m/z = 246 (M + H)⁺

### Intermediate 138

### Ethyl 2-[2-(4-cyanophenyl)hydrazinylidene]-3-(hydroxyimino)propanoate

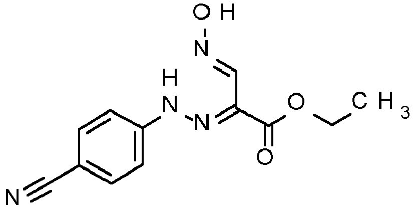

1.70 g (6.93 mmol) of ethyl 2-[2-(4-cyanophenyl)hydrazinylidene]-3-oxopropanoate, 0.58 g (8.32 mmol) of hydroxylamine hydrochloride and 1.70 g (17.30 mmol) of potassium acetate were mixed in 30 mL of ethanol and heated to 78 °C for 30 min. The reaction was cooled to room temperature and 20 mL of water were added. The mixture was extracted with ethyl acetate and the combined organic extracts were dried over magnesium sulfate, filtered and concentrated to give 1.63 g (90%) of the product as a red oil. The compound was used in the next step without further purification.
LC-MS (Method 3): Rₜ = 0.755 min. MS (Mass method 1): m/z = 260 (M + H)⁺

### Intermediate 139

### Ethyl 2-(4-cyanophenyl)-2H-1,2,3-triazole-4-carboxylate

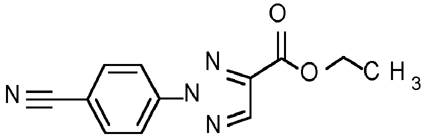

1.63 g (6.24 mmol) of ethyl 2-[2-(4-cyanophenyl)hydrazinylidene]-3-(hydroxyimino)propanoate were dissolved in 20 mL of acetic anhydride and the reaction was heated at 140 °C for 1 hour. The mixture was then cooled down to room temperature and 10 mL of water was added. The dark solution was extracted with dichloromethane and the combined organic layers were dried over magnesium sulfate, filtered and concentrated. The resulting residue was purified by flash chromatography on silica gel eluting with heptane/dichloromethane mixtures to afford 0.55 g (36%) of the product as a yellow solid.
¹H-NMR (300 MHz, DMSO-*d*₆): δ [ppm] = 1.36 (t, 3H), 4.40 (q, 2H), 8.10 (d, 2H), 8.26 (d, 2H), 8.70 (s, 1H).
LC-MS (Method 3): Rₜ = 0.808 min. MS (Mass method 1): m/z = 243 (M + H)⁺

### Intermediate 140

### 2-(4-cyanophenyl)-2H-1,2,3-triazole-4-carboxylic acid

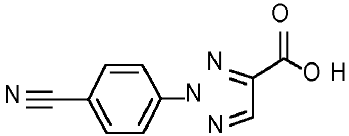

113 mg (2.70 mmol) of lithium hydroxide monohydrate were added to a solution of 545 mg (2.25 mmol) of ethyl 2-(4-cyanophenyl)-2H-1,2,3-triazole-4-carboxylate in 6 mL of tetrahydrofuran and 3 mL of water and the reaction was allowed to stir at room temperature for 2 hours. The pH was adjusted to 4-5 by addition of 2N hydrochloric acid(aq.) and the solvent was totally removed to yield 480 mg (99%) of the product as a yellow powder. The compound was used in the next step without further purification.
¹H-NMR (300 MHz, DMSO-*d*₆): δ [ppm] = 8.10 (d, 2H), 8.25 (d, 2H), 8.61 (s, 1H).
LC-MS (Method 3): Rₜ = 0.513 min. MS (Mass method 1): m/z = 215 (M + H)⁺

### Intermediate 141

### Ethyl 2-[2-(3-cyanophenyl)hydrazinylidene]-3-oxopropanoate

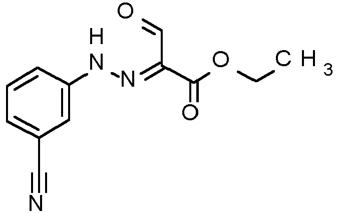

0.48 g (6.98 mmol) of sodium nitrite in 10 mL of water was added drop wise to solution of 0.82 g (6.98 mmol) of 3-aminobenzonitrile in 3 mL of concentrated hydrochloric acid and 5 mL of water at 0 °C and the mixture was stirred for 10 min under the same conditions. After that time, the resulting solution was added drop wise to a solution of 1.00 g (6.98 mmol) of ethyl (2E)-3-(dimethylamino)prop-2-enoate and 1.03 g (10.50 mmol) of potassium acetate in 15 mL of ethanol at 0 °C and then mixture was further stirred at room temperature for 12 hours. The ethanolic fraction of the solvent was removed and the resulting residue was diluted with ethyl acetate and washed with water. The organic layer was dried over magnesium sulfate, filtered and concentrated giving 1.69 g (98%) of the crude product as a deep orange thick oil. The compound was used as such in the next step.
LC-MS (Method 3): Rₜ = 0.752 min. MS (Mass method 1): m/z = 246 (M + H)⁺

### Intermediate 142

### Ethyl 2-[2-(3-cyanophenyl)hydrazinylidene]-3-(hydroxyimino)propanoate

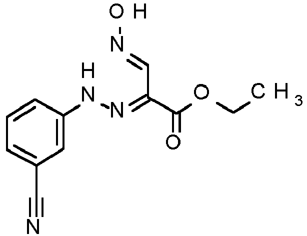

1.69 g (6.89 mmol) of ethyl 2-[2-(3-cyanophenyl)hydrazinylidene]-3-oxopropanoate, 0.58 g (8.27 mmol) of hydroxylamine hydrochloride and 1.69 g (17.20 mmol) potassium acetate were mixed in 30 mL of ethanol and the mixture was heated up to 78 °C for 1 hour. The resulting red suspension was cooled down to room temperature and 20 mL of water was added. The reaction was extracted with ethyl acetate and the combined organic layers were dried over magnesium sulfate, filtered and concentrated to give 1.60 g (89%) of the crude product as a thick red oil. The compound was used as such in the next step.
LC-MS (Method 3): Rₜ = 0.765 min. MS (Mass method 1): m/z = 261 (M + H)⁺

### Intermediate 143

### Ethyl 2-(3-cyanophenyl)-2H-1,2,3-triazole-4-carboxylate

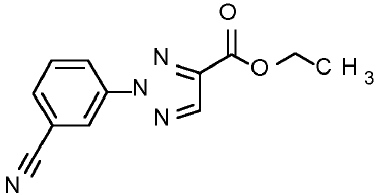

1.60 g (6.15 mmol) of ethyl 2-[2-(3-cyanophenyl)hydrazinylidene]-3-(hydroxyimino) propanoate were dissolved in 20 mL of acetic anhydride and the mixture was stirred at 140 °C for 1 hour. Upon cooling, 20 mL of water were added and the reaction was extracted with dichloromethane. The combined organic layers were dried over magnesium sulfate, filtered and concentrated to give a residue which was further purified by flash chromatography on silica gel using heptane/dichloromethane mixtures to afford 0.68 g (46%) of the product as a yellow solid.
¹H-NMR (300 MHz, DMSO-*d*₆): δ [ppm] = 1.35 (t, 3H), 4.40 (q, 2H), 7.83 (t, 1H), 8.00 (d, 1H), 8.39 (d, 1H), 8.45 (s, 1H), 8.67 (s, 1H).
LC-MS (Method 3): Rₜ = 0.809 min. MS (Mass method 1): m/z = 243 (M + H)⁺

### Intermediate 144

### 2-(3-cyanophenyl)-2H-1,2,3-triazole-4-carboxylic acid

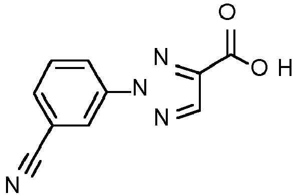

141 mg (3.35 mmol) of lithium hydroxide monohydrate was added to a solution of 677 mg (2.79 mmol) of ethyl 2-(3-cyanophenyl)-2H-1,2,3-triazole-4-carboxylate in 6 mL of tetrahydrofuran and 3 mL of water and the mixture was stirred at room temperature for 2 hours. After that time, pH was set to 3-4 by addition of 2N hydrochloric acid(aq.) and the solvent was totally removed by rotary evaporation. 600 mg (99%) of the crude product was then isolated as a light red powder. The compound was used as such in the next synthetic step.
¹H-NMR (300 MHz, DMSO-*d*₆): δ [ppm] = 7.82 (t, 1H), 7.97 (d, 1H), 8.37 (d, 1H), 8.42 (s, 1H), 8.57 (sm 1H).
LC-MS (Method 3): Rₜ = 0.515 min. MS (Mass method 1): m/z = 215 (M + H)⁺

### Intermediate 145

### Ethyl 2-[2-(3-methylphenyl)hydrazinylidene]-3-oxopropanoate

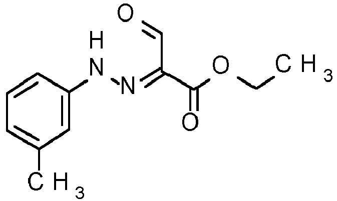

0.48 g (6.98 mmol) of sodium nitrite in 10 mL of water was added drop wise to solution of 0.75 mL (7.00 mmol) of 3-methylaniline in 3 mL of concentrated hydrochloric acid and 5 mL of water at 0 °C and the mixture was stirred for 10 min under the same conditions. After that time, the resulting solution was added drop wise to a solution of 1.00 g (6.98 mmol) of ethyl (2E)-3-(dimethylamino)prop-2-enoate and 1.03 g (10.50 mmol) of potassium acetate in 15 mL of ethanol at 0 °C and then mixture was further stirred at room temperature for 12 hours. The ethanolic fraction of the solvent was removed and the resulting residue was diluted with ethyl acetate and washed with water. The organic layer was dried over magnesium sulfate, filtered and concentrated giving 1.45 g (88%) of the crude product as a deep red thick oil. The compound was used as such in the next step.
LC-MS (Method 3): Rₜ = 0.907 min. MS (Mass method 1): m/z = 235 (M + H)⁺

### Intermediate 146

### Ethyl -3-(hydroxyimino)-2-[2-(3-methylphenyl)hydrazinylidene]propanoate

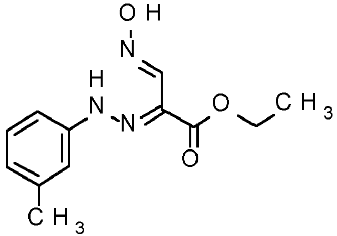

1.45 g (6.19 mmol) of ethyl 2-[2-(3-methylphenyl)hydrazinylidene]-3-oxopropanoate, 0.52 g (7.43 mmol) of hydroxylamine hydrochloride and 1.52 g (15.50 mmol) potassium acetate were mixed in 30 mL of ethanol and the mixture was heated up to 78 °C for 1 hour. The resulting red suspension was cooled down to room temperature and 20 mL of water was added. The reaction was extracted with ethyl acetate and the combined organic layers were dried over magnesium sulfate, filtered and concentrated to give 1.17 g (76%) of the crude product as a thick red oil. The compound was used as such in the next step.
LC-MS (Method 3): Rₜ = 0.855 min. MS (Mass method 1): m/z = 250 (M + H)⁺

### Intermediate 147

### Ethyl 2-(3-methylphenyl)-2H-1,2,3-triazole-4-carboxylate

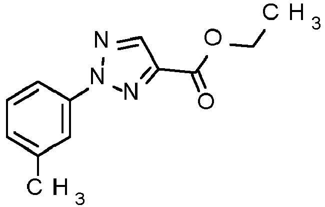

1.17 g (4.69 mmol) of ethyl 3-(hydroxyimino)-2-[2-(3-methylphenyl) hydrazinylidene]propanoate were dissolved in 20 mL of acetic anhydride and the mixture was stirred at 140 °C for 1 hour. Upon cooling, 20 mL of water were added and the reaction was extracted with dichloromethane. The combined organic layers were dried over magnesium sulfate, filtered and concentrated to give a residue which was further purified by flash chromatography on silica gel using heptane/dichloromethane mixtures to afford 0.30 g (28%) of the product as a yellow oil.
¹H-NMR (300 MHz, DMSO-*d*₆): δ [ppm] = 1.34 (t, 3H), 2.43 (s, 3H), 4.38 (q, 2H), 7.33 (d, 1H), 7.48 (t, 1H), 7.82-7.91 (m, 2H), 8.57 (s, 1H).
LC-MS (Method 3): Rₜ = 0.959 min. MS (Mass method 1): m/z = 232 (M + H)⁺

### Intermediate 148

### 2-(3-methylphenyl)-2H-1,2,3-triazole-4-carboxylic acid

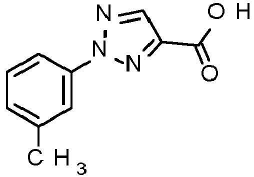

65 mg (1.56 mmol) of lithium hydroxide monohydrate was added to a solution of 300 mg (1.30 mmol) of ethyl 2-(3-methylphenyl)-2H-1,2,3-triazole-4-carboxylate in 4 mL of tetrahydrofuran and 2 mL of water and the mixture was stirred at room temperature for 2 hours. After that time, pH was set to 3-4 by addition of 2N hydrochloric acid(aq.) and the solvent was totally removed by rotary evaporation. 260 mg (98%) of the crude product was then isolated as a light yellow powder. The compound was used as such in the next synthetic step.
¹H-NMR (300 MHz, DMSO-*d*₆): δ [ppm] = 2.40 (s, 3H), 7.21 (d, 1H), 7.42 (t, 1H), 7.78 (d, 1H), 7.83 (s, 1H), 7.99 (s, 1H).
LC-MS (Method 3): Rₜ = 0.671 min. MS (Mass method 1): m/z = 205 (M + H)⁺

### Intermediate 149

### 2-(pyridin-2-yl)-2H-1,2,3-triazole-4-carboxylic acid

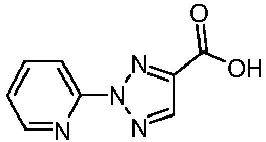

1.00 g (7.87 mmol) of methyl-2H-1,2,3-triazole-4-carboxylate, 0.76 mL (7.20 mmol) of 2-iodopyridine, 0.06 g (0.72 mmol) of copper(II) oxide, 0.76 g (2.15 mmol) of iron(III) acetylacetonate and 4.66 g (14.30 mmol) of cesium carbonate were mixed in 10 mL of N,N-dimethylformamide and the resulting suspension was heated at 100 °C for 12 hours. The reaction mixture was diluted with dichloromethane and extracted with water. The combined aqueous extracts were filtered through a pad of celite and concentrated under vacuum to afford a dark crude, which was further purified by flash column chromatography on silica gel eluting with dichloromethane/methanol mixtures to give 0.30 g (20%) of the product as a yellow powder.
¹H-NMR (300 MHz, DMSO-*d*₆): δ [ppm] = 7.60 (t, 1H), 8.05-8.22 (m, 2H), 8.56 (s, 1H), 8.64 (d, 1H).
LC-MS (Method 3): Rₜ = 0.296 min. MS (Mass method 1): m/z = 191 (M + H)⁺

### Intermediate 150

### 2-(5-fluoropyridin-2-yl)-2H-1,2,3-triazole-4-carboxylic acid

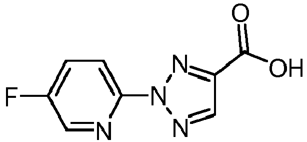

278 mg (2.19 mmol) of methyl-2H-1,2,3-triazole-4-carboxylate, 350 mg (1.99 mmol) of 2-bromo-5-fluoropyridine, 16 mg (0.20 mmol) of copper(II) oxide, 211 mg (0.60 mmol) of iron(III) acetylacetonate and 1300 mg (3.98 mmol) of cesium carbonate were mixed in 5 mL of N,N-dimethylformamide and the resulting suspension was heated at 100 °C for 12 hours. The reaction mixture was diluted with dichloromethane and extracted with water. The combined aqueous extracts were filtered through a pad of celite and concentrated under vacuum to afford a dark crude, which was further purified by flash column chromatography on silica gel eluting with dichloromethane/methanol/acetic acid mixtures to give 400 mg (88%) of the product as a yellow powder.
LC-MS (Method 3): Rₜ = 0.322 min. MS (Mass method 1): m/z = 209 (M + H)⁺

### Intermediate 151

### Methyl 2-(pyrazin-2-yl)-2H-1,2,3-triazole-4-carboxylate

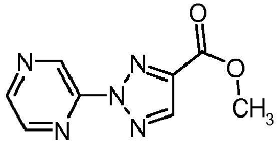

250 mg (1.96 mmol) of methyl-2H-1,2,3-triazole-4-carboxylate, 0.32 mL (3.94 mmol) of 2-fluoropyrazine and 730 mg (5.31 mmol) potassium carbonate were dissolved in 6 mL of N,N-dimethylformamide. To this mixture, 49 mg (0.29 mmol) of potassium iodide and 52 mg (0.20 mmol) of 18-crown-6 were added and the reaction was stirred at 135 °C for 23 hours. After completion of the reaction, the suspension was concentrated under reduced pressure and the residue was dissolved in ethyl acetate and washed with water. The organic layer was dried over magnesium sulfate, filtered, concentrated and chromatographed in heptane/ethyl acetate mixtures to afford 70 mg (17%) of the product as a thick yellow oil, which solidified upon standing. ¹H-NMR (300 MHz, DMSO-*d*₆): δ [ppm] = 3.93 (s, 3H), 8.71-8.78 (m, 2H), 8.85 (s, 1H), 9.37 (s, 1H).
LC-MS (Method 3): Rₜ = 0.401 min. MS (Mass method 1): m/z = 206 (M + H)⁺

### Intermediate 152

### 2-(pyrazin-2-yl)-2H-1,2,3-triazole-4-carboxylic acid

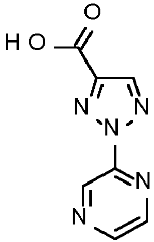

15 mg (0.35 mmol) of lithium hydroxide were added to a solution of 60 mg (0.29 mmol) of methyl 2-(pyrazin-2-yl)-2H-1,2,3-triazole-4-carboxylate in 2 mL of tetrahydrofuran and 1 mL of water and the mixture was stirred at room temperature for 5 hours. Then, pH was set to 4-5 by addition of 2N hydrochloric acid and the solvent was removed *in vacuo* to give 60 mg (98%) of the product as a light yellow powder. The compound was used in the next step without further purification.
LC-MS (Method 3): Rₜ = 0.159 min. MS (Mass method 1): m/z = 192 (M + H)⁺

### Intermediate 153

### 1-(cyclopropylmethyl)-1H-pyrazole-4-carbaldehyde

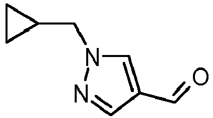

0.5 g (5.20 mmol) of pyrazole-4-carbaldehyde were added to a suspension of 0.23 g (5.72 mmol) 60% sodium hydride (as a dispersion in mineral oil) in 15 mL of N,N-dimethylformamide at 0°C and the mixture was stirred for 30 min. After that time, a solution of 0.56 mL (5.72 mmol) of cyclopropylmethyl bromide in 5 mL of N,N-dimethylformamide was added dropwise and the mixture was stirred at room temperature for 18 hours. The reaction was diluted with ethyl acetate and extracted with water. The organic layer was dried over magnesium sulfate, filtered and concentrated under vacuum affording a crude which was purified by flash column chromatography on silica gel eluting with heptane/ethyl acetate mixtures to give 0.66 g (84%) of the product as a yellow oil.
¹H-NMR (300 MHz, DMSO-*d*₆): δ [ppm] = 0.34-0.42 (m, 2H), 0.50-0.59 (m, 2H), 1.19-1.34 (m, 1H), 4.03 (d, 2H), 7.98 (s, 1H), 8.49 (s, 1H), 9.79 (s, 1H).
LC-MS (Method 3): Rₜ = 0.432 min. MS (Mass method 1): m/z = 151 (M + H)⁺

### Intermediate 154

### N-{[1-(cyclopropylmethyl)-1H-pyrazol-4-yl]methylidene}hydroxylamine

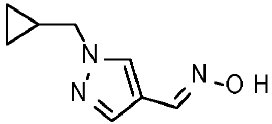

0.53 mL (8.70 mmol) of 50% hydroxylamine in water were dissolved in 6 mL of ethanol. Then, a solution of 0.65 g (4.63 mmol) of 1-(cyclopropylmethyl)-1H-pyrazole-4-carbaldehyde in 6 mL of ethanol was added and the mixture was stirred for 30 min at room temperature. The reaction was concentrated and the resulting residue was dissolved in toluene in order to remove the remains of water azeotropically. 0.71 g (98%) of the crude product were isolated as a tan oil, which required no further purification.
¹H-NMR (300 MHz, DMSO-*d*₆): δ [ppm] = 0.34-0.40 (m, 2H), 0.47-0.57 (d, 2H), 1.16-1.31 (m, 1H), 4.00 (d, 2H), 7.35 (s, 1H), 7.80 (s, 1H), 8.28 (s, 1H), 11.20 (bp, 1H).
LC-MS (Method 3): Rₜ = 0.421 min. MS (Mass method 1): m/z = 166 (M + H)⁺

### Intermediate 155

### Ethyl 3-[1-(cyclopropylmethyl)-1H-pyrazol-4-yl]-1,2-oxazole-5-carboxylate

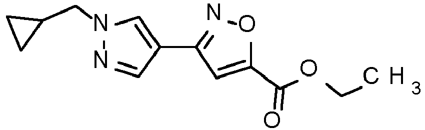

0.56 g (4.18 mmol) of N-chlorosuccinimide were added to a solution of 0.66 g (3.98 mmol) of N-{[1-(cyclopropylmethyl)-1H-pyrazol-4-yl]methylidene}hydroxylamine in 25 mL of dichloromethane at room temperature and the mixture was stirred for 5 min. Then, 1.61 mL (15.93 mmol) of ethyl propiolate and 0.83 mL (5.97 mmol) of triethylamine were sequentially added drop wise and the reaction was stirred at room temperature for 18 hours. The reaction mixture was washed with water and brine and the organic layer was dried over magnesium sulfate, filtered, concentrated and chromatographed by flash column chromatography on silica gel eluting with heptane/ethyl acetate mixtures to give 0.53 g (51%) of the product as an orange oil
LC-MS (Method 3): Rₜ = 0.815 min. MS (Mass method 1): m/z = 262 (M + H)⁺

### Intermediate 156

### 3-[1-(cyclopropylmethyl)-1 H-pyrazol-4-yl]-1 ,2-oxazole-5-carboxylic acid

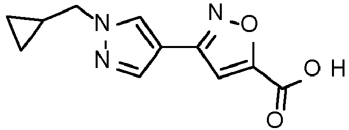

104 mg (2.47 mmol) of lithium hydroxide monohydrate were added to a solution of 430 mg (1.65 mmol) of ethyl 3-[1-(cyclopropylmethyl)-1H-pyrazol-4-yl]-1,2-oxazole-5-carboxylate in 6 mL of tetrahydrofuran and 4 mL of water and the mixture was stirred at room temperature for 1 hour. The pH was then adjusted to 4-5 by addition of 2N hydrochloric acid(aq.) and the reaction was concentrated by rotary evaporation to yield 380 mg (99%) of the crude product as an orange oil. The isolated compound was used as such in the next synthetic step.
LC-MS (Method 3): Rₜ = 0.431 min. MS (Mass method 1): m/z = 234 (M + H)⁺

### Intermediate 157

### Methyl N-benzoyl-beta-alaninate

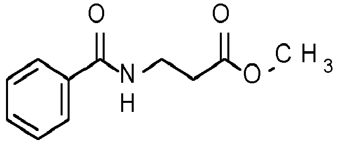

1.50 mL (13.00 mmol) of benzoyl chloride were carefully added to a solution of 1.98 g (14.20 mmol) of beta-alanine methyl ester hydrochloride and 4.50 mL (32.00 mmol) of triethylamine in 30 mL of dichloromethane and the mixture was stirred at room temperature for 12 hours. The reaction was quenched with water and the organic layer was separated, dried over magnesium sulfate, filtered, concentrated and chromatographed in heptane/ethyl acetate mixtures to afford 2.50 g (93%) of the product as a colourless thick oil which crystallised with time.
¹H-NMR (300 MHz, DMSO-*d*₆): δ [ppm] = 2.59 (t, 2H), 3.49 (q, 2H), 3.60 (s, 3H), 7.40-7.56 (m, 3H), 7.82 (d, 2H), 8.54 (t, 1H).
LC-MS (Method 3): Rₜ = 0.515 min. MS (Mass method 1): m/z = 208 (M + H)⁺

### Intermediate 158

### Methyl 2-phenyl-1,3-oxazole-5-carboxylate

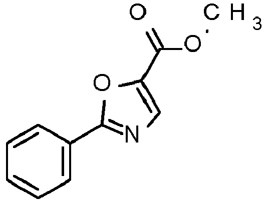

1.50 g (7.24 mmol) of methyl N-benzoyl-beta-alaninate, 3.86 g (21.70 mmol) of N-bromosuccinimide and 2.00 g (14.50 mmol) of potassium carbonate were dissolved in 30 mL of 1,2-dichloroethane and the reaction was stirred at 100 °C for 48 hours. Upon cooling, the mixture was quenched with sodium thiosulfate(sat.) and the reaction was extracted with diethyl ether. The combined organic layers were dried over magnesium sulfate, filtered, concentrated and chromatographed in heptane/ethyl acetate mixtures to afford 0.16 g (11%) of the product as a white powder.
¹H-NMR (300 MHz, DMSO-*d*₆): δ [ppm] = 3.88 (s, 3H), 7.54-7.64 (m, 2H), 8.06 (d, 2H), 8.13 (s, 1H).
LC-MS (Method 3): Rₜ = 0.838 min. MS (Mass method 1): m/z = 204 (M + H)⁺

### Intermediate 159

### 2-Phenyl-1,3-oxazole-5-carboxylic acid

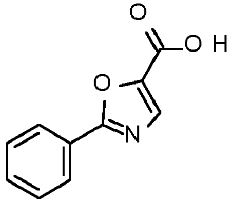

40 mg (0.94 mmol) of lithium hydroxide monohydrate were added to a solution of 160 mg (0.78 mmol) of methyl 2-phenyl-1,3-oxazole-5-carboxylate in 4 mL of tetrahydrofuran and 2 mL of water and the mixture was stirred at room temperature for 10 hours. The pH was then adjusted to 4-5 by addition of 2N hydrochloric acid(aq.) and the mixture was concentrated by rotary evaporation giving 150 mg (100%) of the crude product as a fluffy white solid which was used as such in the next synthetic step.
¹H-NMR (300 MHz, DMSO-*d*₆): δ [ppm] = 7.53-7.61 (m, 3H), 7.80 (s, 1H), 7.99-8.06 (m, 2H).
LC-MS (Method 3): Rₜ = 0.566 min. MS (Mass method 1): m/z = 190 (M + H)⁺

### Intermediate 160

### Ethyl (2E)-2-[(4-methoxyphenyl)hydrazono]-3-oxopropanoate

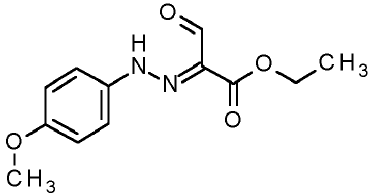

4-Methoxyaniline (1.00 g, 8.12 mmol) was dissolved in 10 ml of water and 2 ml of concentrated hydrochloric acid. The mixture was cooled to 0°C and a solution of sodium nitrite (560 mg, 8.12 mmol) in 5 ml of water was added dropwise. After stirring for 5 minutes this solution was added dropwise to a solution of ethyl-(2E)-3-(dimethylamino)acrylate (1.3 ml, 8.9 mmol) and potassium acetate (1.20 g, 12.2 mmol) in 15 ml of ethanol at 0°C. The mixture was stirred at room temperature for one hour before being diluted with ethyl acetate and washed twice with water and once with brine. The organic layer was dried over sodium sulfate, filtered and evaporated. 2.03 g (67 % yield, 67 % purity) of the title compound were obtained.
LC-MS (Method 7): Rₜ = 1.79 min; MS (ESIpos): m/z = 251 [M+H]⁺

### Intermediate 161

### Ethyl (2E,3E)-3-(hydroxyimino)-2-[(4-methoxyphenyl)hydrazono]propanoate

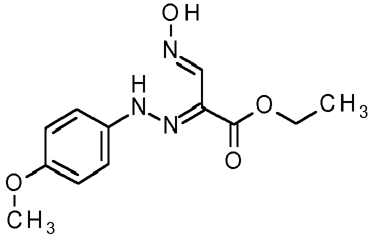

Ethyl (2E)-2-[(4-methoxyphenyl)hydrazono]-3-oxopropanoate (2.03 g, 67 % purity, 5.44 mmol), hydroxylamine hydrochloride (454 mg, 6.53 mmol) and potassium acetate (1.33 g, 13.6 mmol) were dissolved in 20 ml of ethanol and the mixture was stirred for 30 minutes at 80°C. The reaction mixture was diluted with ethyl acetate and washed twice with water and once with brine. The organic layer was dried over sodium sulfate, filtered and evaporated. The residue was dried in vacuum. 1.87 g (46 % purity, 60 % yield) of the title compound were obtained.
LC-MS (Method 7): Rₜ = 1.68 min; MS (ESIpos): m/z = 266 [M+H]⁺

### Intermediate 162

### Ethyl 2-(4-methoxyphenyl)-2H-1,2,3-triazole-4-carboxylate

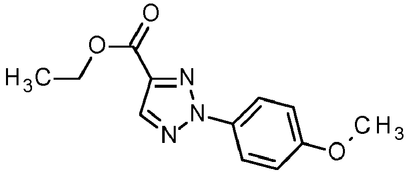

Ethyl (2E,3E)-3-(hydroxyimino)-2-[(4-methoxyphenyl)hydrazono]propanoate (1.87 g, 7.05 mmol) were stirred for one hour in 15 ml of acetic acid anhydride at 140°C. The reaction was then treated with water and extracted with dichlormethane. The organic layer was dried over sodium sulfate, filtered and evaporated. The residue was put on Isolute and purified by chromatography on silica gel (100 g Ultra Snap Cartridge Biotage®; Biotage-Isolera-One®; Cy/EE-gradient: 5% EE - 40% EE; flow: 100 ml/min). Samples containing the desired product were united, the solvents were evaporated and the residue was dried in vacuum. 1.02 g (100 % purity, 58 % yield) of the title compound were obtained.
LC-MS (Method 7): Rₜ = 1.91 min; MS (ESIpos): m/z = 248 [M+H]⁺

### Intermediate 163

### 2-(4-methoxyphenyl)-2H-1,2,3-triazole-4-carboxylic acid

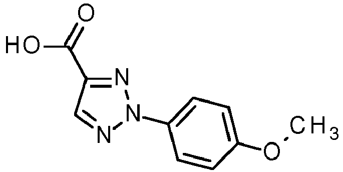

Ethyl 2-(4-methoxyphenyl)-2H-1,2,3-triazole-4-carboxylate (1.02 g, 4.12 mmol) were dissolved in 10 ml of THF, treated with lithium hydroxide solution (4.1 ml, 1.0 M, 4.1 mmol) and the mixture was stirred over night at room temperature. Additional lithium hydroxide solution (2.0 ml, 1.0 M, 2.0 mmol) was added and the mixture was stirred for two more hours. The organic solvent was removed on a rotary evaporator, the aqueous residue was diluted with water and acidified with 1 N hydrochloric acid. The precipitate was filtered off, washed with water and dried in vacuum. 888 mg (99 % purity, 97 % yield) of the title compound were obtained.
LC-MS (Method 7): Rₜ = 1.25 min; MS (ESIpos): m/z = 220 [M+H]⁺

### Intermediate 164

### Ethyl (2E)-2-{[4-(methylsulfonyl)phenyl]hydrazono}-3-oxopropanoate

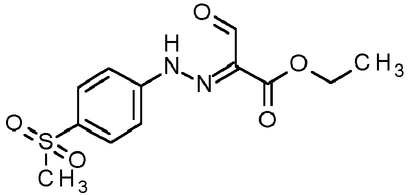

The reaction was carried out analogously to the preparation of ethyl (2E)-2-[(4-methoxyphenyl)hydrazono]-3-oxopropanoate. 1.94 g (75 % purity, 83 % yield) of the title compound were obtained.
LC-MS (Method 7): Rₜ = 1.39 min; MS (ESIpos): m/z = 299 [M+H]⁺

### Intermediate 165

### Ethyl (2E,3E)-3-(hydroxyimino)-2-{[4-(methylsulfonyl)phenyl]hydrazono}propanoate

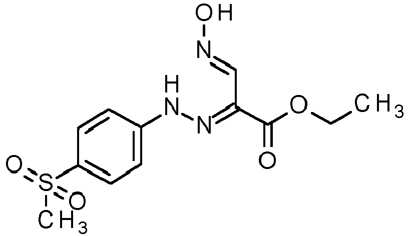

The reaction was carried out analogously to the preparation of ethyl (2E,3E)-3-(hydroxyimino)-2-[(4-methoxyphenyl)hydrazono]propanoate. 1.53 g (100 % purity, 100 % yield) of the title compound were obtained.
LC-MS (Method 8): Rₜ = 0.79 min; MS (ESIpos): m/z = 314 [M+H]⁺

### Intermediate 166

### Ethyl 2-[4-(methylsulfonyl)phenyl]-2H-1,2,3-triazole-4-carboxylate

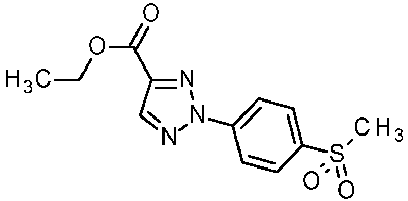

The reaction was carried out analogously to the preparation of ethyl 2-(4-methoxyphenyl)-2H-1,2,3-triazole-4-carboxylate. 989 mg (95 % purity, 65 % yield) of the title compound were obtained.
LC-MS (Method 7): Rₜ = 1.52 min; MS (ESIpos): m/z = 296 [M+H]⁺

### Intermediate 167

### 2-[4-(methylsulfonyl)phenyl]-2H-1,2,3-triazole-4-carboxylic acid

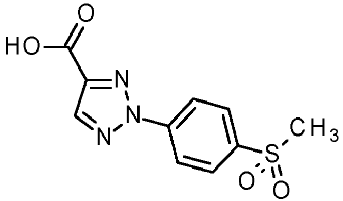

The reaction was carried out analogously to the preparation of 2-(4-methoxyphenyl)-2H-1,2,3-triazole-4-carboxylic acid. 576 mg (96 % purity, 62 % yield) of the title compound were obtained.
LC-MS (Method 7): Rₜ = 0.83 min; MS (ESIneg): m/z = 266 [M-H]⁻

### Intermediate 168

### Ethyl (2E)-2-[2-(2,3-difluorophenyl)hydrazinylidene]-3-oxopropanoate

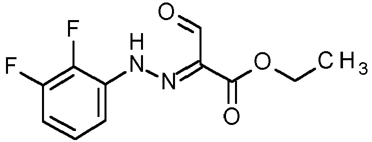

The reaction was carried out analogously to the preparation of ethyl (2E)-2-[(4-methoxyphenyl)hydrazono]-3-oxopropanoate. 913 mg (88 % purity, 81 % yield) of the title compound were obtained.
LC-MS (Method 7): Rₜ = 1.96 min; MS (ESIneg): m/z = 255 [M-H]⁻

### Intermediate 169

### Ethyl (2E,3E)-2-[2-(2,3-difluorophenyl)hydrazinylidene]-3-(hydroxyimino)propanoate

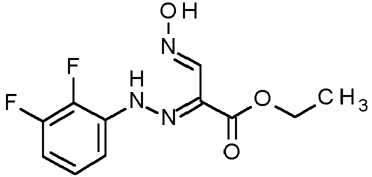

The reaction was carried out analogously to the preparation of ethyl (2E,3E)-3-(hydroxyimino)-2-[(4-methoxyphenyl)hydrazono]propanoate. 869 mg (35 % purity, 36 % yield) of the title compound were obtained.
LC-MS (Method 7): Rₜ = 1.87 min; MS (ESIpos): m/z = 272 [M+H]⁺

### Intermediate 170

### Ethyl 2-(2,3-difluorophenyl)-2H-1,2,3-triazole-4-carboxylate

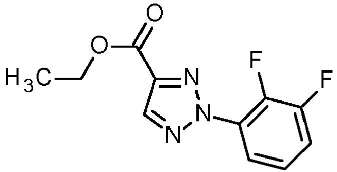

The reaction was carried out analogously to the preparation of ethyl 2-(4-methoxyphenyl)-2H-1,2,3-triazole-4-carboxylate. 236 mg (92 % purity, 75 % yield) of the title compound were obtained.
LC-MS (Method 7): Rₜ = 1.85 min; MS (ESIpos): m/z = 254 [M+H]⁺

### Intermediate 171

### 2-(2,3-difluorophenyl)-2H-1,2,3-triazole-4-carboxylic acid

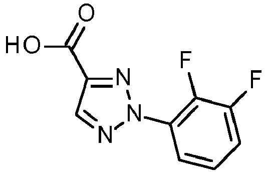

The reaction was carried out analogously to the preparation of 2-(4-methoxyphenyl)-2H-1,2,3-triazole-4-carboxylic acid. 160 mg (100 % purity, 83 % yield) of the title compound were obtained.
LC-MS (Method 7): Rₜ = 1.17 min; MS (ESIneg): m/z = 224 [M-H]⁻

### Intermediate 172

### Ethyl (2E)-2-[2-(2,5-difluorophenyl)hydrazinylidene]-3-oxopropanoate

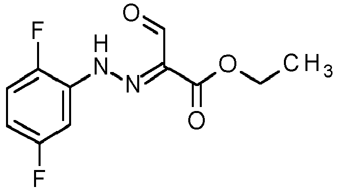

The reaction was carried out analogously to the preparation of ethyl (2E)-2-[(4-methoxyphenyl)hydrazono]-3-oxopropanoate. 947 mg (91 % purity, 87 % yield) of the title compound were obtained.
LC-MS (Method 7): Rₜ = 1.93 min; MS (ESIpos): m/z = 257 [M+H]⁺

### Intermediate 173

### Ethyl (2E,3E)-2-[2-(2,5-difluorophenyl)hydrazinylidene]-3-(hydroxyimino)propanoate

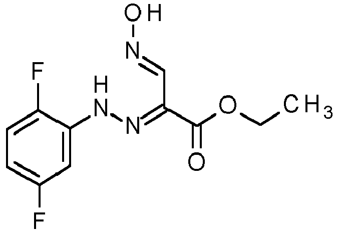

The reaction was carried out analogously to the preparation of ethyl (2E,3E)-3-(hydroxyimino)-2-[(4-methoxyphenyl)hydrazono]propanoate. 794 mg (77 % purity, 67 % yield) of the title compound were obtained.
LC-MS (Method 7): Rₜ = 1.83 min; MS (ESIpos): m/z = 272 [M+H]⁺

### Intermediate 174

### Ethyl 2-(2,5-difluorophenyl)-2H-1,2,3-triazole-4-carboxylate

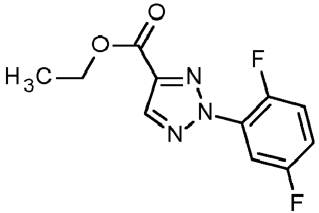

The reaction was carried out analogously to the preparation of ethyl 2-(4-methoxyphenyl)-2H-1,2,3-triazole-4-carboxylate. 411 mg (90 % purity, 65 % yield) of the title compound were obtained.
LC-MS (Method 7): Rₜ = 1.82 min; MS (ESIpos): m/z = 254 [M+H]⁺

### Intermediate 175

### 2-(2,5-difluorophenyl)-2H-1,2,3-triazole-4-carboxylic acid

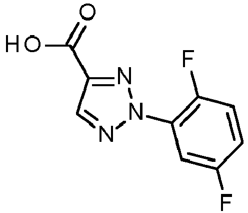

The reaction was carried out analogously to the preparation of 2-(4-methoxyphenyl)-2H-1,2,3-triazole-4-carboxylic acid. 70.0 mg (100 % purity, 21 % yield) of the title compound were obtained.
LC-MS (Method 8): Rₜ = 0.64 min; MS (ESIneg): m/z = 224 [M-H]⁻

### Intermediate 176

### Ethyl (2E)-2-[2-(2-fluorophenyl)hydrazinylidene]-3-oxopropanoate

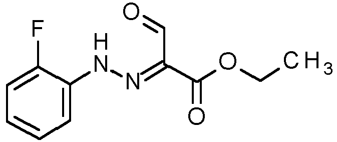

The reaction was carried out analogously to the preparation of ethyl (2E)-2-[(4-methoxyphenyl)hydrazono]-3-oxopropanoate. 1.01 g (84 % purity, 79 % yield) of the title compound were obtained.
LC-MS (Method 7): Rₜ = 1.92 min; MS (ESIpos): m/z = 239 [M+H]⁺

### Intermediate 177

### Ethyl (2E,3E)-2-[2-(2-fluorophenyl)hydrazinylidene]-3-(hydroxyimino)propanoate

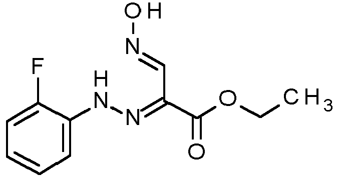

The reaction was carried out analogously to the preparation of ethyl (2E,3E)-3-(hydroxyimino)-2-[(4-methoxyphenyl)hydrazono]propanoate. 1.10 g (81 % purity, 99 % yield) of the title compound were obtained.
LC-MS (Method 7): Rₜ = 1.79 min; MS (ESIpos): m/z = 254 [M+H]⁺

### Intermediate 178

### Ethyl 2-(2-fluorophenyl)-2H-1,2,3-triazole-4-carboxylate

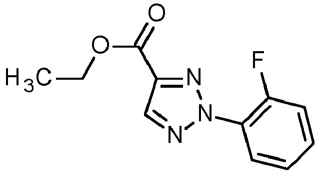

The reaction was carried out analogously to the preparation of ethyl 2-(4-methoxyphenyl)-2H-1,2,3-triazole-4-carboxylate. 594 mg (98 % purity, 71 % yield) of the title compound were obtained.
LC-MS (Method 7): Rₜ = 1.76 min; MS (ESIpos): m/z = 236 [M+H]⁺

### Intermediate 179

### 2-(2-fluorophenyl)-2H-1,2,3-triazole-4-carboxylic acid

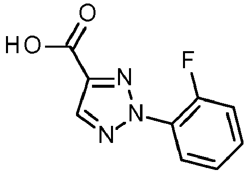

The reaction was carried out analogously to the preparation of 2-(4-methoxyphenyl)-2H-1,2,3-triazole-4-carboxylic acid. 383 mg (98 % purity, 72 % yield) of the title compound were obtained.
LC-MS (Method 7): Rₜ = 1.06 min; MS (ESIpos): m/z = 208 [M+H]⁺

### Intermediate 180

### Ethyl (2E)-2-[2-(3-chloro-4-fluorophenyl)hydrazinylidene]-3-oxopropanoate

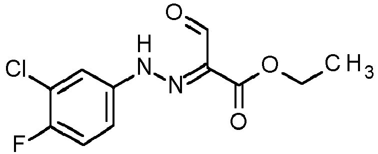

The reaction was carried out analogously to the preparation of ethyl (2E)-2-[(4-methoxyphenyl)hydrazono]-3-oxopropanoate. 883 mg (71 % purity, 66 % yield) of the title compound were obtained.
LC-MS (Method 7): Rₜ = 2.01 min; MS (ESIpos): m/z = 273 [M+H]⁺

### Intermediate 181

### Ethyl (2E,3E)-2-[2-(3-chloro-4-fluorophenyl)hydrazinylidene]-3-(hydroxyimino)propanoate

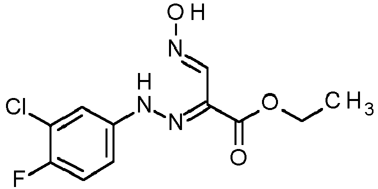

The reaction was carried out analogously to the preparation of ethyl (2E,3E)-3-(hydroxyimino)-2-[(4-methoxyphenyl)hydrazono]propanoate. 852 mg (67 % purity, 87 % yield) of the title compound were obtained.
LC-MS (Method 7): Rₜ = 1.93 min; MS (ESIpos): m/z = 288 [M+H]⁺

### Intermediate 182

### Ethyl 2-(3-chloro-4-fluorophenyl)-2H-1,2,3-triazole-4-carboxylate

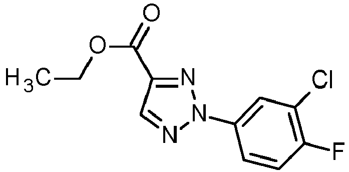

The reaction was carried out analogously to the preparation of ethyl 2-(4-methoxyphenyl)-2H-1,2,3-triazole-4-carboxylate. 287 mg (54 % yield) of the title compound were obtained.
¹H-NMR (400 MHz, DMSO-d6) δ [ppm]: 1.330 (7.67), 1.348 (16.00), 1.366 (7.83), 4.367 (2.45), 4.385 (7.42), 4.403 (7.34), 4.421 (2.37), 7.662 (1.84), 7.684 (3.83), 7.707 (2.10), 8.063 (1.05), 8.070 (1.49), 8.080 (1.24), 8.086 (1.06), 8.093 (1.39), 8.103 (0.95), 8.221 (1.17), 8.227 (1.03), 8.236 (1.22), 8.243 (0.94), 8.643 (4.18).

### Intermediate 183

### 2-(3-chloro-4-fluorophenyl)-2H-1,2,3-triazole-4-carboxylic acid

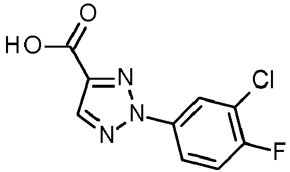

The reaction was carried out analogously to the preparation of 2-(4-methoxyphenyl)-2H-1,2,3-triazole-4-carboxylic acid. 116 mg (100 % purity, 45 % yield) of the title compound were obtained.
LC-MS (Method 7): Rₜ = 1.48 min; MS (ESIpos): m/z = 242 [M+H]⁺

### Intermediate 184

### Ethyl (2E)-2-[2-(4-chloro-3-fluorophenyl)hydrazinylidene]-3-oxopropanoate

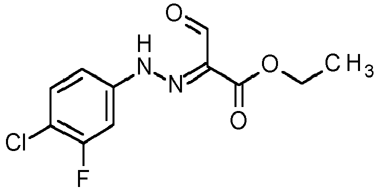

The reaction was carried out analogously to the preparation of ethyl (2E)-2-[(4-methoxyphenyl)hydrazono]-3-oxopropanoate. 846 mg (68 % purity, 61 % yield) of the title compound were obtained.
LC-MS (Method 8): Rₜ = 1.07 min; MS (ESIpos): m/z = 273 [M+H]⁺

### Intermediate 185

### Ethyl (2E,3E)-2-[2-(4-chloro-3-fluorophenyl)hydrazinylidene]-3-(hydroxyimino)propanoate

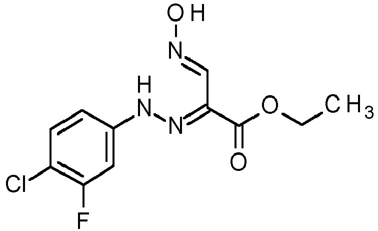

The reaction was carried out analogously to the preparation of ethyl (2E,3E)-3-(hydroxyimino)-2-[(4-methoxyphenyl)hydrazono]propanoate. 801 mg (51 % purity, 68 % yield) of the title compound were obtained.
LC-MS (Method 8): Rₜ = 1.03 min; MS (ESIpos): m/z = 288 [M+H]⁺

### Intermediate 186

### Ethyl 2-(4-chloro-3-fluorophenyl)-2H-1,2,3-triazole-4-carboxylate

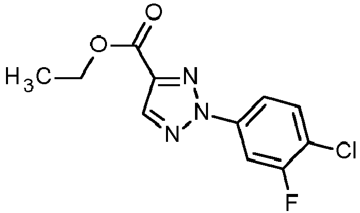

The reaction was carried out analogously to the preparation of ethyl 2-(4-methoxyphenyl)-2H-1,2,3-triazole-4-carboxylate. 278 mg (92 % purity, 68 % yield) of the title compound were obtained.
LC-MS (Method 7): Rₜ = 2.18 min; MS (ESIpos): m/z = 270 [M+H]⁺
¹H-NMR (400 MHz, DMSO-d6) δ [ppm]: -0.008 (1.27), 0.008 (1.09), 1.150 (0.53), 1.290 (0.85), 1.308 (1.64), 1.311 (0.87), 1.331 (7.68), 1.349 (16.00), 1.366 (7.66), 2.524 (0.72), 4.370 (2.51), 4.387 (7.55), 4.405 (7.36), 4.423 (2.31), 7.478 (0.43), 7.499 (0.44), 7.833 (1.53), 7.855 (2.83), 7.875 (2.38), 7.943 (1.82), 7.947 (1.93), 7.965 (1.22), 7.969 (1.28), 8.075 (1.63), 8.081 (1.49), 8.100 (1.69), 8.106 (1.54), 8.663 (4.83), 11.807 (0.43).

### Intermediate 187

### 2-(4-chloro-3-fluorophenyl)-2H-1,2,3-triazole-4-carboxylic acid

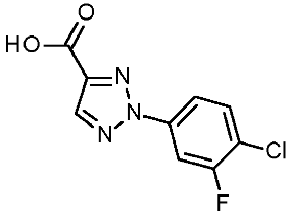

The reaction was carried out analogously to the preparation of 2-(4-methoxyphenyl)-2H-1,2,3-triazole-4-carboxylic acid. 151 mg (100 % purity, 67 % yield) of the title compound were obtained.
LC-MS (Method 7): Rₜ = 1.53 min; MS (ESIneg): m/z = 240 [M-H]⁻

### Intermediate 188

### Ethyl (2E)-2-[2-(2,4-difluorophenyl)hydrazinylidene]-3-oxopropanoate

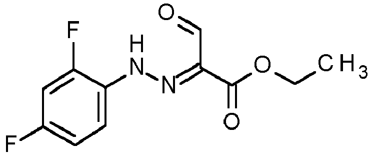

2,4-Difluoroaniline (500 mg, 3.87 mmol) was dissolved in 5 ml of water and 1 ml of concentrated hydrochloric acid. The mixture was cooled to 0°C and a solution of sodium nitrite (267 mg, 3.87 mmol) in 2.5 ml of water was added dropwise. After stirring for 5 minutes this solution was added dropwise to a solution of ethyl-(2E)-3-(dimethylamino)acrylate (610 µl, 4.26 mmol) and potassium acetate (570 mg, 5.81 mmol) in 7.5 ml of ethanol at 0°C. The mixture was stirred at room temperature for 10 minutes before being diluted with ethyl acetate and washed twice with water and once with brine. The organic layer was dried over sodium sulfate, filtered and evaporated. 950 mg (83 % purity, 80 % yield) of the title compound were obtained.
LC-MS (Method 7): Rₜ = 1.93 min; MS (ESIpos): m/z = 257 [M+H]⁺

### Intermediate 189

### Ethyl (2E,3E)-2-[2-(2,4-difluorophenyl)hydrazinylidene]-3-(hydroxyimino)propanoate

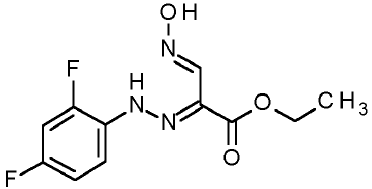

Ethyl (2E)-2-[2-(2,4-difluorophenyl)hydrazinylidene]-3-oxopropanoate (950 mg, 83 % purity, 3.08 mmol), hydroxylamine hydrochloride (256 mg, 3.69 mmol) and potassium acetate (755 mg, 7.69 mmol) were dissolved in 10 ml of ethanol and the mixture was stirred for 30 minutes at 80°C. The reaction mixture was diluted with ethyl acetate and washed twice with water and once with brine. The organic layer was dried over sodium sulfate, filtered and evaporated. The residue was dried in vacuum. 950 mg (80 % purity, 92 % yield) of the title compound were obtained.
LC-MS (Method 7): Rₜ = 1.82 min; MS (ESIpos): m/z = 272 [M+H]⁺

### Intermediate 190

### Ethyl 2-(2,4-difluorophenyl)-2H-1,2,3-triazole-4-carboxylate

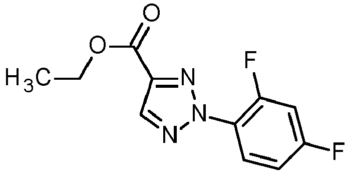

Ethyl (2E,3E)-2-[2-(2,4-difluorophenyl)hydrazinylidene]-3-(hydroxyimino)propanoate (950 mg, 2.80 mmol) were stirred for 30 minutes in 11.5 ml of acetic acid anhydride at 140°C. The reaction was then treated with water and extracted with dichlormethane. The organic layer was dried over sodium sulfate, filtered and evaporated. The residue was put on Isolute and purified by chromatography on silica gel (50 g Ultra Snap Cartridge Biotage®; Biotage-Isolera-One®; Cy/EE-gradient: 5% EE - 40% EE; flow: 100 ml/min). Samples containing the desired product were united, the solvents were evaporated and the residue was dried in vacuum. 605 mg (85 % yield) of the title compound were obtained.
¹H-NMR (400 MHz, DMSO-d6) δ [ppm]: 1.297 (0.53), 1.300 (0.44), 1.315 (1.20), 1.318 (1.17), 1.324 (7.55), 1.333 (0.99), 1.342 (16.00), 1.360 (7.80), 2.025 (0.44), 2.223 (0.83), 2.226 (0.56), 4.292 (0.45), 4.310 (0.45), 4.363 (2.45), 4.381 (7.52), 4.399 (7.41), 4.417 (2.35), 7.340 (0.57), 7.343 (0.68), 7.346 (0.75), 7.350 (0.68), 7.363 (1.28), 7.366 (1.45), 7.369 (1.36), 7.382 (0.67), 7.386 (0.76), 7.389 (0.82), 7.392 (0.72), 7.661 (0.77), 7.668 (0.77), 7.684 (0.88), 7.691 (1.47), 7.696 (0.92), 7.712 (0.81), 7.719 (0.82), 7.946 (1.01), 7.961 (1.10), 7.968 (1.88), 7.983 (1.89), 7.991 (1.03), 8.005 (0.95), 8.647 (4.18), 9.860 (0.43).

### Intermediate 191

### 2-(2,4-difluorophenyl)-2H-1,2,3-triazole-4-carboxylic acid

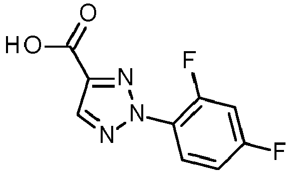

Ethyl 2-(2,4-difluorophenyl)-2H-1,2,3-triazole-4-carboxylate (605 mg, 2.39 mmol) were dissolved in 20 ml of THF, treated with lithium hydroxide solution (2.39 ml, 1.0 M, 2.39 mmol) and the mixture was stirred at room temperature for 2 h. The reaction was then acidified with 2 N hydrochloric acid and put on Isolute before being purified by chromatography on silica gel (25 g Ultra Snap Cartridge Biotage®; Biotage-Isolera-One®; DCM/MeOH-gradient: 2% MeOH - 20% MeOH; flow: 75 ml/min). Samples containing the desired product were united and the solvents were evaporated. The residue was taken up with DCM, the precipitate was filtered off, washed with DCM and dried in vacuum. 211 mg (100 % purity, 39 % yield) of the title compound were obtained.
LC-MS (Method 7): Rₜ = 1.15 min; MS (ESIneg): m/z = 224 [M-H]⁻

### Intermediate 192

### Ethyl (2E)-2-[2-(3,4-dichlorophenyl)hydrazinylidene]-3-oxopropanoate

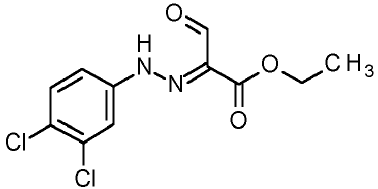

The reaction was carried out analogously to the preparation of ethyl (2E)-2-[(4-methoxyphenyl)hydrazono]-3-oxopropanoate. 570 mg (71 % purity, 45 % yield) of the title compound were obtained.
LC-MS (Method 8): Rₜ = 1.13 min; MS (ESIpos): m/z = 289 [M+H]⁺

### Intermediate 193

### Ethyl (2E,3E)-2-[2-(3,4-dichlorophenyl)hydrazinylidene]-3-(hydroxyimino)propanoate

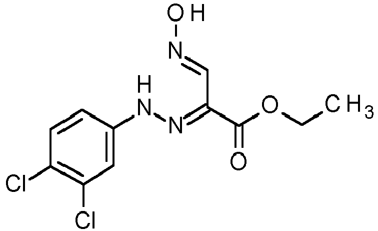

The reaction was carried out analogously to the preparation of ethyl (2E,3E)-3-(hydroxyimino)-2-[(4-methoxyphenyl)hydrazono]propanoate. 429 mg (100 % purity, 101 % yield) of the title compound were obtained.
LC-MS (Method 7): Rₜ = 2.07 min; MS (ESIpos): m/z = 304 [M+H]⁺

### Intermediate 194

### Ethyl 2-(3,4-dichlorophenyl)-2H-1,2,3-triazole-4-carboxylate

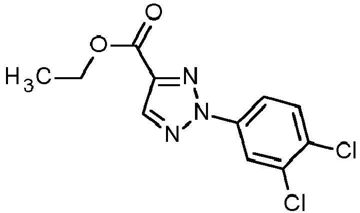

The reaction was carried out analogously to the preparation of ethyl 2-(4-methoxyphenyl)-2H-1,2,3-triazole-4-carboxylate. 140 mg (92 % purity, 32 % yield) of the title compound were obtained.
LC-MS (Method 7): Rₜ = 2.34 min; MS (ESIpos): m/z = 286 [M+H]⁺

### Intermediate 195

### 2-(3,4-dichlorophenyl)-2H-1,2,3-triazole-4-carboxylic acid

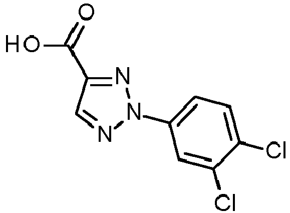

The reaction was carried out analogously to the preparation of 2-(4-methoxyphenyl)-2H-1,2,3-triazole-4-carboxylic acid. 102 mg (100 % purity, 88 % yield) of the title compound were obtained.
LC-MS (Method 7): Rₜ = 1.69 min; MS (ESIneg): m/z = 255 [M-H]⁻

### Intermediate 196

### Ethyl (2E)-2-[2-(4-bromophenyl)hydrazinylidene]-3-oxopropanoate

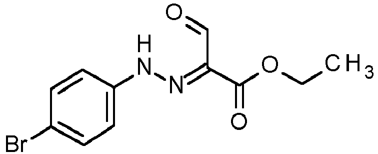

The reaction was carried out analogously to the preparation of ethyl (2E)-2-[(4-methoxyphenyl)hydrazono]-3-oxopropanoate. 844 mg (85 % purity, 83 % yield) of the title compound were obtained.
LC-MS (Method 9): Rₜ = 1.78 min; MS (ESIpos): m/z = 299 [M+H]⁺

### Intermediate 197

### Ethyl (2E,3E)-2-[2-(4-bromophenyl)hydrazinylidene]-3-(hydroxyimino)propanoate

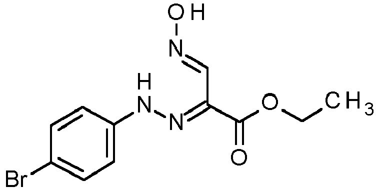

The reaction was carried out analogously to the preparation of ethyl (2E,3E)-3-(hydroxyimino)-2-[(4-methoxyphenyl)hydrazono]propanoate. 873 mg (41 % purity, 48 % yield) of the title compound were obtained.
LC-MS (Method 7): Rₜ = 1.95 min; MS (ESIpos): m/z = 314 [M+H]⁺

### Intermediate 198

### Ethyl 2-(4-bromophenyl)-2H-1,2,3-triazole-4-carboxylate

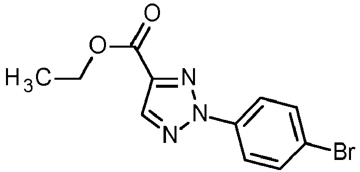

The reaction was carried out analogously to the preparation of ethyl 2-(4-methoxyphenyl)-2H-1,2,3-triazole-4-carboxylate. 270 mg (92 % purity, 72 % yield) of the title compound were obtained.
LC-MS (Method 7): Rₜ = 2.18 min; MS (ESIpos): m/z = 296 [M+H]⁺

### Intermediate 199

### 2-(4-bromophenyl)-2H-1,2,3-triazole-4-carboxylic acid

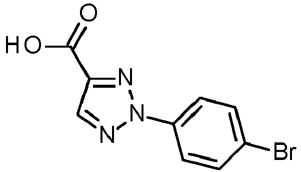

The reaction was carried out analogously to the preparation of 2-(4-methoxyphenyl)-2H-1,2,3-triazole-4-carboxylic acid. 162 mg (100 % purity, 73 % yield) of the title compound were obtained.
LC-MS (Method 7): Rₜ = 1.53 min; MS (ESIneg): m/z = 265 [M-H]⁻

### Intermediate 200

### tert-butyl {[(4R)-4-cyclopropyl-2,5-dioxoimidazolidin-4-yl]methyl}carbamate

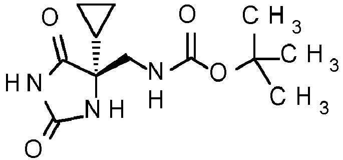

*rac*-tert-Butyl [(4-cyclopropyl-2,5-dioxoimidazolidin-4-yl)methyl]carbamate (26 g) was dissolved in 2600 ml of Methanol at 50°C, filtered and separated into it's enantiomers by chiral chromatography (Machine: SFC Prep Sepiatec 360; Column: Chiralpak AD 20µ, 450 x 50 mm; Eluent: CO₂/ Methanol 70:30; Flow: 350 ml/min; UV Detection: 210 nm; Backpressure: 130 bar; Oven temperature: 35°C). The enantiomer eluting at 1.52 minutes was collected. Enantiomeric purity was determined on an analytical scale (Column: Chiralpak AD-H 5µ, 250 x 4.6 mm; Eluent: CO₂/ Methanol 80:20; Flow: 3 ml/min; UV Detection: 210 nm) as 99.9% ee. 8.6 g (33% yield).
LC-MS (Method 8): Rₜ = 0.60 min; MS (ESIpos): m/z = 270 [M+H]⁺

The absolute stereochemistry was deduced by deprotection (see next intermediate) and synthesis of example 3 from WO2014066151.

### Intermediate 201

### (5R)-5-(aminomethyl)-5-cyclopropylimidazolidine-2,4-dione hydrochloride

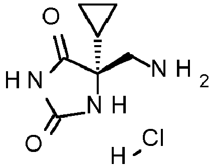

tert-butyl {[(4R)-4-cyclopropyl-2,5-dioxoimidazolidin-4-yl]methyl}carbamate (6 g, 22.28 mmol) was dissolved in 70 ml of dichloromethane and treated with 4N HCl in dioxane (27.85 ml, 111.4 mmol). The mixture was stirred at room temperature over night. The precipitate was filtered off, washed with dichloromethane and dried in vacuo. 4.29 g (94% yield, 100% purity) of the title compound were obtained.
LC-MS (Method 9): Rₜ = 0.27 min; MS (ESIpos): m/z = 170 [M-HCl+H]⁺

Specific optical rotation: -75.92° (Solvent: methanol; Pathlength: 100 mm; Concentration: 0.2665 g/100 cm³; Temperature: 20°C; Wavelength: 589 nm)

### Intermediate 202

### Ethyl 5-(5-methyl-1,3-thiazol-4-yl)-1,3-oxazole-4-carboxylate

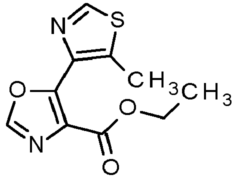

5-methyl-1,3-thiazole-4-carboxylic acid (940 mg, 6.57 mmol) dissolved in 10 ml of THF was treated with 1,1,-carbonyl-diimidazole (1.28 g, 7.88 mmol) and stirred at room temperature. After 2 h ethyl isocyanoacetate (790 µl, 7.2 mmol), dissolved in 10 ml of THF, and a solution of lithium bis(trimethylsilyl)amide in THF (6.6 ml, 1.0 M, 6.6 mmol) were added at 0°C. The mixture was allowed to warm to room temperature and stirred over night. The solvent was removed on a rotary evaporator. The residue was taken up with ethyl acetate and washed with water and saturated aqueous sodium chloride solution. The organic layer was dried over sodium sulfate, filtered, Isolute® was added and the solvent was evaporated on a rotary evaporator. The crude product was purified by column chromatography (Machine: Biotage® Isolera One; column: Biotage® SNAP Ultra 50 g; gradient: Cy/EE-gradient, 16% EE - 100% EE; flow: 100 ml/min). Product containing samples were united and the solvents were removed on a rotary evaporator. 1.13 g (100 % purity, 72 % yield) of the title compound were obtained.
LC-MS (Method 7): Rₜ = 1.15 min; MS (ESIpos): m/z = 239 [M+H]⁺

### Intermediate 203

### 2-amino-1-(5-methyl-1,3-thiazol-4-yl)ethan-1-one-hydrogen chloride (1/1)

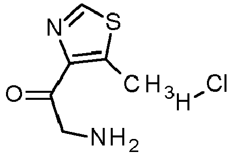

Ethyl 5-(5-methyl-1,3-thiazol-4-yl)-1,3-oxazole-4-carboxylate (1.13 g, 4.74 mmol) was taken up in 25 ml of 6 N hydrochloric acid and stirred at 100°C. After 2 h the solvent was removed on a rotary evaporator and the residue was treated with DCM/MeOH 20:1. The precipitate was filtered off, washed with DCM/MeOH 20:1 and dried in vacuo. 730 mg (98 % purity, 79 % yield) of the title compound were obtained.
LC-MS (Method 9): Rₜ = 0.80 min; MS (ESIpos): m/z = 157 [M-HCl+H]⁺

### Intermediate 204

### tert-butyl [2-(5-methyl-1,3-thiazol-4-yl)-2-oxoethyl]carbamate

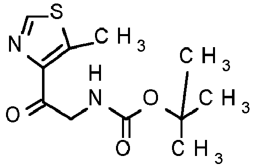

2-amino-1-(5-methyl-1,3-thiazol-4-yl)ethan-1-one-hydrogen chloride (730 mg, 3.79 mmol) dissolved in 15 ml of dichloromethane was treated with di-tert-butyl dicarbonate (960 µl, 4.2 mmol) and triethylamine (1.6 ml, 11 mmol). The mixture was stirred at room temperature. After 2 h the solvent was removed on a rotary evaporator. The residue was taken up with ethyl acetate and washed with water and saturated aqueous sodium chloride solution. The organic layer was dried over sodium sulfate, filtered and evaporated on a rotary evaporator. 980 mg (100 % purity, 101 % yield) of the title compound were obtained.
LC-MS (Method 7): Rₜ = 1.57 min; MS (ESIpos): m/z = 257 [M+H]⁺

### Intermediate 205

### rac-tert-butyl {[4-(5-methyl-1,3-thiazol-4-yl)-2,5-dioxoimidazolidin-4-yl]methyl}carbamate

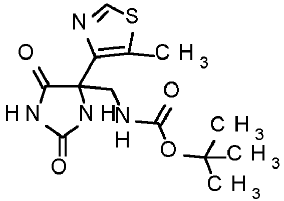

In a microwave vial tert-butyl [2-(5-methyl-1,3-thiazol-4-yl)-2-oxoethyl]carbamate (980 mg, 3.82 mmol) was dissolved in 7 ml of methanol. Potassium cyanide (996 mg, 15.3 mmol) and ammonium carbonate (1.47 g, 15.3 mmol) were added. The vial was sealed and the mixture was stirred at 40°C for 48 h. Further potassium cyanide (996 mg, 15.3 mmol) and ammonium carbonate (1.47 g, 15.3 mmol) were added and the mixture was stirred for 72 h. Isolute® was added and the solvent was evaporated on a rotary evaporator. The crude product was purified by column chromatography (Machine: Biotage® Isolera One; column: Biotage® SNAP Ultra 50 g; gradient: DCM/MeOH-gradient, 2% MeOH - 20% MeOH; flow: 100 ml/min). Product containing samples were united and the solvents were removed on a rotary evaporator. 766 mg (100 % purity, 61 % yield) of the title compound were obtained.
LC-MS (Method 7): Rₜ = 1.09 min; MS (ESIneg): m/z = 325 [M-H]⁻

### Intermediate 206

### rac-5-(aminomethyl)-5-(5-methyl-1,3-thiazol-4-yl)imidazolidine-2,4-dione hydrochloride

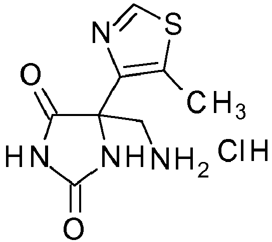

rac-tert-butyl {[4-(5-methyl-1,3-thiazol-4-yl)-2,5-dioxoimidazolidin-4-yl]methyl}carbamate (766 mg, 2.35 mmol) was dissolved in 9 ml of dichloromehane. 4 M hydrochloride acid in 1,4-dioxane (2.9 ml, 4.0 M, 12 mmol) was added and the mixture was stirred for 30 min. The precipitate was filtered off, washed with dichloromethane and dried in vacuo. 640 mg (86 % purity, 89 % yield) of the title compound were obtained.
LC-MS (Method 9): Rₜ = 0.25 min; MS (ESIpos): m/z = 227 [M-HCl+H]⁺

### Intermediate 207

### 3-fluoro-2-hydrazinylpyridine

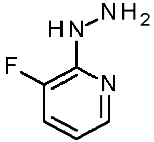

2,3-difluoropyridine (1.00 g, 8.69 mmol) were dissolved in hydrazine hydrate (4.2 ml, 87 mmol). The mixture was stirred at 120°C for 1 h. The mixture was extracted with dichloromethane, the organic layer was dried over sodium sulfate, filtered and evaporated on a rotary evaporator. 1.03 g (93 % yield) of the title compound were obtained.

### Intermediate 208

### N-{(1E,2Z)-2-[2-(3-fluoropyridin-2-yl)hydrazinylidene]propylidene}hydroxylamine

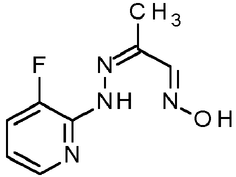

3-fluoro-2-hydrazinylpyridine (1.03 g, 8.10 mmol) was dissolved in 35 ml of ethanol. (1E)-1-(hydroxyimino)propan-2-one (847 mg, 9.72 mmol) was added and the mixture was stirred at 80°C. After 2 h the solvent was removed on a rotary evaporator. The residue was taken up with pentane. The precipitate was filtered off, washed with pentane and dried in vacuo. 1.64 g (103 % yield) of the title compound were obtained.

### Intermediate 209

### 3-fluoro-2-(4-methyl-2H-1,2,3-triazol-2-yl)pyridine

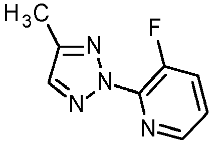

N-{(1E,2Z)-2-[2-(3-fluoropyridin-2-yl)hydrazinylidene]propylidene}hydroxylamine (1.64 g, 8.36 mmol) was dissolved in 30 ml of acetic anhydride and stirred at 130°C. After 3 h Isolute® was added and the solvent was evaporated on a rotary evaporator. The crude product was purified by column chromatography (Machine: Biotage® Isolera One; column: Biotage® SNAP Ultra 50 g; gradient: Cy/EE-gradient, 12% EE - 100% EE; flow: 100 ml/min). Product containing samples were united and the solvents were removed on a rotary evaporator. 844 mg (79 % purity, 45 % yield) of the title compound were obtained.
LC-MS (Method 7): Rₜ = 1.03 min; MS (ESIpos): m/z = 179 [M+H]⁺
¹H-NMR (400 MHz, DMSO-d6) δ [ppm]: 2.387 (16.00), 2.509 (1.26), 3.327 (2.56), 7.653 (0.56), 7.664 (0.93), 7.674 (1.21), 7.685 (1.05), 7.694 (0.65), 7.994 (2.43), 8.071 (0.82), 8.094 (1.10), 8.097 (0.95), 8.118 (0.78), 8.450 (1.33), 8.461 (1.32).

### Intermediate 210

### 2-(3-fluoropyridin-2-yl)-2H-1,2,3-triazole-4-carboxylic acid

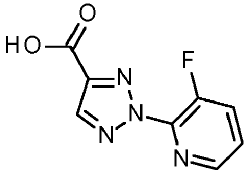

3-fluoro-2-(4-methyl-2H-1,2,3-triazol-2-yl)pyridine (740 mg, 4.15 mmol) was suspended in 45 ml of water. Potassium permanganate (1.31 g, 8.31 mmol) were added and the mixture was stirred at 100°C. After 24 h more potassium permanganate (500 mg, 3.16 mmol) were added and the mixture was stirred at 100°C for 24 h. Once again more potassium permanganate (500 mg, 3.16 mmol) was added and the mixture was stirred for another 72 h at 100°C. The mixture was filtered over Celite® and the filtrate was acidified with 2 N hydrochloric acid. The solvent was removed on a rotary evaporator. Dichloromethane / methanol 10:1 were added to the crude product and the precipitate was filtered off. The filtrate was purified by preparative HPLC (Column: Chromatorex C18 10 µm, 250 x 30 mm, eluent A=water, B=acetonitrile; gradient: 0.0 min 5% B; 3 min 5% B; 20 min 50% B; 23 min 100% B; 26 min 5% B; flow: 50 ml/min; 0.1% formic acid). 149 mg (100 % purity, 17 % yield) of the title compound were obtained.
LC-MS (Method 7): Rₜ = 0.63 min; MS (ESIpos): m/z = 209 [M+H]⁺

### Intermediate 211

### rac-Ethyl 5-sec-butyl-1,3-oxazole-4-carboxylate

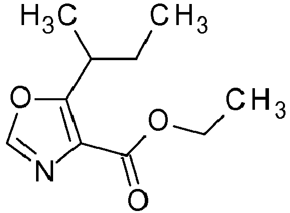

A solution of rac-2-methylbutanoic acid (1.1 ml, 9.8 mmol) and 1,1'-carbonyldiimidazole (1.91 g, 11.7 mmol) in 10 ml of THF was stirred for 3 h at room temperature. After that time, a solution of ethyl isocyanoacetate (1.2 ml, 11 mmol) in 10 ml of THF and a solution of lithium bis(trimethylsilyl)amide in THF (9.8 ml, 1.0 M, 9.8 mmol) were added dropwise at 0°C. When the addition was complete the resulting mixture was stirred at room temperature for 3 h. The reaction was concentrated in vacuo and extracted with ethyl acetate. The combined organic layers were dried over sodium sulfate, filtered and concentrated. The crude product was purified by column chromatography (Machine: Biotage® Isolera One; column: Biotage® SNAP Ultra 50 g; gradient: Cy/EE-gradient, 8% EE - 66% EE; flow: 100 ml/min). Product containing samples were united and the solvents were removed on a rotary evaporator. 446 mg (97 % purity, 22 % yield) of the title compound were obtained.
LC-MS (Method 7): Rₜ = 1.70 min; MS (ESIpos): m/z = 198 [M+H]⁺

### Intermediate 212

### rac-1-amino-3-methylpentan-2-one hydrochloride

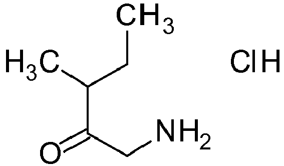

*rac*-ethyl 5-sec-butyl-1,3-oxazole-4-carboxylate (446 mg, 2.26 mmol) were taken up in 7.5 ml of 6 N hydrochloric acid and stirred at 100°C over night. The solvent was removed on a rotary evaporator and the residue was dried in vacuo. 372 mg (108 % yield) of the title compound were obtained.

### Intermediate 213

### rac-tert-butyl (3-methyl-2-oxopentyl)carbamate

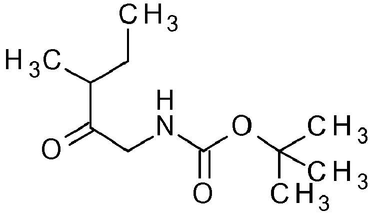

*rac*-1-amino-3-methylpentan-2-one hydrochloride (372 mg, 2.45 mmol) dissolved in 15 ml of dichloromethane were treated with di-tert-butyl dicarbonate (620 µl, 2.7 mmol) and triethylamine (1.0 ml, 7.4 mmol). The mixture was stirred at room temperature over night. The solvent was removed on a rotary evaporator. The residue was taken up with ethyl acetate and washed with water and saturated aqueous sodium chloride solution. The organic layer was dried over sodium sulfate, filtered and evaporated on a rotary evaporator. 480 mg (100 % purity, 91 % yield) of the title compound were obtained.
¹H-NMR (400 MHz, DMSO-d6) δ [ppm]: 0.786 (2.10), 0.804 (4.90), 0.823 (2.39), 0.958 (3.46), 0.975 (3.56), 1.290 (0.48), 1.308 (0.75), 1.324 (1.46), 1.343 (0.69), 1.366 (2.11), 1.377 (16.00), 1.468 (1.14), 1.532 (0.41), 1.550 (0.50), 1.567 (0.50), 1.584 (0.41), 2.519 (0.94), 3.778 (0.94), 3.789 (1.12), 3.793 (1.06), 3.804 (0.89), 6.977 (0.42).

### Intermediate 214

### diamix-tert-butyl [(4-sec-butyl-2,5-dioxoimidazolidin-4-yl)methyl]carbamate

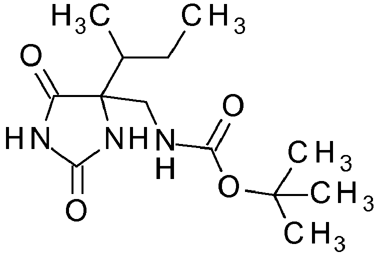

In a microwave vial rac-tert-butyl (3-methyl-2-oxopentyl)carbamate (480 mg, 2.23 mmol) was dissolved in 4 ml of methanol. Potassium cyanide (581 mg, 8.92 mmol) and ammonium carbonate (857 mg, 8.92 mmol) were added. The vial was sealed and the mixture was stirred at 40°C over night. The mixture was filtered an the filtrate was purified by preparative HPLC (Column: Chromatorex C18 10 µm 250 x 30 mm; eluent A=water, B=acetonitrile; gradient: 0.0 min 30% B; 4.5 min 50% B; 11.5 min 70% B; 12 min 100% B; 14.75 min 30% B; flow: 50 ml/min; 0.1% formic acid). Product containing samples were united and the solvents were removed on a rotary evaporator. 237 mg (100 % purity, 37 % yield) of the title compound were obtained.
LC-MS (Method 7): Rₜ = 1.27 min; MS (ESIneg): m/z = 284 [M-H]⁻
LC-MS (Method 8): Rₜ = 0.74 min; MS (ESIneg): m/z = 284 [M-H]⁻

### Intermediate 215

### diamix-5-(aminomethyl)-5-sec-butylimidazolidine-2,4-dione hydrochloride

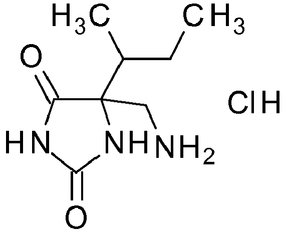

*diamix*-tert-butyl [(4-sec-butyl-2,5-dioxoimidazolidin-4-yl)methyl]carbamate (235 mg, 824 µmol) was dissolved in 3 ml of dichloromethane. 4 M hydrochloric acid in 1,4-dioxane (1.0 ml, 4.0 M, 4.1 mmol) was added and the mixture was stirred for 4 h. The solvent was removed on a rotary evaporator. The residue was taken up in dichloromethane, the precipitate was filtered off, washed with dichloromethane and dried in vacuo. 155 mg (100 % purity, 85 % yield) of the title compound were obtained.
LC-MS (Method 9): Rₜ = 0.36 min; MS (ESIpos): m/z = 186 [M-HCl+H]⁺

### Intermediate 216

### Ethyl 2-(1,3-benzothiazol-2-yl)-2H-1,2,3-triazole-4-carboxylate

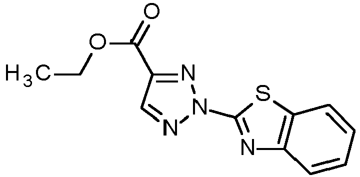

To a solution of ethyl 2H-1,2,3-triazole-4-carboxylate (165 mg, 1.17 mmol) and 2-bromo-1,3-benzothiazole (250 mg, 1.17 mmol) in 5 ml of DMF sodium hydrogen carbonate (196 mg, 2.34 mmol) was added and the mixture was stirred for 72 h at 60°C. The precipitate was filtered off and the filtrate was purified by preparative HPLC (Column: Chromatorex C18 10 µm 250 x 30 mm; eluent A=water, B=acetonitrile; gradient: 0.0 min 30% B; 4.5 min 50% B; 11.5 min 70% B; 12 min 100% B; 14.75 min 30% B; flow: 50 ml/min; 0.1% formic acid). Product containing samples were united and the solvents were removed on a rotary evaporator. 46.8 mg (100 % purity, 15 % yield) of the title compound were obtained.
LC-MS (Method 7): Rₜ = 1.91 min; MS (ESIpos): m/z = 275 [M+H]⁺
¹H-NMR (600 MHz, DMSO-d6) δ [ppm]: 1.361 (7.74), 1.372 (16.00), 1.384 (7.83), 4.407 (2.50), 4.419 (7.64), 4.431 (7.48), 4.443 (2.38), 7.546 (1.58), 7.560 (3.38), 7.572 (2.30), 7.617 (2.09), 7.618 (2.00), 7.630 (3.35), 7.642 (1.63), 8.079 (3.55), 8.093 (3.41), 8.210 (3.37), 8.224 (3.28), 8.798 (9.95).

### Intermediate 217

### 2-(1,3-benzothiazol-2-yl)-2H-1,2,3-triazole-4-carboxylic acid

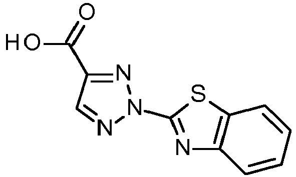

Ethyl 2-(1,3-benzothiazol-2-yl)-2H-1,2,3-triazole-4-carboxylate (46.0 mg, 168 µmol) was dissolved in 2 ml of THF, treated with lithium hydroxide solution (200 µl, 1.0 M, 200 µmol) and the mixture was stirred for 2 h at room temperature. The solvent was removed on a rotary evaporator. Water was added and the mixture was acidified with 2 N hydrochloric acid. The precipitate was filtered off, washed with water an dried in vacuo. 36.0 mg (100 % purity, 87 % yield) of the title compound were obtained.
LC-MS (Method 8): Rₜ = 0.75 min; MS (ESIpos): m/z = 247 [M+H]⁺

### Intermediate 218

### Ethyl (2E)-3-oxo-2-{2-[4-(trifluoromethoxy)phenyl]hydrazinylidene}propanoate

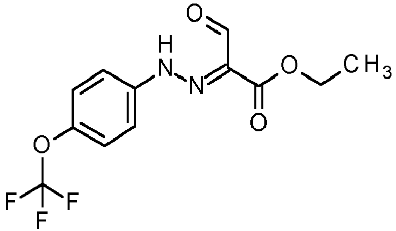

4-(trifluoromethoxy)aniline (250 mg, 1.41 mmol) was dissolved in 2.5 ml of water and 380 µl of concentrated hydrochloric acid. The mixture was cooled to 0°C and a solution of sodium nitrite (97.4 mg, 1.41 mmol) in 1.25 ml of water was added dropwise. After stirring for 5 minutes this solution was added dropwise to a solution of ethyl-(2E)-3-(dimethylamino)acrylate (220 µl, 1.6 mmol) and potassium acetate (208 mg, 2.12 mmol) in 3.6 ml of ethanol at 0°C. The mixture was stirred at room temperature for 30 min before being diluted with ethyl acetate and washed twice with water and once with brine. The organic layer was dried over sodium sulfate, filtered and evaporated. 394 mg (92 % yield) of the title compound were obtained.

### Intermediate 219

### Ethyl (2E,3E)-3-(hydroxyimino)-2-{2-[4-(trifluoromethoxy)phenyl]hydrazinylidene}propanoate

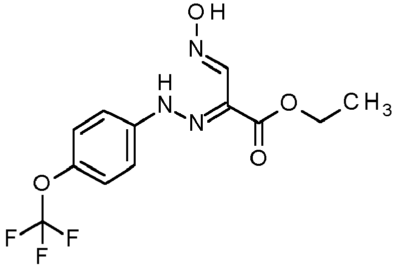

Ethyl (2E)-3-oxo-2-{2-[4-(trifluoromethoxy)phenyl]hydrazinylidene}propanoate (393 mg, 1.29 mmol), hydroxylamine hydrochloride (108 mg, 1.55 mmol) and potassium acetate (317 mg, 3.23 mmol) were dissolved in 7 ml of ethanol and the mixture was stirred for 3 h at room temperature. The reaction mixture was diluted with ethyl acetate and washed twice with water and once with brine. The organic layer was dried over sodium sulfate, filtered and evaporated. The residue was dried in vacuum. 350 mg (39 % purity, 33 % yield) of the title compound were obtained.
LC-MS (Method 7): Rₜ = 2.02 min; MS (ESIpos): m/z = 320 [M+H]⁺

### Intermediate 220

### Ethyl 2-[4-(trifluoromethoxy)phenyl]-2H-1,2,3-triazole-4-carboxylate

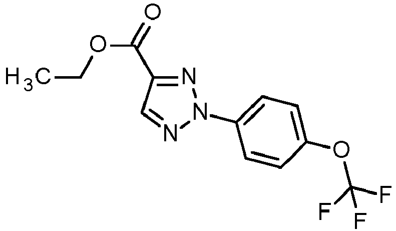

Ethyl (2E,3E)-3-(hydroxyimino)-2-{2-[4-(trifluoromethoxy)phenyl]hydrazinylidene}propanoate (350 mg, 1.10 mmol) was stirred for 1.5 h in 5 ml of acetic acid anhydride at 60°C. The reaction was then diluted in ethyl acetate and washed twice with water and once with brine. The organic layer was dried over sodium sulfate, filtered and evaporated. The residue was put on Isolute® and purified by chromatography on silica gel (25 g Ultra Snap Cartridge Biotage®; Biotage-Isolera-One®; Cy/EE-gradient: 12% EE - 100% EE; flow: 75 ml/min). Samples containing the desired product were united, the solvents were evaporated and the residue was dried in vacuum. 95.6 mg (58 % purity, 17 % yield) of the title compound were obtained.
LC-MS (Method 7): Rₜ = 2.25 min; MS (ESIpos): m/z = 302 [M+H]⁺

### Intermediate 221

### 2-[4-(trifluoromethoxy)phenyl]-2H-1,2,3-triazole-4-carboxylic acid

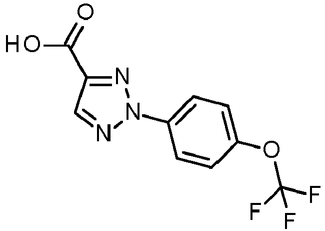

Ethyl 2-[4-(trifluoromethoxy)phenyl]-2H-1,2,3-triazole-4-carboxylate (95.0 mg, 315 µmol) was dissolved in 2.5 ml of THF, treated with lithium hydroxide solution (320 µl, 1.0 M, 320 µmol) and the mixture was stirred for 1.5 h at room temperature. The solvent was removed on a rotary evaporator. Water was added an the mixture was acidified with 2 N hydrochloric acid. The precipitate was filtered off, washed with water an dried in vacuo. 14.7 mg (69 % purity, 12 % yield) of the title compound were obtained
LC-MS (Method 7): Rₜ = 1.69 min; MS (ESIneg): m/z = 272 [M-H]⁻

### Intermediate 222

### rac-tert-butyl [(4-cyclobutyl-2,5-dioxoimidazolidin-4-yl)methyl]carbamate

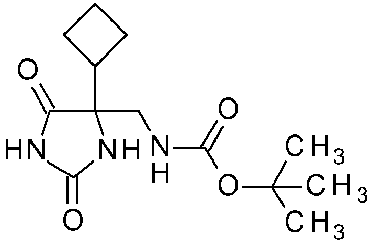

*rac*-5-(aminomethyl)-5-cyclobutylimidazolidine-2,4-dione-hydrogen chloride (1.00 g, 4.55 mmol) was dissolved in 10 ml of THF and cooled at 0°C. At this temperature triethylamine (1.9 ml, 14 mmol), 4-(dimethylamino)pyridine (83.4 mg, 683 µmol) and di-tert-butyl dicarbonate (1.04 g, 4.78 mmol) were added and the mixture was stirred over night at room temperature. The reaction was then diluted in ethyl acetate and washed twice with water and once with brine. The organic layer was dried over sodium sulfate, filtered and evaporated. The residue was put on Isolute® and purified by chromatography on silica gel (25 g Ultra Snap Cartridge Biotage®; Biotage-Isolera-One®; DCM/MeOH-gradient: 2% MeOH - 20% MeOH; flow: 75 ml/min). Samples containing the desired product were united, the solvents were evaporated and the residue was dried in vacuum. 247 mg (100 % purity, 19 % yield) of the title compound were obtained.
LC-MS (Method 9): Rₜ = 0.91 min; MS (ESIneg): m/z = 282 [M-H]⁻

### Intermediate 223

### ent-tert-butyl [(4-cyclobutyl-2,5-dioxoimidazolidin-4-yl)methyl]carbamate

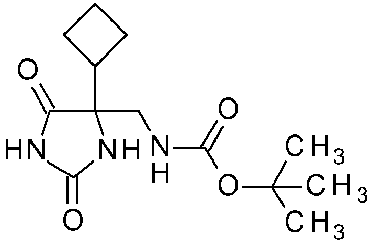

Enantiomeric separation of rac-tert-butyl [(4-cyclobutyl-2,5-dioxoimidazolidin-4-yl)methyl]carbamate (245 mg, 865 µmol) using the following method
- Column: Maisch Diacel AD-H 5 µm 250^{∗}25 mm
- Eluent A: 80% CO₂, eluent B: 20% methanol
- Flow: 80 ml/min
- UV-detection: 210 nm
- Temperature: 40°C
afforded 92.9 mg (100 % purity, 38 % yield) of the desired product.
Chiral-HPLC (Method Info: AD-15 MeOH, 3.0 ml/min, 210 nm, 10 min, 5µl): Rt= 1.87 min; 98 %ee
LC-MS (Method 7): Rₜ = 1.25 min; MS (ESIneg): m/z = 282 [M-H]⁻
¹H-NMR (400 MHz, DMSO-d6) δ [ppm]: -0.008 (0.60), 0.008 (0.54), 1.364 (16.00), 1.867 (0.41), 3.085 (0.49), 7.724 (0.80), 10.536 (0.51).

### Intermediate 224

### ent-5-(aminomethyl)-5-cyclobutylimidazolidine-2,4-dione hydrochloride

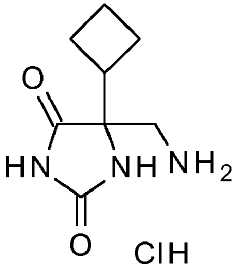

*ent*-tert-butyl [(4-cyclobutyl-2,5-dioxoimidazolidin-4-yl)methyl]carbamate (92.0 mg, 325 µmol) was dissolved in 1.5 ml of dichloromehane. 4 M hydrochloric acid in 1,4-dioxane (410 µl, 4.0 M, 1.6 mmol) was added and the mixture was stirred over night at room temperature. The solvent was removed on a rotary evaporator and the residue was dried in vacuo. 73.0 mg (100 % purity, 102 % yield) of the title compound were obtained.
LC-MS (Method 9): Rₜ = 0.36 min; MS (ESIpos): m/z = 184 [M-HCl+H]⁺

### Intermediate 225

### tert-Butyl (R)-(1-cyclopropyl-1-oxopropan-2-yl)carbamate

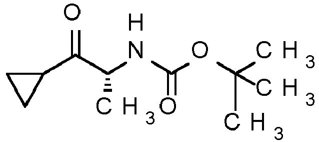

A solution of isopropylmagnesium chloride (3.40 mL, 2.0 M in THF) was added dropwise to a solution of (R)-*tert*-butyl (1-(methoxy(methyl)amino)-1-oxopropan-2-yl)carbamate (1.50 g, 6.46 mmol) in dry THF (25 mL) at 0 °C under inert atmosphere. After 45 minutes, a solution of cyclopropylmagnesium bromide (15.4 mL, 0.5 M in THF) was added and the reacting mixture was stirred at room temperature for 72 hours. The reaction mixture was quenched by addition of a saturated aqueous ammonium chloride solution followed by extraction with dichloromethane. The combined organic layers were dried over magnesium sulfate, filtered and concentrated. The residue was purified by column chromatography on silica gel (0 to 30% ethyl acetate in hexanes). 880 mg (90 % purity, 64 % yield,) of the title compound were obtained.
¹H NMR (400 MHz, Chloroform-d) δ 5.36 (brs, 1H), 4.54 (m, 1H), 2.01 (m, 1H), 1.46 (s, 9H), 1.43 (d, *J* = 7.1 Hz, 3H), 1.17-1.03 (m, 2H), 1.02-0.94 (m, 2H).

### Intermediate 226

### ent-tert-Butyl ((1R)-1-(4-cyclopropyl-2,5-dioxoimidazolidin-4-yl)ethyl)carbamate

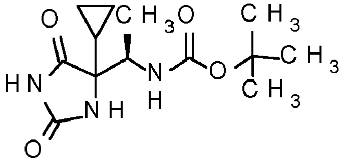

A solution of (R)-*tert*-butyl (1-cyclopropyl-1 -oxopropan-2-yl)carbamate (0.80 g, 3.8 mmol), potassium cyanide (0.73 g, 11 mmol) and ammonium carbonate (1.80 g, 18.8 mmol) in ethanol/water (1:1, 20 mL) was stirred at reflux for 6 days (-50 % conversion, dr = -2:1). The solvent was removed under reduced pressure and the residue loaded onto silica gel for dry loading and purified via column chromatography on silica gel (35 - 65 % ethyl acetate in hexanes) to afford both diastereomers. 101 mg (95 % purity, 13 % yield) of diastereomer A and 84 mg (75 % purity, 14 % yield) of diastereomer B of the title compound were obtained.
Diastereomer A: LC-MS (Method 13): Rₜ = 0.96 min; MS (ESIpos): m/z = 284 [M+H]⁺
Diastereomer B: LC-MS (Method 13): Rₜ = 0.91 min; MS (ESIpos): m/z = 284 [M+H]⁺

### Intermediate 227

### ent-5-(-Aminoethyl)-5-cyclopropylimidazolidine-2,4-dione bis (2,2,2-trifluoroacetate)

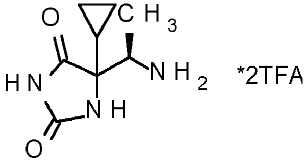

Diastereomer A of *ent-tert-Butyl* ((1*R*)-1-(4-cyclopropyl-2,5-dioxoimidazolidin-4-yl)ethyl)carbamate (110 mg) was stirred in a solution of 20 % trifluoroacetic acid in dichloromethane (7 mL) at room temperature for 3 hours. The solvent was removed under reduced pressure and the excess trifluoroacetic acid was azeotroped with toluene to afford 5-((R-1-aminoethyl)-5-cyclopropylimidazoline-2,4-dione bis (2,2,2-tridluoroacetate). The material was used in the next step without further purification or determining the yield.
¹H NMR (400 MHz, DMSO-d₆) δ 11.07 (brs, 1H), 8.02 (brs, 3H), 3.55 (q, *J* = 6.5, 6.0 Hz, 1H), 1.26 (m, 4H), 0.58 (m, 1H), 0.47 (m, 2H), 0.44 (m, 1H).

### Intermediate 228

### tert-Butyl (S)-(1-cyclopropyl-1-oxopropan-2-yl)carbamate

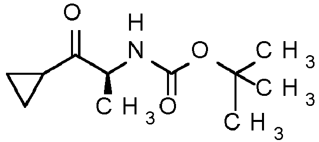

The reaction was carried out analogously to the preparation of *tert*-butyl (*R*)-(1-cyclopropyl-1-oxopropan-2-yl)carbamate. 800 mg (90 % purity, 60 % yield) of the title compound were obtained.
¹H NMR (400 MHz, Chloroform-d) δ 5.36 (brs, 1H), 4.54 (m, 1H), 2.01 (m, 1H), 1.46 (s, 9H), 1.43 (d, *J* = 7.1 Hz, 3H), 1.17-1.03 (m, 2H), 1.02-0.94 (m, 2H).

### Intermediate 229

### ent-tert-Butyl ((1S)-1-(4-cyclopropyl-2,5-dioxoimidazolidin-4-yl)ethyl)carbamate

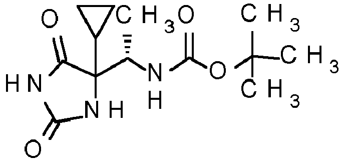

The reaction was carried out analogously to the preparation of *ent-tert-*Butyl ((1R)-1-(4-cyclopropyl-2,5-dioxoimidazolidin-4-yl)ethyl)carbamate. 68 mg (95 % purity, 13 % yield) of diastereomer A and 147 mg (95 % purity, 28 % yield) of diastereomer B of the title compound were obtained.
Diastereomer A: LC-MS (Method 13): Rₜ = 0.96 min; MS (ESIpos): m/z = 282 [M+H]⁺
Diastereomer B: LC-MS (Method 13): Rₜ = 0.92 min; MS (ESIpos): m/z = 282 [M+H]⁺

### Intermediate 230

### ent-5-((S)-1-Aminoethyl)-5-cyclopropylimidazolidine-2,4-dione bis (2,2,2-trifluoroacetate)

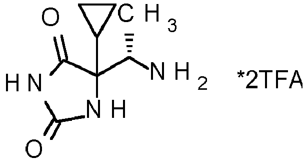

The reaction was carried out analogously to the preparation of *ent*-5-((R)-1-Aminoethyl)-5-cyclopropylimidazolidine-2,4-dione bis (2,2,2-trifluoroacetate). Using Diastereomer B of *ent-tert-*Butyl ((1S)-1-(4-cyclopropyl-2,5-dioxoimidazolidin-4-yl)ethyl)carbamate (147 mg), the title cpd was obtained and was used without further purification or determining the yield in the next step.
¹H NMR (400 MHz, DMSO-d6) δ 11.00 (brs, 1H), 8.00 (brs, 3H), 3.62 (m, 1H), 1.25 (d, J = 6.2 Hz, 3H), 1.14 (m, 1H), 0.57 (m, 2H), 0.43 (m, 1H), 0.07 (m, 1H).

### Intermediate 231

### Ethyl (2E)-3-oxo-2-[2-(3,4,5-trifluorophenyl)hydrazinylidene]propanoate

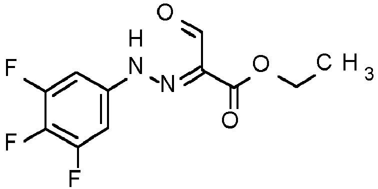

3,4,5-Trifluoroaniline (1.87 g, 12.7 mmol) was dissolved in 20 ml of water and 3.7 ml of concentrated hydrochloric acid. The mixture was cooled to 0°C and a solution of sodium nitrite (876 mg, 12.7 mmol) in 10 ml of water was added dropwise. After stirring for 5 minutes, this solution was added dropwise to a solution of ethyl-(2E)-3-(dimethylamino)acrylate (2.0 ml, 14 mmol) and potassium acetate (1.87 g, 19.0 mmol) in 30 ml of ethanol at 0°C. The mixture was stirred at room temperature for one hour before being diluted with ethyl acetate and washed twice with water and once with brine. The organic layer was dried over sodium sulfate, filtered and evaporated. 3.20 g (91 % purity, 83 % yield) of the title compound were obtained.
LC-MS (Method 7): Rₜ = 2.00 min; MS (ESIpos): m/z = 275 [M+H]⁺

### Intermediate 232

### Ethyl (2E,3E)-3-(hydroxyimino)-2-[2-(3,4,5-trifluorophenyl)hydrazinylidene]propanoate

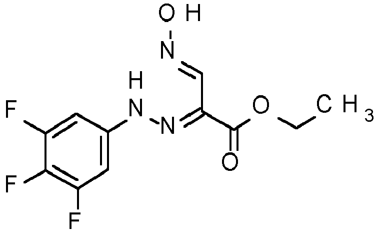

Ethyl (2E)-3-oxo-2-[2-(3,4,5-trifluorophenyl)hydrazinylidene]propanoate (3.20 g, 91 % purity, 10.6 mmol), hydroxylamine hydrochloride (881 mg, 12.7 mmol) and potassium acetate (2.59 g, 26.4 mmol) were dissolved in 46 ml of ethanol and the mixture was stirred for 30 minutes at 80°C. The reaction mixture was diluted with ethyl acetate and washed twice with water and once with brine. The organic layer was dried over sodium sulfate, filtered and evaporated. The residue was dried in vacuum. 3.17 g (79 % purity, 82 % yield) of the title compound were obtained
LC-MS (Method 7): Rₜ = 1.94 min; MS (ESIpos): m/z = 290 [M+H]⁺

### Intermediate 233

### Ethyl 2-(3,4,5-trifluorophenyl)-2H-1,2,3-triazole-4-carboxylate

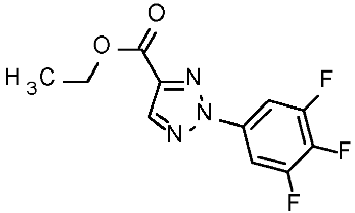

Ethyl (2E,3E)-3-(hydroxyimino)-2-[2-(3,4,5-trifluorophenyl)hydrazinylidene]propanoate (3.17 g, 79 % purity, 8.66 mmol) was stirred for one hour in 44 ml of acetic acid anhydride at 60°C. The reaction was then treated with water and extracted with ethyl acetate. The organic layer was dried over sodium sulfate, filtered and evaporated. The residue purified by chromatography on silica gel (100 g Ultra Snap Cartridge Biotage; eluent ethyl acetate/ cyclohexane, elution gradient 5%→25%; flow: 100 ml/min). Samples containing the desired product were united, the solvents were evaporated and the residue was dried in vacuum. 1.96 g (100 % purity, 83 % yield) of the title compound were obtained.
LC-MS (Method 8): Rₜ = 1.10 min; MS (ESIpos): m/z = 272 [M+H]⁺
¹H-NMR (500 MHz, DMSO-d6) δ [ppm]: 1.306 (0.66), 1.321 (0.59), 1.334 (7.89), 1.348 (16.00), 1.362 (7.67), 4.365 (0.42), 4.377 (2.69), 4.391 (7.71), 4.405 (7.48), 4.419 (2.36), 8.021 (2.92), 8.034 (3.42), 8.038 (3.42), 8.051 (2.95), 8.675 (9.00).

### Intermediate 234

### 2-(3,4,5-Trifluorophenyl)-2H-1,2,3-triazole-4-carboxylic acid

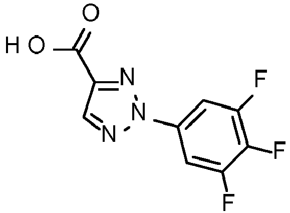

ethyl 2-(3,4,5-trifluorophenyl)-2H-1,2,3-triazole-4-carboxylate (1.33 g, 4.91 mmol) was dissolved in 30 ml of THF, treated with lithium hydroxide solution (4.9 ml, 1.0 M, 4.9 mmol) and the reaction mixture was stirred for 2 h at RT. After acidification with 2 N hydrochloric acid (pH2-3), the resulting mixture was concentrated *in vacuo,* diluted with ethylacetate and a small amount of water. After phase separation, the organic phase was dried, concentrated *in vacuo* and 1.12 g (97 % purity, 91 % yield) of the desired compound were obtained.
LC-MS (Method 7): Rₜ = 1.44 min; MS (ESIneg): m/z = 242 [M-H]⁻
¹H-NMR (500 MHz, DMSO-d6) δ [ppm]: 0.007 (0.64), 3.307 (1.28), 7.999 (4.83), 8.011 (5.61), 8.016 (5.37), 8.028 (4.75), 8.580 (16.00).

### Intermediate 235

### tert-butyl [2-(1-methyl-1H-pyrazol-5-yl)-2-oxoethyl]carbamate

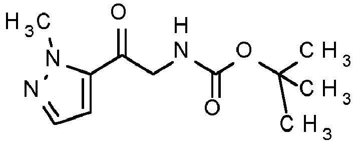

To a solution of 2-amino-1-(2-methylpyrazol-3-yl)ethanone hydrochloride (3.00 g, 17.1 mmol) in dichloromethane (77 ml) was added di-tert-butyl dicarbonate (4.10 g, 18.8 mmol) and triethylamine (7.1 ml, 51 mmol). After 2h of stirring at RT, the reaction mixture was diluted with dichloromethane and washed with water. The organic phase was dried, concentrated in vacuo and 4.40 g (91 % purity, 98 % yield) of the title compound was used without further purification.
LC-MS (Method 7): Rₜ = 1.47 min; MS (ESIpos): m/z = 240 [M+H]⁺
¹H-NMR (400 MHz, DMSO-d6) δ [ppm]: 0.943 (0.60), 1.111 (3.12), 1.280 (0.80), 1.366 (0.51), 1.372 (0.47), 1.394 (16.00), 4.046 (6.83), 4.256 (1.81), 4.271 (1.81), 7.143 (0.56), 7.202 (1.19), 7.207 (1.20), 7.553 (1.30), 7.558 (1.32).

### Intermediate 236

### rac-tert-butyl {[4-(1-methyl-1H-pyrazol-5-yl)-2,5-dioxoimidazolidin-4-yl]methyl}carbamate

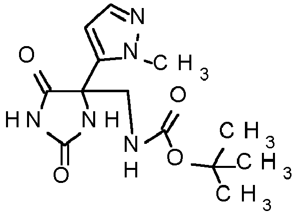

The reaction was performed in 2 batches.
A) To a solution of tert-butyl [2-(1-methyl-1H-pyrazol-5-yl)-2-oxoethyl]carbamate (2.00 g, 8.36 mmol) in methanol (15.3 ml) was added potassium cyanide (2.18 g, 33.4 mmol) and ammonium carbonate (3.21 g, 33.4 mmol) at RT. The reaction mixture was heated up to 80°C overnight into a sealed pressure flask. After filtration at RT, the filtrate was concentrated in vacuo and the crude was suspended in methanol. The suspension was filtered off and the resulting filtrate was concentrated in vacuo. (Batch 1)
B) To a solution of tert-butyl [2-(1-methyl-1H-pyrazol-5-yl)-2-oxoethyl]carbamate (2.00 g, 8.36 mmol) in a mixture of Ethanol (15 ml) and water (6 ml) was added potassium cyanide (2.18 g, 33.4 mmol) and ammonium carbonate (3.21 g, 33.4 mmol) at RT. The reaction mixture was heated up to 40°C overnight into a sealed pressure flask. After filtration at RT, the resulting filtrate was concentrated in vacuo. (Batch 2)

The combined crude product (batch1+2) was purified by preparative HPLC (sample preparation: 15 g dissolved in a mixture of methanol, water and acetonitrile; column: XBridge C18 5µm, 100x30mm; eluent: water→ acetonitrile/water 80:20 + 1% ammonia solution→ acetonitrile; flow rate: 80 ml/min; temperature: 40°C; UV detection: 220 nm). After lyophilisation, 1.8 g of the desired product was obtained and used without further purification.
LC-MS (Method 9): Rₜ = 0.36 min; MS (ESIpos): m/z = 310 [M+H]⁺
¹H-NMR (400 MHz, DMSO-d6) δ [ppm]: -0.008 (0.43), 0.008 (0.54), 1.359 (16.00), 1.754 (0.51), 3.524 (0.41), 3.540 (0.41), 3.693 (4.71), 6.230 (0.96), 7.250 (0.96).

The title compound can also be synthesized *via* the procedure described in J: Med. Chem. 2014, 57, 10476-10485

### Intermediate 237

### rac-5-(aminomethyl)-5-(1-methyl-1H-pyrazol-5-yl)imidazolidine-2,4-dione hydrochloride

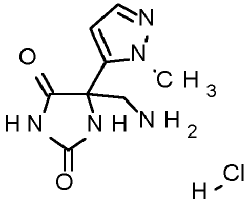

To a solution of *rac*-tert-butyl {[4-(1-methyl-1H-pyrazol-5-yl)-2,5-dioxoimidazolidin-4-yl]methyl}carbamate (520 mg, 1.68 mmol) in dichloromethane (14.5 ml) were added 2.1 ml (8.4mmol) of 4M hydrochloric acid in dioxane at RT. The reaction mixture was stirred overnight and concentrated in vacuo. 400 mg (100 % purity, 97 % yield) of the title compound was used without further purification.
LC-MS (Method 9): Rₜ = 0.22 min; MS (ESIneg): m/z = 208 [M-HCl-H]⁻
¹H-NMR (600 MHz, DMSO-d6) δ [ppm]: 1.767 (3.19), 2.119 (1.47), 2.390 (0.50), 3.166 (0.76), 3.463 (1.91), 3.473 (2.29), 3.485 (2.90), 3.494 (2.58), 3.590 (2.66), 3.598 (2.94), 3.612 (2.25), 3.620 (1.90), 3.725 (0.42), 4.091 (0.50), 5.692 (1.73), 6.501 (15.58), 6.504 (16.00), 7.358 (0.46), 7.425 (15.98), 7.428 (15.92), 8.608 (10.32), 8.873 (12.22), 11.515 (9.95).

The title compound can also be synthesized *via* the procedure described in J: Med. Chem. 2014, 57, 10476-10485

### Intermediate 238

### ethyl 5-[1-(difluoromethyl)-1H-pyrazol-5-yl]-1,3-oxazole-4-carboxylate

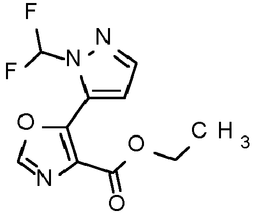

1-(difluoromethyl)-1H-pyrazole-5-carboxylic acid (2.60 g, 16.0 mmol) dissolved in 25 ml of THF was treated with 1,1,-carbonyl-diimidazole (3.12 g, 19.2 mmol) and stirred at room temperature. After 2 h ethyl isocyanoacetate (1.9 ml, 18 mmol), dissolved in 25 ml of THF, and a solution of lithium bis(trimethylsilyl)amide in THF (16 ml, 1.0 M, 16 mmol) were added at 0°C. The mixture was allowed to warm to room temperature, stirred overnight and then concentrated *in vacuo* The residue was taken up with ethyl acetate and washed with water. The organic layer was dried over sodium sulfate, filtered, Isolute® was added and the solvent was evaporated on a rotary evaporator. The crude product was purified by column chromatography (100 g Ultra Snap Cartridge Biotage; eluent ethyl acetate/ cyclohexane, elution gradient 10%→65%). Samples containing the desired product were united, the solvents were evaporated and the residue was dried in vacuum. 1.60 g (100 % purity, 39 % yield) of the title compound was obtained.
LC-MS (Method 7): Rₜ = 1.43 min; MS (ESIpos): m/z = 258 [M+H]⁺
¹H-NMR (400 MHz, DMSO-d6) δ [ppm]: 1.173 (7.68), 1.191 (16.00), 1.209 (7.93), 4.212 (2.57), 4.230 (7.90), 4.247 (7.83), 4.265 (2.52), 7.086 (4.50), 7.090 (4.56), 7.709 (2.14), 7.851 (4.21), 7.994 (2.25), 8.000 (4.40), 8.004 (4.29), 8.759 (6.93).

### Intermediate 239

### 2-amino-1-[1-(difluoromethyl)-1H-pyrazol-5-yl]ethanone hydrochloride

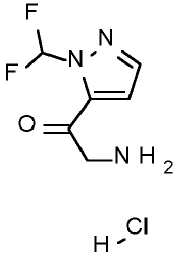

ethyl 5-[1-(difluoromethyl)-1H-pyrazol-5-yl]-1,3-oxazole-4-carboxylate (1.60 g, 6.22 mmol) was taken up in 35 ml of 6 N hydrochloric acid and stirred at 100°C. After 2 h the resulting mixture was concentrated *in vacuo* and the residue was treated with DCM and a small amount of methanol. The precipitate was filtered off and dried in vacuo. 1.10 g (84 % yield) of the title compound was obtained.
LC-MS (Method 9): Rₜ = 0.74 min; MS (ESIpos): m/z = 176 [M-HCl+H]⁺
¹H-NMR (400 MHz, DMSO-d6) δ [ppm]: 1.091 (0.50), 1.109 (1.11), 1.127 (0.59), 2.328 (0.75), 2.367 (1.14), 2.670 (0.78), 2.711 (1.17), 4.421 (0.53), 4.492 (13.60), 7.583 (16.00), 7.587 (15.25), 7.957 (6.86), 8.047 (13.41), 8.102 (13.69), 8.248 (6.77), 8.418 (8.00).

### Intermediate 240

### tert-butyl {2-[1-(difluoromethyl)-1H-pyrazol-5-yl]-2-oxoethyl}carbamate

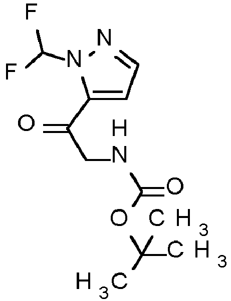

To a solution of 2-amino-1-[1-(difluoromethyl)-1H-pyrazol-5-yl]ethanone hydrochloride (1.10 g, 5.20 mmol) in dichloromethane (23 ml) was added di-tert-butyl dicarbonate (1.25 g, 5.72 mmol) and triethylamine (3.6 ml, 26 mmol). After 2h of stirring at RT, the reaction mixture was diluted with dichloromethane and washed with water. The organic phase was dried, concentrated in vacuo and 1.44 g of the title compound was used without further purification.
LC-MS (Method 7): Rₜ = 1.58 min; MS (ESIpos): m/z = 276 [M+H]⁺

### Intermediate 241

### rac-tert-butyl ({4-[1-(difluoromethyl)-1H-pyrazol-5-yl]-2,5-dioxoimidazolidin-4-yl}methyl)carbamate

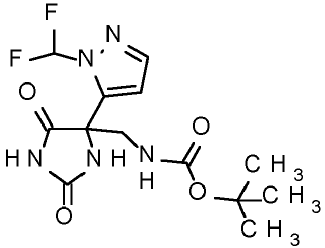

To a solution of tert-butyl {2-[1-(difluoromethyl)-1H-pyrazol-5-yl]-2-oxoethyl}carbamate (1.44 g, 5.23 mmol) in methanol (9.6 ml) was added potassium cyanide (1.36 g, 20.9 mmol) and ammonium carbonate (2.01 g, 20.9 mmol) at RT. The reaction mixture was stirred for 2 days at 60°C into a sealed pressure flask. After filtration at RT, the resulting filtrate was concentrated *in vacuo.* The residue was purified by preparative HPLC (sample preparation: 3.9 g dissolved in a mixture of methanol, water, acetonitrile and ammonia; column: XBridge C18 5µm, 100x30mm; eluent: water→ acetonitrile/water 80:20 + 1% ammonia solution→ acetonitrile; flow rate: 80 ml/min; temperature: 40°C; UV detection: 220 nm). 699 mg of the desired product was obtained and used without further purification.
LC-MS (Method 7): Rₜ = 1.08 min; MS (ESIneg): m/z = 344 [M-H]⁻

### Intermediate 242

### rac-5-(aminomethyl)-5-[1-(difluoromethyl)-1H-pyrazol-5-yl]imidazolidine-2,4-dione hydrochloride

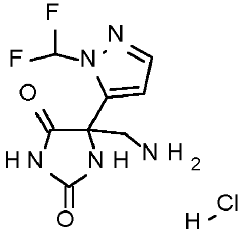

To a solution of *rac*-tert-butyl ({4-[1-(difluoromethyl)-1H-pyrazol-5-yl]-2,5-dioxoimidazolidin-4-yl}methyl)carbamate (340 mg, 985 µmol) in dichloromethane (8.5 ml) were added 1.2 ml (4.9 mmol) of 4M hydrochloric acid in dioxane at RT. The reaction mixture was stirred for 2h and concentrated in vacuo. 280 mg (99 % purity, 100 % yield) of the title compound was used without further purification.
LC-MS (Method 9): Rₜ = 0.22 min; MS (ESIpos): m/z = 246 [M-HCl+H]⁺
¹H-NMR (400 MHz, DMSO-d6) δ [ppm]: -0.149 (0.44), -0.008 (3.88), 0.008 (3.67), 0.146 (0.44), 1.110 (0.42), 1.596 (0.84), 1.754 (5.24), 2.111 (2.45), 2.324 (0.50), 2.329 (0.71), 2.333 (0.50), 2.367 (0.75), 2.524 (2.73), 2.666 (0.52), 2.671 (0.71), 2.675 (0.52), 2.711 (0.73), 3.457 (5.03), 3.491 (6.16), 3.568 (2.20), 3.689 (5.72), 3.723 (4.42), 5.756 (1.11), 6.811 (15.81), 6.816 (16.00), 7.820 (4.47), 7.835 (12.96), 7.839 (12.85), 7.960 (4.43), 7.969 (4.22), 8.109 (3.69), 8.504 (6.88), 8.825 (4.30), 11.497 (3.56).

### Intermediate 243

### ethyl 5-[5-(trifluoromethyl)-1,3-thiazol-4-yl]-1,3-oxazole-4-carboxylate

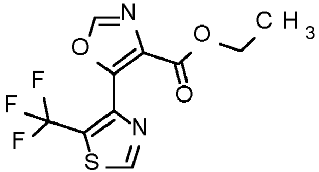

5-(trifluoromethyl)-1,3-thiazole-4-carboxylic acid (2.00 g, 10.1 mmol) dissolved in 15 ml of THF was treated with 1,1,-carbonyl-diimidazole (1.97 g, 12.2 mmol) and stirred at room temperature. After 1 h ethyl isocyanoacetate (1.2 ml, 11 mmol), dissolved in 15 ml of THF, and a solution of lithium bis(trimethylsilyl)amide in THF (10 ml, 1.0 M, 10 mmol) were added at 0°C. The mixture was allowed to warm to room temperature, stirred over 2 days and then concentrated *in vacuo.* The residue was taken up with ethyl acetate and washed with water. The organic layer was dried and concentrated *in vacuo.* The crude product was purified by column chromatography (100 g Ultra Snap Cartridge Biotage; eluent ethyl acetate/ cyclohexane, elution gradient 8%→85%). Samples containing the desired product were united, the solvents were evaporated and the residue was dried in vacuum. 1.43 g (48 % yield) of the title compound was obtained.
LC-MS (Method 7): Rₜ = 1.62 min; MS (ESIpos): m/z = 293 [M+H]⁺
¹H-NMR (400 MHz, DMSO-d6) δ [ppm]: 1.086 (7.76), 1.104 (16.00), 1.122 (8.00), 4.148 (2.68), 4.166 (8.12), 4.184 (8.05), 4.201 (2.61), 8.780 (5.87), 9.570 (4.98).

### Intermediate 244

### 2-amino-1-[5-(trifluoromethyl)-1,3-thiazol-4-yl]ethanone hydrochloride

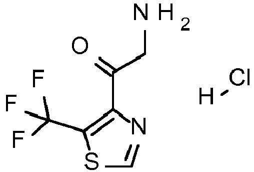

ethyl 5-[5-(trifluoromethyl)-1,3-thiazol-4-yl]-1,3-oxazole-4-carboxylate (1.43 g, 4.89 mmol) was taken up in 200 ml of 6 N hydrochloric acid and stirred for 2h at 100°C. After further stirring overnight at room temperature, the resulting mixture was concentrated *in vacuo.* 1.25 g (95 % purity, 98 % yield) of the title compound was obtained.
LC-MS (Method 9): Rₜ = 1.00 min; MS (ESIpos): m/z = 211 [M-HCl+H]⁺
¹H-NMR (400 MHz, DMSO-d6) δ [ppm]: -0.008 (1.13), 0.008 (1.18), 4.571 (16.00), 8.411 (4.83), 9.517 (12.18).

### Intermediate 245

### tert-butyl {2-oxo-2-[5-(trifluoromethyl)-1,3-thiazol-4-yl]ethyl}carbamate

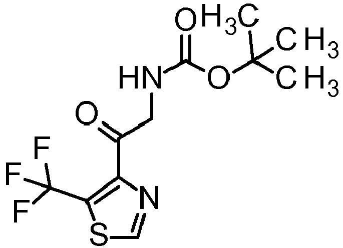

To a solution of 2-amino-1-[5-(trifluoromethyl)-1,3-thiazol-4-yl]ethanone hydrochloride (1.25 g, 5.07 mmol) in dichloromethane (23 ml) was added di-tert-butyl dicarbonate (1.22 g, 5.57 mmol) and triethylamine (3.5 ml, 25 mmol). After 2h of stirring at RT, the reaction mixture was diluted with dichloromethane and washed with water. The organic phase was dried, concentrated in vacuo and 1.66 g of the title compound was used without further purification.
LC-MS (Method 9): Rₜ = 1.58 min; MS (ESIpos): m/z = 310 [M-H]⁺

### Intermediate 246

### rac-tert-butyl ({2,5-dioxo-4-[5-(trifluoromethyl)-1,3-thiazol-4-yl]imidazolidin-4-yl}methyl)carbamate

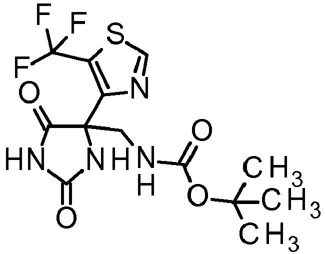

To a solution of tert-butyl {2-oxo-2-[5-(trifluoromethyl)-1,3-thiazol-4-yl]ethyl}carbamate (800 mg, 2.58 mmol) in methanol (4.6 ml) was added potassium cyanide (672 mg, 10.3 mmol) and ammonium carbonate (991 mg, 10.3 mmol) at RT. The reaction mixture was stirred overnight at 60°C into a sealed pressure flask. After filtration at RT, the resulting filtrate was concentrated *in vacuo.* The residue was purified by preparative HPLC (sample preparation: 473 mg dissolved in a mixture of methanol, water, acetonitrile and ammonia; column: XBridge C18 5µm, 100x30mm; eluent: water→ acetonitrile/water 80:20 + 1% ammonia solution→ acetonitrile; flow rate: 80 ml/min; temperature: 40°C; UV detection: 220 nm). 227 mg (100 % purity, 23 % yield) of the desired product was obtained and used without further purification.
LC-MS (Method 9): Rₜ = 0.76 min; MS (ESIneg): m/z = 379 [M-H]⁻

### Intermediate 247

### rac-5-(aminomethyl)-5-[5-(trifluoromethyl)-1,3-thiazol-4-yl]imidazolidine-2,4-dione hydrochloride

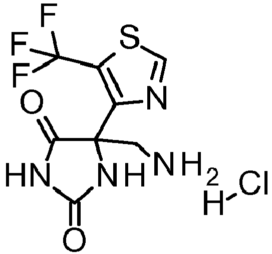

To a solution of *rac*-tert-butyl ({2,5-dioxo-4-[5-(trifluoromethyl)-1,3-thiazol-4-yl]imidazolidin-4-yl}methyl)carbamate (485 mg, 1.28 mmol) in dichloromethane (20 ml) were added 1.2 ml (1.6 ml, 4.0 M, 6.4 mmol) of 4M hydrochloric acid in dioxane at RT. The reaction mixture was stirred overnight and concentrated in vacuo. 413 mg (95 % purity, 97 % yield) of the title compound was used without further purification.
LC-MS (Method 9): Rₜ = 0.29 min; MS (ESIpos): m/z = 281 [M-HCl+H]⁺
¹H-NMR (400 MHz, DMSO-d6) δ [ppm]: -0.008 (0.97), 0.008 (0.91), 1.754 (10.12), 2.108 (3.70), 2.367 (0.43), 2.519 (1.81), 2.524 (1.46), 2.558 (0.54), 2.560 (0.45), 2.710 (0.41), 3.167 (16.00), 3.708 (3.29), 3.741 (2.54), 8.321 (2.94), 8.628 (1.96), 9.440 (7.56), 11.450 (3.19).

### Intermediate 248

### Ethyl 5-(1-ethyl-1H-pyrazol-5-yl)-1,3-oxazole-4-carboxylate

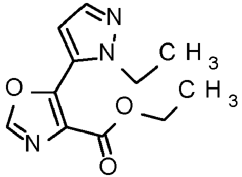

A solution of 1-ethyl-1H-pyrazole-5-carboxylic acid (1.00 g, 7.14 mmol) and 1,1'-carbonyldiimidazole (1.39 g, 8.56 mmol) in 10 ml of THF was stirred for 2 h at room temperature. After that time, a solution of ethyl isocyanoacetate (860 µl, 7.8 mmol) in 10 ml of THF and a solution of lithium bis(trimethylsilyl)amide in THF (7.1 ml, 1.0 M, 7.1 mmol) were added dropwise at 0°C. When the addition was complete the resulting mixture was stirred at room temperature for 2 h. The reaction was concentrated in vacuo and extracted with ethyl acetate. The combined organic layers were dried over sodium sulfate, filtered and concentrated. The crude product was purified by column chromatography (Machine: Biotage® Isolera One; column: Biotage® SNAP Ultra 50 g; gradient: Cy/EE-gradient, 16% EE - 100% EE; flow: 100 ml/min). Product containing samples were united and the solvents were removed on a rotary evaporator. 1.30 g (95 % purity, 74 % yield) of the title compound were obtained.
LC-MS (Method 7): Rₜ = 1.23 min; MS (ESIpos): m/z = 236 [M+H]⁺

### Intermediate 249

### 2-amino-1-(1-ethyl-1H-pyrazol-5-yl)ethan-1-one-hydrogen chloride (1/1)

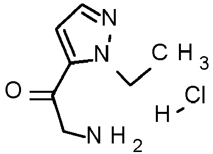

Ethyl 5-(1-ethyl-1H-pyrazol-5-yl)-1,3-oxazole-4-carboxylate (1.30 g, 5.53 mmol) were taken up in 29 ml of 6 N hydrochloric acid and stirred at 100°C for 2 h. The solvent was removed on a rotary evaporator and the residue was taken up in DCM/MeOH 20:1. The solvent was removed an the residue was dried in vacuo. 1.19 g (114 % yield) of the title compound were obtained.

### Intermediate 250

### tert-butyl [2-(1-ethyl-1H-pyrazol-5-yl)-2-oxoethyl]carbamate

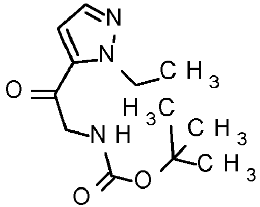

2-amino-1-(1-ethyl-1H-pyrazol-5-yl)ethan-1-one-hydrogen chloride (1/1) (1.10 g, 5.80 mmol) dissolved in 23 ml of dichloromethane were treated with di-tert-butyl dicarbonate (1.5 ml, 6.4 mmol) and triethylamine (2.4 ml, 17 mmol). The mixture was stirred at room temperature for 2 h. The solvent was removed on a rotary evaporator. The residue was taken up with ethyl acetate and washed with water and saturated aqueous sodium chloride solution. The organic layer was dried over sodium sulfate, filtered and evaporated on a rotary evaporator. The crude product was purified by column chromatography (Machine: Biotage® Isolera One; column: Biotage® SNAP Ultra 25 g; gradient: Cy/EE-gradient, 12% EE - 100% EE; flow: 75 ml/min). Product containing samples were united and the solvents were removed on a rotary evaporator. 1.07 g (100 % purity, 73 % yield) of the title compound were obtained.
LC-MS (Method 9): Rₜ = 1.40 min; MS (ESIpos): m/z = 254 [M+H]⁺

### Intermediate 251

### rac-tert-butyl {[4-(1-ethyl-1H-pyrazol-5-yl)-2,5-dioxoimidazolidin-4-yl]methyl}carbamate

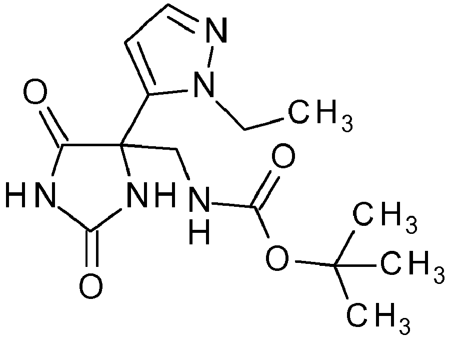

In a microwave vial tert-butyl [2-(1-ethyl-1H-pyrazol-5-yl)-2-oxoethyl]carbamate (1.00 g, 3.95 mmol) was dissolved in 8 ml of methanol. Potassium cyanide (1.03 g, 15.8 mmol) and ammonium carbonate (1.52 g, 15.8 mmol) were added. The vial was sealed and the mixture was stirred at 50°C for 48 h. The mixture was filtered and the filtrate was purified by preparative HPLC (Column: Chromatorex C18 10 µm 250 x 30 mm; eluent A=water, B=acetonitrile; gradient: 0.0 min 30% B; 4.5 min 50% B; 11.5 min 70% B; 12 min 100% B; 14.75 min 30% B; flow: 50 ml/min; 0.1% formic acid). Product containing samples were united and the solvents were removed on a rotary evaporator. 137 mg (100 % purity, 11 % yield) of the title compound were obtained.
LC-MS (Method 7): Rₜ = 1.02 min; MS (ESIpos): m/z = 324 [M+H]⁺

### Intermediate 252

### rac-5-(aminomethyl)-5-(1-ethyl-1H-pyrazol-5-yl)imidazolidine-2,4-dione-hydrogen chloride

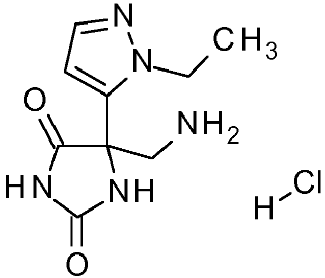

tert-butyl {[4-(1-ethyl-1H-pyrazol-5-yl)-2,5-dioxoimidazolidin-4-yl]methyl}carbamate (137 mg, 424 µmol) was dissolved in 2 ml of dichloromethane. 4 M hydrochloric acid in 1,4-dioxane (530 µl, 4.0 M, 2.1 mmol) was added and the mixture was stirred over night. The solvent was removed on a rotary evaporator and the residue was dried in vacuo. 142 mg (90 % purity, 116 % yield) of the title compound were obtained.
LC-MS (Method 9): Rₜ = 0.22 min; MS (ESIpos): m/z = 224 [M-HCl+H]⁺

### Intermediate 253

### Ethyl 5-(4-methyl-1,3-thiazol-5-yl)-1,3-oxazole-4-carboxylate

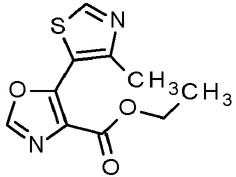

A solution of 4-methyl-1,3-thiazole-5-carboxylic acid (5.00 g, 34.9 mmol) and 1,1'-carbonyldiimidazole (6.80 g, 41.9 mmol) in 50 ml of THF was stirred for 2 h at room temperature. After that time, a solution of ethyl isocyanoacetate (4.2 ml, 38 mmol) in 50 ml of THF and a solution of lithium bis(trimethylsilyl)amide in THF (35 ml, 1.0 M, 35 mmol) were added dropwise at 0°C. When the addition was complete the resulting mixture was stirred at room temperature over night. The reaction was concentrated in vacuo and extracted with ethyl acetate. The combined organic layers were dried over sodium sulfate, filtered and concentrated. The crude product was purified by column chromatography (Machine: Biotage® Isolera One; column: Biotage® SNAP Ultra 100 g; gradient: Cy/EE-gradient, 12% EE - 100% EE; flow: 100 ml/min). Product containing samples were united and the solvents were removed on a rotary evaporator. 5.76 g (100 % purity, 69 % yield) of the title compound were obtained.
LC-MS (Method 7): Rₜ = 1.15 min; MS (ESIpos): m/z = 239 [M+H]⁺

### Intermediate 254

### 2-amino-1-(4-methyl-1,3-thiazol-5-yl)ethan-1-one-hydrogen chloride

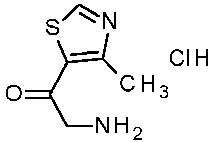

Ethyl 5-(4-methyl-1,3-thiazol-5-yl)-1,3-oxazole-4-carboxylate (5.76 g, 24.2 mmol) were taken up in 130 ml of 6 N hydrochloric acid and stirred at 100°C for 1 h. The solvent was removed on a rotary evaporator and the residue was taken up in DCM/MeOH 20:1. The precipitate was filtered off, washed with dichloromethane and dried in vacuo. 4.79 g (97 % purity, 100 % yield) of the title compound were obtained.
LC-MS (Method 9): Rₜ = 0.62 min; MS (ESIpos): m/z = 157 [M-HCl+H]⁺

### Intermediate 255

### tert-butyl [2-(4-methyl-1,3-thiazol-5-yl)-2-oxoethyl]carbamate

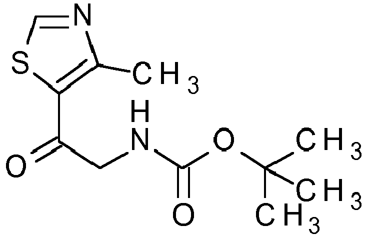

2-amino-1-(4-methyl-1,3-thiazol-5-yl)ethan-1-one-hydrogen chloride (4.79 g, 24.9 mmol) dissolved in 100 ml of dichloromethane were treated with di-tert-butyl dicarbonate (6.3 ml, 27 mmol) and triethylamine (10 ml, 75 mmol). The mixture was stirred at room temperature for 4 h. The solvent was removed on a rotary evaporator. The residue was taken up with ethyl acetate and washed with water and saturated aqueous sodium chloride solution. The organic layer was dried over sodium sulfate, filtered and evaporated on a rotary evaporator. 4.98 g (100 % purity, 78 % yield) of the title compound were obtained.
LC-MS (Method 7): Rₜ = 1.50 min; MS (ESIpos): m/z = 257 [M+H]⁺

### Intermediate 256

### rac-tert-butyl {[4-(4-methyl-1,3-thiazol-5-yl)-2,5-dioxoimidazolidin-4-yl]methyl}carbamate

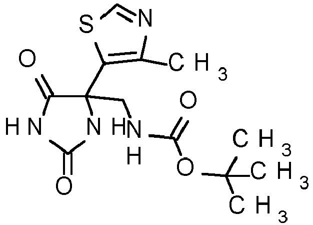

In a microwave vial tert-butyl [2-(4-methyl-1,3-thiazol-5-yl)-2-oxoethyl]carbamate (4.98 g, 19.4 mmol) was dissolved in 30 ml of methanol. Potassium cyanide (5.06 g, 77.7 mmol) and ammonium carbonate (7.47 g, 77.7 mmol) were added. The vial was sealed and the mixture was stirred at 60°C for 3 d. The crude product was purified by column chromatography (Machine: Biotage® Isolera One; column: Biotage® SNAP Ultra 100 g; gradient: DCM/MeOH-gradient, 2% MeOH - 20% MeOH; flow: 100 ml/min). Product containing samples were united and the solvents were removed on a rotary evaporator. 3.00 g (100 % purity, 47 % yield) of the title compound were obtained.
LC-MS (Method 7): Rₜ = 1.11 min; MS (ESIpos): m/z = 327 [M+H]⁺

### Intermediate 257

### rac-5-(aminomethyl)-5-(4-methyl-1,3-thiazol-5-yl)imidazolidine-2,4-dione-hydrogen chloride

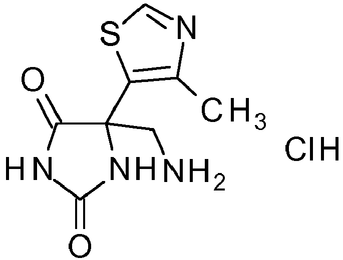

rac-tert-butyl {[4-(4-methyl-1,3-thiazol-5-yl)-2,5-dioxoimidazolidin-4-yl]methyl}carbamate (200 mg, 613 µmol) was dissolved in 3 ml of dichloromethane. 4 M hydrochloric acid in 1,4-dioxane (766 µl, 4.0 M, 3.1 mmol) was added and the mixture was stirred for 2 h. The precipitate was filtered off, washed with dichloromethane and dried in vacuo. 225 mg (95 % purity, 133 % yield) of the title compound were obtained.
LC-MS (Method 9): Rₜ = 0.23 min; MS (ESIpos): m/z = 227 [M-HCl+H]⁺

### Intermediate 258

### Ethyl (2E)-3-oxo-2-[2-(1,2-thiazol-4-yl)hydrazinylidene]propanoate

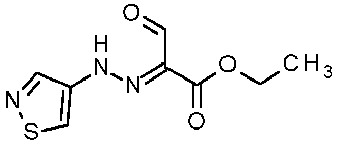

1,2-thiazol-4-amine-hydrogen chloride (250 mg, 1.83 mmol) was dissolved in 3 ml of water and 400 µl of concentrated hydrochloric acid. The mixture was cooled to 0°C and a solution of sodium nitrite (126 mg, 1.83 mmol) in 3 ml of water was added dropwise. After stirring for 5 minutes this solution was added dropwise to a solution of ethyl-(2E)-3-(dimethylamino)acrylate (290 µl, 2.0 mmol) and potassium acetate (269 mg, 2.75 mmol) in 3 ml of ethanol at 0°C. The mixture was stirred at room temperature for 30 min before being diluted with water. The precipitate was filtered off, washed with water and dried in vacuo. 170 mg (100 % purity, 41 % yield) of the title compound were obtained.
LC-MS (Method 7): Rₜ = 1.33 min; MS (ESIpos): m/z = 228 [M+H]⁺

### Intermediate 259

### Ethyl (2E,3E)-3-(hydroxyimino)-2-[2-(1,2-thiazol-4-yl)hydrazinylidene]propanoate

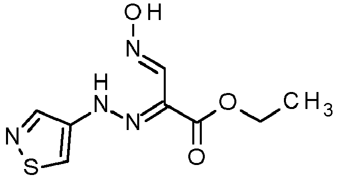

Ethyl (2E)-3-oxo-2-[2-(1,2-thiazol-4-yl)hydrazinylidene]propanoate (170 mg, 748 µmol), hydroxylamine hydrochloride (62.4 mg, 898 µmol) and potassium acetate (184 mg, 1.87 mmol) were dissolved in 7 ml of ethanol and the mixture was stirred over night at room temperature. The reaction mixture was diluted with water. The precipitate was filtered off, washed with water and dried in vacuo. 80.0 mg (100 % purity, 44 % yield) of the title compound were obtained.
LC-MS (Method 7): Rₜ = 1.45 min; MS (ESIpos): m/z = 243 [M+H]⁺

### Intermediate 260

### Ethyl 2-(1,2-thiazol-4-yl)-2H-1,2,3-triazole-4-carboxylate

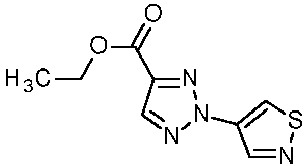

Ethyl (2E,3E)-3-(hydroxyimino)-2-[2-(1,2-thiazol-4-yl)hydrazinylidene]propanoate (80.0 mg, 330 µmol) was stirred for 1.5 h in 2 ml of acetic acid anhydride at 60°C. The mixture was purified by preparative HPLC (Column: Chromatorex C18 10 µm 250 x 30 mm; eluent A=water, B=acetonitrile; gradient: 0.0 min 30% B; 4.5 min 50% B; 11.5 min 70% B; 12 min 100% B; 14.75 min 30% B; flow: 50 ml/min; 0.1% formic acid). Product containing samples were united and the solvents were removed on a rotary evaporator. 60.0 mg (100 % purity, 81 % yield) of the title compound were obtained.
LC-MS (Method 8): Rₜ = 0.78 min; MS (ESIpos): m/z = 225 [M+H]⁺

### Intermediate 261

### 2-(1,2-thiazol-4-yl)-2H-1,2,3-triazole-4-carboxylic acid

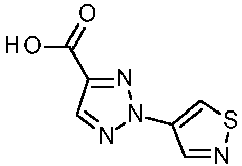

Ethyl 2-(1,2-thiazol-4-yl)-2H-1,2,3-triazole-4-carboxylate (60.0 mg, 268 µmol) was dissolved in 2.5 ml of THF, treated with lithium hydroxide solution (270 µl, 1.0 M, 270 µmol) and the mixture was stirred for 1 h at room temperature. The solvent was removed on a rotary evaporator. Water was added an the mixture was acidified with 2 N hydrochloric acid. The precipitate was filtered off, washed with water an dried in vacuo. 45.0 mg (100 % purity, 86 % yield) of the title compound were obtained
LC-MS (Method 8): Rₜ = 0.41 min; MS (ESIpos): m/z = 197 [M+H]⁺

### Intermediate 262

### Ethyl 5-[1-(2,2,2-trifluoroethyl)-1H-pyrazol-5-yl]-1,3-oxazole-4-carboxylate

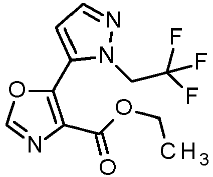

A solution of 1-(2,2,2-trifluoroethyl)-1H-pyrazole-5-carboxylic acid (1.00 g, 5.15 mmol) and 1,1'-carbonyldiimidazole (1.00 g, 6.18 mmol) in 11 ml of THF was stirred for 2 h at room temperature. After that time, a solution of ethyl isocyanoacetate (620 µl, 5.7 mmol) in 11 ml of THF and a solution of lithium bis(trimethylsilyl)amide in THF (5.2 ml, 1.0 M, 5.2 mmol) were added dropwise at 0°C. When the addition was complete the resulting mixture was stirred at room temperature for 1 h. The reaction was concentrated in vacuo and extracted with ethyl acetate. The combined organic layers were dried over sodium sulfate, filtered and concentrated. The crude product was purified by column chromatography (Machine: Biotage® Isolera One; column: Biotage® SNAP Ultra 25 g; gradient: Cy/EE-gradient, 12% EE - 100% EE; flow: 75 ml/min). Product containing samples were united and the solvents were removed on a rotary evaporator. 1.16 g (100 % purity, 78 % yield) of the title compound were obtained.
LC-MS (Method 7): Rₜ = 1.43 min; MS (ESIpos): m/z = 290 [M+H]⁺

### Intermediate 263

### 2-amino-1-[1-(2,2,2-trifluoroethyl)-1H-pyrazol-5-yl]ethan-1-one-hydrogen chloride

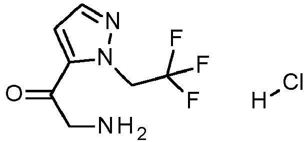

Ethyl 5-[1-(2,2,2-trifluoroethyl)-1H-pyrazol-5-yl]-1,3-oxazole-4-carboxylate (1.16 g, 4.01 mmol) were taken up in 20 ml of 6 N hydrochloric acid and stirred at 100°C for 1 h. The solvent was removed on a rotary evaporator and the residue was taken up in DCM/MeOH 20:1. The solvent was removed and the residue was dried in vacuo. 1.07 g (100 % purity, 110 % yield of the title compound were obtained.
LC-MS (Method 9): Rₜ = 0.95 min; MS (ESIpos): m/z = 208 [M-HCl+H]⁺

### Intermediate 264

### tert-butyl {2-0x0-2-[1-(2,2,2-trifluoroethyl)-1H-pyrazol-5-yl]ethyl}carbamate

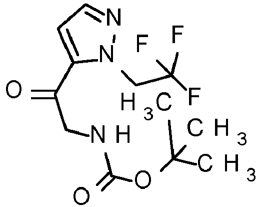

2-amino-1-[1-(2,2,2-trifluoroethyl)-1H-pyrazol-5-yl]ethan-1-one-hydrogen chloride (1.07 g, 4.39 mmol) dissolved in 17 ml of dichloromethane were treated with di-tert-butyl dicarbonate (1.1 ml, 4.8 mmol) and triethylamine (1.8 ml, 13 mmol). The mixture was stirred at room temperature for 3 h. The solvent was removed on a rotary evaporator. The residue was taken up with ethyl acetate and washed with water and saturated aqueous sodium chloride solution. The organic layer was dried over sodium sulfate, filtered and evaporated on a rotary evaporator. 1.37 g (71 % purity, 72 % yield) of the title compound were obtained.
LC-MS (Method 9): Rₜ = 1.52 min; MS (ESIpos): m/z = 308 [M+H]⁺

### Intermediate 265

### rac-tert-butyl({2,5-dioxo-4-[1-(2,2,2-trifluoroethyl)-1H-pyrazol-5-yl]imidazolidin-4-yl}methyl)carbamate

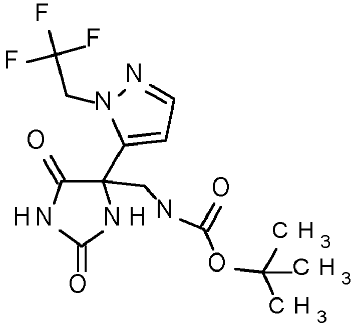

In a microwave vial tert-butyl {2-oxo-2-[1-(2,2,2-trifluoroethyl)-1H-pyrazol-5-yl]ethyl}carbamate (689 mg, 2.24 mmol) was dissolved in 5 ml of methanol. Potassium cyanide (730 mg, 11.2 mmol) and ammonium carbonate (1.08 g, 11.2 mmol) were added. The vial was sealed and the mixture was stirred at 50°C for 48 h. The mixture was filtered and the filtrate was purified by preparative HPLC (Column: Chromatorex C18 10 µm 250 x 30 mm; eluent A=water, B=acetonitrile; gradient: 0.0 min 30% B; 4.5 min 50% B; 11.5 min 70% B; 12 min 100% B; 14.75 min 30% B; flow: 50 ml/min; 0.1% formic acid). Product containing samples were united and the solvents were removed on a rotary evaporator. 195 mg (100 % purity, 23 % yield) of the title compound were obtained.
LC-MS (Method 7): Rₜ = 1.21 min; MS (ESIpos): m/z = 378 [M+H]⁺

### Intermediate 266

### rac-5-(aminomethyl)-5-[1-(2,2,2-trifluoroethyl)-1H-pyrazol-5-yl]imidazolidine-2,4-dione hydrochloride

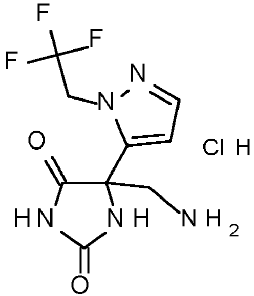

*rac*-tert-butyl({2,5-dioxo-4-[1-(2,2,2-trifluoroethyl)-1H-pyrazol-5-yl]imidazolidin-4-yl}methyl)carbamate (195 mg, 517 µmol) was dissolved in 2 ml of dichloromethane. 4 M hydrochloric acid in 1,4-dioxane (650 µl, 4.0 M, 2.6 mmol) was added and the mixture was stirred for 3 h. The solvent was removed on a rotary evaporator. The residue was taken up in dichloromethane, the solvent was removed and the residue was dried in vacuo. 155 mg (96 % purity, 92 % yield) of the title compound were obtained.
LC-MS (Method 9): Rₜ = 0.23 min; MS (ESIpos): m/z = 278 [M-HCl+H]⁺

### Intermediate 267

### 3,5-difluoro-2-hydrazinylpyridine

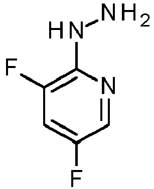

2,3,5-trifluoropyridine (670 µl, 7.5 mmol) were dissolved in hydrazine hydrate (3.7 ml, 75 mmol). The mixture was stirred at 120°C for 1 h. The mixture was extracted with dichloromethane, the organic layer was dried over sodium sulfate, filtered and evaporated on a rotary evaporator. 403 mg (100 % purity, 37 % yield) of the title compound were obtained.
GC-MS (Method A): Rₜ = 2.52 min; MS (EI-MS-POS): m/z = 145 [M]⁺

### Intermediate 268

### N-{(1E,2Z)-2-[2-(3,5-difluoropyridin-2-yl)hydrazinylidene]propylidene}hydroxylamine

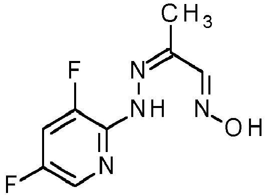

3,5-difluoro-2-hydrazinylpyridine (403 mg, 2.78 mmol) was dissolved in 12 ml of ethanol. (1E)-1-(hydroxyimino)propan-2-one (290 mg, 3.33 mmol) was added and the mixture was stirred at 80°C. After 2 h the solvent was removed on a rotary evaporator. The residue was taken up with pentane. The precipitate was filtered off, washed with pentane and dried in vacuo. 601 mg (38 % purity, 38 % yield) of the title compound were obtained.
LC-MS (Method 9): Rₜ = 1.01 min; MS (ESIpos): m/z = 215 [M+H]⁺

### Intermediate 269

### 3,5-difluoro-2-(4-methyl-2H-1,2,3-triazol-2-yl)pyridine

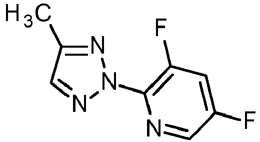

N-{(1E,2Z)-2-[2-(3,5-difluoropyridin-2-yl)hydrazinylidene]propylidene}hydroxylamine (601 mg, 38 % purity, 1.05 mmol) was dissolved in 10 ml of acetic anhydride and stirred at 130°C. After 3 h Isolute® was added and the solvent was evaporated on a rotary evaporator. The crude product was purified by column chromatography (Machine: Biotage® Isolera One; column: Biotage® SNAP Ultra 50 g; gradient: Cy/EE-gradient, 8% EE - 66% EE; flow: 100 ml/min). Product containing samples were united and the solvents were removed on a rotary evaporator. 249 mg (68 % purity, 82 % yield) of the title compound were obtained.
LC-MS (Method 7): Rₜ = 1.19 min; MS (ESIpos): m/z = 197 [M+H]⁺

### Intermediate 270

### 2-(3,5-difluoropyridin-2-yl)-2H-1,2,3-triazole-4-carboxylic acid

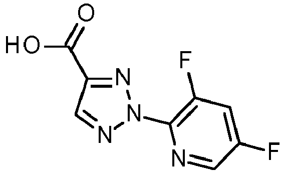

3,5-difluoro-2-(4-methyl-2H-1,2,3-triazol-2-yl)pyridine (249 mg, 68 % purity, 863 µmol) was suspended in 10 ml of water. Potassium permanganate (682 mg, 4.32 mmol) was added and the mixture was stirred at 100°C. After 48 h more potassium permanganate (500 mg, 3.16 mmol) was added and the mixture was stirred at 100°C for 24 h. Once again more potassium permanganate (500 mg, 3.16 mmol) was added and the mixture was stirred for another 5 d at 100°C. The mixture was filtered over Celite® and the filtrate was purified by preparative HPLC (Column: Chromatorex C18 10 µm 250 x 30 mm; eluent A=water, B=acetonitrile; gradient: 0.0 min 5% B; 3 min 5% B; 20 min 50% B; 23 min 100% B; 26 min 5% B; flow: 50 ml/min; 0.1% formic acid). Product containing samples were united and the solvents were removed on a rotary evaporator. 8.00 mg (100 % purity, 4 % yield) of the title compound were obtained.
LC-MS (Method 7): Rₜ = 0.72 min; MS (ESIpos): m/z = 227 [M+H]⁺

### Intermediate 271

### tert-butyl [2-oxo-2-(1,3-thiazol-2-yl)ethyl]carbamate

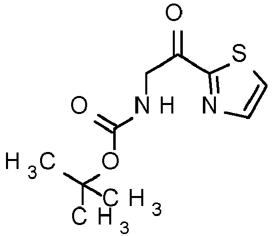

Under argon, thiazole (3.6 ml, 51 mmol) was dissolved in THF (20 ml) and cooled in an acetonitrile/dry ice bath to - 40°C. The solution was treated dropwise with n-butyllithium in hexane (20.3 ml, 2.5 M, 51 mmol). After 45 minutes at -35 °C, a solution of tert-butyl {2-[methoxy(methyl)amino]-2-oxoethyl}carbamate (5.00 g, 22.9 mmol) in 30 ml of THF was added dropwise, while keeping the temperature below -40°C. After 2 h at - 40 to -45 °C, the reaction was quenched with 1 N citric acid (25 ml) and diluted with ethyl acetate. The layers were separated. The aqueous layer was extracted with ethyl acetate and the organic phases were combined, washed with brine, dried and concentrated under reduced pressure. The crude product was purified by column chromatography (100 g Ultra Snap Cartridge Biotage; eluent ethyl acetate/ cyclohexane, elution gradient: 25%→60%) to give. 3.04 g (98 % purity, 24 % yield) of the title compound.
LC-MS (Method 8): Rₜ = 0.78 min; MS (ESIpos): m/z = 243 [M+H]⁺
¹H-NMR (500 MHz, DMSO-d6) δ [ppm]: 1.263 (0.77), 1.398 (16.00), 4.515 (1.84), 4.527 (1.80), 7.182 (0.52), 8.167 (1.36), 8.173 (1.47), 8.257 (1.73), 8.263 (1.52).

### Intermediate 272

### rac-tert-butyl {[2,5-dioxo-4-(1,3-thiazol-2-yl)imidazolidin-4-yl]methyl}carbamate

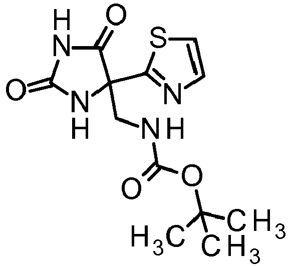

To a solution of tert-butyl [2-oxo-2-(1,3-thiazol-2-yl)ethyl]carbamate (3.04 g, 12.5 mmol) in methanol (20 ml) was added potassium cyanide (3.27 g, 50.2 mmol) and ammonium carbonate (4.82 g, 50.2 mmol) at RT. The reaction mixture was stirred overnight at 40°C into a sealed pressure flask and then diluted with water. The resulting suspension was extracted with dichloromethane. After phase separation; the aqueous phase was concentrated under reduced pressure and the crude product was suspended in a mixture of 30 ml acetonitrile and 20 ml methanol. After filtration of the residue, the filtrate was concentrated in vacuo and 1.4 g of the desired product was obtained and used without further purification.
LC-MS (Method 7): Rₜ = 0.99 min; MS (ESIneg): m/z = 311 [M-H]⁻

The title compound can also be synthesized *via* the procedure described in J. Med. Chem. 2014, 57, 10476-10485.

### Intermediate 273

### rac-5-(aminomethyl)-5-(1,3-thiazol-2-yl)imidazolidine-2,4-dione hydrochloride

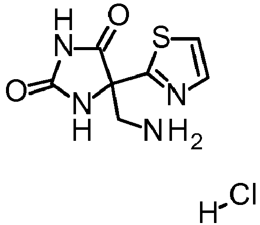

To a solution of rac-tert-butyl {[2,5-dioxo-4-(1,3-thiazol-2-yl)imidazolidin-4-yl]methyl}carbamate (1.40 g, 4.48 mmol) in methanol (28 ml) were added 22.4 ml (90 mmol) of 4M hydrochloric acid in dioxane at 0°C. After 1 h at 0°C, the reaction mixture was stirred overnight at RT and concentrated in vacuo. 1.30 g of the title compound was used without further purification.
LC-MS (Method 9): Rₜ = 0.26 min; MS (ESIneg): m/z = 211 [M-HCl-H]⁻
¹H-NMR (600 MHz, DMSO-d6) δ [ppm]: 0.005 (1.86), 1.234 (0.56), 1.246 (0.80), 1.596 (0.54), 1.759 (0.78), 2.387 (0.62), 2.426 (1.00), 2.655 (0.64), 2.887 (0.46), 3.167 (0.62), 3.462 (0.48), 3.472 (0.58), 3.492 (0.52), 3.502 (0.56), 3.527 (1.42), 3.537 (1.68), 3.550 (1.90), 3.559 (1.52), 3.667 (0.54), 3.676 (0.42), 3.704 (0.58), 3.713 (0.54), 3.722 (1.86), 3.754 (1.74), 3.763 (1.94), 3.776 (1.62), 3.785 (1.34), 3.917 (0.52), 5.084 (1.26), 7.183 (2.00), 7.268 (2.21), 7.352 (2.00), 7.774 (0.52), 7.780 (0.46), 7.881 (10.95), 7.886 (15.10), 7.909 (16.00), 7.914 (10.55), 8.484 (5.59), 9.120 (5.57), 11.420 (5.69).

The title compound can also be synthesized *via* the procedure described in J. Med. Chem. 2014, 57, 10476-10485.

### Intermediate 274

### tert-Butyl [2-(1-methyl-1H-imidazol-2-yl)-2-oxoethyl]carbamate

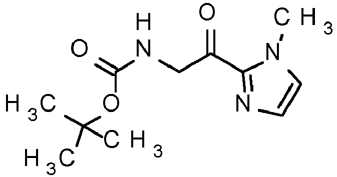

A solution of 2.70 mL (6.70 mmol) of 2.5M n-butyllithium in hexanes was added to a solution of 0.49 mL (6.10 mmol) of 1-methyl-1H-imidazole in 10 mL of anhydrous tetrahydrofuran under nitrogen atmosphere at 0 °C over 5 min and the resulting yellow solution was stirred under these conditions for 10 min. After that, the lithiated imidazole solution was transferred to a solution of 1.46 g (6.70 mmol) of tert-butyl {2-[methoxy(methyl)amino]-2-oxoethyl}carbamate at -78 °C over 15 min and the corresponding solution was further stirred for 1 hour under the same conditions.

After that time, the solution was taken out of the cooling bath and it was stirred for 15 min while warming up to room temperature. The mixture was quenched with 5 mL of a 1M solution of hydrochloric acid(aq.), stirred for 5 min, and 5 mL of brine and 5 mL of saturated hydrogencarbonate(aq.) were added. The resulting mixture was transferred to a separatory funnel and extracted with ethyl acetate. The organic layer was dried over magnesium sulfate, filtered and evaporated to dryness to give 0.70 g (48%) of the product as a pale orange oil. The compound was pure enough to be used as such without further purification.
¹H-NMR (300 MHz, DMSO-*d*₆): δ [ppm] = 1.39 (s, 9H), 3.91 (s, 3H), 4.40 (d, 2H), 6.99 (t, 1H), 7.11 (s, 1H), 7.52 (s, 1H).
LC-MS (Method 3): Rₜ = 0.546 min. MS (Mass method 1): m/z = 240 (M + H)⁺

### Intermediate 275

### rac-tert-Butyl {[4-(1-methyl-1H-imidazol-2-yl)-2,5-dioxoimidazolidin-4-yl]methyl}carbamate

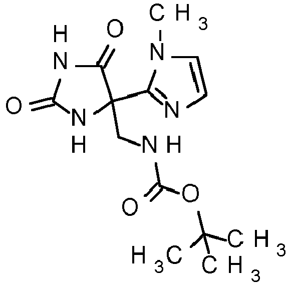

To a stirring solution of 4.22 g (43.90 mmol) of ammonium carbonate and 0.63 g (11.70 mmol) of ammonium chloride in 10 mL of water was added 0.70 g (2.93 mmol) of tert-butyl [2-(1-methyl-1H-imidazol-2-yl)-2-oxoethyl]carbamate in 10 mL of ethanol. After 15 min, 0.86 g (13.20 mmol) of potassium cyanide were added and the mixture was heated up to 60 °C for 16 hours into a sealed pressure flask. The yellow solution was concentrated until only a small fraction of water remained (a white precipitate appeared). Then, more water was added and the suspension was allowed to stand at 0 °C for 2 hour. After that time, the resulting solid was filtered off and washed with cold water and diethyl ether to give 0.90 g (98%) of the product as an off-white powder. The compound was used as such in the next step.
LC-MS (Method 3): Rₜ = 0.253 min. MS (Mass method 1): m/z = 310 (M + H)⁺

### Intermediate 276

### rac-5-(aminomethyl)-5-(1-methyl-1H-imidazol-2-yl)imidazolidine-2,4-dione hydrochloride

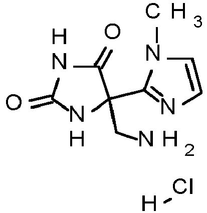

3.60 mL (15.00 mmol) of 4N hydrochloric acid in dioxane were added to a solution of 0.90 g (2.91 mmol) of *rac-*tert-Butyl {[4-(1-methyl-1H-imidazol-2-yl)-2,5-dioxoimidazolidin-4-yl]methyl}carbamate in 20 mL of dichloromethane and the mixture was allowed to stir at room temperature for 16 hours. The resulting precipitate was collected by filtration and washed with dichloromethane, ethyl acetate and diethyl ether to give 0.71 g (99%) of the product as a white solid. The compound was used as such in the next step.
LC-MS (Method 3): Rₜ = 0.089 min. MS (Mass method 1): m/z = 157 (M + H)⁺

### Intermediate 277

### ethyl 5-(1,3-thiazol-4-yl)-1,3-oxazole-4-carboxylate

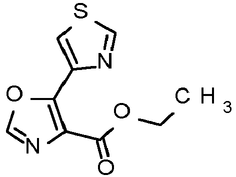

1,3-thiazole-4-carboxylic acid (1.00 g, 7.74 mmol) dissolved in 12 ml of THF was treated with 1,1,-carbonyl-diimidazole (1.51 g, 9.29 mmol) and stirred at room temperature. After 2 h, ethyl isocyanoacetate (930 µl, 8.5 mmol), dissolved in 12 ml of THF, and a solution of lithium bis(trimethylsilyl)amide in THF (7.7 ml, 1.0 M, 7.7 mmol) were added at 0°C. The mixture was allowed to warm to room temperature, stirred overnight and then concentrated *in vacuo.* The residue was taken up with ethyl acetate and washed with water. The organic layer was dried and concentrated *in vacuo.* The crude product was purified by column chromatography (100 g Ultra Snap Cartridge Biotage; eluent ethyl acetate/ cyclohexane, elution gradient 15%→100%). Samples containing the desired product were united, the solvents were evaporated and the residue was dried in vacuum. 783 mg (44 % yield) of the title compound was used without further purification.
LC-MS (Method 8): Rₜ = 0.65 min; MS (ESIpos): m/z = 225 [M+H]⁺
¹H-NMR (600 MHz, DMSO-d6) δ [ppm]: 1.291 (7.74), 1.302 (16.00), 1.314 (7.70), 4.313 (2.51), 4.325 (7.74), 4.337 (7.62), 4.349 (2.39), 8.580 (9.32), 8.744 (5.07), 8.747 (5.10), 9.273 (4.82), 9.276 (4.76).

### Intermediate 278

### 2-amino-1-(1,3-thiazol-4-yl)ethanone hydrochloride

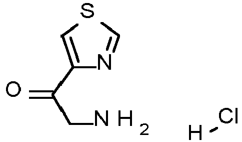

ethyl 5-(1,3-thiazol-4-yl)-1,3-oxazole-4-carboxylate (782 mg, 3.49 mmol) was taken up in 19 ml of 6 N hydrochloric acid. After 2 h the resulting mixture was concentrated *in vacuo* and the residue was treated with DCM and a small amount of methanol. The precipitate was filtered off and dried in vacuo. 613 mg (98 % yield) of the title compound was obtained and used without further purification
LC-MS (Method 9): Rₜ = 0.48 min; MS (ESIpos): m/z = 143 [M-HCl+H]⁺
¹H-NMR (600 MHz, DMSO-d6) δ [ppm]: 1.067 (4.56), 1.079 (9.66), 1.091 (4.71), 2.525 (0.47), 4.145 (1.10), 4.156 (3.13), 4.168 (3.10), 4.180 (1.18), 4.437 (4.70), 4.479 (5.69), 4.488 (11.43), 4.497 (11.15), 5.938 (1.13), 8.478 (5.26), 8.827 (15.55), 8.830 (16.00), 8.982 (3.87), 8.985 (4.11), 9.202 (1.56), 9.303 (15.03), 9.307 (15.02), 9.322 (4.08), 9.325 (4.09).

### Intermediate 279

### tert-butyl [2-oxo-2-(1,3-thiazol-4-yl)ethyl]carbamate

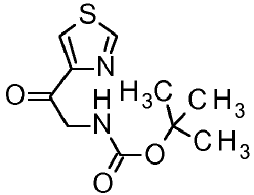

To a solution of 2-amino-1-(1,3-thiazol-4-yl)ethanone hydrochloride (610 mg, 3.41 mmol) in dichloromethane (14 ml) was added di-tert-butyl dicarbonate (860 µl, 3.8 mmol) and triethylamine (1.4 ml, 10 mmol). After 1.5 h of stirring at RT, the reaction mixture was concentrated under reduced pressure, diluted with ethylacetate and washed with water and brine. After phase separation, the organic phase was dried, concentrated in vacuo and 781 mg of the title compound was used without further purification.
LC-MS (Method 7): Rₜ = 1.33 min; MS (ESIpos): m/z = 143 [M-Boc+H]
¹H-NMR (400 MHz, DMSO-d6) δ [ppm]: -0.008 (0.42), 0.008 (0.42), 1.093 (0.66), 1.110 (0.45), 1.282 (0.71), 1.306 (0.40), 1.366 (0.48), 1.387 (2.15), 1.398 (14.02), 1.468 (16.00), 4.430 (1.68), 4.445 (1.65), 7.061 (0.46), 8.635 (0.72), 8.640 (0.78), 9.236 (0.92), 9.240 (0.90).

### Intermediate 280

### rac-tert-butyl {[2,5-dioxo-4-(1,3-thiazol-4-yl)imidazolidin-4-yl]methyl}carbamate

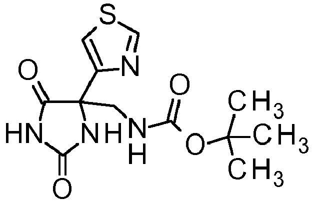

To a solution of tert-butyl [2-oxo-2-(1,3-thiazol-4-yl)ethyl]carbamate (781 mg, 3.22 mmol) in methanol (5.9 ml) was added potassium cyanide (840 mg, 12.9 mmol) and ammonium carbonate (1.24 g, 12.9 mmol) at RT. The reaction mixture was stirred for 2 days at 40°C into a sealed pressure flask. After filtration at RT, the resulting filtrate was concentrated *in vacuo.* The residue was purified by preparative HPLC (Method 2f). 283 mg of the desired product was obtained and used without further purification
LC-MS (Method 8): Rₜ = 0.63 min; MS (ESIneg): m/z = 311 [M-H]⁻
¹H-NMR (400 MHz, DMSO-d6) δ [ppm]: 1.366 (16.00), 1.434 (0.81), 7.733 (1.28), 7.738 (1.32), 8.136 (0.77), 9.107 (0.96), 9.111 (0.97), 10.776 (0.53).

### Intermediate 281

### rac-5-(aminomethyl)-5-(1,3-thiazol-4-yl)imidazolidine-2,4-dione hydrochloride

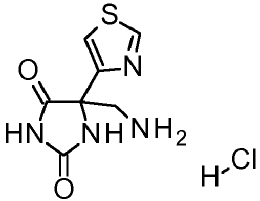

To a solution of rac-tert-butyl {[2,5-dioxo-4-(1,3-thiazol-4-yl)imidazolidin-4-yl]methyl}carbamate (283 mg, 906 µmol) in dichloromethane (7.8 ml) were added 1.1 ml of 4M hydrochloric acid in dioxane at RT. The reaction mixture was stirred for 4h and concentrated in vacuo. 200 mg (84 % yield) of the title compound was used without further purification.
LC-MS (Method 9): Rₜ = 0.24 min; MS (ESIpos): m/z = 213 [M-HCl+H]⁺
¹H-NMR (400 MHz, DMSO-d6) δ [ppm]: -0.008 (2.08), 0.008 (2.39), 1.366 (1.42), 1.596 (1.40), 2.525 (0.83), 3.433 (0.84), 3.446 (1.03), 3.466 (1.26), 3.479 (1.11), 3.568 (16.00), 3.637 (0.42), 3.660 (1.41), 3.676 (1.25), 3.695 (1.03), 3.709 (0.84), 3.872 (0.44), 4.304 (1.61), 5.755 (1.98), 7.647 (0.43), 7.652 (0.46), 7.895 (8.71), 7.900 (8.69), 8.343 (3.74), 8.524 (4.89), 9.180 (4.89), 9.185 (4.86), 11.201 (4.04).

### Intermediate 282

### Ethyl (2E)-2-[2-(2-chloro-4-fluorophenyl)hydrazinylidene]-3-oxopropanoate

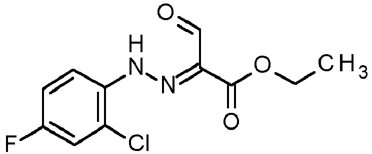

2-chloro-4-fluoroaniline (210 µl, 1.7 mmol) was dissolved in 2.5 ml of water and 470 µl of concentrated hydrochloric acid. The mixture was cooled to 0°C and a solution of sodium nitrite (118 mg, 1.72 mmol) in 1.25 ml of water was added dropwise. After stirring for 5 minutes this solution was added dropwise to a solution of ethyl-(2E)-3-(dimethylamino)acrylate (270 µl, 1.9 mmol) and potassium acetate (253 mg, 2.58 mmol) in 3.75 ml of ethanol at 0°C. The mixture was stirred at room temperature for 30 min before being diluted with ethyl acetate and washed twice with water and once with brine. The organic layer was dried over sodium sulfate, filtered and evaporated. 427 mg (46 % purity, 42 % yield) of the title compound were obtained.
LC-MS (Method 7): Rₜ = 2.09 min; MS (ESIneg): m/z = 271 [M-H]⁻

### Intermediate 283

### Ethyl (2E,3E)-2-[2-(2-chloro-4-fluorophenyl)hydrazinylidene]-3-(hydroxyimino)propanoate

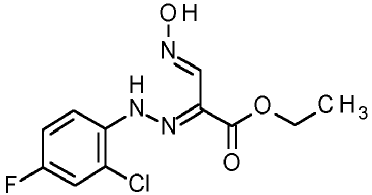

Ethyl (2E)-2-[2-(2-chloro-4-fluorophenyl)hydrazinylidene]-3-oxopropanoate (417 mg, 1.53 mmol), hydroxylamine hydrochloride (128 mg, 1.84 mmol) and potassium acetate (375 mg, 3.82 mmol) were dissolved in 8.7 ml of ethanol and the mixture was stirred at room temperature over night. The reaction mixture was diluted with ethyl acetate and washed twice with water and once with brine. The organic layer was dried over sodium sulfate, filtered and evaporated. The residue was dried in vacuum. 341 mg (75 % purity, 58 % yield) of the title compound were obtained.
LC-MS (Method 7): Rₜ = 1.92 min; MS (ESIpos): m/z = 288 [M+H]⁺

### Intermediate 284

### Ethyl 2-(2-chloro-4-fluorophenyl)-2H-1,2,3-triazole-4-carboxylate

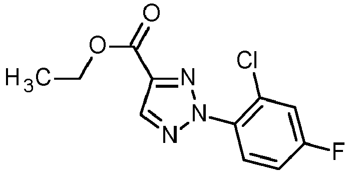

Ethyl (2E,3E)-2-[2-(2-chloro-4-fluorophenyl)hydrazinylidene]-3-(hydroxyimino)propanoate (330 mg, 1.15 mmol) was stirred for 2 h in 4 ml of acetic acid anhydride at 60°C. The reaction was then diluted in ethyl acetate and washed twice with water and once with brine. The organic layer was dried over sodium sulfate, filtered and evaporated. The residue was adsorbed on Isolute® and purified by chromatography on silica gel (10 g Ultra Snap Cartridge Biotage®; Biotage-Isolera-One®; Cy/EE-gradient: 2% EE - 20% EE; flow: 36 ml/min). Samples containing the desired product were united, the solvents were evaporated and the residue was dried in vacuum. 37.8 mg (98 % purity, 12 % yield) of the title compound were obtained.
LC-MS (Method 7): Rₜ = 1.92 min; MS (ESIpos): m/z = 270 [M+H]⁺

### Intermediate 285

### 2-(2-chloro-4-fluorophenyl)-2H-1,2,3-triazole-4-carboxylic acid

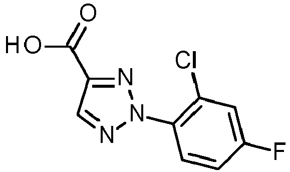

Ethyl 2-(2-chloro-4-fluorophenyl)-2H-1,2,3-triazole-4-carboxylate (37.8 mg, 140 µmol) was dissolved in 8 ml of THF, treated with lithium hydroxide solution (140 µl, 1.0 M, 140 µmol) and the mixture was stirred for 1 h at room temperature. More lithium hydroxide solution (140 µl, 1.0 M, 140 µmol) was added and the mixture was stirred over night at room temperature. The solvent was removed on a rotary evaporator. Water was added and the mixture was acidified with 2 N hydrochloric acid. The resulting precipitate was collected by filtration. 19.9 mg (96 % purity, 56 % yield) of the title compound were obtained.
LC-MS (Method 7): Rₜ = 1.24 min; MS (ESIneg): m/z = 240 [M-H]⁻

### Intermediate 286

### rac-tert-butyl [(4-methyl-2,5-dioxoimidazolidin-4-yl)methyl]carbamate

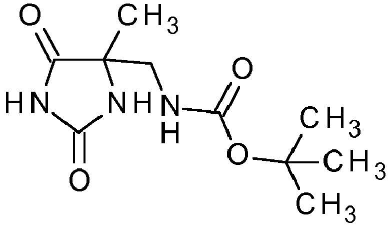

The reaction was performed in 2 Microwave Vials. To a solution of 1.85 g (34.6 mmol) of ammonium chloride and 12.5 g (130 mmol) of ammonium carbonate in 7.5 mL of water was added a solution of 1.50 g (8.66 mmol) of tert-butyl (2-oxopropyl)carbamate in 7.5 ml ethanol. The reaction mixture was stirred 15 minutes at room temperature and then 2.54 g (39.0 mmol) of potassium cyanide were added into the mixture and the vials were sealed. The mixture was stirred at 65 °C for 24 hours. After that time, the solvent was partially removed and the mixture was solved in water and extracted with ethyl acetate. The resulting organic phase was washed with Brine, filtered over an hydrophobic filter and evaporated under vacuo to give 1.69 g (79%) of the product which was used as such in the next step.
¹H-NMR (400 MHz, DMSO-d6) δ [ppm]: -0.008 (0.42), 0.008 (0.40), 1.186 (4.35), 1.371 (16.00), 3.129 (0.51), 3.145 (0.48), 7.626 (0.69).
LC-MS (Method 7): Rₜ = 0.85 min; MS (ESIneg): m/z = 242 [M-H]⁻

### Intermediate 287

### rac-5-(aminomethyl)-5-methylimidazolidine-2,4-dione-hydrogen chloride

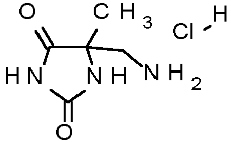

1.70 g (6.95 mmol) of rac-tert-butyl [(4-methyl-2,5-dioxoimidazolidin-4-yl)methyl]carbamate was dissolved in 25 mL of dioxane and 26.05 mL (104.21 mmol) of 4M hydrochloric acid in dioxane were added and the resulting suspension was stirred at room temperature for 16 hours. After that time, the solvent was evaporated and dried under *vacuo* to give 1.18 g (100%) of the product. The compound was used as such in the next step.
¹H-NMR (400 MHz, DMSO-d6) δ [ppm]: 1.343 (16.00), 2.908 (0.74), 2.942 (1.07), 3.074 (1.08), 3.107 (0.75), 8.012 (1.56), 8.215 (1.60), 10.948 (1.58).
LC-MS (Method 9): Rₜ = 0.25 min; MS (ESIpos): m/z = 144 [M+H-HCl]⁺

### Intermediate 288

### tert-butyl {2-oxo-2-[3-(trifluoromethyl)pyridin-2-yl]ethyl}carbamate

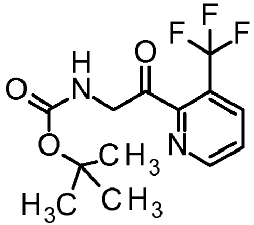

To a solution of 2-amino-1-[3-(trifluoromethyl)pyridin-2-yl]ethanone hydrochloride (2.00 g, 8.31 mmol) in dichloromethane (37 ml) was added di-tert-butyl dicarbonate (2.00 g, 9.14 mmol) and triethylamine (3.5 ml, 25 mmol). After 2 h of stirring at RT, the reaction mixture was diluted with dichloromethane and extracted with water. After phase separation, the organic phase was dried; concentrated in vacuo and 2.30 g (91 % yield) of the title compound was obtained.
LC-MS (Method 9): Rₜ = 1.58 min; MS (ESIpos): m/z = 205 [M-Boc+H]⁺
¹H-NMR (400 MHz, DMSO-d6) δ [ppm]: 1.284 (0.44), 1.322 (1.44), 1.371 (0.52), 1.394 (16.00), 4.481 (1.92), 4.496 (1.89), 7.197 (0.61), 7.842 (0.57), 7.853 (0.62), 7.862 (0.65), 7.874 (0.65), 8.378 (0.80), 8.399 (0.76), 8.939 (0.88), 8.950 (0.89).

### Intermediate 289

### rac-tert-butyl ({2,5-dioxo-4-[3-(trifluoromethyl)pyridin-2-yl]imidazolidin-4-yl}methyl)carbamate

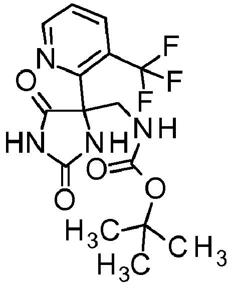

To a solution of tert-butyl {2-oxo-2-[3-(trifluoromethyl)pyridin-2-yl]ethyl}carbamate (2.30 g, 7.56 mmol) in methanol (13.6 ml) was added potassium cyanide (1.97 g, 30.2 mmol) and ammonium carbonate (2.91 g, 30.2 mmol) at RT. The reaction mixture was first stirred overnight at 60°C into a sealed pressure flask. After additional 24 h stirring at 80°C, extra portion of potassium cyanide (738 mg, 11.3 mmol) and ammonium carbonate (1.09 g, 11.3 mmol) were added due to incomplete conversion. The reaction mixture was additionally stirred overnight at 80°C. After filtration at RT, the resulting filtrate was concentrated *in vacuo.* The residue was purified by preparative HPLC (sample preparation: 7.7g dissolved in a mixture of methanol, water, acetonitrile; column: XBridge C18 5µm, 100x30mm; eluent: water→ acetonitrile/water 80:20 + 1% ammonia solution→ acetonitrile; flow rate: 80 ml/min; temperature: 40°C; UV detection: 210 nm). 730 mg of the desired product was obtained and used without further purification.
LC-MS (Method 9): Rₜ = 0.87 min; MS (ESIneg): m/z = 373 [M-H]⁻
¹H-NMR (600 MHz, DMSO-d6) δ [ppm]: 1.368 (16.00), 1.378 (2.03), 1.755 (0.91), 7.624 (0.46), 7.632 (0.55), 7.644 (0.48), 8.240 (0.67), 8.253 (0.66), 8.855 (0.75), 8.862 (0.75).

### Intermediate 290

### rac-5-(aminomethyl)-5-[3-(trifluoromethyl)pyridin-2-yl]imidazolidine-2,4-dione hydrochloride

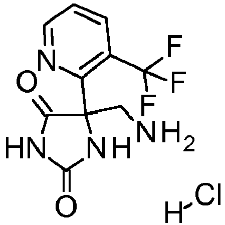

To a solution of rac-tert-butyl ({2,5-dioxo-4-[3-(trifluoromethyl)pyridin-2-yl]imidazolidin-4-yl}methyl)carbamate (725 mg, 1.94 mmol) in dichloromethane (7.6 ml) were added 2.42 ml of 4M hydrochloric acid (9.7 mmol) in dioxane at 0°C. The reaction mixture was stirred for 1h at 0°C then for 2h at room temperature. The precipitate was filtered off, washed with dichloromethane and dried in vacuo. 610 mg of the title compound were obtained and used without further purification.
LC-MS (Method 9): Rₜ = 0.28 min; MS (ESIneg): m/z = 273 [M-HCl-H]⁻
¹H-NMR (600 MHz, DMSO-d6) δ [ppm]: 1.596 (0.55), 1.757 (2.53), 2.501 (16.00), 3.168 (2.76), 3.483 (0.75), 3.493 (0.85), 3.795 (0.96), 3.807 (0.84), 7.745 (1.54), 7.754 (1.68), 7.759 (1.72), 7.767 (1.66), 8.362 (5.66), 8.375 (3.57), 8.708 (3.61), 8.898 (2.56), 8.905 (2.54), 11.399 (3.00).

### Intermediate 291

### ethyl 5-(3,3-difluorocyclobutyl)-1,3-oxazole-4-carboxylate

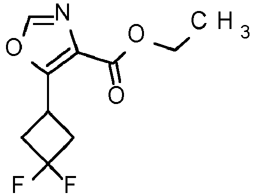

3,3-difluorocyclobutane-1-carboxylic acid (3.00 g, 22.0 mmol) dissolved in 30 ml of THF was treated with 1,1,-carbonyl-diimidazole (4.29 g, 26.5 mmol) and stirred at room temperature. After 1 h, ethyl isocyanoacetate (2.7 ml, 24 mmol), dissolved in 30 ml of THF, and a solution of lithium bis(trimethylsilyl)amide in THF (22 ml, 1.0 M, 22 mmol) were added at 0°C. The mixture was allowed to warm to room temperature, stirred overnight and then concentrated *in vacuo.* The residue was taken up with ethyl acetate and washed with water. The organic layer was dried and concentrated *in vacuo.* The crude product was purified by column chromatography (340 g Ultra Snap Cartridge Biotage; eluent ethyl acetate/ cyclohexane, elution gradient 8%→58%). Samples containing the desired product were united, the solvents were evaporated and the residue was dried in vacuum. 2.50 g (100 % purity, 49 % yield) of the title compound was used without further purification.
LC-MS (Method 9): Rₜ = 1.39 min; MS (ESIpos): m/z = 232 [M+H]⁺
¹H-NMR (400 MHz, DMSO-d6) δ [ppm]: 1.272 (7.71), 1.290 (16.00), 1.308 (7.89), 2.831 (0.57), 2.852 (0.60), 2.864 (0.99), 2.868 (1.15), 2.873 (0.96), 2.884 (1.04), 2.889 (1.24), 2.893 (1.23), 2.901 (1.49), 2.906 (1.08), 2.910 (1.36), 2.921 (1.29), 2.926 (1.03), 2.930 (1.27), 2.943 (0.91), 2.963 (0.87), 3.004 (0.83), 3.013 (0.41), 3.022 (0.97), 3.027 (1.24), 3.031 (0.94), 3.038 (1.36), 3.045 (1.30), 3.061 (1.74), 3.075 (1.07), 3.080 (1.33), 3.088 (0.76), 3.093 (0.89), 3.098 (0.67), 3.116 (0.57), 4.005 (0.79), 4.026 (1.16), 4.032 (1.16), 4.047 (0.74), 4.249 (2.55), 4.267 (7.82), 4.285 (7.74), 4.303 (2.49), 8.450 (6.13).

### Intermediate 292

### 2-amino-1-(3,3-difluorocyclobutyl)ethanone hydrochloride

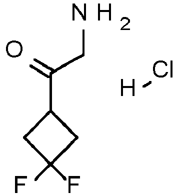

Ethyl 5-(3,3-difluorocyclobutyl)-1,3-oxazole-4-carboxylate (2.50 g, 10.8 mmol) was taken up in 70 ml of 6 N hydrochloric acid and stirred for 2h at 100°C, then overnight at room temperature. The resulting mixture was concentrated *in vacuo.* Due to incomplete conversion, the crude was taken up in 70 ml of 6 N hydrochloric acid, stirred for 2h at 100°C and concentrated *in vacuo.* 1.77 g of the title compound was obtained and used without further purification.
LC-MS (Method 9): Rₜ = 0.82 min; MS (ESIpos): m/z = 150 [M-HCl+H]⁺
¹H-NMR (400 MHz, DMSO-d6) δ [ppm]: -0.008 (1.34), 0.008 (1.24), 1.267 (0.48), 2.329 (0.48), 2.368 (0.73), 2.524 (2.03), 2.671 (0.48), 2.712 (1.34), 2.728 (0.98), 2.744 (4.36), 2.750 (2.01), 2.763 (5.03), 2.768 (5.47), 2.771 (5.05), 2.774 (5.44), 2.782 (7.33), 2.791 (9.26), 2.803 (9.07), 2.812 (7.08), 2.819 (5.74), 2.826 (8.00), 2.834 (4.36), 2.841 (3.64), 2.847 (3.75), 2.858 (1.51), 2.881 (0.78), 3.167 (1.28), 3.295 (0.80), 3.301 (0.82), 3.316 (2.32), 3.323 (2.55), 3.338 (3.25), 3.343 (3.54), 3.361 (2.01), 3.366 (2.03), 3.380 (0.63), 3.386 (0.54), 3.961 (6.24), 3.975 (16.00), 3.989 (15.43), 4.003 (4.82), 8.212 (5.51).

### Intermediate 293

### tert-butyl [2-(3,3-difluorocyclobutyl)-2-oxoethyl]carbamate

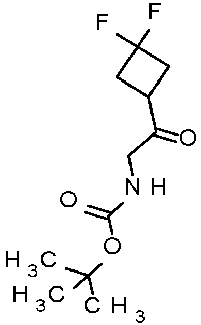

To a solution of 2-amino-1-(3,3-difluorocyclobutyl)ethanone hydrochloride (1.77 g, 9.54 mmol) in dichloromethane (43 ml) was added di-tert-butyl dicarbonate (2.29 g, 10.5 mmol) and triethylamine (4.0 ml, 29 mmol). After 2 h of stirring at RT, the reaction mixture was diluted with dichloromethane and extracted with water. After phase separation, the organic phase was dried; concentrated in vacuo and 2.50 g (88 % purity) of the title compound was obtained and used without further purification.
¹H-NMR (400 MHz, DMSO-d6) δ [ppm]: 1.382 (5.06), 1.468 (16.00), 2.517 (0.48), 3.324 (0.46), 3.789 (0.73), 3.804 (0.73).

### Intermediate 294

### rac-tert-butyl {[4-(3,3-difluorocyclobutyl)-2,5-dioxoimidazolidin-4-yl]methyl}carbamate

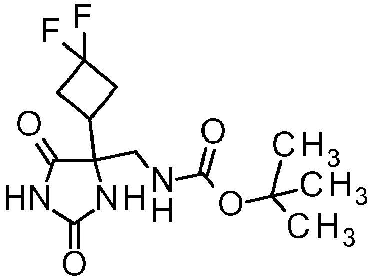

To a solution of tert-butyl [2-(3,3-difluorocyclobutyl)-2-oxoethyl]carbamate (2.50 g, 88 % purity, 8.83 mmol) in methanol (16 ml) was added potassium cyanide (2.30 g, 35.3 mmol) and ammonium carbonate (3.39 g, 35.3 mmol) at RT. The reaction mixture was first stirred overnight at 80°C into a sealed pressure flask. Extra portion of potassium cyanide (1.15 g, 17.7 mmol) and ammonium carbonate (1.70 g, 17.7 mmol) were added due to incomplete conversion. The reaction mixture was additionally stirred overnight at 80°C.

After filtration and washing of the suspension with methanol at RT, the resulting filtrate was concentrated *in vacuo.* The residue was purified by preparative HPLC (sample preparation: 6.4g dissolved in a mixture of methanol, water, acetonitrile; column: XBridge C18 5µm, 100x30mm; eluent: water→ acetonitrile/water 80:20 + 1% ammonia solution→ acetonitrile; flow rate: 80 ml/min; temperature: 40°C; UV detection: 210 nm). 1.68 g of the desired product was obtained and used without further purification.
LC-MS (Method 9): Rₜ = 0.92 min; MS (ESIneg): m/z = 318 [M-H]⁻

### Intermediate 295

### rac-5-(aminomethyl)-5-(3,3-difluorocyclobutyl)imidazolidine-2,4-dione hydrochloride

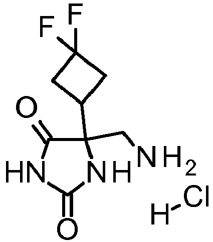

To a solution of rac-tert-butyl {[4-(3,3-difluorocyclobutyl)-2,5-dioxoimidazolidin-4-yl]methyl}carbamate (1.68 g, 5.26 mmol) in dichloromethane (21 ml) were added 6.6 ml of 4M hydrochloric acid (26 mmol) in dioxane at 0°C. The reaction mixture was stirred for 1h at 0°C then for 2h at room temperature. The precipitate was filtered off, washed with dichloromethane and dried in vacuo. 1.46 g of the title compound was obtained and used without further purification.
LC-MS (Method 9): Rₜ = 0.29 min; MS (ESIpos): m/z = 220 [M-HCl+H]⁺
¹H-NMR (600 MHz, DMSO-d6) δ [ppm]: 2.316 (0.52), 2.333 (0.53), 2.347 (0.45), 2.518 (1.08), 2.575 (0.84), 2.587 (1.27), 2.597 (1.19), 2.619 (0.73), 2.641 (0.62), 2.659 (0.75), 2.666 (0.72), 2.674 (0.61), 2.682 (0.51), 2.689 (0.41), 2.910 (1.65), 2.932 (2.01), 3.114 (2.03), 3.137 (1.66), 3.568 (16.00), 8.324 (2.83), 8.366 (3.74), 11.128 (1.25).

### Intermediate 296

### ethyl 5-(1-fluorocyclopropyl)-1,3-oxazole-4-carboxylate

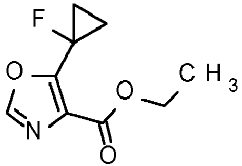

1-fluorocyclopropane-1-carboxylic acid (3.00 g, 28.8 mmol) dissolved in 45 ml of THF was treated with 1,1,-carbonyl-diimidazole (5.61 g, 34.6 mmol) and stirred at room temperature. After 2h, ethyl isocyanoacetate (3.5 ml, 32 mmol), dissolved in 45 ml of THF, and a solution of lithium bis(trimethylsilyl)amide in THF (29 ml, 1.0 M, 29 mmol) were added at 0°C. The mixture was allowed to warm to room temperature, stirred overnight and then concentrated *in vacuo.* The residue was taken up with ethyl acetate and washed with water. The organic layer was dried and concentrated *in vacuo.* The crude product was purified by column chromatography (340 g Ultra Snap Cartridge Biotage; eluent ethyl acetate/ cyclohexane, elution gradient 10%→65%). Samples containing the desired product were united, the solvents were evaporated and the residue was dried in vacuum. 2.84 g (100 % purity, 49 % yield) of the title compound was used without further purification.
LC-MS (Method 7): Rₜ = 1.37 min; MS (ESIpos): m/z = 200 [M+H]⁺
¹H-NMR (500 MHz, DMSO-d6) δ [ppm]: 1.205 (0.54), 1.226 (7.72), 1.240 (16.00), 1.254 (7.77), 1.264 (0.41), 1.308 (0.68), 1.421 (0.81), 1.428 (0.83), 1.439 (1.20), 1.445 (1.15), 1.452 (0.62), 1.456 (1.04), 1.461 (0.66), 1.467 (1.15), 1.475 (1.00), 1.480 (0.83), 1.483 (0.77), 1.490 (0.81), 1.493 (0.80), 1.528 (0.66), 1.531 (0.78), 1.538 (0.85), 1.543 (1.22), 1.548 (0.95), 1.554 (1.08), 1.559 (0.97), 1.563 (0.70), 1.570 (0.53), 1.577 (0.58), 1.579 (0.54), 1.585 (0.51), 1.596 (0.75), 1.603 (0.81), 1.621 (0.40), 1.632 (0.73), 1.639 (0.74), 1.652 (0.71), 1.676 (2.04), 1.679 (2.23), 1.689 (2.12), 1.712 (2.11), 1.715 (2.23), 1.722 (1.82), 1.725 (1.63), 4.220 (1.69), 4.234 (0.92), 4.242 (1.04), 4.249 (0.96), 4.256 (3.51), 4.263 (0.62), 4.269 (5.01), 4.276 (0.59), 4.283 (3.52), 4.290 (0.91), 4.297 (0.97), 4.305 (0.81), 4.307 (0.45), 5.551 (4.54), 5.554 (4.40), 9.124 (1.25).

### Intermediate 297

### 2-amino-1-(1-fluorocyclopropyl)ethanone hydrochloride

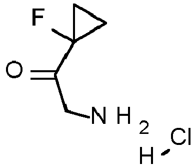

ethyl 5-(1-fluorocyclopropyl)-1,3-oxazole-4-carboxylate (2.84 g, 14.3 mmol) was taken up in 70 ml of 6 N hydrochloric acid and stirred for 2h at 100°C. The resulting mixture was concentrated *in vacuo.* 1.88 g (100 % purity, 86 % yield) of the title compound was obtained and used without further purification.
¹H-NMR (400 MHz, DMSO-d6) δ [ppm]: 1.241 (0.49), 1.402 (2.22), 1.418 (6.53), 1.426 (7.45), 1.439 (9.53), 1.447 (7.17), 1.461 (3.46), 1.485 (0.57), 1.506 (0.48), 1.537 (0.47), 1.582 (3.31), 1.596 (6.52), 1.604 (6.02), 1.620 (2.26), 1.627 (3.14), 1.641 (6.56), 1.650 (6.39), 1.665 (2.14), 4.235 (16.00), 8.356 (5.64).

### Intermediate 298

### tert-butyl [2-(1-fluorocyclopropyl)-2-oxoethyl]carbamate

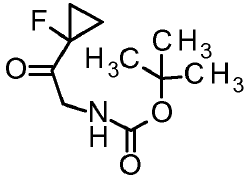

To a solution of 2-amino-1-(1-fluorocyclopropyl)ethanone hydrochloride (1.88 g, 12.2 mmol) in dichloromethane (55 ml) was added di-tert-butyl dicarbonate (2.94 g, 13.5 mmol) and triethylamine (5.1 ml, 37 mmol). After 1.5 h of stirring at RT, the reaction mixture was washed with water. After phase separation, the organic phase was dried; concentrated in vacuo and 2.48 g of the title compound was obtained and used without further purification.
LC-MS (Method 9): Rₜ = 1.39 min; MS (ESIpos): m/z = 218 [M+H]⁺

### Intermediate 299

### rac-tert-butyl {[4-(1-fluorocyclopropyl)-2,5-dioxoimidazolidin-4-yl]methyl}carbamate

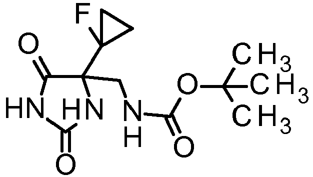

To a solution of tert-butyl [2-(1-fluorocyclopropyl)-2-oxoethyl]carbamate (2.46 g, 11.3 mmol) in methanol (20 ml) was added potassium cyanide (2.95 g, 45.3 mmol) and ammonium carbonate (4.35 g, 45.3 mmol) at RT. The reaction mixture was stirred overnight at 80°C into a sealed pressure flask. After filtration at RT, the resulting filtrate was concentrated *in vacuo.* The residue was purified by preparative HPLC (sample preparation: 7.4g dissolved in a mixture of methanol, water, acetonitrile; column: XBridge C18 5µm, 100x30mm; eluent: water→ acetonitrile/water 80:20 + 1% ammonia solution → acetonitrile; flow rate: 80 ml/min; temperature: 40°C; UV detection: 220 nm). 2.31 g of the desired product was obtained and used without further purification
LC-MS (Method 9): Rₜ = 0.65 min; MS (ESIneg): m/z = 286 [M-H]⁻

### Intermediate 300

### rac-5-(aminomethyl)-5-(1-fluorocyclopropyl)imidazolidine-2,4-dione hydrochloride

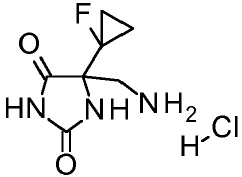

To a solution of rac-tert-butyl {[4-(1-fluorocyclopropyl)-2,5-dioxoimidazolidin-4-yl]methyl}carbamate (1.20 g, 4.18 mmol) in dichloromethane (36 ml) were added 5.2 ml (21 mmol) of 4M hydrochloric acid in dioxane at RT. The reaction mixture was stirred for 3 days and then concentrated in vacuo. 1.04 g of the title compound was used without further purification.
LC-MS (Method 9): Rₜ = 0.25 min; MS (ESIpos): m/z = 188 [M-HCl+H]⁺
¹H-NMR (600 MHz, DMSO-d6) δ [ppm]: 0.814 (0.66), 0.833 (2.66), 0.842 (2.91), 0.847 (3.07), 0.851 (3.10), 0.859 (2.45), 0.869 (2.25), 0.892 (0.51), 0.965 (0.55), 0.999 (0.64), 1.020 (2.80), 1.039 (5.31), 1.043 (5.25), 1.048 (3.48), 1.060 (9.17), 1.076 (2.31), 1.081 (3.58), 1.091 (5.77), 1.109 (1.82), 1.120 (0.63), 1.182 (1.41), 1.194 (2.97), 1.206 (1.66), 1.368 (11.79), 1.757 (5.46), 2.117 (2.39), 3.109 (7.54), 3.132 (9.02), 3.347 (8.25), 3.568 (4.33), 3.864 (1.61), 3.874 (1.62), 4.084 (0.44), 4.096 (1.31), 4.108 (1.33), 4.120 (0.50), 7.902 (0.50), 8.098 (0.91), 8.444 (16.00), 8.474 (9.62), 11.159 (4.19).

### Intermediate 301

### ethyl 5-(5-cyclopropyl-1,3-thiazol-4-yl)-1,3-oxazole-4-carboxylate

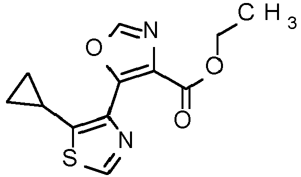

5-cyclopropyl-1,3-thiazole-4-carboxylic acid (2.00 g, 11.8 mmol) dissolved in 30 ml of THF was treated with 1,1,-carbonyl-diimidazole (2.30 g, 14.2 mmol) and stirred at room temperature. After 1h, ethyl isocyanoacetate (1.4 ml, 13 mmol), dissolved in 20 ml of THF, and a solution of lithium bis(trimethylsilyl)amide in THF (12 ml, 1.0 M, 12 mmol) were added at 0°C. The mixture was allowed to warm to room temperature, stirred overnight and then concentrated *in vacuo.* The residue was taken up with ethyl acetate and washed with water. The organic layer was dried and concentrated *in vacuo.* The crude product was purified by column chromatography (50 g Ultra Snap Cartridge Biotage; eluent ethyl acetate/ cyclohexane, elution gradient 10%→100%). Samples containing the desired product were united, the solvents were evaporated and the residue was dried in vacuum. 2.30 g (100 % purity, 74 % yield) of the title compound was used without further purification.
LC-MS (Method 7): Rₜ = 1.37 min; MS (ESIpos): m/z = 265 [M+H]⁺
¹H-NMR (600 MHz, DMSO-d6) δ [ppm]: 0.614 (1.40), 0.622 (5.17), 0.625 (4.35), 0.630 (4.44), 0.633 (5.10), 0.641 (1.42), 1.077 (1.50), 1.084 (4.03), 1.088 (4.16), 1.098 (4.20), 1.101 (4.01), 1.109 (1.32), 1.162 (7.86), 1.174 (16.00), 1.186 (7.91), 1.989 (2.00), 2.046 (0.62), 2.054 (1.27), 2.060 (1.37), 2.068 (2.39), 2.076 (1.30), 2.082 (1.16), 2.090 (0.56), 4.026 (0.49), 4.038 (0.48), 4.198 (2.59), 4.209 (7.79), 4.221 (7.66), 4.233 (2.49), 8.623 (9.20), 8.975 (8.85).

### Intermediate 302

### 2-amino-1-(5-cyclopropyl-1,3-thiazol-4-yl)ethanone hydrochloride

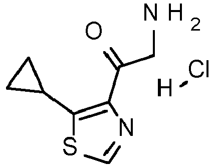

ethyl 5-(5-cyclopropyl-1,3-thiazol-4-yl)-1,3-oxazole-4-carboxylate (2.30 g, 8.70 mmol) was taken up in 43 ml of 6 N hydrochloric acid and stirred for 2h at 100°C. The resulting mixture was concentrated *in vacuo.* 1.99 g (100 % purity) of the title compound was obtained and used without further purification.
LC-MS (Method 9): Rₜ = 1.15 min; MS (ESIpos): m/z = 183 [M-HCl+H]⁺

### Intermediate 303

### tert-butyl [2-(5-cyclopropyl-1,3-thiazol-4-yl)-2-oxoethyl]carbamate

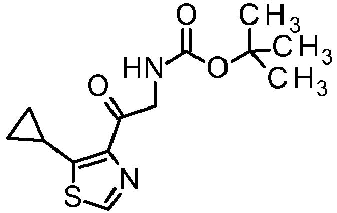

To a solution of 2-amino-1-(5-cyclopropyl-1,3-thiazol-4-yl)ethanone hydrochloride (1.99 g, 9.10 mmol) in dichloromethane (41 ml) was added di-tert-butyl dicarbonate (2.18 g, 10.0 mmol) and triethylamine (7.6 ml, 55 mmol). After 2 h of stirring at RT, the reaction mixture was washed with water. After phase separation, the organic phase was dried; concentrated in vacuo and 2.44 g of the title compound was obtained and used without further purification.
LC-MS (Method 9): Rₜ = 1.58 min; MS (ESIpos): m/z = 283 [M+H]⁺
¹H-NMR (400 MHz, DMSO-d6) δ [ppm]: 0.724 (1.35), 0.729 (1.19), 0.741 (1.36), 0.752 (0.43), 1.109 (0.60), 1.268 (0.47), 1.279 (1.24), 1.285 (1.29), 1.300 (1.80), 1.306 (2.06), 1.316 (0.84), 1.364 (0.70), 1.388 (1.53), 1.402 (16.00), 1.467 (8.35), 3.129 (0.61), 4.394 (1.90), 4.409 (1.87), 6.976 (0.58), 8.892 (1.62).

### Intermediate 304

### rac-tert-butyl {[4-(5-cyclopropyl-1,3-thiazol-4-yl)-2,5-dioxoimidazolidin-4-yl]methyl}carbamate

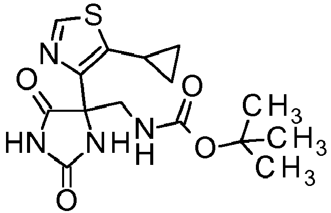

To a solution of tert-butyl [2-(5-cyclopropyl-1,3-thiazol-4-yl)-2-oxoethyl]carbamate (2.44 g, 8.64 mmol) in methanol (20 ml) was added potassium cyanide (2.25 g, 34.6 mmol) and ammonium carbonate (3.32 g, 34.6 mmol) at RT. The reaction mixture was stirred for 5 hours at 60°C, followed by 48 h at RT. into a sealed pressure flask. After additional 24h stirring at 80°C, the reaction mixture was filtered at RT and the resulting filtrate was concentrated *in vacuo.*

The residue was purified by preparative HPLC (sample preparation: 5.6g dissolved in acetonitrile; column: XBridge C18 5µm, 100x30mm; eluent: water→ acetonitrile/water 80:20 + 1% ammonia solution→ acetonitrile; flow rate: 80 ml/min; temperature: 40°C; UV detection: 210 nm). 1.98 g of the desired product was obtained and used without further purification.
LC-MS (Method 7): Rt = 1.22 min; MS (ESIpos): m/z = 353 [M+H]⁺

### Intermediate 305

### rac-5-(aminomethyl)-5-(5-cyclopropyl-1,3-thiazol-4-yl)imidazolidine-2,4-dione hydrochloride

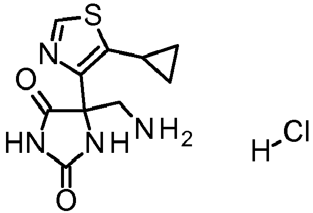

To a solution of rac-tert-butyl {[4-(5-cyclopropyl-1,3-thiazol-4-yl)-2,5-dioxoimidazolidin-4-yl]methyl}carbamate (500 mg, 1.42 mmol) in dichloromethane (12 ml) were added 1.8 ml of 4M hydrochloric acid (7.1 mmol) in dioxane at RT. The reaction mixture was stirred for 1h at room temperature, after which extra portion of 4M hydrochloric acid (180 µl, 710 µmol) was added due to incomplete conversion. After stirring for 1 at RT, the precipitate was filtered off, washed with dichloromethane and dried in vacuo. 435 mg of the title compound was obtained and used without further purification.
LC-MS (Method 9): Rₜ = 0.32 min; MS (ESIpos): m/z = 253 [M-HCl+H]⁺
¹H-NMR (600 MHz, DMSO-d6) δ [ppm]: 0.005 (0.77), 0.553 (0.63), 0.562 (1.05), 0.570 (1.98), 0.576 (1.14), 0.578 (2.43), 0.584 (1.74), 0.587 (1.07), 0.593 (0.85), 0.719 (0.75), 0.725 (1.02), 0.728 (1.68), 0.734 (2.45), 0.742 (2.06), 0.751 (1.31), 0.759 (0.76), 1.014 (0.53), 1.021 (0.52), 1.024 (0.48), 1.029 (1.40), 1.036 (1.57), 1.039 (1.34), 1.045 (1.89), 1.049 (1.86), 1.054 (2.04), 1.058 (2.06), 1.063 (1.84), 1.068 (1.52), 1.071 (1.57), 1.078 (1.43), 1.083 (0.58), 1.086 (0.55), 1.093 (0.53), 1.596 (0.75), 1.910 (0.78), 1.919 (1.58), 1.924 (1.69), 1.927 (0.97), 1.932 (3.13), 1.938 (1.02), 1.941 (1.62), 1.946 (1.54), 1.955 (0.75), 2.523 (0.42), 2.573 (0.85), 3.058 (0.68), 3.168 (12.10), 3.568 (0.84), 3.615 (0.43), 3.626 (0.72), 3.637 (1.65), 3.647 (1.89), 3.653 (1.97), 3.662 (1.71), 3.684 (0.47), 8.368 (3.59), 8.628 (4.46), 8.862 (16.00), 11.327 (3.71).

### Intermediate 306

### methyl 5-(2,5-dimethyl-1,3-thiazol-4-yl)-1,3-oxazole-4-carboxylate

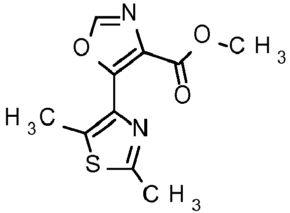

2,5-dimethyl-1,3-thiazole-4-carboxylic acid (3.00 g, 19.1 mmol) dissolved in 30 ml of THF was treated with 1,1,-carbonyl-diimidazole (3.71 g, 22.9 mmol) and stirred at room temperature. After 1h, ethyl isocyanoacetate (2.3 ml, 21 mmol), dissolved in 30 ml of THF, and a solution of lithium bis(trimethylsilyl)amide in THF (19 ml, 1.0 M, 19 mmol) were added at 0°C. The mixture was allowed to warm to room temperature, stirred for 2 days and then concentrated *in vacuo.* The residue was taken up with ethyl acetate and washed with water. The organic layer was dried and concentrated *in vacuo.* The crude product (ethyl ester derivative) was diluted in 50 ml of methanol; Isolute® was added, and the solvent was evaporated on a rotary evaporator at higher temperature. The crude was then purified by column chromatography (340 g Ultra Snap Cartridge Biotage; eluent ethyl acetate/ cyclohexane, elution gradient 15%→100%). Samples containing the desired product were united, the solvents were evaporated and the residue was dried in vacuum. 2.67 g (100 % purity, 59 % yield) of the title compound was used without further purification.
LC-MS (Method 7): Rₜ = 1.11 min; MS (ESIpos): m/z = 238 [M+H]⁺
¹H-NMR (400 MHz, DMSO-d6) δ [ppm]: 2.401 (13.72), 2.673 (12.80), 3.805 (16.00), 8.602 (2.93).

### Intermediate 307

### 2-amino-1-(2,5-dimethyl-1,3-thiazol-4-yl)ethanone hydrochloride

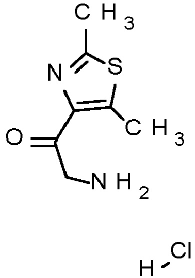

methyl 5-(2,5-dimethyl-1,3-thiazol-4-yl)-1,3-oxazole-4-carboxylate (2.67 g, 11.2 mmol) was taken up in 60 ml of 6 N hydrochloric acid and stirred for 2h at 100°C. The resulting mixture was concentrated *in vacuo.* 2.90 g of the title compound was obtained and used without further purification.
LC-MS (Method 9): Rₜ = 0.77 min; MS (ESIpos): m/z = 171 [M+-HCl+H]⁺

### Intermediate 308

### tert-butyl [2-(2,5-dimethyl-1,3-thiazol-4-yl)-2-oxoethyl]carbamate

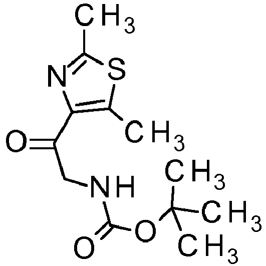

To a solution of 2-amino-1-(2,5-dimethyl-1,3-thiazol-4-yl)ethanone hydrochloride (2.90 g, 14.0 mmol) in dichloromethane (80 ml) was added di-tert-butyl dicarbonate (2.86 g, 13.1 mmol) and triethylamine (13 ml, 95 mmol). After 2 h of stirring at RT, the reaction mixture was washed with water. After phase separation, the organic phase was dried; concentrated in vacuo and 3.17 g of the title compound was obtained and used without further purification.
LC-MS (Method 9): Rₜ = 1.38 min; MS (ESIpos): m/z = 271 [M+H]⁺
¹H-NMR (600 MHz, DMSO-d6) δ [ppm]: 1.111 (2.20), 1.275 (0.74), 1.367 (0.75), 1.394 (16.00), 1.412 (0.44), 2.500 (4.08), 2.607 (9.87), 2.663 (9.51), 4.143 (1.81), 4.153 (1.78), 7.156 (0.63).

### Intermediate 309

### rac-tert-butyl {[4-(2,5-dimethyl-1,3-thiazol-4-yl)-2,5-dioxoimidazolidin-4-yl]methyl}carbamate

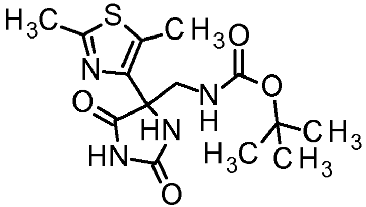

To a solution of tert-butyl [2-(2,5-dimethyl-1,3-thiazol-4-yl)-2-oxoethyl]carbamate (3.17 g, 11.7 mmol) in methanol (21 ml) was added potassium cyanide (3.05 g, 46.9 mmol) and ammonium carbonate (4.51 g, 46.9 mmol) at RT. The reaction mixture was first stirred overnight at 60°C into a sealed pressure flask. Extra portion of potassium cyanide (1.07 g, 16.4 mmol) and ammonium carbonate (1.58 g, 16.4 mmol) were added due to incomplete conversion. The reaction mixture was first stirred for 7h at 60°C, then for 48h at RT.

After filtration and washing of the suspension with methanol at RT, the resulting filtrate was concentrated *in vacuo.* The residue was purified by preparative HPLC (sample preparation: 5.4g dissolved in acetonitrile; column: XBridge C18 5µm, 100x30mm; eluent: water→ acetonitrile/water 80:20 + 1% ammonia solution → acetonitrile; flow rate: 80 ml/min; temperature: 45°C; UV detection: 210 nm). 1.22 g of the desired product was obtained and used without further purification.
LC-MS (Method 7): Rₜ = 1.09 min; MS (ESIpos): m/z = 341 [M+H]⁺

### Intermediate 310

### rac-5-(aminomethyl)-5-(2,5-dimethyl-1,3-thiazol-4-yl)imidazolidine-2,4-dione hydrochloride

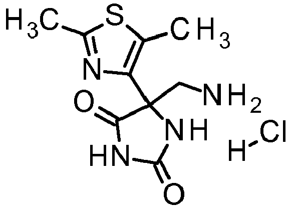

To a solution rac-tert-butyl {[4-(2,5-dimethyl-1,3-thiazol-4-yl)-2,5-dioxoimidazolidin-4-yl]methyl}carbamate (300 mg, 881 µmol) in dichloromethane (3.5 ml) were added 1.1 ml of 4M hydrochloric acid (4.4 mmol) in dioxane at RT. After stirring for 1 at RT, the precipitate was filtered off, washed with dichloromethane and dried in vacuo. 243 mg of the title compound was obtained and used without further purification.
LC-MS (Method 9): Rₜ = 0.24 min; MS (ESIpos): m/z = 241 [M-HCl+H]⁺
¹H-NMR (400 MHz, DMSO-d6) δ [ppm]: -0.008 (2.30), 0.008 (2.40), 1.110 (0.56), 1.596 (1.42), 1.757 (2.42), 2.074 (2.42), 2.273 (0.40), 2.368 (0.52), 2.418 (12.62), 2.525 (1.42), 2.567 (16.00), 2.712 (0.47), 3.168 (8.55), 3.403 (0.64), 3.568 (1.07), 3.579 (0.83), 3.600 (0.74), 8.432 (1.64), 8.931 (0.84), 11.379 (2.14).

### Intermediate 311

### ethyl 5-(1-chlorocyclopropyl)-1,3-oxazole-4-carboxylate

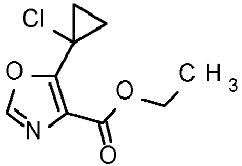

1-chlorocyclopropane-1-carboxylic acid (4.12 g, 34.2 mmol) dissolved in 50 ml of THF was treated with 1,1,-carbonyl-diimidazole (6.65 g, 41.0 mmol) and stirred at room temperature. After 2h, ethyl isocyanoacetate (4.1 ml, 38 mmol), dissolved in 50 ml of THF, and a solution of lithium bis(trimethylsilyl)amide in THF (34 ml, 1.0 M, 34 mmol) were added at 0°C. The mixture was allowed to warm to room temperature, stirred overnight and then concentrated *in vacuo.* The residue was taken up with ethyl acetate and washed with water. The organic layer was dried and concentrated *in vacuo.* The crude product was purified by column chromatography (340 g Ultra Snap Cartridge Biotage; eluent ethyl acetate/ cyclohexane, elution gradient 10%→100%). Samples containing the desired product were united, the solvents were evaporated and the residue was dried in vacuum. 3.38 g of the title compound was used without further purification.
LC-MS (Method 7): Rₜ = 1.50 min; MS (ESIpos): m/z = 216 [M+H]⁺
¹H-NMR (400 MHz, DMSO-d6) δ [ppm]: -0.008 (0.64), 0.008 (0.67), 1.314 (7.63), 1.332 (16.00), 1.350 (7.76), 1.533 (3.46), 1.540 (13.18), 1.543 (13.20), 1.550 (3.34), 2.524 (0.43), 4.298 (2.49), 4.315 (7.69), 4.333 (7.55), 4.351 (2.42), 8.489 (5.44).

### Intermediate 312

### 2-amino-1-(1-chlorocyclopropyl)ethanone hydrochloride

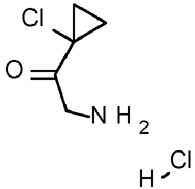

ethyl 5-(1-chlorocyclopropyl)-1,3-oxazole-4-carboxylate (3.38 g, 15.7 mmol) was taken up in 77 ml of 6 N hydrochloric acid and stirred for 2h at 100°C. The resulting mixture was concentrated *in vacuo.* 2.56 g of the title compound was obtained and used without further purification.
LC-MS (Method 9): Rₜ = 0.82 min; MS (ESIpos): m/z = 134 [M-HCl+H]⁺
¹H-NMR (600 MHz, DMSO-d6) δ [ppm]: 1.253 (0.53), 1.574 (4.17), 1.583 (12.73), 1.589 (12.55), 1.597 (5.57), 1.625 (0.64), 1.721 (0.61), 1.749 (5.48), 1.758 (12.77), 1.763 (12.59), 1.773 (4.38), 3.652 (0.41), 4.187 (16.00), 7.297 (1.22), 7.381 (1.40), 7.466 (1.22), 8.403 (5.29).

### Intermediate 313

### tert-butyl [2-(1-chlorocyclopropyl)-2-oxoethyl]carbamate

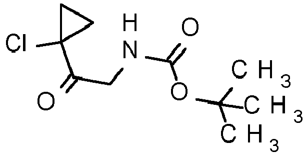

To a solution of 2-amino-1-(1-chlorocyclopropyl)ethanone hydrochloride (2.56 g, 15.1 mmol) in dichloromethane (100 ml) was added di-tert-butyl dicarbonate (3.61 g, 16.6 mmol) and triethylamine (17 ml, 120 mmol). After 2 h of stirring at RT, the reaction mixture was washed with water. After phase separation, the organic phase was dried; concentrated in vacuo and 3.22 g of the title compound was obtained and used without further purification.
¹H-NMR (600 MHz, DMSO-d6) δ [ppm]: 1.015 (0.44), 1.113 (1.18), 1.327 (0.64), 1.340 (0.71), 1.368 (4.07), 1.377 (9.45), 1.390 (0.93), 1.425 (0.51), 1.449 (0.50), 1.458 (1.39), 1.463 (1.83), 1.470 (16.00), 1.631 (0.53), 1.639 (1.01), 1.644 (0.90), 2.501 (1.09), 4.091 (1.08), 4.101 (1.07).

### Intermediate 314

### rac-tert-butyl {[4-(1-chlorocyclopropyl)-2,5-dioxoimidazolidin-4-yl]methyl}carbamate

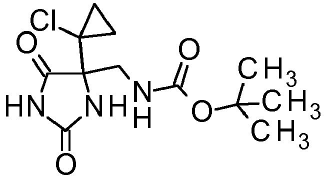

To a solution of tert-butyl [2-(1-chlorocyclopropyl)-2-oxoethyl]carbamate (3.20 g, 13.7 mmol) in methanol (12 ml) was added potassium cyanide (3.57 g, 54.8 mmol) and ammonium carbonate (5.26 g, 54.8 mmol) at RT. The reaction mixture was stirred for 6 hours at 80°C, followed by 48 h at 40°C. into a sealed pressure flask. After filtration and washing of the suspension with methanol at RT, the resulting filtrate was concentrated *in vacuo.* The residue was purified by preparative HPLC (sample preparation: 5.14g dissolved in acetonitrile; column: XBridge C18 5µm, 100x30mm; eluent: water→ acetonitrile/water 80:20 + 1% ammonia solution→ acetonitrile; flow rate: 80 ml/min; temperature: 40°C; UV detection: 210 nm). 342 mg of the desired product was obtained and used without further purification.
LC-MS (Method 9): Rₜ = 0.72 min; MS (ESIneg): m/z = 302 [M-H]⁻
¹H-NMR (400 MHz, DMSO-d6) δ [ppm]: 1.007 (0.66), 1.025 (0.99), 1.153 (0.56), 1.165 (1.09), 1.362 (16.00), 1.409 (0.62), 7.833 (0.77).

### Intermediate 315

### rac-5-(aminomethyl)-5-(1-chlorocyclopropyl)imidazolidine-2,4-dione hydrochloride

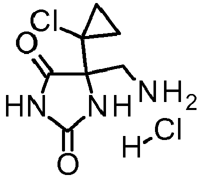

To a solution of *rac*-tert-butyl {[4-(1-chlorocyclopropyl)-2,5-dioxoimidazolidin-4-yl]methyl}carbamate (340 mg, 1.12 mmol) in dichloromethane (10 ml) were added 1.4 ml of 4M hydrochloric acid (5.6 mmol) in dioxane at RT. The reaction mixture was stirred for 2h at room temperature and concentrated in vacuo. The crude was again diluted in 10 ml DCM after which extra portion of 4M hydrochloric acid (1.4 ml, 4.0 M, 5.6 mmol) was added due to incomplete conversion. After stirring overnight at RT, the precipitate was filtered off, washed with dichloromethane and dried in vacuo. 256 mg of the title compound was obtained and used without further purification.
LC-MS (Method 9): Rₜ = 0.27 min; MS (ESIpos): m/z = 204 [M-HCl+H]⁺

### Intermediate 316

### Ethyl (2E)-2-[2-(4-chloro-2-fluorophenyl)hydrazinylidene]-3-oxopropanoate

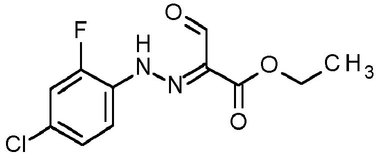

4-chloro-2-fluoroaniline (190 µl, 1.7 mmol) was dissolved in 2.5 ml of water and 470 µl of concentrated hydrochloric acid. The mixture was cooled to 0°C and a solution of sodium nitrite (118 mg, 1.72 mmol) in 1.25 ml of water was added dropwise. After stirring for 5 minutes this solution was added dropwise to a solution of ethyl-(2E)-3-(dimethylamino)acrylate (270 µl, 1.9 mmol) and potassium acetate (253 mg, 2.58 mmol) in 3.75 ml of ethanol at 0°C. The mixture was stirred at room temperature for 30 min before being diluted with ethyl acetate and washed twice with water and once with brine. The organic layer was dried over sodium sulfate, filtered and evaporated. 414 mg (78 % purity, 69 % yield) of the title compound were obtained.
LC-MS (Method 7): Rₜ = 2.13 min; MS (ESIneg): m/z = 271 [M-H]⁻

### Intermediate 317

### Ethyl (2E,3E)-2-[2-(4-chloro-2-fluorophenyl)hydrazinylidene]-3-(hydroxyimino)propanoate

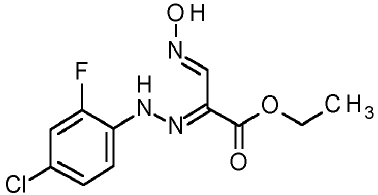

Ethyl (2E)-2-[2-(4-chloro-2-fluorophenyl)hydrazinylidene]-3-oxopropanoate (404 mg, 78 % purity, 1.16 mmol), hydroxylamine hydrochloride (96.4 mg, 1.39 mmol) and potassium acetate (284 mg, 2.89 mmol) were dissolved in 7.0 ml of ethanol and the mixture was stirred at room temperature over night. The reaction mixture was diluted with ethyl acetate and washed twice with water and once with brine. The organic layer was dried over sodium sulfate, filtered and evaporated. The residue was dried in vacuum. 349 mg (77 % purity, 81 % yield) of the title compound were obtained.
LC-MS (Method 7): Rₜ = 2.00 min; MS (ESIpos): m/z = 288 [M+H]⁺

### Intermediate 318

### Ethyl 2-(4-chloro-2-fluorophenyl)-2H-1,2,3-triazole-4-carboxylate

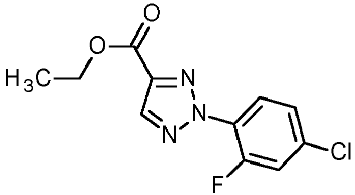

Ethyl (2E,3E)-2-[2-(4-chloro-2-fluorophenyl)hydrazinylidene]-3-(hydroxyimino)propanoate (348 mg, 77 % purity, 931 µmol) was stirred in 3.5 ml of acetic acid anhydride at 60°C over night. The reaction was then diluted in ethyl acetate and washed twice with water and once with brine. The organic layer was dried over sodium sulfate, filtered and evaporated together with Isolute®. The residue was purified by chromatography on silica gel (25 g Ultra Snap Cartridge Biotage®; Biotage-Isolera-One®; Cy/EE-gradient: 2% EE - 20% EE; flow: 75 ml/min). Samples containing the desired product were united, the solvents were evaporated and the residue was dried in vacuum. 173 mg (85 % purity, 59 % yield) of the title compound were obtained.
LC-MS (Method 7): Rₜ = 2.02 min; MS (ESIpos): m/z = 270 [M+H]⁺

### Intermediate 319

### 2-(4-chloro-2-fluorophenyl)-2H-1,2,3-triazole-4-carboxylic acid

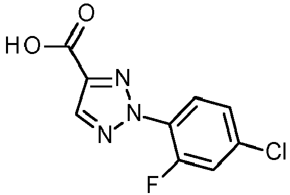

Ethyl 2-(4-chloro-2-fluorophenyl)-2H-1,2,3-triazole-4-carboxylate (173 mg, 85 % purity, 549 µmol) was dissolved in 12 ml of THF, treated with lithium hydroxide solution (550 µl, 1.0 M, 550 µmol) and the mixture was stirred for 2 h at room temperature. More lithium hydroxide solution (550 µl, 1.0 M, 550 µmol) was added and the mixture was stirred 1 h at room temperature. The solvent was removed on a rotary evaporator. Water was added and the mixture was acidified with 2 N hydrochloric acid. The resulting precipitate was collected by filtration. 138 mg (95 % purity, 98 % yield) of the title compound were obtained.
LC-MS (Method 7): Rₜ = 1.36 min; MS (ESIpos): m/z = 242 [M+H]⁺

### Intermediate 320

### Ethyl (2E)-2-[2-(2,4-dichlorophenyl)hydrazinylidene]-3-oxopropanoate

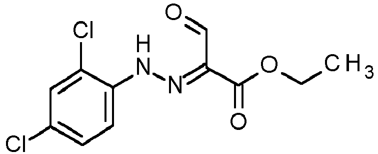

2,4-dichloroaniline (250 mg, 1.52 mmol) was dissolved in 2.5 ml of water and 470 µl of concentrated hydrochloric acid. The mixture was cooled to 0°C and a solution of sodium nitrite (105 mg, 1.52 mmol) in 1.25 ml of water was added dropwise. After stirring for 5 minutes this solution was added dropwise to a solution of ethyl-(2E)-3-(dimethylamino)acrylate (240 µl, 1.7 mmol) and potassium acetate (224 mg, 2.29 mmol) in 3.75 ml of ethanol at 0°C. The mixture was stirred at room temperature for 30 min before being diluted with ethyl acetate and washed twice with water and once with brine. The organic layer was dried over sodium sulfate, filtered and evaporated. 264 mg (54 % purity, 33 % yield) of the title compound were obtained.
LC-MS (Method 9): Rₜ = 1.96 min; MS (ESIpos): m/z = 289 [M+H]⁺

### Intermediate 321

### Ethyl (2E,3E)-2-[2-(2,4-dichlorophenyl)hydrazinylidene]-3-(hydroxyimino)propanoate

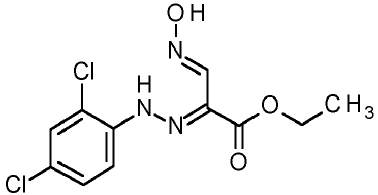

Ethyl (2E)-2-[2-(2,4-dichlorophenyl)hydrazinylidene]-3-oxopropanoate (264 mg, 54 % purity, 495 µmol), hydroxylamine hydrochloride (41.3 mg, 595 µmol) and potassium acetate (122 mg, 1.24 mmol) were dissolved in 3 ml of ethanol and the mixture was stirred at room temperature over night. The reaction mixture was diluted with ethyl acetate and washed twice with water and once with brine. The organic layer was dried over sodium sulfate, filtered and evaporated. The residue was dried in vacuum. 239 mg (89 % purity, 141 % yield) of the title compound were obtained.
LC-MS (Method 7): Rₜ = 2.11 min; MS (ESIpos): m/z = 304 [M+H]⁺

### Intermediate 322

### Ethyl 2-(2,4-dichlorophenyl)-2H-1,2,3-triazole-4-carboxylate

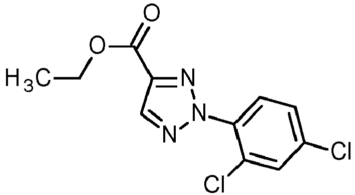

Ethyl (2E,3E)-2-[2-(2,4-dichlorophenyl)hydrazinylidene]-3-(hydroxyimino)propanoate (238 mg, 89 % purity, 696 µmol) was stirred in 4 ml of acetic acid anhydride at 60°C over night. The reaction was then diluted in ethyl acetate and washed twice with water and once with brine. The organic layer was dried over sodium sulfate, filtered and evaporated together with Isolute®. The residue was purified by chromatography on silica gel (25 g Ultra Snap Cartridge Biotage®; Biotage-Isolera-One®; Cy/EE-gradient: 2% EE - 20% EE; flow: 75 ml/min). Samples containing the desired product were united, the solvents were evaporated and the residue was dried in vacuum. 118 mg (75 % purity, 44 % yield) of the title compound were obtained.
LC-MS (Method 7): Rₜ = 2.15 min; MS (ESIpos): m/z = 286 [M+H]⁺

### Intermediate 323

### 2-(2,4-dichlorophenyl)-2H-1,2,3-triazole-4-carboxylic acid

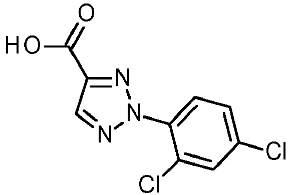

Ethyl 2-(2,4-dichlorophenyl)-2H-1,2,3-triazole-4-carboxylate (117 mg, 75 % purity, 307 µmol) was dissolved in 5 ml of THF, treated with lithium hydroxide solution (310 µl, 1.0 M, 310 µmol) and the mixture was stirred for 1 h at room temperature. The solvent was removed on a rotary evaporator. Water was added an the mixture was acidified with 2 N hydrochloric acid. The resulting precipitate was collected by filtration. 86.0 mg (100 % purity, 109 % yield) of the title compound were obtained.
LC-MS (Method 7): Rₜ = 1.52 min; MS (ESIneg): m/z = 255 [M-H]⁻

### Intermediate 324

### Ethyl 5-ethyl-1,3-oxazole-4-carboxylate

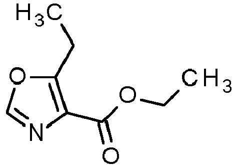

Propanoic acid (1.00 g, 13.5 mmol) dissolved in 15 ml of THF was treated with 1,1,-carbonyl-diimidazole (2.63 g, 16.2 mmol) and stirred at room temperature. After 2 h ethyl isocyanoacetate (1.6 ml, 15 mmol), dissolved in 10 ml of THF, and a solution of lithium bis(trimethylsilyl)amide in THF (13 ml, 1.0 M, 13 mmol) were added at 0°C. The mixture was allowed to warm to room temperature and stirred over night. The solvent was removed on a rotary evaporator. The residue was taken up with ethyl acetate and washed with water and saturated aqueous sodium chloride solution. The organic layer was dried over sodium sulfate, filtered, Isolute® was added and the solvent was evaporated on a rotary evaporator. The crude product was purified by column chromatography (Machine: Biotage® Isolera One; column: Biotage® SNAP Ultra 50 g; gradient: Cy/EE-gradient, 8% EE - 80% EE; flow: 100 ml/min). Product containing samples were united and the solvents were removed on a rotary evaporator. 1.40 g (97 % purity, 59 % yield) of the title compound were obtained.
LC-MS (Method 7): Rₜ = 1.30 min; MS (ESIpos): m/z = 170 [M+H]⁺

### Intermediate 325

### 1-aminobutan-2-one hydrogen chloride

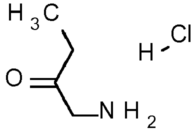

Ethyl 5-ethyl-1,3-oxazole-4-carboxylate (1.40 g, 8.28 mmol) was taken up in 28 ml of 6 N hydrochloric acid and stirred at 100°C. After 4 h the solvent was removed on a rotary evaporator and the residue was dried in vacuo. 0.99 g (97 % yield) of the title compound were obtained.

### Intermediate 326

### tert-butyl (2-oxobutyl)carbamate

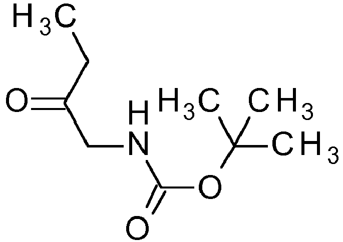

1-aminobutan-2-one-hydrogen chloride (990 mg, 8.01 mmol) dissolved in 30 ml of dichloromethane was treated with di-tert-butyl dicarbonate (2.0 ml, 8.8 mmol) and triethylamine (3.3 ml, 24 mmol). The mixture was stirred at room temperature over night. The solvent was removed on a rotary evaporator. The residue was taken up with ethyl acetate and washed with water and saturated aqueous sodium chloride solution. The organic layer was dried over sodium sulfate, filtered and evaporated on a rotary evaporator. 1.2 g (80 % yield) of the title compound were obtained.

### Intermediate 327

### rac-tert-butyl [(4-ethyl-2,5-dioxoimidazolidin-4-yl)methyl]carbamate

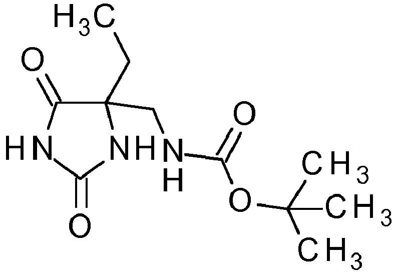

Ammonium carbonate (6.16 g, 64.1 mmol) and ammonium chloride (1.37 g, 25.6 mmol) were dissolved in 10 ml of water. Tert-butyl (2-oxobutyl)carbamate (1.20 g, 6.41 mmol), dissolved in 10 ml of ethanol was added and the mixture was stirred for 15 min at room temperature. Then potassium cyanide (1.88 g, 28.8 mmol) was added, the vial was sealed and the mixture was stirred at 80°C over night. The solvent was removed on a rotary evaporator. The residue was taken up with ethyl acetate and washed with water and saturated aqueous sodium chloride solution. The organic layer was dried over sodium sulfate, filtered and concentrated on a rotary evaporator. 1.17 g (100 % purity, 71 % yield) of the title compound were obtained.
LC-MS (Method 7): Rₜ = 0.96 min; MS (ESIneg): m/z = 256 [M-H]⁻

### Intermediate 328

### rac-5-(aminomethyl)-5-ethylimidazolidine-2,4-dione hydrochloride

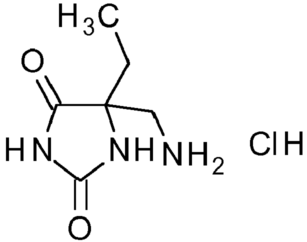

rac-tert-butyl [(4-ethyl-2,5-dioxoimidazolidin-4-yl)methyl]carbamate (1.17 g, 4.55 mmol) was dissolved in 26 ml of dichloromethane. 4 M hydrochloric acid in 1,4-dioxane (5.7 ml, 4.0 M, 23 mmol) was added and the mixture was stirred for 2 h. The solvent was removed on a rotary evaporator and the residue was taken up in dichlorormethane . The solvent was removed and the residue was dried in vacuo. 1.00 g (95 % purity, 108 % yield) of the title compound were obtained.
LC-MS (Method 9): Rₜ = 0.25 min; MS (ESIpos): m/z = 158 [M+H-HCl]⁺

### Intermediate 329

### ent-tert-butyl {[4-(1-methyl-1H-imidazol-2-yl)-2,5-dioxoimidazolidin-4-yl]methyl}carbamate

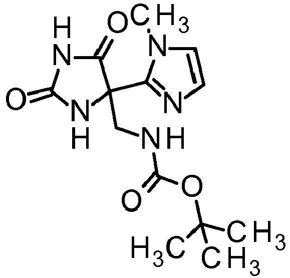

To a solution of tert-butyl [2-(1-methyl-1H-imidazol-2-yl)-2-oxoethyl]carbamate (4.96 g, 20.7 mmol, Example 3d in J: Med. Chem. 2014, 57, 10476-10485) in a mixture of Ethanol (30 ml) and water (12 ml) was added potassium cyanide (5.40 g, 82.9 mmol) and ammonium carbonate (7.97 g, 82.9 mmol) at RT. The reaction mixture was heated up to 80°C for 2 days into a sealed pressure flask. The resulting mixture was diluted with water, partially concentrated in vacuo and filtered. The resulting filtrate was concentrated in vacuo and 10.78 g of the crude product were isolated. 9.3 g of this crude product were suspended in ethanol and the residue was filtered off and washed 3 times with ethanol. The combined filtrate was concentrated in vacuo and the resulting product was purified by preparative chiral SFC (sample preparation: 7.3 g dissolved in a mixture of methanol and acetonitrile; column: Chirapak AD-H (SFC) 5µm, 250x30mm; eluent: carbon dioxide/methanol 78:22; flow rate: 125 ml/min; temperature: 40°C; UV detection: 210 nm). 2g (6.47 mmol) of the desired product was obtained
Analytical chiral SFC: Rₜ = 1.70 min, e.e. = 99% [column: AD 3 µm, 100 x 4.6 mm; eluent: carbon dioxide/methanol 80:20; flow rate: 3 ml/min; temperature: 40°C; UV detection: 210 nm]
¹H NMR (600 MHz, DMSO-*d*₆) δ ppm 11.09 (bs, 1 H), 8.16 (s, 1 H), 7.20 (s, 1 H), 6.67 - 6.95 (m, 2 H), 3.73 - 3.91 (m, 2 H), 3.49 (s, 3 H), 1.37 (s, 9 H).
The title compound can also be synthesized *via* the procedure described in J: Med. Chem. 2014, 57, 10476-10485

### Intermediate 330

### ent-5-(aminomethyl)-5-(1-methyl-1H-imidazol-2-yl)imidazolidine-2,4-dione hydrochloride

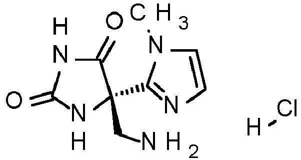

To a solution of *ent*-tert-butyl {[4-(1-methyl-1H-imidazol-2-yl)-2,5-dioxoimidazolidin-4-yl]methyl}carbamate (2.00 g, 6.46 mmol) in methanol (46 ml) were added 16 ml (65 mmol) of 4M hydrochloric acid in dioxane at 0°C. The resulting suspension was stirred overnight at room temperature. After filtration, the filtrate was concentrated *in vacuo* (1.23 g of the title compound) and the remaining residue was washed twice with methanol and dried under vacuum (0.41 g of of the title compound). Both fractions were used in the next step without further purification.
LC-MS (Method 9): Rₜ = 0.23 min; MS (ESIpos): m/z = 210 [M-HCl+H]⁺
¹H-NMR (600 MHz, DMSO-d6) δ [ppm]: 2.389 (0.48), 2.428 (0.49), 3.061 (1.66), 3.166 (1.50), 3.296 (0.55), 3.388 (1.15), 3.494 (0.46), 3.510 (0.45), 3.666 (0.48), 3.677 (0.51), 3.702 (0.83), 3.738 (6.54), 3.782 (16.00), 3.846 (2.02), 3.895 (1.13), 3.910 (1.64), 3.924 (0.90), 3.929 (0.64), 3.988 (0.48), 4.021 (0.52), 4.050 (0.72), 4.080 (0.42), 4.119 (0.80), 4.129 (0.89), 4.160 (0.45), 4.259 (0.45), 4.697 (0.54), 7.250 (1.55), 7.322 (2.88), 7.335 (2.41), 7.419 (1.52), 7.545 (5.13), 7.729 (0.53), 7.776 (0.43), 8.699 (4.41), 9.125 (3.30), 11.735 (5.00).

### EXPERIMENTAL SECTION - Examples

### General Method A for the synthesis of hydantoin amides 1-27

The suitable carboxylic acid (1.0 eq.) and the corresponding amine (1.1 eq.) were dissolved in the appropriate amount of N,N-dimethylformamide and 1-(3-dimethylaminopropyl)-3-ethylcarbodiimide hydrochloride (3.0 eq.), 1-hydroxybenzotriazole (1.3 eq.) or 1-hydroxy-7-azabenzotriazole (1.3 eq., 1M in N,N-dimethylacetamide) and N,N-diisopropylethylamine (2.8 eq.) or trimethylamine (2.8 eq.) were added allowing the mixture to stir at room temperature for 16 hours. After reaction's completion, the crude was dissolved in ethyl acetate and sequentially extracted with 1N hydrochloric acid and sodium hydrogencarbonate. The organic layer was dried over magnesium sulfate, filtered and concentrated *in vacuo* to give the crude product, which was purified either by precipitation with diethyl ether and heptane or by column chromatography in a suitable mixture of solvents.

### Example 1

### N-{[(4R)-4-cyclopropyl-2,5-dioxoimidazolidin-4-yl]methyl}-2-(3-fluorophenyl)-2H-1,2,3-triazole-4-carboxamide

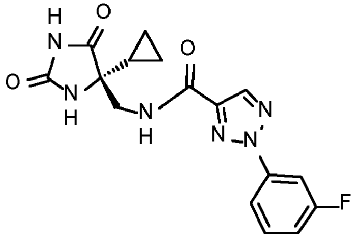

The compound was synthesized according to the General Method A using 92 mg (0.44 mmol) of 2-(3-fluorophenyl)-2H-1,2,3-triazole-4-carboxylic acid, 100 mg (0.48 mmol) of (5R)-5-(aminomethyl)-5-cyclopropylimidazolidine-2,4-dione hydrochloride, 110 mg (0.57 mmol) of 1-(3-dimethylaminopropyl)-3-ethylcarbodiimide hydrochloride, 88 mg (0.57 mmol) of 1-hydroxybenzotriazole and 0.22 mL (1.20 mmol) of N,N-diisopropylethylamine in 3 mL of N,N-dimethylformamide. The crude product obtained from the workup was precipitated with diethyl ether and heptane to give 67 mg (41% yield, 97% purity) of the pure compound as an off-white powder.
¹H-NMR (300 MHz, DMSO-*d*₆): δ [ppm] = 0.06-0.19 (m, 1H), 0.23-0.46 (m, 3H), 1.08-1.19 (m, 1H), 3.66 (ddd, 2H), 7.29 (bp, 1H), 7.36 (t, 1H), 7.68 (q, 1H), 7.83-7.96 (m, 2H), 8.47-8.58 (m, 2H).
LC-MS (Method 1): Rₜ = 2.496 min. MS (Mass method 1): m/z = 359 (M + H)⁺

### Example 2

### rac-N-{[4-tert-butyl-2,5-dioxoimidazolidin-4-yl]methyl}-2-[3-fluoro-4-(trifluoromethyl)phenyl]-2H-1,2,3-triazole-4-carboxamide

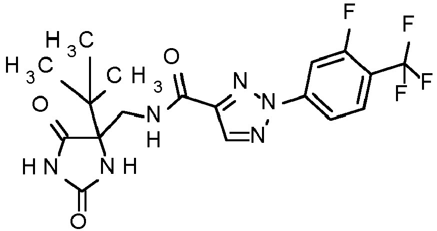

The compound was synthesized according to the General Method A using 150 mg (0.54 mmol) of 2-[3-fluoro-4-(trifluoromethyl)phenyl]-2H-1,2,3-triazole-4-carboxylic acid, 101 mg (0.54 mmol) of rac-5-(aminomethyl)-5-tert-butylimidazolidine-2,4-dione, 157 mg (0.54 mmol) of 1-(3-dimethylaminopropyl)-3-ethylcarbodiimide hydrochloride, 0.82 mL (0.82 mmol) of 1M 1-hydroxy-7-azabenzotriazole in N,N-dimethylacetamide and 0.23 mL (1.60 mmol) of triethylamine in 3 mL of N,N-dimethylformamide. The crude product obtained from the workup was purified by flash column chromatography on silica gel eluting with dichloromethane/methanol mixtures to afford 100 mg (41% yield, 99% purity) of the racemic product as a white powder.
¹H-NMR (300 MHz, DMSO-*d*₆): δ [ppm] = 1.03 (s, 9H), 3.75 (d, 2H), 7.64 (s, 1H), 8.09 (bp, 2H), 8.16 (d, 1H), 8.51 (t, 1H), 8.58 (s, 1H), 10.61 (s, 1H).
LC-MS (Method 1): Rₜ = 3.186 min. MS (Mass method 1): m/z = 443 (M + H)⁺

### Example 3

### ent-N-{[4-tert-butyl-2,5-dioxoimidazolidin-4-yl]methyl}-2-(4-fluorophenyl)-2H-1,2,3-triazole-4-carboxamide

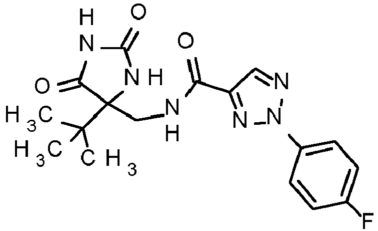

The compound was synthesized according to the General Method A using 76 mg (0.37 mmol) of 2-(4-fluorophenyl)-2H-1,2,3-triazole-4-carboxylic acid, 75 mg (0.40 mmol) of *rac-5-*(aminomethyl)-5-tert-butylimidazolidine-2,4-dione, 92 mg (0.48 mmol) of 1-(3-dimethylaminopropyl)-3-ethylcarbodiimide hydrochloride, 73 mg (0.48 mmol) of 1-hydroxybenzotriazole and 0.18 mL (1.00 mmol) of N,N-diisopropylethylamine in 3 mL of N,N-dimethylformamide. The crude product obtained from the workup was precipitated with diethyl ether and heptane to give the pure racemic compound. The racemate was resolved using a chiral column with a gradient from 75% n-heptane / 25% ethanol to 100% ethanol, to give 3 mg (2% yield, 96% purity) of the enantiopure product as a white powder.
¹H-NMR (300 MHz, DMSO-*d*₆): δ [ppm] = 1.01 (s, 9H), 3.73 (ddd, 2H), 7.48 (t, 2H), 7.61 (s, 1H), 8.07 (dd, 2H), 8.31 (t, 1H), 8.45 (s, 1H), 10.60 (s, 1H).
LC-MS (Method 1): Rₜ = 2.648 min. MS (Mass method 1): m/z = 375 (M + H)⁺

### Example 4

### rac-N-{[4-tert-butyl-2,5-dioxoimidazolidin-4-yl]methyl}-2-(3-fluorophenyl)-2H-1,2,3-triazole-4-carboxamide

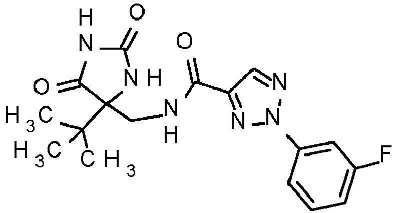

The compound was synthesized according to the General Method A using 76 mg (0.37 mmol) of 2-(3-fluorophenyl)-2H-1,2,3-triazole-4-carboxylic acid, 75 mg (0.40 mmol) of *rac-5-*(aminomethyl)-5-tert-butylimidazolidine-2,4-dione, 92 mg (0.48 mmol) of 1-(3-dimethylaminopropyl)-3-ethylcarbodiimide hydrochloride, 73 mg (0.48 mmol) of 1-hydroxybenzotriazole and 0.18 mL (1.00 mmol) of N,N-diisopropylethylamine in 3 mL of N,N-dimethylformamide. The crude product obtained from the workup was precipitated with diethyl ether and heptane to give 5 mg (3% yield, 99% purity) of the racemic product as a white powder.
¹H-NMR (300 MHz, DMSO-*d*₆): δ [ppm] = 1.03 (s, 9H), 3.74 (ddd, 2H), 7.38 (t, 1H), 7.61 (s, 1H), 7.69 (dd, 1H), 7.83-7.96 (m, 2H), 8.38 (t, 1H), 8.49 (s, 1H), 10.61 (bp, 1H).
LC-MS (Method 1): Rₜ = 2.687 min. MS (Mass method 1): m/z = 375 (M + H)⁺

### Example 5

### N-{[(4R)-4-cyclopropyl-2,5-dioxoimidazolidin-4-yl]methyl}-2-[3-fluoro-4-(trifluoromethyl)phenyl]-2H-1,2,3-triazole-4-carboxamide

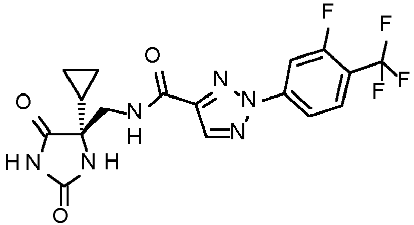

The compound was synthesized according to the General Method A using 100 mg (0.36 mmol) of 2-[3-fluoro-4-(trifluoromethyl)phenyl]-2H-1,2,3-triazole-4-carboxylic acid, 75 mg (0.36 mmol) of (5R)-5-(aminomethyl)-5-cyclopropylimidazolidine-2,4-dione hydrochloride, 105 mg (0.54 mmol) of 1-(3-dimethylaminopropyl)-3-ethylcarbodiimide hydrochloride, 0.55 mL (0.55 mmol) of 1M 1-hydroxy-7-azabenzotriazole in N,N-dimethylacetamide and 0.15 mL (1.10 mmol) of triethylamine in 3 mL of N,N-dimethylformamide. The crude product obtained from the workup was purified by flash column chromatography on silica gel eluting with dichloromethane/methanol mixtures to afford 78 mg (49% yield, 98% purity) of the product as a white powder.
¹H-NMR (300 MHz, DMSO-*d*₆): δ [ppm] = 0.07-0.20 (m, 1H), 0.28-0.51 (m, 3H), 1.11-1.22 (m, 1H), 3.71 (ddd, 2H), 7.64 (s, 1H), 803-8.14 (m, 2H), 8.17 (d, 1H), 8.59 (s, 1H), 8.64 (t, 1H), 10.65 (bp, 1H).
LC-MS (Method 1): Rₜ = 3.026 min. MS (Mass method 1): m/z = 427 (M + H)⁺

### Example 6

### rac-N-[(4-tert-butyl-2,5-dioxoimidazolidin-4-yl)methyl]-2-[4-(trifluoromethyl)phenyl]-2H-1,2,3-triazole-4-carboxamide

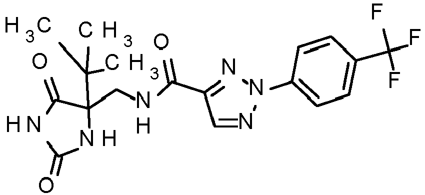

The compound was synthesized according to the General Method A using 115 mg (0.45 mmol) of 2-[4-(trifluoromethyl)phenyl]-2H-1,2,3-triazole-4-carboxylic acid, 83 mg (0.45 mmol) of *rac-5-*(aminomethyl)-5-tert-butylimidazolidine-2,4-dione, 129 mg (0.67 mmol) of 1-(3-dimethylaminopropyl)-3-ethylcarbodiimide hydrochloride, 0.67 mL (0.67 mmol) of 1M 1-hydroxy-7-azabenzotriazole in N,N-dimethylacetamide and 0.19 mL (1.30 mmol) of triethylamine in 3 mL of N,N-dimethylformamide. The crude product obtained from the workup was purified by flash column chromatography on silica gel eluting with dichloromethane/methanol mixtures to afford 35 mg (18% yield, 99% purity) of the racemic product as a white powder.
¹H-NMR (300 MHz, DMSO-*d*₆): δ [ppm] = 1.03 (s, 9H), 3.75 (ddd, 2H), 7.62 (s, 1H), 8.03 (d, 2H), 8.28 (d, 2H), 8.44 (t, 1H), 8.54 (s, 1H), 10.61 (bp, 1H).
LC-MS (Method 1): Rₜ = 3.092 min. MS (Mass method 1): m/z = 425 (M + H)⁺

### Example 7

### rac-N-{[2,5-dioxo-4-(tetrahydro-2H-pyran-3-yl)imidazolidin-4-yl]methyl}-2-phenyl-2H-1,2,3-triazole-4-carboxamide

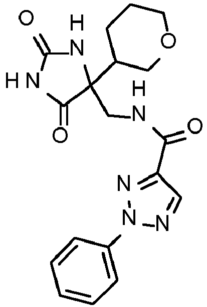

The compound was synthesized according to the General Method A using 53 mg (0.28 mmol) of 2-phenyl-2H-1,2,3-triazole-4-carboxylic acid, 70 mg (0.28 mmol) of rac-5-(aminomethyl)-5-[oxan-3-yl]imidazolidine-2,4-dione-hydrogen chloride, 81 mg (0.42 mmol) of 1-(3-dimethylaminopropyl)-3-ethylcarbodiimide hydrochloride, 0.42 mL (0.42 mmol) of 1M 1-hydroxy-7-azabenzotriazole in N,N-dimethylacetamide and 0.12 mL (0.84 mmol) of triethylamine in 3 mL of N,N-dimethylformamide. The crude product obtained from the workup was purified by flash column chromatography on silica gel eluting with dichloromethane/methanol mixtures to afford 60 mg (55% yield, 99% purity) of the racemic product as a white powder.
¹H-NMR (300 MHz, DMSO-*d*₆): δ [ppm] = 1.34-1.68 (m, 3H), 1.87-2.02 (m, 2H), 3.11-3.23 (m, 3H), 3.54-3.84 (m, 4H), 3.98-4.14 (m, 1H), 7.47-7.55 (m, 1H), 7.63 (t, 2H), 7.87 (s, 1H), 8.08 (d, 2H), 8.37-8.45 (m, 1H), 8.47 (s, 1H), 10.76 (bp, 1H).
LC-MS (Method 1): Rt = 2.272 min. MS (Mass method 1): m/z = 385 (M + H)⁺

### Example 8

### N-{[(4R)-4-cyclopropyl-2,5-dioxoimidazolidin-4-yl]methyl}-2-(3,4-difluorophenyl)-2H-1,2,3-triazole-4-carboxamide

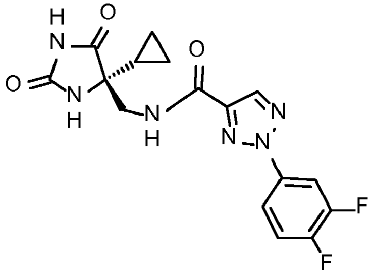

The compound was synthesized according to the General Method A using 75 mg (0.33 mmol) of 2-(3,4-difluorophenyl)-2H-1,2,3-triazole-4-carboxylic acid, 75 mg (0.37 mmol) of (5R)-5-(aminomethyl)-5-cyclopropylimidazolidine-2,4-dione hydrochloride, 83 mg (0.43 mmol) of 1-(3-dimethylaminopropyl)-3-ethylcarbodiimide hydrochloride, 66 mg (0.43 mmol) of 1-hydroxybenzotriazole and 0.16 mL (0.93 mmol) of N,N-diisopropylethylamine in 3 mL of N,N-dimethylformamide. The crude product obtained from the workup was precipitated with diethyl ether and heptane to give 32 mg (23% yield, 99% purity) of the pure compound as an off-white powder.
¹H-NMR (300 MHz, DMSO-*d*₆): δ [ppm] = 0.08-0.20 (m, 1H), 0.27-0.52 (m, 3H), 1.10-1.25 (m, 1H), 3.70 (ddd, 2H), 7.61 (s, 1H), 7.73 (q, 1H), 7.88-7.97 (m, 1H), 8.07-8.17 (m, 1H), 8.47-8.59 (m, 2H), 10.66 (bp, 1H).
LC-MS (Method 1): Rₜ = 2.600 min. MS (Mass method 1): m/z = 377 (M + H)⁺

### Example 9

### 2-(4-chlorophenyl)-N-{[(4R)-4-cyclopropyl-2,5-dioxoimidazolidin-4-yl]methyl}-2H-1,2,3-triazole-4-carboxamide

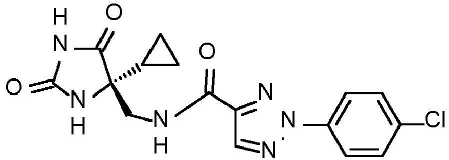

The compound was synthesized according to the General Method A using 82 mg (0.36 mmol) of 2-(4-chlorophenyl)-2H-1,2,3-triazole-4-carboxylic acid, 75 mg (0.36 mmol) of (5R)-5-(aminomethyl)-5-cyclopropylimidazolidine-2,4-dione hydrochloride, 105 mg (0.55 mmol) of 1-(3-dimethylaminopropyl)-3-ethylcarbodiimide hydrochloride, 0.55 mL (0.55 mmol) of 1M 1-hydroxy-7-azabenzotriazole in N,N-dimethylacetamide and 0.15 mL (1.10 mmol) of triethylamine in 3 mL of N,N-dimethylformamide. The crude product obtained from the workup was precipitated with diethyl ether and heptane to give 63 mg (50% yield, 99% purity) of the pure compound as an off-white powder.
¹H-NMR (300 MHz, DMSO-*d*₆): δ [ppm] = 0.07-0.20 (m, 1H), 0.28-0.39 (m, 1H), 0.40-0.50 (m, 2H), 1.10-1.23 (m, 1H), 3.70 (ddd, 2H), 7.63 (s, 1H), 7.70 (d, 2H), 8.08 (d, 2H), 8.46-8.56 (m, 2H), 10.64 (bp, 1H).
LC-MS (Method 1): Rₜ = 2.731 min. MS (Mass method 1): m/z = 375 (M + H)⁺

### Example 10

### 2-(3-chlorophenyl)-N-{[(4R)-4-cyclopropyl-2,5-dioxoimidazolidin-4-yl]methyl}-2H-1,2,3-triazole-4-carboxamide

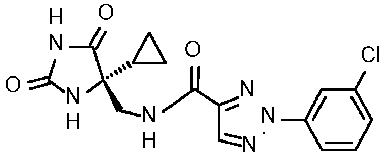

The compound was synthesized according to the General Method A using 82 mg (0.36 mmol) of 2-(3-chlorophenyl)-2H-1,2,3-triazole-4-carboxylic acid, 75 mg (0.36 mmol) of (5R)-5-(aminomethyl)-5-cyclopropylimidazolidine-2,4-dione hydrochloride, 105 mg (0.55 mmol) of 1-(3-dimethylaminopropyl)-3-ethylcarbodiimide hydrochloride, 0.55 mL (0.55 mmol) of 1M 1-hydroxy-7-azabenzotriazole in N,N-dimethylacetamide and 0.15 mL (1.10 mmol) of triethylamine in 3 mL of N,N-dimethylformamide. The crude product obtained from the workup was precipitated with diethyl ether and heptane to give 85 mg (66% yield, 99% purity) of the pure compound as an off-white powder.
¹H-NMR (300 MHz, DMSO-*d*₆): δ [ppm] = 0.07-0.20 (m, 1H), 0.28-0.50 (m, 3H), 1.11-1.23 (m, 1H), 3.70 (ddd, 2H), 7.54-7.70 (m, 3H), 8.04 (d, 1H), 8.11 (s, 1H), 8.50 (s, 1H), 8.57 (t, 1H), 10.66 (s, 1H.
LC-MS (Method 1): Rₜ = 2.735 min. MS (Mass method 1): m/z = 375 (M + H)⁺

### Example 11

### 2-(2-chlorophenyl)-N-{[(4R)-4-cyclopropyl-2,5-dioxoimidazolidin-4-yl]methyl}-2H-1,2,3-triazole-4-carboxamide

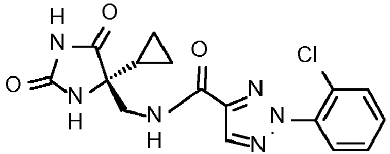

The compound was synthesized according to the General Method A using 82 mg (0.36 mmol) of 2-(2-chlorophenyl)-2H-1,2,3-triazole-4-carboxylic acid, 75 mg (0.36 mmol) of (5R)-5-(aminomethyl)-5-cyclopropylimidazolidine-2,4-dione hydrochloride, 105 mg (0.55 mmol) of 1-(3-dimethylaminopropyl)-3-ethylcarbodiimide hydrochloride, 0.55 mL (0.55 mmol) of 1M 1-hydroxy-7-azabenzotriazole in N,N-dimethylacetamide and 0.15 mL (1.10 mmol) of triethylamine in 3 mL of N,N-dimethylformamide. The crude product obtained from the workup was precipitated with diethyl ether and heptane to give 74 mg (61% yield, 98% purity) of the pure compound as an off-white powder.
¹H-NMR (300 MHz, DMSO-*d*₆): δ [ppm] = 0.06-0.17 (m, 1H), 0.28-0.50 (m, 3H), 1.10-1.20 (m, 1H), 3.69 (d, 2H), 7.55-7.71 (m, 3H), 7.79 (d, 2H), 8.44 (t, 1H), 8.50 (s, 1H), 10.64 (s, 1H). LC-MS (Method 1): Rₜ = 2.365 min. MS (Mass method 1): m/z = 375 (M + H)⁺

### Example 12

### N-{[(4R)-4-cyclopropyl-2,5-dioxoimidazolidin-4-yl]methyl}-2-(2-methylphenyl)-2H-1,2,3-triazole-4-carboxamide

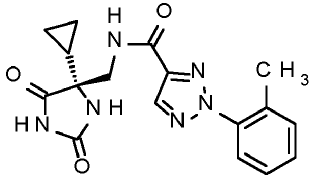

The compound was synthesized according to the General Method A using 74 mg (0.36 mmol) of 2-(2-methylphenyl)-2H-1,2,3-triazole-4-carboxylic acid, 75 mg (0.36 mmol) of (5R)-5-(aminomethyl)-5-cyclopropylimidazolidine-2,4-dione hydrochloride, 105 mg (0.55 mmol) of 1-(3-dimethylaminopropyl)-3-ethylcarbodiimide hydrochloride, 0.55 mL (0.55 mmol) of 1M 1-hydroxy-7-azabenzotriazole in N,N-dimethylacetamide and 0.15 mL (1.10 mmol) of triethylamine in 3 mL of N,N-dimethylformamide. The crude product obtained from the workup was precipitated with diethyl ether and heptane to give 76 mg (58% yield, 99% purity) of the pure compound as an off-white powder.
¹H-NMR (300 MHz, DMSO-*d*₆): δ [ppm] = 0.06-0.18 (m, 1H), 0.26-0.50 (m, 3H), 1.10-1.21 (m, 1H), 2.28 (s, 3H), 3.69 (d, 2H), 7.37-7.52 (m, 3H), 7.57-7.64 (m, 2H), 8.38 (t, 1H), 8.44 (s, 1H), 10.65 (s, 1H).
LC-MS (Method 1): Rₜ = 2.443 min. MS (Mass method 1): m/z = 355 (M + H)⁺

### Example 13

### N-{[(4R)-4-cyclopropyl-2,5-dioxoimidazolidin-4-yl]methyl}-2-(4-methylphenyl)-2H-1,2,3-triazole-4-carboxamide

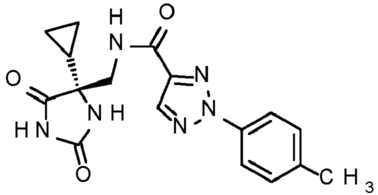

The compound was synthesized according to the General Method A using 74 mg (0.36 mmol) of 2-(4-methylphenyl)-2H-1,2,3-triazole-4-carboxylic acid, 75 mg (0.36 mmol) of (5R)-5-(aminomethyl)-5-cyclopropylimidazolidine-2,4-dione hydrochloride, 105 mg (0.55 mmol) of 1-(3-dimethylaminopropyl)-3-ethylcarbodiimide hydrochloride, 0.55 mL (0.55 mmol) of 1M 1-hydroxy-7-azabenzotriazole in N,N-dimethylacetamide and 0.15 mL (1.10 mmol) of triethylamine in 3 mL of N,N-dimethylformamide. The crude product obtained from the workup was chromatographed in dichloromethane/methanol mixtures to give 73 mg (56% yield, 99% purity) of the pure compound as an off-white powder.
¹H-NMR (300 MHz, DMSO-*d*₆): δ [ppm] = 0.07-0.20 (m, 1H), 0.29-0.51 (m, 3H), 1.11-1.26 (m, 1H), 2.39 (s, 3H), 3.70 (ddd, 2H), 7.42- (d, 2H), 7.62 (s, 1H), 7.95 (d, 2H), 8.39-8.49 (m, 2H), 10.66 (s, 1H).
LC-MS (Method 1): Rₜ = 2.642 min. MS (Mass method 1): m/z = 355 (M + H)⁺

### Example 14

### 2-(4-cyanophenyl)-N-{[(4R)-4-cyclopropyl-2,5-dioxoimidazolidin-4-yl]methyl}-2H-1,2,3-triazole-4-carboxamide

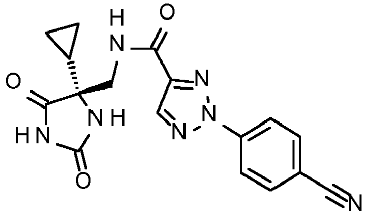

The compound was synthesized according to the General Method A using 78 mg (0.36 mmol) of 2-(4-cyanophenyl)-2H-1,2,3-triazole-4-carboxylic acid, 75 mg (0.36 mmol) of (5R)-5-(aminomethyl)-5-cyclopropylimidazolidine-2,4-dione hydrochloride, 105 mg (0.55 mmol) of 1-(3-dimethylaminopropyl)-3-ethylcarbodiimide hydrochloride, 0.55 mL (0.55 mmol) of 1M 1-hydroxy-7-azabenzotriazole in N,N-dimethylacetamide and 0.15 mL (1.10 mmol) of triethylamine in 3 mL of N,N-dimethylformamide. The crude product obtained from the workup was chromatographed in dichloromethane/methanol mixtures to give 103 mg (77% yield, 99% purity) of the pure compound as an off-white powder.
¹H-NMR (300 MHz, DMSO-*d*₆): δ [ppm] = 0.08-0.20 (m, 1H), 0.28-0.51 (m, 3H), 1.10-1.24 (m, 1H), 3.70 (ddd, 2H), 7.63 (s, 1H), 8.12 (d, 2H), 8.24 (d, 2H), 8.54-8.65 (m, 2H), 10.66 (s, 1H). LC-MS (Method 1): Rₜ = 2.311 min. MS (Mass method 1): m/z = 366 (M + H)⁺

### Example 15

### N-{[(4R)-4-cyclopropyl-2,5-dioxoimidazolidin-4-yl]methyl}-2-(5-fluoropyridin-2-yl)-2H-1,2,3-triazole-4-carboxamide

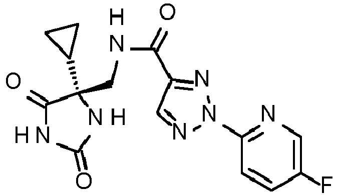

The compound was synthesized according to the General Method A using 111 mg (0.53 mmol) of 2-(5-fluoropyridin-2-yl)-2H-1,2,3-triazole-4-carboxylic acid, 100 mg (0.48 mmol) of (5R)-5-(aminomethyl)-5-cyclopropylimidazolidine-2,4-dione hydrochloride, 121 mg (0.63 mmol) of 1-(3-dimethylaminopropyl)-3-ethylcarbodiimide hydrochloride, 97 mg (0.63 mmol) of 1-hydroxybenzotriazole and 0.24 mL (1.40 mmol) of N,N-diisopropylethylamine in 3 mL of N,N-dimethylformamide. The crude product obtained from the workup was purified by reversed-phase chromatography using a gradient from 95% 65mM ammonium acetate:acetonitrile 90:10 / 5% acetonitrile:methanol 1:1 to 63% 65mM ammonium acetate:acetonitrile 90:10 / 37% acetonitrile:methanol 1:1 to give 5 mg (3% yield, 99% purity) of the pure compound as an off-white powder.
¹H-NMR (300 MHz, DMSO-*d*₆): δ [ppm] = 0.07-0.20 (m, 1H), 0.29-0.49 (m, 3H), 1.10-1.22 (m, 1H), 3.71 (d, 2H), 7.61 (s, 1H), 7.99-8.19 (m, 2H), 8.48-8.59 (m, 2H), 8.65 (s, 1H), 10.65 (s, 1H). LC-MS (Method 1): Rₜ = 1.908 min. MS (Mass method 1): m/z = 360 (M + H)⁺

### Example 16

### N-{[(4R)-4-cyclopropyl-2,5-dioxoimidazolidin-4-yl]methyl}-2-(pyrazin-2-yl)-2H-1,2,3-triazole-4-carboxamide

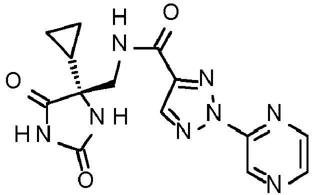

The compound was synthesized according to the General Method A using 60 mg (0.31 mmol) of 2-(pyrazin-2-yl)-2H-1,2,3-triazole-4-carboxylic acid, 71 mg (0.34 mmol) of (5R)-5-(aminomethyl)-5-cyclopropylimidazolidine-2,4-dione hydrochloride, 78 mg (0.41 mmol) of 1-(3-dimethylaminopropyl)-3-ethylcarbodiimide hydrochloride, 62 mg (0.41 mmol) of 1-hydroxybenzotriazole and 0.15 mL (0.88 mmol) of N,N-diisopropylethylamine in 3 mL of N,N-dimethylformamide. The crude product obtained from the workup was precipitated with diethyl ether and heptane to give 11 mg (10% yield, 99% purity) of the pure compound as an off-white powder.
¹H-NMR (300 MHz, DMSO-*d*₆): δ [ppm] = 0.07-0.19 (m, 1H), 0.24-0.46 (m, 3H), 1.18-1.21 (m, 1H), 3.68 (ddd, 2H), 7.39 (bp, 1H), 8.57-8.66 (m, 2H), 8.72 (s, 1H), 8.84 (s, 1H), 9.38 (s, 1H), 10.65 (bp, 1H).
LC-MS (Method 1): Rₜ = 1.580 min. MS (Mass method 1): m/z = 343 (M + H)⁺

### Example 17

### 2-(3-cyanophenyl)-N-{[(4R)-4-cyclopropyl-2,5-dioxoimidazolidin-4-yl]methyl}-2H-1,2,3-triazole-4-carboxamide

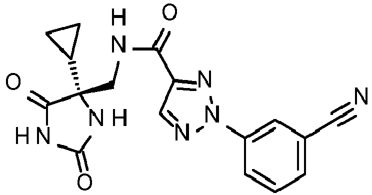

The compound was synthesized according to the General Method A using 78 mg (0.36 mmol) of 2-(3-cyanophenyl)-2H-1,2,3-triazole-4-carboxylic acid, 75 mg (0.36 mmol) of (5R)-5-(aminomethyl)-5-cyclopropylimidazolidine-2,4-dione hydrochloride, 105 mg (0.54 mmol) of 1-(3-dimethylaminopropyl)-3-ethylcarbodiimide hydrochloride, 0.54 mL (0.54 mmol) of 1M 1-hydroxy-7-azabenzotriazole in N,N-dimethylacetamide and 0.15 mL (1.10 mmol) of triethylamine in 2 mL of N,N-dimethylformamide. The crude product obtained from the workup was purified by flash column chromatography eluting with dichloromethane/methanol mixtures to give 49 mg (37% yield, 99% purity) of the enatiopure compound form as a white powder.
¹H-NMR (300 MHz, DMSO-*d*₆): δ [ppm] = 0.08-0.19 (m, 1H), 0.28-0.39 (m, 1H), 0.40-0.52 (m, 2H), 1.11-1.24 (m, 1H), 3.71 (ddd, 2H), 7.63 (s, 1H), 7.84 (t, 1H), 7.98 (d, 1H), 8.38 (s, 1H), 8.48 (s, 1H), 8.54 (s, 1H), 8.59 (t, 1H), 10.66 (s, 1H).
LC-MS (Method 1): Rₜ = 2.322 min. MS (Mass method 1): m/z = 366 (M + H)⁺

### Example 18

### N-{[(4R)-4-cyclopropyl-2,5-dioxoimidazolidin-4-yl]methyl}-2-(3-methylphenyl)-2H-1,2,3-triazole-4-carboxamide

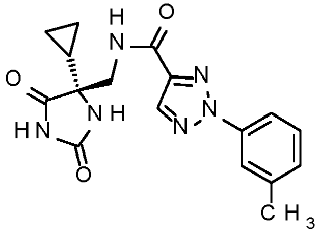

The compound was synthesized according to the General Method A using 74 mg (0.36 mmol) of 2-(3-methylphenyl)-2H-1,2,3-triazole-4-carboxylic acid, 75 mg (0.36 mmol) of (5R)-5-(aminomethyl)-5-cyclopropylimidazolidine-2,4-dione hydrochloride, 105 mg (0.54 mmol) of 1-(3-dimethylaminopropyl)-3-ethylcarbodiimide hydrochloride, 0.54 mL (0.54 mmol) of 1M 1-hydroxy-7-azabenzotriazole in N,N-dimethylacetamide and 0.15 mL (1.10 mmol) of triethylamine in 2 mL of N,N-dimethylformamide. The crude product obtained from the workup was purified by flash column chromatography eluting with dichloromethane/methanol mixtures to give 55 mg (42% yield, 99% purity) of the enatiopure compound form as a white powder.
¹H-NMR (300 MHz, DMSO-*d*₆): δ [ppm] = 0.07-0.19 (m, 1H), 0.28-0.49 (m, 3H), 1.11-1.25 (m, 1H), 2.43 (s, 3H), 3.70 (ddd, 2H), 7.32 (d, 1H), 7.49 (t, 1H), 7.62 (s, 1H), 7.86 (d, 1H), 7.90 (s, 1H), 8.42-8.51 (m, 2H), 10.65 (s, 1H).
LC-MS (Method 1): Rₜ = 2.728 min. MS (Mass method 1): m/z = 355 (M + H)⁺

### Example 19

### rac-N-{[2,5-dioxo-4-(tetrahydrofuran-3-yl)imidazolidin-4-yl]methyl}-2-phenyl-2H-1,2,3-triazole-4-carboxamide

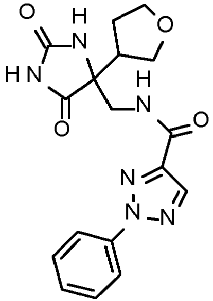

The compound was synthesized according to the General Method A using 60 mg (0.32 mmol) of 2-phenyl-2H-1,2,3-triazole-4-carboxylic acid, 75 mg (0.32 mmol) of rac-5-(aminomethyl)-5-[oxolan-3-yl]imidazolidine-2,4-dione-hydrogen chloride, 92 mg (0.48 mmol) of 1-(3-dimethylaminopropyl)-3-ethylcarbodiimide hydrochloride, 0.48 mL (0.48 mmol) of 1M 1-hydroxy-7-azabenzotriazole in N,N-dimethylacetamide and 0.13 mL (0.95 mmol) of triethylamine in 2 mL of N,N-dimethylformamide. The crude product obtained from the workup was purified by flash column chromatography eluting with dichloromethane/methanol mixtures to give 70 mg (54% yield, 99% purity) of the compound form as a white powder.
¹H-NMR (300 MHz, DMSO-*d*₆): δ [ppm] = 1.52-1.87 (m, 1H), 1.96-2.10 (m, 1H), 2.56-2.71 (m, 1H), 3.47-3.80 (m, 5H), 7.51 (t, 1H), 7.63 (t, sH), 7.99 (d, 1H), 8.08 (d, 2H), 8.45-8.56 (m, 2H), 10.79 (bp, 1H).
LC-MS (Method 1): Rₜ = 2.164 min. MS (Mass method 1): m/z = 371 (M + H)⁺

### Example 20

### rac-N-{[2,5-dioxo-4-(pyridin-2-yl)imidazolidin-4-yl]methyl}-2-phenyl-2H-1,2,3-triazole-4-carboxamide

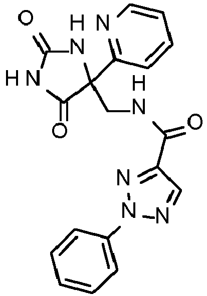

The compound was synthesized according to the General Method A using 195 mg (1.03 mmol) of 2-phenyl-2H-1,2,3-triazole-4-carboxylic acid, 250 mg (1.03 mmol) of rac-5-(aminomethyl)-5-(pyridin-2-yl)imidazolidine-2,4-dione-hydrogen chloride, 296 mg (1.55 mmol) of 1-(3-dimethylaminopropyl)-3-ethylcarbodiimide hydrochloride, 1.55 mL (1.55 mmol) of 1M 1-hydroxy-7-azabenzotriazole in N,N-dimethylacetamide and 0.43 mL (3.10 mmol) of triethylamine in 2 mL of N,N-dimethylformamide. The crude product obtained from the workup was purified by flash column chromatography eluting with dichloromethane/methanol mixtures to give 33 mg (8% yield, 97% purity) of the compound form as a white powder.
¹H-NMR (300 MHz, DMSO-*d*₆): δ [ppm] = 4.18 (ddd, 2H), 7.41 (t, 1H), 7.46-7.66 (m, 4H), 7.87 (t, 1H), 8.05 (d, 2H), 8.40 (s, 1H), 8.45-8.52 (m, 2H), 8.64 (d, 1H), 10.92 (bp, 1H).
LC-MS (Method 1): Rₜ = 2.413 min. MS (Mass method 1): m/z = 378 (M + H)⁺

### Example 21

### rac-N-{[2,5-dioxo-4-(pyridin-4-yl)imidazolidin-4-yl]methyl}-2-phenyl-2H-1,2,3-triazole-4-carboxamide

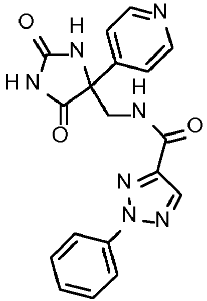

The compound was synthesized according to the General Method A using 185 mg (0.98 mmol) of 2-phenyl-2H-1,2,3-triazole-4-carboxylic acid, 238 mg (0.98 mmol) of rac-5-(aminomethyl)-5-(pyridin-4-yl)imidazolidine-2,4-dione hydrochloride, 282 mg (1.47 mmol) of 1-(3-dimethylaminopropyl)-3-ethylcarbodiimide hydrochloride, 1.47 mL (1.47 mmol) of 1M 1-hydroxy-7-azabenzotriazole in N,N-dimethylacetamide and 0.41 mL (2.90 mmol) of triethylamine in 2 mL of N,N-dimethylformamide. The crude product obtained from the workup was purified by flash column chromatography eluting with dichloromethane/methanol mixtures to give 3 mg (1% yield, 95% purity) of the compound form as a white powder.
¹H-NMR (300 MHz, DMSO-*d*₆): δ [ppm] = 3.90-4.05 (m, 2H), 7.46-7.54 (m, 1H), 7.56-7.66 (m, 4H), 8.05 (d, 2H), 8.45 (s, 1H), 8.61 (d, 2H), 8.64-8.73 (m, 2H), 11.03 (bp, 1H).
LC-MS (Method 1): Rₜ = 1.820 min. MS (Mass method 1): m/z = 378 (M + H)⁺

### Example 22

### rac-N-{[2,5-dioxo-4-(pyridin-2-yl)imidazolidin-4-yl]methyl}-2-(4-fluorophenyl)-2H-1,2,3-triazole-4-carboxamide

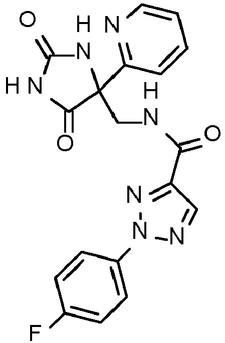

The compound was synthesized according to the General Method A using 179 mg (0.86 mmol) of 2-(4-fluorophenyl)-2H-1,2,3-triazole-4-carboxylic acid, 210 mg (0.86 mmol) of *rac-5-*(aminomethyl)-5-(pyridin-2-yl)imidazolidine-2,4-dione-hydrogen chloride, 249 mg (1.30 mmol) of 1-(3-dimethylaminopropyl)-3-ethylcarbodiimide hydrochloride, 1.30 mL (1.30 mmol) of 1M 1-hydroxy-7-azabenzotriazole in N,N-dimethylacetamide and 0.36 mL (2.60 mmol) of triethylamine in 2 mL of N,N-dimethylformamide. The crude product obtained from the workup was purified by flash column chromatography eluting with dichloromethane/methanol mixtures to give 32 mg (9% yield, 99% purity) of the compound form as a white powder.
¹H-NMR (300 MHz, DMSO-*d*₆): δ [ppm] = 4.17 (ddd, 2H), 7.37-7.52 (m, 3H), 7.55 (d, 1H), 7.87 (t, 1H), 8.04-8.13 (m, 2H), 8.40 (s, 1H), 8.44-8.54 (m, 2H), 8.64 (d, 1H), 10.90 (s, 1H).
LC-MS (Method 1): Rₜ = 2.514 min. MS (Mass method 1): m/z = 396 (M + H)⁺

### Example 23

### rac-N-{[2,5-dioxo-4-(pyridin-3-yl)imidazolidin-4-yl]methyl}-2-(4-fluorophenyl)-2H-1,2,3-triazole-4-carboxamide

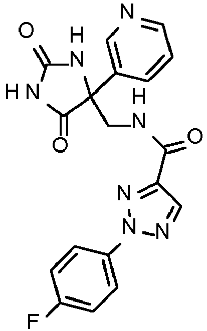

The compound was synthesized according to the General Method A using 213 mg (1.03 mmol) of 2-(4-fluorophenyl)-2H-1,2,3-triazole-4-carboxylic acid, 250 mg (1.03 mmol) of *rac-5-*(aminomethyl)-5-(pyridin-3-yl)imidazolidine-2,4-dione-hydrogen chloride, 296 mg (1.50 mmol) of 1-(3-dimethylaminopropyl)-3-ethylcarbodiimide hydrochloride, 1.50 mL (1.50 mmol) of 1M 1-hydroxy-7-azabenzotriazole in N,N-dimethylacetamide and 0.43 mL (3.10 mmol) of triethylamine in 2 mL of N,N-dimethylformamide. The crude product obtained from the workup was purified by flash column chromatography eluting with dichloromethane/methanol mixtures to give 6 mg (1% yield, 98% purity) of the compound form as a white powder.
¹H-NMR (300 MHz, DMSO-*d*₆): δ [ppm] = 3.89-4.06 (m, 2H), 7.41-7.53 (m, 3H), 7.99 (d, 1H), 8.03-8.12 (m, 2H), 8.44 (s, 1H), 8.55 (d, 1H), 8.65-8.73 (m, 2H), 8.79 (s, 1H), 11.03 (bp, 1H). LC-MS (Method 1): Rₜ = 2.031 min. MS (Mass method 1): m/z = 396 (M + H)⁺

### Example 24

### rac-N-{[2,5-dioxo-4-(pyridin-4-yl)imidazolidin-4-yl]methyl}-2-(4-fluorophenyl)-2H-1,2,3-triazole-4-carboxamide

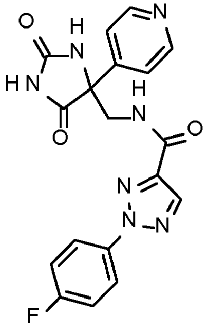

The compound was synthesized according to the General Method A using 125 mg (0.61 mmol) of 2-(4-fluorophenyl)-2H-1,2,3-triazole-4-carboxylic acid, 147 mg (0.61 mmol) of *rac-5-*(aminomethyl)-5-(pyridin-4-yl)imidazolidine-2,4-dione-hydrogen chloride, 174 mg (0.91 mmol) of 1-(3-dimethylaminopropyl)-3-ethylcarbodiimide hydrochloride, 0.91 mL (0.91 mmol) of 1M 1-hydroxy-7-azabenzotriazole in N,N-dimethylacetamide and 0.25 mL (1.80 mmol) of triethylamine in 2 mL of N,N-dimethylformamide. The crude product obtained from the workup was purified by flash column chromatography eluting with dichloromethane/methanol mixtures to give 27 mg (11% yield, 99% purity) of the compound form as a white powder.
¹H-NMR (300 MHz, DMSO-*d*₆): δ [ppm] = 3.94-4.01 (m, 2H), 7.47 (t, 2H), 7.63 (d, 2H), 8.04-8.11 (m, 2H), 8.44 (s, 1H), 8.61-8.73 (m, 4H), 11.05 (s, 1H).
LC-MS (Method 1): Rₜ = 1.936 min. MS (Mass method 1): m/z = 396 (M + H)⁺

### Example 25

### N-{[(4R)-4-cyclopropyl-2,5-dioxoimidazolidin-4-yl]methyl}-3-[1-(cyclopropylmethyl)-1H-pyrazol-4-yl]-1,2-oxazole-5-carboxamide

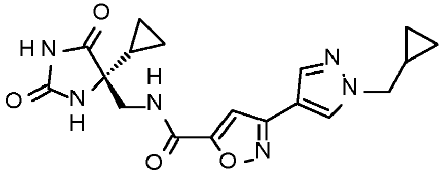

929 mg (2.44 mmol) of HATU were added to a solution of 380 mg (1.63 mmol) of 3-[1-(cyclopropylmethyl)-1H-pyrazol-4-yl]-1,2-oxazole-5-carboxylic acid, 335 mg (1.63 mmol) of (5R)-5-(aminomethyl)-5-cyclopropylimidazolidine-2,4-dione hydrochloride, 2.40 mL (2.40 mmol) of 1M 1-hydroxy-7-azabenzotriazole in N,N-dimethylacetamide and 0.85 mL (4.90 mmol) of N,N-diisopropylethylamine in 5 mL of N,N-dimethylformamide and the reaction was allowed to stir at room temperature for 16 hours. The solvent was evaporated and the residue was diluted with dichloromethane, sequentially washed with an aqueous solution of sodium hydrogencarbonate(aq.) and 2N hydrochloric acid(aq.). The organic layer was dried over magnesium sulfate, filtered and concentrated to afford a residue, which was further purified by flash chromatography on silica gel eluting with dichloromethane/methanol mixtures to give 20 mg (3% yield, 99% purity) of the product as a white solid.
¹H-NMR (300 MHz, DMSO-*d*₆): δ [ppm] = 0.07-0.19 (m, 1H), 0.28-0.50 (m, 5H), 0.51-0.67 (m, 2H), 1.09-1.19 (m, 1H), 1.23-1.34 (m, 1H), 3.66 (ddd, 2H), 4.03 (d, 2H), 7.39 (s, 1H), 7.94 (s, 1H), 8.38 (s, 1H), 8.87 (t, 1H), 10.64 (s, 1H).
LC-MS (Method 1): Rₜ = 2.166 min. MS (Mass method 1): m/z = 385 (M + H)⁺

### Example 26

### rac-{[4-tert-butyl-2,5-dioxoimidazolidin-4-yl]methyl}-2-phenyl-1,3-oxazole-5-carboxamide

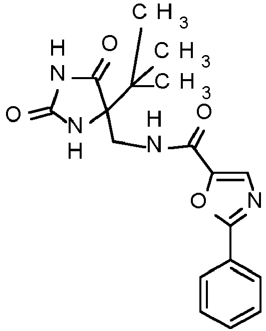

226 mg (0.59 mmol) of HATU were added to a solution of 75 mg (0.39 mmol) of 2-phenyl-1,3-oxazole-5-carboxylic acid, 73 mg (0.39 mmol) of rac-5-(aminomethyl)-5-tert-butylimidazolidine-2,4-dione, 0.59 mL (0.59 mmol) of 1M 1-hydroxy-7-azabenzotriazole in N,N-dimethylacetamide and 0.17 mL, (1.20 mmol) of triethylamine in 5 mL of N,N-dimethylformamide and the reaction was allowed to stir at room temperature for 1 hour. The reaction was quenched with methanol and the solvent was removed *in vacuo.* The corresponding residue was diluted with ethyl acetate and sequentially washed with 1N hydrochloric acid(aq.), saturated sodium hydrogencarbonate(aq.) and brine. The organic layer was dried over magnesium sulfate, filtered, concentrated and purified by flash chromatography on silica gel eluting with dichloromethane and methanol to give 86 mg (58% yield, 95% purity) of the racemic product as a white powder.
¹H-NMR (300 MHz, DMSO-*d*₆): δ [ppm] = 1.03 (s, 9H), 3.71 (ddd, 2H), 7.53-7.65 (m, 4H), 7.90 (s, 1H), 8.06-8.15 (m, 2H), 8.61 (t, 1H), 10.61 (bp, 1H).
LC-MS (Method 1): Rₜ = 2.385 min. MS (Mass method 1): m/z = 357 (M + H)⁺

### Example 27

### N-{[(4R)-4-cyclopropyl-2,5-dioxoimidazolidin-4-yl]methyl}-2-(4-methoxyphenyl)-2H-1,2,3-triazole-4-carboxamide

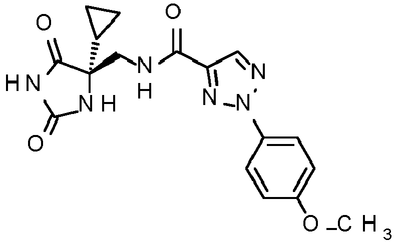

2-(4-Methoxyphenyl)-2H-1,2,3-triazole-4-carboxylic acid (53.3 mg, 243 µmol) dissolved in 3 ml DMF were treated with (5R)-5-(aminomethyl)-5-cyclopropylimidazolidine-2,4-dione hydrochloride (50.0 mg, 243 µmol), 1-ethyl-3-(3-dimethylaminopropyl)carbodiimide hydrochloride (60.6 mg, 316 µmol), 1-hydroxybenzotriazole hydrate (48.4 mg, 316 µmol) and N,N-diisopropylethylamine (120 µl, 681 µmol). The mixture was stirred over night at room temperature. Purification was done by preparative HPLC (column: Chromatorex C18 10 µm, 250 x 30 mm, eluent A = water, B = acetonitrile; gradient: 0.0 min 5% B; 3 min 5% B; 20 min 50% B; 23 min 100% B; 26 min 5% B; flow: 50 ml/min; 0.1% formic acid). Product containing samples were united, the solvents were evaporated and the residue was lyophylized. 28.0 mg (100 % purity, 31 % yield) of the title compound were obtained.
LC-MS (Method 7): Rₜ = 1.29 min; MS (ESIpos): m/z = 371 [M+H]⁺
¹H-NMR (400 MHz, DMSO-d6) δ [ppm]: 0.115 (0.52), 0.124 (0.71), 0.129 (0.69), 0.137 (0.93), 0.145 (0.78), 0.154 (0.66), 0.325 (0.69), 0.335 (0.74), 0.343 (0.98), 0.352 (0.72), 0.358 (0.59), 0.362 (0.58), 0.373 (0.44), 0.414 (1.29), 0.431 (2.47), 0.450 (2.11), 0.469 (0.75), 1.150 (0.73), 1.167 (1.08), 1.183 (0.73), 3.311 (16.00), 3.639 (0.63), 3.654 (0.79), 3.673 (1.68), 3.688 (1.52), 3.709 (1.53), 3.725 (1.64), 3.743 (0.70), 3.759 (0.65), 7.150 (4.70), 7.168 (1.69), 7.173 (4.94), 7.181 (0.56), 7.626 (3.67), 7.957 (0.54), 7.966 (4.94), 7.971 (1.71), 7.983 (1.62), 7.988 (4.67), 7.997 (0.51), 8.396 (0.93), 8.406 (6.71), 8.428 (0.86), 10.653 (1.92).

### Example 28

### N-{[(4R)-4-cyclopropyl-2,5-dioxoimidazolidin-4-yl]methyl}-2-[4-(methylsulfonyl)phenyl]-2H-1,2,3-triazole-4-carboxamide

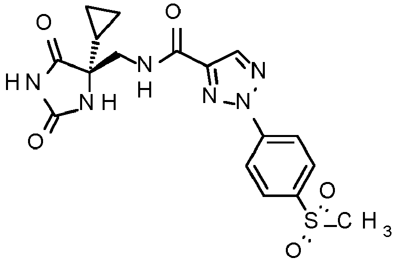

The reaction was carried out analogously to the preparation of N-{[(4R)-4-cyclopropyl-2,5-dioxoimidazolidin-4-yl]methyl}-2-(4-methoxyphenyl)-2H-1,2,3-triazole-4-carboxamide. 74.0 mg (100 % purity, 73 % yield) of the title compound were obtained.
LC-MS (Method 7): Rₜ = 1.04 min; MS (ESIpos): m/z = 419 [M+H]⁺
¹H-NMR (400 MHz, DMSO-d6) δ [ppm]: 0.445 (0.64), 0.462 (0.53), 3.300 (6.16), 3.313 (16.00), 3.677 (0.46), 3.692 (0.40), 3.733 (0.41), 3.750 (0.44), 7.637 (0.98), 8.173 (1.28), 8.178 (0.50), 8.190 (0.56), 8.195 (1.83), 8.309 (1.77), 8.314 (0.58), 8.326 (0.47), 8.331 (1.29), 8.576 (2.12), 8.607 (0.50), 10.660 (0.71).

### Example 29

### 2-(3-chloro-4-fluorophenyl)-N-{[(4R)-4-cyclopropyl-2,5-dioxoimidazolidin-4-yl]methyl}-2H-1,2,3-triazole-4-carboxamide

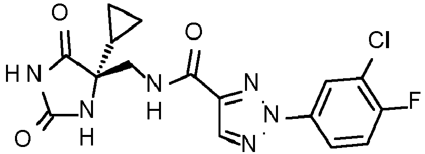

The reaction was carried out analogously to the preparation of N-{[(4R)-4-cyclopropyl-2,5-dioxoimidazolidin-4-yl]methyl}-2-(4-methoxyphenyl)-2H-1,2,3-triazole-4-carboxamide. 53.0 mg (100 % purity, 55 % yield) of the title compound were obtained.
LC-MS (Method 7): Rₜ = 1.49 min; MS (ESIpos): m/z = 393 [M+H]⁺
¹H-NMR (400 MHz, DMSO-d6) δ [ppm]: 0.108 (0.53), 0.119 (1.07), 0.131 (1.73), 0.142 (1.93), 0.146 (1.75), 0.155 (1.53), 0.170 (0.74), 0.307 (0.42), 0.328 (1.32), 0.337 (1.39), 0.344 (1.90), 0.355 (1.70), 0.364 (1.17), 0.375 (0.93), 0.403 (0.49), 0.414 (1.63), 0.420 (1.65), 0.434 (4.36), 0.451 (3.41), 0.470 (1.54), 1.134 (0.72), 1.148 (1.46), 1.155 (1.54), 1.161 (1.16), 1.168 (2.24), 1.182 (1.48), 1.187 (1.23), 1.202 (0.59), 3.619 (1.68), 3.633 (1.90), 3.653 (3.19), 3.668 (2.84), 3.726 (2.89), 3.743 (3.11), 3.760 (1.84), 3.778 (1.71), 7.599 (6.08), 7.683 (3.48), 7.705 (7.25), 7.728 (3.93), 8.051 (2.05), 8.058 (2.45), 8.061 (2.42), 8.068 (2.31), 8.073 (2.05), 8.080 (2.34), 8.084 (2.08), 8.091 (1.99), 8.246 (3.14), 8.252 (3.05), 8.262 (3.22), 8.268 (2.91), 8.506 (16.00), 8.542 (1.69), 8.559 (3.29), 8.575 (1.65), 10.655 (2.72).

### Example 30

### 2-(4-chloro-3-fluorophenyl)-N-{[(4R)-4-cyclopropyl-2,5-dioxoimidazolidin-4-yl]methyl}-2H-1,2,3-triazole-4-carboxamide

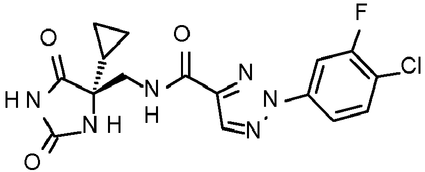

The reaction was carried out analogously to the preparation of N-{[(4R)-4-cyclopropyl-2,5-dioxoimidazolidin-4-yl]methyl}-2-(4-methoxyphenyl)-2H-1,2,3-triazole-4-carboxamide. 23.0 mg (100 % purity, 24 % yield) of the title compound were obtained.
LC-MS (Method 7): Rₜ = 1.50 min; MS (ESIpos): m/z = 393 [M+H]⁺
¹H-NMR (400 MHz, DMSO-d6) δ [ppm]: 0.008 (0.82), 0.108 (0.64), 0.120 (1.37), 0.131 (2.15), 0.142 (2.38), 0.146 (2.23), 0.156 (1.89), 0.170 (0.93), 0.310 (0.54), 0.331 (1.66), 0.340 (1.74), 0.347 (2.32), 0.358 (2.13), 0.367 (1.43), 0.378 (1.12), 0.407 (0.59), 0.419 (2.00), 0.425 (2.02), 0.438 (5.36), 0.455 (4.16), 0.475 (1.89), 1.136 (0.88), 1.150 (1.79), 1.156 (1.91), 1.163 (1.46), 1.170 (2.77), 1.183 (1.78), 1.190 (1.52), 1.203 (0.71), 2.328 (0.50), 2.366 (0.54), 2.523 (2.19), 2.670 (0.54), 2.710 (0.54), 3.621 (2.10), 3.636 (2.37), 3.655 (3.94), 3.670 (3.54), 3.729 (3.58), 3.746 (3.85), 3.763 (2.23), 3.780 (2.10), 7.628 (9.15), 7.857 (2.94), 7.879 (5.75), 7.898 (5.32), 7.934 (4.52), 7.940 (4.35), 7.956 (2.40), 7.962 (2.49), 8.074 (4.08), 8.081 (3.79), 8.100 (4.03), 8.106 (3.79), 8.529 (16.00), 8.549 (2.20), 8.565 (4.19), 8.580 (2.09), 10.654 (4.82).

### Example 31

### N-{[(4R)-4-cyclopropyl-2,5-dioxoimidazolidin-4-yl]methyl}-2-(2,4-difluorophenyl)-2H-1,2,3-triazole-4-carboxamide

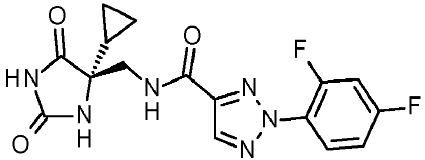

2-(2,4-difluorophenyl)-2H-1,2,3-triazole-4-carboxylic acid (54.7 mg, 243 µmol) dissolved in 5 ml DCM were treated with (5R)-5-(aminomethyl)-5-cyclopropylimidazolidine-2,4-dione hydrochloride (50.0 mg, 243 µmol), 1-ethyl-3-(3-dimethylaminopropyl)carbodiimide hydrochloride (60.6 mg, 316 µmol), 1-hydroxybenzotriazole hydrate (48.4 mg, 316 µmol) and N,N-diisopropylethylamine (120 µl, 681 µmol). The mixture was stirred over night at room temperature. Then the mixture was diluted with ethyl acetate and washed with water and brine. The organic layer was dried over sodium sulfate, filtered and evaporated. The residue was put on Isolute and purified by chromatography on silica gel (10 g Snap Cartridge Biotage®; Biotage-Isolera-One®; DCM/MeOH-gradient: 2% MeOH - 20% MeOH; flow: 36 ml/min). Samples containing the desired product were united, the solvents were evaporated and the residue was was lyophylized. 67.0 mg (100 % purity, 73 % yield) of the title compound were obtained.
LC-MS (Method 7): Rₜ = 1.23 min; MS (ESIpos): m/z = 377 [M+H]⁺
¹H-NMR (400 MHz, DMSO-d6) δ [ppm]: 0.008 (0.50), 0.098 (0.72), 0.109 (1.40), 0.117 (2.01), 0.131 (2.42), 0.140 (2.01), 0.147 (1.79), 0.159 (0.95), 0.299 (0.61), 0.310 (0.78), 0.318 (1.95), 0.330 (2.00), 0.337 (2.48), 0.346 (1.93), 0.356 (1.64), 0.367 (1.23), 0.404 (3.64), 0.421 (7.17), 0.440 (5.74), 0.458 (1.90), 1.122 (0.94), 1.139 (2.16), 1.156 (2.99), 1.172 (2.07), 1.189 (0.78), 3.644 (0.95), 3.659 (1.19), 3.678 (5.50), 3.691 (7.05), 3.706 (5.33), 3.724 (1.09), 3.740 (1.00), 7.351 (1.44), 7.354 (1.55), 7.357 (1.42), 7.374 (3.02), 7.377 (2.85), 7.393 (1.61), 7.396 (1.70), 7.400 (1.46), 7.587 (8.75), 7.657 (2.01), 7.664 (1.97), 7.680 (2.29), 7.685 (3.48), 7.691 (2.18), 7.707 (2.07), 7.714 (1.98), 7.930 (2.04), 7.945 (2.30), 7.952 (3.85), 7.967 (3.88), 7.974 (2.13), 7.989 (1.94), 8.443 (2.26), 8.459 (4.71), 8.474 (2.22), 8.517 (16.00), 10.646 (4.55).

### Example 32

### N-{[(4R)-4-cyclopropyl-2,5-dioxoimidazolidin-4-yl]methyl}-2-(3,4-dichlorophenyl)-2H-1,2,3-triazole-4-carboxamide

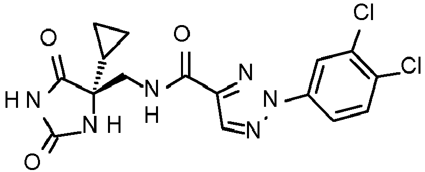

The reaction was carried out analogously to the preparation of N-{[(4R)-4-cyclopropyl-2,5-dioxoimidazolidin-4-yl]methyl}-2-(4-methoxyphenyl)-2H-1,2,3-triazole-4-carboxamide. 54.0 mg (98 % purity, 53 % yield) of the title compound were obtained.
LC-MS (Method 7): Rₜ = 1.62 min; MS (ESIpos): m/z = 409 [M+H]⁺
¹H-NMR (400 MHz, DMSO-d6) δ [ppm]: 0.108 (0.85), 0.121 (1.70), 0.132 (2.64), 0.142 (2.85), 0.146 (2.64), 0.156 (2.33), 0.170 (1.07), 0.309 (0.70), 0.330 (2.01), 0.339 (2.14), 0.346 (2.78), 0.357 (2.58), 0.366 (1.73), 0.377 (1.40), 0.405 (0.83), 0.416 (2.50), 0.422 (2.56), 0.436 (6.37), 0.453 (5.04), 0.472 (2.28), 1.136 (1.05), 1.150 (2.13), 1.157 (2.28), 1.163 (1.77), 1.171 (3.36), 1.184 (2.14), 1.190 (1.86), 1.204 (0.89), 1.234 (0.69), 1.370 (2.89), 3.619 (2.40), 3.634 (2.70), 3.653 (4.49), 3.668 (4.05), 3.729 (4.09), 3.746 (4.35), 3.763 (2.59), 3.780 (2.39), 7.610 (10.15), 7.901 (7.18), 7.923 (9.84), 8.040 (5.40), 8.046 (5.62), 8.062 (4.04), 8.068 (4.31), 8.291 (6.42), 8.297 (6.15), 8.528 (16.00), 8.575 (2.51), 8.591 (4.86), 8.606 (2.46), 10.654 (4.58).

### Example 33

### 2-(4-bromophenyl)-N-{[(4R)-4-cyclopropyl-2,5-dioxoimidazolidin-4-yl]methyl}-2H-1,2,3-triazole-4-carboxamide

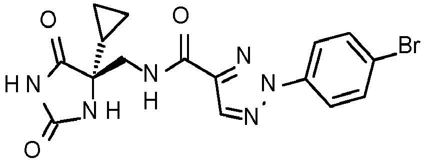

The reaction was carried out analogously to the preparation of N-{[(4R)-4-cyclopropyl-2,5-dioxoimidazolidin-4-yl]methyl}-2-(4-methoxyphenyl)-2H-1,2,3-triazole-4-carboxamide. 15.0 mg (100 % purity, 15 % yield) of the title compound were obtained.
LC-MS (Method 7): Rₜ = 1.52 min; MS (ESIpos): m/z = 419 [M+H]⁺
¹H-NMR (400 MHz, DMSO-d6) δ [ppm]: -0.008 (1.30), 0.008 (1.10), 0.106 (0.54), 0.117 (1.08), 0.129 (1.48), 0.139 (2.00), 0.145 (1.61), 0.152 (1.37), 0.167 (0.71), 0.308 (0.47), 0.328 (1.34), 0.337 (1.64), 0.346 (2.07), 0.355 (1.54), 0.360 (1.21), 0.365 (1.17), 0.376 (0.94), 0.417 (2.40), 0.435 (4.65), 0.453 (4.16), 0.472 (1.51), 1.135 (0.71), 1.150 (1.37), 1.155 (1.47), 1.162 (1.23), 1.169 (2.20), 1.184 (1.44), 1.202 (0.58), 1.370 (0.71), 2.073 (1.58), 3.633 (1.50), 3.648 (1.71), 3.667 (3.37), 3.682 (2.98), 3.716 (3.02), 3.733 (3.29), 3.750 (1.64), 3.767 (1.51), 7.621 (7.17), 7.817 (1.16), 7.824 (10.02), 7.830 (3.41), 7.842 (4.09), 7.847 (12.95), 7.854 (1.65), 7.999 (1.57), 8.006 (12.76), 8.011 (3.79), 8.024 (3.57), 8.029 (10.04), 8.036 (1.21), 8.492 (16.00), 8.510 (3.54), 8.526 (1.73), 10.654 (5.38).

### Example 34

### N-{[(4R)-4-cyclopropyl-2,5-dioxoimidazolidin-4-yl]methyl}-2-(4-fluorophenyl)-2H-1,2,3-triazole-4-carboxamide

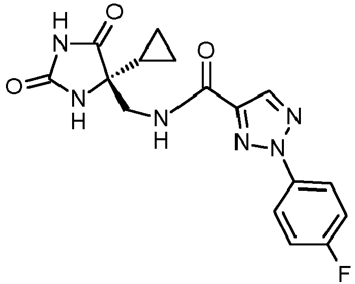

2-(4-fluorophenyl)-2H-1,2,3-triazole-4-carboxylic acid (1.375 g, 6.64 mmol, CAS 833-60-3) were treated with 140 ml dichloromethane, N-ethyl-N-isopropylpropan-2-amine (2.402 g, 18.58 mmol), 1-ethyl-3-(3-dimethylaminopropyl)carbodiimide hydrochloride (1.654 g, 8.63 mmol) and 1-hydroxybenzotriazole hydrate (1.321 g, 8.63 mmol). The mixture was stirred for 5 minutes before (5R)-5-(aminomethyl)-5-cyclopropylimidazolidine-2,4-dione hydrochloride (1.365 g, 6.64 mmol) was added. The mixture was stirred over night at room temperature. The solvent was removed on a rotary evaporator. The residue was taken up with ethyl acetate and washed with water and saturated aqueous sodium chloride solution. The organic layer was dried over sodium sulfate, filtered, Isolute® was added and the solvent was evaporated on a rotary evaporator. The crude product was purified by column chromatography (Machine: Biotage® Isolera One; Column: Biotage® SNAP Ultra 50 g; Gradient: Dichloromethane/Methanol, 2% Methanol -> 20% Methanol; Flow: 100 ml/min). Product containing samples were united and the solvents were removed on a rotary evaporator. The residue was stirred with acetonitrile, filtered and washed with acetonitrile. The product was dried over the weekend in a vacuum oven at 50°C. 1.5 g (63% yield, 100% purity) of the title compound were obtained.
LC-MS (Method 7): Rₜ = 1.28 min; MS (ESIpos): m/z = 359 [M+H]⁺
¹H-NMR (500 MHz, DMSO-d6) δ [ppm]: 0.112 (0.57), 0.122 (1.12), 0.131 (1.58), 0.139 (1.87), 0.143 (1.56), 0.151 (1.35), 0.162 (0.68), 0.317 (0.48), 0.326 (0.69), 0.334 (1.33), 0.342 (1.47), 0.348 (1.83), 0.356 (1.58), 0.363 (1.04), 0.372 (0.79), 0.416 (0.43), 0.425 (1.76), 0.428 (1.81), 0.440 (4.41), 0.454 (3.82), 0.470 (1.58), 1.144 (0.73), 1.156 (1.39), 1.161 (1.49), 1.166 (1.14), 1.172 (2.17), 1.183 (1.41), 1.187 (1.20), 1.198 (0.57), 3.644 (1.64), 3.656 (1.83), 3.671 (3.19), 3.683 (2.84), 3.725 (2.90), 3.738 (3.12), 3.752 (1.77), 3.766 (1.63), 7.461 (0.58), 7.468 (5.01), 7.472 (1.82), 7.486 (8.50), 7.499 (1.84), 7.503 (5.25), 7.510 (0.54), 7.638 (7.28), 8.078 (0.62), 8.085 (5.21), 8.089 (2.27), 8.094 (5.48), 8.098 (3.28), 8.103 (5.37), 8.108 (2.07), 8.112 (5.00), 8.119 (0.51), 8.475 (16.00), 8.484 (1.95), 8.497 (3.48), 8.509 (1.74), 10.671 (5.48).

### Example 35

### N-{[(4R)-4-cyclopropyl-2,5-dioxoimidazolidin-4-yl]methyl}-2-(2,3-difluorophenyl)-2H-1,2,3-triazole-4-carboxamide

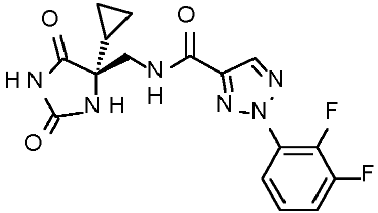

The reaction was carried out analogously to the preparation of N-{[(4R)-4-cyclopropyl-2,5-dioxoimidazolidin-4-yl]methyl}-2-(4-methoxyphenyl)-2H-1,2,3-triazole-4-carboxamide. 45.0 mg (100 % purity, 49 % yield) of the title compound were obtained.
LC-MS (Method 7): Rₜ = 1.25 min; MS (ESIpos): m/z = 377 [M+H]⁺
¹H-NMR (400 MHz, DMSO-d6) δ [ppm]: -0.008 (2.09), 0.008 (1.79), 0.103 (0.69), 0.114 (1.32), 0.123 (1.82), 0.129 (1.68), 0.136 (2.37), 0.142 (1.76), 0.147 (1.84), 0.152 (1.64), 0.165 (0.92), 0.306 (0.60), 0.316 (0.76), 0.325 (1.75), 0.336 (1.90), 0.344 (2.51), 0.353 (1.83), 0.359 (1.51), 0.363 (1.48), 0.373 (1.17), 0.413 (3.43), 0.430 (6.35), 0.449 (5.35), 0.468 (1.87), 1.130 (0.89), 1.147 (1.86), 1.158 (1.57), 1.164 (2.78), 1.180 (1.87), 1.198 (0.70), 1.371 (0.76), 2.525 (0.70), 3.653 (1.12), 3.668 (1.34), 3.687 (5.00), 3.703 (8.77), 3.719 (4.87), 3.737 (1.25), 3.754 (1.15), 7.446 (1.02), 7.451 (1.00), 7.459 (1.25), 7.467 (2.59), 7.472 (2.25), 7.480 (2.54), 7.486 (2.67), 7.493 (1.45), 7.502 (1.56), 7.506 (1.36), 7.621 (9.61), 7.653 (1.11), 7.657 (1.22), 7.675 (2.18), 7.678 (2.12), 7.682 (1.53), 7.692 (1.26), 7.696 (2.15), 7.700 (2.23), 7.718 (0.97), 7.722 (0.98), 7.754 (1.48), 7.758 (2.32), 7.762 (1.41), 7.770 (1.79), 7.774 (3.53), 7.779 (3.21), 7.791 (1.44), 7.795 (2.08), 7.799 (1.12), 8.480 (2.18), 8.495 (4.35), 8.511 (2.04), 8.567 (16.00), 10.653 (4.35).

### Example 36

### N-{[(4R)-4-cyclopropyl-2,5-dioxoimidazolidin-4-yl]methyl}-2-(2,5-difluorophenyl)-2H-1,2,3-triazole-4-carboxamide

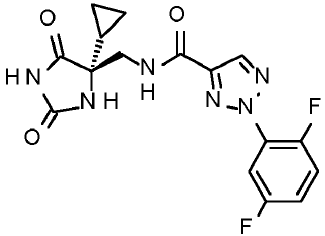

The reaction was carried out analogously to the preparation of N-{[(4R)-4-cyclopropyl-2,5-dioxoimidazolidin-4-yl]methyl}-2-(4-methoxyphenyl)-2H-1,2,3-triazole-4-carboxamide. 45.0 mg (100 % purity, 49 % yield) of the title compound were obtained.
LC-MS (Method 7): Rₜ = 1.22 min; MS (ESIpos): m/z = 377 [M+H]⁺
¹H-NMR (400 MHz, DMSO-d6) δ [ppm]: -0.008 (1.72), 0.006 (1.18), 0.008 (1.47), 0.099 (0.65), 0.111 (1.25), 0.119 (1.78), 0.126 (1.60), 0.133 (2.19), 0.137 (1.65), 0.142 (1.73), 0.148 (1.60), 0.161 (0.87), 0.301 (0.57), 0.312 (0.72), 0.321 (1.72), 0.332 (1.79), 0.339 (2.25), 0.348 (1.71), 0.355 (1.44), 0.358 (1.45), 0.369 (1.12), 0.406 (3.24), 0.423 (6.24), 0.442 (5.09), 0.461 (1.71), 1.124 (0.83), 1.141 (1.87), 1.152 (1.50), 1.158 (2.61), 1.174 (1.82), 1.192 (0.67), 1.371 (1.36), 2.525 (0.52), 3.647 (0.89), 3.663 (1.06), 3.681 (4.83), 3.694 (6.34), 3.709 (4.75), 3.727 (1.02), 3.744 (0.91), 7.348 (1.19), 7.351 (1.39), 7.354 (1.52), 7.358 (1.35), 7.371 (2.52), 7.374 (2.76), 7.377 (2.59), 7.390 (1.36), 7.394 (1.48), 7.397 (1.59), 7.400 (1.39), 7.606 (9.10), 7.656 (1.84), 7.663 (1.80), 7.679 (2.09), 7.685 (3.09), 7.691 (2.02), 7.707 (1.88), 7.713 (1.82), 7.931 (1.88), 7.946 (2.10), 7.953 (3.49), 7.968 (3.53), 7.975 (1.96), 7.990 (1.79), 8.451 (1.98), 8.467 (4.19), 8.483 (1.98), 8.519 (16.00), 10.647 (3.19).

### Example 37

### N-{[(4R)-4-cyclopropyl-2,5-dioxoimidazolidin-4-yl]methyl}-2-(2-fluorophenyl)-2H-1,2,3-triazole-4-carboxamide

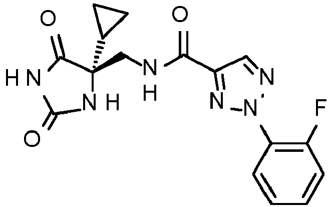

The reaction was carried out analogously to the preparation of N-{[(4R)-4-cyclopropyl-2,5-dioxoimidazolidin-4-yl]methyl}-2-(4-methoxyphenyl)-2H-1,2,3-triazole-4-carboxamide. 25.0 mg (100 % purity, 29 % yield) of the title compound were obtained.
LC-MS (Method 7): Rₜ = 1.16 min; MS (ESIpos): m/z = 359 [M+H]⁺
¹H-NMR (400 MHz, DMSO-d6) δ [ppm]: 0.099 (0.68), 0.110 (1.31), 0.118 (1.87), 0.132 (2.21), 0.141 (1.92), 0.148 (1.64), 0.160 (0.92), 0.301 (0.60), 0.320 (1.84), 0.332 (1.85), 0.338 (2.26), 0.348 (1.77), 0.357 (1.53), 0.368 (1.16), 0.407 (3.35), 0.424 (6.66), 0.443 (5.36), 0.461 (1.77), 1.126 (0.84), 1.143 (1.99), 1.160 (2.74), 1.176 (1.92), 1.193 (0.72), 1.370 (2.45), 3.650 (0.89), 3.666 (1.07), 3.684 (5.17), 3.696 (6.15), 3.700 (6.04), 3.712 (5.05), 3.729 (1.03), 3.746 (0.90), 7.436 (1.99), 7.439 (2.09), 7.456 (4.50), 7.477 (2.79), 7.548 (1.56), 7.551 (1.70), 7.569 (3.26), 7.573 (3.21), 7.579 (1.80), 7.597 (3.65), 7.600 (3.55), 7.613 (10.12), 7.621 (2.98), 7.626 (3.10), 7.630 (2.41), 7.640 (1.87), 7.644 (2.08), 7.652 (1.00), 7.661 (0.79), 7.665 (0.78), 7.865 (2.34), 7.869 (2.32), 7.885 (4.33), 7.889 (4.12), 7.904 (2.21), 7.908 (2.05), 8.442 (1.97), 8.458 (4.10), 8.473 (2.01), 8.517 (16.00), 10.648 (3.42).

### Example 38

### ent-N-{[2,5-dioxo-4-(pyridin-2-yl)imidazolidin-4-yl]methyl}-2-phenyl-2H-1,2,3-triazole-4-carboxamide

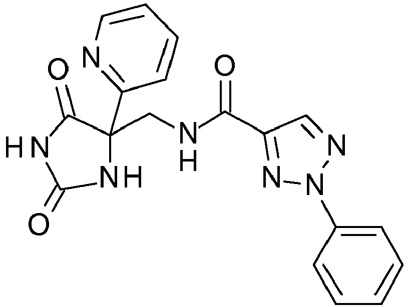

Enantiomeric separation of *rac*-N-{[2,5-dioxo-4-(pyridin-2-yl)imidazolidin-4-yl]methyl}-2-phenyl-2H-1,2,3-triazole-4-carboxamide (30.0 mg, 79.5 µmol) using the following method
- Column: Daicel Chiralpak IG 5 µm 250 x 20 mm
- Solvent: 20% n-heptane / 80% ethanol
- Flow: 15 ml/min
- Column temperature: 70°C
- UV-detection 220 nm
afforded 40.0 mg (100 % purity, 47 % yield) of the title compound.
Chiral-HPLC (Column: 250 x 20 mm Daicel Chiralpak IG, 5 µm; flow: 15 ml/min; temperature: 70°C; eluent: 20% n-heptane / 80% ethanol) Rt: 9.684 min; >99.0% ee
LC-MS (Method 7): Rₜ = 1.23 min; MS (ESIpos): m/z = 378 [M+H]⁺
¹H-NMR (400 MHz, DMSO-d6) δ [ppm]: -0.149 (0.53), 0.008 (4.89), 0.146 (0.51), 1.235 (0.70), 2.366 (0.42), 2.670 (0.41), 2.710 (0.43), 4.071 (1.75), 4.087 (1.89), 4.105 (2.92), 4.121 (2.70), 4.203 (2.69), 4.219 (2.92), 4.237 (1.87), 4.253 (1.71), 7.387 (2.31), 7.399 (2.71), 7.404 (2.72), 7.417 (2.58), 7.482 (1.75), 7.501 (4.59), 7.519 (3.14), 7.543 (4.51), 7.563 (5.01), 7.601 (5.64), 7.621 (8.29), 7.640 (4.37), 7.848 (2.23), 7.853 (2.22), 7.868 (3.62), 7.872 (3.54), 7.887 (1.85), 7.891 (1.76), 8.047 (7.92), 8.066 (7.69), 8.287 (1.66), 8.450 (1.89), 8.465 (16.00), 8.480 (1.72), 8.628 (3.18), 8.638 (3.14), 10.889 (1.19).

### Example 39

### ent-N-((1R)-1-(4-Cyclopropyl-2,5-dioxoimidazolidin-4-yl)ethyl)-2-phenyl-2H-1,2,3-triazole-4-carboxamide

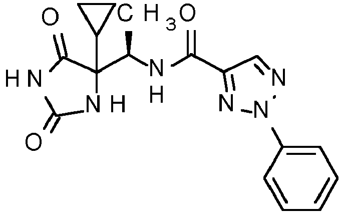

To a solution of *ent*-5-((R)-1-aminoethyl)-5-cyclopropylimidazolidine-2,4-dione bis(2,2,2-trifluoroacetate) (25 mg, 0.061 mmol), and 2-phenyl-2H-1,2,3-triazole-4-carboxylic acid (13.7 mg, 0.073 mmol) in DMF (2 mL) were added HOBt hydrate (9.9 mg, 0.073 mmol), N-(3-dimethylaminopropyl)-N'-ethylcarbodiimide hydrochloride (13.8 mg, 0.073 mmol) and DIPEA (42 µL, 0.24 mmol). The reaction mixture was stirred at room temperature over night, diluted with saturated aqueous sodium bicarbonate solution and dichloromethane followed by extraction with dichloromethane. The combined organic layers were dried over magnesium sulfate, filtered and concentrated. The residue was purified by column chromatography on silica gel (30 - 65 % ethyl acetate in hexanes). 9.4 mg (95 % purity, 65 % yield) of the title compound were obtained.
LC-MS (Method 13): Rₜ = 1.04 min; MS (ESIpos): m/z = 355 [M+H]⁺
¹H NMR (400 MHz, DMSO-d6) δ 10.72 (s, 1H), 8.47 (s, 1H), 8.08-8.00 (m, 3H), 7.86 (s, 1H), 7.63 (t, J = 7.7 Hz, 2H), 7.51 (t, J = 7.3 Hz, 1H), 4.55 (m, 1H), 1.24 (s, J = 6.7 Hz, 3H), 1.22 (m, 1H), 0.54-0.46 (m, 2H), 0.38 (m, 1H), 0.13 (m, 1H).

### Example 40

### ent-N-((1S)-1-(4-Cyclopropyl-2,5-dioxoimidazolidin-4-yl)ethyl)-2-phenyl-2H-1,2,3-triazole-4-carboxamide

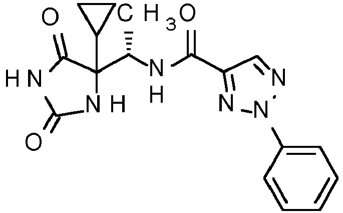

The reaction was carried out analogously to the preparation of *ent*-N-((1R)-1-(4-Cyclopropyl-2,5-dioxoimidazolidin-4-yl)ethyl)-2-phenyl-2H-1,2,3-triazole-4-carboxamide. When *ent*-5-((S)-1-aminoethyl)-5-cyclopropylimidazolidine-2,4-dione bis(2,2,2-trifluoroacetate) is used as starting material, 3.2 mg (95 % purity, 79 % yield) of the title compound were obtained.
LC-MS (Method 13): Rₜ = 1.04 min; MS (ESIpos): m/z = 355 [M-H]⁺
¹H NMR (400 MHz, DMSO-*d*₆) δ 10.83 (s, 1H), 8.49 (s, 1H), 8.07-8.13 (m, 3H), 7.74 (s, 1H), 7.64 (t, *J* = 7.7 Hz, 2H), 7.52 (t, *J* = 7.3 Hz, 1H), 4.66 (dq, *J* = 13.8, 6.8 Hz, 1H), 1.13 (d, *J* = 6.7 Hz, 3H), 1.11 (m, 1H), 0.37-0.21 (m, 3H), 0.05 (m, 1H).

### Example 41

### N-{[(4R)-4-cyclopropyl-2,5-dioxoimidazolidin-4-yl]methyl}-2-(4-fluorophenyl)-1,3-oxazole-4-carboxamide

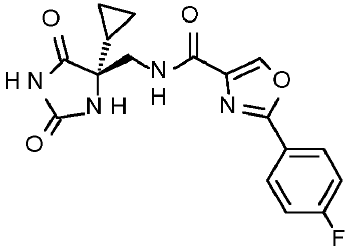

The reaction was carried out analogously to the preparation of N-{[(4R)-4-cyclopropyl-2,5-dioxoimidazolidin-4-yl]methyl}-2-(4-methoxyphenyl)-2H-1,2,3-triazole-4-carboxamide. 71.0 mg (100 % purity, 81 % yield) of the title compound were obtained.
LC-MS (Method 7): Rₜ = 1.27 min; MS (ESIpos): m/z = 359 [M+H]⁺
¹H-NMR (500 MHz, DMSO-d6) δ [ppm]: -0.007 (2.94), 0.006 (1.69), 0.120 (0.58), 0.131 (1.11), 0.140 (1.69), 0.150 (1.86), 0.159 (1.36), 0.169 (0.64), 0.316 (0.50), 0.327 (0.79), 0.334 (1.41), 0.343 (1.71), 0.351 (1.52), 0.362 (1.10), 0.370 (0.86), 0.395 (0.63), 0.404 (1.61), 0.415 (2.84), 0.425 (2.43), 0.433 (3.13), 0.445 (2.28), 0.451 (1.04), 0.463 (0.76), 1.135 (0.74), 1.146 (1.39), 1.151 (1.58), 1.162 (2.54), 1.168 (1.02), 1.173 (1.32), 1.178 (1.25), 1.189 (0.58), 1.234 (0.52), 3.611 (1.82), 3.623 (1.97), 3.638 (2.97), 3.651 (2.68), 3.717 (2.75), 3.730 (3.01), 3.744 (1.97), 3.758 (1.77), 7.411 (0.80), 7.417 (5.01), 7.421 (1.82), 7.435 (9.94), 7.448 (1.79), 7.453 (5.13), 7.459 (0.56), 7.650 (6.58), 7.984 (1.64), 7.997 (3.34), 8.009 (1.59), 8.044 (0.79), 8.050 (5.07), 8.054 (2.35), 8.061 (5.49), 8.068 (5.45), 8.074 (2.23), 8.078 (4.94), 8.743 (16.00), 10.666 (1.95).

### Example 42

### N-{[(4R)-4-cyclopropyl-2,5-dioxoimidazolidin-4-yl]methyl}-5-(4-fluorophenyl)-1,3,4-oxadiazole-2-carboxamide

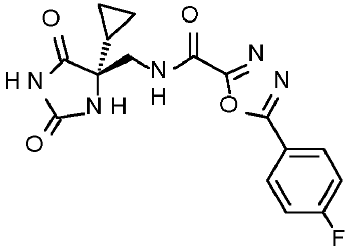

The reaction was carried out analogously to the preparation of N-{[(4R)-4-cyclopropyl-2,5-dioxoimidazolidin-4-yl]methyl}-2-(4-methoxyphenyl)-2H-1,2,3-triazole-4-carboxamide. 5.0 mg (99 % purity, 6 % yield) of the title compound were obtained.
LC-MS (Method 7): Rₜ = 1.16 min; MS (ESIpos): m/z = 360 [M+H]⁺
¹H-NMR (500 MHz, DMSO-d6) δ [ppm]: 0.113 (1.04), 0.122 (1.85), 0.129 (2.40), 0.139 (2.68), 0.147 (2.45), 0.153 (2.03), 0.163 (1.02), 0.324 (0.91), 0.333 (1.25), 0.340 (2.55), 0.349 (2.63), 0.354 (2.81), 0.362 (2.19), 0.367 (1.77), 0.379 (1.22), 0.442 (4.38), 0.456 (8.76), 0.472 (6.91), 0.487 (2.19), 1.147 (1.25), 1.161 (2.71), 1.173 (3.52), 1.187 (2.32), 1.201 (0.81), 1.650 (1.04), 2.362 (0.50), 2.636 (0.42), 3.268 (0.83), 3.300 (9.20), 3.326 (0.60), 3.665 (1.46), 3.678 (1.64), 3.693 (6.57), 3.705 (10.61), 3.717 (6.38), 3.732 (1.51), 3.744 (1.28), 7.480 (7.97), 7.484 (3.02), 7.498 (16.00), 7.516 (8.29), 7.645 (8.96), 8.138 (8.26), 8.142 (3.99), 8.149 (8.81), 8.156 (8.81), 8.162 (3.57), 8.167 (7.84), 9.203 (2.87), 9.216 (5.76), 9.228 (2.66), 10.688 (7.56).

### Example 43

### ent-N-{[4-(2,2-dimethylcyclopropyl)-2,5-dioxoimidazolidin-4-yl]methyl}-2-phenyl-2H-1,2,3-triazole-4-carboxamide

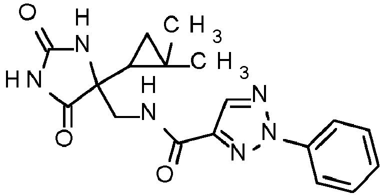

Enantiomeric separation of *diamix*-N-{[4-(2,2-dimethylcyclopropyl)-2,5-dioxoimidazolidin-4-yl]methyl}-2-phenyl-2H-1,2,3-triazole-4-carboxamide (229 mg, 621 µmol) using the following method
- Machine: Sepiatec
- Column: AD-H 5u 250 x 20 mm
- Solvent: 15% i-propanol / 85% CO₂
- Flow: 70 ml/min
- Wavelength: 210 nm
- System setting: backpressure: 100 bar
- Temperature fraction module: 15°C
- Temperature pumphead: -2°C
- Temperature column department: 40°C
afforded 31.0 mg (100 % purity, 14 % yield) of the title compound.
Chiral-HPLC (Column: AD 15; eluent: i-propanole; flow rate: 3.0 ml/min; UV-detection: 210nm) Rₜ: 2.932 min; >99.0% ee
LC-MS (Method 7): Rₜ = 1.53 min; MS (ESIpos): m/z = 369 [M+H]⁺
¹H-NMR (500 MHz, DMSO-d6) δ [ppm]: 0.397 (1.11), 0.406 (1.61), 0.415 (1.25), 0.424 (1.52), 0.450 (1.49), 0.461 (1.80), 0.470 (1.12), 0.936 (1.54), 0.948 (1.72), 0.954 (1.64), 0.966 (1.52), 0.984 (15.14), 1.089 (16.00), 3.668 (0.80), 3.680 (0.89), 3.695 (1.81), 3.707 (1.64), 3.732 (1.68), 3.745 (1.75), 3.759 (0.82), 3.773 (0.78), 7.496 (0.99), 7.511 (2.43), 7.526 (1.52), 7.588 (4.26), 7.616 (2.98), 7.633 (4.21), 7.648 (2.32), 8.062 (4.24), 8.079 (4.05), 8.450 (0.96), 8.463 (1.97), 8.477 (1.09), 8.484 (7.90), 10.764 (2.26).

### Example 44

### ent-N-{[4-(2,2-dimethylpropyl)-2,5-dioxoimidazolidin-4-yl]methyl}-2-phenyl-2H-1,2,3-triazole-4-carboxamide

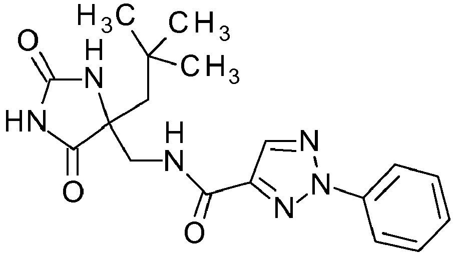

4.5 mg (6 % yield) of the title compound were obtained as by-product in the synthesis of *ent*-N-{[4-(2,2-dimethylcyclopropyl)-2,5-dioxoimidazolidin-4-yl]methyl}-2-phenyl-2H-1,2,3-triazole-4-carboxamide.
¹H-NMR (500 MHz, DMSO-d6) δ [ppm]: 0.900 (16.00), 1.603 (0.69), 1.633 (0.92), 1.767 (0.95), 1.797 (0.69), 3.447 (0.58), 3.459 (0.52), 3.495 (0.53), 3.508 (0.57), 7.508 (0.87), 7.522 (0.54), 7.613 (1.04), 7.630 (1.50), 7.645 (0.85), 7.841 (1.41), 8.070 (1.51), 8.085 (1.47), 8.449 (0.67), 8.479 (3.06), 10.739 (0.83).

### Example 45

### N-{[(4R)-4-cyclopropyl-2,5-dioxoimidazolidin-4-yl]methyl}-2-phenyl-2H-1,2,3-triazole-4-carboxamide

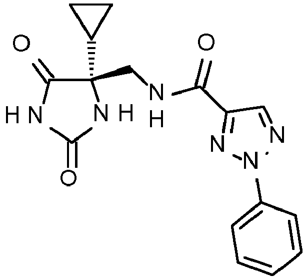

To a solution of (5R)-5-(aminomethyl)-5-cyclopropylimidazolidine-2,4-dione hydrochloride (40.0 mg, 195 µmol) and 2-phenyl-2H-1,2,3-triazole-4-carboxylic acid (36.8 mg, 195 µmol) in N,N-dimethylformamide (1 ml) was added 1-ethyl-3-(3-dimethylaminopropyl)carbodiimide hydrochloride (48.5 mg, 253 µmol), 1-hydroxybenzotriazole hydrate (38.7 mg, 253 µmol) and N,N-diisopropylethylamine (95 µl, 545 µmol). The reaction mixture was stirred at RT for 16 h. Water was added and the resulting precipitate was filtered off, washed with water and dried in vacuo. The obtained amount of product was 55 mg (100 % purity, 82 % of theory).
LC/MS (Method 10): Rₜ = 0.93 min, MS (ESIpos): m/z = 341 [M+H]⁺
¹H-NMR (400 MHz, DMSO-d6) δ [ppm]: 0.110 (0.60), 0.122 (1.17), 0.134 (1.51), 0.143 (2.04), 0.159 (1.39), 0.171 (0.74), 0.310 (0.54), 0.330 (1.49), 0.340 (1.65), 0.349 (2.15), 0.357 (1.56), 0.363 (1.25), 0.368 (1.22), 0.378 (0.97), 0.421 (2.82), 0.439 (5.15), 0.457 (4.49), 0.476 (1.65), 1.140 (0.77), 1.160 (1.59), 1.175 (2.40), 1.190 (1.55), 1.208 (0.64), 1.371 (1.33), 2.732 (0.89), 2.891 (1.02), 3.646 (1.46), 3.662 (1.66), 3.681 (3.54), 3.696 (3.28), 3.722 (3.29), 3.738 (3.43), 3.755 (1.57), 3.772 (1.45), 7.487 (1.78), 7.506 (4.58), 7.524 (3.04), 7.607 (5.85), 7.628 (11.15), 7.642 (2.77), 7.647 (4.67), 8.063 (8.44), 8.082 (7.60), 8.451 (2.09), 8.469 (16.00), 8.483 (1.99), 10.661 (3.31).

### Example 46

### N-{[(4R)-4-cyclopropyl-2,5-dioxoimidazolidin-4-yl]methyl}-3-(1-methyl-1H-pyrazol-4-yl)-1,2-oxazole-5-carboxamide

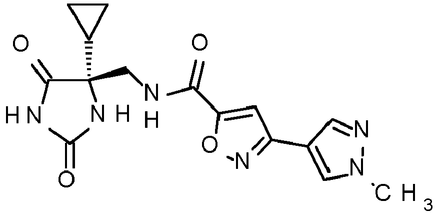

To a solution of (5R)-5-(aminomethyl)-5-cyclopropylimidazolidine-2,4-dione hydrochloride (60.0 mg, 292 µmol) and 3-(1-methyl-1H-pyrazol-4-yl)-1,2-oxazole-5-carboxylic acid (62.0 mg, 321 µmol) in N,N-dimethylformamide (1 ml) was added 1-ethyl-3-(3-dimethylaminopropyl)carbodiimide hydrochloride (72.7 mg, 379 µmol), (1.3 eq) 1-hydroxybenzotriazole hydrate (58.1 mg, 379 µmol) and N,N-diisopropylethylamine (140 µl, 817 µmol). The reaction mixture was stirred at RT for 16 h. The reaction was partitioned between ethyl acetate and a saturated solution of NaHCO₃ . The water layer was washed with ethyl acetate, dried over magnesium sulfate, filtered and concentrated. Acetonitrile was added to the crude and the resulting precipitate was filtered off and dried in vacuo to afford the product. The obtained amount of productof product was 35 mg (100 % purity, 35 % of theory).
LC/MS (Method 7): Rₜ = 0.84 min, MS (ESIpos): m/z = 345 [M+H]⁺
¹H-NMR (500 MHz, DMSO-d6) δ [ppm]: 0.116 (0.71), 0.125 (1.04), 0.135 (1.08), 0.144 (0.82), 0.333 (0.73), 0.345 (0.93), 0.354 (0.89), 0.361 (0.58), 0.370 (0.42), 0.422 (0.75), 0.439 (1.51), 0.450 (1.55), 0.455 (1.57), 0.466 (1.24), 0.476 (0.64), 1.135 (0.76), 1.140 (0.83), 1.151 (1.30), 1.161 (0.76), 1.167 (0.65), 3.600 (0.74), 3.612 (0.82), 3.628 (1.62), 3.640 (1.45), 3.668 (1.50), 3.682 (1.57), 3.695 (0.79), 3.709 (0.72), 3.904 (16.00), 7.373 (6.69), 7.599 (3.81), 7.932 (5.28), 8.309 (5.13), 8.872 (0.96), 8.884 (1.77), 8.897 (0.89), 10.651 (2.32).

### Example 47

### N-{[(4R)-4-cyclopropyl-2,5-dioxoimidazolidin-4-yl]methyl}-2-(pyridin-2-yl)-2H-1,2,3-triazole-4-carboxamide

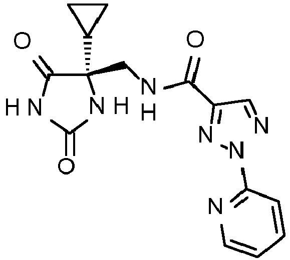

To a solution of (5R)-5-(aminomethyl)-5-cyclopropylimidazolidine-2,4-dione hydrochloride (100.0 mg, 486 µmol) and 2-(pyridin-2-yl)-2H-1,2,3-triazole-4-carboxylic acid (92.4 mg, 486µmol) in N,N-dimethylformamide (2.5 ml) was added 1-ethyl-3-(3-dimethylaminopropyl)carbodiimide hydrochloride (121.2 mg, 632 µmol), 1-hydroxybenzotriazole hydrate (96.8 mg, 632 µmol) and N,N-diisopropylethylamine (237 µl, 1.36 mmol). The reaction mixture was stirred at RT for 16 h. The reaction was partitioned between ethyl acetate and water. The organic layer was washed with water, dried over magnesium sulfate, filtered and concentrated. The mixture was filtered through a ion exchange filter, evaporated and dried in vacuo to afford the desired product. The obtained amount of product was 43 mg (100 % purity, 26 % of theory).
LC/MS (Method 7): Rₜ = 0.90 min, MS (ESIpos): m/z = 342 [M+H]⁺
¹H-NMR (600 MHz, DMSO-d6) δ [ppm]: 0.005 (2.69), 0.108 (0.65), 0.115 (1.29), 0.123 (1.51), 0.127 (2.02), 0.135 (1.73), 0.141 (1.52), 0.149 (0.80), 0.316 (0.58), 0.323 (0.77), 0.329 (1.78), 0.337 (1.84), 0.341 (1.91), 0.348 (1.65), 0.352 (1.31), 0.362 (0.94), 0.418 (3.02), 0.430 (5.81), 0.443 (4.95), 0.454 (1.70), 1.145 (0.86), 1.158 (1.86), 1.168 (2.61), 1.179 (1.70), 1.190 (0.75), 3.679 (1.08), 3.690 (1.26), 3.702 (4.57), 3.713 (7.80), 3.724 (4.46), 3.736 (1.21), 3.747 (1.10), 7.584 (2.65), 7.592 (3.04), 7.595 (3.09), 7.603 (2.90), 7.640 (6.39), 8.084 (3.81), 8.098 (6.52), 8.128 (3.02), 8.131 (3.07), 8.140 (3.49), 8.143 (3.70), 8.154 (1.77), 8.157 (1.77), 8.524 (16.00), 8.562 (2.09), 8.573 (4.23), 8.583 (2.08), 8.628 (3.62), 8.634 (3.64), 10.680 (5.62).

### Example 48

### rac-N-[(2,5-dioxo-4-(propan-2-yl)imidazolidin-4-yl)methyl]-2-phenyl-2H-1,2,3-triazole-4-carboxamide

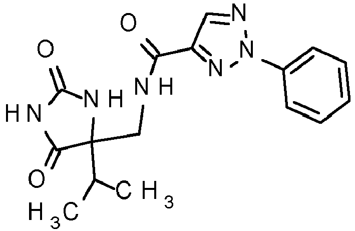

To a solution of rac-5-(aminomethyl)-5-(propan-2-yl)imidazolidine-2,4-dione (100.0 mg, 584 µmol) and 2-phenyl-2H-1,2,3-triazole-4-carboxylic acid (110.5 mg, 584 µmol) in N,N-dimethylformamide (2.4 ml) was added 1-ethyl-3-(3-dimethylaminopropyl)carbodiimide hydrochloride (145.6 mg, 759 µmol), 1-hydroxybenzotriazole hydrate (116.3 mg, 759 µmol) and N,N-diisopropylethylamine (284 µl, 1.6 mmol). The reaction mixture was stirred at RT for 16 h. Water was added in to the mixture and the resulting precipitate was filtered off, washed with water and dried in vacuo. The obtained amount of product was was 157 mg (100 % purity, 79% of theory).
LC-MS (Method 7): Rₜ = 1.25 min; MS (ESIpos): m/z = 343 [M+H]⁺
¹H-NMR (500 MHz, DMSO-d6) δ [ppm]: 0.006 (1.40), 0.848 (15.82), 0.861 (15.83), 0.956 (15.67), 0.969 (15.81), 1.979 (0.99), 1.992 (2.44), 2.006 (3.20), 2.019 (2.25), 2.033 (0.83), 2.074 (2.02), 3.604 (1.07), 3.617 (1.22), 3.631 (4.30), 3.645 (6.91), 3.658 (4.10), 3.674 (1.09), 3.686 (0.97), 7.491 (2.10), 7.506 (5.17), 7.521 (3.22), 7.609 (6.28), 7.625 (9.11), 7.641 (4.89), 7.810 (6.64), 8.056 (9.26), 8.072 (8.77), 8.315 (2.04), 8.327 (3.96), 8.340 (1.88), 8.464 (16.00), 10.661 (5.67).

### Example 49

### rac-[(4-tert-butyl-2,5-dioxoimidazolidin-4-yl)methyl]-2-phenyl-2H-1,2,3-triazole-4-carboxamide

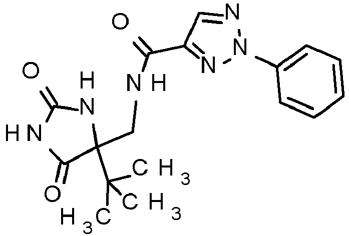

To a solution of *rac-*5-(aminomethyl)-5-tert-butylimidazolidine-2,4-dione (50.0 mg, 267 µmol) and 2-phenyl-2H-1,2,3-triazole-4-carboxylic acid (46.0 mg, 243 µmol) in N,N-dimethylformamide (2.2 ml) was added 1-ethyl-3-(3-dimethylaminopropyl)carbodiimide hydrochloride (60.5 mg, 316 µmol), (1.3 eq) 1-hydroxybenzotriazole hydrate (48.4 mg, 316 µmol) and N,N-diisopropylethylamine(120 µl, 680 µmol). The reaction mixture was stirred at RT for 16 h and then the mixture was purified by preparative HPLC (gradient acetonitrile/water with 0.1 % trifluoroacetic acid). Evaporation of the combined product fractions yielded the title compound. The obtained amount of product was 34 mg (100 % purity, 36% of theory).
LC-MS (Method 7): Rₜ = 1.36 min; MS (ESIpos): m/z = 357 [M+H]⁺
¹H-NMR (500 MHz, DMSO-d6) δ [ppm]: -0.007 (0.43), 1.022 (16.00), 2.074 (0.66), 3.688 (0.55), 3.700 (0.52), 3.779 (0.55), 3.792 (0.60), 3.806 (0.40), 7.491 (0.40), 7.506 (0.97), 7.521 (0.63), 7.610 (2.31), 7.626 (1.66), 7.638 (0.43), 7.641 (0.95), 8.045 (1.59), 8.047 (1.66), 8.062 (1.65), 8.065 (1.19), 8.288 (0.68), 8.447 (3.47), 10.599 (1.00).

### Example 50

### rac-N-[(4-cyclopentyl-2,5-dioxoimidazolidin-4-yl)methyl]-2-phenyl-2H-1,2,3-triazole-4-carboxamide

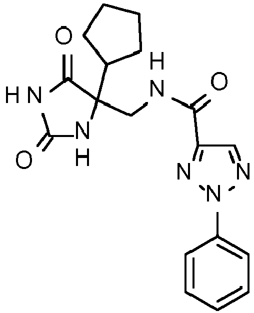

To a solution of rac-5-(aminomethyl)-5-cyclopentylimidazolidine-2,4-dione hydrochloride (50.0 mg, 214 µmol) and 2-phenyl-2H-1,2,3-triazole-4-carboxylic acid (40.5 mg, 214 µmol) in N,N-dimethylformamide (100 µl, 600 µmol) was added 1-ethyl-3-(3-dimethylaminopropyl)carbodiimide hydrochloride (53.3 mg, 278 µmol), 1-hydroxybenzotriazole hydrate (42.6 mg, 278 µmol) and N,N-diisopropylethylamine (100 µl, 600 µmol). The reaction mixture was stirred at RT for 16 h and then the mixture was purified by preparative HPLC (gradient acetonitrile/water with 0.1 % trifluoroacetic acid). Evaporation of the combined product fractions yielded the title compound. The obtained amount of product was 25 mg (100 % purity, 31% of theory).
LC-MS (Method 7): Rₜ = 1.46 min; MS (ESIpos): m/z = 369 [M+H]⁺
¹H-NMR (500 MHz, DMSO-d6) δ [ppm]: -0.007 (2.42), 0.006 (1.63), 1.214 (0.86), 1.223 (1.02), 1.233 (1.09), 1.241 (1.00), 1.339 (0.82), 1.359 (1.09), 1.374 (0.87), 1.397 (0.44), 1.490 (3.50), 1.506 (2.70), 1.523 (2.14), 1.540 (1.74), 1.548 (1.96), 1.556 (1.89), 1.565 (1.97), 1.771 (1.23), 1.787 (1.05), 2.073 (6.51), 2.180 (0.48), 2.196 (1.14), 2.214 (1.58), 2.231 (1.04), 3.615 (6.91), 3.628 (6.92), 7.490 (1.79), 7.505 (4.33), 7.520 (2.79), 7.609 (5.41), 7.626 (7.21), 7.637 (1.80), 7.641 (4.19), 7.845 (5.15), 8.055 (7.04), 8.057 (7.38), 8.072 (7.37), 8.074 (5.27), 8.381 (1.59), 8.394 (3.29), 8.406 (1.51), 8.462 (16.00), 10.654 (4.53).

### Example 51

### rac-N-[(4-butyl-2,5-dioxoimidazolidin-4-yl)methyl]-2-phenyl-2H-1,2,3-triazole-4-carboxamide

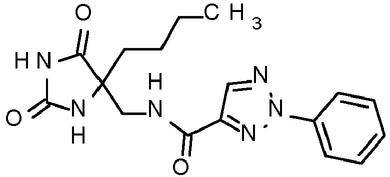

To a solution of rac-5-(aminomethyl)-5-butylimidazolidine-2,4-dione (40.0 mg, 216 µmol) and 2-phenyl-2H-1,2,3-triazole-4-carboxylic acid (44.9 mg, 238 µmol) in N,N-dimethylformamide (1.0 ml) was added 1-ethyl-3-(3-dimethylaminopropyl)carbodiimide hydrochloride (53.8 mg, 281 µmol), 1-hydroxybenzotriazole hydrate (43.0 mg, 281 µmol) and N,N-diisopropylethylamine (105 µl, 605 µmol). The reaction mixture was stirred at RT for 2 h and then the mixture was purified by preparative HPLC (gradient acetonitrile/water with 0.1 % trifluoroacetic acid). Evaporation of the combined product fractions yielded the title compound. The obtained amount of product was 33 mg (100 % purity, 43% of theory).
LC-MS (Method 7): Rₜ = 1.45 min; MS (ESIpos): m/z = 357 [M+H]⁺
¹H-NMR (500 MHz, DMSO-d6) δ [ppm]: -0.120 (0.42), -0.006 (5.65), 0.007 (3.17), 0.117 (0.40), 0.837 (5.51), 0.852 (12.53), 0.866 (6.41), 1.020 (0.45), 1.030 (0.79), 1.036 (0.85), 1.044 (1.23), 1.054 (1.10), 1.068 (0.94), 1.225 (0.68), 1.240 (1.86), 1.254 (3.94), 1.259 (3.54), 1.265 (4.44), 1.273 (3.56), 1.304 (0.50), 1.570 (0.74), 1.578 (0.83), 1.598 (1.80), 1.621 (1.38), 1.629 (1.06), 1.650 (1.07), 1.660 (1.06), 1.672 (1.53), 1.700 (0.76), 3.510 (1.03), 3.523 (1.22), 3.537 (3.51), 3.550 (3.55), 3.556 (3.41), 3.569 (3.30), 3.584 (1.09), 3.597 (1.01), 7.490 (1.89), 7.505 (4.44), 7.520 (2.88), 7.610 (5.56), 7.627 (7.48), 7.638 (1.92), 7.642 (4.25), 7.781 (5.40), 8.066 (7.16), 8.068 (7.70), 8.083 (7.64), 8.085 (5.58), 8.473 (16.00), 8.496 (1.71), 8.509 (3.40), 8.522 (1.60), 10.678 (4.59).

### Example 52

### rac-N-[4-cyclobutyl-2,5-dioxoimidazolidin-4-yl)methyl]-2-phenyl-2H-1,2,3-triazole-4-carboxamide

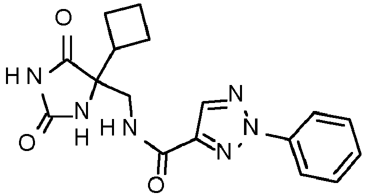

To a solution of rac-5-(aminomethyl)-5-cyclobutylimidazolidine-2,4-dione hydrochloride (100.0 mg, 455 µmol) and 2-phenyl-2H-1,2,3-triazole-4-carboxylic acid (86.1 mg, 455 µmol) in N,N-dimethylformamide (2.5 ml) was added 1-ethyl-3-(3-dimethylaminopropyl)carbodiimide hydrochloride (113.4 mg, 592 µmol), 1-hydroxybenzotriazole hydrate (90.6 mg, 592 µmol) and N,N-diisopropylethylamine (222 µl, 1.28 mmol). The reaction mixture was stirred at RT for 16 h. Water was added in to the mixture and the resulting precipitate was filtered off, washed with water and dried under vacuo afforded to afford the product. The obtained amount of product was 132 mg (100 % purity, 82 % of theory).
LC-MS (Method 9): Rₜ = 1.06 min; MS (ESIpos): m/z = 355 [M+H]⁺
¹H-NMR (400 MHz, DMSO-d6) δ [ppm]: -0.008 (2.37), 0.008 (2.13), 1.669 (1.44), 1.706 (1.29), 1.731 (1.54), 1.747 (1.33), 1.768 (2.12), 1.791 (2.41), 1.811 (2.37), 1.832 (1.42), 1.852 (0.92), 1.905 (1.94), 1.928 (2.71), 1.951 (1.92), 2.675 (0.71), 2.700 (1.41), 2.720 (2.13), 2.731 (2.11), 2.743 (1.31), 2.891 (1.81), 3.452 (0.94), 3.468 (1.10), 3.487 (3.61), 3.506 (4.33), 3.523 (3.49), 3.541 (1.00), 3.558 (0.93), 7.487 (1.76), 7.506 (4.63), 7.524 (3.16), 7.607 (5.67), 7.628 (8.11), 7.647 (4.31), 7.990 (5.77), 8.058 (8.03), 8.077 (7.90), 8.390 (1.62), 8.406 (3.16), 8.422 (1.52), 8.461 (16.00), 10.661 (4.60).

### Example 53

### rac-N-[(4-cyclohexyl-2,5-dioxoimidazolidin-4-yl)methyl]-2-phenyl-2H-1,2,3-triazole-4-carboxamide

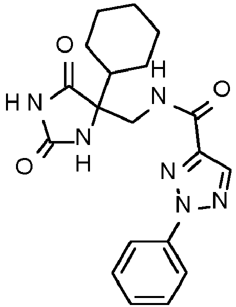

To a solution of *rac-*5-(aminomethyl)-5-cyclohexylimidazolidine-2,4-dione hydrochloride (50.0 mg, 202 µmol) and 2-phenyl-2H-1,2,3-triazole-4-carboxylic acid (38.2 mg, 202 µmol) in N,N-dimethylformamide (2.0 ml) was added 1-ethyl-3-(3-dimethylaminopropyl)carbodiimide hydrochloride (50.3 mg, 262 µmol), 1-hydroxybenzotriazole hydrate (40.2 mg, 262 µmol) and N,N-diisopropylethylamine (98 µl, 570 µmol). The reaction mixture was stirred at RT for 16 h and then the mixture was purified by preparative HPLC (gradient acetonitrile/water with 0.1 % trifluoroacetic acid). Evaporation of the combined product fractions yielded the title compound. The obtained amount of product was 18 mg (98 % purity, 23% of theory).
LC-MS (Method 7): Rₜ = 1.53 min; MS (ESIpos): m/z = 383 [M+H]⁺
¹H-NMR (600 MHz, DMSO-d6) δ [ppm]: 1.027 (1.36), 1.048 (3.98), 1.069 (4.72), 1.089 (2.16), 1.125 (2.01), 1.145 (5.18), 1.163 (4.96), 1.179 (3.43), 1.200 (2.27), 1.486 (2.54), 1.502 (2.99), 1.600 (2.78), 1.620 (2.60), 1.653 (1.88), 1.672 (3.19), 1.690 (4.50), 1.706 (2.34), 1.736 (2.71), 1.756 (2.34), 1.810 (2.64), 1.831 (2.47), 2.078 (2.14), 3.593 (2.21), 3.604 (2.43), 3.616 (4.37), 3.627 (4.07), 3.660 (4.09), 3.670 (4.33), 3.683 (2.32), 3.693 (2.14), 7.495 (2.82), 7.508 (6.49), 7.520 (4.15), 7.614 (7.19), 7.627 (12.77), 7.640 (6.19), 7.821 (10.47), 8.059 (12.44), 8.072 (11.76), 8.331 (2.89), 8.342 (5.84), 8.352 (2.89), 8.472 (16.00), 10.667 (9.33).

### Example 54

### rac-N-[(4-(cyclopropylmethyl)-2,5-dioxoimidazolidin-4-yl)methyl}]-2-phenyl-2H-1,2,3-triazole-4-carboxamide

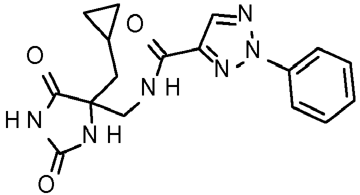

To a solution of *rac*-5-(aminomethyl)-5-(cyclopropylmethyl)imidazolidine-2,4-dione hydrochloride (50.0 mg, 225 µmol) and 2-phenyl-2H-1,2,3-triazole-4-carboxylic acid (38.8 mg, 205 µmol) in N,N-dimethylformamide (1.9 ml) was added 1-ethyl-3-(3-dimethylaminopropyl)carbodiimide hydrochloride (51.1 mg, 266 µmol), 1-hydroxybenzotriazole hydrate (40.8 mg, 266 µmol) and N,N-diisopropylethylamine (100 µl, 570 µmol). The reaction mixture was stirred at RT for 16 h and then the mixture was purified by preparative HPLC (gradient acetonitrile/water with 0.1 % trifluoroacetic acid). Evaporation of the combined product fractions yielded the title compound. The obtained amount of product was 13 mg (95 % purity, 16% of theory).
LC-MS (Method 7): Rₜ = 1.33 min; MS (ESIpos): m/z = 355 [M+H]⁺
¹H-NMR (500 MHz, DMSO-d6) δ [ppm]: 1.678 (0.83), 1.688 (1.02), 1.706 (2.09), 1.715 (2.01), 1.729 (2.38), 1.739 (2.26), 1.749 (1.78), 1.759 (2.26), 1.772 (2.13), 1.781 (2.45), 1.799 (1.08), 1.809 (1.18), 1.824 (0.87), 1.837 (1.21), 1.863 (1.78), 1.876 (1.49), 1.888 (1.02), 2.033 (1.39), 2.045 (1.63), 2.054 (1.66), 2.073 (1.59), 3.523 (1.63), 3.536 (1.84), 3.551 (4.40), 3.563 (4.19), 3.575 (4.10), 3.588 (4.03), 3.602 (1.58), 3.616 (1.41), 4.940 (3.90), 4.958 (4.01), 5.002 (3.79), 5.005 (3.52), 5.036 (4.08), 5.739 (1.02), 5.751 (1.99), 5.759 (1.23), 5.764 (1.29), 5.772 (2.57), 5.785 (2.45), 5.793 (1.08), 5.798 (1.09), 5.806 (1.61), 5.819 (0.73), 7.491 (2.51), 7.506 (5.52), 7.521 (3.55), 7.611 (6.88), 7.627 (9.87), 7.642 (5.09), 7.819 (7.46), 8.069 (10.50), 8.085 (9.30), 8.475 (16.00), 8.518 (1.36), 8.537 (2.51), 8.549 (4.54), 8.562 (2.12), 10.720 (6.49).

### Example 55

### rac-N-[(2,5-dioxo-4-propylimidazolidin-4-yl)methyl]-2-phenyl-2H-1,2,3-triazole-4-carboxamide

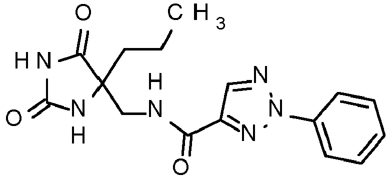

To a solution of *rac*-5-(aminomethyl)-5-propylimidazolidine-2,4-dione hydrochloride (40.0 mg, 193 µmol) and 2-phenyl-2H-1,2,3-triazole-4-carboxylic acid (40.1 mg, 212 µmol) in N,N-dimethylformamide (1.0 ml) was added 1-ethyl-3-(3-dimethylaminopropyl)carbodiimide hydrochloride (48.0 mg, 250 µmol), 1-hydroxybenzotriazole hydrate (38.3 mg, 250 µmol) and N,N-diisopropylethylamine (94 µl, 539 µmol). The reaction mixture was stirred at RT for 16 h and then the mixture was purified by preparative HPLC (gradient acetonitrile/water with 0.1 % trifluoroacetic acid). Evaporation of the combined product fractions yielded the title compound. The obtained amount of product was 11 mg (98 % purity, 17% of theory).
LC-MS (Method 7): Rₜ = 1.32 min; MS (ESIpos): m/z = 343 [M+H]⁺
¹H-NMR (600 MHz, DMSO-d6) δ [ppm]: 0.852 (7.15), 0.865 (16.00), 0.877 (8.21), 1.065 (0.49), 1.074 (0.91), 1.086 (1.47), 1.095 (1.46), 1.106 (1.51), 1.118 (0.92), 1.127 (0.55), 1.278 (0.55), 1.287 (0.91), 1.298 (1.49), 1.307 (1.32), 1.319 (1.43), 1.331 (0.80), 1.339 (0.47), 1.557 (0.91), 1.565 (1.02), 1.580 (2.04), 1.587 (1.84), 1.600 (1.70), 1.608 (1.38), 1.628 (1.62), 1.636 (1.79), 1.648 (1.78), 1.655 (1.80), 1.670 (0.93), 1.678 (0.75), 3.502 (6.00), 3.513 (4.13), 3.525 (4.77), 3.536 (4.06), 3.563 (3.60), 3.574 (3.70), 3.585 (1.95), 3.596 (1.81), 7.496 (2.07), 7.508 (4.80), 7.520 (2.99), 7.616 (5.42), 7.629 (9.31), 7.642 (4.61), 7.821 (7.24), 8.073 (9.26), 8.086 (8.55), 8.484 (12.56), 8.537 (2.13), 8.548 (4.25), 8.559 (2.07), 10.710 (6.17).

### Example 56

### N-{[(4R)-4-cyclopropyl-2,5-dioxoimidazolidin-4-yl]methyl}-1-phenyl-1H-pyrazole-4-carboxamide

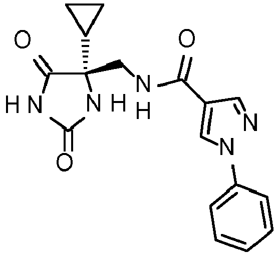

To a solution of (5R)-5-(aminomethyl)-5-cyclopropylimidazolidine-2,4-dione hydrochloride (40.0 mg, 195 µmol) and 1-phenyl-1H-pyrazole-4-carboxylic acid (36.6 mg, 195 µmol) in N,N-dimethylformamide (1.0 ml) was added 1-ethyl-3-(3-dimethylaminopropyl)carbodiimide hydrochloride (48.5 mg, 253 µmol), 1-hydroxybenzotriazole hydrate (38.7 mg, 253 µmol) and N,N-diisopropylethylamine (95 µl, 545 µmol). The reaction mixture was stirred at RT for 16 h. The reaction was partitioned between ethyl acetate and water. The organic layer was washed with water, dried over magnesium sulfate, filtered and concentrated. The obtained amount of product was 55 mg (97 % purity, 81% of theory).
LC-MS (Method 7): Rₜ = 1.11 min; MS (ESIpos): m/z = 340 [M+H]⁺
¹H-NMR (600 MHz, DMSO-d6) δ [ppm]: 0.005 (0.47), 0.111 (0.69), 0.120 (1.60), 0.127 (2.63), 0.136 (2.75), 0.144 (1.95), 0.152 (0.89), 0.318 (0.60), 0.325 (0.96), 0.328 (0.98), 0.333 (1.69), 0.341 (2.18), 0.349 (1.94), 0.356 (1.45), 0.363 (0.98), 0.414 (0.80), 0.422 (1.54), 0.429 (1.61), 0.431 (1.70), 0.437 (2.53), 0.446 (1.96), 0.451 (1.28), 0.463 (1.31), 0.472 (2.12), 0.480 (2.64), 0.489 (2.56), 0.496 (1.75), 0.505 (0.65), 0.983 (0.55), 1.125 (0.91), 1.133 (1.78), 1.138 (1.94), 1.142 (1.35), 1.147 (3.32), 1.152 (1.37), 1.156 (1.83), 1.161 (1.71), 1.169 (0.80), 2.732 (2.24), 2.891 (2.46), 3.478 (2.66), 3.487 (3.02), 3.501 (3.40), 3.510 (2.99), 3.774 (3.02), 3.786 (3.22), 3.797 (2.82), 3.810 (2.65), 7.359 (2.38), 7.371 (5.54), 7.383 (3.28), 7.521 (6.31), 7.535 (9.83), 7.547 (13.09), 7.833 (9.86), 7.846 (8.91), 8.150 (15.81), 8.206 (2.32), 8.217 (3.39), 8.227 (2.34), 8.953 (16.00), 10.625 (6.09).

### Example 57

### diamix-N-[4-(1-hydroxypropan-2-yl)-2,5-dioxoimidazolidin-4-yl)methyl]-2-phenyl-2H-1,2,3-triazole-4-carboxamide

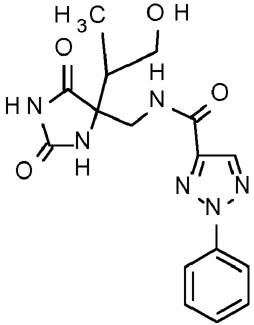

To a solution of *rac*-5-(aminomethyl)-5-(1-hydroxypropan-2-yl)imidazolidine-2,4-dione hydrochloride (50.0 mg, 224 µmol) and 2-phenyl-2H-1,2,3-triazole-4-carboxylic acid (42.3 mg, 224 µmol) in N,N-dimethylformamide (2 ml) was added 1-ethyl-3-(3-dimethylaminopropyl)carbodiimide hydrochloride (55.7 mg, 291 µmol), 1-hydroxybenzotriazole hydrate (44.5 mg, 291 µmol) and N,N-diisopropylethylamine (110 µl, 630 µmol). The reaction mixture was stirred at RT for 16 h and the 4 h at 50 °C. The mixture was purified by preparative HPLC (gradient acetonitrile/water with 0.1 % trifluoroacetic acid). Evaporation of the combined product fractions yielded the title compound which was criystallized after that wih acetonitrile, filtered and dried in vacuo. The obtained amount of product was 5 mg (95 % purity, 6% of theory).
LC-MS (Method 7): Rₜ = 1.02 min; MS (ESIpos): m/z = 359 [M+H]⁺
¹H-NMR (500 MHz, DMSO-d6) δ [ppm]: 0.849 (15.95), 0.862 (16.00), 0.946 (1.25), 0.959 (1.22), 1.976 (1.48), 1.989 (2.65), 2.001 (2.62), 2.014 (1.47), 2.074 (4.38), 3.356 (1.42), 3.367 (2.16), 3.378 (2.88), 3.388 (2.42), 3.399 (1.48), 3.570 (1.64), 3.581 (2.98), 3.592 (3.00), 3.602 (2.73), 3.612 (3.43), 3.625 (2.52), 3.639 (3.07), 3.653 (2.73), 3.788 (2.75), 3.800 (2.90), 3.815 (2.21), 3.828 (2.04), 4.666 (0.51), 4.796 (3.11), 4.806 (6.27), 4.816 (2.85), 7.490 (2.25), 7.505 (5.27), 7.520 (3.39), 7.608 (6.51), 7.624 (9.73), 7.640 (10.90), 7.850 (0.53), 8.055 (9.46), 8.072 (9.08), 8.368 (2.09), 8.381 (3.97), 8.393 (1.93), 8.456 (14.98), 8.465 (1.53), 10.611 (0.51), 10.657 (5.78).

### Example 58

### rac-N-[4-tert-butyl-2,5-dioxoimidazolidin-4-yl)methyl]-1-phenyl-1H-pyrazole-4-carboxamide

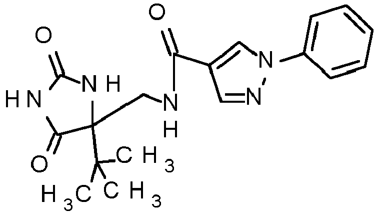

To a solution of *rac*-5-(aminomethyl)-5-tert-butylimidazolidine-2,4-dione (50.0 mg, 267 µmol) and 1-phenyl-1H-pyrazole-4-carboxylic acid (45.7 mg, 243 µmol) in N,N-dimethylformamide (2.2 ml) was added 1-ethyl-3-(3-dimethylaminopropyl)carbodiimide hydrochloride (60.5 mg, 316 µmol), 1-hydroxybenzotriazole hydrate (48.4 mg, 316 µmol) and N,N-diisopropylethylamine (120 µl, 680 µmol). The reaction mixture was stirred at RT for 16 h and then the mixture was purified by preparative HPLC (gradient acetonitrile/water with 0.1 % trifluoroacetic acid). Evaporation of the combined product fractions yielded the title compound. The obtained amount of product was 59 mg (100 % purity, 62% of theory).
LC-MS (Method 7): Rₜ = 1.25 min; MS (ESIpos): m/z = 356 [M+H]⁺
¹H-NMR (400 MHz, DMSO-d6) δ [ppm]: -0.008 (0.54), 0.008 (0.46), 1.019 (16.00), 2.073 (1.20), 3.497 (0.43), 3.517 (0.50), 3.532 (0.45), 3.814 (0.45), 3.831 (0.46), 7.350 (0.41), 7.369 (1.01), 7.387 (0.63), 7.434 (1.15), 7.511 (1.16), 7.532 (1.69), 7.551 (1.03), 7.812 (1.78), 7.831 (1.59), 8.115 (2.82), 8.142 (0.61), 8.910 (2.67), 10.554 (1.04).

### Example 59

### N-{[(4R)-4-cyclopropyl-2,5-dioxoimidazolidin-4-yl]methyl}-2-phenyl-1,3-oxazole-5-carboxamide

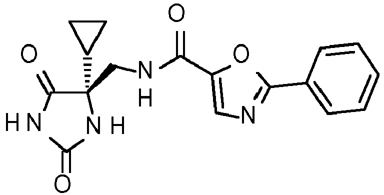

To a solution of (5R)-5-(aminomethyl)-5-cyclopropylimidazolidine-2,4-dione hydrochloride (40.0 mg, 195 µmol) and 2-phenyl-1,3-oxazole-5-carboxylic acid (33.5 mg, 177 µmol) in N,N-dimethylformamide (1.6 ml) was added 1-ethyl-3-(3-dimethylaminopropyl)carbodiimide hydrochloride (44.1 mg, 230 µmol), 1-hydroxybenzotriazole hydrate (35.2 mg, 230 µmol) and N,N-diisopropylethylamine (86 µl, 500 µmol). The reaction mixture was stirred at RT for 16 h and then the mixture was purified by preparative HPLC (gradient acetonitrile/water with 0.1 % trifluoroacetic acid). Evaporation of the combined product fractions yielded the title compound. The obtained amount of product was 44 mg (100 % purity, 66% of theory).
LC-MS (Method 7): Rₜ = 1.13 min; MS (ESIpos): m/z = 341 [M+H]⁺
¹H-NMR (400 MHz, DMSO-d6) δ [ppm]: -0.008 (1.33), 0.008 (1.26), 0.126 (0.97), 0.136 (1.67), 0.149 (1.71), 0.160 (1.25), 0.174 (0.57), 0.339 (1.06), 0.354 (1.37), 0.360 (1.08), 0.365 (1.38), 0.375 (0.97), 0.386 (0.68), 0.421 (0.49), 0.432 (1.15), 0.453 (2.27), 0.468 (2.42), 0.475 (2.18), 0.489 (1.97), 0.503 (0.93), 1.132 (0.57), 1.146 (1.16), 1.152 (1.26), 1.166 (2.20), 1.179 (1.15), 1.186 (1.02), 1.200 (0.47), 3.545 (1.54), 3.560 (1.75), 3.580 (2.34), 3.594 (2.08), 3.735 (2.15), 3.753 (2.25), 3.769 (1.67), 3.787 (1.61), 7.576 (1.72), 7.586 (5.80), 7.590 (6.34), 7.598 (11.21), 7.603 (14.42), 7.916 (16.00), 8.099 (4.69), 8.110 (4.78), 8.118 (5.28), 8.124 (4.21), 8.642 (1.32), 8.658 (2.38), 8.674 (1.26), 10.647 (3.88).

### Example 60

### N-{[(4R)-4-cyclopropyl-2,5-dioxoimidazolidin-4-yl]methyl}-2-(pyridin-2-yl)-2H-1,2,3-triazole-4-carboxamide

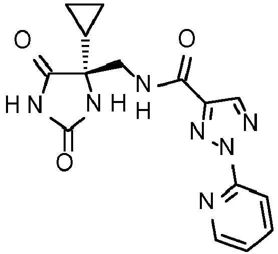

To a solution of (5R)-5-(aminomethyl)-5-cyclopropylimidazolidine-2,4-dione hydrochloride and 2-(pyridin-2-yl)-2H-1,2,3-triazole-4-carboxylic acid (92.5 mg, 486 µmol) in N,N-dimethylformamide (0.63 ml) was added 1-ethyl-3-(3-dimethylaminopropyl)carbodiimide hydrochloride (121 mg, 632 µmol), 1-hydroxybenzotriazole hydrate (96.8 mg, 632 µmol) and N,N-diisopropylethylamine (240 µl, 136 mmol). The reaction mixture was stirred at RT for 16h. The mixture was partitioned between ethyl acetate and water. The organic layer was washed with water, dried over magnesium sulfate, filtered and concentrated. The crude was solved in acetonitrile and filtered through an ion exchange filter and then dried in vacuo. The obtained amount of product was 43 mg (100 % purity, 26 % of theory).
LC-MS (Method 7): Rₜ = 0.90 min; MS (ESIpos): m/z = 342 [M+H]⁺
¹H-NMR (400 MHz, DMSO-d6) δ [ppm]: -0.008 (1.16), 0.132 (1.38), 0.162 (0.64), 0.308 (1.21), 0.326 (1.45), 0.357 (0.76), 0.394 (2.23), 0.412 (4.22), 0.431 (3.37), 0.447 (1.07), 1.125 (0.59), 1.145 (1.38), 1.160 (1.85), 1.176 (1.21), 1.192 (0.47), 2.096 (1.35), 2.328 (1.04), 2.524 (2.92), 2.671 (0.99), 3.691 (4.88), 3.707 (4.76), 3.926 (1.04), 7.512 (1.68), 7.573 (2.08), 7.576 (2.25), 7.584 (2.16), 7.588 (2.58), 7.590 (2.59), 7.593 (2.44), 7.603 (2.35), 7.606 (2.44), 8.074 (2.63), 8.095 (5.81), 8.118 (3.01), 8.122 (3.03), 8.136 (2.77), 8.140 (3.11), 8.156 (1.30), 8.161 (1.40), 8.514 (16.00), 8.537 (2.78), 8.552 (1.35), 8.624 (3.04), 8.634 (3.04), 10.653 (1.07).

### Example 61

### rac-N-[(4-(oxan-4-yl)-2,5-dioxoimidazolidin-4-yl)methyl]-2-phenyl-2H-1,2,3-triazole-4-carboxamide

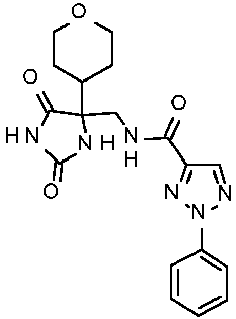

To a solution of *rac*-5-(aminomethyl)-5-(oxan-4-yl)imidazolidine-2,4-dione hydrochloride (264 mg, 1.06 mmol) and 2-phenyl-2H-1,2,3-triazole-4-carboxylic acid (200 mg, 1.06 mmol) in N,N-dimethylformamide (5.43 ml) was added 1-ethyl-3-(3-dimethylaminopropyl)carbodiimide hydrochloride (263 mg, 1.37 mmol), 1-hydroxybenzotriazole hydrate (210 mg, 1.37 mmol) and N,N-diisopropylethylamine (520 µl, 2.96 mmol). The reaction mixture was stirred at RT for 16 h. Water was added in to the mixture and the resulting precipitate was filtered off, washed with water and dried in vacuo. The obtained amount of product was 327 mg (100 % purity, 80 % of theory).
LC-MS (Method 7): Rₜ = 1.16 min; MS (ESIpos): m/z = 385 [M+H]⁺
¹H-NMR (400 MHz, DMSO-d6) δ [ppm]: 1.324 (2.26), 1.353 (4.79), 1.372 (2.96), 1.384 (2.30), 1.405 (1.90), 1.424 (2.16), 1.435 (2.26), 1.455 (1.90), 1.465 (1.83), 1.485 (0.73), 1.497 (0.63), 1.654 (2.69), 1.685 (2.26), 1.922 (1.56), 1.951 (2.79), 1.981 (1.26), 2.328 (0.47), 2.366 (0.53), 2.670 (0.53), 2.710 (0.60), 2.731 (1.70), 2.890 (2.10), 3.222 (2.36), 3.255 (4.69), 3.275 (2.59), 3.287 (2.89), 3.589 (1.66), 3.605 (1.90), 3.623 (4.46), 3.639 (4.16), 3.659 (4.19), 3.674 (4.42), 3.693 (1.90), 3.709 (1.73), 3.833 (2.49), 3.841 (2.69), 3.868 (2.49), 3.885 (2.73), 3.893 (2.73), 3.914 (2.46), 7.487 (2.16), 7.506 (5.92), 7.524 (4.02), 7.606 (7.28), 7.626 (11.04), 7.645 (5.79), 7.896 (8.81), 8.056 (10.41), 8.076 (10.25), 8.379 (2.43), 8.395 (5.19), 8.410 (2.49), 8.465 (16.00), 10.714 (7.62).

### Example 62

### rac-N-[(2,5-dioxo-4-(propan-2-yl)imidazolidin-4-yl)methyl]-2-(4-fluorophenyl)-2H-1,2,3-triazole-4-carboxamide

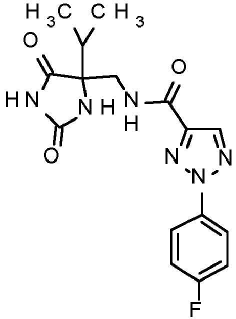

To a solution of *rac*-5-(aminomethyl)-5-(propan-2-yl)imidazolidine-2,4-dione hydrochloride (70.0 mg, 409 µmol)and 2-(4-fluorophenyl)-2H-1,2,3-triazole-4-carboxylic acid (84.7 mg, 409 µmol) in N,N-dimethylformamide (2.10 ml) was added 1-ethyl-3-(3-dimethylaminopropyl)carbodiimide hydrochloride (102 mg, 532 µmol), 1-hydroxybenzotriazole hydrate (81.4 mg, 532 µmol) and N,N-diisopropylethylamine (200 µl, 1.14 mmol). The reaction mixture was stirred at RT for 16 h. Water was added in to the mixcture and the resulting precipitate was filtered off, washed with water and dried in vacuo. The obtained amount of product was 53 mg (98 % purity, 35 % of theory).
LC-MS (Method 7): Rₜ = 1.35 min; MS (ESIpos): m/z = 361 [M+H]⁺
¹H-NMR (400 MHz, DMSO-d6) δ [ppm]: 0.842 (15.96), 0.859 (16.00), 0.951 (15.76), 0.968 (15.92), 1.967 (1.03), 1.984 (2.48), 2.001 (3.24), 2.018 (2.26), 2.035 (0.82), 2.328 (0.65), 2.367 (0.46), 2.670 (0.69), 2.710 (0.49), 2.731 (0.63), 2.891 (0.78), 3.585 (1.19), 3.602 (1.41), 3.619 (4.25), 3.637 (5.06), 3.656 (4.06), 3.675 (1.24), 3.691 (1.08), 7.458 (5.67), 7.464 (2.23), 7.480 (10.01), 7.502 (5.86), 7.511 (0.64), 7.804 (8.44), 8.073 (6.02), 8.084 (6.24), 8.090 (3.83), 8.095 (5.94), 8.107 (5.39), 8.323 (2.09), 8.339 (4.05), 8.354 (1.90), 8.464 (13.50), 10.654 (5.41).

### Example 63

### rac-N-[(4-tert-butyl-2,5-dioxoimidazolidin-4-yl)methyl]-2-(4-fluorophenyl)-2H-1,2,3-triazole-4-carboxamide

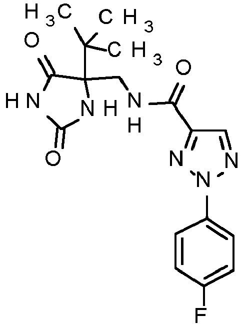

To a solution of *rac*-5-(aminomethyl)-5-tert-butylimidazolidine-2,4-dione (70.0 mg, 378 µmol) and 2-(4-fluorophenyl)-2H-1,2,3-triazole-4-carboxylic acid (78.3 mg, 378 µmol) in N,N-dimethylformamide (1.9 ml) was added 1-ethyl-3-(3-dimethylaminopropyl)carbodiimide hydrochloride (94.2 mg, 491 µmol), 1-hydroxybenzotriazole hydrate (75.2 mg, 491 µmol) and N,N-diisopropylethylamine (180 µl, 1.05 mmol). The reaction mixture was stirred at RT for 16 h. Water was added in to the mixture and the resulting precipitate was filtered off, washed with water and dried in vacuo. The obtained amount of product was 14 mg (100 % purity, 10 % of theory).
LC-MS (Method 7): Rₜ = 1.44 min; MS (ESIpos): m/z = 375 [M+H]⁺
¹H-NMR (400 MHz, DMSO-d6) δ [ppm]: 1.018 (16.00), 3.690 (0.62), 3.705 (0.57), 3.759 (0.60), 3.776 (0.64), 7.462 (1.07), 7.484 (1.93), 7.506 (1.11), 7.599 (1.62), 8.061 (1.18), 8.073 (1.24), 8.078 (0.79), 8.084 (1.17), 8.096 (1.05), 8.285 (0.40), 8.302 (0.75), 8.447 (2.82), 10.594 (0.92).

### Example 64

### rac-N-[4-(cyclopropylmethyl)-2,5-dioxoimidazolidin-4-yl)methyl]-2-(4-fluorophenyl)-2H-1,2,3-triazole-4-carboxamide

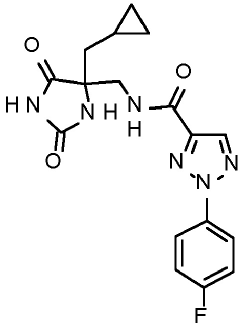

To a solution of *rac*-5-(aminomethyl)-5-(cyclopropylmethyl)imidazolidine-2,4-dione hydrochloride (70.0 mg, 319 µmol) and 2-(4-fluorophenyl)-2H-1,2,3-triazole-4-carboxylic acid (66.0 mg, 319 µmol) in N,N-dimethylformamide (1.6 ml) was added 1-ethyl-3-(3-dimethylaminopropyl)carbodiimide hydrochloride (79.4 mg, 414 µmol), 1-hydroxybenzotriazole hydrate (63.4 mg, 414 µmol) and N,N-diisopropylethylamine (160 µl, 892 µmol). The reaction mixture was stirred at RT for 16 h. Water was added in to the mixture and the resulting precipitate was filtered off, washed with water and dried in vacuo. The obtained amount of product was 43 mg (98 % purity, 36 % of theory).
LC-MS (Method 7): Rₜ = 1.42 min; MS (ESIpos): m/z = 373 [M+H]⁺
¹H-NMR (400 MHz, DMSO-d6) δ [ppm]: 0.008 (1.19), 1.663 (0.58), 1.676 (0.85), 1.698 (1.78), 1.710 (1.79), 1.726 (2.48), 1.739 (3.51), 1.751 (2.35), 1.766 (1.95), 1.778 (2.41), 1.800 (0.93), 1.812 (1.95), 1.828 (1.05), 1.844 (1.31), 1.861 (1.57), 1.876 (1.36), 1.890 (0.94), 2.025 (1.19), 2.052 (1.40), 2.087 (0.74), 2.328 (0.69), 2.367 (0.45), 2.670 (0.78), 2.710 (0.53), 2.731 (0.94), 2.891 (1.17), 3.508 (1.32), 3.524 (1.59), 3.542 (4.28), 3.558 (4.19), 3.567 (4.17), 3.584 (4.09), 3.602 (1.42), 3.618 (1.28), 4.938 (3.57), 4.963 (3.72), 4.995 (3.34), 5.000 (3.21), 5.038 (3.72), 5.042 (3.50), 5.727 (0.94), 5.742 (1.99), 5.752 (1.11), 5.758 (1.11), 5.768 (2.56), 5.785 (2.42), 5.795 (0.97), 5.801 (0.99), 5.811 (1.64), 5.827 (0.71), 7.460 (6.15), 7.466 (2.43), 7.483 (10.76), 7.499 (2.39), 7.504 (6.47), 7.811 (8.95), 8.085 (6.55), 8.096 (6.86), 8.102 (4.17), 8.107 (6.55), 8.119 (6.01), 8.475 (16.00), 8.541 (2.17), 8.557 (4.35), 8.572 (2.05), 10.713 (4.61).

### Example 65

### rac-N-[(2,5-dioxo-4-propylimidazolidin-4-yl)methyl]-2-(4-fluorophenyl)-2H-1,2,3-triazole-4-carboxamide

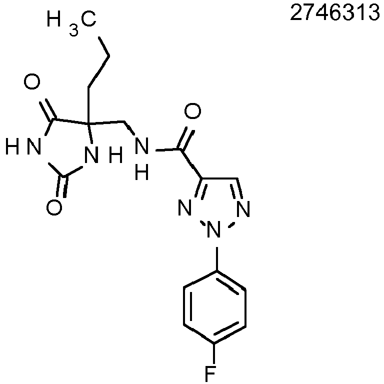

To a solution of *rac*-5-(aminomethyl)-5-propylimidazolidine-2,4-dione hydrochloride (70.0 mg, 337 µmol) and 2-(4-fluorophenyl)-2H-1,2,3-triazole-4-carboxylic acid (69.8 mg, 337 µmol) in N,N-dimethylformamide (1.7 ml) was added 1-ethyl-3-(3-dimethylaminopropyl)carbodiimide hydrochloride (84.0 mg, 438 µmol), 1-hydroxybenzotriazole hydrate (67.1 mg, 438 µmol) and N,N-diisopropylethylamine (160 µl, 944 µmol). The reaction mixture was stirred at RT for 16 h. Water was added in to the mixture and the resulting precipitate was filtered off, washed with water and dried in vacuo. The obtained amount of product was 45 mg (100 % purity, 36 % of theory).
LC-MS (Method 7): Rₜ = 1.38 min; MS (ESIpos): m/z = 361 [M+H]⁺
¹H-NMR (400 MHz, DMSO-d6) δ [ppm]: 0.844 (6.75), 0.863 (16.00), 0.881 (8.10), 1.052 (0.48), 1.066 (0.89), 1.083 (1.36), 1.098 (1.41), 1.113 (1.38), 1.130 (0.89), 1.145 (0.56), 1.264 (0.62), 1.277 (0.96), 1.294 (1.40), 1.308 (1.30), 1.325 (1.38), 1.337 (0.73), 1.343 (0.78), 1.355 (0.46), 1.540 (0.80), 1.552 (0.97), 1.574 (2.06), 1.586 (1.84), 1.603 (2.08), 1.615 (3.09), 1.628 (2.04), 1.644 (1.77), 1.656 (1.67), 1.678 (0.81), 1.691 (0.55), 3.488 (1.31), 3.503 (1.52), 3.522 (3.84), 3.538 (3.63), 3.551 (3.60), 3.567 (3.66), 3.585 (1.37), 3.602 (1.23), 7.460 (5.03), 7.482 (9.13), 7.504 (5.21), 7.784 (8.05), 8.084 (5.41), 8.096 (5.59), 8.101 (3.45), 8.107 (5.28), 8.118 (4.83), 8.473 (11.89), 8.500 (2.01), 8.516 (3.89), 8.532 (1.81), 10.679 (5.50).

### Example 66

### rac-N-[(4-cyclopentyl-2,5-dioxoimidazolidin-4-yl]methyl]-2-(4-fluorophenyl)-2H-1,2,3-triazole-4-carboxamide

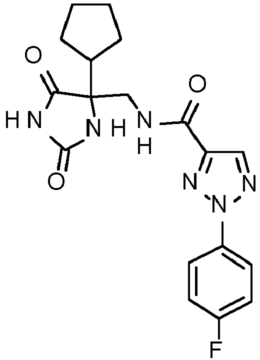

To a solution of *rac*-5-(aminomethyl)-5-cyclopentylimidazolidine-2,4-dione hydrochloride (100 mg, 507 µmol) and 2-(4-fluorophenyl)-2H-1,2,3-triazole-4-carboxylic acid (105 mg, 507 µmol) in N,N-dimethylformamide (2.6 ml) was added 1-ethyl-3-(3-dimethylaminopropyl)carbodiimide hydrochloride (126 mg, 659 µmol), 1-hydroxybenzotriazole hydrate (101 mg, 659 µmol) and N,N-diisopropylethylamine (250 µl, 1.420 mmol). The reaction mixture was stirred at RT for 16 h. Water was added in to the mixture and the resulting precipitate was filtered off, washed with water and dried in vacuo. The obtained amount of product was 102 mg (96 % purity, 50 % of theory).
LC-MS (Method 7): Rₜ = 1.51 min; MS (ESIpos): m/z = 387 [M+H]⁺
¹H-NMR (400 MHz, DMSO-d6) δ [ppm]: -0.149 (0.40), -0.008 (3.37), 0.008 (4.08), 0.937 (0.52), 0.953 (0.53), 1.230 (1.42), 1.274 (0.56), 1.332 (0.98), 1.354 (1.42), 1.370 (1.65), 1.401 (0.73), 1.469 (2.88), 1.488 (4.63), 1.502 (4.62), 1.530 (3.62), 1.546 (3.19), 1.555 (3.01), 1.566 (2.67), 1.767 (1.59), 2.167 (0.57), 2.189 (1.49), 2.210 (1.99), 2.230 (1.33), 2.328 (0.40), 2.366 (0.52), 2.670 (0.46), 2.710 (0.55), 2.731 (0.60), 2.891 (0.77), 3.571 (0.42), 3.587 (0.59), 3.605 (5.73), 3.609 (5.77), 3.624 (6.26), 3.644 (0.65), 3.660 (0.44), 7.458 (5.85), 7.464 (2.25), 7.480 (10.02), 7.497 (2.22), 7.502 (6.40), 7.511 (0.79), 7.839 (8.55), 8.063 (0.70), 8.072 (6.03), 8.084 (6.47), 8.089 (3.96), 8.095 (6.39), 8.101 (2.53), 8.107 (5.88), 8.388 (1.95), 8.405 (4.12), 8.420 (1.97), 8.462 (16.00), 10.648 (3.85).

### Example 67

### rac-2-(4-fluorophenyl)-N-[4-(oxan-4-yl)-2,5-dioxoimidazolidin-4-yl)methyl]-2H-1,2,3-triazole-4-carboxamide

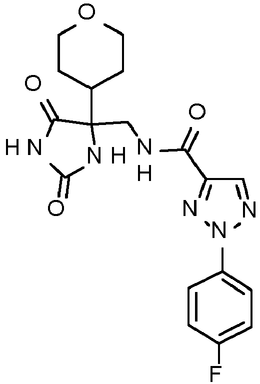

To a solution of *rac*-5-(aminomethyl)-5-(oxan-4-yl)imidazolidine-2,4-dione hydrochloride (183 mg, 733 µmol) and 2-(4-fluorophenyl)-2H-1,2,3-triazole-4-carboxylic acid (152 mg, 733 µmol) in N,N-dimethylformamide (3.8 ml) was added 1-ethyl-3-(3-dimethylaminopropyl)carbodiimide hydrochloride (183 mg, 953 µmol), 1-hydroxybenzotriazole hydrate (146 mg, 953 µmol) and N,N-diisopropylethylamine (360 µl, 2.05 mmol). The reaction mixture was stirred at RT for 16 h. Water was added into the mixture and the resulting precipitate was filtered off, washed with water and dried in vacuo. The obtained amount of product was 32 mg (100 % purity, 11 % of theory).
LC-MS (Method 7): Rₜ = 1.21 min; MS (ESIpos): m/z = 403 [M+H]⁺
¹H-NMR (400 MHz, DMSO-d6) δ [ppm]: 1.235 (0.60), 1.323 (2.31), 1.353 (4.77), 1.373 (2.44), 1.384 (2.23), 1.404 (1.81), 1.422 (2.15), 1.433 (2.20), 1.452 (1.89), 1.464 (1.84), 1.484 (0.81), 1.651 (2.60), 1.682 (2.20), 1.919 (1.50), 1.949 (2.60), 1.978 (1.21), 2.328 (0.55), 2.367 (0.60), 2.670 (0.55), 2.710 (0.60), 2.731 (1.00), 2.891 (1.21), 3.231 (2.44), 3.253 (4.72), 3.580 (1.65), 3.596 (1.84), 3.614 (3.88), 3.631 (3.62), 3.658 (3.67), 3.674 (3.88), 3.692 (1.86), 3.708 (1.68), 3.840 (2.60), 3.869 (2.41), 3.884 (2.62), 3.894 (2.60), 3.913 (2.36), 7.460 (6.22), 7.482 (11.38), 7.503 (6.66), 7.890 (9.26), 8.075 (6.77), 8.087 (7.29), 8.092 (5.01), 8.098 (7.06), 8.110 (6.37), 8.391 (2.39), 8.406 (4.77), 8.422 (2.31), 8.466 (16.00), 10.710 (5.69).

### Example 68

### rac-N-[(4-butyl-2,5-dioxoimidazolidin-4-yl)methyl]-2-(4-fluorophenyl)-2H-1,2,3-triazole-4-carboxamide

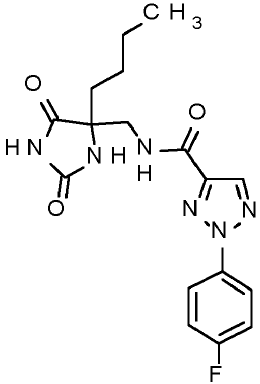

To a solution of *rac*-5-(aminomethyl)-5-butylimidazolidine-2,4-dione (200.0 mg, 1.08 mmol) and 2-(4-fluorophenyl)-2H-1,2,3-triazole-4-carboxylic acid (223.7 mg, 1.080 mmol) in N,N-dimethylformamide (5.5 ml) was added 1-ethyl-3-(3-dimethylaminopropyl)carbodiimide hydrochloride (269.1 mg, 1.40 mmol), 1-hydroxybenzotriazole hydrate (215.0 mg, 1.40 mmol) and N,N-diisopropylethylamine (527 µl, 3.02 mmol). The reaction mixture was stirred at RT for 16 h. Water was added in to the mixture and the resulting precipitate was filtered off, washed with water and dried in vacuo. The obtained amount of product was 50 mg (100 % purity, 12 % of theory).
LC-MS (Method 7): Rₜ = 1.49 min; MS (ESIpos): m/z = 375 [M+H]⁺
¹H-NMR (400 MHz, DMSO-d6) δ [ppm]: -0.149 (0.49), -0.008 (5.01), 0.008 (4.65), 0.146 (0.49), 0.832 (6.56), 0.850 (16.00), 0.867 (8.38), 1.007 (0.53), 1.021 (1.02), 1.038 (1.60), 1.050 (1.51), 1.068 (1.33), 1.213 (0.89), 1.232 (2.70), 1.249 (5.14), 1.262 (6.12), 1.274 (5.27), 1.290 (2.08), 1.557 (0.80), 1.567 (1.02), 1.592 (2.26), 1.620 (1.95), 1.631 (1.77), 1.639 (1.60), 1.652 (1.51), 1.667 (1.99), 1.701 (0.93), 1.714 (0.53), 2.367 (0.49), 2.670 (0.40), 2.710 (0.49), 3.495 (1.15), 3.511 (1.37), 3.529 (4.48), 3.547 (6.60), 3.564 (4.39), 3.582 (1.33), 3.598 (1.20), 7.459 (6.03), 7.465 (2.35), 7.481 (10.50), 7.498 (2.39), 7.503 (6.65), 7.512 (0.80), 7.774 (8.02), 8.074 (0.75), 8.083 (6.29), 8.088 (2.88), 8.094 (6.69), 8.100 (4.21), 8.105 (6.60), 8.112 (2.66), 8.117 (6.12), 8.472 (15.96), 8.502 (2.13), 8.518 (4.39), 8.534 (2.08), 10.674 (5.81).

### Example 69

### rac-N-[(4-cyclobutyl-2,5-dioxoimidazolidin-4-yl)methyl]-2-(4-fluorophenyl)-2H-1,2,3-triazole-4-carboxamide

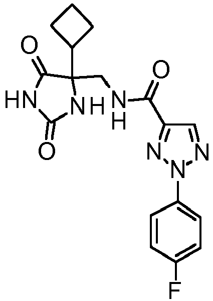

To a solution of *rac*-5-(aminomethyl)-5-cyclobutylimidazolidine-2,4-dione hydrochloride (100 mg, 546 µmol) and 2-(4-fluorophenyl)-2H-1,2,3-triazole-4-carboxylic acid (113 mg, 546 µmol) in N,N-dimethylformamide (2.80 ml) was added 1-ethyl-3-(3-dimethylaminopropyl)carbodiimide hydrochloride (136 mg, 710 µmol), 1-hydroxybenzotriazole hydrate (109 mg, 710 µmol) and N,N-diisopropylethylamine (266 µl, 1.58 mmol). The reaction mixture was stirred at RT for 16 h. Water was added in to the mixtue and the resulting precipitate was filtered off, washed with water and dried in vacuo. The obtained amount of product was 104 mg (98 % purity, 50 % of theory).
LC-MS (Method 7): Rₜ = 1.43 min; MS (ESIpos): m/z = 373 [M+H]⁺
¹H-NMR (400 MHz, DMSO-d6) δ [ppm]: -0.008 (1.97), 0.008 (2.47), 1.668 (1.58), 1.710 (1.43), 1.724 (1.64), 1.730 (1.70), 1.746 (1.45), 1.767 (2.31), 1.789 (2.67), 1.809 (2.60), 1.821 (1.54), 1.830 (1.64), 1.851 (1.00), 1.872 (0.62), 1.904 (2.09), 1.927 (2.96), 1.939 (1.72), 1.950 (2.12), 1.972 (0.76), 2.674 (0.64), 2.696 (1.53), 2.717 (2.37), 2.732 (1.64), 2.738 (1.53), 2.759 (0.45), 2.891 (1.12), 3.445 (1.15), 3.460 (1.35), 3.479 (3.79), 3.495 (3.69), 3.504 (3.74), 3.520 (3.77), 3.538 (1.31), 3.555 (1.20), 7.452 (0.56), 7.461 (5.45), 7.466 (2.05), 7.483 (9.43), 7.499 (2.01), 7.505 (6.00), 7.513 (0.69), 7.983 (7.71), 8.066 (0.63), 8.075 (5.73), 8.080 (2.47), 8.087 (6.08), 8.092 (3.67), 8.098 (6.01), 8.104 (2.31), 8.110 (5.56), 8.399 (1.82), 8.414 (3.77), 8.430 (1.79), 8.461 (16.00), 8.476 (0.46), 10.657 (4.43).

### Example 70

### ent-N-[(2,5-dioxo-4-(propan-2-yl)imidazolidin-4-yl)methyl]-2-phenyl-2H-1,2,3-triazole-4-carboxamide

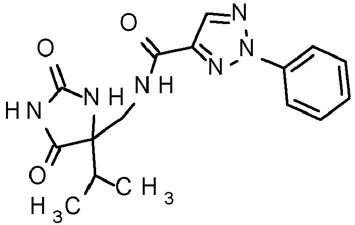

Enantiomeric separation of *rac*-N-[(2,5-dioxo-4-(propan-2-yl)imidazolidin-4-yl)methyl]-2-phenyl-2H-1,2,3-triazole-4-carboxamide (149 mg) using method 1D afforded 65 mg of desired product.
LC-MS (Method 7): Rₜ = 1.25 min; MS (ESIpos): m/z = 343 [M+H]⁺
HPLC (Methode 1E): Rₜ = 6.28 min, >99.0% ee
¹H-NMR (400 MHz, DMSO-d6) δ [ppm]: -0.008 (1.50), 0.008 (1.29), 0.845 (14.14), 0.862 (14.54), 0.953 (13.80), 0.970 (14.25), 1.236 (0.40), 1.971 (0.81), 1.988 (2.13), 2.005 (2.81), 2.022 (1.98), 2.038 (0.71), 3.595 (0.87), 3.611 (0.97), 3.629 (3.91), 3.644 (5.92), 3.658 (3.85), 3.677 (0.86), 3.692 (0.76), 7.487 (1.79), 7.505 (4.73), 7.524 (3.18), 7.605 (5.78), 7.626 (8.26), 7.645 (4.38), 7.810 (5.60), 8.055 (8.10), 8.074 (7.99), 8.077 (5.76), 8.312 (1.66), 8.328 (3.47), 8.343 (1.60), 8.464 (16.00), 10.660 (4.84).

### Example 71

### ent-N-[(4-tert-butyl-2,5-dioxoimidazolidin-4-yl)methyl]-2-phenyl-2H-1,2,3-triazole-4-carboxamide

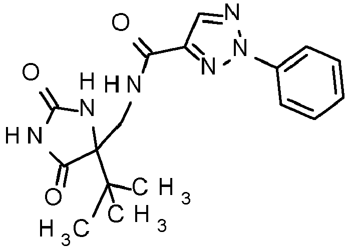

Enantiomeric separation of *rac*-N-[(4-tert-butyl-2,5-dioxoimidazolidin-4-yl)methyl]-2-phenyl-2H-1,2,3-triazole-4-carboxamide (26 mg) using method 2D afforded 9 mg of desired product.
LC-MS (Method 7): Rₜ = 1.39 min; MS (ESIpos): m/z = 357 [M+H]⁺
HPLC (Methode 2E): Rₜ = 4.54 min, >99.0% ee
¹H-NMR (400 MHz, DMSO-d6) δ [ppm]: -0.008 (0.41), 1.020 (16.00), 3.688 (0.59), 3.703 (0.54), 3.772 (0.54), 3.789 (0.59), 7.506 (0.94), 7.524 (0.63), 7.606 (2.35), 7.626 (1.70), 7.645 (0.90), 8.044 (1.57), 8.063 (1.58), 8.066 (1.13), 8.288 (0.63), 8.447 (2.77), 10.590 (0.44).

### Example 72

### ent-N-[(4-(cyclopropylmethyl)-2,5-dioxoimidazolidin-4-yl]methyl]-2-(4-fluorophenyl)-2H-1,2,3-triazole-4-carboxamide

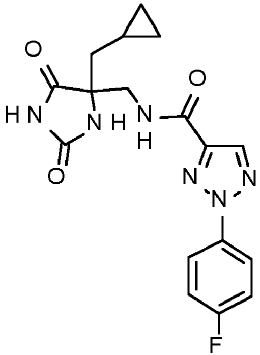

Enantiomeric separation of *rac*-N-[(4-(cyclopropylmethyl)-2,5-dioxoimidazolidin-4-yl]methyl]-2-(4-fluorophenyl)-2H-1,2,3-triazole-4-carboxamide (38 mg) using method 4D afforded 10 mg of desired product.
LC-MS (Method 8): Rₜ = 0.77 min; MS (ESIpos): m/z = 373 [M+H]⁺
HPLC (Methode 4E): Rₜ = 2.63 min, >99.0% ee
¹H-NMR (400 MHz, DMSO-d6) δ [ppm]: -0.149 (1.03), -0.008 (9.10), 0.008 (8.84), 0.146 (0.96), 0.860 (0.63), 1.101 (1.29), 1.109 (0.94), 1.234 (1.47), 1.711 (1.22), 1.725 (1.68), 1.739 (2.46), 1.751 (1.64), 1.778 (1.68), 1.812 (1.36), 1.863 (1.15), 2.052 (1.01), 2.160 (0.42), 2.328 (0.58), 2.367 (0.65), 2.670 (0.70), 2.710 (0.84), 3.508 (1.05), 3.524 (1.01), 3.542 (2.97), 3.558 (2.81), 3.567 (3.11), 3.583 (2.85), 3.601 (1.03), 3.618 (0.98), 3.722 (0.80), 3.740 (0.73), 4.033 (2.01), 4.938 (2.67), 4.963 (2.78), 4.995 (2.48), 5.038 (2.90), 5.742 (1.54), 5.768 (1.99), 5.785 (1.89), 5.811 (1.31), 5.826 (0.56), 7.460 (4.65), 7.482 (7.77), 7.504 (5.05), 7.813 (4.77), 8.085 (4.80), 8.096 (5.31), 8.102 (3.11), 8.107 (5.03), 8.119 (4.82), 8.474 (16.00), 8.541 (1.40), 8.557 (3.09), 8.572 (1.45), 10.714 (4.33).

### Example 73

### ent-N-[(2,5-dioxo-4-propylimidazolidin-4-yl)methyl]-2-(4-fluorophenyl)-2H-1,2,3-triazole-4-carboxamide

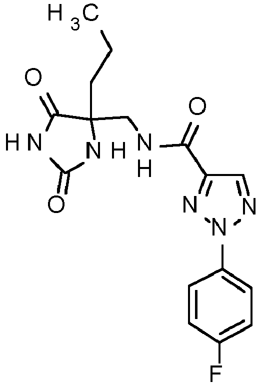

Enantiomeric separation of *rac*-N-[(2,5-dioxo-4-propylimidazolidin-4-yl)methyl]-2-(4-fluorophenyl)-2H-1,2,3-triazole-4-carboxamide (39 mg) using method 5D afforded 20 mg of desired product.
LC-MS (Method 7): Rₜ = 1.36 min; MS (ESIpos): m/z = 361 [M+H]⁺
HPLC (Methode 5E): Rₜ = 2.21 min, >99.0% ee
¹H-NMR (400 MHz, DMSO-d6) δ [ppm]: -0.149 (0.40), -0.008 (4.24), 0.008 (3.87), 0.844 (6.50), 0.862 (16.00), 0.881 (8.05), 1.035 (0.48), 1.052 (0.72), 1.065 (1.09), 1.083 (2.10), 1.101 (2.64), 1.113 (1.80), 1.119 (1.82), 1.127 (1.51), 1.144 (1.30), 1.171 (1.27), 1.235 (2.20), 1.259 (1.13), 1.277 (1.29), 1.295 (1.63), 1.306 (1.40), 1.325 (1.46), 1.337 (0.84), 1.343 (0.87), 1.355 (0.59), 1.540 (0.74), 1.552 (0.89), 1.575 (1.87), 1.586 (1.69), 1.603 (1.87), 1.615 (2.87), 1.628 (1.94), 1.644 (1.65), 1.656 (1.62), 1.678 (0.81), 1.691 (0.57), 3.488 (1.24), 3.504 (1.44), 3.522 (3.58), 3.538 (3.39), 3.551 (3.39), 3.567 (3.49), 3.585 (1.39), 3.601 (1.30), 3.705 (0.50), 3.723 (0.94), 3.741 (1.06), 3.759 (0.87), 3.839 (1.77), 7.460 (5.12), 7.465 (1.94), 7.482 (8.80), 7.498 (2.01), 7.504 (5.56), 7.512 (0.64), 7.786 (5.77), 8.084 (5.32), 8.089 (2.44), 8.096 (5.66), 8.101 (3.48), 8.107 (5.54), 8.113 (2.20), 8.119 (5.08), 8.473 (14.35), 8.500 (1.78), 8.516 (3.64), 8.532 (1.72), 10.679 (4.99).

### Example 74

### ent-N-[(4-cyclopentyl-2,5-dioxoimidazolidin-4-yl)methyl]-2-(4-fluorophenyl)-2H-1,2,3-triazole-4-carboxamide

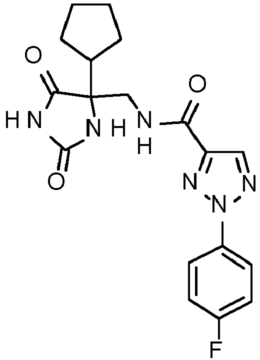

Enantiomeric separation of *rac*-N-[(4-cyclopentyl-2,5-dioxoimidazolidin-4-yl)methyl]-2-(4-fluorophenyl)-2H-1,2,3-triazole-4-carboxamide (93 mg) using method 6D afforded 33 mg of desired product.
LC-MS (Method 7): Rₜ = 1.51 min; MS (ESIpos): m/z = 387 [M+H]⁺
HPLC (Methode 6E): Rₜ = 3.74 min, >99.0% ee
¹H-NMR (400 MHz, DMSO-d6) δ [ppm]: -0.008 (3.04), 0.008 (3.05), 1.134 (1.32), 1.152 (2.79), 1.170 (1.56), 1.235 (1.94), 1.273 (0.62), 1.310 (0.47), 1.356 (1.43), 1.374 (1.24), 1.400 (0.67), 1.469 (2.82), 1.488 (4.56), 1.502 (4.51), 1.530 (3.52), 1.546 (3.10), 1.555 (2.92), 1.567 (2.54), 1.767 (1.58), 1.784 (1.33), 2.168 (0.57), 2.189 (1.47), 2.210 (1.96), 2.230 (1.31), 2.903 (1.00), 2.921 (0.97), 3.571 (0.41), 3.588 (0.57), 3.606 (5.80), 3.610 (5.78), 3.625 (6.23), 3.644 (0.59), 3.660 (0.41), 7.458 (5.63), 7.464 (2.09), 7.480 (10.15), 7.497 (2.17), 7.502 (6.15), 7.511 (0.71), 7.841 (9.12), 8.072 (6.04), 8.084 (6.39), 8.089 (3.86), 8.095 (6.21), 8.102 (2.42), 8.107 (5.78), 8.389 (1.94), 8.405 (4.02), 8.421 (1.91), 8.462 (16.00).

### Example 75

### ent-N-[(4-cyclohexyl-2,5-dioxoimidazolidin-4-yl)methyl]-2-(4-fluorophenyl)-2H-1,2,3-triazole-4-carboxamide

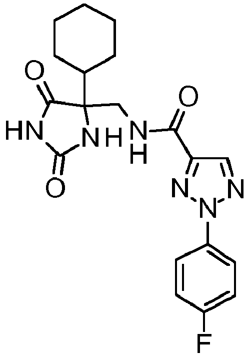

Enantiomeric separation of rac-N-[(4-cyclohexyl-2,5-dioxoimidazolidin-4-yl)methyl]-2-(4-fluorophenyl)-2H-1,2,3-triazole-4-carboxamide (98 mg) using method 7D afforded 20 mg of desired product.
LC-MS (Method 8): Rₜ = 0.89 min; MS (ESIpos): m/z = 401 [M+H]⁺
HPLC (Methode 7E): Rₜ = 3.28 min, >99.0% ee
¹H-NMR (400 MHz, DMSO-d6) δ [ppm]: -0.008 (3.63), 0.008 (2.78), 0.844 (0.55), 0.857 (0.67), 1.011 (0.90), 1.042 (2.33), 1.072 (2.97), 1.101 (2.47), 1.119 (2.21), 1.141 (5.16), 1.159 (5.83), 1.169 (4.23), 1.176 (4.46), 1.193 (1.87), 1.235 (2.43), 1.484 (1.66), 1.507 (1.87), 1.596 (1.78), 1.627 (2.01), 1.688 (2.66), 1.727 (2.13), 1.760 (1.49), 1.803 (1.63), 1.833 (1.53), 2.328 (0.43), 2.367 (0.46), 2.919 (1.06), 2.937 (1.05), 3.580 (1.25), 3.596 (1.45), 3.614 (3.28), 3.630 (3.04), 3.648 (3.05), 3.663 (3.20), 3.682 (1.34), 3.698 (1.17), 7.458 (5.40), 7.463 (1.99), 7.480 (9.38), 7.496 (2.14), 7.502 (5.88), 7.510 (0.76), 7.785 (7.88), 8.063 (0.67), 8.071 (5.72), 8.077 (2.46), 8.083 (6.07), 8.089 (3.67), 8.094 (5.96), 8.101 (2.35), 8.106 (5.50), 8.300 (1.79), 8.316 (3.67), 8.332 (1.79), 8.462 (16.00), 10.638 (1.93).

### Example 76

### ent-N-[(2,5-dioxo-4-(propan-2-yl)imidazolidin-4-yl)methyl]-2-(4-fluorophenyl)-2H-1,2,3-triazole-4-carboxamide

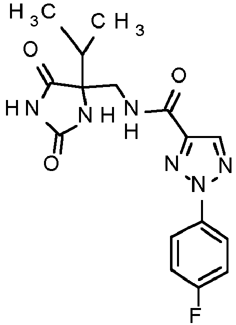

Enantiomeric separation of *rac*-N-[(2,5-dioxo-4-(propan-2-yl)imidazolidin-4-yl)methyl]-2-(4-fluorophenyl)-2H-1,2,3-triazole-4-carboxamide (48 mg) using method 8D afforded 19 mg of desired product.
HPLC (Methode 8E): Rₜ = 2.01 min, >95.0% ee

### Example 77

### ent-N-[(4-cyclobutyl-2,5-dioxoimidazolidin-4-yl)methyl]-2-(4-fluorophenyl)-2H-1,2,3-triazole-4-carboxamide

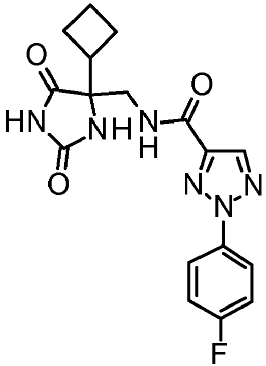

Enantiomeric separation of *rac*-N-[(4-cyclobutyl-2,5-dioxoimidazolidin-4-yl)methyl]-2-(4-fluorophenyl)-2H-1,2,3-triazole-4-carboxamide (98 mg) using method 9D afforded 34 mg of desired product.
LC-MS (Method 8): Rₜ = 0.80 min; MS (ESIpos): m/z = 373 [M+H]⁺
HPLC (Methode 9E): Rₜ = 2.60 min, >98.0% ee
¹H-NMR (400 MHz, DMSO-d6) δ [ppm]: -0.008 (2.17), 0.008 (2.54), 1.234 (0.41), 1.668 (1.42), 1.703 (1.27), 1.730 (1.53), 1.745 (1.28), 1.767 (2.07), 1.789 (2.40), 1.809 (2.33), 1.821 (1.36), 1.830 (1.45), 1.850 (0.88), 1.872 (0.53), 1.904 (1.87), 1.928 (2.63), 1.938 (1.53), 1.950 (1.90), 1.971 (0.67), 2.674 (0.68), 2.696 (1.42), 2.717 (2.15), 2.738 (1.37), 3.445 (1.06), 3.460 (1.22), 3.479 (3.47), 3.495 (3.38), 3.504 (3.43), 3.520 (3.46), 3.538 (1.17), 3.555 (1.07), 7.461 (4.96), 7.466 (1.74), 7.483 (8.75), 7.499 (1.84), 7.505 (5.43), 7.983 (7.78), 8.066 (0.56), 8.075 (5.33), 8.080 (2.21), 8.087 (5.64), 8.092 (3.30), 8.098 (5.48), 8.104 (2.09), 8.109 (5.11), 8.399 (1.60), 8.415 (3.44), 8.431 (1.64), 8.461 (16.00), 10.559 (0.56).

### Example 78

### ent-N-[(4-butyl-2,5-dioxoimidazolidin-4-yl)methyl]-2-phenyl-2H-1,2,3-triazole-4-carboxamide

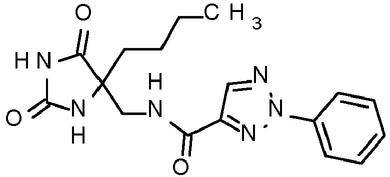

Enantiomeric separation of *rac*-N-[(4-butyl-2,5-dioxoimidazolidin-4-yl)methyl]-2-phenyl-2H-1,2,3-triazole-4-carboxamide (23 mg) using method 10D afforded 10 mg of desired product.
LC-MS (Method 7): Rₜ = 1.45 min; MS (ESIpos): m/z = 357 [M+H]⁺
HPLC (Methode 9E): Rₜ = 2.49 min, >99.0% ee
¹H-NMR (400 MHz, DMSO-d6) δ [ppm]: -0.008 (1.24), 0.008 (1.17), 0.832 (6.26), 0.850 (15.48), 0.867 (8.03), 1.009 (0.51), 1.022 (0.98), 1.040 (1.49), 1.052 (1.36), 1.069 (1.21), 1.084 (0.66), 1.213 (0.87), 1.232 (2.85), 1.249 (4.84), 1.263 (5.58), 1.274 (4.83), 1.290 (1.87), 1.555 (0.81), 1.565 (0.97), 1.590 (2.12), 1.618 (1.92), 1.629 (1.66), 1.638 (1.45), 1.651 (1.37), 1.666 (1.79), 1.700 (0.83), 1.712 (0.48), 2.524 (1.07), 3.498 (0.91), 3.514 (1.09), 3.532 (4.47), 3.547 (6.81), 3.562 (4.31), 3.579 (1.00), 3.596 (0.88), 7.486 (2.00), 7.504 (5.33), 7.522 (3.59), 7.606 (6.57), 7.627 (9.42), 7.640 (2.03), 7.645 (5.06), 7.745 (4.45), 8.065 (8.85), 8.084 (8.88), 8.087 (6.42), 8.472 (16.00), 8.494 (1.89), 8.510 (3.88), 8.526 (1.81), 10.659 (1.57).

### Example 79

### ent-N-[(4-cyclobutyl-2,5-dioxoimidazolidin-4-yl)methyl]-2-phenyl-2H-1,2,3-triazole-4-carboxamide

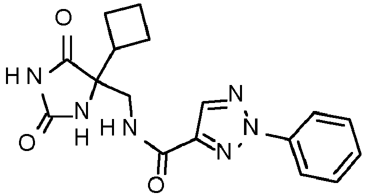

Enantiomeric separation of *rac*-N-{[(4R)-4-cyclobutyl-2,5-dioxoimidazolidin-4-yl]methyl}-2-phenyl-2H-1,2,3-triazole-4-carboxamide (126 mg) using method 11D afforded 55 mg of desired product.
LC-MS (Method 7): Rₜ = 1.34 min; MS (ESIpos): m/z = 355 [M+H]⁺
HPLC (Methode 10E): Rₜ = 5.78 min, >99.0% ee
¹H-NMR (400 MHz, DMSO-d6) δ [ppm]: -0.008 (1.50), 0.008 (1.63), 1.235 (0.55), 1.669 (1.17), 1.711 (1.05), 1.732 (1.27), 1.747 (1.06), 1.769 (1.74), 1.791 (1.97), 1.811 (1.89), 1.832 (1.21), 1.852 (0.74), 1.905 (1.57), 1.929 (2.19), 1.952 (1.59), 1.975 (0.55), 2.676 (0.54), 2.701 (1.17), 2.721 (1.80), 2.742 (1.18), 3.453 (0.83), 3.469 (0.96), 3.487 (3.08), 3.507 (3.75), 3.523 (3.10), 3.541 (0.92), 3.558 (0.86), 7.487 (1.61), 7.506 (4.07), 7.524 (2.85), 7.607 (5.00), 7.628 (6.96), 7.642 (1.52), 7.647 (3.76), 7.990 (6.18), 8.059 (7.00), 8.077 (7.04), 8.080 (5.10), 8.391 (1.35), 8.406 (2.86), 8.422 (1.36), 8.461 (16.00), 10.661 (3.78).

### Example 80

### ent-N-[(4-(cyclopropylmethyl)-2,5-dioxoimidazolidin-4-yl)methyl]-3-phenyl-1,2,4-oxadiazole-5-carboxamide

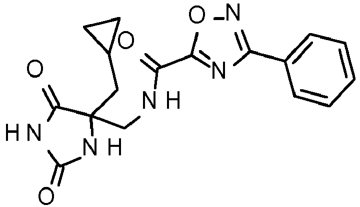

Enantiomeric separation of *rac*-N-[(4-(cyclopropylmethyl)-2,5-dioxoimidazolidin-4-yl)methyl]-3-phenyl-1,2,4-oxadiazole-5-carboxamide (13 mg) using method 12D afforded 3 mg of desired product.
HPLC (Methode 10E): Rₜ = 6.20 min, >99.0% ee
¹H-NMR (400 MHz, DMSO-d6) δ [ppm]: 1.236 (0.58), 1.717 (0.60), 1.736 (2.32), 1.750 (3.62), 1.762 (6.97), 1.778 (5.13), 1.784 (4.55), 1.807 (1.45), 1.824 (1.56), 1.843 (1.70), 1.861 (2.17), 2.004 (0.91), 2.021 (1.82), 2.040 (2.18), 2.057 (1.60), 2.078 (1.06), 2.328 (0.64), 2.366 (0.54), 2.670 (0.82), 2.711 (0.67), 3.482 (3.56), 3.516 (5.95), 3.597 (5.83), 3.631 (3.44), 4.946 (4.87), 4.971 (5.19), 5.006 (4.52), 5.010 (4.50), 5.049 (5.20), 5.053 (5.01), 5.733 (1.30), 5.749 (2.80), 5.759 (1.45), 5.765 (1.48), 5.775 (3.54), 5.792 (3.30), 5.802 (1.29), 5.808 (1.35), 5.818 (2.33), 5.833 (1.05), 7.594 (2.33), 7.600 (3.33), 7.616 (12.07), 7.634 (16.00), 7.654 (4.34), 7.673 (1.21), 7.827 (3.87), 8.055 (10.59), 8.060 (11.07), 8.075 (11.07), 8.079 (9.36), 9.426 (1.53), 10.771 (1.41).

### Example 81

### ent-2-(4-fluorophenyl)-N-[4-(oxan-4-yl)-2,5-dioxoimidazolidin-4-yl)methyl]-2H-1,2,3-triazole-4-carboxamide

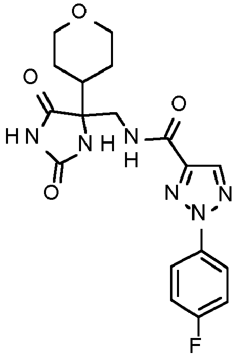

Enantiomeric separation *of ent*-2-(4-fluorophenyl)-N-[4-(oxan-4-yl)-2,5-dioxoimidazolidin-4-yl)methyl]-2H-1,2,3-triazole-4-carboxamide (20 mg) using method 15D afforded 7 mg of desired product.
LC-MS (Method 8): Rₜ = 0.68 min; MS (ESIpos): m/z = 403 [M+H]⁺
HPLC (Methode 14E): Rₜ = 2.59 min, >99.0% ee
¹H-NMR (400 MHz, DMSO-d6) δ [ppm]: 0.008 (0.92), 0.854 (0.49), 0.996 (0.40), 1.235 (1.92), 1.322 (1.87), 1.353 (3.69), 1.372 (1.96), 1.384 (1.77), 1.403 (1.46), 1.422 (1.69), 1.433 (1.73), 1.452 (1.49), 1.463 (1.41), 1.483 (0.68), 1.495 (0.60), 1.651 (2.10), 1.679 (1.73), 1.919 (1.22), 1.949 (2.07), 1.978 (1.05), 2.328 (0.51), 2.367 (0.55), 2.670 (0.44), 2.711 (0.46), 3.230 (1.79), 3.251 (3.28), 3.273 (1.98), 3.284 (2.17), 3.580 (1.34), 3.596 (1.51), 3.615 (3.20), 3.631 (2.90), 3.656 (2.92), 3.672 (3.12), 3.691 (1.47), 3.707 (1.34), 3.832 (1.79), 3.840 (1.93), 3.860 (1.67), 3.868 (1.71), 3.884 (1.83), 3.893 (1.86), 3.913 (1.64), 3.921 (1.53), 7.460 (5.55), 7.465 (1.99), 7.482 (9.40), 7.498 (2.12), 7.504 (5.90), 7.890 (7.70), 8.075 (5.81), 8.081 (2.50), 8.087 (6.13), 8.092 (3.65), 8.098 (5.90), 8.105 (2.31), 8.110 (5.40), 8.394 (1.81), 8.410 (3.72), 8.425 (1.72), 8.467 (16.00), 10.646 (0.69), 11.680 (0.64).

### Example 82

### ent-N-[4-butyl-2,5-dioxoimidazolidin-4-yl)methyl]-2-(4-fluorophenyl)-2H-1,2,3-triazole-4-carboxamide

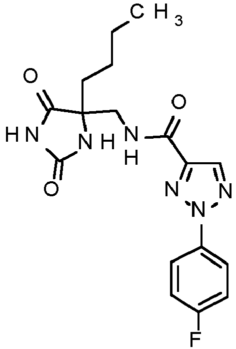

Enantiomeric separation of *rac*-N-[4-butyl-2,5-dioxoimidazolidin-4-yl)methyl]-2-(4-fluorophenyl)-2H-1,2,3-triazole-4-carboxamide (50 mg) using method 15D afforded 21 mg of desired product.
LC-MS (Method 9): Rₜ = 1.18 min; MS (ESIpos): m/z = 375 [M+H]⁺
HPLC (Methode 15E): Rₜ = 6.84 min, >99.0% ee
¹H-NMR (400 MHz, DMSO-d6) δ [ppm]: -0.008 (2.71), 0.008 (2.27), 0.832 (6.56), 0.850 (15.73), 0.868 (8.35), 1.008 (0.59), 1.021 (1.08), 1.039 (1.60), 1.051 (1.51), 1.069 (1.30), 1.083 (0.80), 1.102 (0.62), 1.139 (0.51), 1.157 (0.96), 1.175 (0.57), 1.213 (0.97), 1.233 (3.02), 1.249 (5.25), 1.263 (6.07), 1.275 (5.21), 1.290 (2.08), 1.557 (0.83), 1.567 (0.99), 1.592 (2.24), 1.621 (1.95), 1.631 (1.75), 1.640 (1.55), 1.652 (1.46), 1.668 (1.90), 1.702 (0.89), 1.714 (0.51), 3.496 (1.17), 3.512 (1.43), 3.530 (4.37), 3.547 (5.95), 3.565 (4.25), 3.583 (1.26), 3.600 (1.13), 7.451 (0.69), 7.459 (5.97), 7.465 (2.13), 7.481 (10.41), 7.498 (2.30), 7.503 (6.39), 7.512 (0.67), 7.775 (9.36), 8.074 (0.78), 8.083 (6.25), 8.088 (2.69), 8.095 (6.59), 8.100 (3.95), 8.106 (6.39), 8.112 (2.49), 8.118 (5.89), 8.126 (0.59), 8.472 (16.00), 8.502 (2.08), 8.518 (4.31), 8.534 (1.98), 10.673 (3.30).

### Example 83

### ent-N-[(4-cyclopentyl-2,5-dioxoimidazolidin-4-yl)methyl]-2-phenyl-2H-1,2,3-triazole-4-carboxamide

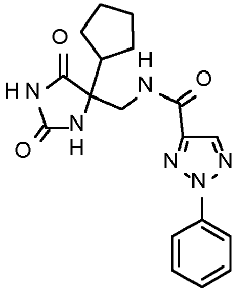

Enantiomeric separation of *rac*-N-[(4-cyclopentyl-2,5-dioxoimidazolidin-4-yl)methyl]-2-phenyl-2H-1,2,3-triazole-4-carboxamide (17 mg) using method 16D afforded 8 mg of desired product.
LC-MS (Method 7): Rₜ = 1.46 min; MS (ESIpos): m/z = 369 [M+H]⁺
HPLC (Methode 16E): Rₜ = 3.39 min, >99.0% ee
¹H-NMR (400 MHz, DMSO-d6) δ [ppm]: 0.853 (0.48), 1.235 (3.04), 1.357 (1.47), 1.488 (4.58), 1.504 (4.51), 1.532 (3.60), 1.769 (1.59), 2.171 (0.60), 2.192 (1.44), 2.213 (1.94), 2.232 (1.34), 2.329 (0.41), 2.671 (0.48), 3.509 (0.42), 3.612 (8.08), 3.628 (8.19), 7.486 (1.87), 7.504 (4.90), 7.523 (3.30), 7.605 (6.00), 7.625 (8.87), 7.644 (4.60), 7.845 (7.68), 8.054 (8.85), 8.074 (8.54), 8.383 (1.85), 8.399 (3.84), 8.414 (1.85), 8.463 (16.00), 10.653 (2.77).

### Example 84

### dia1-N-{[4-(-oxan-3-yl)-2,5-dioxoimidazolidin-4-yl]methyl}-2-phenyl-2H-1,2,3-triazole-4-carboxamide

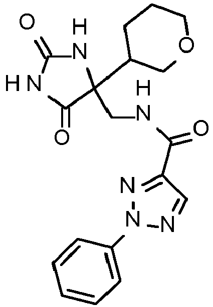

Enantiomeric separation of *rac*-N-{[4-(-oxan-3-yl)-2,5-dioxoimidazolidin-4-yl]methyl}-2-phenyl-2H-1,2,3-triazole-4-carboxamide (60 mg) using method 17D afforded 14 mg of desired product.
LC-MS (Method 7): Rₜ = 1.19 min; MS (ESIpos): m/z = 385 [M+H]⁺
HPLC (Methode 17E): Rₜ = 6.76 min, >99.0% ee
¹H-NMR (400 MHz, DMSO-d6) δ [ppm]: -0.150 (0.51), 0.146 (0.50), 0.854 (0.45), 1.237 (2.18), 1.282 (3.21), 1.312 (3.43), 1.341 (1.54), 1.479 (2.78), 1.509 (3.01), 1.600 (4.87), 1.631 (3.18), 1.931 (8.00), 1.955 (5.68), 2.328 (0.58), 2.365 (0.47), 2.670 (0.78), 2.709 (0.62), 3.126 (2.81), 3.155 (5.34), 3.184 (3.09), 3.209 (3.65), 3.236 (6.69), 3.263 (3.97), 3.552 (2.60), 3.567 (3.08), 3.587 (5.36), 3.602 (5.12), 3.649 (4.77), 3.665 (5.21), 3.682 (3.04), 3.700 (2.74), 3.770 (8.82), 3.792 (8.38), 7.488 (2.89), 7.506 (7.18), 7.525 (5.26), 7.607 (8.63), 7.627 (14.56), 7.646 (7.60), 7.870 (11.47), 8.059 (13.78), 8.079 (13.01), 8.430 (6.92), 8.445 (4.07), 8.470 (16.00), 10.748 (5.19).

### Example 85

### dia2-N-{[4-(-oxan-3-yl)-2,5-dioxoimidazolidin-4-yl]methyl}-2-phenyl-2H-1,2,3-triazole-4-carboxamide

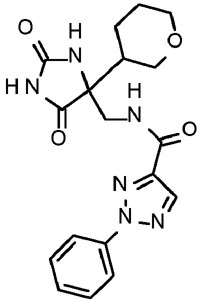

Enantiomeric separation of *rac*-N-{[4-(-oxan-3-yl)-2,5-dioxoimidazolidin-4-yl]methyl}-2-phenyl-2H-1,2,3-triazole-4-carboxamide (60 mg) using method 17D afforded 8 mg of desired product.
LC-MS (Method 7): Rₜ = 1.19 min; MS (ESIpos): m/z = 385 [M+H]⁺
HPLC (Methode 17E): Rₜ = 7.50 min, >99.0% ee
¹H-NMR (400 MHz, DMSO-d6) δ [ppm]: -0.151 (1.27), 0.144 (1.51), 0.849 (2.75), 1.018 (2.01), 1.177 (3.69), 1.236 (8.97), 1.449 (4.87), 1.560 (6.20), 1.603 (5.93), 1.950 (4.22), 2.245 (2.21), 2.328 (1.77), 2.669 (2.45), 3.153 (8.71), 3.180 (9.86), 3.207 (4.34), 3.609 (12.84), 3.622 (13.46), 3.765 (4.78), 3.793 (4.55), 4.004 (4.99), 4.029 (4.84), 7.508 (7.17), 7.524 (5.61), 7.607 (9.21), 7.626 (14.61), 7.645 (7.82), 7.846 (7.29), 8.057 (14.55), 8.077 (13.67), 8.408 (6.91), 8.469 (16.00), 10.743 (2.21).

### Example 86

### ent-N-{[4-cyclobutyl-2,5-dioxoimidazolidin-4-yl]methyl}-2-(2,4-difluorophenyl)-2H-1,2,3-triazole-4-carboxamide

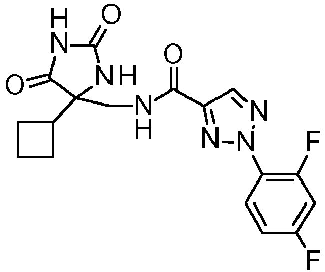

To a solution of *ent*-5-(aminomethyl)-5-cyclobutylimidazolidine-2,4-dione hydrochloride (32.5 mg, 148 µmol) and 2-(2,4-difluorophenyl)-2H-1,2,3-triazole-4-carboxylic acid (33.4 mg, 148 µmol) in dichloromethane (3 ml) was added 1-ethyl-3-(3-dimethylaminopropyl)carbodiimide hydrochloride (36.9 mg, 193 µmol), 1-hydroxybenzotriazole hydrate (29.5 mg, 193 µmol) and N,N-diisopropylethylamine (72 µl, 410 µmol). The reaction mixture was stirred overnight at RT and was diluted with a small amount of water and acetonitrile. The resulting mixture was purified by preparative HPLC (Method 1f) The combined organic phases were concentrated in vacuo and the remaining TFA was removed, using a Stratospheres PL-HCO3 MP SPE cartridge. After lyophilization, 33.3 mg (58 % yield) of the desired product were obtained.
LC-MS (Method 7): Rₜ = 1.33 min; MS (ESIpos): m/z = 391 [M+H]⁺
¹H-NMR (500 MHz, DMSO-d6) δ [ppm]: -0.007 (2.12), 0.006 (1.50), 1.644 (1.24), 1.660 (1.23), 1.681 (0.85), 1.696 (1.14), 1.710 (1.33), 1.717 (1.30), 1.733 (0.63), 1.743 (0.74), 1.761 (1.71), 1.779 (2.01), 1.793 (2.05), 1.800 (2.08), 1.818 (1.32), 1.835 (0.80), 1.881 (0.99), 1.887 (1.28), 1.895 (1.99), 1.902 (1.83), 1.914 (2.27), 1.933 (1.70), 1.950 (0.62), 2.668 (0.43), 2.686 (1.27), 2.703 (1.98), 2.720 (1.27), 3.481 (7.11), 3.494 (7.01), 7.353 (1.05), 7.356 (1.14), 7.359 (1.07), 7.369 (2.00), 7.372 (2.09), 7.374 (1.99), 7.387 (1.11), 7.390 (1.16), 7.393 (1.03), 7.659 (1.55), 7.664 (1.55), 7.677 (1.77), 7.681 (2.52), 7.686 (1.62), 7.699 (1.55), 7.704 (1.49), 7.931 (5.37), 7.943 (1.95), 7.949 (2.92), 7.961 (2.85), 7.967 (1.58), 7.979 (1.43), 8.383 (1.54), 8.396 (3.16), 8.408 (1.45), 8.508 (16.00), 10.640 (1.34).

### Example 87

### rac-2-(2,4-difluorophenyl)-N-[(4-isopropyl-2,5-dioxoimidazolidin-4-yl)methyl]-2H-1,2,3-triazole-4-carboxamide

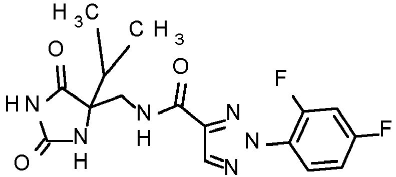

To a solution of rac-5-(aminomethyl)-5-isopropylimidazolidine-2,4-dione hydrochloride (40.0 mg, 193 µmol) and 2-(2,4-difluorophenyl)-2H-1,2,3-triazole-4-carboxylic acid (43.4 mg, 193 µmol) in dichloromethane (4 ml) was added 1-ethyl-3-(3-dimethylaminopropyl)carbodiimide hydrochloride (48.0 mg, 250 µmol), 1-hydroxybenzotriazole hydrate (38.3 mg, 250 µmol) and N,N-diisopropylethylamine (94 µl, 540 µmol). The reaction mixture was stirred overnight at RT and was diluted with a small amount of water and acetonitrile. The resulting mixture was purified by preparative HPLC (Method 2f). The combined organic phases were dried over sodium sulfate, filtered, and concentrated in vacuo, affording 44.9 mg of the desired product (96 % purity, 59 % yield)
LC-MS (Method 7): Rₜ = 1.29 min; MS (ESIpos): m/z = 379 [M+H]⁺
¹H-NMR (600 MHz, DMSO-d6) δ [ppm]: 0.835 (15.42), 0.847 (15.44), 0.943 (15.28), 0.955 (15.19), 1.966 (0.94), 1.977 (2.35), 1.989 (3.10), 2.000 (2.19), 2.011 (0.82), 3.601 (0.51), 3.612 (0.79), 3.625 (5.84), 3.627 (5.57), 3.635 (5.22), 3.638 (5.28), 3.650 (0.50), 3.661 (0.44), 7.359 (1.34), 7.361 (1.41), 7.374 (2.47), 7.376 (2.40), 7.387 (1.30), 7.389 (1.29), 7.667 (1.71), 7.672 (1.72), 7.683 (2.04), 7.686 (2.89), 7.690 (1.88), 7.701 (1.70), 7.705 (1.60), 7.799 (6.03), 7.937 (1.60), 7.947 (1.86), 7.952 (3.02), 7.962 (3.02), 7.967 (1.69), 7.977 (1.48), 8.309 (1.97), 8.320 (3.91), 8.330 (1.84), 8.515 (16.00), 8.568 (0.51), 10.652 (5.21).

### Example 88

### ent-2-(2,4-difluorophenyl)-N-[(4-isopropyl-2,5-dioxoimidazolidin-4-yl)methyl]-2H-1,2,3-triazole-4-carboxamide

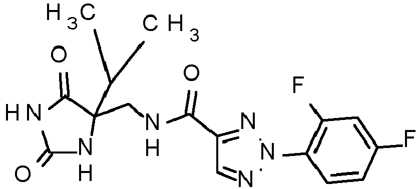

Enantiomeric separation of *rac*-2-(2,4-difluorophenyl)-N-[(4-isopropyl-2,5-dioxoimidazolidin-4-yl)methyl]-2H-1,2,3-triazole-4-carboxamide by preparative chiral HPLC [sample preparation: 42.3 mg dissolved in a mixture of methanol (3 ml) and acetonitrile (2 ml); injection volume: 0.3 ml; column: Daicel Chiralpak AD-H 5 µm, 250 x 20 mm; eluent: 50% *n*-heptane/50% ethanol; flow rate: 15 ml/min; temperature: 40°C; UV detection: 210 nm] afforded 12.6 mg of the desired product. (98% purity)
Analytical chiral HPLC: Rₜ = 4.77 min, e.e. = > 99% [column: Chiraltek AD-H 3 µm, 100 x 4.6 mm; eluent: 50% n-heptane/50% ethanol; flow rate: 1 ml/min; UV detection: 220 nm]
LC-MS (Method 7): Rₜ = 1.29 min; MS (ESIpos): m/z = 379 [M+H]⁺
¹H-NMR (600 MHz, DMSO-d6) δ [ppm]: 0.835 (16.00), 0.847 (15.94), 0.943 (15.68), 0.954 (15.69), 1.102 (0.43), 1.234 (0.58), 1.966 (1.02), 1.977 (2.50), 1.989 (3.23), 2.000 (2.33), 2.011 (0.85), 2.426 (0.42), 3.601 (0.56), 3.612 (0.88), 3.625 (6.18), 3.627 (5.99), 3.635 (5.64), 3.638 (5.62), 3.650 (0.57), 3.662 (0.48), 7.361 (1.44), 7.374 (2.69), 7.389 (1.44), 7.667 (1.73), 7.672 (1.78), 7.682 (2.10), 7.686 (3.04), 7.690 (2.02), 7.700 (1.72), 7.705 (1.65), 7.803 (8.89), 7.937 (1.64), 7.947 (1.93), 7.952 (3.13), 7.962 (3.12), 7.967 (1.82), 7.976 (1.55), 8.312 (2.06), 8.323 (4.09), 8.333 (2.00), 8.516 (15.77).

### Example 89

### ent-2-(4-chloro-3-fluorophenyl)-N-[(4-cyclobutyl-2,5-dioxoimidazolidin-4-yl)methyl]-2H-1,2,3-triazole-4-carboxamide

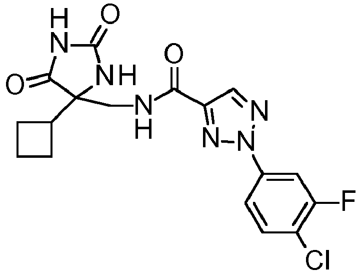

To a solution of *ent*-5-(aminomethyl)-5-cyclobutylimidazolidine-2,4-dione hydrochloride (28.1 mg, 128 µmol) and 2-(4-chloro-3-fluorophenyl)-2H-1,2,3-triazole-4-carboxylic acid (30.9 mg, 128 µmol) in dichloromethane (2.6 ml) was added 1-ethyl-3-(3-dimethylaminopropyl)carbodiimide hydrochloride (31.8 mg, 166 µmol), 1-hydroxybenzotriazole hydrate (25.4 mg, 166 µmol) and N,N-diisopropylethylamine (62 µl, 360 µmol). The reaction mixture was stirred overnight at RT and concentrated under reduced pressure. The crude product was purified twice by preparative HPLC (Method 1f) The combined organic phases were concentrated in vacuo and the remaining TFA was removed, using a Stratospheres PL-HCO3 MP SPE cartridge. After lyophilization, 10.6 mg (20 % yield) of the desired product was obtained.
LC-MS (Method 7): Rₜ = 1.60 min; MS (ESIpos): m/z = 407 [M+H]⁺
¹H-NMR (500 MHz, DMSO-d6) δ [ppm]: -0.120 (0.69), 0.116 (0.62), 1.663 (1.65), 1.685 (1.09), 1.720 (1.60), 1.753 (0.95), 1.772 (1.91), 1.789 (2.93), 1.803 (2.92), 1.817 (1.83), 1.836 (0.93), 1.908 (2.60), 1.926 (2.62), 1.945 (1.91), 1.963 (0.88), 2.096 (1.05), 2.361 (0.59), 2.634 (0.56), 2.691 (1.52), 2.709 (2.26), 2.726 (1.44), 3.432 (1.47), 3.443 (1.62), 3.459 (3.16), 3.471 (2.83), 3.501 (2.63), 3.514 (2.80), 3.528 (1.45), 3.542 (1.34), 7.857 (2.42), 7.875 (4.76), 7.891 (4.11), 7.908 (1.74), 7.929 (4.14), 7.934 (4.05), 7.947 (2.36), 8.069 (3.26), 8.073 (3.11), 8.089 (3.21), 8.093 (3.02), 8.509 (1.98), 8.521 (16.00), 8.533 (1.78), 8.561 (0.50), 10.646 (1.11).

### Example 90

### ent-N-[(4-cyclobutyl-2,5-dioxoimidazolidin-4-yl)methyl]-2-(3,4,5-trifluorophenyl)-2H-1,2,3-triazole-4-carboxamide

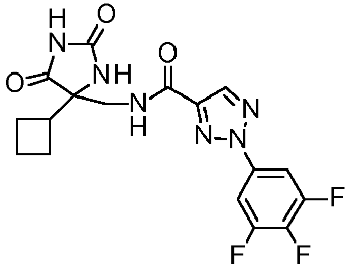

To a solution of *ent*-5-(aminomethyl)-5-cyclobutylimidazolidine-2,4-dione hydrochloride (80.0 mg, 364 µmol) and 2-(3,4,5-trifluorophenyl)-2H-1,2,3-triazole-4-carboxylic acid (88.5 mg, 364 µmol) in dichloromethane (7.5 ml) was added 1-ethyl-3-(3-dimethylaminopropyl)carbodiimide hydrochloride (90.8 mg, 473 µmol), 1-hydroxybenzotriazole hydrate (72.5 mg, 473 µmol) and N,N-diisopropylethylamine (180 µl, 1.0 mmol). The reaction mixture was stirred overnight at RT and concentrated under reduced pressure. The crude product was purified by preparative HPLC (Method 2f) The combined organic phases were concentrated in vacuo and the remaining TFA was removed, using a Stratospheres PL-HCO3 MP SPE cartridge. After lyophilization, 57.5 mg (39 % yield) of the desired product were obtained.
LC-MS (Method 8): Rₜ = 0.86 min; MS (ESIpos): m/z = 409 [M+H]⁺
¹H-NMR (500 MHz, DMSO-d6) δ [ppm]: -0.007 (1.59), 0.006 (1.18), 1.649 (1.18), 1.661 (1.29), 1.698 (1.12), 1.709 (1.21), 1.716 (1.30), 1.732 (0.60), 1.747 (0.79), 1.765 (1.53), 1.784 (2.03), 1.795 (1.97), 1.802 (2.22), 1.818 (1.29), 1.836 (0.75), 1.888 (1.19), 1.897 (1.15), 1.904 (1.52), 1.920 (0.96), 1.929 (1.67), 1.949 (1.52), 1.969 (0.67), 2.687 (1.25), 2.704 (1.84), 2.721 (1.19), 3.409 (1.56), 3.420 (1.67), 3.436 (2.52), 3.448 (2.19), 3.512 (2.17), 3.526 (2.29), 3.540 (1.49), 3.553 (1.38), 7.939 (1.96), 8.008 (3.19), 8.021 (3.73), 8.025 (3.73), 8.037 (3.27), 8.549 (16.00), 8.564 (2.75), 8.577 (1.35), 10.669 (0.80).

### Example 91

### ent-2-(3-chloro-4-fluorophenyl)-N-[(4-cyclobutyl-2,5-dioxoimidazolidin-4-yl)methyl]-2H-1,2,3-triazole-4-carboxamide

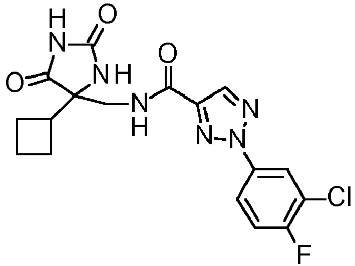

To a solution of *ent*-5-(aminomethyl)-5-cyclobutylimidazolidine-2,4-dione hydrochloride (19.0 mg, 86.3 µmol) and 2-(3-chloro-4-fluorophenyl)-2H-1,2,3-triazole-4-carboxylic acid (20.9 mg, 86.3 µmol) in dichloromethane (1.8 ml) was added 1-ethyl-3-(3-dimethylaminopropyl)carbodiimide hydrochloride (21.5 mg, 112 µmol), 1-hydroxybenzotriazole hydrate (17.2 mg, 112 µmol) and N,N-diisopropylethylamine (42 µl, 240 µmol). The reaction mixture was stirred overnight at RT and was diluted with a small amount of Water and acetonitrile. The resulting mixture was purified by preparative HPLC (Method 1f). A second purification by preparative chiral HPLC [sample preparation: 25 mg dissolved in a mixture of ethanol (1 ml) and DCM (1 ml); injection volume: 0.4 ml; column: Daicel Chiralpak IG 5 µm, 250 x 20 mm; eluent: n-heptan/ethanol 50:50; flow rate: 15 ml/min; temperature: 50°C; UV detection: 220 nm] gave 21.0 mg (60 % yield) of the desired compound.
LC-MS (Method 7): Rₜ = 1.58 min; MS (ESIpos): m/z = 407 [M+H]⁺
¹H-NMR (500 MHz, DMSO-d6) δ [ppm]: 1.651 (1.13), 1.667 (1.15), 1.691 (0.77), 1.708 (1.05), 1.722 (1.20), 1.728 (1.22), 1.744 (0.60), 1.752 (0.77), 1.769 (1.60), 1.787 (1.84), 1.801 (1.90), 1.808 (1.88), 1.827 (1.24), 1.844 (0.71), 1.896 (0.92), 1.902 (1.16), 1.917 (1.75), 1.932 (2.25), 1.950 (1.52), 1.969 (0.62), 2.077 (1.16), 2.679 (0.41), 2.697 (1.15), 2.714 (1.82), 2.731 (1.16), 3.305 (0.53), 3.352 (0.83), 3.430 (1.45), 3.442 (1.63), 3.457 (2.61), 3.469 (2.31), 3.520 (2.35), 3.533 (2.52), 3.547 (1.54), 3.561 (1.43), 7.692 (2.87), 7.710 (5.84), 7.728 (3.10), 7.999 (6.61), 8.053 (1.69), 8.058 (1.99), 8.061 (1.95), 8.066 (1.86), 8.071 (1.69), 8.076 (1.95), 8.079 (1.71), 8.084 (1.60), 8.248 (3.00), 8.253 (2.93), 8.261 (3.04), 8.266 (2.78), 8.505 (16.00), 8.519 (2.95), 8.532 (1.45), 10.671 (3.77).

### Example 92

### ent-2-(3-cyanophenyl)-N-[(4-cyclobutyl-2,5-dioxoimidazolidin-4-yl)methyl]-2H-1,2,3-triazole-4-carboxamide

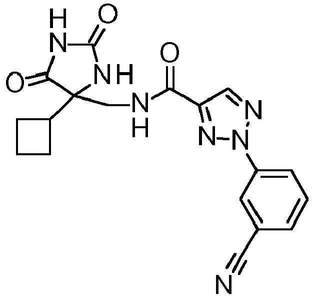

To a solution of *ent*-5-(aminomethyl)-5-cyclobutylimidazolidine-2,4-dione hydrochloride (51.3 mg, 233 µmol) and 2-(3-cyanophenyl)-2H-1,2,3-triazole-4-carboxylic acid (50.0 mg, 233 µmol) in DMF (1.5 ml) was added 1-ethyl-3-(3-dimethylaminopropyl)carbodiimide hydrochloride (58.2 mg, 303 µmol), 1-hydroxybenzotriazole hydrate (46.5 mg, 303 µmol) and N,N-diisopropylethylamine (110 µl, 650 µmol). The reaction mixture was stirred for 3 days at RT and concentrated under reduced pressure. The crude product was purified by preparative HPLC (Method 1f) The combined organic phases were concentrated in vacuo and the remaining TFA was removed, using a Stratospheres PL-HCO3 MP SPE cartridge. After lyophilization, 38.1 mg (99 % purity, 43 % yield) of the desired product were obtained.
LC-MS (Method 7): Rₜ = 1.30 min; MS (ESIpos): m/z = 380 [M+H]⁺
¹H-NMR (600 MHz, DMSO-d6) δ [ppm]: 1.646 (1.35), 1.659 (1.36), 1.680 (0.77), 1.700 (1.21), 1.715 (1.41), 1.755 (0.60), 1.771 (1.66), 1.786 (2.48), 1.794 (2.23), 1.800 (2.56), 1.807 (1.36), 1.814 (1.43), 1.828 (0.80), 1.887 (1.15), 1.891 (1.34), 1.898 (1.28), 1.905 (1.64), 1.913 (1.24), 1.929 (1.84), 1.945 (1.69), 1.962 (0.81), 2.071 (0.69), 2.515 (0.77), 2.518 (0.77), 2.521 (0.79), 2.679 (0.49), 2.694 (1.38), 2.708 (2.10), 2.722 (1.35), 3.435 (1.70), 3.445 (1.82), 3.458 (2.93), 3.468 (2.59), 3.505 (2.54), 3.515 (2.67), 3.527 (1.56), 3.538 (1.41), 7.827 (2.96), 7.840 (5.79), 7.854 (3.63), 7.899 (1.75), 7.972 (3.06), 7.974 (4.03), 7.976 (2.88), 7.985 (2.57), 7.987 (3.33), 7.989 (2.27), 8.367 (2.66), 8.369 (2.94), 8.371 (2.93), 8.372 (2.72), 8.381 (2.53), 8.382 (2.59), 8.385 (2.79), 8.386 (2.38), 8.465 (4.02), 8.467 (5.54), 8.470 (3.31), 8.540 (16.00), 8.551 (1.48), 8.562 (2.66), 8.572 (1.34).

### Example 93

### ent-N-[(4-cyclobutyl-2,5-dioxoimidazolidin-4-yl)methyl]-2-(3-fluorophenyl)-2H-1,2,3-triazole-4-carboxamide

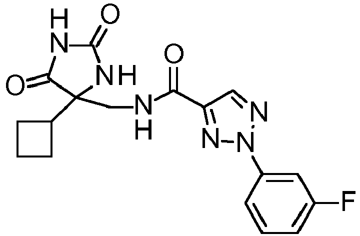

To a solution of *ent*-5-(aminomethyl)-5-cyclobutylimidazolidine-2,4-dione hydrochloride (53.0 mg, 241 µmol) and 2-(3-fluorophenyl)-2H-1,2,3-triazole-4-carboxylic acid (50.0 mg, 241 µmol) in dichloromethane (5 ml) was added 1-ethyl-3-(3-dimethylaminopropyl)carbodiimide hydrochloride (60.1 mg, 314 µmol), 1-hydroxybenzotriazole hydrate (48.0 mg, 314 µmol) and N,N-diisopropylethylamine (120 µl, 680 µmol). The reaction mixture was stirred overnight at RT, after which extra portion of N,N-diisopropylethylamine (120 µl, 680 µmol) and DMF (1.5 ml) were added due to incomplete conversion. After stirring for 24 h at RT and for 4 h at 40°C, the reaction mixture was additionally stirred for 24 h at RT. The resulting reaction mixture was concentrated under reduced pressure and the crude product was purified by preparative HPLC (Method 3f). After lyophilization, 15.1 mg (17 % yield) of the desired product were obtained.
LC-MS (Method 7): Rₜ = 1.41 min; MS (ESIpos): m/z = 373 [M+H]⁺
¹H-NMR (600 MHz, DMSO-d6) δ [ppm]: 1.656 (1.35), 1.669 (1.21), 1.685 (0.57), 1.714 (1.14), 1.726 (1.38), 1.732 (1.35), 1.745 (0.60), 1.762 (0.67), 1.777 (1.84), 1.792 (2.31), 1.805 (2.16), 1.813 (2.13), 1.827 (1.42), 1.842 (0.82), 1.903 (1.06), 1.908 (1.28), 1.920 (2.27), 1.935 (2.59), 1.951 (1.77), 1.966 (0.71), 2.521 (0.60), 2.691 (0.46), 2.706 (1.35), 2.720 (2.06), 2.734 (1.38), 3.450 (1.77), 3.460 (1.95), 3.472 (3.19), 3.483 (2.80), 3.522 (2.87), 3.533 (3.02), 3.545 (1.81), 3.556 (1.63), 6.573 (0.43), 7.358 (1.17), 7.362 (1.31), 7.372 (2.34), 7.376 (2.45), 7.386 (1.24), 7.390 (1.28), 7.664 (1.53), 7.675 (1.77), 7.678 (3.05), 7.688 (2.98), 7.692 (1.77), 7.702 (1.53), 7.871 (1.63), 7.875 (2.77), 7.879 (1.84), 7.887 (1.67), 7.891 (2.73), 7.895 (1.77), 7.922 (3.12), 7.924 (2.66), 7.935 (2.66), 7.938 (2.41), 7.995 (5.14), 8.469 (1.77), 8.480 (3.33), 8.491 (1.88), 8.499 (16.00), 10.668 (4.22).

### Example 94

### ent-N-[(4-cyclobutyl-2,5-dioxoimidazolidin-4-yl)methyl]-2-(3,4-difluorophenyl)-2H-1,2,3-triazole-4-carboxamide

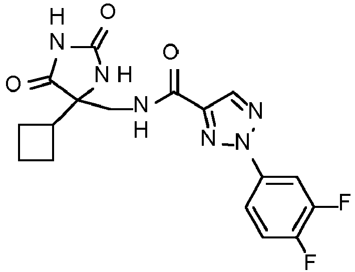

To a solution of *ent*-5-(aminomethyl)-5-cyclobutylimidazolidine-2,4-dione hydrochloride (53.0 mg, 241 µmol) and 2-(3,4-difluorophenyl)-2H-1,2,3-triazole-4-carboxylic acid (54.3 mg, 241 µmol) in DCM (5 ml) was added 1-ethyl-3-(3-dimethylaminopropyl)carbodiimide hydrochloride (60.1 mg, 314 µmol), 1-hydroxybenzotriazole hydrate (48.0 mg, 314 µmol) and N,N-diisopropylethylamine (120 µl, 680 µmol). The reaction mixture was stirred overnight at RT, after which extra portion of N,N-diisopropylethylamine (120 µl, 680 µmol) and DMF (1.5 ml) were added due to incomplete conversion. After stirring for 24 h at RT, the reaction mixture was additionally stirred for 8 h at 40°C. The resulting reaction mixture was concentrated under reduced pressure and the crude product was purified by preparative HPLC (Method 3f). After lyophilization, 34.4 mg (37 % yield) of the desired product were obtained.
LC-MS (Method 7): Rₜ = 1.47 min; MS (ESIpos): m/z = 391 [M+H]⁺
¹H-NMR (600 MHz, DMSO-d6) δ [ppm]: 1.653 (1.23), 1.667 (1.12), 1.712 (1.06), 1.723 (1.29), 1.729 (1.23), 1.742 (0.50), 1.760 (0.67), 1.775 (1.68), 1.790 (2.07), 1.803 (1.96), 1.811 (1.90), 1.825 (1.29), 1.840 (0.73), 1.899 (1.01), 1.905 (1.17), 1.918 (2.18), 1.933 (2.24), 1.949 (1.57), 1.966 (0.62), 2.425 (0.50), 2.524 (1.06), 2.655 (0.50), 2.686 (0.45), 2.701 (1.23), 2.715 (1.90), 2.729 (1.29), 3.439 (1.68), 3.450 (1.85), 3.462 (2.85), 3.473 (2.52), 3.521 (2.52), 3.532 (2.69), 3.543 (1.73), 3.555 (1.57), 7.712 (1.06), 7.727 (2.18), 7.744 (2.24), 7.758 (1.29), 7.915 (1.79), 7.922 (1.12), 7.931 (1.57), 7.988 (4.64), 8.102 (1.23), 8.106 (1.23), 8.114 (1.40), 8.118 (1.51), 8.120 (1.45), 8.125 (1.29), 8.132 (1.29), 8.136 (1.12), 8.465 (1.62), 8.476 (3.02), 8.487 (1.68), 8.498 (16.00), 10.663 (3.97).

### Example 95

### ent-2-(4-chlorophenyl)-N-[(4-cyclobutyl-2,5-dioxoimidazolidin-4-yl)methyl]-2H-1,2,3-triazole-4-carboxamide

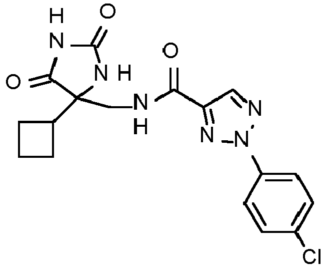

To a solution of *ent*-5-(aminomethyl)-5-cyclobutylimidazolidine-2,4-dione hydrochloride (49.1 mg, 224 µmol) and 2-(4-chlorophenyl)-2H-1,2,3-triazole-4-carboxylic acid (50.0 mg, 224 µmol) in DMF (1.4 ml) was added 1-ethyl-3-(3-dimethylaminopropyl)carbodiimide hydrochloride (55.7 mg, 291 µmol), 1-hydroxybenzotriazole hydrate (44.5 mg, 291 µmol) and N,N-diisopropylethylamine (110 µl, 630 µmol). After stirring for 3 days at RT, the reaction mixture was stirred for 6 h at 40°C. The resulting reaction mixture was concentrated under reduced pressure and the crude product was purified by preparative HPLC (Method 4f). After lyophilization, 35.1 mg (40 % yield) of the desired product were obtained.
LC-MS (Method 7): Rₜ = 1.54 min; MS (ESIpos): m/z = 389 [M+H]⁺
¹H-NMR (600 MHz, DMSO-d6) δ [ppm]: 1.653 (1.44), 1.667 (1.26), 1.682 (0.61), 1.711 (1.19), 1.723 (1.44), 1.728 (1.41), 1.742 (0.61), 1.758 (0.69), 1.774 (1.91), 1.789 (2.38), 1.802 (2.24), 1.810 (2.31), 1.825 (1.48), 1.839 (0.83), 1.900 (1.12), 1.905 (1.34), 1.917 (2.17), 1.931 (2.71), 1.947 (1.84), 1.962 (0.69), 2.689 (0.47), 2.703 (1.41), 2.717 (2.17), 2.731 (1.44), 3.455 (1.63), 3.465 (1.84), 3.477 (3.54), 3.488 (3.03), 3.511 (3.03), 3.522 (3.29), 3.534 (1.66), 3.545 (1.48), 7.693 (1.41), 7.698 (9.97), 7.701 (3.11), 7.709 (3.68), 7.713 (10.47), 7.718 (1.05), 7.990 (5.38), 8.067 (1.63), 8.072 (11.45), 8.075 (3.36), 8.083 (3.61), 8.087 (10.08), 8.092 (1.01), 8.445 (1.84), 8.456 (3.54), 8.466 (1.77), 8.485 (16.00), 10.665 (4.55).

### Example 96

### ent-2-(3-chlorophenyl)-N-[(4-cyclobutyl-2,5-dioxoimidazolidin-4-yl)methyl]-2H-1,2,3-triazole-4-carboxamide

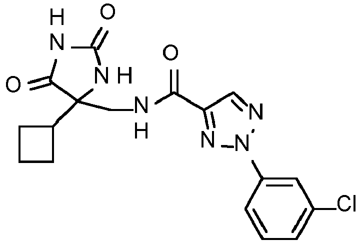

To a solution of *ent*-5-(aminomethyl)-5-cyclobutylimidazolidine-2,4-dione hydrochloride (49.1 mg, 224 µmol) and 2-(3-chlorophenyl)-2H-1,2,3-triazole-4-carboxylic acid (50.0 mg, 224 µmol) in DMF (1.4 ml) was added 1-ethyl-3-(3-dimethylaminopropyl)carbodiimide hydrochloride (55.7 mg, 291 µmol), 1-hydroxybenzotriazole hydrate (44.5 mg, 291 µmol) and N,N-diisopropylethylamine (110 µl, 630 µmol). After stirring for 3 days at RT, the reaction mixture was stirred for 6 h at 40°C. The resulting reaction mixture was concentrated under reduced pressure and the crude product was purified by preparative HPLC (Method 4f). After lyophilization, 35.1 mg (40 % yield) of the desired product were obtained.
LC-MS (Method 7): Rₜ = 1.54 min; MS (ESIpos): m/z = 389 [M+H]⁺
¹H-NMR (600 MHz, DMSO-d6) δ [ppm]: 1.655 (1.21), 1.669 (1.13), 1.714 (1.05), 1.725 (1.25), 1.731 (1.25), 1.744 (0.56), 1.761 (0.60), 1.776 (1.65), 1.791 (2.10), 1.804 (1.97), 1.813 (1.93), 1.826 (1.29), 1.841 (0.73), 1.902 (0.97), 1.907 (1.17), 1.920 (2.02), 1.934 (2.42), 1.950 (1.61), 1.964 (0.60), 2.689 (0.44), 2.704 (1.25), 2.719 (1.93), 2.733 (1.33), 3.446 (1.57), 3.456 (1.77), 3.469 (2.90), 3.479 (2.50), 3.521 (2.54), 3.532 (2.74), 3.544 (1.65), 3.555 (1.49), 7.577 (1.93), 7.579 (2.26), 7.580 (2.18), 7.582 (2.14), 7.591 (2.74), 7.592 (2.98), 7.594 (3.06), 7.595 (2.78), 7.650 (3.95), 7.663 (6.49), 7.677 (2.94), 7.989 (4.63), 8.033 (2.34), 8.034 (2.70), 8.036 (2.70), 8.038 (2.66), 8.046 (2.18), 8.048 (2.38), 8.049 (2.62), 8.051 (2.42), 8.115 (3.67), 8.118 (6.49), 8.121 (3.22), 8.501 (16.00), 8.509 (3.14), 8.519 (1.49), 10.666 (3.99).

### Example 97

### ent-N-{[2,5-dioxo-4-(pyridin-2-yl)imidazolidin-4-yl]methyl}-2-(4-fluorophenyl)-2H-1,2,3-triazole-4-carboxamide

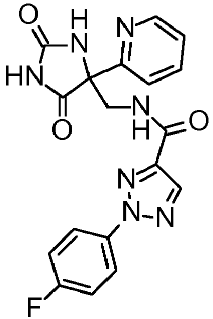

Enantiomeric separation of *rac*-N-{[2,5-dioxo-4-(pyridin-2-yl)imidazolidin-4-yl]methyl}-2-(4-fluorophenyl)-2H-1,2,3-triazole-4-carboxamide (31.7 mg) by preparative chiral HPLC [column: Daicel Chiralcel® OX-H 5 µm, 250 x 30 mm; eluent: 50% *n*-heptane/50% ethanol +0.2% diethylamine; flow rate: 40 ml/min; temperature: 40°C; UV detection: 220 nm] afforded 13 mg of the desired product. The product was further purified by preparative HPLC (Method 1f)The combined organic phases were concentrated in vacuo and the remaining TFA was removed, using a Stratospheres PL-HCO3 MP SPE cartridge. After lyophilization, 5.9 mg of the desired product were obtained.
Analytical chiral HPLC: Rt = 7.63 min, e.e. = 99% [column: Daicel Chiralcel® OX-H 5 µm, 250 x 30 mm; eluent: 50% *n*-heptane/50% ethanol +0.2% diethylamine; flow rate: 40 ml/min; temperature: 40°C; UV detection: 220 nm]
¹H-NMR (500 MHz, DMSO-d6) δ [ppm]: 0.007 (1.73), 2.094 (0.60), 2.363 (0.49), 2.637 (0.48), 4.079 (1.82), 4.092 (2.00), 4.106 (2.78), 4.119 (2.52), 4.210 (2.40), 4.222 (2.60), 4.237 (1.88), 4.250 (1.66), 7.396 (2.33), 7.405 (2.58), 7.409 (2.61), 7.420 (2.48), 7.461 (5.00), 7.465 (1.90), 7.479 (8.45), 7.491 (1.84), 7.496 (5.24), 7.544 (4.51), 7.560 (4.85), 7.856 (2.15), 7.860 (2.21), 7.872 (3.49), 7.875 (3.52), 7.887 (1.87), 7.891 (1.84), 8.072 (5.19), 8.081 (5.55), 8.086 (3.28), 8.090 (5.39), 8.099 (4.99), 8.338 (1.52), 8.474 (16.00), 8.497 (1.67), 8.510 (3.24), 8.522 (1.58), 8.631 (3.25), 8.639 (3.16), 10.914 (0.96).

### Example 98

### N-{[(4R)-4-cyclopropyl-2,5-dioxoimidazolidin-4-yl]methyl}-2-(3,4,5-trifluorophenyl)-2H-1,2,3-triazole-4-carboxamide

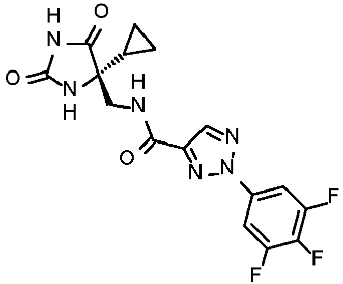

To a solution of (5R)-5-(aminomethyl)-5-cyclopropylimidazolidine-2,4-dione hydrochloride (120 mg, 584 µmol) and 2-(3,4,5-trifluorophenyl)-2H-1,2,3-triazole-4-carboxylic acid (142 mg, 584 µmol) in dichloromethane (12 ml) was added 1-ethyl-3-(3-dimethylaminopropyl)carbodiimide hydrochloride (145 mg, 759 µmol), 1-hydroxybenzotriazole hydrate (116 mg, 759 µmol) and N,N-diisopropylethylamine (285 µl, 1.6 mmol). The reaction mixture was stirred overnight at RT and concentrated under reduced pressure. The crude product was purified by preparative HPLC (Method 2f) The combined organic phases were concentrated in vacuo and the remaining TFA was removed, using a Stratospheres PL-HCO3 MP SPE cartridge. After concentration *in vacuo,* 116 mg (50 % yield) of the desired product were obtained.
LC-MS (Method 7): Rₜ = 1.46 min; MS (ESIpos): m/z = 395 [M+H]⁺
¹H-NMR (500 MHz, DMSO-d6) δ [ppm]: 0.117 (0.55), 0.128 (1.16), 0.136 (1.88), 0.146 (1.86), 0.156 (1.47), 0.167 (0.69), 0.310 (0.49), 0.328 (1.26), 0.331 (1.13), 0.340 (1.62), 0.349 (1.68), 0.356 (1.04), 0.365 (0.82), 0.405 (0.55), 0.415 (1.36), 0.426 (2.29), 0.432 (2.67), 0.445 (2.87), 0.456 (2.06), 0.468 (1.12), 1.137 (0.73), 1.148 (1.48), 1.153 (1.53), 1.158 (1.13), 1.164 (2.46), 1.175 (1.44), 1.180 (1.26), 1.191 (0.58), 2.121 (0.57), 3.612 (1.72), 3.624 (1.89), 3.639 (2.76), 3.651 (2.45), 3.731 (2.49), 3.745 (2.64), 3.758 (1.80), 3.772 (1.67), 7.572 (3.77), 8.003 (3.59), 8.016 (4.21), 8.020 (4.10), 8.033 (3.59), 8.543 (16.00), 8.565 (1.56), 8.578 (2.80), 8.590 (1.40).

### Example 99

### rac-N-[(4-isopropyl-2,5-dioxoimidazolidin-4-yl)methyl]-2-(3,4,5-trifluorophenyl)-2H-1,2,3-triazole-4-carboxamide

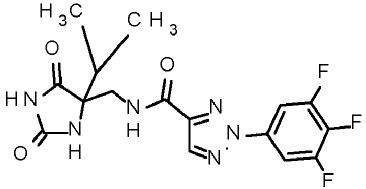

To a solution of *rac*-5-(aminomethyl)-5-(propan-2-yl)imidazolidine-2,4-dione (120 mg, 701 µmol) and 2-(3,4,5-trifluorophenyl)-2H-1,2,3-triazole-4-carboxylic acid (170 mg, 701 µmol) in DCM (14 ml) was added 1-ethyl-3-(3-dimethylaminopropyl)carbodiimide hydrochloride (175 mg, 911 µmol), 1-hydroxybenzotriazole hydrate (140 mg, 911 µmol) and N,N-diisopropylethylamine (340 µl, 2.0 mmol). The reaction mixture was stirred overnight at RT and concentrated under reduced pressure. The crude product was purified by preparative HPLC (Method 2f) The combined organic phases were concentrated in vacuo and the remaining TFA was removed, using a Stratospheres PL-HCO3 MP SPE cartridge. After lyophilization, 53.8 mg (94 % purity, 18 % yield) of the desired product were obtained.
LC-MS (Method 8): Rₜ = 0.83 min; MS (ESIpos): m/z = 397 [M+H]⁺
¹H-NMR (500 MHz, DMSO-d6) δ [ppm]: 0.006 (0.93), 0.844 (12.17), 0.857 (12.22), 0.957 (11.98), 0.971 (12.18), 1.974 (0.74), 1.987 (1.88), 2.001 (2.45), 2.014 (1.74), 2.028 (0.65), 2.121 (0.41), 3.546 (1.67), 3.559 (1.82), 3.574 (2.49), 3.587 (2.20), 3.686 (2.29), 3.699 (2.43), 3.714 (1.75), 3.727 (1.60), 7.286 (0.43), 7.861 (0.45), 7.874 (0.53), 7.899 (6.54), 8.014 (3.30), 8.027 (3.92), 8.031 (3.85), 8.044 (3.36), 8.542 (1.73), 8.557 (16.00), 8.567 (1.62), 10.661 (2.15).

### Example 100

### rac-2-(4-fluorophenyl)-N-{[4-(5-methyl-1,3-thiazol-4-yl)-2,5-dioxoimidazolidin-4-yl]methyl}-2H-1,2,3-triazole-4-carboxamide

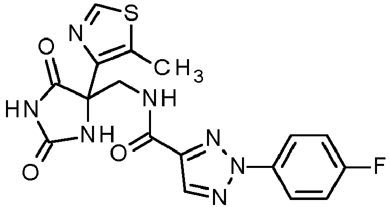

2-(4-fluorophenyl)-2H-1,2,3-triazole-4-carboxylic acid (101 mg, 485 µmol) dissolved in 10 ml of dichloromethane was treated with N-ethyl-N-isopropylpropan-2-amine (240 µl, 1.4 mmol), 3-{[(ethylimino)methylene]amino}-N,N-dimethylpropan-1-amine hydrochloride (121 mg, 631 µmol), 1H-benzotriazol-1-ol hydrate (96.6 mg, 631 µmol) and rac-5-(aminomethyl)-5-(5-methyl-1,3-thiazol-4-yl)imidazolidine-2,4-dione hydrochloride (150 mg, 85 % purity, 485 µmol). The mixture was stirred over night at room temperature. The solvent was removed on a rotary evaporator. The residue was taken up with ethyl acetate and washed with water and saturated aqueous sodium chloride solution. The organic layer was dried over sodium sulfate, filtered, Isolute® was added and the solvent was evaporated on a rotary evaporator. The crude product was purified by column chromatography (Machine: Biotage® Isolera One; column: Biotage® SNAP Ultra 25 g; gradient: DCM/MeOH-gradient, 2% MeOH - 20% MeOH; flow: 75 ml/min). Product containing samples were united and the solvents were removed on a rotary evaporator. 80.0 mg (100 % purity, 40 % yield) of the title compound were obtained.
LC-MS (Method 8): Rₜ = 0.74 min; MS (ESIpos): m/z = 416 [M+H]⁺
¹H-NMR (400 MHz, DMSO-d6) δ [ppm]: -0.008 (2.26), 0.008 (2.45), 2.356 (16.00), 4.187 (0.46), 4.206 (1.38), 4.222 (1.55), 4.226 (1.56), 4.242 (1.42), 4.260 (0.43), 4.277 (0.40), 7.456 (2.11), 7.461 (0.75), 7.478 (3.34), 7.494 (0.75), 7.500 (2.28), 8.073 (2.16), 8.078 (0.88), 8.085 (2.28), 8.090 (1.35), 8.096 (2.30), 8.102 (0.83), 8.107 (2.11), 8.356 (2.84), 8.473 (0.68), 8.483 (7.58), 8.505 (0.67), 8.930 (7.41), 11.027 (2.07).

### Example 101

### N-{[(4R)-4-cyclopropyl-2,5-dioxoimidazolidin-4-yl]methyl}-2-(3-fluoropyridin-2-yl)-2H-1,2,3-triazole-4-carboxamide

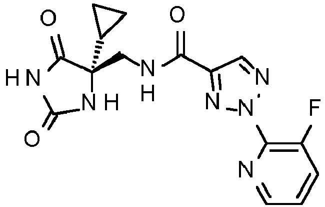

2-(3-fluoropyridin-2-yl)-2H-1,2,3-triazole-4-carboxylic acid (97.0 mg, 83 % purity, 387 µmol) dissolved in 5 ml of dichloromethane was treated with N-ethyl-N-isopropylpropan-2-amine (190 µl, 1.1 mmol), 3-{[(ethylimino)methylene]amino}-N,N-dimethylpropan-1-amine hydrochloride (96.4 mg, 503 µmol), 1H-benzotriazol-1-ol hydrate (77.0 mg, 503 µmol) and (5R)-5-(aminomethyl)-5-cyclopropylimidazolidine-2,4-dione hydrochloride (79.5 mg, 387 µmol). The mixture was stirred over night at room temperature. The solvent was removed on a rotary evaporator. The residue was taken up with dimethylformamide and purified by preparative HPLC (Instrument: Waters Prep LC/MS System, column: Phenomenex Kinetex C18 5 µm 100 x 30 mm; eluent A : water, eluent B : acetonitrile, eluent C : 2 % formic acid in water, eluent D : acetonitrile/water (80 Vol.% / 20 Vol%) flow rate: 80 ml/min, room temperature, UV-detection 200-400 nm, At-Column Injektion; gradient: eluent A 0 bis 2 min 70 ml, eluent B 0 bis 2 min 0 ml, eluent A 2 bis 10 min from 70 ml to 55 ml and eluent B from 0 ml to 15 ml, 10 bis 12 min 0 ml eluent A and 70 ml eluent B. Eluent C and eluent D constant flow 5 ml/min over the whole runtime). After lyophilization, 3.00 mg (100 % purity, 2 % yield) of the title compound were obtained.
LC-MS (Method 7): Rₜ = 0.95 min; MS (ESIpos): m/z = 360 [M+H]⁺
¹H-NMR (400 MHz, DMSO-d6) δ [ppm]: -0.149 (1.88), -0.008 (16.00), 0.008 (15.37), 0.146 (2.20), 0.335 (0.78), 0.402 (1.15), 0.420 (2.09), 0.438 (1.57), 1.146 (0.78), 1.161 (0.84), 2.135 (0.99), 2.323 (1.57), 2.327 (2.25), 2.332 (1.62), 2.366 (1.88), 2.381 (1.10), 2.523 (8.58), 2.665 (1.67), 2.670 (2.41), 2.674 (1.73), 2.710 (1.88), 3.695 (3.03), 3.711 (3.08), 7.602 (2.72), 7.772 (0.73), 7.783 (0.94), 7.793 (1.73), 7.804 (1.10), 7.813 (0.94), 8.163 (0.94), 8.166 (0.99), 8.189 (1.41), 8.210 (0.99), 8.214 (0.94), 8.519 (1.93), 8.530 (2.51), 8.565 (6.80), 10.647 (1.15).

### Example 102

### diamix-N-[(4-sec-butyl-2,5-dioxoimidazolidin-4-yl)methyl]-2-(4-fluorophenyl)-2H-1,2,3-triazole-4-carboxamide

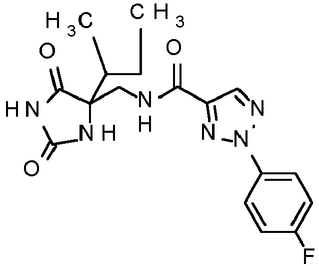

2-(4-fluorophenyl)-2H-1,2,3-triazole-4-carboxylic acid (145 mg, 699 µmol) dissolved in 15 ml of dichloromethane was treated with N-ethyl-N-isopropylpropan-2-amine (340 µl, 2.0 mmol), 3-{[(ethylimino)methylene]amino}-N,N-dimethylpropan-1-amine hydrochloride (174 mg, 909 µmol), 1H-benzotriazol-1-ol hydrate (139 mg, 909 µmol) and *diamix*-5-(aminomethyl)-5-sec-butylimidazolidine-2,4-dione hydrochloride (155 mg, 699 µmol). The mixture was stirred over night at room temperature. The solvent was removed on a rotary evaporator. The residue was taken up with ethyl acetate and washed with water and saturated aqueous sodium chloride solution. The organic layer was dried over sodium sulfate, filtered, Isolute® was added and the solvent was evaporated on a rotary evaporator. The crude product was purified by column chromatography (Machine: Biotage® Isolera One; column: Biotage® SNAP Ultra 10 g; cyclohexane/ethyl acetate-gradient, 12% ethyl acetate - 100% ethyl acetate; flow: 36 ml/min). Product containing samples were united and the solvents were removed on a rotary evaporator. 122 mg (100 % purity, 47 % yield) of the title compound were obtained.
LC-MS (Method 8): Rₜ = 0.79 min; MS (ESIpos): m/z = 375 [M+H]⁺
¹H-NMR (400 MHz, DMSO-d6) δ [ppm]: -0.008 (1.37), 0.008 (1.52), 0.831 (4.13), 0.850 (14.86), 0.868 (16.00), 0.883 (10.07), 0.901 (5.42), 0.945 (9.24), 0.962 (9.70), 0.986 (0.72), 1.004 (0.74), 1.013 (0.85), 1.019 (0.97), 1.031 (1.00), 1.050 (1.04), 1.065 (0.93), 1.077 (0.79), 1.084 (0.95), 1.095 (0.81), 1.102 (0.75), 1.110 (0.88), 1.129 (0.66), 1.299 (0.72), 1.306 (0.79), 1.319 (0.82), 1.325 (0.87), 1.332 (0.78), 1.339 (0.75), 1.351 (0.62), 1.364 (1.39), 1.618 (0.82), 1.636 (0.94), 1.651 (1.13), 1.668 (1.68), 1.685 (1.50), 1.695 (1.81), 1.702 (1.68), 1.712 (1.66), 1.720 (1.66), 1.729 (1.08), 1.739 (0.88), 1.746 (0.66), 2.366 (0.53), 2.410 (0.40), 2.710 (0.52), 3.585 (0.79), 3.602 (0.97), 3.610 (1.10), 3.620 (2.47), 3.626 (1.50), 3.637 (2.50), 3.645 (3.32), 3.663 (3.53), 3.675 (2.47), 3.683 (1.50), 3.691 (2.54), 3.710 (0.95), 3.725 (0.81), 7.450 (0.75), 7.458 (7.13), 7.464 (2.54), 7.480 (12.17), 7.497 (2.67), 7.502 (7.76), 7.511 (0.88), 7.752 (4.09), 7.839 (3.86), 8.063 (0.81), 8.072 (7.01), 8.077 (2.96), 8.084 (7.43), 8.089 (4.47), 8.094 (7.26), 8.101 (2.83), 8.106 (6.76), 8.115 (0.78), 8.299 (1.20), 8.314 (2.64), 8.322 (1.86), 8.331 (1.82), 8.338 (2.79), 8.354 (1.27), 8.463 (12.37), 10.661 (5.23).

### Example 103

### ent-2-(4-fluorophenyl)-N-{[(4R)-4-(5-methyl-1,3-thiazol-4-yl)-2,5-dioxoimidazolidin-4-yl]methyl}-2H-1,2,3-triazole-4-carboxamide

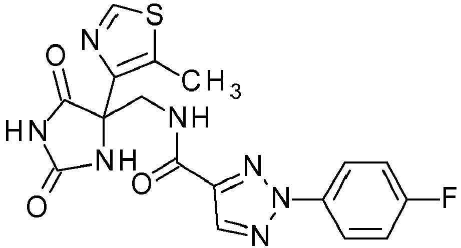

Enantiomeric separation of *rac*-2-(4-fluorophenyl)-N-{[4-(5-methyl-1,3-thiazol-4-yl)-2,5-dioxoimidazolidin-4-yl]methyl}-2H-1,2,3-triazole-4-carboxamide (75.0 mg, 181 µmol) using the following method
- Column: Diacel Chiralpak IH 5 µm 250^{∗}20 mm
- Eluent A: 70% n-heptane, eluent B: 30% ethanol
- Flow: 15 ml/min
- UV-detection: 220 nm
- Temperature: 35°C
afforded 35.0 mg (100 % purity, 47 % yield) of the desired product.
Chiral-HPLC (Column: Diacel Chiralpak IH 5 µm 250^{∗}4,6 mm, eluent A: 50% n-heptane, eluent B: 50% ethanol, flow: 1 ml/min, UV-detection: 220 nm, temperature: 50°C): Rt= 8.31 min; 99 % ee
LC-MS (Method 7): Rₜ = 1.42 min; MS (ESIpos): m/z = 416 [M+H]⁺
¹H-NMR (600 MHz, DMSO-d6) δ [ppm]: 0.005 (0.48), 2.354 (16.00), 2.517 (0.99), 2.568 (0.50), 4.185 (0.51), 4.196 (0.58), 4.208 (1.42), 4.218 (1.27), 4.228 (1.32), 4.239 (1.47), 4.251 (0.61), 4.262 (0.54), 7.468 (1.94), 7.472 (0.70), 7.483 (3.06), 7.494 (0.73), 7.497 (2.09), 8.081 (1.95), 8.085 (0.83), 8.089 (2.07), 8.093 (1.25), 8.096 (2.13), 8.100 (0.81), 8.104 (1.99), 8.379 (3.30), 8.494 (7.05), 8.511 (0.74), 8.522 (1.45), 8.533 (0.72), 8.935 (7.00).

### Example 104

### ent-N-[(4-sec-butyl-2,5-dioxoimidazolidin-4-yl)methyl]-2-(4-fluorophenyl)-2H-1,2,3-triazole-4-carboxamide

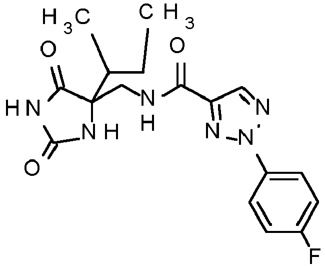

Enantiomeric separation of *diamix*-N-[(4-sec-butyl-2,5-dioxoimidazolidin-4-yl)methyl]-2-(4-fluorophenyl)-2H-1,2,3-triazole-4-carboxamide (120 mg, 321 µmol) using the following method
- Column: Diacel Chiralpak OZ-H 5 µm 250^{∗}20 mm
- Eluent A: 70% n-heptane, eluent B: 30% ethanol
- Flow: 15 ml/min
- UV-detection: 220 nm
- Temperature: 40°C
afforded 25.0 mg (100 % purity, 21 % yield) of the desired product.
Chiral-HPLC (Column: Diacel Chiralpak IA 5 µm 250^{∗}4,6 mm, eluent A: 0% n-heptane, eluent B: 100% ethanol, flow: 1 ml/min, UV-detection: 220 nm, temperature: 60°C): Rt= 5.29 min; 99 % ee
LC-MS (Method 7): Rₜ = 1.56 min; MS (ESIpos): m/z = 375 [M+H]⁺
¹H-NMR (400 MHz, DMSO-d6) δ [ppm]: -0.008 (2.79), 0.008 (3.23), 0.831 (5.67), 0.849 (14.79), 0.868 (7.43), 0.946 (13.29), 0.963 (13.97), 1.051 (0.79), 1.069 (0.92), 1.077 (1.00), 1.084 (1.25), 1.095 (1.11), 1.102 (1.22), 1.110 (1.28), 1.129 (0.93), 1.288 (0.50), 1.300 (1.07), 1.307 (1.17), 1.319 (1.20), 1.325 (1.29), 1.333 (1.14), 1.339 (1.09), 1.351 (0.83), 1.358 (0.78), 1.696 (1.00), 1.703 (1.26), 1.713 (1.22), 1.721 (1.79), 1.729 (1.17), 1.739 (1.12), 1.746 (0.89), 3.586 (1.11), 3.603 (1.24), 3.620 (3.27), 3.637 (3.13), 3.649 (3.13), 3.665 (3.27), 3.683 (1.24), 3.699 (1.09), 7.449 (0.52), 7.458 (5.49), 7.464 (1.85), 7.480 (8.96), 7.490 (1.00), 7.496 (1.92), 7.502 (5.92), 7.511 (0.63), 7.840 (8.03), 8.063 (0.57), 8.071 (5.57), 8.077 (2.17), 8.083 (5.89), 8.089 (3.36), 8.094 (5.79), 8.100 (2.04), 8.106 (5.39), 8.115 (0.53), 8.299 (1.68), 8.315 (3.45), 8.331 (1.64), 8.463 (16.00), 10.659 (2.93).

### Example 105

### 2-(1,3-benzothiazol-2-yl)-N-{[(4R)-4-cyclopropyl-2,5-dioxoimidazolidin-4-yl]methyl}-2H-1,2,3-triazole-4-carboxamide

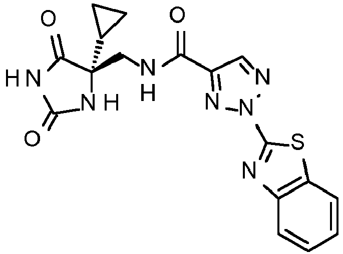

2-(1,3-benzothiazol-2-yl)-2H-1,2,3-triazole-4-carboxylic acid (36.0 mg, 146 µmol) dissolved in 3.5 ml of dichloromethane was treated with N-ethyl-N-isopropylpropan-2-amine (71 µl, 410 µmol), 3-{[(ethylimino)methylene]amino}-N,N-dimethylpropan-1-amine hydrochloride (36.4 mg, 190 µmol), 1H-benzotriazol-1-ol hydrate (29.1 mg, 190 µmol) and (5R)-5-(aminomethyl)-5-cyclopropylimidazolidine-2,4-dione hydrochloride (30.1 mg, 146 µmol). The mixture was stirred 48 h at room temperature. The solvent was removed on a rotary evaporator. The residue was taken up with dimethylformamide and purified by preparative HPLC (Instrument: Waters Prep LC/MS System, Column: Phenomenex Kinetex C18 5µm 100x30 mm; eluent A: water, eluent B: acetonitrile, eluent C: 2 % formic acid in water, eluent D: acetonitrile/water (80Vol.% / 20Vol%); flow: 80 ml/min , room temperature, UV-detection: 200-400 nm, At-Column Injektion; gradient: eluent A 0 bis 2 min 63 ml, eluent B 0 bis 2 min 7 ml, eluent A 2 bis 10 min from 63 ml to 39 ml and eluent B from 7 ml to 31 ml, 10 to 12 min 0 ml eluent A and 70 ml eluent B. Eluent C und Eluent D constant flow 5 ml/min over the whole runtime). After lyophilization, 28.0 mg (92 % purity, 44 % yield) of the title compound were obtained.
LC-MS (Method 7): Rₜ = 1.31 min; MS (ESIpos): m/z = 398 [M+H]⁺
¹H-NMR (400 MHz, DMSO-d6) δ [ppm]: -0.149 (0.43), -0.008 (3.11), 0.008 (3.54), 0.123 (0.98), 0.131 (1.47), 0.146 (2.02), 0.155 (1.53), 0.161 (1.34), 0.173 (0.73), 0.314 (0.43), 0.334 (1.47), 0.346 (1.53), 0.352 (1.77), 0.361 (1.40), 0.372 (1.16), 0.382 (0.92), 0.425 (2.63), 0.442 (5.25), 0.461 (4.27), 0.480 (1.47), 1.146 (0.67), 1.163 (1.59), 1.180 (2.20), 1.196 (1.53), 1.213 (0.55), 2.328 (0.61), 2.367 (0.92), 2.524 (2.63), 2.670 (0.55), 2.710 (0.92), 3.679 (0.43), 3.695 (0.49), 3.714 (5.07), 3.718 (4.89), 3.730 (4.76), 3.734 (4.95), 3.752 (0.55), 3.768 (0.43), 6.571 (6.60), 7.505 (0.43), 7.528 (1.95), 7.531 (2.02), 7.549 (4.15), 7.566 (3.24), 7.569 (3.18), 7.600 (2.87), 7.604 (3.11), 7.621 (4.21), 7.624 (4.03), 7.639 (7.45), 7.671 (0.55), 7.692 (0.79), 7.712 (0.55), 7.823 (1.04), 7.845 (0.73), 8.061 (4.46), 8.080 (4.09), 8.114 (0.85), 8.136 (0.79), 8.205 (4.21), 8.223 (3.85), 8.678 (16.00), 8.742 (1.71), 8.758 (3.79), 8.774 (1.77), 10.673 (5.01).

### Example 106

### N-{[(4R)-4-cyclopropyl-2,5-dioxoimidazolidin-4-yl]methyl}-2-[4-(trifluoromethoxy)phenyl]-2H-1,2,3-triazole-4-carboxamide

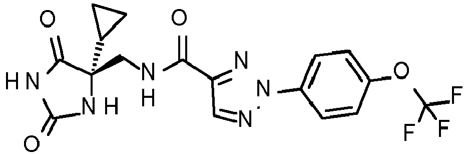

2-[4-(trifluoromethoxy)phenyl]-2H-1,2,3-triazole-4-carboxylic acid (14.7 mg, 53.6 µmol) dissolved in 2 ml of dichloromethane was treated with N-ethyl-N-isopropylpropan-2-amine (26 µl, 150 µmol 3-{[(ethylimino)methylene]amino}-N,N-dimethylpropan-1-amine hydrochloride (13.4 mg, 69.7 µmol), 1H-benzotriazol-1-ol hydrate (10.7 mg, 69.7 µmol) and (5R)-5-(aminomethyl)-5-cyclopropylimidazolidine-2,4-dione hydrochloride (11.0 mg, 53.6 µmol). The mixture was stirred over night at room temperature. The solvent was removed on a rotary evaporator. The residue was taken up with dimethylformamide and purified by preparative HPLC (Instrument: Waters Prep LC/MS System, Column: Phenomenex Kinetex C18 5µm 100x30 mm; eluent A: water, eluent B: acetonitrile, eluent C: 2 % formic acid in water, eluent D: acetonitrile/water (80Vol.% / 20Vol%); flow: 80 ml/min, room temperature, UV-detection: 200-400 nm, At-Column Injektion; gradient: eluent A 0 bis 2 min 55 ml, eluent B 0 bis 2 min 15 ml, eluent A 2 bis 10 min from 55 ml to 31 ml and eluent B from 15 ml to 39 ml, 10 to 12 min 0 ml eluent A and 70 ml eluent B. Eluent C und Eluent D constant flow 5 ml/min over the whole runtime). After lyophilization, 7.00 mg (100 % purity, 31 % yield) of the title compound were obtained.
LC-MS (Method 7): Rₜ = 1.61 min; MS (ESIpos): m/z = 425 [M+H]⁺
¹H-NMR (600 MHz, DMSO-d6) δ [ppm]: 0.118 (0.53), 0.126 (1.06), 0.133 (1.51), 0.141 (1.80), 0.144 (1.47), 0.152 (1.31), 0.160 (0.69), 0.323 (0.49), 0.337 (1.27), 0.344 (1.39), 0.348 (1.67), 0.356 (1.59), 0.362 (0.98), 0.369 (0.78), 0.429 (1.59), 0.432 (1.55), 0.443 (4.29), 0.454 (3.67), 0.467 (1.63), 1.150 (0.73), 1.159 (1.35), 1.163 (1.43), 1.173 (2.12), 1.182 (1.35), 1.195 (0.57), 2.426 (0.53), 2.521 (0.69), 2.655 (0.53), 3.296 (0.53), 3.303 (0.57), 3.349 (0.49), 3.355 (0.73), 3.361 (0.41), 3.651 (1.76), 3.661 (1.92), 3.674 (3.14), 3.684 (2.73), 3.728 (2.82), 3.739 (3.10), 3.750 (1.84), 3.762 (1.67), 6.639 (0.65), 7.635 (7.14), 7.646 (6.16), 7.660 (6.33), 8.171 (1.31), 8.176 (10.86), 8.180 (3.31), 8.188 (3.22), 8.191 (10.20), 8.197 (0.98), 8.510 (16.00), 8.524 (3.43), 8.535 (1.71), 10.662 (4.49).

### Example 107

### ent-2-(3,4-difluorophenyl)-N-{[2,5-dioxo-4-(1,3-thiazol-2-yl)imidazolidin-4-yl]methyl}-2H-1,2,3-triazole-4-carboxamide

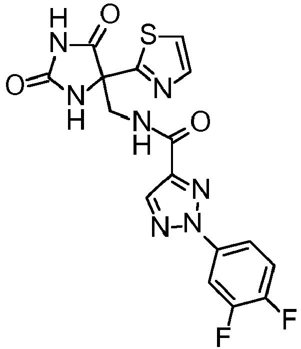

To a solution of rac-5-(aminomethyl)-5-(1,3-thiazol-2-yl)imidazolidine-2,4-dione hydrochloride (80.0 mg, 322 µmol) and 2-(3,4-difluorophenyl)-2H-1,2,3-triazole-4-carboxylic acid (72.4 mg, 322 µmol) in DMF (2 ml) was added 1-ethyl-3-(3-dimethylaminopropyl)carbodiimide hydrochloride (80.2 mg, 418 µmol), 1-hydroxybenzotriazole hydrate (64.0 mg, 418 µmol) and N,N-diisopropylethylamine (280 µl, 1.6 mmol). The reaction mixture was first stirred overnight at RT, then, for 8 h at 60°C and finally overnight at RT. The reaction mixture was concentrated under reduced pressure and the crude product was first purified by preparative HPLC (Method 2f). A second purification by preparative chiral HPLC [sample preparation: 26 mg dissolved in a mixture of ethanol and acetonitrile (4/1); injection volume: 0.5 ml; column: Daicel Chiralpack IG 5 µm 250^{∗}20 mm; eluent: ethanol; flow rate: 15 ml/min; temperature: 60 °C; UV detection: 220 nm] gave 7 mg (100 % purity, 5 % yield) of the desired product
Analytical chiral HPLC: Rₜ = 7.20 min, e.e. = > 99% [column: 250 x 4.6 mm filled with Daicel Chiralpak IG 5 µm; eluent: 100% ethanol; flow rate: 1 ml/min; temperature: 60°C; UV detection: 220 nm]
¹H-NMR (500 MHz, DMSO-d6) δ [ppm]: -0.120 (0.48), -0.007 (4.66), 0.006 (4.28), 0.116 (0.50), 2.363 (0.51), 2.637 (0.51), 4.066 (2.07), 4.079 (2.18), 4.093 (2.86), 4.107 (2.57), 4.227 (2.33), 4.239 (2.57), 4.254 (1.89), 4.267 (1.70), 7.709 (1.29), 7.727 (2.74), 7.747 (2.74), 7.765 (1.47), 7.817 (6.95), 7.823 (8.04), 7.891 (8.57), 7.898 (7.16), 7.915 (2.21), 7.933 (1.80), 8.105 (1.53), 8.110 (1.44), 8.119 (1.65), 8.125 (1.79), 8.133 (1.45), 8.141 (1.47), 8.147 (1.35), 8.522 (16.00), 8.732 (1.91), 8.745 (3.62), 8.757 (2.26), 11.105 (0.92).

### Example 108

### rac-N-{[2,5-dioxo-4-(1,3-thiazol-2-yl)imidazolidin-4-yl]methyl}-2-(3-fluorophenyl)-2H-1,2,3-triazole-4-carboxamide

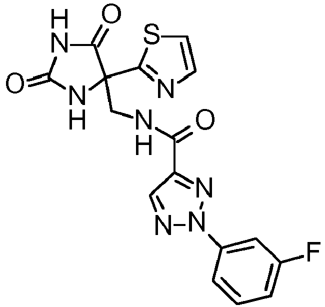

To a solution of rac-5-(aminomethyl)-5-(1,3-thiazol-2-yl)imidazolidine-2,4-dione hydrochloride (84.0 mg, 338 µmol) and 2-(3-fluorophenyl)-2H-1,2,3-triazole-4-carboxylic acid (70.0 mg, 338 µmol) in DMF (2.1 ml) was added 1-ethyl-3-(3-dimethylaminopropyl)carbodiimide hydrochloride (84.2 mg, 439 µmol), 1-hydroxybenzotriazole hydrate (67.3 mg, 439 µmol) and N,N-diisopropylethylamine (290 µl, 1.7 mmol). The reaction mixture was stirred overnight at RT and concentrated under reduced pressure. The crude product was purified by preparative HPLC (Method 3f). After lyophilisation, 5.00 mg (100 % purity, 4 % yield) of the desired product were obtained.
LC-MS (Method 8): Rₜ = 0.78 min; MS (ESIpos): m/z = 402 [M+H]⁺
¹H-NMR (500 MHz, DMSO-d6) δ [ppm]: 0.006 (1.47), 3.283 (0.48), 4.073 (2.37), 4.086 (2.53), 4.100 (3.26), 4.113 (2.97), 4.246 (3.08), 4.258 (3.31), 4.273 (2.57), 4.286 (2.25), 7.359 (1.63), 7.363 (1.75), 7.375 (3.24), 7.380 (3.29), 7.392 (1.75), 7.397 (1.68), 7.660 (1.82), 7.673 (2.46), 7.677 (3.61), 7.689 (3.63), 7.693 (2.14), 7.705 (1.77), 7.828 (8.23), 7.834 (9.40), 7.874 (2.37), 7.878 (3.70), 7.882 (2.62), 7.901 (11.79), 7.908 (8.21), 7.920 (4.55), 7.937 (3.72), 8.525 (16.00), 8.750 (2.25), 8.763 (4.44), 8.775 (2.14), 8.849 (9.01), 11.121 (3.43).

### Example 109

### rac-2-(4-chloro-3-fluorophenyl)-N-{[2,5-dioxo-4-(1,3-thiazol-2-yl)imidazolidin-4-yl]methyl}-2H-1,2,3-triazole-4-carboxamide

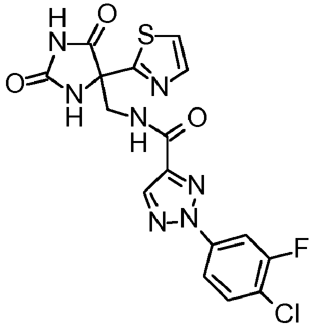

To a solution of rac-5-(aminomethyl)-5-(1,3-thiazol-2-yl)imidazolidine-2,4-dione hydrochloride (90.0 mg, 362 µmol) and 2-(4-chloro-3-fluorophenyl)-2H-1,2,3-triazole-4-carboxylic acid (87.4 mg, 362 µmol) in DMF (2.2 ml) was added 1-ethyl-3-(3-dimethylaminopropyl)carbodiimide hydrochloride (90.2 mg, 470 µmol), 1-hydroxybenzotriazole hydrate (72.0 mg, 470 µmol) and N,N-diisopropylethylamine (320 µl, 1.8 mmol). The reaction mixture was stirred overnight at RT and concentrated under reduced pressure. The crude product was purified by preparative HPLC (Method 4f). After lyophilisation, 44.5 mg (94 % purity, 27 % yield) of the desired product were obtained.
LC-MS (Method 12): Rₜ = 2.46 min; MS (ESIpos): m/z = 436 [M+H]⁺
¹H-NMR (400 MHz, DMSO-d6) δ [ppm]: 0.008 (1.11), 2.073 (12.53), 2.328 (0.82), 2.366 (0.61), 2.670 (0.77), 2.710 (0.58), 4.068 (1.98), 4.084 (2.13), 4.102 (3.03), 4.119 (2.74), 4.236 (2.79), 4.252 (3.13), 4.271 (2.27), 4.286 (1.97), 7.821 (8.05), 7.829 (10.38), 7.854 (2.72), 7.876 (5.12), 7.895 (14.21), 7.903 (8.23), 7.930 (4.45), 7.934 (4.54), 7.952 (2.32), 7.956 (2.39), 8.053 (0.47), 8.060 (0.51), 8.073 (3.84), 8.079 (4.00), 8.098 (3.80), 8.104 (3.65), 8.479 (1.29), 8.540 (16.00), 8.554 (0.58), 8.747 (1.83), 8.762 (3.86), 8.778 (1.85), 8.822 (6.42), 11.100 (5.15).

### Example 110

### ent-2-(4-chloro-3-fluorophenyl)-N-{[2,5-dioxo-4-(1,3-thiazol-2-yl)imidazolidin-4-yl]methyl}-2H-1,2,3-triazole-4-carboxamide

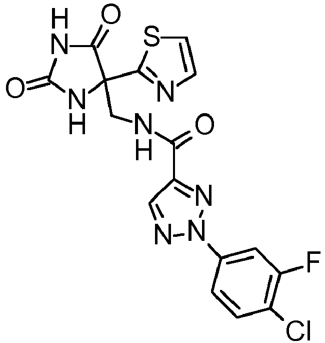

Enantiomeric separation of rac-2-(4-chloro-3-fluorophenyl)-N-{[2,5-dioxo-4-(1,3-thiazol-2-yl)imidazolidin-4-yl]methyl}-2H-1,2,3-triazole-4-carboxamide (44 mg) by preparative chiral HPLC [column: Daicel Chiralpak IC 5 µm, 250 x 20 mm; eluent: 70% n-heptane/30% ethanol; flow rate: 15 ml/min; temperature: 55°C; UV detection: 220 nm] afforded 21.7 mg of the desired product (99 % purity).
Analytical chiral HPLC: Rₜ = 9.22 min, e.e. = > 99% [column: 250 x 4.6 mm filled with Daicel Chiralpak IC 5 µm; eluent: 70% n-heptane/30% ethanol; flow rate: 1 ml/min; temperature: 55°C; UV detection: 220 nm]
LC-MS (Method 7): Rₜ = 1.52 min; MS (ESIpos): m/z = 436 [M+H]⁺
¹H-NMR (500 MHz, DMSO-d6) δ [ppm]: 0.846 (0.64), 0.861 (0.79), 0.872 (0.60), 1.088 (0.50), 1.101 (0.52), 2.403 (0.58), 2.634 (0.39), 2.678 (0.52), 3.261 (0.52), 3.288 (1.08), 3.392 (0.94), 4.074 (2.35), 4.087 (2.51), 4.102 (3.30), 4.115 (3.03), 4.243 (3.01), 4.255 (3.35), 4.270 (2.54), 4.282 (2.29), 7.825 (8.00), 7.831 (9.41), 7.860 (2.83), 7.878 (5.42), 7.893 (4.95), 7.898 (9.79), 7.905 (8.04), 7.932 (4.30), 7.936 (4.16), 7.950 (2.51), 7.955 (2.54), 8.077 (3.78), 8.082 (3.59), 8.098 (3.78), 8.102 (3.62), 8.543 (16.00), 8.763 (2.22), 8.775 (4.51), 8.788 (2.24), 8.832 (9.10), 11.099 (1.23), 11.139 (0.54).

### Example 111

### rac-2-(3-chloro-4-fluorophenyl)-N-{[2,5-dioxo-4-(1,3-thiazol-2-yl)imidazolidin-4-yl]methyl}-2H-1,2,3-triazole-4-carboxamide

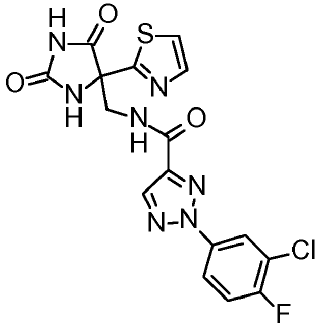

To a solution of rac-5-(aminomethyl)-5-(1,3-thiazol-2-yl)imidazolidine-2,4-dione hydrochloride (22.6 mg, 91.1 µmol) and 2-(3-chloro-4-fluorophenyl)-2H-1,2,3-triazole-4-carboxylic acid (22.0 mg, 91.1 µmol) in DMF (570 µl) was added 1-ethyl-3-(3-dimethylaminopropyl)carbodiimide hydrochloride (22.7 mg, 118 µmol), 1-hydroxybenzotriazole hydrate (18.1 mg, 118 µmol) and N,N-diisopropylethylamine (79 µl, 460 µmol). The reaction mixture was stirred overnight at RT and concentrated under reduced pressure. The crude product was purified by preparative HPLC (Method 3f). After lyophilisation, 7.00 mg (100 % purity, 18 % yield) of the desired product were obtained.
LC-MS (Method 8): Rₜ = 0.86 min; MS (ESIpos): m/z = 436 [M+H]⁺
¹H-NMR (500 MHz, DMSO-d6) δ [ppm]: 0.006 (2.72), 2.076 (0.40), 2.364 (0.55), 2.637 (0.55), 3.373 (0.51), 4.072 (2.28), 4.085 (2.46), 4.100 (3.16), 4.113 (2.79), 4.239 (2.94), 4.251 (3.19), 4.266 (2.46), 4.279 (2.13), 7.691 (3.30), 7.709 (6.68), 7.727 (3.52), 7.826 (8.37), 7.833 (9.43), 7.898 (9.76), 7.904 (7.89), 8.054 (2.24), 8.059 (2.79), 8.062 (2.79), 8.067 (2.50), 8.072 (2.31), 8.077 (2.61), 8.080 (2.39), 8.086 (1.94), 8.251 (3.78), 8.256 (3.67), 8.264 (3.78), 8.269 (3.23), 8.525 (16.00), 8.774 (2.24), 8.786 (4.26), 8.799 (2.09), 8.835 (8.37), 11.115 (4.00).

### Example 112

### ent-2-(3-chloro-4-fluorophenyl)-N-{[2,5-dioxo-4-(1,3-thiazol-2-yl)imidazolidin-4-yl]methyl}-2H-1,2,3-triazole-4-carboxamide

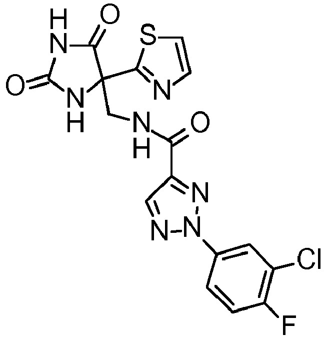

Enantiomeric separation of rac-2-(3-chloro-4-fluorophenyl)-N-{[2,5-dioxo-4-(1,3-thiazol-2-yl)imidazolidin-4-yl]methyl}-2H-1,2,3-triazole-4-carboxamide (6 mg) by preparative chiral HPLC [column: Daicel Chiralpak IC 5 µm, 250 x 20 mm; eluent: 60% n-heptane/40% ethanol; flow rate: 20 ml/min; temperature: 45°C; UV detection: 210 nm] afforded 1.73 mg of the desired product (98 % purity).
Analytical chiral HPLC: Rₜ = 1.84 min, e.e. = > 99% [column: Daicel Chiralpak IC-3 3 µm, 50 x 4.6 mm; eluent: 50% n-heptane /50% ethanol; flow rate: 1 ml/min; UV detection: 220 nm]
LC-MS (Method 7): Rₜ = 1.49 min; MS (ESIpos): m/z = 436 [M+H]⁺
¹H-NMR (500 MHz, DMSO-d6) δ [ppm]: 0.858 (1.22), 1.031 (0.46), 1.045 (0.46), 1.141 (0.86), 1.154 (1.42), 1.169 (0.86), 1.235 (2.69), 2.365 (1.07), 2.639 (0.97), 2.911 (0.66), 2.925 (0.66), 4.072 (3.30), 4.085 (3.66), 4.100 (4.67), 4.113 (4.32), 4.240 (4.42), 4.252 (4.83), 4.267 (3.71), 4.280 (3.25), 7.692 (3.96), 7.710 (8.03), 7.728 (4.42), 7.827 (8.84), 7.833 (10.57), 7.898 (10.16), 7.904 (9.45), 8.062 (4.77), 8.080 (4.52), 8.257 (5.64), 8.265 (5.64), 8.526 (16.00), 8.780 (3.56), 8.792 (6.86), 8.804 (3.96), 8.840 (11.17), 11.098 (0.81).

### Example 113

### rac-N-{[2,5-dioxo-4-(1,3-thiazol-2-yl)imidazolidin-4-yl]methyl}-2-(4-fluorophenyl)-2H-1,2,3-triazole-4-carboxamide

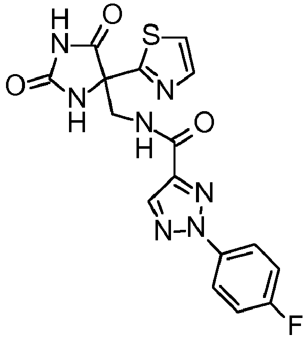

To a solution of *rac*-5-(aminomethyl)-5-(1,3-thiazol-2-yl)imidazolidine-2,4-dione hydrochloride (90.0 mg, 362 µmol) and 2-(4-fluorophenyl)-2H-1,2,3-triazole-4-carboxylic acid (75.0 mg, 362 µmol) in DMF (2.2 ml) was added 1-ethyl-3-(3-dimethylaminopropyl)carbodiimide hydrochloride (90.2 mg, 470 µmol), 1-hydroxybenzotriazole hydrate (72.0 mg, 470 µmol) and N,N-diisopropylethylamine (320 µl, 1.8 mmol). The reaction mixture was stirred overnight at RT and concentrated under reduced pressure. The crude product was purified by preparative HPLC (Method 3f). After lyophilisation, 50.7 mg (100 % purity, 35 % yield) of the desired product were obtained.
LC-MS (Method 8): Rₜ = 0.78 min; MS (ESIpos): m/z = 402 [M+H]⁺
¹H-NMR (400 MHz, DMSO-d6) δ [ppm]: -0.149 (0.46), -0.008 (3.72), 0.008 (3.56), 0.146 (0.41), 2.328 (0.45), 2.367 (0.48), 2.524 (1.58), 2.671 (0.50), 2.711 (0.50), 4.055 (2.34), 4.071 (2.55), 4.089 (3.39), 4.106 (3.12), 4.245 (3.12), 4.260 (3.47), 4.279 (2.58), 4.295 (2.30), 7.429 (0.47), 7.437 (4.43), 7.443 (1.58), 7.450 (1.35), 7.459 (12.31), 7.464 (4.14), 7.481 (14.23), 7.490 (1.64), 7.497 (2.29), 7.503 (6.56), 7.511 (0.70), 7.820 (8.18), 7.828 (10.07), 7.895 (10.28), 7.903 (8.36), 8.065 (1.08), 8.074 (9.61), 8.079 (4.00), 8.086 (10.16), 8.091 (6.03), 8.097 (9.79), 8.103 (3.77), 8.109 (9.23), 8.117 (0.99), 8.480 (16.00), 8.490 (5.46), 8.666 (2.00), 8.682 (4.29), 8.698 (2.01), 8.822 (8.40), 11.100 (3.30).

### Example 114

### ent-N-{[2,5-dioxo-4-(1,3-thiazol-2-yl)imidazolidin-4-yl]methyl}-2-(4-fluorophenyl)-2H-1,2,3-triazole-4-carboxamide

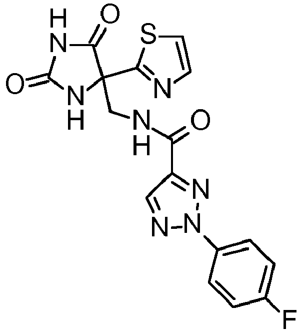

Enantiomeric separation of rac-N-{[2,5-dioxo-4-(1,3-thiazol-2-yl)imidazolidin-4-yl]methyl}-2-(4-fluorophenyl)-2H-1,2,3-triazole-4-carboxamide (50.4 mg) by preparative chiral HPLC [column: Daicel Chiralpak IC 5 µm, 250 x 20 mm; eluent: 60% n-heptane/40% isopropanol; flow rate: 20 ml/min; temperature: 50°C; UV detection: 210 nm] afforded 15.8 mg of the desired product (98 % purity).
Analytical chiral HPLC: Rₜ = 3.05 min, e.e. = > 99% [column: Daicel Chiralpak IC-3 3 µm, 50 x 4.6 mm; eluent: 50% n-heptane/50% isopropanol; flow rate: 1 ml/min; UV detection: 220 nm]
LC-MS (Method 7): Rₜ = 1.28 min; MS (ESIpos): m/z = 402 [M+H]⁺
¹H-NMR (400 MHz, DMSO-d6) δ [ppm]: -0.008 (0.93), 0.008 (0.93), 1.139 (0.57), 1.157 (1.24), 1.175 (0.63), 1.234 (0.55), 1.283 (1.40), 2.328 (0.57), 2.366 (0.73), 2.524 (2.90), 2.670 (0.61), 2.710 (0.73), 2.917 (0.49), 2.934 (0.47), 4.054 (2.07), 4.071 (2.25), 4.089 (2.98), 4.105 (2.76), 4.244 (2.68), 4.259 (3.04), 4.278 (2.27), 4.293 (2.05), 7.459 (5.45), 7.464 (1.99), 7.481 (9.00), 7.497 (2.03), 7.503 (5.86), 7.820 (8.05), 7.828 (9.86), 7.894 (9.75), 7.903 (8.01), 8.074 (5.66), 8.080 (2.39), 8.086 (5.96), 8.092 (3.59), 8.097 (5.82), 8.103 (2.23), 8.109 (5.39), 8.479 (16.00), 8.661 (1.82), 8.677 (3.83), 8.693 (1.78), 8.815 (8.03), 10.989 (0.45), 11.096 (0.87).

### Example 115

### rac-2-(2,4-difluorophenyl)-N-{[2,5-dioxo-4-(1,3-thiazol-2-yl)imidazolidin-4-yl]methyl}-2H-1,2,3-triazole-4-carboxamide

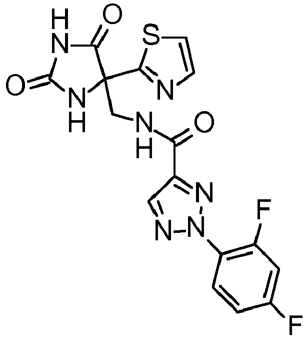

To a solution of rac-5-(aminomethyl)-5-(1,3-thiazol-2-yl)imidazolidine-2,4-dione hydrochloride (44.2 mg, 178 µmol) and 2-(2,4-difluorophenyl)-2H-1,2,3-triazole-4-carboxylic acid (40.0 mg, 178 µmol) in DMF (1.1 ml) was added 1-ethyl-3-(3-dimethylaminopropyl)carbodiimide hydrochloride (44.3 mg, 231 µmol), 1-hydroxybenzotriazole hydrate (35.4 mg, 231 µmol) and N,N-diisopropylethylamine (150 µl, 890 µmol). The reaction mixture was stirred overnight at RT and concentrated under reduced pressure. The crude product was purified by preparative HPLC (Method 3f). After lyophilisation, 15.0 mg (96 % purity, 19 % yield) of the desired product were obtained.
LC-MS (Method 8): Rₜ = 0.74 min; MS (ESIpos): m/z = 420 [M+H]⁺
¹H-NMR (400 MHz, DMSO-d6) δ [ppm]: -0.008 (2.57), 0.008 (2.41), 4.027 (2.46), 4.042 (2.67), 4.061 (3.41), 4.077 (3.10), 4.255 (3.12), 4.271 (3.45), 4.289 (2.69), 4.305 (2.42), 7.346 (1.28), 7.349 (1.42), 7.352 (1.49), 7.356 (1.37), 7.369 (2.64), 7.372 (2.81), 7.375 (2.61), 7.389 (1.38), 7.392 (1.49), 7.395 (1.55), 7.399 (1.36), 7.656 (1.93), 7.663 (1.90), 7.679 (2.19), 7.684 (3.21), 7.690 (2.05), 7.706 (1.99), 7.713 (1.83), 7.814 (7.93), 7.822 (9.94), 7.884 (9.99), 7.892 (8.11), 7.925 (1.98), 7.940 (2.20), 7.947 (3.59), 7.962 (3.59), 7.969 (1.96), 7.984 (1.79), 8.527 (16.00), 8.639 (2.10), 8.654 (4.41), 8.670 (2.04), 8.801 (9.27), 11.091 (2.70).

### Example 116

### ent-2-(2,4-difluorophenyl)-N-{[2,5-dioxo-4-(1,3-thiazol-2-yl)imidazolidin-4-yl]methyl}-2H-1,2,3-triazole-4-carboxamide

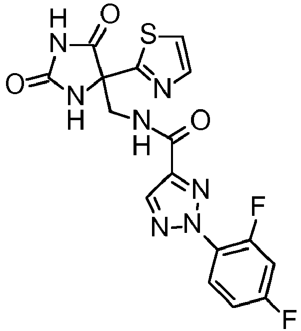

Enantiomeric separation of *rac*-2-(2,4-difluorophenyl)-N-{[2,5-dioxo-4-(1,3-thiazol-2-yl)imidazolidin-4-yl]methyl}-2H-1,2,3-triazole-4-carboxamide (14 mg) by preparative chiral HPLC [column: Daicel Chiralpak IE 5 µm, 250 x 20 mm; eluent: 50% n-heptane/50% ethanol; flow rate: 20 ml/min; temperature: 45°C; UV detection: 210 nm] afforded 1.98 mg of the desired product (98 % purity).
Analytical chiral HPLC: Rₜ = 3.74 min, e.e. = > 99% [column: Daicel Chiralpak IE-3 3 µm, 50 x 4.6 mm; eluent: 50% n-heptane /50% ethanol; flow rate: 1 ml/min; UV detection: 220 nm]
LC-MS (Method 7): Rₜ = 1.23 min; MS (ESIpos): m/z = 420 [M+H]⁺
¹H-NMR (400 MHz, DMSO-d6) δ [ppm]: 1.137 (0.48), 1.156 (0.88), 1.174 (0.49), 1.235 (0.62), 2.328 (0.83), 2.367 (0.46), 2.670 (0.96), 2.711 (0.51), 4.026 (2.34), 4.042 (2.50), 4.060 (3.22), 4.076 (2.93), 4.254 (2.96), 4.270 (3.25), 4.289 (2.52), 4.304 (2.31), 7.352 (1.45), 7.372 (2.88), 7.395 (1.59), 7.656 (1.83), 7.663 (1.83), 7.679 (2.18), 7.684 (3.30), 7.690 (2.13), 7.706 (1.94), 7.713 (1.81), 7.813 (7.72), 7.821 (9.58), 7.883 (9.63), 7.892 (7.83), 7.925 (1.89), 7.939 (2.15), 7.947 (3.47), 7.961 (3.55), 7.969 (1.97), 7.983 (1.75), 8.527 (16.00), 8.638 (2.01), 8.653 (4.22), 8.668 (2.01), 8.799 (8.31), 11.084 (0.60).

### Example 117

### ent-N-[(4-isopropyl-2,5-dioxoimidazolidin-4-yl)methyl]-2-(3,4,5-trifluorophenyl)-2H-1,2,3-triazole-4-carboxamide

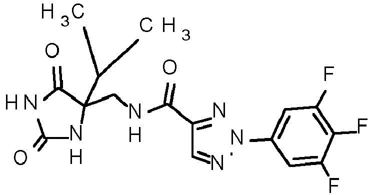

Enantiomeric separation of rac-N-[(4-isopropyl-2,5-dioxoimidazolidin-4-yl)methyl]-2-(3,4,5-trifluorophenyl)-2H-1,2,3-triazole-4-carboxamide (51 mg) by preparative chiral HPLC [column: Daicel Chiralcel® OX-H 5 µm, 250 x 20 mm; eluent: 60% n-heptane/40% isopropanol; flow rate: 15 ml/min; temperature: 40°C; UV detection: 210 nm] afforded 23.2 mg of the desired product (98 % purity).
Analytical chiral HPLC: Rₜ = 1.36 min, e.e. = > 99% [column: 50 x 4.6 mm filled with Daicel Chiralpak OX-3 3 µm; eluent: 50% n-heptane / 50% isopropanol; flow rate: 1 ml/min; UV detection: 220 nm
¹H NMR (500 MHz, DMSO-*d*₆) δ ppm 10.66 (bs, 1 H), 8.41 - 8.63 (m, 2 H), 8.03 (dd, 2 H), 7.82 (bs, 1 H), 3.46 - 3.79 (m, 2 H), 1.92 - 2.10 (m, 1 H), 0.77 - 1.02 (m, 6 H)

### Example 118

### rac-2-(3-fluorophenyl)-N-[(4-isopropyl-2,5-dioxoimidazolidin-4-yl)methyl]-2H-1,2,3-triazole-4-carboxamide

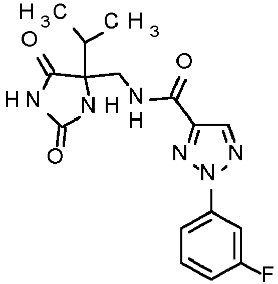

To a solution of rac-5-(aminomethyl)-5-(propan-2-yl)imidazolidine-2,4-dione (80.0 mg, 467 µmol) and 2-(3-fluorophenyl)-2H-1,2,3-triazole-4-carboxylic acid (96.8 mg, 467 µmol) in DMF (9.6 ml) was added 1-ethyl-3-(3-dimethylaminopropyl)carbodiimide hydrochloride (116 mg, 607 µmol), 1-hydroxybenzotriazole hydrate (93.0 mg, 607 µmol) and N,N-diisopropylethylamine (230 µl, 1.3 mmol). After stirring overnight at RT, extra portion of N,N-diisopropylethylamine (23 µl, 130 µmol) was added and the reaction mixture was stirred for 1h at 60°C and concentrated under reduced pressure. The crude product was first purified by preparative HPLC (Method 2f). A second purification by preparative HPLC (Method 3f) gave 56.4 mg (98 % purity, 33 % yield) of the desired product
LC-MS (Method 11): Rₜ = 0.99 min; MS (ESIpos): m/z = 361 [M+H]⁺
¹H-NMR (400 MHz, DMSO-d6) δ [ppm]: 0.845 (15.54), 0.862 (16.00), 0.955 (15.29), 0.972 (15.87), 1.970 (0.91), 1.986 (2.38), 2.004 (3.17), 2.020 (2.23), 2.038 (0.80), 2.073 (1.01), 2.328 (0.56), 2.670 (0.56), 3.573 (1.63), 3.589 (1.78), 3.607 (3.58), 3.624 (3.32), 3.657 (3.28), 3.673 (3.48), 3.691 (1.67), 3.707 (1.57), 7.349 (1.31), 7.354 (1.43), 7.370 (2.78), 7.375 (2.99), 7.391 (1.55), 7.396 (1.65), 7.652 (1.78), 7.668 (2.17), 7.673 (3.65), 7.688 (3.63), 7.693 (2.23), 7.709 (1.84), 7.805 (6.55), 7.863 (1.79), 7.868 (3.09), 7.874 (2.09), 7.887 (1.80), 7.893 (3.10), 7.898 (2.21), 7.914 (4.00), 7.935 (3.22), 7.938 (2.93), 8.387 (1.90), 8.403 (3.92), 8.419 (1.88), 8.500 (13.37), 10.656 (5.58).

### Example 119

### ent-2-(3-fluorophenyl)-N-[(4-isopropyl-2,5-dioxoimidazolidin-4-yl)methyl]-2H-1,2,3-triazole-4-carboxamide

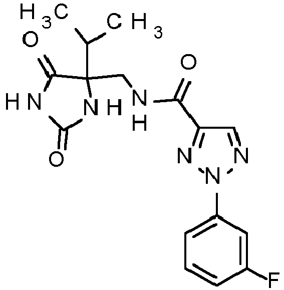

Enantiomeric separation of *rac*-2-(3-fluorophenyl)-N-[(4-isopropyl-2,5-dioxoimidazolidin-4-yl)methyl]-2H-1,2,3-triazole-4-carboxamide (42.4 mg) by preparative chiral HPLC [column: Daicel Chiralpak IC 5 µm, 250 x 20 mm; eluent: 80% n-heptan/20% ethanol; flow rate: 20 ml/min; temperature: 40°C; UV detection: 220 nm] afforded 5.4 mg of the desired product (99 % purity).
Analytical chiral HPLC: Rₜ = 8.46 min, e.e. = > 99% [column: 250 x 4.6 mm filled with Daicel Chiralpak IC 5 µm; eluent: 80% n-heptane / 20% ethanol; flow rate: 1 ml/min; temperature: 40°C; UV detection: 220 nm]
¹H-NMR (600 MHz, DMSO-d6) δ [ppm]: -0.189 (0.52), 0.659 (4.24), 0.670 (4.37), 0.771 (4.19), 0.782 (4.26), 1.805 (0.71), 1.816 (0.90), 1.827 (0.67), 2.318 (5.89), 3.163 (16.00), 3.389 (0.57), 3.399 (0.63), 3.411 (0.92), 3.423 (0.83), 3.484 (0.85), 3.495 (0.91), 3.507 (0.60), 3.518 (0.56), 7.177 (0.53), 7.191 (1.01), 7.202 (0.55), 7.477 (0.44), 7.490 (0.97), 7.500 (0.98), 7.513 (0.44), 7.658 (2.49), 7.690 (1.00), 7.706 (0.99), 7.733 (1.24), 7.747 (1.12), 8.255 (0.65), 8.266 (1.23), 8.276 (0.64), 8.326 (3.34), 10.486 (1.00).

### Example 120

### rac-2-(3-cyanophenyl)-N-[(4-isopropyl-2,5-dioxoimidazolidin-4-yl)methyl]-2H-1,2,3-triazole-4-carboxamide

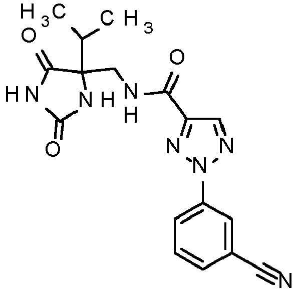

To a solution of rac-5-(aminomethyl)-5-(propan-2-yl)imidazolidine-2,4-dione (80.0 mg, 467 µmol) and 2-(3-cyanophenyl)-2H-1,2,3-triazole-4-carboxylic acid (100 mg, 467 µmol) in DMF (9.6 ml) was added 1-ethyl-3-(3-dimethylaminopropyl)carbodiimide hydrochloride (116 mg, 607 µmol), 1-hydroxybenzotriazole hydrate (93.0 mg, 607 µmol) and N,N-diisopropylethylamine (230 µl, 1.3 mmol). After stirring overnight at RT, extra portion of N,N-diisopropylethylamine (23 µl, 130 µmol) was added and the reaction mixture was stirred for 1h at 60°C and concentrated under reduced pressure. The crude product was first purified by preparative HPLC (Method 2f). A second purification by preparative HPLC (Method 3f) gave 63.5 mg (97 % purity, 27.4 % yield) of the desired product
LC-MS (Method 11): Rₜ = 0.93 min; MS (ESIpos): m/z = 368 [M+H]⁺
¹H-NMR (500 MHz, DMSO-d6) δ [ppm]: 0.847 (15.77), 0.860 (16.00), 0.961 (15.61), 0.974 (15.97), 1.222 (0.71), 1.235 (0.87), 1.755 (1.06), 1.978 (0.97), 1.992 (2.42), 2.006 (3.23), 2.019 (2.29), 2.033 (0.84), 2.364 (0.48), 2.638 (0.61), 3.563 (2.23), 3.576 (2.45), 3.591 (3.45), 3.604 (3.13), 3.684 (3.10), 3.696 (3.32), 3.711 (2.26), 3.724 (2.13), 7.830 (10.52), 7.847 (7.03), 7.863 (4.39), 7.983 (5.19), 7.998 (4.23), 8.374 (3.71), 8.378 (3.84), 8.391 (3.29), 8.393 (3.42), 8.427 (0.39), 8.478 (6.23), 8.482 (8.16), 8.486 (7.03), 8.501 (2.13), 8.548 (16.00), 8.559 (0.48), 10.670 (4.87).

### Example 121

### ent-2-(3-cyanophenyl)-N-[(4-isopropyl-2,5-dioxoimidazolidin-4-yl)methyl]-2H-1,2,3-triazole-4-carboxamide

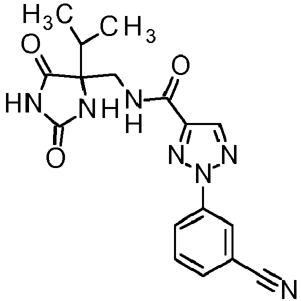

Enantiomeric separation of rac-2-(3-cyanophenyl)-N-[(4-isopropyl-2,5-dioxoimidazolidin-4-yl)methyl]-2H-1,2,3-triazole-4-carboxamide (51 mg) by preparative chiral HPLC [column: Daicel Chiralpak OX-H 5 µm, 250 x 20 mm; eluent: 50% n-heptane/50% ethanol; flow rate: 20 ml/min; temperature: 40°C; UV detection: 220 nm] afforded 23.5 mg of the desired product (98.5 % purity).
Analytical chiral HPLC: Rₜ = 2.43 min, e.e. = > 99% [column: Daicel OX-3 3 µm, 50 x 4.6 mm; eluent: 50% n-heptane / 50% ethanol; flow rate: 1 ml/min; UV detection: 220 nm]
LC-MS (Method 7): Rₜ = 1.26 min; MS (ESIpos): m/z = 368 [M+H]⁺
¹H-NMR (600 MHz, DMSO-d6) δ [ppm]: 0.850 (16.00), 0.861 (15.92), 0.962 (15.79), 0.974 (15.66), 1.112 (0.54), 1.124 (0.54), 1.985 (1.01), 1.996 (2.47), 2.007 (3.18), 2.018 (2.26), 2.030 (0.84), 2.162 (0.46), 2.426 (0.46), 2.655 (0.42), 3.300 (2.05), 3.360 (1.34), 3.573 (2.18), 3.583 (2.39), 3.596 (3.31), 3.606 (2.89), 3.684 (3.06), 3.695 (3.23), 3.707 (2.26), 3.718 (2.09), 7.815 (8.88), 7.833 (3.52), 7.846 (6.58), 7.859 (4.10), 7.981 (4.73), 7.994 (3.94), 8.375 (3.27), 8.378 (3.31), 8.391 (3.10), 8.460 (2.26), 8.471 (4.77), 8.475 (5.57), 8.478 (7.50), 8.481 (5.95), 8.543 (15.66), 10.661 (3.60).

### Example 122

### rac-2-(3,4-difluorophenyl)-N-[(4-isopropyl-2,5-dioxoimidazolidin-4-yl)methyl]-2H-1,2,3-triazole-4-carboxamide

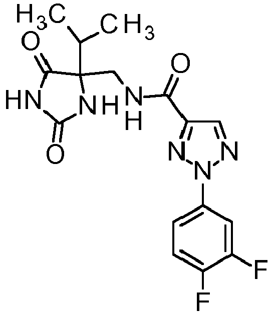

To a solution of *rac*-5-(aminomethyl)-5-(propan-2-yl)imidazolidine-2,4-dione (100 mg, 584 µmol) and 2-(3,4-difluorophenyl)-2H-1,2,3-triazole-4-carboxylic acid (132 mg, 584 µmol) in DMF (12 ml) was added 1-ethyl-3-(3-dimethylaminopropyl)carbodiimide hydrochloride (146 mg, 759 µmol), 1-hydroxybenzotriazole hydrate (116 mg, 759 µmol) and N,N-diisopropylethylamine (280 µl, 1.6 mmol). The reaction mixture was stirred overnight, then treated with water and extracted with ethyl acetate. The combined organic phases were concentrated under reduced pressure and the crude product was purified by preparative HPLC (Column: Chromatorex C18 10 µm;, 125 x 40mm, Eluent A: water + 0.05% trifluoroacetic acid, Eluent B: acetonitrile + 0.05% trifluoroacetic acid; Gradient: 15% B → 70% B) After lyophilisation, 52.2 mg (97 % purity, 23 % yield) of the desired product were obtained.
LC-MS (Method 11): Rₜ = 1.03 min; MS (ESIpos): m/z = 379 [M+H]⁺
¹H-NMR (400 MHz, DMSO-d6) δ [ppm]: 0.008 (0.52), 0.843 (15.48), 0.860 (16.00), 0.953 (15.26), 0.970 (15.85), 1.968 (0.87), 1.984 (2.36), 2.001 (3.13), 2.018 (2.23), 2.035 (0.79), 2.328 (0.69), 2.367 (0.47), 2.524 (2.24), 2.670 (0.70), 2.675 (0.52), 2.710 (0.51), 3.563 (1.69), 3.580 (1.88), 3.598 (3.41), 3.614 (3.11), 3.659 (3.05), 3.675 (3.29), 3.694 (1.77), 3.710 (1.67), 7.697 (1.41), 7.720 (3.08), 7.745 (3.14), 7.767 (1.90), 7.803 (6.14), 7.910 (2.36), 7.920 (1.60), 7.929 (1.54), 7.932 (1.93), 7.942 (1.02), 8.094 (1.52), 8.100 (1.49), 8.111 (1.70), 8.118 (1.82), 8.122 (1.87), 8.129 (1.64), 8.140 (1.65), 8.146 (1.54), 8.385 (1.80), 8.401 (3.82), 8.417 (1.80), 8.500 (14.10), 10.652 (5.36).

### Example 123

### ent-2-(3,4-difluorophenyl)-N-[(4-isopropyl-2,5-dioxoimidazolidin-4-yl)methyl]-2H-1,2,3-triazole-4-carboxamide

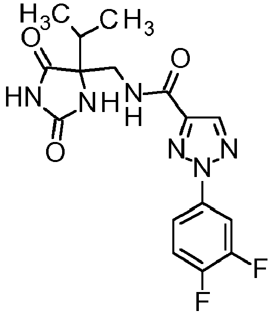

Enantiomeric separation of *rac*-2-(3,4-difluorophenyl)-N-[(4-isopropyl-2,5-dioxoimidazolidin-4-yl)methyl]-2H-1,2,3-triazole-4-carboxamide (50.4 mg) by preparative chiral HPLC [column: Daicel Chiralpak IC 5 µm, 250 x 20 mm; eluent: 80% n-heptan/20% ethanol; flow rate: 20 ml/min; temperature: 40°C; UV detection: 220 nm] afforded 4.3 mg of the desired product (99 % purity).
Analytical chiral HPLC: Rₜ = 8.47 min, e.e. = > 99% [column: 250 x 4.6 mm filled with Daicel Chiralpak IC 5 µm; eluent: 80% n-heptan / 20% ethanol; flow rate: 1 ml/min; temperature: 40°C; UV detection: 220 nm]
LC-MS (Method 8): Rₜ = 0.80 min; MS (ESIpos): m/z = 379 [M+H]⁺
¹H-NMR (400 MHz, DMSO-d6) δ [ppm]: -0.008 (1.94), 0.008 (2.25), 0.843 (15.28), 0.860 (16.00), 0.953 (14.75), 0.970 (15.38), 1.235 (0.62), 1.967 (0.86), 1.984 (2.37), 2.001 (3.11), 2.018 (2.16), 2.035 (0.77), 2.327 (0.62), 2.366 (0.93), 2.669 (0.74), 2.710 (0.93), 3.563 (1.68), 3.579 (1.84), 3.597 (3.35), 3.614 (3.04), 3.659 (3.09), 3.675 (3.31), 3.693 (1.77), 3.709 (1.72), 7.697 (1.39), 7.719 (3.16), 7.745 (3.23), 7.767 (1.89), 7.802 (8.53), 7.909 (2.40), 7.920 (1.77), 7.932 (2.06), 8.094 (1.60), 8.100 (1.60), 8.111 (1.87), 8.122 (1.99), 8.128 (1.77), 8.139 (1.80), 8.146 (1.65), 8.385 (1.82), 8.401 (3.86), 8.417 (1.84), 8.500 (15.86), 10.641 (2.20).

### Example 124

### rac-2-(4-chloro-3-fluorophenyl)-N-[(4-isopropyl-2,5-dioxoimidazolidin-4-yl)methyl]-2H-1,2,3-triazole-4-carboxamide

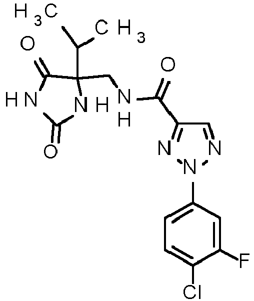

To a solution of *rac*-5-(aminomethyl)-5-(propan-2-yl)imidazolidine-2,4-dione (100 mg, 584 µmol) and 2-(4-chloro-3-fluorophenyl)-2H-1,2,3-triazole-4-carboxylic acid (141 mg, 584 µmol) in DMF (12 ml) was added 1-ethyl-3-(3-dimethylaminopropyl)carbodiimide hydrochloride (146 mg, 759 µmol), 1-hydroxybenzotriazole hydrate (116 mg, 759 µmol) and N,N-diisopropylethylamine (280 µl, 1.6 mmol). The reaction mixture was stirred overnight, then treated with water and extracted with ethyl acetate. The combined organic phases were concentrated under reduced pressure and the crude product was purified by preparative HPLC (Column: Chromatorex C18 10 µm;, 125 x 40mm, Eluent A: water + 0.05% trifluoroacetic acid, Eluent B: acetonitrile + 0.05% trifluoroacetic acid; Gradient: 15% B → 70% B) After lyophilisation, 35.5 mg (100 % purity, 15 % yield) of the desired product were obtained.
LC-MS (Method 7): Rₜ = 1.55 min; MS (ESIpos): m/z = 395 [M+H]⁺
¹H-NMR (400 MHz, DMSO-d6) δ [ppm]: 0.008 (1.05), 0.844 (15.52), 0.861 (16.00), 0.954 (15.22), 0.971 (15.82), 1.968 (0.90), 1.985 (2.33), 2.002 (3.14), 2.019 (2.21), 2.036 (0.81), 2.328 (0.81), 2.366 (0.57), 2.665 (0.66), 2.670 (0.84), 2.710 (0.57), 3.562 (1.76), 3.578 (1.94), 3.596 (3.41), 3.612 (3.11), 3.663 (3.05), 3.678 (3.29), 3.697 (1.85), 3.713 (1.70), 7.803 (6.43), 7.854 (2.54), 7.876 (5.05), 7.895 (4.73), 7.928 (4.13), 7.933 (4.01), 7.950 (2.15), 7.956 (2.21), 8.071 (3.56), 8.077 (3.32), 8.096 (3.56), 8.102 (3.32), 8.420 (1.88), 8.435 (4.04), 8.451 (1.91), 8.523 (13.79), 10.653 (5.62).

### Example 125

### ent-2-(4-chloro-3-fluorophenyl)-N-[(4-isopropyl-2,5-dioxoimidazolidin-4-yl)methyl]-2H-1,2,3-triazole-4-carboxamide

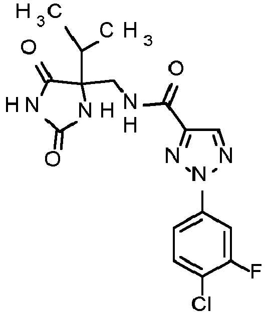

Enantiomeric separation of *rac*-2-(4-chloro-3-fluorophenyl)-N-[(4-isopropyl-2,5-dioxoimidazolidin-4-yl)methyl]-2H-1,2,3-triazole-4-carboxamide (33.7 mg) by preparative chiral HPLC [column: Daicel Chiralpak IC 5 µm, 250 x 20 mm; eluent: 75% n-heptan / 25% ethanol; flow rate: 20 ml/min; temperature: 40°C; UV detection: 220 nm] afforded 3.9 mg of the desired product (98 % purity).
Analytical chiral HPLC: Rₜ = 7.25 min, e.e. = > 99% [column: 250 x 4.6 mm filled with Daicel Chiralpak IC 5 µm; eluent: 75% n-heptan / 25% ethanol; flow rate: 1 ml/min; temperature: 40°C; UV detection: 220 nm]
¹H-NMR (400 MHz, DMSO-d6) δ [ppm]: -0.150 (0.48), -0.008 (4.14), 0.008 (4.47), 0.146 (0.44), 0.844 (13.62), 0.861 (14.24), 0.954 (13.00), 0.971 (13.51), 1.028 (0.44), 1.045 (0.40), 1.135 (0.62), 1.154 (1.17), 1.172 (0.70), 1.235 (1.32), 1.968 (0.81), 1.985 (2.05), 2.002 (2.71), 2.019 (1.94), 2.036 (0.70), 2.328 (0.92), 2.366 (1.28), 2.670 (0.99), 2.710 (1.32), 3.561 (1.50), 3.578 (1.68), 3.596 (2.89), 3.612 (2.64), 3.662 (2.60), 3.678 (2.86), 3.696 (1.65), 3.712 (1.50), 7.802 (7.18), 7.854 (2.49), 7.876 (4.76), 7.895 (4.54), 7.928 (3.73), 7.933 (3.81), 7.950 (1.94), 7.957 (2.01), 8.072 (3.59), 8.078 (3.33), 8.097 (3.55), 8.103 (3.33), 8.419 (1.61), 8.435 (3.41), 8.451 (1.65), 8.523 (16.00), 10.650 (2.53).

### Example 126

### ent-2-(3-chloro-4-fluorophenyl)-N-[(4-isopropyl-2,5-dioxoimidazolidin-4-yl)methyl]-2H-1,2,3-triazole-4-carboxamide

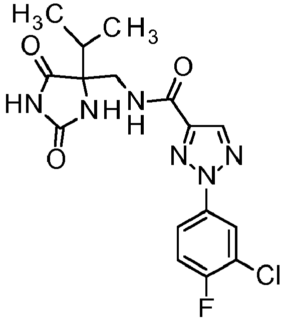

To a solution of rac-5-(aminomethyl)-5-(propan-2-yl)imidazolidine-2,4-dione (100 mg, 584 µmol) and 2-(3-chloro-4-fluorophenyl)-2H-1,2,3-triazole-4-carboxylic acid (141 mg, 584 µmol) in DMF (12 ml) was added 1-ethyl-3-(3-dimethylaminopropyl)carbodiimide hydrochloride (146 mg, 759 µmol), 1-hydroxybenzotriazole hydrate (116 mg, 759 µmol) and N,N-diisopropylethylamine (280 µl, 1.6 mmol). The reaction mixture was stirred overnight, then treated with water and extracted with ethyl acetate. The combined organic phases were concentrated under reduced pressure. The crude product was first purified by preparative HPLC (Column: Chromatorex C18 10 µm;, 125 x 30mm, Eluent A: water + 0.05% trifluoroacetic acid, Eluent B: acetonitrile + 0.05% trifluoroacetic acid; Gradient: 10% B → 90% B) A second purification by preparative chiral HPLC [sample preparation: 7.8 mg dissolved in a mixture of ethanol and DCM; injection volume: 1 ml; column: Daicel Chiralpack IC 5 µm 250^{∗}20 mm; eluent: 75% n-heptane/ 25% ethanol; flow rate: 20 ml/min; temperature: 40 °C; UV detection: 220 nm] gave 3.10 mg (99 % purity, 1 % yield) of the desired product
Analytical chiral HPLC: Rₜ = 7.12 min, e.e. = > 99% [column: 250 x 4.6 mm filled with Daicel Chiralpak Ic 5 µm; eluent: 75% n-heptane / 25% ethanol; flow rate: 1 ml/min; temperature: 40°C; UV detection: 220 nm]
¹H-NMR (500 MHz, DMSO-d6) δ [ppm]: 0.844 (15.48), 0.858 (16.00), 0.956 (14.86), 0.970 (15.30), 1.087 (0.44), 1.234 (0.87), 1.973 (0.96), 1.986 (2.36), 1.999 (3.06), 2.013 (2.19), 2.026 (0.87), 2.086 (0.52), 2.363 (0.70), 2.638 (0.79), 3.278 (1.40), 3.292 (3.15), 3.367 (2.19), 3.380 (1.49), 3.424 (0.44), 3.436 (0.44), 3.564 (1.92), 3.577 (2.19), 3.592 (3.41), 3.605 (3.06), 3.667 (2.97), 3.679 (3.23), 3.694 (2.19), 3.706 (1.92), 7.688 (3.23), 7.706 (6.47), 7.724 (3.32), 7.808 (8.04), 8.051 (2.01), 8.058 (2.54), 8.065 (2.27), 8.069 (2.10), 8.075 (2.45), 8.083 (1.84), 8.246 (3.50), 8.252 (3.41), 8.259 (3.58), 8.264 (3.06), 8.438 (2.10), 8.451 (4.20), 8.464 (2.01), 8.504 (14.25), 10.661 (3.93).

### Example 127

### rac-2-(4-fluorophenyl)-N-{[4-(1-methyl-1H-pyrazol-5-yl)-2,5-dioxoimidazolidin-4-yl]methyl}-2H-1,2,3-triazole-4-carboxamide

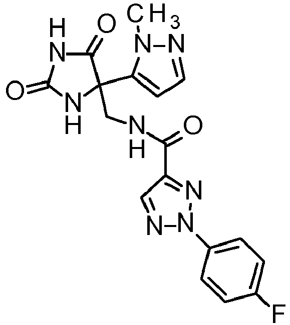

To a solution of *rac*-5-(aminomethyl)-5-(1-methyl-1H-pyrazol-5-yl)imidazolidine-2,4-dione hydrochloride (100 mg, 78 % purity, 317 µmol) and 2-(4-fluorophenyl)-2H-1,2,3-triazole-4-carboxylic acid (65.8 mg, 317 µmol) in DMF (1.6 ml) was added 1-ethyl-3-(3-dimethylaminopropyl)carbodiimide hydrochloride (79.1 mg, 413 µmol), 1-hydroxybenzotriazole hydrate (63.2 mg, 413 µmol) and N,N-diisopropylethylamine (280 µl, 1.6 mmol). The reaction mixture was stirred overnight at RT, after which extra portion of N,N-diisopropylethylamine (170 µl, 950 µmol) was added due to incomplete conversion. After stirring for 2 h at 50°C and overnight at RT, the resulting reaction mixture was concentrated under reduced pressure and the crude product was purified by preparative HPLC (Method 3f). After lyophilization, 5.10 mg (99 % purity, 4 % yield) of the desired product were obtained.
LC-MS (Method 7): Rₜ = 1.32 min; MS (ESIpos): m/z = 399 [M+H]⁺
¹H-NMR (500 MHz, DMSO-d6) δ [ppm]: 0.006 (0.57), 2.073 (0.66), 3.808 (16.00), 4.017 (0.64), 4.029 (0.72), 4.044 (1.34), 4.057 (1.20), 4.086 (1.21), 4.099 (1.31), 4.113 (0.67), 4.127 (0.62), 6.555 (3.41), 6.559 (3.48), 7.401 (3.57), 7.405 (3.56), 7.468 (2.20), 7.473 (1.03), 7.486 (3.66), 7.499 (0.84), 7.503 (2.23), 8.083 (2.23), 8.087 (1.15), 8.093 (2.39), 8.097 (1.57), 8.101 (2.39), 8.106 (1.03), 8.111 (2.20), 8.436 (3.26), 8.489 (6.59), 8.705 (0.77), 8.718 (1.54), 8.731 (0.75), 11.211 (1.02).

### Example 128

### rac-N-({4-[1-(difluoromethyl)-1H-pyrazol-5-yl]-2,5-dioxoimidazolidin-4-yl}methyl)-2-(4-fluorophenyl)-2H-1,2,3-triazole-4-carboxamide

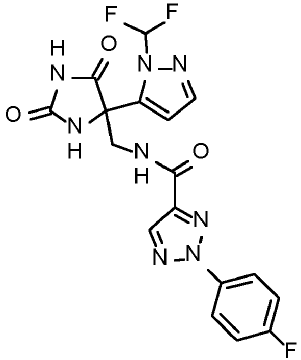

To a solution of *rac*-5-(aminomethyl)-5-[1-(difluoromethyl)-1H-pyrazol-5-yl]imidazolidine-2,4-dione hydrochloride (26.5 mg, 94.1 µmol) and 2-(4-fluorophenyl)-2H-1,2,3-triazole-4-carboxylic acid (19.5 mg, 94.1 µmol) in DMF (460 µl) was added 1-ethyl-3-(3-dimethylaminopropyl)carbodiimide hydrochloride (23.4 mg, 122 µmol), 1-hydroxybenzotriazole hydrate (18.7 mg, 122 µmol) and N,N-diisopropylethylamine (82 µl, 470 µmol). After stirring overnight at RT, extra portion of N,N-diisopropylethylamine (49 µl, 280 µmol) was added and the reaction mixture was stirred for 2h at 50°C and concentrated under reduced pressure. The crude product was purified by preparative HPLC (Method 3f). After lyophilization, 16.9 mg (99 % purity, 41 % yield) of the desired product were obtained
LC-MS (Method 7): Rₜ = 1.45 min; MS (ESIpos): m/z = 435 [M+H]⁺
¹H-NMR (500 MHz, DMSO-d6) δ [ppm]: -0.006 (1.41), 0.007 (0.44), 2.073 (0.82), 2.362 (0.53), 3.990 (0.87), 4.003 (0.97), 4.017 (3.35), 4.032 (5.28), 4.045 (3.25), 4.060 (0.92), 4.073 (0.78), 6.770 (7.03), 6.773 (6.79), 7.464 (0.58), 7.471 (4.80), 7.475 (1.89), 7.488 (8.00), 7.501 (1.84), 7.506 (4.99), 7.513 (0.58), 7.802 (6.74), 7.805 (6.50), 7.859 (1.84), 7.970 (1.84), 7.978 (1.75), 8.075 (0.78), 8.082 (5.19), 8.086 (2.86), 8.091 (6.59), 8.096 (3.49), 8.100 (5.38), 8.105 (2.13), 8.109 (4.85), 8.116 (0.58), 8.492 (16.00), 8.505 (0.44), 8.595 (7.27), 8.828 (1.70), 8.840 (3.49), 8.853 (1.70), 11.216 (2.18).

### Example 129

### ent-N-({4-[1-(difluoromethyl)-1H-pyrazol-5-yl]-2,5-dioxoimidazolidin-4-yl}methyl)-2-(4-fluorophenyl)-2H-1,2,3-triazole-4-carboxamide

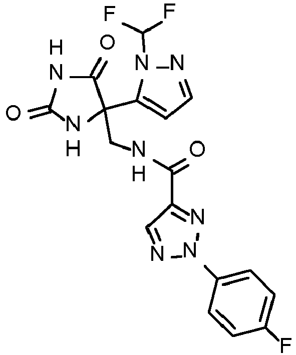

Enantiomeric separation of *rac*-N-({4-[1-(difluoromethyl)-1H-pyrazol-5-yl]-2,5-dioxoimidazolidin-4-yl}methyl)-2-(4-fluorophenyl)-2H-1,2,3-triazole-4-carboxamide (15.1 mg) by preparative chiral HPLC [column: Daicel Chiralpak ID 5 µm, 250 x 20 mm; eluent: 50% n-heptan/50% ethanol; flow rate: 15 ml/min; temperature: 30°C; UV detection: 220 nm] afforded 4.8 mg of the desired product (97 % purity).
Analytical chiral HPLC: Rₜ = 6.93 min, e.e. = 99% [column: 250 x 4.6 mm filled with Daicel Chiralpak ID 5 µm; eluent: 50% n-heptan / 50% ethanol; flow rate: 1 ml/min; temperature: 40°C; UV detection: 220 nm]
¹H-NMR (400 MHz, DMSO-d6) δ [ppm]: -0.149 (0.62), -0.008 (4.76), 0.008 (5.39), 0.146 (0.55), 0.844 (0.43), 0.858 (0.59), 0.874 (0.43), 1.128 (1.09), 1.146 (2.15), 1.164 (1.09), 1.235 (1.17), 2.073 (0.66), 2.323 (0.94), 2.327 (1.29), 2.332 (0.94), 2.366 (1.25), 2.523 (5.89), 2.665 (1.05), 2.670 (1.37), 2.674 (1.01), 2.710 (1.25), 2.893 (0.70), 2.911 (0.66), 3.980 (0.70), 3.997 (0.82), 4.015 (4.18), 4.027 (4.80), 4.032 (4.88), 4.042 (4.29), 4.061 (0.86), 4.077 (0.70), 6.767 (8.16), 6.771 (8.27), 7.466 (5.81), 7.472 (2.07), 7.489 (9.64), 7.505 (2.11), 7.510 (6.36), 7.519 (0.70), 7.800 (7.06), 7.804 (7.06), 7.829 (2.42), 7.969 (2.34), 7.978 (2.22), 8.069 (0.66), 8.078 (5.97), 8.083 (2.46), 8.090 (6.32), 8.095 (3.75), 8.101 (6.28), 8.107 (2.42), 8.113 (6.24), 8.492 (16.00), 8.586 (3.98), 8.822 (1.87), 8.838 (4.06), 8.854 (1.91).

### Example 130

### rac-N-{[4-(1-ethyl-1H-pyrazol-5-yl)-2,5-dioxoimidazolidin-4-yl]methyl}-2-(4-fluorophenyl)-2H-1,2,3-triazole-4-carboxamide

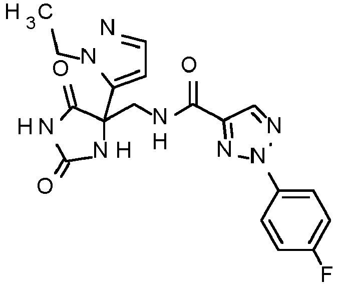

2-(4-fluorophenyl)-2H-1,2,3-triazole-4-carboxylic acid (21.5 mg, 104 µmol) dissolved in 3 ml dichloromethan was treated with N,N-diisopropylethylamine (51 µl, 290 µmol), 1-ethyl-3-(3-dimethylaminopropyl)carbodiimide hydrochloride (25.9 mg, 135 µmol) and 1-hydroxybenzotriazole hydrate (20.7 mg, 135 µmol) and stirred for 5 min at room temperature before *rac*-5-(aminomethyl)-5-(1-ethyl-1H-pyrazol-5-yl)imidazolidine-2,4-dione-hydrogen chloride (30 mg, 104 µmol, 90% purity) was added. The mixture was stirred at room temperature for 3 h. Purification was done by preparative HPLC (Instrument: Waters Prep LC/MS System, column: Phenomenex Kinetex C18 5µm 100x30 mm; eluent A : water, eluent B : acetonitrile, eluent C : 2 % formic acid in water, eluent D : acetonitrile/water (80Vol.% / 20Vol%) flow: 80 ml/min, room temperature, UV 200-400 nm, At-Column Injektion. Gradient: eluent A 0 to 2 min 63 ml, eluent B 0 to 2min 7 ml, eluent A 2 to 10 min from 63 ml to 39 ml and eluent B from 7 ml to 31 ml, 10 to 12 min 0 ml eluent A and 70 ml eluent B. Eluent C and eluent D constant flow each 5 ml/min over the whole time). Further purification was needed by preparative HPLC (Column: 250^{∗}20mm Daicel Chiralpak AZ-H 5µm; eluent: 50% n-heptane, 50% 2-propanol; flow: 15 ml/min; temperature: 40°C). Product containing samples were united, the solvents were evaporated and the residue was lyophylized. 5.00 mg (100 % purity, 12 % yield) of the title compound were obtained.
LC-MS (Method 7): Rₜ = 1.32 min; MS (ESIpos): m/z = 413 [M+H]⁺
¹H-NMR (500 MHz, DMSO-d6) δ [ppm]: 1.300 (7.74), 1.314 (16.00), 1.329 (7.62), 2.073 (4.46), 4.006 (0.87), 4.021 (2.29), 4.035 (4.93), 4.049 (8.24), 4.064 (6.82), 4.070 (3.73), 4.078 (2.76), 4.084 (3.35), 4.092 (1.30), 4.098 (1.25), 4.111 (1.06), 6.548 (6.89), 6.552 (6.63), 7.460 (7.69), 7.464 (7.67), 7.469 (5.03), 7.487 (7.76), 7.504 (4.46), 8.085 (4.74), 8.095 (5.07), 8.099 (3.49), 8.103 (4.86), 8.113 (4.34), 8.461 (7.01), 8.492 (12.53), 8.700 (1.72), 8.713 (3.40), 8.726 (1.68), 11.213 (3.19).

### Example 131

### rac-2-(4-fluorophenyl)-N-{[4-(4-methyl-1,3-thiazol-5-yl)-2,5-dioxoimidazolidin-4-yl]methyl}-2H-1,2,3-triazole-4-carboxamide

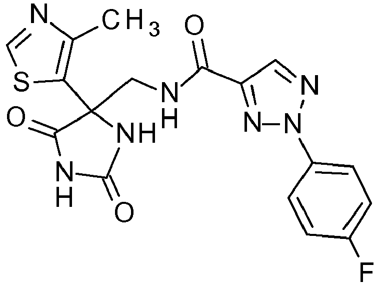

2-(4-fluorophenyl)-2H-1,2,3-triazole-4-carboxylic acid (78.9 mg, 381 µmol) dissolved in 10 ml dichloromethane was treated with N,N-diisopropylethylamine (190 µl, 1.1 mmol), 1-ethyl-3-(3-dimethylaminopropyl)carbodiimide hydrochloride (94.9 mg, 495 µmol) and 1-hydroxybenzotriazole hydrate (75.8 mg, 495 µmol) and stirred for 5 min at room temperature before *rac-*5-(aminomethyl)-5-(4-methyl-1,3-thiazol-5-yl)imidazolidine-2,4-dione-hydrogen chloride (100 mg, 381 µmol) was added. The mixture was stirred at room temperature over night. Acetonitrile was added, the precipitate was filtered of, washed with acetonitrile and dried in vacuo. 40.0 mg (100 % purity, 25 % yield) of the title compound were obtained.
LC-MS (Method 7): Rₜ = 1.25 min; MS (ESIpos): m/z = 416 [M+H]⁺
¹H-NMR (400 MHz, DMSO-d6) δ [ppm]: 2.501 (16.00), 4.034 (1.44), 4.041 (1.48), 4.049 (1.44), 4.057 (1.39), 7.465 (1.52), 7.470 (0.56), 7.487 (2.76), 7.504 (0.60), 7.509 (1.65), 8.073 (1.59), 8.085 (1.68), 8.090 (1.03), 8.096 (1.64), 8.107 (1.52), 8.476 (3.81), 8.597 (2.44), 8.682 (0.59), 8.698 (1.24), 8.714 (0.59), 8.927 (2.64), 11.171 (1.28).

### Example 132

### N-{[(4R)-4-cyclopropyl-2,5-dioxoimidazolidin-4-yl]methyl}-2-(1,2-thiazol-4-yl)-2H-1,2,3-triazole-4-carboxamide

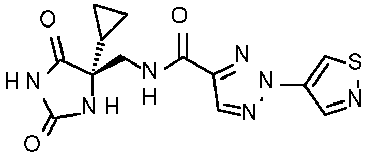

2-(1,2-thiazol-4-yl)-2H-1,2,3-triazole-4-carboxylic acid (45.0 mg, 229 µmol) dissolved in 5 ml dichloromethane was treated with N,N-diisopropylethylamine (110 µl, 640 µmol), 1-ethyl-3-(3-dimethylaminopropyl)carbodiimide hydrochloride (57.2 mg, 298 µmol) and 1-hydroxybenzotriazole hydrate (45.7 mg, 298 µmol) and stirred for 5 min at room temperature before (5R)-5-(aminomethyl)-5-cyclopropylimidazolidine-2,4-dione hydrochloride (47.2 mg, 229 µmol) was added. The mixture was stirred at room temperature over night. Purification was done by preparative HPLC (column: Chromatorex C18 10 µm, 250 x 30 mm, eluent A = water, B = acetonitrile; gradient: 0.0 min 5% B; 3 min 5% B; 20 min 50% B; 23 min 100% B; 26 min 5% B; flow: 50 ml/min; 0.1% formic acid). Product containing samples were united, the solvents were evaporated and the residue was lyophylized. 54.0 mg (100 % purity, 68 % yield) of the title compound were obtained.
LC-MS (Method 7): Rₜ = 0.96 min; MS (ESIpos): m/z = 348 [M+H]⁺
¹H-NMR (400 MHz, DMSO-d6) δ [ppm]: 0.105 (0.59), 0.116 (1.19), 0.128 (1.67), 0.137 (2.23), 0.143 (1.75), 0.151 (1.56), 0.166 (0.83), 0.308 (0.50), 0.328 (1.47), 0.337 (1.77), 0.346 (2.32), 0.355 (1.73), 0.365 (1.28), 0.376 (1.05), 0.417 (2.60), 0.435 (5.25), 0.452 (4.72), 0.472 (1.77), 1.133 (0.78), 1.148 (1.53), 1.153 (1.64), 1.167 (2.49), 1.182 (1.64), 1.201 (0.65), 2.367 (0.40), 3.633 (1.67), 3.648 (1.84), 3.667 (3.79), 3.682 (3.47), 3.714 (3.44), 3.731 (3.69), 3.748 (1.75), 3.765 (1.67), 7.616 (6.65), 8.487 (2.15), 8.495 (16.00), 8.502 (4.32), 8.518 (2.06), 9.100 (11.53), 9.459 (10.74), 10.650 (5.43).

### Example 133

### rac-N-({2,5-dioxo-4-[1-(2,2,2-trifluoroethyl)-1H-pyrazol-5-yl]imidazolidin-4-yl}methyl)-2-(4-fluorophenyl)-2H-1,2,3-triazole-4-carboxamide

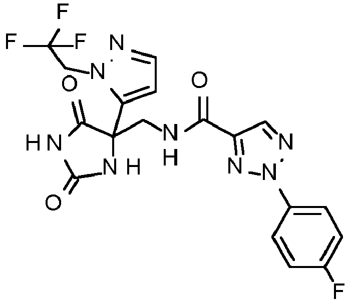

2-(4-fluorophenyl)-2H-1,2,3-triazole-4-carboxylic acid (19.8 mg, 95.6 µmol) dissolved in 3 ml dichloromethane was treated with N,N-diisopropylethylamine (47 µl, 270 µmol), 1-ethyl-3-(3-dimethylaminopropyl)carbodiimide hydrochloride (23.8 mg, 124 µmol) and 1-hydroxybenzotriazole hydrate (19.0 mg, 124 µmol) and stirred for 5 min at room temperature before *rac-*5-(aminomethyl)-5-[1-(2,2,2-trifluoroethyl)-1H-pyrazol-5-yl]imidazolidine-2,4-dione hydrochloride (30.0 mg, 95.6 µmol) was added. The mixture was stirred at room temperature for 2 h. Purification was done by preparative HPLC (Instrument: Waters Prep LC/MS System, column: Phenomenex Kinetex C18 5µm 100x30 mm; eluent A : water, eluent B : acetonitrile, eluent C : 2 % formic acid in water, eluent D : acetonitrile/water (80Vol.% / 20Vol%) flow: 80 ml/min, room temperature, UV 200-400 nm, At-Column Injektion. Gradient: eluent A 0 to 2 min 63 ml, eluent B 0 to 2min 7 ml, eluent A 2 to 10 min from 63 ml to 39 ml and eluent B from 7 ml to 31 ml, 10 to 12 min 0 ml eluent A and 70 ml eluent B. Eluent C and eluent D constant flow each 5 ml/min over the whole time). Product containing samples were united, the solvents were evaporated and the residue was lyophylized. 18.0 mg (100 % purity, 40 % yield) of the title compound were obtained.
LC-MS (Method 7): Rₜ = 1.53 min; MS (ESIpos): m/z = 467 [M+H]⁺
¹H-NMR (600 MHz, DMSO-d6) δ [ppm]: 0.005 (0.62), 2.520 (1.50), 3.967 (1.50), 3.979 (1.68), 3.990 (2.78), 4.002 (2.55), 4.039 (2.51), 4.049 (2.87), 4.062 (1.73), 4.072 (1.52), 5.153 (0.54), 5.164 (0.91), 5.179 (2.69), 5.194 (3.71), 5.208 (2.54), 5.220 (1.07), 5.234 (0.58), 6.654 (7.65), 6.658 (7.86), 7.468 (0.47), 7.474 (4.62), 7.478 (1.76), 7.489 (7.44), 7.500 (1.83), 7.503 (4.94), 7.509 (0.73), 7.628 (8.16), 7.632 (8.06), 8.077 (0.49), 8.082 (4.62), 8.086 (2.04), 8.090 (4.97), 8.094 (3.09), 8.098 (5.10), 8.102 (2.09), 8.106 (4.78), 8.111 (0.70), 8.488 (16.00), 8.503 (7.46), 8.785 (1.64), 8.796 (3.34), 8.807 (1.72), 11.261 (2.81).

### Example 134

### N-{[(4R)-4-cyclopropyl-2,5-dioxoimidazolidin-4-yl]methyl}-2-(3,5-difluoropyridin-2-yl)-2H-1,2,3-triazole-4-carboxamide

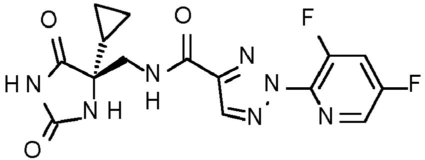

2-(3,5-difluoropyridin-2-yl)-2H-1,2,3-triazole-4-carboxylic acid (8.00 mg, 35.4 µmol) dissolved in 1 ml dichloromethane was treated with N,N-diisopropylethylamine (17 µl, 99 µmol), 1-ethyl-3-(3-dimethylaminopropyl)carbodiimide hydrochloride (8.82 mg, 46.0 µmol) and 1-hydroxybenzotriazole hydrate (7.04 mg, 46.0 µmol) and stirred for 5 min at room temperature before (5R)-5-(aminomethyl)-5-cyclopropylimidazolidine-2,4-dione hydrochloride (7.27 mg, 35.4 µmol) was added. The mixture was stirred at room temperature over night. Purification was done by preparative HPLC (Instrument: Waters Prep LC/MS System, column: Phenomenex Kinetex C18 5µm 100x30 mm; eluent A: water, eluent B: acetonitrile, eluent C: 2 % formic acid in water, eluent D: acetonitrile/water (80Vol.% / 20Vol%); flow: 80 ml/min, room temperature, UV 200-400 nm, At-Column Injektion; Gradient: eluent A 0 to 2 min 63 ml, eluent B 0 to 2min 7 ml, eluent A 2 to 10 min from 63 ml to 39 ml and eluent B from 7 ml to 31 ml, 10 to 12 min 0 ml eluent A and 70 ml eluent B. Eluent C and eluent D constant flow each 5 ml/min over the whole time). Product containing samples were united, the solvents were evaporated and the residue was lyophylized. 5.00 mg (100 % purity, 37 % yield) of the title compound were obtained.
LC-MS (Method 7): Rₜ = 0.98 min; MS (ESIpos): m/z = 378 [M+H]⁺
¹H-NMR (600 MHz, DMSO-d6) δ [ppm]: 0.005 (0.62), 0.104 (0.54), 0.111 (1.04), 0.119 (1.21), 0.123 (1.65), 0.131 (1.35), 0.137 (1.21), 0.145 (0.65), 0.310 (0.49), 0.317 (0.62), 0.323 (1.52), 0.332 (1.49), 0.335 (1.55), 0.342 (1.35), 0.346 (1.05), 0.356 (0.78), 0.409 (2.62), 0.421 (4.81), 0.433 (4.07), 0.444 (1.34), 1.135 (0.71), 1.147 (1.50), 1.157 (2.07), 1.168 (1.30), 1.180 (0.58), 2.074 (3.28), 2.424 (0.47), 2.520 (0.57), 2.522 (0.57), 2.654 (0.41), 3.695 (7.50), 3.705 (7.48), 7.603 (5.95), 8.426 (1.66), 8.430 (1.78), 8.440 (1.86), 8.444 (2.72), 8.447 (1.92), 8.457 (1.67), 8.461 (1.69), 8.532 (1.68), 8.543 (3.54), 8.553 (1.68), 8.574 (16.00), 8.635 (6.47), 8.639 (6.10), 10.653 (4.29).

### Example 135

### rac-N-({2,5-dioxo-4-[5-(trifluoromethyl)-1,3-thiazol-4-yl]imidazolidin-4-yl}methyl)-2-(4-fluorophenyl)-2H-1,2,3-triazole-4-carboxamide

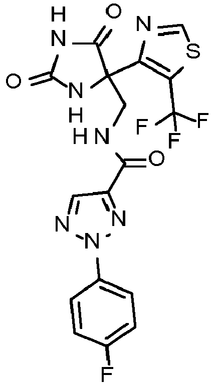

To a solution of Rac-5-(aminomethyl)-5-[5-(trifluoromethyl)-1,3-thiazol-4-yl]imidazolidine-2,4-dione hydrochloride (100 mg, 316 µmol) and 2-(4-fluorophenyl)-2H-1,2,3-triazole-4-carboxylic acid (78.5 mg, 379 µmol) in DMF (2.0 ml) was added 1-ethyl-3-(3-dimethylaminopropyl)carbodiimide hydrochloride (78.7 mg, 410 µmol), 1-hydroxybenzotriazole hydrate (62.9 mg, 410 µmol) and N,N-diisopropylethylamine (280 µl, 1.6 mmol). The reaction mixture was stirred for 2 days at RT and concentrated under reduced pressure. The crude product was purified by preparative HPLC (Method 4f). After lyophilisation, 81.0 mg (100 % purity, 55 % yield) of the desired product were obtained.
LC-MS (Method 7): Rₜ = 1.46 min; MS (ESIpos): m/z = 470 [M+H]⁺
¹H-NMR (600 MHz, DMSO-d6) δ [ppm]: 2.074 (0.92), 2.392 (1.19), 2.426 (0.78), 2.523 (0.87), 2.655 (0.78), 2.891 (0.41), 3.289 (0.64), 3.298 (1.65), 3.303 (1.70), 3.363 (0.78), 3.369 (1.05), 4.166 (2.61), 4.177 (2.84), 4.189 (3.35), 4.200 (3.03), 4.397 (3.16), 4.408 (3.44), 4.420 (2.84), 4.431 (2.61), 7.468 (5.59), 7.483 (10.27), 7.498 (5.78), 8.082 (5.87), 8.090 (6.10), 8.093 (3.81), 8.097 (6.01), 8.105 (5.64), 8.336 (8.80), 8.485 (16.00), 8.497 (2.38), 8.507 (4.77), 8.518 (2.25), 9.451 (13.57), 11.087 (5.55).

### Example 136

### ent-N-({2,5-dioxo-4-[5-(trifluoromethyl)-1,3-thiazol-4-yl]imidazolidin-4-yl}methyl)-2-(4-fluorophenyl)-2H-1,2,3-triazole-4-carboxamide

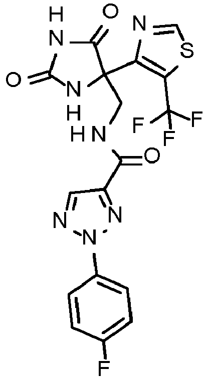

Enantiomeric separation of *rac*-N-({2,5-dioxo-4-[5-(trifluoromethyl)-1,3-thiazol-4-yl]imidazolidin-4-yl}methyl)-2-(4-fluorophenyl)-2H-1,2,3-triazole-4-carboxamide (80 mg) by preparative chiral HPLC [column: Daicel Chiralcel OD-H 5 µm, 250 x 20 mm; eluent: 70% n-heptane/30% ethanol+0.2% diethylamine; flow rate: 20 ml/min; temperature: 40°C; UV detection: 220 nm] afforded 27.86 mg of the desired product (96 % purity).
Analytical chiral HPLC: Rₜ = 0.92 min, e.e. = 99% [column: 50 x 4.6 mm filled with Phenomenex cellulose-1 3 µm; eluent: 50% n-heptan / 50% ethanol +0.2% diethylamine; flow rate: 1 ml/min; UV detection: 220 nm]
LC-MS (Method 7): Rₜ = 1.46 min; MS (ESIpos): m/z = 470 [M+H]⁺
¹H-NMR (400 MHz, DMSO-d6) δ [ppm]: -0.008 (0.91), 0.008 (1.07), 0.845 (0.42), 0.858 (0.52), 1.053 (2.18), 1.071 (4.44), 1.089 (2.38), 1.103 (0.54), 1.236 (0.50), 2.328 (0.52), 2.366 (0.67), 2.523 (2.16), 2.666 (0.50), 2.670 (0.67), 2.675 (0.57), 2.710 (1.69), 2.724 (1.13), 4.157 (1.92), 4.172 (2.14), 4.191 (2.68), 4.207 (2.42), 4.385 (2.44), 4.402 (2.72), 4.419 (2.14), 4.436 (1.90), 7.451 (0.46), 7.460 (4.98), 7.466 (1.71), 7.482 (8.13), 7.491 (0.95), 7.498 (1.78), 7.504 (5.47), 7.512 (0.57), 8.066 (0.57), 8.075 (5.12), 8.081 (2.04), 8.087 (5.39), 8.092 (3.15), 8.098 (5.37), 8.104 (1.88), 8.110 (4.96), 8.119 (0.48), 8.321 (1.92), 8.483 (16.00), 8.497 (3.55), 8.513 (1.61), 9.449 (8.68).

### Example 137

### ent-N-[(4-sec-butyl-2,5-dioxoimidazolidin-4-yl)methyl]-2-(4-fluorophenyl)-2H-1,2,3-triazole-4-carboxamide

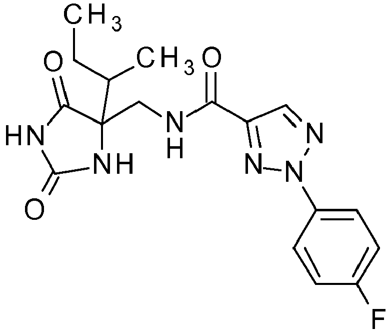

Enantiomeric separation *of diamix-* N-[(4-sec-butyl-2,5-dioxoimidazolidin-4-yl)methyl]-2-(4-fluorophenyl)-2H-1,2,3-triazole-4-carboxamide (120 mg; 321 µmol) using the following method
- Column: Daicel Chiralpak OZ-H 5 µm 250 x 20 mm
- Solvent: 70% n-heptane / 30% ethanol
- Flow: 15 ml/min
- Column Temperature: 40 °C
afforded 27 mg (100 % purity, 23 % yield) of the desired product.
LC-MS (Method 7): Rₜ = 1.56 min; MS (ESIpos): m/z = 375 [M+H]⁺
Chiral HPLC (Column: Daicel Chiralpak OZ-H 5 µm 250 x 4.6 mm; eluent: 70% n-heptane / 30% ethanole; flow: 1 ml/min; column temperature: 35 °C): Rₜ = 6.538 min, >99.0% ee
¹H-NMR (400 MHz, DMSO-d6) δ [ppm]: -0.008 (2.73), 0.008 (2.62), 0.852 (13.18), 0.869 (16.00), 0.884 (13.14), 0.902 (7.15), 0.987 (0.75), 1.004 (0.88), 1.014 (1.17), 1.019 (1.30), 1.031 (1.20), 1.046 (1.13), 1.064 (0.68), 1.618 (1.08), 1.637 (1.28), 1.651 (1.53), 1.669 (2.25), 1.685 (1.67), 1.694 (1.67), 1.701 (1.22), 1.711 (1.15), 1.718 (0.86), 3.611 (1.15), 3.627 (1.28), 3.645 (3.27), 3.661 (3.08), 3.676 (3.06), 3.691 (3.21), 3.710 (1.22), 3.725 (1.12), 7.450 (0.46), 7.459 (4.71), 7.464 (1.69), 7.481 (8.61), 7.497 (1.73), 7.503 (5.18), 7.511 (0.57), 7.754 (7.70), 8.064 (0.53), 8.072 (4.96), 8.078 (2.07), 8.084 (5.25), 8.090 (3.09), 8.095 (5.16), 8.107 (4.82), 8.116 (0.50), 8.323 (1.67), 8.339 (3.50), 8.354 (1.66), 8.465 (12.09), 10.655 (3.88).

### Example 138

### ent-N-{[2,5-dioxo-4-(pyridin-2-yl)imidazolidin-4-yl]methyl}-2-(4-fluorophenyl)-2H-1,2,3-triazole-4-carboxamide

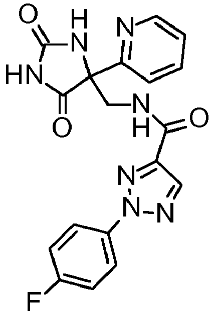

Enantiomeric separation of *rac*-N-{[2,5-dioxo-4-(pyridin-2-yl)imidazolidin-4-yl]methyl}-2-(4-fluorophenyl)-2H-1,2,3-triazole-4-carboxamide (31.7 mg) by preparative chiral HPLC [column: Daicel Chiralcel® OX-H 5 µm, 250 x 30 mm; eluent: 50% n-heptane/50% ethanol +0.2% diethylamine; flow rate: 40 ml/min; temperature: 40°C; UV detection: 220 nm] afforded 14 mg of the desired product. The product was further purified by preparative HPLC (Method 1f) The combined organic phases were concentrated in vacuo and the remaining TFA was removed, using a Stratospheres PL-HCO3 MP SPE cartridge. After lyophilization, 4.1 mg of the desired product were obtained.
Analytical chiral HPLC: Rₜ = 9.61 min, e.e. = 99% [column: Daicel Chiralcel® OX-H 5 µm, 250 x 30 mm; eluent: 50% n-heptane/50% ethanol +0.2% diethylamine; flow rate: 40 ml/min; temperature: 40°C; UV detection: 220 nm]
¹H-NMR (500 MHz, DMSO-d6) δ [ppm]: 2.095 (0.66), 2.364 (0.47), 2.637 (0.47), 3.091 (0.47), 4.082 (1.93), 4.095 (2.12), 4.110 (2.99), 4.122 (2.63), 4.214 (2.58), 4.226 (2.92), 4.241 (2.06), 4.254 (1.80), 7.398 (2.56), 7.408 (2.80), 7.412 (2.78), 7.423 (2.69), 7.461 (5.11), 7.465 (1.97), 7.479 (8.84), 7.496 (5.43), 7.545 (4.75), 7.561 (5.17), 7.859 (2.25), 7.862 (2.27), 7.874 (3.71), 7.878 (3.71), 7.889 (1.95), 7.893 (1.95), 8.072 (5.24), 8.082 (5.57), 8.086 (3.47), 8.090 (5.59), 8.100 (5.21), 8.364 (2.22), 8.475 (16.00), 8.502 (1.80), 8.515 (3.64), 8.527 (1.70), 8.633 (3.58), 8.641 (3.50), 10.912 (0.97).

### Example 139

### rac-N-[(2,5-dioxo-4-phenylimidazolidin-4-yl)methyl]-2-(4-fluorophenyl)-2H-1,2,3-triazole-4-carboxamide

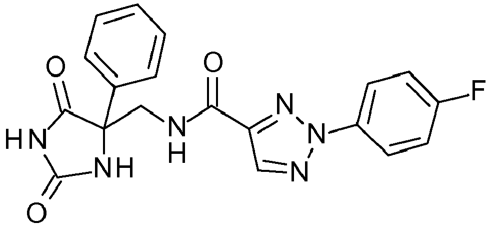

2-(4-fluorophenyl)-2H-1,2,3-triazole-4-carboxylic acid (42.9 mg, 207 µmol) dissolved in 2 ml DMF was treated with N,N-diisopropylethylamine (100 µl, 580 µmol), 1-ethyl-3-(3-dimethylaminopropyl)carbodiimide hydrochloride (51.6 mg, 269 µmol) and 1-hydroxybenzotriazole hydrate (41.2 mg, 269 µmol) and stirred for 5 min at room temperature before *rac-*5-(aminomethyl)-5-phenylimidazolidine-2,4-dione hydrochloride (50.0 mg, 207 µmol) was added. The mixture was stirred at room temperature for 2 h. Purification was done by preparative HPLC (Instrument: Waters Prep LC/MS System, column: Phenomenex Kinetex C18 5µm 100x30 mm; eluent A : water, eluent B : acetonitrile, eluent C : 2 % formic acid in water, eluent D : acetonitrile/water (80Vol.% / 20Vol%) flow: 80 ml/min, room temperature, UV 200-400 nm, At-Column Injektion. Gradient: eluent A 0 to 2 min 63 ml, eluent B 0 to 2 min 7 ml, eluent A 2 to 10 min from 63 ml to 39 ml and eluent B from 7 ml to 31 ml, 10 to 12 min 0 ml eluent A and 70 ml eluent B. Eluent C and eluent D constant flow each 5 ml/min over the whole time). Product containing samples were united, the solvents were evaporated and the residue was lyophylized. 25.0 mg (100 % purity, 31 % yield) of the title compound were obtained.
LC-MS (Method 7): Rₜ = 1.49 min; MS (ESIpos): m/z = 395 [M+H]⁺
¹H-NMR (400 MHz, DMSO-d6) δ [ppm]: -0.008 (1.46), 0.008 (1.53), 2.327 (0.97), 2.366 (1.11), 2.524 (3.61), 2.670 (0.90), 2.710 (1.11), 3.956 (6.39), 3.972 (6.39), 7.335 (1.18), 7.353 (4.06), 7.371 (3.33), 7.400 (4.96), 7.419 (7.57), 7.437 (3.30), 7.456 (4.89), 7.462 (1.67), 7.478 (7.81), 7.495 (1.70), 7.500 (5.31), 7.589 (7.08), 7.607 (6.59), 7.610 (4.30), 8.061 (5.03), 8.073 (5.31), 8.079 (3.05), 8.084 (5.24), 8.096 (4.82), 8.454 (16.00), 8.545 (1.67), 8.560 (8.68), 8.577 (1.60), 10.865 (4.34).

### Example 140

### rac-N-{[4-(1-methyl-1H-imidazol-2-yl)-2,5-dioxoimidazolidin-4-yl]methyl}-2-phenyl-2H-1,2,3-triazole-4-carboxamide

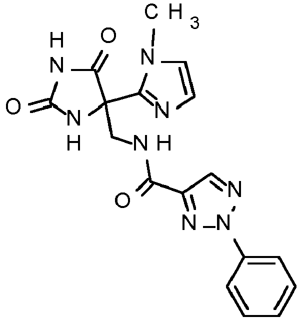

The compound was synthesized according to the General Method A using 193 mg (1.02 mmol) of 2-phenyl-2H-1,2,3-triazole-4-carboxylic acid, 250 mg (1.02 mmol) of rac-5-(aminomethyl)-5-(1-methyl-1H-imidazol-2-yl)imidazolidine-2,4-dione-hydrogen chloride, 293 mg (1.53 mmol) of 1-(3-dimethylaminopropyl)-3-ethylcarbodiimide hydrochloride, 1.50 mL (1.50 mmol) of 1M 1-hydroxy-7-azabenzotriazole in N,N-dimethylacetamide and 0.43 mL (3.10 mmol) of triethylamine in 2 mL of N,N-dimethylformamide. The crude product obtained from the workup was purified by flash column chromatography eluting with dichloromethane/methanol mixtures to give 8 mg (2% yield, 98% purity) of the compound form as a white powder.
¹H-NMR (300 MHz, DMSO-*d*₆): δ [ppm] = 3.50 (s, 3H), 4.20 (d, 2H), 6.95 (s, 1H), 7.26 (s, 1H), 7.35 (t, 1H), 7.62 (t, 2H), 8.05 (d, 2H), 8.47 (d, 2H), 8.60 (t, 1H), 11.24 (bp, 1H).
LC-MS (Method 1): Rₜ = 1.827 min. MS (Mass method 1): m/z = 381 (M + H)⁺

### Example 141

### rac-2-(4-fluorophenyl)-N-{[4-(1-methyl-1H-imidazol-2-yl)-2,5-dioxoimidazolidin-4-yl]methyl}-2H-1,2,3-triazole-4-carboxamide

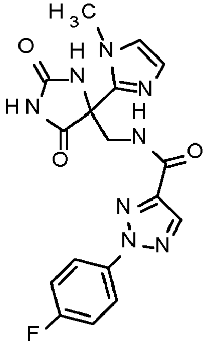

The compound was synthesized according to the General Method A using 295 mg (1.42 mmol) of 2-(4-fluorophenyl)-2H-1,2,3-triazole-4-carboxylic acid, 350 mg (1.42 mmol) of *rac*-5-(aminomethyl)-5-(1-methyl-1H-imidazol-2-yl)imidazolidine-2,4-dione-hydrogen chloride, 410 mg (2.14 mmol) of 1-(3-dimethylaminopropyl)-3-ethylcarbodiimide hydrochloride, 2.14 mL (2.14 mmol) of 1M 1-hydroxy-7-azabenzotriazole in N,N-dimethylacetamide and 0.60 mL (4.30 mmol) of triethylamine in 2 mL of N,N-dimethylformamide. The crude product obtained from the workup was purified by flash column chromatography eluting with dichloromethane/methanol mixtures to give 11 mg (2% yield, 97% purity) of the compound form as a white powder.
¹H-NMR (300 MHz, DMSO-*d*₆): δ [ppm] = 3.50 (s, 3H), 4.19 (d, 2H), 6.94 (s, 1H), 7.25 (s, 1H), 7.48 (t, 2H), 8.04-8.12 (m, 2H), 8.44 (s, 1H), 8.48 (s, 1H), 8.59 (t, 1H), 11.23 (s, 1H).
LC-MS (Method 1): Rₜ = 1.939 min. MS (Mass method 1): m/z = 399 (M + H)⁺

### Example 142

### ent-N-{[4-(1-methyl-1H-imidazol-2-yl)-2,5-dioxoimidazolidin-4-yl]methyl}-2-phenyl-2H-1,2,3-triazole-4-carboxamide

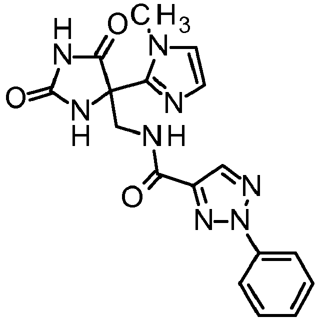

Enantiomeric separation of *rac*-N-{[4-(1-methyl-1H-imidazol-2-yl)-2,5-dioxoimidazolidin-4-yl]methyl}-2-phenyl-2H-1,2,3-triazole-4-carboxamide (6.6 mg) by preparative chiral HPLC [column: Daicel Chiralpak IF 5 µm, 250 x 20 mm; eluent: 60% n-heptane/40% isopropanol; flow rate: 20 ml/min; temperature: 45°C; UV detection: 220 nm] afforded 1.44 mg of the desired product.
Analytical chiral HPLC: Rₜ = 16.06 min, e.e. = > 99% [column: 250 x 4.6 mm filled with Daicel Chiralpak IF 5 µm; eluent: 60% n-heptane/40% isopropanol; flow rate: 1 ml/min; temperature: 45°C; UV detection: 220 nm]
¹H-NMR (500 MHz, DMSO-d6) δ [ppm]: 0.006 (0.75), 1.174 (0.45), 1.235 (0.80), 2.695 (0.87), 3.303 (0.50), 3.506 (16.00), 4.188 (3.52), 4.201 (3.45), 6.956 (4.18), 6.958 (4.27), 7.260 (3.88), 7.262 (3.94), 7.494 (0.85), 7.509 (2.04), 7.524 (1.33), 7.614 (2.49), 7.631 (3.55), 7.646 (2.02), 8.048 (3.58), 8.063 (3.47), 8.471 (3.71), 8.491 (7.49), 8.598 (0.83), 8.611 (1.76), 8.624 (0.81).

### Example 143

### rac-N-{[4-(1-methyl-1H-imidazol-2-yl)-2,5-dioxoimidazolidin-4-yl]methyl}-2-(3,4,5-trifluorophenyl)-2H-1,2,3-triazole-4-carboxamide

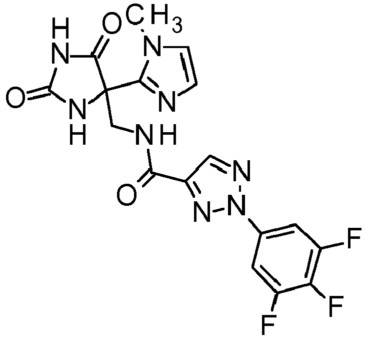

To a solution of *rac*-5-(aminomethyl)-5-(1-methyl-1H-imidazol-2-yl)imidazolidine-2,4-dione hydrochloride (80.0 mg) and 2-(3,4,5-trifluorophenyl)-2H-1,2,3-triazole-4-carboxylic acid (79.2 mg, 326 µmol) in dichloromethane (9 ml) was added 1-ethyl-3-(3-dimethylaminopropyl)carbodiimide hydrochloride (81.2 mg, 423 µmol), 1-hydroxybenzotriazole hydrate (64.8 mg, 423 µmol) and N,N-diisopropylethylamine (160 µl, 910 µmol). The reaction mixture was stirred overnight at RT and concentrated under reduced pressure. The crude product was purified by preparative HPLC (method 3f). After lyophilization, 10.5 mg (7.4 % yield) of the desired product were obtained.
LC-MS (Method 8): Rₜ = 0.69 min; MS (ESIpos): m/z = 435 [M+H]⁺
¹H-NMR (500 MHz, DMSO-d6) δ [ppm]: -0.120 (0.55), -0.007 (5.27), 0.006 (3.72), 0.117 (0.52), 3.505 (16.00), 4.141 (0.78), 4.155 (0.87), 4.169 (1.40), 4.182 (1.22), 4.228 (1.27), 4.241 (1.40), 4.256 (0.87), 4.269 (0.78), 6.938 (4.09), 6.941 (4.15), 7.255 (3.81), 7.257 (3.83), 8.006 (1.50), 8.018 (1.80), 8.022 (1.85), 8.035 (1.64), 8.444 (3.61), 8.572 (7.45), 8.684 (0.78), 8.697 (1.65), 8.710 (0.80), 11.241 (0.86).

### Example 144

### ent-2-(4-fluorophenyl)-N-{[4-(1-methyl-1H-imidazol-2-yl)-2,5-dioxoimidazolidin-4-yl]methyl}-2H-1,2,3-triazole-4-carboxamide

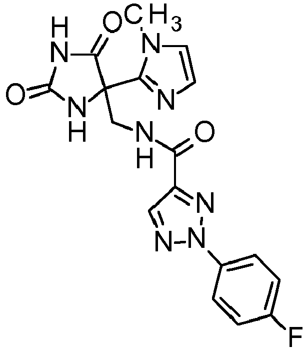

To a solution of *ent*-5-(aminomethyl)-5-(1-methyl-1H-imidazol-2-yl)imidazolidine-2,4-dione hydrochloride (1:1) (35.0 mg, 142 µmol) and 2-(4-fluorophenyl)-2H-1,2,3-triazole-4-carboxylic acid (29.5 mg, 142 µmol) in DMF (890 µl) was added 1-ethyl-3-(3-dimethylaminopropyl)carbodiimide hydrochloride (35.5 mg, 185 µmol), 1-hydroxybenzotriazole hydrate (28.4 mg, 185 µmol) and N,N-diisopropylethylamine (120 µl, 710 µmol). The reaction mixture was stirred overnight at RT and concentrated under reduced pressure. The crude product was purified by preparative HPLC (Methode 5f). After lyophilisation, 24.3 mg (42 % yield, 98 % purity) of the desired product were obtained.
LC-MS (Method 7): Rₜ = 1.08 min; MS (ESIpos): m/z = 399 [M+H]⁺
¹H-NMR (500 MHz, DMSO-d6) δ [ppm]: 3.505 (16.00), 4.186 (3.47), 4.199 (3.59), 6.948 (4.22), 6.950 (4.20), 7.257 (4.15), 7.468 (2.28), 7.472 (0.86), 7.486 (4.05), 7.503 (2.35), 8.068 (2.43), 8.073 (1.19), 8.078 (2.56), 8.082 (1.63), 8.086 (2.48), 8.096 (2.25), 8.460 (3.90), 8.490 (6.76), 8.599 (0.91), 8.612 (1.85), 8.625 (0.86), 11.246 (0.48).

### Example 145

### ent-2-(4-chloro-3-fluorophenyl)-N-{[4-(1-methyl-1H-imidazol-2-yl)-2,5-dioxoimidazolidin-4-yl]methyl}-2H-1,2,3-triazole-4-carboxamide

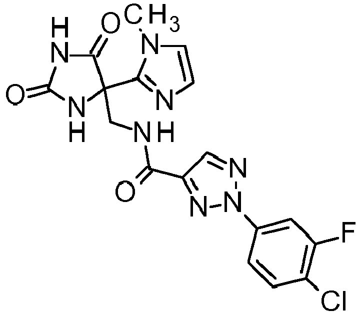

To a solution of *ent*-5-(aminomethyl)-5-(1-methyl-1H-imidazol-2-yl)imidazolidine-2,4-dione hydrochloride (70.0 mg, 285 µmol) and 2-(4-chloro-3-fluorophenyl)-2H-1,2,3-triazole-4-carboxylic acid (68.8 mg, 285 µmol) in DMF (1.75 ml) was added 1-ethyl-3-(3-dimethylaminopropyl)carbodiimide hydrochloride (71.0 mg, 370 µmol), 1-hydroxybenzotriazole hydrate (56.7 mg, 370 µmol) and N,N-diisopropylethylamine (800 µmol). The reaction mixture was stirred overnight at RT and concentrated under reduced pressure. The crude product was purified by preparative HPLC (method 3f). After lyophilisation, 64.2 mg (98 % purity, 51 % yield) of the desired product were obtained.
LC-MS (Method 8): Rₜ = 0.76 min; MS (ESIpos): m/z = 433 [M+H]⁺
¹H-NMR (400 MHz, DMSO-d6) δ [ppm]: -0.008 (0.72), 0.008 (0.74), 2.524 (0.41), 3.508 (16.00), 4.149 (0.47), 4.166 (0.52), 4.183 (1.69), 4.203 (2.10), 4.219 (1.71), 4.238 (0.54), 4.254 (0.46), 6.941 (3.81), 6.944 (4.01), 7.253 (3.81), 7.255 (3.97), 7.856 (0.95), 7.878 (1.99), 7.897 (1.97), 7.919 (1.88), 7.925 (1.77), 7.941 (0.82), 7.947 (0.88), 8.060 (1.42), 8.066 (1.32), 8.085 (1.40), 8.091 (1.31), 8.436 (3.97), 8.547 (5.64), 8.653 (0.79), 8.670 (1.68), 8.686 (0.80), 11.231 (0.62).

### Example 146

### ent-N-{[4-(1-methyl-1H-imidazol-2-yl)-2,5-dioxoimidazolidin-4-yl]methyl}-2-(3,4,5-trifluorophenyl)-2H-1,2,3-triazole-4-carboxamide

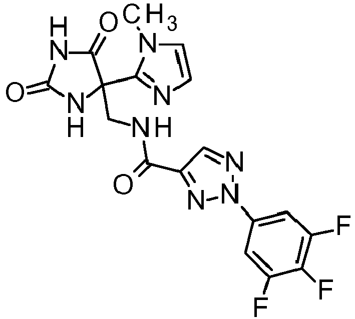

To a solution of *ent*-5-(aminomethyl)-5-(1-methyl-1H-imidazol-2-yl)imidazolidine-2,4-dione hydrochloride (1:1) (80.0 mg, 326 µmol) and 2-(3,4,5-trifluorophenyl)-2H-1,2,3-triazole-4-carboxylic acid (79.2 mg, 326 µmol) in DMF (2 ml) was added 1-ethyl-3-(3-dimethylaminopropyl)carbodiimide hydrochloride (81.2 mg, 423 µmol), 1-hydroxybenzotriazole hydrate (64.8 mg, 423 µmol) and N,N-diisopropylethylamine (912 µmol). The reaction mixture was stirred overnight at RT and concentrated under reduced pressure. The crude product was purified by preparative HPLC (method 3f). After lyophilisation, 74.8 mg (99 % purity, 52 % yield) of the desired product were obtained.
LC-MS (Method 8): Rₜ = 0.75 min; MS (ESIpos): m/z = 435 [M+H]⁺
¹H-NMR (400 MHz, DMSO-d6) δ [ppm]: -0.008 (1.24), 0.008 (1.26), 2.523 (0.58), 3.507 (16.00), 4.139 (0.67), 4.156 (0.75), 4.173 (1.51), 4.190 (1.35), 4.222 (1.37), 4.238 (1.51), 4.256 (0.74), 4.272 (0.67), 6.937 (3.81), 6.939 (3.97), 7.250 (3.83), 7.252 (3.91), 7.996 (1.50), 8.011 (1.78), 8.017 (1.82), 8.033 (1.65), 8.427 (3.74), 8.564 (5.66), 8.661 (0.79), 8.677 (1.69), 8.693 (0.78), 11.223 (0.93).

### Example 147

### ent-2-(3,4-difluorophenyl)-N-{[4-(1-methyl-1H-imidazol-2-yl)-2,5-dioxoimidazolidin-4-yl]methyl}-2H-1,2,3-triazole-4-carboxamide

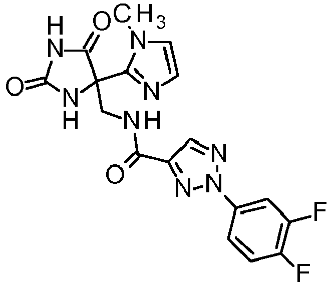

To a solution of *ent*-5-(aminomethyl)-5-(1-methyl-1H-imidazol-2-yl)imidazolidine-2,4-dione hydrochloride (1:1) (80.0 mg, 326 µmol) and 2-(3,4-difluorophenyl)-2H-1,2,3-triazole-4-carboxylic acid (73.3 mg, 326 µmol) in DMF (2 ml) was added 1-ethyl-3-(3-dimethylaminopropyl)carbodiimide hydrochloride (81.2 mg, 423 µmol), 1-hydroxybenzotriazole hydrate (64.8 mg, 423 µmol) and N,N-diisopropylethylamine (160 µl, 910 µmol). The reaction mixture was stirred overnight at RT and concentrated under reduced pressure. The crude product was purified by preparative HPLC (method 3f). After lyophilisation, 79.1 mg (99 % purity, 58 % yield) of the desired product were obtained.
LC-MS (Method 8): Rₜ = 0.70 min; MS (ESIpos): m/z = 417 [M+H]⁺
¹H-NMR (400 MHz, DMSO-d6) δ [ppm]: -0.008 (0.91), 0.008 (0.86), 3.507 (16.00), 4.148 (0.42), 4.165 (0.48), 4.182 (1.72), 4.200 (2.92), 4.216 (1.76), 4.235 (0.50), 4.251 (0.42), 6.941 (3.95), 6.944 (4.06), 7.252 (3.95), 7.254 (4.03), 7.699 (0.57), 7.722 (1.25), 7.747 (1.28), 7.769 (0.73), 7.901 (1.00), 7.911 (0.67), 7.923 (0.80), 8.084 (0.64), 8.090 (0.63), 8.101 (0.73), 8.108 (0.79), 8.111 (0.82), 8.118 (0.68), 8.129 (0.69), 8.136 (0.64), 8.436 (3.80), 8.523 (6.12), 8.624 (0.82), 8.641 (1.69), 8.656 (0.80), 11.229 (1.08).

### Example 148

### ent-N-{[2,5-dioxo-4-(1,3-thiazol-4-yl)imidazolidin-4-yl]methyl}-2-(4-fluorophenyl)-2H-1,2,3-triazole-4-carboxamide

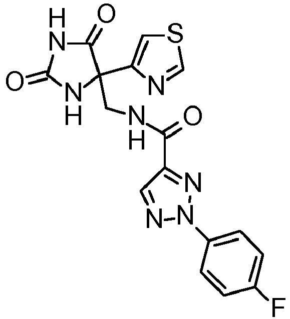

To a solution of rac-5-(aminomethyl)-5-(1,3-thiazol-4-yl)imidazolidine-2,4-dione hydrochloride (67.0 mg, 269 µmol) and 2-(4-fluorophenyl)-2H-1,2,3-triazole-4-carboxylic acid (55.8 mg, 269 µmol) in DMF (1.7 ml) was added 1-ethyl-3-(3-dimethylaminopropyl)carbodiimide hydrochloride (67.1 mg, 350 µmol), 1-hydroxybenzotriazole hydrate (53.6 mg, 350 µmol) and N,N-diisopropylethylamine (230 µl, 1.3 mmol). The reaction mixture was stirred for 3 days at RT and concentrated under reduced pressure. The crude product was purified by preparative HPLC (method 3f). A second purification by preparative chiral HPLC [amount: 60 mg; column: Daicel Chiralpack IF 5 µm 250^{∗}20 mm; eluent: ethanol; flow rate: 15 ml/min; temperature: 60 °C; UV detection: 220 nm] gave 30.0 mg (96 % purity, 27 % yield) of the desired product.
Analytical chiral HPLC: Rₜ = 8.16 min, e.e. = >99% [column: 250 x 4.6 mm filled with Daicel Chiralpack IF 5 µm; eluent: ethanol; flow rate: 1 ml/min; temperature: 60°C; UV detection: 220 nm]
LC-MS (Method 7): Rₜ = 1.23 min; MS (ESIpos): m/z = 402 [M+H]⁺
¹H-NMR (400 MHz, DMSO-d6) δ [ppm]: -0.150 (0.46), -0.008 (3.93), 0.008 (4.16), 0.146 (0.45), 1.235 (0.71), 2.323 (0.41), 2.328 (0.57), 2.332 (0.41), 2.366 (0.45), 2.523 (2.54), 2.665 (0.49), 2.670 (0.68), 2.674 (0.50), 2.710 (0.52), 4.049 (1.33), 4.065 (1.47), 4.083 (3.57), 4.099 (3.30), 4.117 (3.31), 4.132 (3.68), 4.151 (1.53), 4.167 (1.33), 7.447 (0.48), 7.456 (5.35), 7.462 (1.87), 7.479 (8.98), 7.488 (1.09), 7.495 (1.93), 7.501 (5.92), 7.509 (0.68), 7.850 (9.41), 7.855 (9.52), 8.066 (0.56), 8.075 (5.47), 8.081 (2.21), 8.087 (5.92), 8.092 (3.45), 8.098 (5.81), 8.104 (2.11), 8.110 (5.42), 8.119 (0.62), 8.380 (9.13), 8.483 (16.00), 8.553 (1.82), 8.569 (3.97), 8.585 (1.83), 9.149 (6.57), 9.154 (6.78), 10.884 (2.06).

### Example 149

### diamix-N-{[4-(2,2-dimethylcyclopropyl)-2,5-dioxoimidazolidin-4-yl]methyl}-2-phenyl-2H-1,2,3-triazole-4-carboxamide

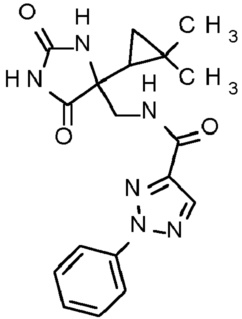

381 mg (1.99 mmol) of 1-(3-dimethylaminopropyl)-3-ethylcarbodiimide hydrochloride were added to a solution of 310 mg (1.33 mmol) of 5-(aminomethyl)-5-(2,2-dimethylcyclopropyl)imidazolidine-2,4-dione hydrochloride, 251 mg (1.33 mmol) of 2-phenyl-2H-1,2,3-triazole-4-carboxylic acid, 2.0 mL (2.00 mmol) of a 1N solution of 1-hydroxy-7-azabenzotriazole in N,N-dimethylformamide and 0.55 mL (4.00 mmol) of triethylamine in 5 mL of N,N-dimethylformamide and the reaction was allowed to stir at room temperature for 16 hours. The solvent was evaporated and the residue was dissolved in dichloromethane, washed with saturated sodium bicarbonate solution (aq.) and 2N hydrocloric acid (aq.). The organic extract was separated, dried over magnesium sulfate, filtered and concentrated to afford a residue, which was purified by flash column chromatography on silica gel eluting with dichloromethane/methanol mixtures to give 230 mg (47%, 85% purity) of the product as an off-white sticky solid.
¹H-NMR (300 MHz, DMSO-*d*₆): δ [ppm] = 0.35-0.51 (m, 2H), 0.84-1.12 (m, 7H), 3.53-3.81 (m, 2H), 7.53 (t, 1H), 7.56-7.65 (m, 3H), 8.07 (d, 2H), 8.43-8.55 (m, 2H), 10.76 (bp, 1H).
LC-MS (Method 1): Rₜ = 2.911 min. MS (Mass method 1): m/z = 369 (M + H)⁺

### Example 150

### ent-N-({4-[rac-2,2-dimethylcyclopropyl]-2,5-dioxoimidazolidin-4-yl}methyl)-2-phenyl-2H-1,2,3-triazole-4-carboxamide

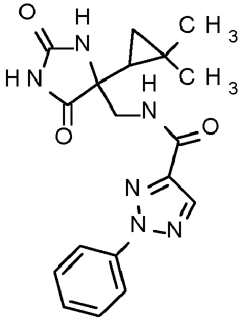

Enantiomeric separation of *diamix*-N-{[4-(2,2-dimethylcyclopropyl)-2,5-dioxoimidazolidin-4-yl]methyl}-2-phenyl-2H-1,2,3-triazole-4-carboxamide (228.6 mg) by preparative chiral HPLC [column: AD-H 5u 250x20 mmO; flow rate: 70 ml/min; temperature: 40°C; UV detection: 210 nm] afforded 7.30 mg of the desired product.
Analytical chiral HPLC: Rₜ = 3.809 min, e.e. = > 99% [column: Daicel AD; eluent: CO₂ :85% Isopropanol 15%; flow rate: 3 ml/min; UV detection: 210 nm]
¹H-NMR (400 MHz, DMSO-d6) δ [ppm]: -0.008 (1.69), 0.008 (1.33), 0.449 (1.10), 0.461 (1.46), 0.472 (1.35), 0.483 (1.38), 0.593 (1.27), 0.606 (1.85), 0.619 (1.24), 0.867 (1.49), 0.882 (1.60), 0.889 (1.61), 0.900 (1.88), 0.974 (15.40), 1.011 (16.00), 2.524 (0.57), 3.567 (1.00), 3.582
(1.14), 3.601 (1.57), 3.616 (1.41), 3.728 (1.46), 3.745 (1.57), 3.761 (1.10), 3.779 (1.03), 7.488 (0.98), 7.506 (2.55), 7.525 (1.77), 7.608 (3.16), 7.629 (4.49), 7.643 (1.08), 7.648 (2.44), 7.725 (3.35), 8.062 (3.94), 8.064 (4.23), 8.083 (4.24), 8.086 (3.15), 8.469 (7.65), 8.477 (0.44), 8.494 (0.94), 8.510 (1.78), 8.526 (0.92), 10.633 (2.68).
LC-MS (Method 7): Rₜ = 0.82 min; MS (ESIpos): m/z = 369 [M+H]⁺

### Example 151

### 2-(2-chloro-4-fluorophenyl)-N-{[(4R)-4-cyclopropyl-2,5-dioxoimidazolidin-4-yl]methyl}-2H-1,2,3-triazole-4-carboxamide

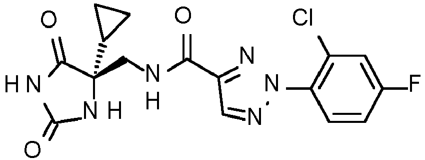

2-(2-chloro-4-fluorophenyl)-2H-1,2,3-triazole-4-carboxylic acid (20.7 mg, 96 % purity, 82.4 µmol) dissolved in 2 ml of DMF was treated with N-ethyl-N-isopropylpropan-2-amine (40 µl, 230 µmol), 3-{[(ethylimino)methylene]amino}-N,N-dimethylpropan-1-amine hydrochloride (20.5 mg, 107 µmol), 1H-benzotriazol-1-ol hydrate (16.4 mg, 107 µmol) and (5R)-5-(aminomethyl)-5-cyclopropylimidazolidine-2,4-dione hydrochloride (16.9 mg, 82.4 µmol). The mixture was stirred over night at room temperature. The product was purified by preparative HPLC (Instrument: Waters Prep LC/MS System, Column: Phenomenex Kinetex C18 5µm 100x30 mm; eluent A: water, eluent B: acetonitrile, eluent C: 2 % formic acid in water, eluent D: acetonitrile/water (80Vol.% / 20Vol.%); flow: 80 ml/min , room temperature, UV-detection: 200-400 nm, At-Column Injektion; gradient: eluent A 0 bis 2 min 63 ml, eluent B 0 to 2 min 7 ml, eluent A 2 to 10 min from 63 ml to 39 ml and eluent B from 7 ml to 31 ml, 10 to 12 min 0 ml eluent A and 70 ml eluent B. Eluent C and eluent D constant flow 5 ml/min over the whole runtime). After lyophilization, 12.8 mg (100 % purity, 40 % yield) of the title compound were obtained.
LC-MS (Method 7): Rₜ = 1.34 min; MS (ESIpos): m/z = 393 [M+H]⁺
¹H-NMR (400 MHz, DMSO-d6) δ [ppm]: -0.008 (0.44), 0.008 (0.48), 0.094 (0.46), 0.105 (0.90), 0.113 (1.26), 0.127 (1.45), 0.137 (1.42), 0.144 (1.15), 0.155 (0.64), 0.316 (1.31), 0.328 (1.29), 0.334 (1.52), 0.343 (1.22), 0.354 (1.08), 0.365 (0.83), 0.399 (2.36), 0.416 (4.73), 0.435 (3.67), 0.452 (1.15), 1.115 (0.64), 1.133 (1.38), 1.149 (1.93), 1.165 (1.35), 1.183 (0.53), 2.328 (0.53), 2.670 (0.53), 3.660 (0.51), 3.679 (4.38), 3.683 (4.22), 3.695 (4.18), 3.699 (4.36), 3.718 (0.48), 7.483 (1.77), 7.490 (1.91), 7.505 (2.48), 7.512 (2.69), 7.525 (1.97), 7.532 (2.11), 7.580 (6.54), 7.824 (3.26), 7.831 (3.33), 7.846 (3.35), 7.853 (3.35), 7.865 (3.58), 7.879 (3.70), 7.887 (3.44), 7.901 (3.24), 8.434 (1.49), 8.450 (3.19), 8.466 (1.49), 8.506 (16.00), 10.640 (4.59).

### Example 152

### rac-2-(4-fluorophenyl)-N-[(4-methyl-2,5-dioxoimidazolidin-4-yl)methyl]-2H-1,2,3-triazole-4-carboxamide

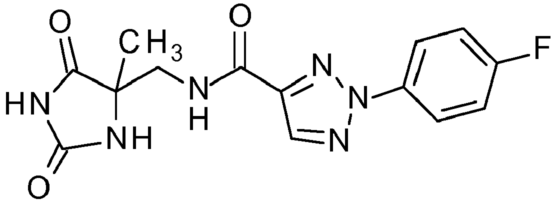

2-(4-fluorophenyl)-2H-1,2,3-triazole-4-carboxylic acid (115 mg, 557 µmol) dissolved in 5 ml of DMF was treated with N-ethyl-N-isopropylpropan-2-amine (270 µl, 1.6 mmol), 3-{[(ethylimino)methylene]amino}-N,N-dimethylpropan-1-amine hydrochloride (139 mg, 724 µmol), 1H-benzotriazol-1-ol hydrate (111 mg, 724 µmol) and rac-5-(aminomethyl)-5-methylimidazolidine-2,4-dione hydrochloride (100 mg, 557 µmol). The mixture was stirred over night at room temperature. The product was purified by preparative HPLC (Instrument: Waters Prep LC/MS System, Column: Phenomenex Kinetex C18 5µm 100x30 mm; eluent A: water, eluent B: acetonitrile, eluent C: 2 % formic acid in water, eluent D: acetonitrile/water (80Vol.% / 20Vol.%); flow: 80 ml/min , room temperature, UV-detection: 200-400 nm, At-Column Injektion; gradient: eluent A 0 bis 2 min 63 ml, eluent B 0 to 2 min 7 ml, eluent A 2 to 10 min from 63 ml to 39 ml and eluent B from 7 ml to 31 ml, 10 to 12 min 0 ml eluent A and 70 ml eluent B. Eluent C and eluent D constant flow 5 ml/min over the whole runtime). After lyophilization, 86.0 mg (100 % purity, 46 % yield) and 71.0 mg (98 % purity, 37 % yield) of the title compound were obtained.
LC-MS (Method 7): Rₜ = 1.15 min; MS (ESIpos): m/z = 333 [M+H]⁺
¹H-NMR (400 MHz, DMSO-d6) δ [ppm]: 0.008 (0.61), 1.292 (16.00), 2.074 (0.56), 3.479 (0.70), 3.495 (0.79), 3.513 (1.89), 3.529 (1.73), 3.549 (1.77), 3.565 (1.89), 3.582 (0.78), 3.599 (0.74), 7.460 (2.54), 7.466 (0.90), 7.482 (4.65), 7.499 (0.94), 7.504 (2.73), 7.868 (2.92), 8.087 (2.75), 8.099 (2.91), 8.105 (1.71), 8.110 (2.80), 8.117 (1.08), 8.122 (2.64), 8.477 (7.49), 8.536 (0.86), 8.552 (1.80), 8.568 (0.86), 10.672 (2.34).

### Example 153

### ent-2-(4-fluorophenyl)-N-[(4-methyl-2,5-dioxoimidazolidin-4-yl)methyl]-2H-1,2,3-triazole-4-carboxamide

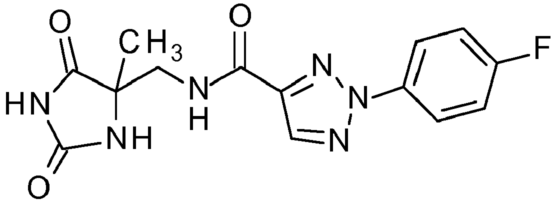

Enantiomeric separation of *rac*-2-(4-fluorophenyl)-N-[(4-methyl-2,5-dioxoimidazolidin-4-yl)methyl]-2H-1,2,3-triazole-4-carboxamide (157 mg, 472 µmol) was done using the following method.
- Diacel Chiralcel AD SFC 250 x 20 mm
- Eluent A: 70% CO₂, eluent B: 30% methanol
- Flow: 80 ml/min
- UV-detection: 210 nm
- Temperature: 40°C
After lyophilisation 34.0 mg (100 % purity, 22 % yield) of the title compound were obtained.
Chiral-HPLC: (Column: SFC; Diacel AD, eluent A: 70% CO₂, eluent B: 30% methanol, flow: 3 ml/min, UV-detection: 210 nm): Rt= 1.781 min; 100 % ee
LC-MS (Method 7): Rₜ = 1.17 min; MS (ESIpos): m/z = 333 [M+H]⁺
¹H-NMR (600 MHz, DMSO-d6) δ [ppm]: 1.294 (16.00), 2.071 (0.46), 3.167 (0.94), 3.175 (0.98), 3.494 (0.85), 3.505 (0.93), 3.517 (1.77), 3.527 (1.61), 3.555 (1.69), 3.566 (1.80), 3.578 (0.94), 3.589 (0.87), 7.463 (2.54), 7.467 (0.90), 7.478 (4.22), 7.489 (0.88), 7.492 (2.63), 7.847 (3.40), 8.091 (2.62), 8.095 (1.08), 8.099 (2.72), 8.103 (1.59), 8.106 (2.72), 8.111 (0.98), 8.114 (2.52), 8.469 (8.20), 8.514 (0.84), 8.525 (1.66), 8.535 (0.80), 10.654 (2.08).

### Example 154

### rac-N-{[4-(1-fluorocyclopropyl)-2,5-dioxoimidazolidin-4-yl]methyl}-2-(4-fluorophenyl)-2H-1,2,3-triazole-4-carboxamide

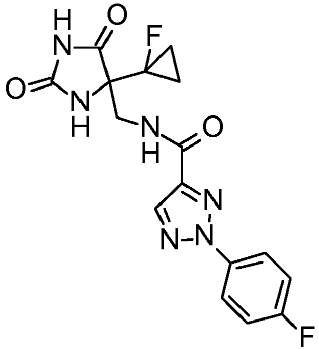

To a solution of rac-5-(aminomethyl)-5-(1-fluorocyclopropyl)imidazolidine-2,4-dione hydrochloride (100 mg, 447 µmol) and 2-(4-fluorophenyl)-2H-1,2,3-triazole-4-carboxylic acid (92.6 mg, 447 µmol) in DMF (2.2 ml) was added 1-ethyl-3-(3-dimethylaminopropyl)carbodiimide hydrochloride (111 mg, 581 µmol), 1-hydroxybenzotriazole hydrate (89.0 mg, 581 µmol) and N,N-diisopropylethylamine (390 µl, 2.2 mmol). The reaction mixture was stirred overnight at RT, after which extra portion of 2-(4-fluorophenyl)-2H-1,2,3-triazole-4-carboxylic acid (8.57 mg, 44.7 µmol) and N,N-diisopropylethylamine (39 µl, 220 µmol) were added due to incomplete conversion. After stirring for 2 h at 50°C, the reaction mixture was concentrated under reduced pressure and the crude product was purified by preparative HPLC (Method 3f). After lyophilization, 26.6 mg (95 % purity, 15 % yield) of the desired product were obtained.
LC-MS (Method 8): Rₜ = 0.78 min; MS (ESIpos): m/z = 377 [M+H]⁺
¹H-NMR (400 MHz, DMSO-d6) δ [ppm]: -0.149 (0.92), -0.024 (0.44), -0.021 (0.52), -0.019 (0.60), -0.016 (0.84), -0.014 (1.12), -0.011 (1.76), -0.008 (7.28), -0.007 (4.84), 0.006 (5.52), 0.008 (8.04), 0.011 (2.36), 0.013 (1.52), 0.016 (1.16), 0.018 (0.88), 0.021 (0.64), 0.023 (0.52), 0.025 (0.52), 0.027 (0.44), 0.146 (0.88), 0.866 (0.52), 0.889 (1.96), 0.920 (3.16), 0.946 (2.52), 0.980 (0.76), 0.989 (0.84), 1.024 (3.68), 1.030 (2.28), 1.074 (3.72), 1.080 (2.68), 1.194 (0.40), 1.217 (0.60), 1.235 (0.76), 2.073 (0.88), 2.366 (1.28), 2.391 (0.40), 2.517 (5.40), 2.521 (4.08), 2.522 (4.12), 2.524 (3.72), 2.557 (1.96), 2.560 (1.56), 2.562 (1.24), 2.565 (1.08), 2.567 (0.92), 2.569 (0.72), 2.572 (0.64), 2.574 (0.52), 2.577 (0.44), 2.710 (1.28), 3.716 (0.84), 3.732 (0.96), 3.750 (3.20), 3.767 (4.48), 3.785 (3.00), 3.804 (0.84), 3.820 (0.76), 7.452 (0.92), 7.461 (5.56), 7.466 (2.24), 7.483 (8.64), 7.493 (1.16), 7.499 (1.96), 7.505 (5.92), 7.514 (0.72), 8.068 (0.76), 8.077 (5.60), 8.083 (2.28), 8.089 (5.92), 8.094 (3.44), 8.100 (5.84), 8.106 (2.12), 8.112 (5.44), 8.121 (0.76), 8.144 (5.52), 8.474 (16.00), 8.533 (1.56), 8.549 (3.32), 8.565 (1.60), 10.865 (3.96).

### Example 155

### rac-N-{[4-(3,3-difluorocyclobutyl)-2,5-dioxoimidazolidin-4-yl]methyl}-2-(4-fluorophenyl)-2H-1,2,3-triazole-4-carboxamide

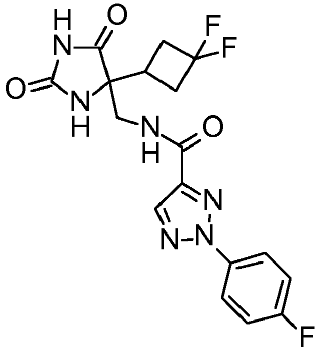

To a solution of rac-5-(aminomethyl)-5-(3,3-difluorocyclobutyl)imidazolidine-2,4-dione hydrochloride (100 mg, 391 µmol) and 2-(4-fluorophenyl)-2H-1,2,3-triazole-4-carboxylic acid (81.0 mg, 391 µmol) in DMF (1.9 ml) was added 1-ethyl-3-(3-dimethylaminopropyl)carbodiimide hydrochloride (97.5 mg, 509 µmol), 1-hydroxybenzotriazole hydrate (77.9 mg, 509 µmol) and N,N-diisopropylethylamine (340 µl, 2.0 mmol). The reaction mixture was stirred overnight at RT and concentrated under reduced pressure. The crude product was purified by preparative HPLC (Method 3f). After lyophilisation, 32.1 mg (95 % purity, 19 % yield) of the desired product were obtained.
LC-MS (Method 7): Rₜ = 1.44 min; MS (ESIneg): m/z = 407 [M-H]⁻
¹H-NMR (600 MHz, DMSO-d6) δ [ppm]: 2.327 (0.67), 2.341 (1.02), 2.361 (1.00), 2.374 (0.81), 2.474 (1.36), 2.568 (2.42), 2.581 (3.08), 2.595 (2.10), 2.613 (1.49), 2.623 (1.32), 2.639 (1.16), 2.649 (1.09), 3.266 (0.59), 3.568 (9.46), 3.579 (9.50), 7.468 (5.58), 7.483 (9.89), 7.497 (5.63), 8.086 (5.88), 8.094 (5.89), 8.097 (3.61), 8.101 (5.86), 8.109 (5.68), 8.121 (8.86), 8.469 (16.00), 8.549 (2.09), 8.560 (4.32), 8.571 (2.03), 10.856 (2.74).

### Example 156

### rac-N-({2,5-dioxo-4-[3-(trifluoromethyl)pyridin-2-yl]imidazolidin-4-yl}methyl)-2-(4-fluorophenyl)-2H-1,2,3-triazole-4-carboxamide

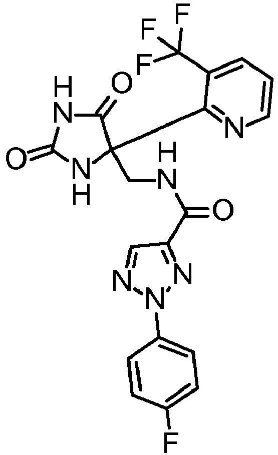

To a solution of rac-5-(aminomethyl)-5-[3-(trifluoromethyl)pyridin-2-yl]imidazolidine-2,4-dione hydrochloride (100 mg, 322 µmol) and 2-(4-fluorophenyl)-2H-1,2,3-triazole-4-carboxylic acid (75.6 mg, 365 µmol) in DMF (1.8 ml) was added 1-ethyl-3-(3-dimethylaminopropyl)carbodiimide hydrochloride (90.9 mg, 474 µmol), 1-hydroxybenzotriazole hydrate (72.6 mg, 474 µmol) and N,N-diisopropylethylamine (320 µl, 1.8 mmol). The reaction mixture was stirred overnight at RT and concentrated under reduced pressure. The crude product was purified by preparative HPLC (Method 3f). After lyophilisation, 50.3 mg (98 % purity, 33 % yield) of the desired product were obtained.
LC-MS (Method 7): Rₜ = 1.48 min; MS (ESIpos): m/z = 464 [M+H]⁺
¹H-NMR (600 MHz, DMSO-d6) δ [ppm]: 2.572 (0.61), 3.329 (0.74), 3.916 (0.48), 4.163 (2.80), 4.173 (3.03), 4.185 (3.43), 4.196 (3.18), 4.446 (3.22), 4.457 (3.50), 4.469 (3.01), 4.480 (2.77), 7.459 (5.69), 7.474 (10.56), 7.488 (5.85), 7.721 (3.00), 7.730 (3.17), 7.735 (3.15), 7.743 (3.07), 8.080 (5.97), 8.088 (6.24), 8.091 (3.94), 8.095 (6.19), 8.103 (5.83), 8.215 (8.90), 8.322 (6.69), 8.334 (9.20), 8.344 (2.38), 8.470 (16.00), 8.975 (4.62), 8.982 (4.56), 11.007 (6.17).

### Example 157

### rac-N-{[4-(5-cyclopropyl-1,3-thiazol-4-yl)-2,5-dioxoimidazolidin-4-yl]methyl}-2-(4-fluorophenyl)-2H-1,2,3-triazole-4-carboxamide

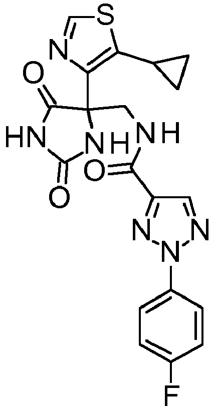

To a solution of rac-5-(aminomethyl)-5-(5-cyclopropyl-1,3-thiazol-4-yl)imidazolidine-2,4-dione hydrochloride (110 mg, 381 µmol) and 2-(4-fluorophenyl)-2H-1,2,3-triazole-4-carboxylic acid (78.9 mg, 381 µmol) in DMF (2 ml) was added 1-ethyl-3-(3-dimethylaminopropyl)carbodiimide hydrochloride (94.9 mg, 495 µmol), 1-hydroxybenzotriazole hydrate (75.8 mg, 495 µmol) and N,N-diisopropylethylamine (460 µl, 2.7 mmol). The reaction mixture was stirred overnight at RT and concentrated under reduced pressure. The crude product was purified by preparative HPLC (Method 3f). After lyophilisation, 70.1 mg (100 % purity, 42 % yield) of the desired product were obtained.
LC-MS (Method 8): Rₜ = 0.80 min; MS (ESIpos): m/z = 442 [M+H]⁺
¹H-NMR (400 MHz, DMSO-d6) δ [ppm]: -0.008 (1.00), 0.008 (1.05), 0.528 (0.68), 0.538 (0.98), 0.542 (1.27), 0.552 (1.48), 0.556 (1.72), 0.562 (1.35), 0.566 (1.85), 0.569 (1.61), 0.574 (1.40), 0.579 (1.24), 0.584 (0.98), 0.684 (0.92), 0.689 (1.22), 0.693 (1.35), 0.699 (1.66), 0.702 (1.79), 0.706 (1.46), 0.712 (1.59), 0.716 (1.53), 0.726 (1.49), 0.729 (1.07), 0.740 (0.78), 0.977 (0.55), 0.986 (0.52), 0.990 (0.44), 0.999 (1.53), 1.009 (1.99), 1.013 (1.83), 1.019 (4.15), 1.024 (2.23), 1.029 (2.64), 1.034 (2.18), 1.039 (4.39), 1.045 (1.99), 1.050 (1.83), 1.059 (1.48), 1.068 (0.50), 1.072 (0.46), 1.082 (0.50), 1.895 (0.85), 1.907 (1.70), 1.916 (1.81), 1.920 (1.07), 1.928 (3.28), 1.936 (1.11), 1.941 (1.70), 1.949 (1.59), 1.962 (0.72), 2.524 (0.79), 3.048 (0.41), 4.201 (0.59), 4.216 (0.78), 4.235 (3.93), 4.244 (4.17), 4.250 (4.06), 4.260 (3.97), 4.278 (0.72), 4.295 (0.65), 7.447 (0.55), 7.455 (5.26), 7.461 (1.83), 7.478 (8.29), 7.487 (1.00), 7.494 (1.86), 7.499 (5.63), 7.508 (0.59), 8.066 (0.61), 8.075 (5.31), 8.081 (2.12), 8.087 (5.61), 8.093 (3.27), 8.098 (5.61), 8.105 (1.97), 8.110 (5.13), 8.119 (0.54), 8.358 (6.18), 8.465 (1.72), 8.481 (3.78), 8.490 (16.00), 8.496 (1.96), 8.877 (14.17), 10.980 (4.67).

### Example 158

### rac-N-{[4-(2,5-dimethyl-1,3-thiazol-4-yl)-2,5-dioxoimidazolidin-4-yl]methyl}-2-(4-fluorophenyl)-2H-1,2,3-triazole-4-carboxamide

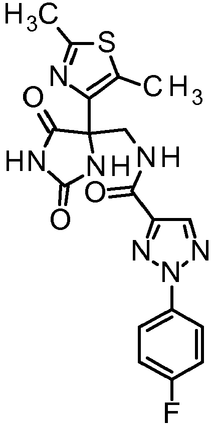

To a solution of rac-5-(aminomethyl)-5-(2,5-dimethyl-1,3-thiazol-4-yl)imidazolidine-2,4-dione hydrochloride (60.0 mg, 217 µmol) and 2-(4-fluorophenyl)-2H-1,2,3-triazole-4-carboxylic acid (44.9 mg, 217 µmol) in DMF (1.1 ml) was added 1-ethyl-3-(3-dimethylaminopropyl)carbodiimide hydrochloride (54.0 mg, 282 µmol), 1-hydroxybenzotriazole hydrate (43.2 mg, 282 µmol) and N,N-diisopropylethylamine (260 µl, 1.5 mmol). The reaction mixture was stirred overnight at RT and concentrated under reduced pressure. The crude product was purified by preparative HPLC (Method 3f). After lyophilisation, 27.2 mg (98 % purity, 29 % yield) of the desired product were obtained.
LC-MS (Method 8): Rₜ = 0.73 min; MS (ESIpos): m/z = 430 [M+H]⁺
¹H-NMR (400 MHz, DMSO-d6) δ [ppm]: -0.008 (0.94), 0.008 (0.94), 2.392 (16.00), 2.524 (0.81), 3.980 (1.51), 3.994 (2.32), 4.009 (1.51), 7.465 (2.21), 7.470 (0.79), 7.487 (3.62), 7.503 (0.77), 7.508 (2.40), 8.073 (2.32), 8.078 (0.94), 8.085 (2.45), 8.090 (1.40), 8.096 (2.42), 8.102 (0.84), 8.107 (2.23), 8.478 (7.43), 8.548 (3.34), 8.655 (0.68), 8.671 (1.48), 8.686 (0.69), 11.126 (0.48).

### Example 159

### rac-N-{[4-(1-chlorocyclopropyl)-2,5-dioxoimidazolidin-4-yl]methyl}-2-(4-fluorophenyl)-2H-1,2,3-triazole-4-carboxamide

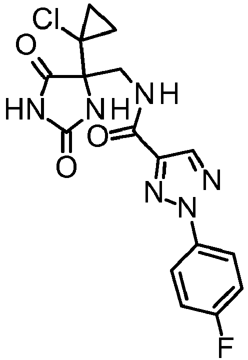

To a solution of rac-5-(aminomethyl)-5-(1-chlorocyclopropyl)imidazolidine-2,4-dione hydrochloride (50.0 mg, 208 µmol) and 2-(4-fluorophenyl)-2H-1,2,3-triazole-4-carboxylic acid (47.5 mg, 229 µmol) in DMF (1.1 ml) was added 1-ethyl-3-(3-dimethylaminopropyl)carbodiimide hydrochloride (51.9 mg, 271 µmol), 1-hydroxybenzotriazole hydrate (41.5 mg, 271 µmol) and N,N-diisopropylethylamine (220 µl, 1.2 mmol). The reaction mixture was stirred overnight at RT and concentrated under reduced pressure. The crude product was purified by preparative HPLC (Method 3f). After lyophilisation, 12.9 mg (99 % purity, 16 % yield) of the desired product were obtained.
LC-MS (Method 7): Rₜ = 1.43 min; MS (ESIpos): m/z = 393 [M+H]⁺
¹H-NMR (400 MHz, DMSO-d6) δ [ppm]: -0.008 (1.95), -0.006 (1.52), 0.006 (1.61), 0.008 (2.12), 0.014 (0.42), 1.051 (0.93), 1.066 (1.86), 1.075 (4.06), 1.091 (5.59), 1.104 (2.20), 1.118 (1.19), 1.214 (1.27), 1.226 (2.12), 1.240 (4.99), 1.255 (3.64), 1.268 (1.78), 1.280 (1.10), 2.073 (2.20), 2.323 (0.68), 2.328 (0.93), 2.333 (0.68), 2.367 (0.59), 2.391 (0.59), 2.524 (3.72), 2.558 (1.02), 2.560 (0.85), 2.563 (0.68), 2.565 (0.59), 2.568 (0.51), 2.666 (0.76), 2.670 (1.02), 2.675 (0.76), 2.710 (0.59), 3.048 (1.02), 3.269 (0.42), 3.272 (0.42), 3.274 (0.51), 3.277 (0.51), 3.279 (0.68), 3.282 (0.76), 3.285 (1.10), 3.299 (5.08), 3.347 (1.52), 3.352 (1.10), 3.663 (1.61), 3.678 (1.78), 3.697 (2.88), 3.713 (2.71), 3.774 (2.79), 3.790 (3.05), 3.809 (1.86), 3.825 (1.69), 7.452 (0.85), 7.461 (5.33), 7.466 (1.95), 7.483 (8.72), 7.493 (1.10), 7.499 (1.95), 7.505 (5.84), 7.514 (0.68), 8.062 (0.76), 8.071 (5.50), 8.077 (2.46), 8.083 (6.52), 8.094 (11.43), 8.100 (3.13), 8.106 (5.67), 8.114 (0.76), 8.467 (16.00), 8.483 (3.72), 8.499 (1.69), 10.860 (4.32).

### Example 160

### 2-(4-chloro-2-fluorophenyl)-N-{[(4R)-4-cyclopropyl-2,5-dioxoimidazolidin-4-yl]methyl}-2H-1,2,3-triazole-4-carboxamide

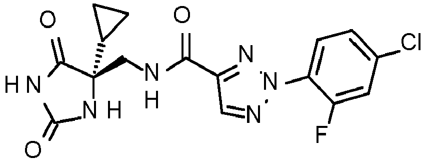

2-(4-chloro-2-fluorophenyl)-2H-1,2,3-triazole-4-carboxylic acid (58.7 mg, 243 µmol) dissolved in 1.5 ml of DMF was treated with N-ethyl-N-isopropylpropan-2-amine (120 µl, 680 µmol), 3-{[(ethylimino)methylene]amino}-N,N-dimethylpropan-1-amine hydrochloride (60.6 mg, 316 µmol), 1H-benzotriazol-1-ol hydrate (48.4 mg, 316 µmol) and (5R)-5-(aminomethyl)-5-cyclopropylimidazolidine-2,4-dione hydrochloride (58.7 mg, 243 µmol). The mixture was stirred over night at room temperature. The product was purified by preparative HPLC (Instrument: Waters Prep LC/MS System, Column: Phenomenex Kinetex C18 5µm 100x30 mm; eluent A: water, eluent B: acetonitrile, eluent C: 2 % formic acid in water, eluent D: acetonitrile/water (80Vol.% / 20Vol.%); flow: 80 ml/min , room temperature, UV-detection: 200-400 nm, At-Column Injektion; gradient: eluent A 0 bis 2 min 63 ml, eluent B 0 to 2 min 7 ml, eluent A 2 to 10 min from 63 ml to 39 ml and eluent B from 7 ml to 31 ml, 10 to 12 min 0 ml eluent A and 70 ml eluent B. Eluent C and eluent D constant flow 5 ml/min over the whole runtime). After lyophilization, 65.0 mg (100 % purity, 68 % yield) of the title compound were obtained.
LC-MS (Method 7): Rₜ = 1.44 min; MS (ESIpos): m/z = 393 [M+H]⁺
¹H-NMR (600 MHz, CHLOROFORM-d) δ [ppm]: 0.363 (0.53), 0.371 (1.27), 0.380 (1.89), 0.388 (2.18), 0.397 (1.87), 0.405 (0.86), 0.413 (0.68), 0.422 (1.21), 0.428 (1.47), 0.436 (2.03), 0.444 (1.84), 0.453 (2.02), 0.461 (2.60), 0.470 (2.55), 0.478 (2.10), 0.487 (1.47), 0.496 (0.49), 0.595 (0.87), 0.603 (1.45), 0.609 (1.83), 0.613 (1.47), 0.618 (2.28), 0.623 (1.32), 0.627 (1.41), 0.631 (1.02), 0.642 (0.54), 1.202 (0.90), 1.211 (1.77), 1.216 (1.93), 1.220 (1.35), 1.225 (3.03), 1.233 (1.63), 1.238 (1.49), 1.247 (0.65), 1.864 (1.08), 3.731 (2.45), 3.741 (2.65), 3.755 (3.04), 3.764 (2.54), 4.086 (2.81), 4.099 (2.91), 4.110 (2.59), 4.123 (2.44), 6.468 (3.64), 7.207 (2.86), 7.208 (2.99), 7.223 (3.33), 7.234 (3.78), 7.238 (2.58), 7.252 (3.60), 7.255 (3.11), 7.664 (2.92), 7.678 (4.83), 7.692 (2.70), 7.896 (1.56), 8.197 (16.00).

### Example 161

### N-{[(4R)-4-cyclopropyl-2,5-dioxoimidazolidin-4-yl]methyl}-2-(2,4-dichlorophenyl)-2H-1,2,3-triazole-4-carboxamide

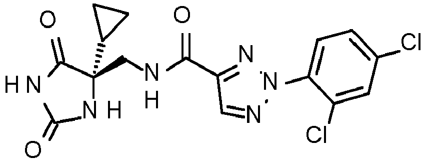

2-(2,4-dichlorophenyl)-2H-1,2,3-triazole-4-carboxylic acid (62.7 mg, 243 µmol) dissolved in 1.5 ml of DMF was treated with N-ethyl-N-isopropylpropan-2-amine (120 µl, 680 µmol), 3-{[(ethylimino)methylene]amino}-N,N-dimethylpropan-1-amine hydrochloride (60.6 mg, 316 µmol), 1H-benzotriazol-1-ol hydrate (48.4 mg, 316 µmol) and (5R)-5-(aminomethyl)-5-cyclopropylimidazolidine-2,4-dione hydrochloride (50.0 mg, 243 µmol). The mixture was stirred over night at room temperature. The product was purified by preparative HPLC (Instrument: Waters Prep LC/MS System, Column: Phenomenex Kinetex C18 5µm 100x30 mm; eluent A: water, eluent B: acetonitrile, eluent C: 2 % formic acid in water, eluent D: acetonitrile/water (80Vol.% / 20Vol.%); flow: 80 ml/min , room temperature, UV-detection: 200-400 nm, At-Column Injektion; gradient: eluent A 0 bis 2 min 63 ml, eluent B 0 to 2 min 7 ml, eluent A 2 to 10 min from 63 ml to 39 ml and eluent B from 7 ml to 31 ml, 10 to 12 min 0 ml eluent A and 70 ml eluent B. Eluent C and eluent D constant flow 5 ml/min over the whole runtime). After lyophilization, 64.0 mg (100 % purity, 64 % yield) of the title compound were obtained.
LC-MS (Method 7): Rₜ = 1.52 min; MS (ESIpos): m/z = 409 [M+H]⁺
¹H-NMR (600 MHz, CHLOROFORM-d) δ [ppm]: 0.380 (0.43), 0.387 (0.89), 0.396 (1.77), 0.405 (2.36), 0.411 (1.36), 0.414 (1.60), 0.421 (0.97), 0.430 (1.77), 0.439 (3.61), 0.444 (3.33), 0.450 (4.71), 0.457 (2.79), 0.459 (2.60), 0.465 (1.35), 0.584 (0.55), 0.597 (1.51), 0.602 (1.38), 0.604 (1.32), 0.608 (1.48), 0.612 (1.84), 0.618 (1.69), 0.626 (0.92), 0.631 (0.54), 1.194 (0.81), 1.203 (1.48), 1.207 (1.59), 1.212 (1.23), 1.217 (2.88), 1.221 (1.18), 1.226 (1.45), 1.229 (1.32), 1.239 (0.65), 2.002 (0.47), 2.638 (15.90), 3.747 (2.30), 3.757 (2.53), 3.771 (2.92), 3.781 (2.55), 4.047 (2.64), 4.060 (2.76), 4.071 (2.43), 4.083 (2.31), 6.416 (3.14), 7.367 (3.87), 7.371 (3.81), 7.381 (4.68), 7.385 (4.47), 7.397 (0.67), 7.401 (0.60), 7.411 (0.73), 7.415 (0.68), 7.515 (7.99), 7.530 (6.70), 7.560 (8.03), 7.563 (7.19), 7.588 (1.43), 7.596 (1.68), 7.599 (2.03), 7.602 (2.09), 7.613 (1.70), 8.242 (16.00), 8.302 (2.27), 8.695 (0.86).

### Example 162

### rac-N-[(4-ethyl-2,5-dioxoimidazolidin-4-yl)methyl]-2-(4-fluorophenyl)-2H-1,2,3-triazole-4-carboxamide

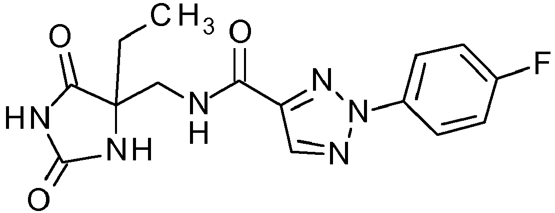

2-(4-fluorophenyl)-2H-1,2,3-triazole-4-carboxylic acid (53.5 mg, 258 µmol) dissolved in 2 ml of DMF was treated with N-ethyl-N-isopropylpropan-2-amine (130 µl, 720 µmol), 3-{[(ethylimino)methylene]amino}-N,N-dimethylpropan-1-amine hydrochloride (64.4 mg, 336 µmol), 1H-benzotriazol-1-ol hydrate (51.4 mg, 336 µmol) and rac-5-(aminomethyl)-5-ethylimidazolidine-2,4-dione hydrochloride (50.0 mg, 258 µmol). The mixture was stirred over night at room temperature. The product was purified by preparative HPLC (Instrument: Waters Prep LC/MS System, Column: Phenomenex Kinetex C18 5 µm 100x30 mm; eluent A: water, eluent B: acetonitrile, eluent C: 2 % formic acid in water, eluent D: acetonitrile/water (80Vol.% / 20Vol.%); flow: 80 ml/min , room temperature, UV-detection: 200-400 nm, At-Column Injektion; gradient: eluent A 0 bis 2 min 63 ml, eluent B 0 to 2 min 7 ml, eluent A 2 to 10 min from 63 ml to 39 ml and eluent B from 7 ml to 31 ml, 10 to 12 min 0 ml eluent A and 70 ml eluent B. Eluent C and eluent D constant flow 5 ml/min over the whole runtime). After lyophilization, 66.0 mg (100 % purity, 74 % yield) of the title compound were obtained.
LC-MS (Method 7): Rₜ = 1.23 min; MS (ESIpos): m/z = 347 [M+H]⁺
¹H-NMR (400 MHz, DMSO-d6) δ [ppm]: -0.008 (0.52), 0.008 (0.54), 0.767 (6.30), 0.785 (15.08), 0.804 (6.90), 1.595 (1.10), 1.613 (1.66), 1.630 (2.55), 1.648 (2.17), 1.665 (1.14), 1.683 (2.35), 1.701 (2.62), 1.718 (1.73), 1.736 (1.10), 2.074 (1.42), 3.504 (1.01), 3.520 (1.18), 3.538 (3.66), 3.554 (3.84), 3.560 (3.85), 3.577 (3.58), 3.594 (1.14), 3.611 (1.04), 7.451 (0.52), 7.460 (5.27), 7.465 (1.85), 7.482 (8.97), 7.491 (1.02), 7.498 (1.88), 7.504 (5.79), 7.512 (0.61), 7.766 (5.54), 8.075 (0.58), 8.084 (5.45), 8.089 (2.22), 8.096 (5.79), 8.101 (3.36), 8.107 (5.73), 8.113 (2.05), 8.119 (5.33), 8.128 (0.55), 8.470 (16.00), 8.502 (1.66), 8.518 (3.46), 8.534 (1.62), 10.686 (4.45).

### EXPERIMENTAL SECTION - Evaluation of pharmacological activity

### Example B1: Production of human ADAMTS-7

To study the activity of human ADAMTS-7 (hADAMTS-7), the inventors generated multiple constructs for the production of active ADAMTS-7 in *E coli* cells. These constructs contain the catalytic domain alone or catalytic domain with the Prodomain (Pro), Disintegrin domain (Dis), or TSR1 (Figure 2 panel A). Different tags such as 6xHis, GST, MBP, SUMO or Trigger factor (TF) were incorporated to the N-terminus of the constructs to improve the solubility of the protein and to facilitate protein purification, but none of these constructs produced soluble and active ADAMTS-7 to support further studies. Therefore, we tested catalytic domain containing constructs along with secretion signals in Expi293 mammalian cells. ADAMTS-7 proteins secreted into culture media were captured by affinity column and analyzed by analytical size exclusion column (SEC). A construct containing the secretion signal peptide (SP), Pro and CD domains of hADAMTS-7 (residues 1-537, now referred to as "hPro-hCD" in light of the later discovered hybrids in which the prodomain can be from a non-human species) with an affinity tag at the C-terminus was eluted largely in the void volume from SEC and yielded -0.2 mg/L of soluble ADAMTS-7 proteins in the elution fractions 3-6 (Figure 1 panel A). Based on the size and N-terminal sequencing results of the bands on the SDS-page, the soluble ADAMTS-7 is confirmed to be a mixture of amino acid (aa) 28-537 (hPro-hCD, unprocessed ADAMTS-7), aa 237-537 (hCD domain only, furin processed ADAMTS-7), and fragments from Pro. The mixture was dominated by the unprocessed ADAMTS-7, which accounts for -90% of the population. A construct containing the secretion SP, an affinity/solubility tag and only hADAMTS-7 CD domain (residues 237-537) yielded mostly soluble aggregates (void). Soluble ADAMTS-7 proteins were only detectible by western blot (fractions B8-B12) (Figure 3 panel A). Another construct that ends at TSR1 (hSP-hPro-hCD-hTSR1, residues 1-593), instead failed to overexpress soluble ADAMTS-7 proteins. Neither SDS-PAGE nor western blot can detect significant ADAMTS-7 proteins (Figure 3 panel B). In comparison, rat ADAMTS-7 (rADAMTS-7) residues 1-575 (rSP-rPro-rCD-rTSR1) produced -0.6 mg/L soluble proteins (fractions1-6) from Expi293 cells with negligible aggregates (void) eluted from SEC. Purified rat ADAMTS-7 contains -1:1 molar ratio of unprocessed (aa 25-575) and processed (aa 218-575) ADAMTS-7, and some fragments from Pro (Figure 1 panel B).

### Example B2: Hybrid molecules to improve the production of soluble hADAMTS-7

To improve the production of hADAMTS-7 protein, we also explored various other options. For example, we compared and analyzed the sequences of rat and human ADAMTS-7 in the Pro and CD domains (Figure 2 panel B). CD domain sequence is well conserved. The amino acid sequence in the CD domain of rat and human is 84% identical (97% similar), compared to Prodomain which is 70% identical (89% similar). The rest of the sequences are different (i.e., neither identical nor similar) between the two species. Specifically, the CD domain has only 10 different residues between rat and human over 302 residues, while Pro has 22 different ones over 202 residues (Figure 2 panel B). To test whether rat Pro played a role in facilitating protein folding and yielding more soluble ADAMTS-7 proteins, we designed hybrid molecules of rat SP-Pro (1-217) followed by human CD (237-537), named rPro-hCD (Figure 1 panel C). The hybrid molecule was purified by Ni affinity column and analyzed by SEC as done to hADAMTS-7 (1-537). Little was eluted as aggregates in the void volume preceding the soluble peak (fractions 2-8). The soluble proteins were analyzed as a mixture of the unprocessed (aa 25-537) and processed (aa 237-537) ADAMTS-7, and Pro (Figure 1 panel C). The yield of ADAMTS-7 protein after affinity and SEC two-step purification was 2.2mg/L for rPro-hCD. This is in contrast with ∼0.2mg/L yield of the hPro-hCD, namely hADAMTS-7 (1-537). Our result suggests that the rat Pro is more effective in driving effective folding of the CD domain, therefore improving the yield of the soluble ADAMTS-7 proteins -10 fold. Therefore, this example demonstrates a solution for the problem of protein solubility as well as for the problem of expression yield, both of which were lower for the fully human construct (hPro-hCD) as compared to the hybrid construct (rPro-hCD).

### Example B3: Engineering furin cleavage site to manipulate the production ratio of the processed and unprocessed ADAMTS-7

We hypothesized multiple furin cleavage sites in rat Pro (Figure 4 panel A), one after residue R58 or R60 (RVLR⁵⁸↓KR⁶⁰↓D) of Pro, and another between Pro and CD domains after R217 (RQQR ²¹⁷↓S). These cleavage sites were found conserved in rat, mouse and human Pro. Sequential cleavage or processing at these furin sites by cellular furin enzyme leading to a complete removal of the Pro domain from the rest of the protein is likely a necessary step to a fully active or mature ADAMTS-7. Recombinant production of hPro-hCD and rPro-hCD revealed an inefficient furin processing in the mammalian cell culture (Figure 1 panels A and C). Both constructs yielded a mixture of processed and unprocessed ADAMTS-7. We hypothesized that this could be attributed to a less sufficient amount of endogenous furin produced by mammalian cells or less optimal furin recognition sequence in current ADAMTS-7 constructs. We tested the idea of co-transfection DNAs of rPro-hCD and furin protease into Expi293 cells to coexpress the two proteins. That yielded overexpression of furin in the media but significantly reduced the production of ADAMTS-7 proteins without increasing the ratio of processed versus unprocessed ADAMTS-7. Next, we focused on the optimization of furin recognition site. We introduced mutation Q216K into rPro-hCD to convert ²¹⁴RQQR²¹⁷ to ²¹⁴RQKR²¹⁷, and named it rPro-hCD-FM2 (SEQ ID No. 2) (Figure 4 panel A). Q216K mutation significantly increased furin cleavage efficiency of ADAMTS-7 in the same mammalian cell culture compared to the wild type (WT). The ratio of hCD or processed ADAMTS-7 in the raw expression media versus rPro-hCD or unprocessed ADAMTS-7 increased at least 6 fold (Figure 4 panel B). As a control, a triple mutation R58A/R60A/R217A was introduced into rPro-hCD to silent predicted furin cleavage sites (Figure 4 panel A). rPro-hCD-3RA completely abolished furin processing and yielded only the unprocessed ADAMTS-7 (Figure 4 panel B). We conclude that manipulating the sequence of predicted furin recognition site has proved capable of changing the amount of processed and unprocessed of ADAMTS-7 molecules produced in mammalian cell culture. Thus, both the polypeptide of SEQ ID NO: 01 and that of SEQ ID NO: 02 are working solutions for the problems of protein solubility and expression yield, although the polypeptide of SEQ ID NO: 02 achieves a particularly improved ratio for the furin-processed polypeptide.

### Example B4: Expression constructs for production of recombinant hybrid ADAMTS-7 enzymes

The human ADAMTS-7 catalytic domain with the endogenous human prodomain did not yield well folded secreted protein with enzymatic activity. Exchange of the human prodomain with the rat prodomain dramatically increased production of the human catalytic domain from hybrid rat-human constructs. Rat/human ADAMTS-7 chimera sequence (rPro-hCD (Rat 1-217/Human 237-537)-TEV-2Strep-6His, SEQ ID No. 01) encoding rat pro-domain of ADAMTS-7 (amino acids 1-217 of rat sequence UniProt Q1EHB3, which also includes the signal peptide) and catalytic domain of human ADAMTS-7 (amino acids 237-537 of human sequence UniProt Q9UKP4, which also includes a disintegrin domain) followed by TEV cleavage sequence, 2xStrep tag and a His Tag was cloned into the mammalian pcDNA6mycHis (ThermoFischer Scientific) expression vector (or into pcDNA3.4 vector in some embodiments).

Rat/human ADAMTS-7 chimera sequence (rPro-hCD-FM2 (Rat 1-217/Human 237-537 FM2 (Q216K))-TEV-2Strep-6His, SEQ ID No. 02) encoding rat pro-domain of ADAMTS-7 (amino acids 1-217 of rat sequence UniProt Q1EHB3, which also includes the signal peptide) and catalytic domain of human ADAMTS-7 (amino acids 237-537 of human sequence UniProt Q9UKP4, which also includes a disintegrin domain) followed by TEV cleavage sequence, 2xStrep tag and a His Tag was cloned into the mammalian pcDNA6mycHis (ThermoFischer Scientific) expression vector (or into pcDNA3.4 vector in some embodiments). SEQ ID No. 02 contains an additional mutation Glutamine 216 to Lysine within the rat pro-domain sequence (RQQR217↓S to RQKR217↓S), which improved cleavage by Furin for zymogen processing.

These expression constructs allow production of recombinant ADAMTS-7 enzyme - either containing at least parts of both domains (e.g., prodomain plus catalytic domain as encoded, in which the prodomain can optionally be preceded by a signal peptide and/or the catalytic domain can optionally be followed by a disintegrin domain) or containing primarily the catalytic domain (e.g., catalytic domain as encoded, for example as generated after furin cleavage between the prodomain and the catalytic domain, which catalytic domain can optionally be followed by a disintegrin domain).

### Example B5 Recombinant production of active ADAMTS-7 enzyme

Expi293 cells (A14635, ThermoFischer Scientific) were grown and transfected in accordance to the manufacturer instruction. In brief, at the final cell density of 2.5 × 10⁶ cells/mL with >95% viability Expi 293 cells were transfected with 1 mg/liter of vector plasmid DNA as generated according to example 4 using Expifectamine transfection reagent (Thermo Fischer Scientific).

Approximately 96 hours post transfection, Expi293 cell culture was centrifuged at 4000 rpm (-3700 rcf) for 10 mins, and supernatant was collected. Supernatant was neutralized with 50 mM Tris pH8.0, 5 mM CaCl₂, 10 uM ZnCl₂ and 5 mM imidazole pH 8.0 and filtered through 0.22 µm filter.

The filtered supernatant was loaded on Ni-NTA column (GE healthcare #17-3712-06), equilibrated with buffer A (50 mM Tris pH 8.0, 300 mM NaCl, 10% glycerol, 5 mM CaCl₂, 10 µM ZnCl2 and 5 mM Imidazole (pH 8.0)) on Acta FPLC system. The column was washed with 20 volumes of buffer A and the bound proteins were eluted by linear gradient of 20 column volumes up to 100% buffer B (50 mM Tris pH 8.0, 300 mM NaCl, 10% glycerol, 5mM CaCl₂, 10 µM ZnCl₂ and 500mM Imidazole (pH 8.0)). The collected fractions were analyzed on the SDS gel and the fractions containing ADAMTS-7 protein were combined and concentrated 10 times.

The concentrated material from the Ni-NTA purification was loaded onto superdex S200 (SEC) column equilibrated in column buffer: 50 mM Tris pH 8.0, 300 mM NaCl, 10% glycerol, 5 mM CaCl2 and 10 µM ZnCl2. The collected fractions were analyzed on the SDS gel and the fractions containing ADAMTS-7 protein were combined and concentrated 10 times.

The concentrated material from the Ni-NTA purification was loaded onto superdex S200 (SEC) column equilibrated in column buffer: 50 mM Tris pH 8.0, 300 mM NaCl, 10% glycerol, 5 mM CaCl2 and 10 µM ZnCl₂. The collected fractions were analyzed on the SDS gel and the fractions containing ADAMTS-7 protein were combined.

Combined S200 fractions were loaded to strep-tactin column (Qiagen #1057981) equilibrated in Buffer A (50 mM Tris 8.0, 300 mM NaCl, 10% glycerol, 5 mM CaCl₂, 10 µM ZnCl₂). The column was washed with 20 column volumes of the buffer A and the bound proteins were eluted by linear gradient of 20 column volumes up to 100% buffer B (50 mM Tris 8.0, 300 mM NaCl, 10% glycerol, 5 mM CaCl₂, 10 uM ZnCl₂ and 2.5 mM D-desthiobiotin). The collected fractions were analyzed on the SDS gel and the fractions containing ADAMTS-7 protein were combined. Combined fractions were dialysed overnight at +4°C against the storage buffer (20 mM Tris pH 8.0, 300 mM NaCl, 10% glycerol, 5 mM CaCl₂ and 10 µM ZnCl₂).

Dialyzed protein was concentrated to 1 mg/ml, aliquoted, flash frozen in dry ice/ethanol and stored at -80°C. The final yield of purified ADAMTS-7 was 0.5 - 1 mg per liter of Expi293 cell culture.

In some embodiments, the produced polypeptide is further processed (e.g., by TEV protease) to remove some of the parts C-terminal to the catalytic domain.

### Example B6: Identification of ADAMTS-7 substrates and cleavage assay development

Based on the interaction mapping data and the apparent fragment size in reducing and non-reducing conditions, the results were consistent with a ADAMTS-7 cleavage site near the second EGF repeat of COMP, however the substrate cleavage site has not been defined.

In an attempt to identify ADAMTS-7 substrate cleavage sites, we scanned the potential regions of COMP and TSP1 to identify ADAMTS consensus sites and generated a series of internally-quenched fluorescently-labeled peptides for use with our purified ADAMTS-7 enzyme (Figures 7). Since ADAMTS-7 was reported to produce the 100 kDA COMP fragment resolvable on a gel under reducing and non-reducing conditions, we hypothesized that cleavage site would not be internal to a disulfide bond. Given the 1-3, 2-4, 5-6 ensemble of disulfide bonds within the EGF repeats, only the E152↓A153 site between the 4^{th} and 5^{th} cysteines committed to separate disulfide bonds was considered, which we attempted to emulate using acetamidomethyl (Acm) modified cysteines in the COMP candidate peptide (See Figure 7) A second peptide from COMP (amino acids 73-84 GMQQ↓SVRTGLPS) was chosen based on observed ADAMTS cleavage of full-length COMP identified by N-terminal sequencing (data not shown). TSP1 contained a potential E↓LRG upstream from the ADAMTS1 cleavage site at E311↓L312. We chose to analyze this portion of TSP1 using a series of overlapping peptides. The COMP and TSP1 candidate regions contained a modified rhodamine AF488 dye coupled to the N-terminus and a local acceptor QXL520 quencher at the C-terminus that prevents fluorescence in the uncleaved configuration (Figure 7). Following endopeptidic cleavage, the quencher is released to allow fluorescence signal detection from the substrate's amino terminus. Purified recombinant ADAMTS-7 (as of SEQ ID No. 01 or SEQ ID No. 02) and purified recombinant rADAMTS-7 (rat ADAMTS-7 residues 1-575 with a carboxyl terminal Flag, SEQ ID No. 03) were used to identify substrates from the TSP1 and COMP candidate peptides. ADAMTS-7 was diluted in reaction buffer (20 mM HEPES pH 8.0, 150 mM NaCl, 5 mM CaCl₂, 0,004 % Brij, 10 µM ZnCl₂) for a concentration of approximately 100 nM and pre-incubated for 2 hr at room temperature prior to reaction start with 10 µM candidate peptide substrates. There were some differences between optimized buffer data and reported 2 mM Zn²⁺ buffer data (Liu 2006 FASEB J); for example, the published amount of Zinc in assay buffer caused the purified ADAMTS-7 proteins to precipitate. Candidate FRET substrates based on SEQ ID No. 04 to 10 were generated through custom synthesis by Anaspec - AnaSpec, EGT (34801 Campus Drive, Fremont, CA 94555, USA); as explained by the manufacturer; AnaSpec, EGT Group's pH insensitive HiLyte™ Fluor dyes are a series of fluorescent labeling dyes with fluorescence emissions that span the full visible and near infrared spectrum. HiLyte™ Fluor dyes and AnaSpec's proprietary quenchers QXL™ have been used as fluorophore and quencher pairs for fluorescence resonance energy transfer (FRET) in our examples) in the reaction buffer. Activity data plotted as Relative Fluorescence Units over time and as a calculated rate (RFU/min) are shown in Figure 8. Significant activity was not observed with substrates in buffer alone (Figure 8) or with incubation with purified ADAMTS-7 E389Q catalytic inactive proteins (data not shown).

ADAMTS-7 human catalytic domain constructs rPro-hCD hybrid (SEQ ID No. 01) and optimized furin site construct FM2 (SEQ ID No. 02) demonstrated the greatest specificity for the TSP1 S1 (amino acids 275-289: DELSSMVLELRGLRT, SEQ ID No. 04). ADAMTS-7 human catalytic domain constructs also cleaved the overlapping TSP1 S2 substrate (amino acids 278-292: SSMVLELRGLRTIVT, SEQ ID No. 05). Substrates S1 and S2 are overlapping at candidate site to E289↓L290 (Figure 7). Cleavage at this site was confirmed by mass spec using an unlabeled peptide (data not shown). No significant activity was detected at the defined ADAMTS1 cleavage site E311↓L312 from TSP1 S3 (amino acids 300-314: KVTEENKELANERR, SEQ ID No. 06) or TSP1 S4 (amino acids 303-317 EENKELANERRPPL, SEQ ID No. 07). This suggests that the ADAMTS-7 substrate is distinct from ADAMTS1. To confirm the TSP1 E289↓L290 substrate site, a minimum overlap between the S1 and S2 peptides was tested as TSP1 S5 substrate (amino acids 278-289: SSMVLELRGLRT, SEQ ID No. 08). Activity was observed for the S5 substrate with ADAMTS-7 human catalytic domain constructs compared to buffer alone, however the removal of the amino DEL sequence greatly reduced the signal for activity compared to the S1 substrate DELSSMVLELRGLRT, SEQ ID No. 04. No activity was observed with COMP candidate peptides COMP1 amino acids 73-84: GMQQSVRTGLPS, SEQ ID No. 09 or COMP2 amino acids 146-159: SPGFRCEACPPGYS, SEQ ID No. 10.

Activity data from the rat ADAMTS-7 construct identified TSP1 S1 (SEQ ID No. 04) as the preferred substrate, along with S2 and S5 peptides containing the E289↓L290 cleavage site (Figure 8). S1 relative fluorescence signal was not as strong for rat ADAMTS-7 compared to the ADAMTS-7 human catalytic domain constructs, resulting in a lower ratio of S1 to S2 activity.

TSP1 S2 substrate (SSMVLELRGLRTIVT, SEQ ID No. 05) presented limited solubility compared to the preferred TSP1 S1 substrate (DELSSMVLELRGLRT, SEQ ID No. 04), potentially due to the additional hydrophobic residues at the carboxyl terminal side (data not shown). To further improve solubility of the S1 peptide, which contained a number of internal hydrophobic residues, modified versions of the S1 peptide ending in -K(QXL520)-NH2 were generated to include an additional hydrophilic moiety: -K(QXL520)-E-NH2 (SEQ ID No. 11), - K(QXL520)-K-NH2 (SEQ ID No. 12) and -K(QXL520)-OH (SEQ ID No. 13). Activity profiles for these substrates were not significantly affected, however the substrate solubility profile was improved with the additional carboxyl glutamic acid (i.e., a glutamic acid that has been conjugated at a carboxyl position on the peptide) added after the QXL520 quencher for SEQ ID No. 11 (data not shown).

### Example B7 Assay for ADAMTS-7 enzymatic activity and testing of inhibitory compounds

Purified recombinant ADAMTS-7 (as of SEQ ID No. 01 or SEQ ID No. 02) was diluted in reaction buffer (20 mM HEPES pH 8.0, 150 mM NaCl, 5 mM CaCl₂, 0,004 % Brij, 10 µM ZnCl₂) for a concentration of approximately 20 nM. 25 µl of the solution were transferred into each well of a 384-well white microtiter plate (Greiner Bio-One 781075) and 1 µl test compound solution (modulator/inhibitor dissolved in DMSO, at the corresponding concentration) or pure DMSO as a control were added per well. The enzymatic reaction was initiated by addition of 25 µl of a 1 µM solution of the FRET substrate, HiLyteFluor-488 DELSSMVLELRGLRT-K(QXL520)-E-NH2; (SEQ ID No. 11, custom synthesis by Anaspec) in the reaction buffer. Amino acids DELSSMVLELRGLRT are derived from Thrombospondin-1 sequence (275-289). An additional carboxyl glutamic acid was added after the QXL520 quencher to increase substrate solubility. The microtiter plate was incubated for 120 min at the temperature of 32°C. The increase of fluorescence intensity was measured in appropriate fluorescence plate reader (e.g. TECAN Ultra) using excitation wavelength of 485 nm and emission wavelength of 520 nm. IC50 values were calculated from percentage of inhibition of ADAMTS-7 activity as a function of test compound concentration. IC50 values derived using functional ADAMTS-7 according to SEQ ID No. 01 or SEQ ID No. 02, respectively, were not distinguishable, both laying within the experimental error.

### Example B8: Expression constructs for production of recombinant ADAMTS-12 enzymes

Rat/human ADAMTS-12 chimera sequence (rPro-hCD (Rat 1-244/Human 241-543)-3xFLAG, SEQ ID 15) encoding rat pro-domain of ADAMTS-12 (amino acids 1-244 of rat sequence UniProt D3ZTJ3, which also includes the signal peptide) and catalytic domain of human ADAMTS-12 (amino acids 241-543 of human sequence UniProt P58397, which also includes a disintegrin domain) followed by 3xFLAG Tag was cloned into the mammalian pcDNA3.4 expression vector.

Rat/human ADAMTS-12 WT demonstrated a better expression profile compared to human ADAMTS-12 (1-543) WT with a human prodomain (Figure 6). Optimization of the furin cleavage site (L237R) in the context of the human prodomain did not improve the yield of processed CD proteins compared to the rat/human ADAMTS-12 construct. Each ADAMTS-12 construct was mutated in parallel at the catalytic site with an E393Q substitution (EQ) resulting in increased protein yield similar to ADAMTS-7 catalytic mutations. Corresponding yield for the rat/human ADAMTS-12 EQ construct was also higher compared to ADAMTS-12 (1-543) EQ containing the human prodomain.

This expression construct allows production of recombinant ADAMTS-12 enzymes - either containing at least parts of both domains (e.g., prodomain plus catalytic domain as encoded, in which the prodomain can optionally be preceded by a signal peptide and/or the catalytic domain can optionally be followed by a disintegrin domain) or containing primarily the catalytic domain (e.g., catalytic domain as encoded, which can optionally be followed by a disintegrin domain).

### Example B9: Recombinant production of active ADAMTS-12 enzyme

Expi293 cells (Life technologies, A14635) were grown and transfected in accordance to the manufacturer instruction. Briefly, the Expi 293 cells at the final cell density of 2.5 × 10⁶ cells/mL with >95% viability were transfected by the 1 mg/liter of vector plasmid DNA using Expifectamine transfection reagent (Life technologies, A14525). The overall purification scheme was similar to that used for ratADAMTS-7 (SEQ ID NO: 03).

Approximately 72 hours post transfection, Expi293 cell culture was centrifuged at 4000rpm (∼3700rcf) for 10 mins. Supernatant was collected and neutralized with 50mM Tris pH 8.0, 5 mM CaCl₂, 10 µM ZnCl₂ before centrifuged again at 4000rpm (∼3700rcf) for 10 min. Final supernatant was filtered through 0.22 µm filter.

The filtered supernatant was incubated overnight at 4°C with anti-FLAG M2 affinity gel (Sigma-Aldrich A2220) equilibrated with buffer A (50 mM Tris pH 8.0, 300 mM NaCl, 10% glycerol, 5 mM CaCl₂, 10 µM ZnCl₂). The gel was collected and washed with 10 bed volumes of buffer A. The bound proteins were eluted by 100% buffer B (50 mM Tris pH 8.0, 300 mM NaCl, 10% glycerol, 5 mM CaCl₂, 10 µM ZnCl₂, 150 ng/µl FLAG peptide (Sigma-Aldrich F4799)). The collected fractions were analyzed on the SDS gel. The fractions containing ADAMTS-12 protein were combined and concentrated 10 times.

The concentrated material from the FLAG affinity purification was loaded onto superdex S200 (SEC) column equilibrated in column buffer: 20 mM Tris pH 8.0, 300 mM NaCl, 10% glycerol, 5 mM CaCl₂ and 10 µM ZnCl₂. The collected fractions were analyzed on the SDS gel. Fractions containing ADAMTS-12 protein were combined and concentrated to 0.5 mg/ml. Aliquoted proteins were flash frozen in liquid nitrogen and stored at -80°C.

### Example B10: Assay for ADAMTS-12 enzymatic activity and testing of inhibitory compounds

Purified recombinant ADAMTS-12 was diluted in the reaction buffer (20 mM HEPES pH 8.0; 10 mM NaCl; 7,5 mM CaCl₂; 0,004% Brij; 7,5 µM ZnCl₂; 0,1% SmartBlock (Candor Bioscience 113125)) to the concentration of approximately 20 nM and 25 µl were transferred into each single well of 384-well white microtiter plate (Greiner Bio One 781075). 1 µl of the inhibitor compound solution (dissolved in DMSO, at the corresponding concentration) or pure DMSO as a control was added to the same wells. The enzymatic reaction was initiated by addition of 25 µl of 2 µM solution of the FRET substrate HiLyte Fluor 488-DELSSMVLELRGLRT-K(QXL520)E-NH2; (cf. SEQ ID No. 11) in the reaction buffer. It was surprisingly found that the same substrate could be used for the paralogs ADAMTS-7 and ADAMTS-12. The microtiter plate was incubated for 120 min at the temperature of 32°C. The increase of fluorescence intensity was measured in appropriate fluorescence plate reader (e.g. TECAN Ultra) using excitation wavelength of 485 nm and emission wavelength of 520 nm. IC50 values were calculated from percentage of inhibition of ADAMTS-12 activity as a function of test compound concentration.

### Example B11: Selectivity assay for ADAMTS-7 and/or ADAMTS-12 modulators

The respective enzyme (see table 2 below) was diluted in reaction buffer (50 mM Tris, 2.5 µM ZnCl₂, 0.05 % BSA, 0.001 % Brij, pH 7.5 for ADAM17; 50 mM Tris 7.5, 150 mM NaCl, 10 mM CaCl₂, 0.05% Brij for all other enzymes) to the respective concentration and 25 µl were transferred into each well of a 384-well white microtiter plate (Greiner Bio One 781075). 1 µl test compound solution (dissolved in DMSO, at the corresponding concentration) or pure DMSO as a control was added per well. The enzymatic reaction was initiated by addition of 25 µl of the respective concentration of the respective FRET substrate (see table 2 below) in reaction buffer. The microtiter plate was incubated for 120 min at the temperature of 32°C. The increase of fluorescence intensity was measured in appropriate fluorescence plate reader (e.g. TECAN Ultra) using the respective wavelengths for excitation and emission. IC50 values were calculated from percentage of inhibition of enzyme activity as a function of test compound concentration.

**Table 2. Assay conditions for the evaluation of the selectivity of ADAMTS-7 modulators. Abbreviations: Dabcyl: e.g. Dabcyl quencher in 3-DAB form N-[4-(4-dimethylamino)phenylazo]benzoic acid; Glu(Edans): e.g. EDANS fluor (5-((2-Aminoethyl)amino)naphthalene-1-sulfonic acid) at modified Glutamic acid; Mca: (7-Methoxycoumarin-4-yl)acetyl; Dap(Dnp): e.g. N-beta-(2,4-dinitrophenyl)-L-2,3-diaminopropionic acid; Dpa: N-3-(2, 4-Dinitrophenyl)-L-2,3-diaminopropionyl.**

| Enzyme | Source Enzyme | Conc. Enzyme | FRET substrate | Conc. Substrate | Source Substrate | Excitation Wavelength | Emission Wavelength |
|---|---|---|---|---|---|---|---|
| ADAMTS4 | R&D 4307-AD | 50 nM | Dabcyl-EEVKAKVQPY-Glu(Edans)-NH2 | 1 µM | Jerini Peptide Technologies | 340 nm | 480 nm |
| | | | (cf. SEQ ID No. 14) | | | | |
| ADAMTS5 | R&D 2198-AD | 100 nM | Dabcyl-E EVKAKVQ PY-Glu(Edans)-NH2 | 25 µM | Jerini Peptide Technologies | 340 nm | 480 nm |
| | | | (cf. SEQ ID No. 14) | | | | |
| MMP12 | R&D 917-MPB, activated according to manufacturers instruction | 1 nM | Mca-PLGLEEA-Dap(Dnp)-NH2 | 10 µM | Bachem M-2670 | 325 nm | 393 nm |
| | | | (cf. SEQ ID No. 17) | | | | |
| MMP15 | R&D 916-MP, activated according to manufacturers instruction | 6 nM | Mca-KPLGL-Dpa-AR-NH2 (cf. SEQ ID No. 18) | 20 µM | R&D ES010 | 320 nm | 405 nm |
| | | | (Neumann, U. et al., 2004, Anal. Biochem. 328:166 - 173) | | | | |
| MMP2 | R&D 902-MP, activated according to manufacturers instruction | 0,06 nM | Mca-PLGL -Dpa-AR-NH2 | 20 µM | R&D ES001 | 320 nm | 405 nm |
| | | | (cf. SEQ ID No. 19) | | | | |
| ADAM17 | R&D 930-ADB | 20 nM | Mca-PLAQAV-Dap(Dnp)-RSSSR-NH2 | 20 µM | Bachem M-2255 | 320 nm | 405 nm |
| | | | (cf. SEQ ID No. 20) | | | | |

**Table 3:**

| Example Nr. | ADAMTS7-IC₅₀ [nM] | ADAMTS4-IC₅₀ [nM] |
|---|---|---|
| 1 | 13 | 580 |
| 2 | 210 | 50000 |
| 3 | 79 | 2200 |
| 4 | 260 | 6600 |
| 5 | 120 | 4600 |
| 6 | 620 | 50000 |
| 7 | 62 | 8500 |
| 8 | 7 | 175 |
| 9 | 26 | 480 |
| 10 | 21 | 450 |
| 11 | 470 | 10000 |
| 12 | 980 | 12000 |
| 13 | 47 | 950 |
| 14 | 400 | 17000 |
| 15 | 140 | 4750 |
| 16 | 355 | 24000 |
| 17 | 32 | 600 |
| 18 | 170 | 2200 |
| 19 | 170 | 22000 |
| 20 | 26 | 4800 |
| 21 | 200 | 2800 |
| 22 | 10 | 3000 |
| 23 | 30 | 4500 |
| 24 | 140 | 2900 |
| 25 | 550 | 50000 |
| 26 | 480 | 50000 |
| 27 | 100 | 6000 |
| 28 | 870 | 67000 |
| 29 | 5 | 250 |
| 30 | 12 | 500 |
| 31 | 41 | 4250 |
| 32 | 35 | 910 |
| 33 | 58 | 1400 |
| 34 | 26 | 1420 |
| 35 | 74 | 3100 |
| 36 | 110 | 5500 |
| 37 | 180 | 7600 |
| 38 | 10 | 2800 |
| 39 | 710 | 50000 |
| 40 | 980 | 25000 |
| 41 | 55 | 1700 |
| 42 | 350 | 12000 |
| 43 | 320 | 13000 |
| 44 | 250 | 5000 |
| 45 | 69 | 1450 |
| 46 | 52 | 4690 |
| 47 | 98 | 7650 |
| 48 | 265 | 14500 |
| 49 | 70 | 15000 |
| 50 | 191 | 50000 |
| 51 | 89 | 15000 |
| 52 | 55 | 14000 |
| 53 | 145 | 50000 |
| 54 | 670 | 25000 |
| 55 | 240 | 19000 |
| 56 | 129 | 1800 |
| 57 | 190 | 17000 |
| 58 | 550 | 5600 |
| 59 | 550 | 11300 |
| 60 | 259 | 8400 |
| 61 | 128 | 9950 |
| 62 | 42 | 16000 |
| 63 | 69 | 6000 |
| 64 | 240 | 50000 |
| 65 | 170 | 19000 |
| 66 | 380 | 50000 |
| 67 | 260 | 9300 |
| 68 | 120 | 8300 |
| 69 | 98 | 35000 |
| 70 | 76 | 10000 |
| 71 | 47 | 13100 |
| 72 | 130 | 6200 |
| 73 | 60 | 5100 |
| 74 | 33 | 5900 |
| 75 | 14 | 4100 |
| 76 | 8 | 2770 |
| 77 | 24 | 4380 |
| 78 | 191 | 19000 |
| 79 | 78 | 11000 |
| 80 | 250 | 2900 |
| 81 | 37 | 1500 |
| 82 | 48 | 3100 |
| 83 | 120 | 11000 |
| 84 | 150 | 4000 |
| 85 | 250 | 6500 |
| 86 | 63 | 8500 |
| 87 | 98 | 20000 |
| 88 | 98 | 13000 |
| 89 | 6 | 975 |
| 90 | 16 | 3100 |
| 91 | 18 | 1800 |
| 92 | 78 | 4000 |
| 93 | 8 | 2200 |
| 94 | 6 | 890 |
| 95 | 10 | 980 |
| 96 | 48 | 2200 |
| 97 | 10 | 1900 |
| 98 | 25 | 1100 |
| 99 | 52 | 6000 |
| 100 | 8 | 680 |
| 101 | 7 | 14000 |
| 102 | 62 | 2900 |
| 103 | 2 | 200 |
| 104 | 10 | 2200 |
| 105 | 190 | 11000 |
| 106 | 190 | >50000 |
| 107 | 7 | 310 |
| 108 | 12 | 600 |
| 109 | 19 | 1100 |
| 110 | 5 | 400 |
| 111 | 35 | 1200 |
| 112 | 14 | 780 |
| 113 | 39 | 2400 |
| 114 | 15 | 1400 |
| 115 | 87 | 6600 |
| 116 | 78 | 5500 |
| 117 | 20 | 3300 |
| 118 | 46 | 4500 |
| 119 | 13 | 2300 |
| 120 | 170 | 6000 |
| 121 | 78 | 2600 |
| 122 | 11 | 2500 |
| 123 | 7 | 910 |
| 124 | 13 | 2800 |
| 125 | 7 | 1000 |
| 126 | 190 | 12000 |
| 127 | 10 | 2400 |
| 128 | 29 | 2400 |
| 129 | 18 | 1700 |
| 130 | 14 | 2800 |
| 131 | 37 | 2500 |
| 132 | 230 | 7400 |
| 133 | 50 | 3200 |
| 134 | 190 | 6200 |
| 135 | 48 | 6600 |
| 136 | 27 | 3500 |
| 137 | 37 | 1000 |
| 138 | 590 | 50000 |
| 139 | 69 | >50000 |
| 140 | 4 | 360 |
| 141 | 4 | 320 |
| 142 | 4 | 320 |
| 143 | 5 | 200 |
| 144 | 2 | 130 |
| 145 | 2 | 41 |
| 146 | 3 | 108 |
| 147 | 3 | 46 |
| 148 | 12 | 2500 |
| 150 | 38 | 2000 |
| 151 | 320 | 17000 |
| 152 | 560 | 5000 |
| 153 | 83 | 8900 |
| 154 | 190 | 7900 |
| 155 | 220 | >50000 |
| 156 | 59 | 15000 |
| 157 | 8 | 460 |
| 158 | 100 | 2800 |
| 159 | 74 | 3700 |
| 160 | 60 | 1000 |
| 161 | 130 | 7100 |
| 162 | 200 | 13000 |

### Example B12 Rat carotid artery balloon injury model

The balloon injury model is an important method to study the molecular and cellular mechanisms involved in vascular smooth muscle dedifferentiation, neointima formation and vascular remodeling.

The left carotid is injured using a balloon catheter with the right carotid serving as a negative control; the inflated balloon denudes the endothelium and distends the vessel wall. Following injury, potential therapeutic strategies such as the use of pharmacological compounds can be evaluated.

Following sedation to a surgical plane, male Sprague-Dawley rats are laid supine on a sterile surface, the neck area is shaved, cleansed and the surgical area aseptically draped to prevent contamination at the surgical site. A straight incision is made centrally in the neck region from below the chin to the top of the sternum. With continued blunt dissection, the left common carotid artery and the distal aspect of the carotid artery cephalic to the internal and external bifurcation are isolated and made free of overlying fascia and adjacent nerves. Sterile sutures and an arterial clamp are used to control blood flow in order to perform the injury. Isolating a section on the internal carotid artery and using sterile small microscissors, a transverse arteriotomy on the branch is performed. The uninflated 2 French arterial balloon catheter (Edwards Life Sciences, Germany) is inserted through the arteriotomy, inflated and moved down the entire length of the common carotid artery to the aortic arch. The balloon is inflated to a predetermined volume, deflated and withdrawn through the arteriotomy. Following adequate hemostasis, all remaining sutures are removed and the operational field is closed up. Neointimal thickening representing proliferative vascular smooth muscle cells usually peaks at 2 weeks after injury. Vessels are harvested at this time point for histological assessment of neointimal development, vascular growth, molecular analysis as well as gene and protein expression.

## Claims

1. A compound of general formula (I): 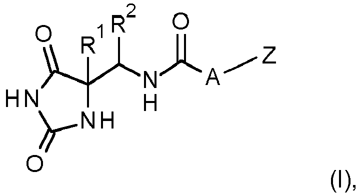 in which
R¹ represents a group selected from hydrogen, (C₁-C₆)-alkyl, (C₃-C₆)-cycloalkyl, 5- to 6-membered heterocycloalkyl, 5- to 6-membered heteroaryl and phenyl,
wherein said (C₁-C₆)-alkyl, (C₃-C₆)-cycloalkyl, 5- to 6-membered heterocycloalkyl, 5- to 6-membered heteroaryl and phenyl are optionally substituted, with one or two groups independently selected from cyano, halogen, amino, hydroxy, oxo, (C₁-C₃)-alkyl, (C₁-C₄)-alkoxy, (C₁-C₄)-alkylcarbonyl, mono-(C₁-C₄)-alkylamino, di-(C₁-C₄)-alkylamino, (C₁-C₄)-alkylsulfonyl, (C₃-C₆)-cycloalkyl, ,
wherein each said (C₁-C₃)-alkyl, (C₃-C₆)-cycloalkyland, (C₁-C₄)-alkoxy is optionally substituted with up to 5 fluorine atoms
R² represents a group selected from hydrogen or (C₁-C₄)-alkyl,
wherein said (C₁-C₄)-alkyl is optionally substituted with up to five fluorine atoms,
A represents a group selected from 5-membered heteroaryl,
wherein said 5-membered heteroaryl is optionally substituted with one, two or three groups independently selected from halogen, cyano, hydroxy, methylsulfonyl, (C₃-C₆)-cycloalkyl, (C₁-C₄)-alkyl, and (C₁-C₄)-alkoxy,
wherein each said (C₃-C₆)-cycloalkyl, (C₁-C₄)-alkylor (C₁-C₄)-alkoxy is optionally substituted with up to three fluorine atoms,
Z represents a group selected from 6- to 10-membered aryl and 5- to 10-membered heteroaryl,
wherein said 6- to 10-membered aryl or 5- to 10-membered heteroaryl is optionally substituted with one, two or three groups independently selected from halogen, cyano, hydroxy, (C₁-C₄)-alkylsulfonyl, (C₃-C₆)-cycloalkyl, (C₁-C₄)-alkyl, and (C₁-C₄)-alkoxy,
wherein each said (C₁-C₄)-alkyl and (C₁-C₄)-alkoxy is optionally substituted with (C₃-C₆)-cycloalkyl and optionally substituted with up to five fluorine atoms,
and pharmaceutically acceptable salts thereof, solvates thereof and the solvates of the salts thereof.

2. The compound of claim 1, with the proviso that the following compounds (Xa) to (Xi) are excluded
4-(4-chlorophenyl)-N-[[4-(1-methylimidazol-2-yl)-2,5-dioxo-imidazolidin-4-yl]methyl]-1H-pyrrole-2-carboxamide 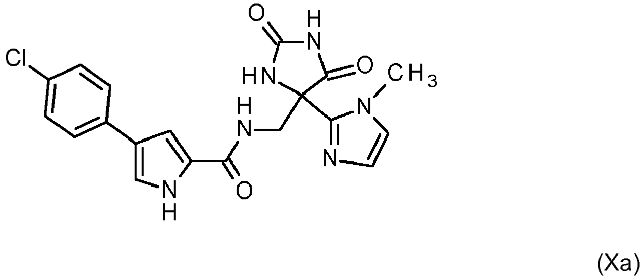
1-(4-fluorophenyl)-N-[[4-(1-methylimidazol-2-yl)-2,5-dioxo-imidazolidin-4-yl]methyl]pyrazole-4-carboxamide 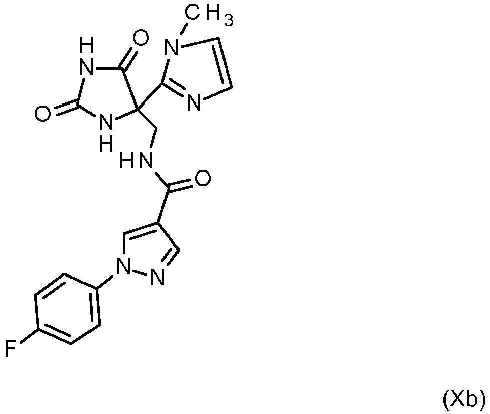
1-methyl-N-[[4-(1-methylimidazol-2-yl)-2,5-dioxo-imidazolidin-4-yl]methyl]-5-phenyl-pyrrole-2-carboxamide 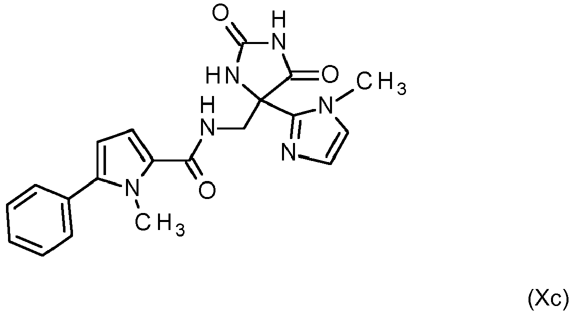
4-(5-chloro-2-thienyl)-N-[[4-(1-methylimidazol-2-yl)-2,5-dioxo-imidazolidin-4-yl]methyl]-1 H-pyrrole-3-carboxamide 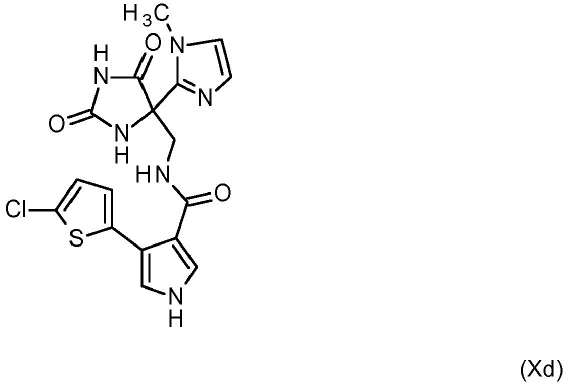
4-methyl-N-[[4-(1-methylimidazol-2-yl)-2,5-dioxo-imidazolidin-4-yl]methyl]-2-(2-thienyl)thiazole-5-carboxamide 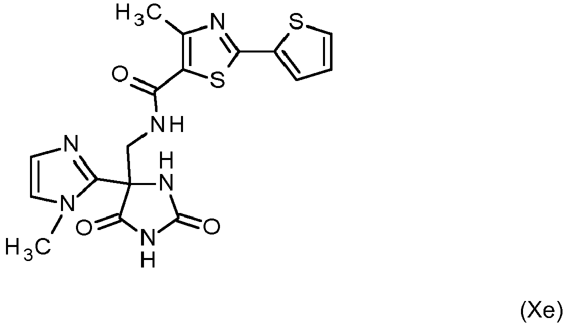
N-[[4-(1-methylimidazol-2-yl)-2,5-dioxo-imidazolidin-4-yl]methyl]-2-(4-pyridyl)thiazole-5-carboxamide 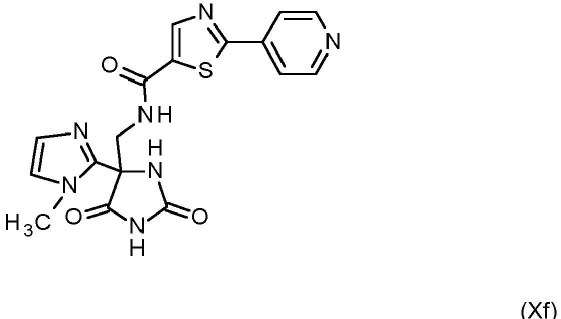
5-methyl-N-[[4-(1-methylimidazol-2-yl)-2,5-dioxo-imidazolidin-4-yl]methyl]-1-(4-pyridyl)pyrazole-4-carboxamide 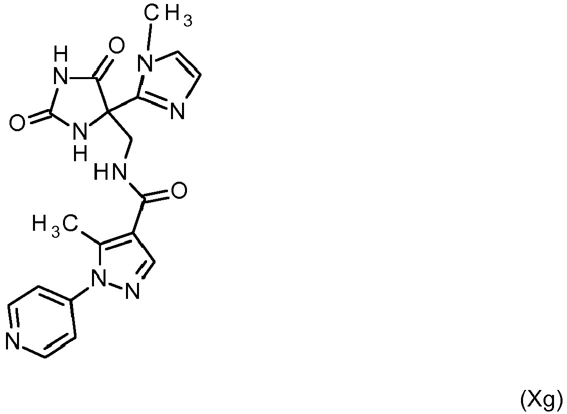
N-[[4-(1-methylimidazol-2-yl)-2,5-dioxo-imidazolidin-4-yl]methyl]-5-(1 H-pyrazol-3-yl)thiophene-2-carboxamide 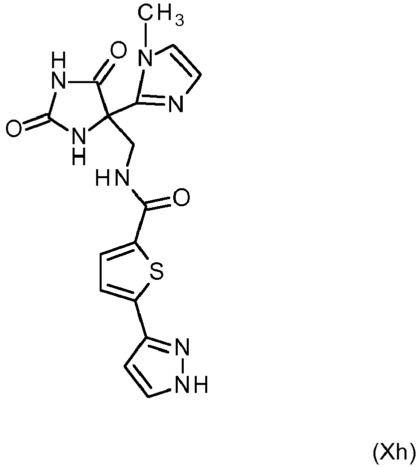
N-[[4-(1-methylimidazol-2-yl)-2,5-dioxo-imidazolidin-4-yl]methyl]-5-phenyl-1H-pyrazole-3-carboxamide

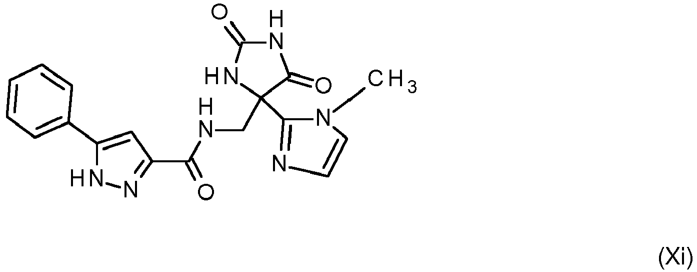

3. A compound of general formula (I) according to Claim 1 or Claim 2,
in which
R¹ represents a group selected from hydrogen, (C₁-C₆)-alkyl, (C₃-C₆)-cycloalkyl, 5- to 6-membered heterocycloalykl, 5- to 6-membered heteroaryl and phenyl
wherein said (C₁-C₆)-alkyl (C₃-C₆)-cycloalkyl, 5- to 6-membered heterocycloalkyl, 5- to 6-membered heteroaryl and phenyl are optionally substituted, with one or two groups independently selected from cyano, halogen, amino, hydroxy, oxo, C₁-C₃-alkyl, (C₁-C₄)-alkoxy, (C₁-C₄)-alkylcarbonyl, mono-(C₁-C₄)-alkylamino, di-(C₁-C₄)-alkylamino, (C₁-C₄)-alkylsulfonyl, (C₃-C₆)-cycloalkyl, ,
wherein each said (C₁-C₃)-alkyl, (C₃-C₆)-cycloalkyl, and (C₁-C₄)-alkoxy is optionally substituted with up to 5 fluorine atoms,
R² represents a group selected from hydrogen, (C₁-C₄)-alkyl,
wherein said (C₁-C₄)-alkyl is optionally substituted with up to five halogen atoms,
A represents a group selected from 5-membered aza-heteroaryl,
wherein said 5-membered aza-heteroaryl is optionally substituted with one, two or three groups independently selected from halogen, cyano, hydroxy, methylsulfonyl, (C₃-C₆)-cycloalkyl, (C₁-C₄)-alkyl, and (C₁-C₄)-alkoxy,
wherein each said (C₁-C₄)-alkyl, (C₁-C₄)-alkoxy is optionally substituted with up to three fluorine atoms,
Z represents a group selected from Phenyl and 5- to 6- membered heteroaryl,
optionally substituted, with one, two or three groups independently selected from halogen, cyano, hydroxy, (C₁-C₄)-alkylsulfonyl, (C₃-C₆)-cycloalkyl, (C₁-C₄)-alkyl, and (C₁-C₄)-alkoxy,
wherein each said (C₁-C₄)-alkyl and (C₁-C₄)-alkoxy is optionally substituted with (C₃-C₆)-cycloalkyl and optionally substituted with up to five fluorine atoms,
and pharmaceutically acceptable salts thereof, solvates thereof and the solvates of the salts thereof.

4. A compound of general formula (I) according to any one of Claims 1-3, in which
R¹ represents a group selected from hydrogen, (C₁-C₆)-alkyl, (C₃-C₆)-cycloalkyl, 5- to 6-membered heterocycloalkyl, 5- to 6-membered heteroaryl and phenyl
wherein said (C₁-C₆)-alkyl (C₃-C₆)-cycloalkyl, 5- to 6-membered heterocycloalkyl, 5- to 6-membered heteroaryl and phenyl are optionally substituted, with one or two groups independently selected from cyano, halogen, amino, hydroxy, oxo, C₁-C₃-alkyl, (C₁-C₄)-alkoxy, (C₁-C₄)-alkylcarbonyl, mono-(C₁-C₄)-alkylamino, di-(C₁-C₄)-alkylamino, , , (C₁-C₄)-alkylsulfonyl, (C₃-C₆)-cycloalkyl ,
wherein each said C₁-C₃-alkyl, (C₃-C₆)-cycloalkyl and (C₁-C₄)-alkoxy is optionally substituted with up to 5 fluorine atoms
R² represents a group selected from hydrogen or methyl
A represents a group selected from triazolyl, oxazolyl, isoxazolyl, pyrazolyl, thiazolyl, 1,2,4-oxadiazolyl and 1,3,4-oxadiazolyl
optionally substituted with one, two or three groups independently selected from halogen, cyano, hydroxy, methylsulfonyl, (C₃-C₆)-cycloalkyl, (C₁-C₄)-alkyl and (C₁-C₄)-alkoxy
wherein each said (C₁-C₄)-alkyl, (C₁-C₄)-alkoxy is optionally substituted with up to three fluorine atoms,
Z represents a group selected from Phenyl and 5- to 6-membered heteroaryl,
optionally substituted, with one, two or three groups independently selected from halogen, cyano, hydroxy, methylsulfonyl, (C₃-C₆)-cycloalkyl, (C₁-C₄)-alkyl, (C₁-C₄)-alkoxy,
wherein each said (C₁-C₄)-alkyl and (C₁-C₄)-alkoxy is optionally substituted with, (C₃-C₆)-cycloalkyl and optionally substituted with up to five fluorine atoms,
and pharmaceutically acceptable salts thereof, solvates thereof and the solvates of the salts thereof.

5. Compound of the formula (I) according to Claim 1, 2 3, or 4, in which
R¹ represents a group selected from hydrogen, (C₁-C₆)-alkyl, (C₃-C₆)-cycloalkyl, 5- to 6-membered heterocycloalkyl, 5- to 6-membered heteroaryl and phenyl
wherein said (C₁-C₆)-alkyl (C₃-C₆)-cycloalkyl, 5- to 6-membered heterocycloalkyl, 5- to 6-membered heteroaryl and phenyl are optionally substituted, with one or two groups independently selected from cyano, halogen, amino, hydroxy, oxo, C₁-C₃-alkyl, (C₁-C₄)-alkoxy, (C₁-C₄)-alkylcarbonyl, mono-(C₁-C₄)-alkylamino, di-(C₁-C₄)-alkylamino, (C₁-C₄)-alkylsulfonyl, (C₃-C₆)-cycloalkyl,
wherein each said C₁-C₃-alkyl, (C₃-C₆)-cycloalkyl, (C₁-C₄)-alkoxy is optionally substituted with up to 5 fluorine atoms
R² represents a group selected from hydrogen or methyl,
A represents a triazolyl
optionally substituted with one, two or three groups independently selected from halogen, cyano, hydroxy, methylsulfonyl, (C₃-C₆)-cycloalkyl, (C₁-C₄)-alkyl, and (C₁-C₄)-alkoxy,
wherein each said (C₁-C₄)-alkyl, (C₁-C₄)-alkoxy is optionally substituted with up to three fluorine atoms,
Z represents a group selected from Phenyl and 5 to 6 membered heteroaryl,
optionally substituted, with one, two or three groups independently selected from halogen, cyano, hydroxy, methylsulfonyl, (C₃-C₆)-cycloalkyl, (C₁-C₄)-alkyl, and (C₁-C₄)-alkoxy,
wherein each said (C₁-C₄)-alkyl and (C₁-C₄)-alkoxy is optionally substituted with (C₃-C₆)-cycloalkyl and optionally substituted with up to five fluorine atoms,
and pharmaceutically acceptable salts thereof, solvates thereof and the solvates of the salts thereof.

6. Compound of the formula (I) according to Claim 1 to 5 selected from the group consisting of
N-{[(4R)-4-cyclopropyl-2,5-dioxolmidazolidin-4-yl]methyl}-2-(2,4-difluorophenyl)-2H-1,2,3-triazole-4-carboxamide 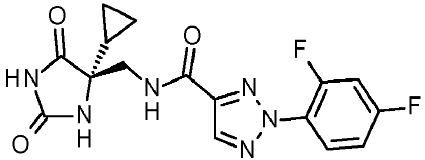
N-{[(4R)-4-cyclopropyl-2,5-dioxoimidazolidin-4-yl]methyl}-2-(4-fluorophenyl)-2H-1,2,3-triazole-4-carboxamide 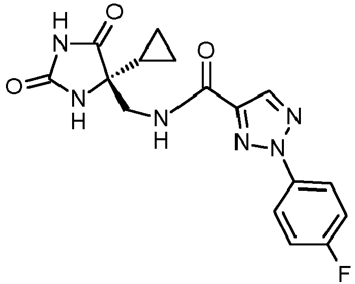
2-(3-chloro-4-fluorophenyl)-N-{[(4R)-4-cyclopropyl-2,5-dioxoimidazolidin-4-yl]methyl}-2H-1,2,3-triazole-4-carboxamide 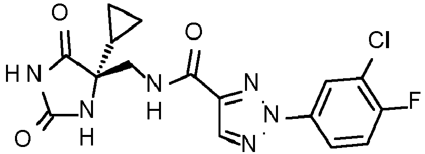
*ent*-N-[(2,5-dioxo-4-(propan-2-yl)imidazolidin-4-yl)methyl]-2-(4-fluorophenyl)-2H-1,2,3-triazole-4-carboxamide 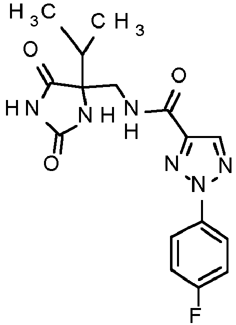
*ent*-N-[(4-cyclobutyl-2,5-dioxoimidazolidin-4-yl)methyl]-2-(4-fluorophenyl)-2H-1,2,3-triazole-4-carboxamide 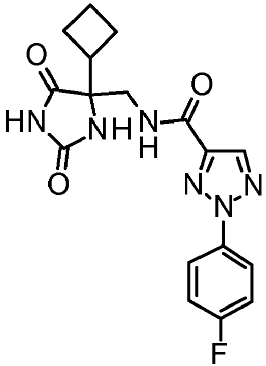
*ent*-N-[(4-cyclobutyl-2,5-dioxoimidazolidin-4-yl)methyl]-2-(3,4,5-trifluorophenyl)-2H-1,2,3-triazole-4-carboxamide 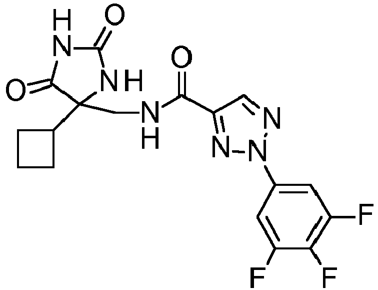
*ent*-N-[(4-cyclobutyl-2,5-dioxoimidazolidin-4-yl)methyl]-2-(3-fluorophenyl)-2H-1,2,3-triazole-4-carboxamide 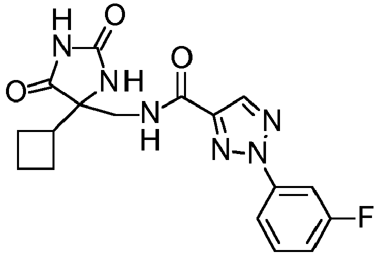 and
*ent*-N-[(4-cyclobutyl-2,5-dioxoimidazolidin-4-yl)methyl]-2-(3,4-difluorophenyl)-2H-1,2,3-triazole-4-carboxamide 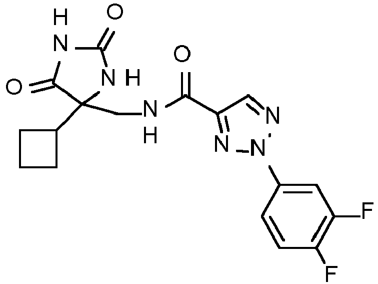
and pharmaceutically acceptable salts thereof, solvates thereof and the solvates of the salts thereof.

7. Compound of formula (I) for the treatment and/or prevention of diseases.

8. Compound of formula (I) for use in a method for the treatment and/or prevention of heart diseases, vascular diseases, and/or cardiovascular diseases, lung diseases, inflammatory diseases, fibrotic diseases, metabolic diseases, cardiometabolic diseases.

9. Compound of formula (I) for use in a method for the treatment and/or prevention of atherosclerosis ,athersclerosis-related diseases such as coronary artery disease or peripheral vascular disease/arterial occlusive disease as well as post-surgery complications of these diseases such as restenosis after angioplasty.

10. Use of a compound according to formula (I) for the manufacture of a pharmaceutical composition for the treatment and/or prevention of atherosclerosis ,athersclerosis-related diseases such as coronary artery disease or peripheral vascular disease/arterial occlusive disease as well as post-surgery complications of these diseases such as restenosis after angioplasty.

11. Use of a compound as defined in any of Claims 1 to 6 for the manufacture of a pharmaceutical composition for the treatment and/or prevention of atherosclerosis ,athersclerosis-related diseases such as coronary artery disease or peripheral vascular disease/arterial occlusive disease as well as post-surgery complications of these diseases such as restenosis after angioplasty.

12. Pharmaceutical composition comprising a compound as defined in any of Claims 1 to 6 and one or more pharmaceutically acceptable excipients.

13. Pharmaceutical composition of claim 12 comprising one or more first active ingredients, in particular compounds of general formula (I) according to any one of claims 1 to 6, and one or more further active ingredients, in particular one or more additional therapeutic agents selected from the group consisting of angiotensin-converting enzyme inhibitors, angiotensin-receptor blockers, mineralocorticoid-receptor antagonists, endothelin antagonists, renin inhibitors, calcium blockers, beta-receptor blockers, vasopeptidase inhibitors, Sodium-Glucose-Transport-Antagonists, Metformin, Pioglitazones and Dipeptidyl-peptidase-IV inhibitors.

14. The pharmaceutical composition as defined in Claim 12 or 13 for the treatment and/or prevention of atherosclerosis ,athersclerosis-related diseases such as coronary artery disease or peripheral vascular disease/arterial occlusive disease as well as post-surgery complications of these diseases such as restenosis after angioplasty.

15. Method for the treatment and/or prevention atherosclerosis ,athersclerosis-related diseases such as coronary artery disease or peripheral vascular disease/arterial occlusive disease as well as post-surgery complications of these diseases such as restenosis after angioplastyin a human or other mammal, comprising administering to a human or other mammal in need thereof a therapeutically effective amount of one or more compounds as defined in any of Claims 1 to 6, or of a pharmaceutical composition as defined in any of claims 12-13.

16. A recombinant nucleic acid for expression of an ADAMTS-7 polypeptide that comprises a rodent prodomain of ADAMTS-7 as a first portion and a functional human ADAMTS-7 as a second portion

17. A recombinant nucleic acid for expression of an ADAMTS-7 polypeptide, wherein the polypeptide comprises
a first portion having a sequence identity of > 80 % with the sequence of residues 1-217 of SEQ ID NO: 1 or with the sequence of residues 1-217 of SEQ ID NO: 2; and
a second portion having a sequence identity of > 80 % with the sequence of residues 218-518 of SEQ ID NO: 1.

18. The recombinant nucleic acid of any one of claims 16 or 17, wherein the first portion of the polypeptide comprises residues 1-217 of SEQ ID NO: 1 or residues 1-217 of SEQ ID NO: 2, and/or wherein the second portion amino acid sequence comprises residues 218-518 of SEQ ID NO: 1.

19. A recombinant nucleic acid for expression of an ADAMTS-7 polypeptide, wherein the recombinant nucleic acid encodes for a recombinant polypeptide that comprises
a first portion having an amino acid sequence that aligns with a segment of an ADAMTS-7 prodomain amino acid sequence from a first species with a Needleman-Wunsch score greater than 700 when BLOSUM62 matrix, a gap opening penalty of 11, and a gap extension penalty of 1 are used; and
a second portion having an amino acid sequence that aligns with a segment of an ADAMTS-7 catalytic domain amino acid sequence from a second species with a Needleman-Wunsch score greater than 1000 when BLOSUM62 matrix, a gap opening penalty of 11, and a gap extension penalty of 1 are used,
wherein optionally the first species is rat and the second species is human.

20. The recombinant nucleic acid of any one of claims 16 to 19, wherein the motif RQQR within the first portion of the polypeptide is altered, preferably into RQKR.

21. A recombinant nucleic acid for expression of an ADAMTS-12 polypeptide that comprises a rodent prodomain of ADAMTS-12 as a first portion and a functional human ADAMTS-12 as a second portion.

22. A recombinant nucleic acid for expression of an ADAMTS-12 polypeptide that comprises
a first portion having a sequence identity of > 80 % with the sequence of residues 1-244 of SEQ ID NO: 15, and
a second portion having a sequence identity of > 80 % with the sequence of residues 245-547 of SEQ ID NO: 15.

23. The recombinant nucleic acid of any one of claims 21 or 22, wherein the first portion comprises residues 1-244 of SEQ ID NO: 15, and/or wherein the second portion amino acid sequence comprises residues 245-547 of SEQ ID NO: 15.

24. A recombinant nucleic acid for expression of an ADAMTS-12 polypeptide, wherein the recombinant nucleic acid encodes for a recombinant polypeptide that comprises
a first portion having an amino acid sequence that aligns with a segment of an ADAMTS-12 prodomain amino acid sequence from a first species with a Needleman-Wunsch score greater than 800 when BLOSUM62 matrix, a gap opening penalty of 11, and a gap extension penalty of 1 are used; and
a second portion having an amino acid sequence that aligns with a segment of an ADAMTS12 catalytic domain amino acid sequence from a second species with a Needleman-Wunsch score greater than 1000 when BLOSUM62 matrix, a gap opening penalty of 11, and a gap extension penalty of 1 are used
wherein optionally the first species is rat and the second species is human.

25. The recombinant nucleic acid of any one of claims 21 to 24, wherein the second portion comprises an E393Q mutation.

26. The recombinant nucleic acid of any of claims 16 to 25, wherein the encoded polypeptide or the second portion thereof is suited to cleave a peptide comprising standard residues 1-15 of the amino acid sequence of SEQ ID NO: 4, preferably with a kcat/KM of at least 20% of a corresponding kcat/KM of human ADAMTS-7 or human ADAMTS-12.

27. A recombinant polypeptide encoded by the recombinant nucleic acid according to any of claims 16 to 26, or a fragment of that recombinant polypeptide, wherein said recombinant polypeptide or fragment thereof is suited to cleave a peptide substrate comprising standard residues 1-15 of SEQ ID NO: 4.

28. A peptide substrate for ADAMTS-7 and/or ADAMTS-12, the peptide substrate comprising
residues 1-15 of the amino acid sequence of SEQ ID NO: 4, or
residues 1-15 of the amino acid sequence of SEQ ID NO: 5, or
residues 1-13 of the amino acid sequence of SEQ ID NO: 8, or
a fragment of any of the sequences according to a), b) or c), the fragment comprising the amino acid sequence EL.

29. The peptide substrate of claim 28, wherein the peptide substrate comprises a first moiety conjugated to a residue that is N-terminal to sequence fragment EL comprised within SEQ ID No. 4, 5, or 8 or the fragment thereof, and a second moiety conjugated to a residue that is C-terminal to said sequence fragment EL.

30. The peptide substrate of claim 29, wherein the first moiety comprises a fluorophore and the second moiety comprises a quencher, or wherein the first moiety comprises a quencher and the second moiety comprises a fluorophore.

31. A method for the identification or characterization of an ADAMTS-7 and/or ADAMTS-12 modulator comprising the steps of
contacting a recombinant polypeptide or a fragment thereof according to claim 27 with at least one test compound;
contacting said recombinant polypeptide or fragment thereof with a peptide substrate according to any one of claims 28 to 30, wherein the peptide substrate comprises a fluorophore and a quencher; and
detecting fluorescence as a measure for the activity of said recombinant polypeptide or a fragment thereof.

32. A modulator of ADAMTS-7 and/or ADAMTS-12 identified by a method according to claim 31 for use in the treatment of coronary artery disease (CAD), peripheral vascular disease (PAD) and myocardial infarction (MI).

33. A method of producing a recombinant polypeptide according to claim 32, the method comprising
cultivating a recombinant host cell comprising a recombinant nucleic acid according to any of claims 16 to 26
recovering the recombinant polypeptide of a fragment thereof according to claim 34.

34. Kit of parts comprising at least one recombinant nucleic acid according to any of claims 16 to 26 or at least one polypeptide according to claim 27 and a peptide substrate according to any of claims 28 to 30.
